(19) Europäisches Patentamt
European Patent Office
Office européen des brevets

(11) EP 2 818 472 A1

(12) **EUROPEAN PATENT APPLICATION**

(43) Date of publication:
**31.12.2014 Bulletin 2015/01**

(21) Application number: **13173968.2**

(22) Date of filing: **27.06.2013**

(51) Int Cl.:
***C07D 471/04*** *(2006.01)* ***A61K 31/437*** *(2006.01)*
***A61P 3/00*** *(2006.01)* ***A61P 3/10*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.**
**80539 München (DE)**

(72) Inventors:
- **Ullrich, Axel (Prof. Dr.)**
  **80331 München (DE)**
- **Falcenberg, Mathias (Dr.)**
  **81377 München (DE)**

(74) Representative: **Arth, Hans-Lothar**
**ABK Patent Attorneys**
**Jasminweg 9**
**14052 Berlin (DE)**

(54) **Imidazo[4,5-c]pyridine and pyrrolo[3,2-c]pyridine compounds as G-protein-coupled receptor kinase 5 (GRK5) modulators**

(57) The present invention relates to imidazo[5,4-c] pyridine or pyrrolo[3,2-c]pyridine compounds and stereoisomeric forms, solvates, hydrates and/or pharmaceutically acceptable salts of these compounds, as well as pharmaceutical compositions containing at least one of these pyridine-based bicyclic compounds together with pharmaceutically acceptable carrier, excipient and/or diluents. Said pyridine-based bicyclic compounds are assumed to be modulators of the GRK5 protein, thereby regulating the expression and/or release of insulin and are useful for the treatment or prophylaxis of a metabolic disease and in particular for the treatment and prophylaxis of diabetes, obesity and impaired adipogenesis.

EP 2 818 472 A1

## Description

The present invention relates to pyridine-based bicyclic compounds and stereoisomeric forms, solvates, hydrates and/or pharmaceutically acceptable salts of these compounds, as well as pharmaceutical compositions containing at least one of these pyridine-based bicyclic compounds together with pharmaceutically acceptable carrier, excipient and/or diluents. Said pyridine-based bicyclic compounds are assumed to be modulators of the protein GRK5, thereby regulating the expression and/or release of insulin and are useful for the treatment or prophylaxis of a metabolic disease and in particular for the treatment and prophylaxis of diabetes, obesity and impaired adipogenesis.

## Background of the invention

Diabetes as a leading cause of death in developed countries is a metabolic condition characterized by high blood sugar levels. There are two main types of diabetes: type 1, resulting from insufficient insulin production of the pancreas beta cells, which requires the person to inject insulin; and type 2, resulting from insensitivity of peripheral tissues (such skeletal muscle, liver or adipose tissue) insulin release alterations, and relative insulin deficiency.

Insulin resistance, a condition in which cells fail to respond to the action of insulin, is a key feature in the pathogenesis of metabolic disorders such as type 2 diabetes (T2D) and obesity. Adipocytes are insulin-sensitive cells that take up glucose and store energy in the form of triglycerides. In addition to their storage function, adipocytes have recently been shown to be dynamic endocrine cells that produce and secrete various adipocytokines, such as TNF-a, IL-6, leptin, resistin, and adiponectin. The insulin resistance that accompanies obesity is attributable, at least in part, to changes in adipokine secretion. For example, excess accumulation of adipose tissue is detrimental to many systems, and involved in the pathologies including insulin resistance, type 2 diabetes and fatty infiltration of the liver. In the situation of insulin resistance, the liver inappropriately releases glucose into the blood. The proportion of insulin resistance versus beta cell dysfunction differs individually among patients, with some having primarily insulin resistance and only a minor defect in insulin secretion and others with slight insulin resistance and primarily a lack of insulin secretion. However, the precise mechanisms regulating insulin resistance in physiopathological conditions are not fully understood.

The decreased insulin sensitivity of peripheral tissues in type 2 diabetes, which accounts for 90% of all cases of the disease is initially compensated by an increased release of insulin by the beta cells of the pancreas. At a certain stage of the disease, the pancreas cannot maintain the increases release of insulin anymore. As disease progresses, drugs which are currently available and elevate insulin release have lead to beta-cell damage and loss of insulin production.

A number of diseases, including cancer, diabetes and inflammation are linked to perturbation of protein kinase mediated cell signaling pathways. For some time, a new class of multiple kinase drugs has been undergoing clinical trials. Some have been approved for various applications, mostly for the treatment of cancer. The targets of these multiple kinase inhibitors like Imanitib or Sunitinib interact at all stages of signal transduction: from the receptor tyrosine kinases, which initiate intracellular signaling to second-messenger generators and kinases involved in signaling cascades and finally to those kinases, which regulate the cell cycle governing cellular fate.

Several publications have shown the effect of multi-targeted receptor tyrosine kinase inhibitors like Sunitinib (Sutent®) and Imatinib (Gleevec®) on diabetes during a period of treatment, which leads to a remission of diabetes type 1 or 2 in patients. The fact that multi-targeted receptor tyrosine kinase inhibitors, like Sunitinib, target many different receptors, results in many of its side effects such as the classic hand-foot syndrome, stomatitis, and other dermatologic toxicities. However, WO 2012/028335 A1 discloses only few kinases, which could be identified that affect the insulin release or sensitivity specifically and are suitable as potential targets to develop a more specific treatment strategy. One of said identified targets is GRK5.

A potential influence in the in the insulin regulating mechanism may be attributed to G-protein-coupled receptor (GPCR) kinase 5 (GRK5). GRK5 is an important member of the threonine/serine kinase family that phosphorylates GPCRs as part of feedback inhibition of GPCRs in signal transduction. The *in vivo* physiological functions of GRK5 have been ascribed to its kinase activity, phosphorylating and desensitizing specific GPCRs, as well as its kinase independent function and targeting to non-GPCR substrates. GRK5 interacts with IkB$\alpha$ and inhibits NF$\kappa$B-mediated transcriptional responses. GRK5 also phosphorylates p53 and regulates p53-mediated apoptosis in response to DNA damage. G protein-coupled receptor kinases (GRKs) specifically phosphorylate serine/threonine residues located on the cytoplasmic loops and C terminus of the receptors. Phosphorylation of a GPCR by GRK promotes the high affinity binding of arrestins and induces receptor internalization, and thus feed-back inhibits GPCR responses to extracellular signals. The *in vivo* physiological functions of various subtypes of GRKs (GRK1-7) have been previously ascribed to phosphorylation and desensitizing specific GPCRs. GRK5 deficiency prevents obesity induced by high fat diet in mice, thus suggesting the central role of GRKS in the adipogenesis regulation and the development of diet induced obesity. (Biochem. Biophys Res. Commun. 2012, 421, 312). A genome-wide association study in Chinese Hans identified two novel T2D loci, including GRK5 (rs10886471: P = 7.1 x 10(-9)). This study could further show that the risk increasing allele rs10886471

was also associated with higher GRK5 mRNA expression levels, higher fasting insulin, but not with fasting glucose, suggesting that it might impair insulin sensitivity.

Thus, it is the objective of the present invention to provide compounds, which are useful as new anti-diabetic drugs for raising the blood insulin level by regulating the insulin release of the pancreatic beta cells and enhance an insulin dependent uptake of glucose. This goal is achieved by the compounds according to claim 1, which bind to regulating proteins and most probably the disclosed target GRK5. Further advantageous embodiments, aspects and details of the invention are evident from the pending claims, the description, and the examples.

Therefore, it is further the objective of the present invention to provide compounds and/or pharmaceutically acceptable salts thereof which can be used as pharmaceutically active agents, especially for the treatment of diabetes, obesity and impaired adipogenesis as well as compositions comprising at least one of those compounds and/or pharmaceutically acceptable salts thereof as pharmaceutically active ingredients.

The objective of the present invention is solved by the teaching of the independent claims. Further advantageous features, aspects and details of the invention are evident from the dependent claims, the description, and the examples of the present application.

**Description of the invention**

In order to reveal new negatively regulated kinases, which display increased insulin release after inhibition, the inventors have investigated a kinome wide siRNA screen and presented new negatively regulating kinase GRK5, as new potential target for triggering insulin release and therapeutic anti-diabetes targets.

This invention relates to identified compound structures that bind most probably to the most promising kinase candidate GRK5. The identified GRK5 inhibitors displayed an insulin independent increase of 2-NBDG glucose analog uptake as well as an improved release of insulin.

The present invention therefore relates to compound of the general formula (I)

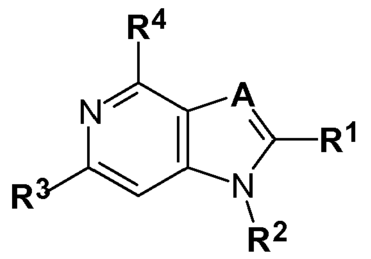

**(I)**

wherein

**A** represents C-H, or N;

**$R^1$** represents $-(CH_2)_n$-**$R^5$**, or $-NH-(CH_2)_n$-**$R^5$**;

**$R^2$** represents -H, $-CH_3$, $-(CH_2)-O-CH_3$, $-(CH_2)_k-NHCOCH_3$, $-(CH_2)_k$-cyclo-$C_3H_5$, $-(CH_2)_k$-Ph, $-(CH_2)_k-R^*$;

**R*** represents

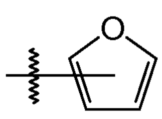 , 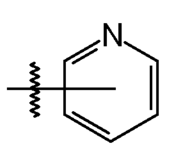 , 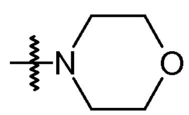 , 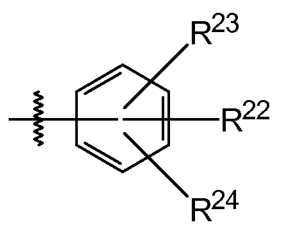 .

**$R^3$** represents $-(CH_2)_m$-**$R^6$**, or **$-NR^7$**$((CH_2)_m$-**$R^6$**);

**$R^4$** represents $-(CH2)_p$-**$R^8$**, or **$-NR^9$**$((CH_2)_p-R^8)$, wherein one of **$R^3$** and **$R^4$** represents -H, namely **$R^3$** represents $-(CH_2)_m$-**$R^6$**, or **$-NR^7$**$((CH_2)_m$-**$R^6$**), and **$R^4$** represents -H; or **$R^4$** represents $-(CH2)_p$-**$R^8$**, or $-NR^9((CH_2)_p$-**$R^8$**), and $R^3$ represents -H;

**$R^5$, $R^6$** and **$R^8$** are independently of each other -H, -F, -Cl, -Br, -I, -CN, $-NO_2$, $-NHCH_3$, $-N(CH_3)_2$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$,

$-C_8H_{16}OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$ $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-COCH_3$, $-COC_2H_5$, $-COC_3H_7$, -CO-cyclo-$C_3H_5$, $-COCH(CH_3)_2$, $-COC(CH_3)_3$, -COOH, $-SO_3H$, $-OCF_3$, $-CH_2OCF_3$, $-C_2H_4-OCF_3$, $-C_3H_6-OCF_3$, $-OC_2F_5$, $-COOCH_3$, $-COOC_2H_5$, $-COOC_3H_7$, -COO-cyclo-$C_3H_5$, $-COOCH(CH_3)_2$, $-COOC(CH_3)_3$, $-OOC-CH_3$, $-OOC-C_2H_5$, $-OOC-C_3H_7$, -OOC-cyclo-$C_3H_5$, $-OOC-CH(CH_3)_2$, $-OOC-C(CH_3)_3$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, -NHCO-O-cyclo-$C_3H_5$, $-NHCO-OCH(CH_3)_2$, $-NHCO-OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $-R^{10}$ , $-R^{11}$,

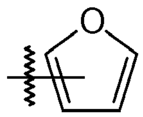 , 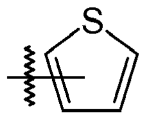 , 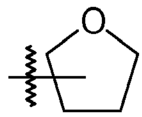 ,

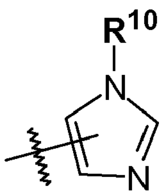 , 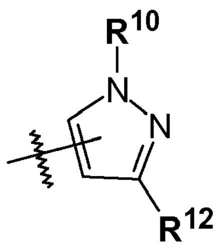 , 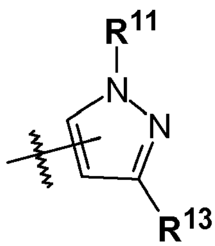 ,

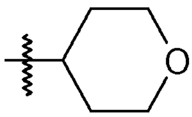 , 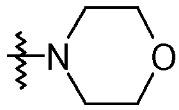 , 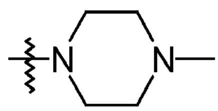 ,

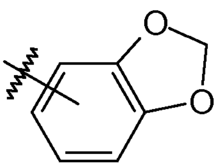 , 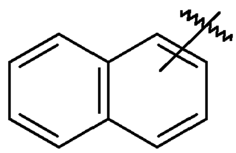 , 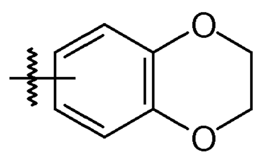 ,

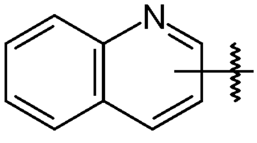 , 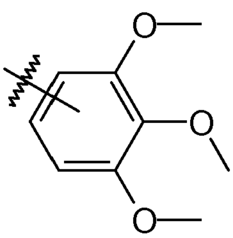 , 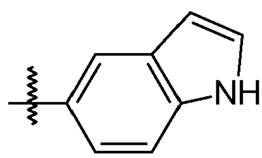 ,

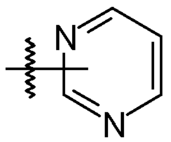 , 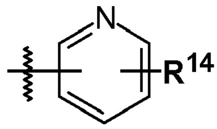 , 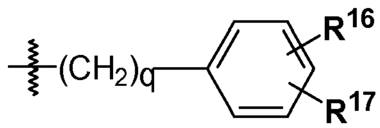 ,

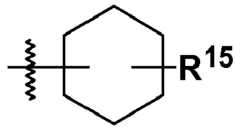 ;

**$R^7$** and **$R^9$** are independently of each other -H, $-CH_3$, $-C_2H_5$, $-C_3H_7$, $-CH(CH_3)^2$, $-C_4H_9$, $-CH_2-CH(CH_3)_2$, $-CH(CH_3)-C_2H_5$, $-C(CH_3)_3$;

**$R^{14}$** and **$R^{15}$** are independently of each other -H, $-NH_2$, -OH, -OMe;

**$R^{16}$** and **$R^{17}$** are independently of each other -H, -F, -Br, -Cl, -OH, -CN, **$-R^{18}$, $-R^{19}$, $-OR^{18}$, $-OR^{19}$,** $-CH_2OH$, $-CH_2NH_2$, $-CH_2CN$; $-CH_2N(R^{18})_2$, $-CH_2N(\mathbf{R}^{19})_2$, $-CH_2NH(\mathbf{R}^{18})$, $-CH_2NH(\mathbf{R}^{19})$, $-O(CH_2)_3N(CH_3)_2$, $-SCH_3$, $-NH_2$, $-NH(R^{18})$, $-NH(R^{19})$, $-NR^{18}COR^{19}$, $-NHSO_2CH_3$, $-N(R^{18})_2$, $-N(R^{19})_2$, $-SO_2CH_3$, $-SO_2NH_2$, $-CH_2CO_2H$, $-C_2H_4CO_2H$, $-CH=CH-CO_2H$, $-COR^{20}$,

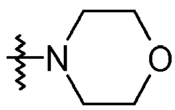 , 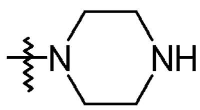 , 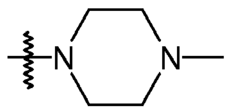 ;

$R^{20}$ is -OH, $-R^{21}$, $-OR^{21}$, $-NH_2$, $-NHR^{21}$, $-N(R^{21})_2$, $-NHC_2H_4OH$, $-NHC_2H_4OCH_3$, $-NH(CH_2)_rN(R^{21})_2$,

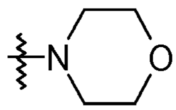 , 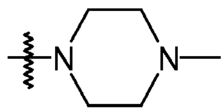 ;

**$R^{10}$, $R^{11}$, $R^{12}$, $R^{13}$, $R^{18}$, $R^{19}$,** and **$R^{21}$** are independently of each other

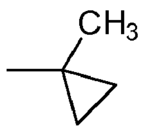

cyclo-$C_3H_5$, cyclo-$C_4H_7$, cyclo-$C_5H_9$, cyclo-$C_6H_{11}$, cyclo-$C_7H_{13}$, -H, $-CH_2-OCH_3$, $-C_2H_4-OCH_3$, $-C_3H_6OCH_3$, $-CH_2OC_2H_5$, $-C_2H_4-OC_2H_5$, $-C_3H_6-OC_2H_5$, $-CH_2.OC_3H_7$, $C_2H_4-OC_3H_7$, $C_3H_6-OC_3H_7$, $-CH_2$-O-cyclo-$C_3H_5$, $-C_2H_4$-O-cyclo-$C_3H_5$, $-C_3H_6$-O-cyclo-$C_3H_5$, $-CH_2-OCH(CH_3)_2$, $-C_2H_4-OCH(CH_3)_2$, $-C_3H_6OCH(CH_3)_2$, $-CH_2-OC(CH_3)_3$, $-C_2H_4-OC(CH_3)_3$, $-C_3H_6OC(CH_3)_3$, $-CH_2-OC_4H_9$, $-C_2H_4-OC_4H_9$, $-C_3H_6OC_4H_9$, $-CH_2$-OPh, $-C_2H_4$-OPh, $-C_3H_6$OPh, $-CH_2-OCH_2$-Ph, $-C_2H_4-OCH_2$-Ph, $-C_3H_6OCH_2$-Ph, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_8H_{15}$, -Ph, $-CH_2$-Ph, $CH_2-CH_2$-Ph, -CH=CH-Ph, $-CPh_3$, $-CH_3$, $-C_2H_5$, $-C_3H_7$, $-CH(CH_3)^2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-CH(CH_3)Ph$, $-C(CH_3)_2Ph$;

**$R^{22}$, $R^{23}$** and **$R^{24}$** represent independently of each other -H, -F, -Cl, -Br, $-OCH_3$, $-CF_3$;

k is the integer 0, 1 or 2;

m, n, p, q and r are integer from 0 to 3;

and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.
preferred are compounds of the general formula (I)

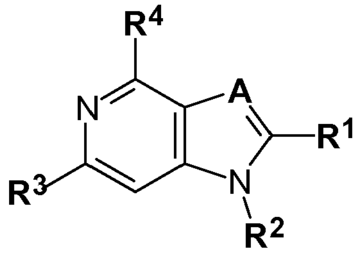

**(I)**

wherein
A represents C-H, or N;
$R^1$ represents $-(CH_2)_n-R^5$, or $-NH-(CH_2)_n-R^5$;
$R^2$ represents -H, $-CH_3$, $-(CH_2)_2-O-CH_3$, $-(CH_2)_2-NHCOCH_3$, $-CH_2$-cyclo-$C_3H_5$, -Ph, $-CH_2Ph$,

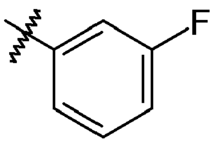
,
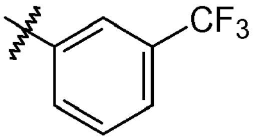
,
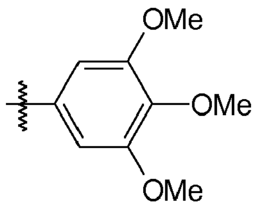
,
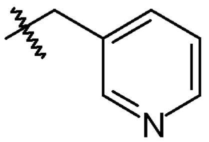
,
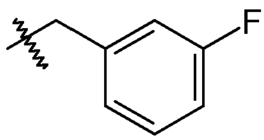
,
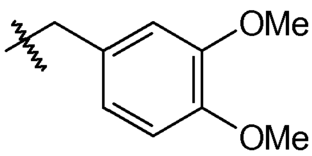

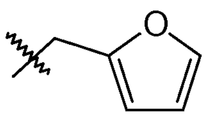
,
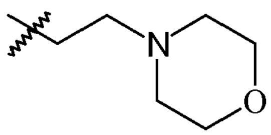
;

$R^3$ represents $-(CH_2)_m-R^6$, or $-NR^7((CH_2)_m-R^6)$;
$R^4$ represents $-(CH2)_p-R^8$, or $-NR^9((CH_2)_p$**-**$R^8$**)**, wherein one of $R^3$ and $R^4$ represents -H;
$R^5$, $R^6$ and $R^8$ are independently of each other -H, -F, -Cl, -Br, -I, -CN, $-NO_2$, $-NHCH_3$, $-N(CH_3)_2$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$,- $C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-COCH_3$, $-COC_2H_5$, $-COC_3H_7$, -CO-cyclo-$C_3H_5$, $-COCH(CH_3)_2$, $-COC(CH_3)_3$, -COOH, $-SO_3H$, $-OCF_3$, $-CH_2OCF_3$, $-C_2H_4-OCF_3$, $-C_3H_6-OCF_3$, $-OC_2F_5$, $-COOCH_3$, $-COOC_2H_5$, $-COOC_3H_7$, -COO-cyclo-$C_3H_5$, $-COOCH(CH_3)_2$, $-COOC(CH_3)_3$, -OOC-$CH_3$, -OOC-$C_2H_5$, -OOC-$C_3H_7$, -OOC-cyclo-$C_3H_5$, -OOC-$CH(CH_3)_2$, -OOC-$C(CH_3)_3$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NH-

CO-cyclo-$C_3H_5$, -NHCO-$CH(CH_3)_2$, -NHCO-$C(CH_3)_3$, -NHCO-$OCH_3$, -NHCO-$OC_2H_5$, -NHCO-$OC_3H_7$, -NHCO-O-cyclo-$C_3H_5$, -NHCO-$OCH(CH_3)_2$, -NHCO-$OC(CH_3)_3$, -$NH_2$, -$NHCH_3$, -$NHC_2H_5$, -$NHC_3H_7$, -NH-cyclo-$C_3H_5$, -$NHCH(CH_3)_2$, -$NHC(CH_3)_3$, -$N(CH_3)_2$, -$N(C_2H_5)_2$, -$N(C_3H_7)_2$, -N(cyclo-$C_3H_5)_2$, -$N[CH(CH_3)_2]_2$, -$N[C(CH_3)_3]_2$, **-$R^{10}$, -$R^{11}$,**

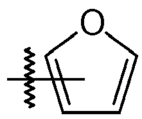 , 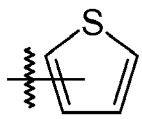 , 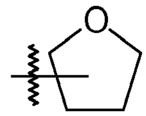 ,

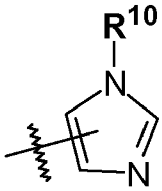 , 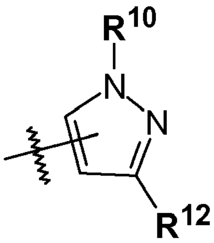 , 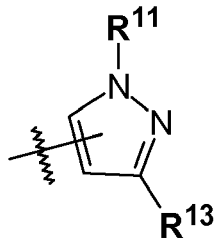 ,

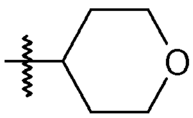 , 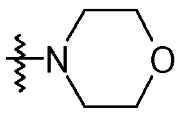 , 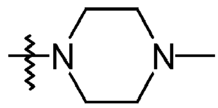 ,

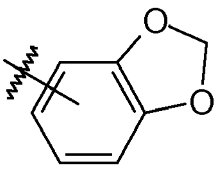 , 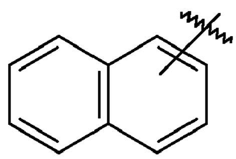 , 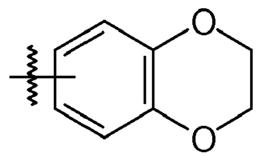 ,

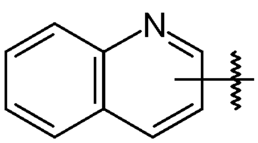 , 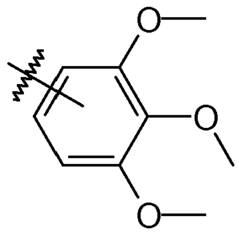 , 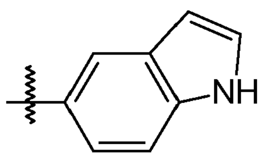 ,

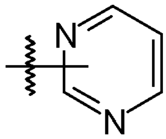 , 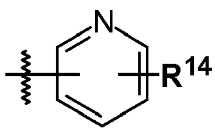 , 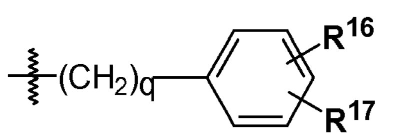 ,

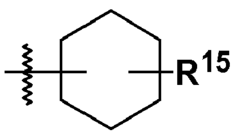 ;

**$R^7$** and **$R^9$** are independently of each other -H, -$CH_3$, -$C_2H_5$, -$C_3H_7$, -$CH(CH_3)^2$, -$C_4H_9$, -$CH_2$-$CH(CH_3)_2$, -$CH(CH_3)$-$C_2H_5$,

$-C(CH_3)_3$;
**$R^{14}$** and **$R^{15}$** are independently of each other -H, $-NH_2$, -OH, -OMe;
$R^{16}$ and $R^{17}$ are independently of each other -H, -F, -Br, -Cl, -OH, -CN, **$-R^{18}$, $-R^{19}$, $-OR^{18}$, $-OR^{19}$,** $-CH_2OH$, $-CH_2NH_2$, $-CH_2CN$; $-CH_2N(R^{18})_2$, $-CH_2N($**$R^{19}$**$)_2$, $-CH_2NH($**$R^{18}$**$)$, $-CH_2NH(R^{19})$, $-O(CH_2)_3N(CH_3)_2$, $-SCH_3$, $-NH_2$, $-NH(R^{18})$, $-NH(R^{19})$, $-NR^{18}COR^{19}$, $-NHSO_2CH_3$, $-N($**$R^{18}$**$)_2$, $-N($**$R^{19}$**$)_2$, - $SO_2CH_3$, $-SO_2NH_2$, $-CH_2CO_2H$, $-C_2H_4CO_2H$, $-CH=CH-CO_2H$, $-CO$**$R^{20}$**,

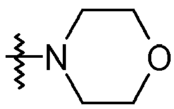 , 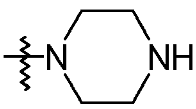 , 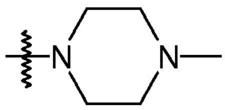 ;

**$R^{20}$** is -OH, **$-R^{21}$, $-OR^{21}$,** $-NH_2$, $-NH$**$R^{21}$**, $-N(R^{21})_2$, $-NHC_2H_4OH$, $-NHC_2H_4OCH_3$, $-NH(CH_2)_rN($**$R^{21}$**$)_2$,

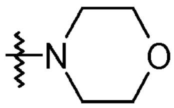 , 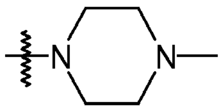 ;

**$R^{10}$, $R^{11}$, $R^{12}$, $R^{13}$, $R^{18}$, $R^{19}$,** and **$R^{21}$** are independently of each other

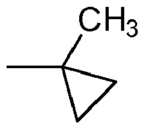

cyclo-$C_3H_5$, cyclo-$C_4H_7$, cyclo-$C_5H_9$, cyclo-$C_6H_{11}$, cyclo-$C_7H_{13,}$ -H, $-CH_2-OCH_3$, $-C_2H_4-OCH_3$, $-C_3H_6OCH_3$, $-CH_2OC_2H_5$, $-C_2H_4-OC_2H_5$, $-C3H_6-OC_2H_5$, $-CH_2-OC_3H_7$, $C_2H_4-OC_3H_7$, $-C_3H_6OC_3H_7$,- $CH_2$-O-cyclo-$C_3H_5$, $-C_2H_4$-O-cyclo-$C_3H_5$, $-C_3H_6$-O-cyclo-$C_3H_5$, $-CH_2-OCH(CH_3)_2$, $-C_2H_4-OCH(CH_3)_2$, $-C_3H_6OCH(CH_3)_2$, $-CH_2-OC(CH_3)_3$, $-C_2H_4-OC(CH_3)_3$, $-C_3H_6OC(CH_3)_3$, $-CH_2-OC_4H_9$, $-C_2H_4-OC_4H_9$, $-C_3H_6OC_4H_9$, $-CH_2$-OPh, $-C_2H_4$-OPh, $-C_3H_6$OPh, $-CH_2-OCH_2$-Ph, $-C_2H_4-OCH_2$-Ph, $-C_3H_6OCH_2$-Ph, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_8H_{15}$, -Ph, $-CH_2$-Ph, $CH_2-CH_2$-Ph, -CH=CH-Ph, $-CPh_3$, $-CH_3$, $-C_2H_5$, $-C_3H_7$, $-CH(CH_3)^2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, -CH=CH-$CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-CH(CH_3)Ph$, $-C(CH_3)_2Ph$;
m, n, p, q and r are integer from 0 to 3;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

In yet another aspect of the prevent invention is related to compound of the general formula (II):

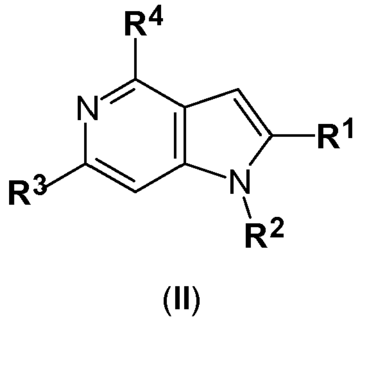

**(II)**

wherein

**$R^1$** represents -$(CH_2)_n$-**$R^5$**, or -NH-$(CH_2)_n$-**$R^5$**;

**$R^2$** represents -H, -$CH_3$, -$(CH_2)_2$-O-$CH_3$, -$(CH_2)_2$-NHCO$CH_3$, -$CH_2$-cyclo-$C_3H_5$, -Ph, -$CH_2$Ph,

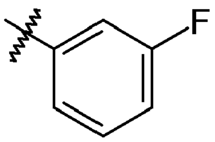 , 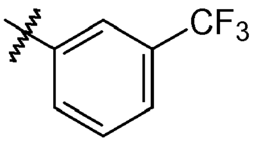 , 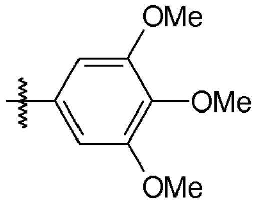 ,

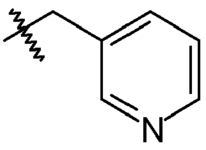 , 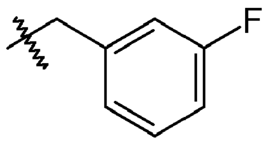 , 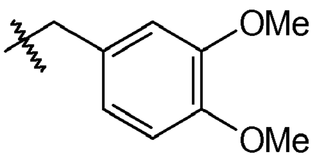 ,

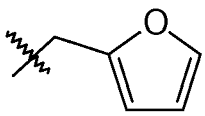 , 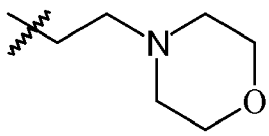 ;

**$R^3$** represents -$(CH_2)_m$-**$R^6$**, or -$NR^7$($(CH_2)_m$-**$R^6$**);

**$R^4$** represents -(CH2)$_p$-**$R^8$**, or -$NR^9$($(CH_2)_p$-$R^8$**)**, wherein one of **$R^3$** and **$R^4$** represents -H;

**$R^5$,** $R^6$ and $R^8$ are independently of each other -H, -F, -Cl, -Br, -I, -CN, -$NO_2$, -NH$CH_3$, -N$(CH_3)_2$, -CH=CH-$C_4H_9$, -CH=CH-$C_5H_{11}$, -CH=CH-Ph, -CH=CH-$C_6H_{13}$, -$CH_2$-OH; -$C_2H_4$-OH; -$C_3H_6$-OH, -$C_4H_9$-OH, -$C_5H_{10}$-OH, -$C_6H_{12}$-OH, -$C_7H_{14}$-OH, -$C_8H_{16}$OH, -CH=CH-$C_3H_6$-OH, -CH=CH-$C_4H_8$-OH, -CH$(CH_2OH)_2$, -CH$(C_2H_5)$-$CH_2$-OH, -CH$(CH_3)$-$C_2H_4$-OH, -C$(CH_3)_2$-OH, -C$(CH_3)_2$-$CH_2$-OH, -CH$(CH_3)$OH, -$CH_2$-CH$(CH_3)$OH, -C(OH)$(CH_3)$-$C_2H_5$, -C(OH)$(CH_3)$-$C_3H_7$, -$CH_2$-C(OH)$(CH_3)$-$C_2H_5$, -CH$(CH_3)$-CH$(CH_3)$OH, -C$(CH_3)_2$-$C_2H_4$OH, -$CH_2$-C$(CH_3)_2$OH, -C(OH)$(C_2H_5)_2$, -$C_2H_4$-C(OH)$(CH_3)_2$, -C(CH$(CH_3)_2$)$CH_2$OH, -$C_3H_6$-C(OH)$(CH_3)_2$, -CH(CH$(CH_3)_2$)$CH_2$-OH, -OH, -O$CH_3$, -O$C_2H_5$, -O$C_3H_7$, -O-cyclo-$C_3H_5$, -OCH$(CH_3)_2$, -OC$(CH_3)_3$, -O$C_4H_9$, -OPh, -O$CH_2$-Ph, -O$CPh_3$, -SH, -S$CH_3$, -S$C_2H_5$,-CO$CH_3$, -CO$C_2H_5$, -CO$C_3H_7$, -CO-cyclo-$C_3H_5$, -COCH$(CH_3)_2$, -COC$(CH_3)_3$, -COOH, -$SO_3$H, -O$CF_3$, -$CH_2$O$CF_3$, -$C_2H_4$-O$CF_3$, -$C_3H_6$O$CF_3$, -O$C_2F_5$, -COO$CH_3$, -COO$C_2H_5$, -COO$C_3H_7$, -COO-cyclo-$C_3H_5$, -COOCH$(CH_3)_2$, -COOC$(CH_3)_3$, -OOC-$CH_3$, -OOC-$C_2H_5$, -OOC-$C_3H_7$, -OOC-cyclo-$C_3H_5$, -OOC-CH$(CH_3)_2$, -OOC-C$(CH_3)_3$, -CON$H_2$, -CONH$CH_3$, -CON-H$C_2H_5$, -CONH$C_3H_7$, -CONH-cyclo-$C_3H_5$, -CONH[CH$(CH_3)_2$], -CONH[C$(CH_3)_3$], -CON$(CH_3)_2$, -CON$(C_2H_5)_2$, -CON$(C_3H_7)_2$, -CON(cyclo-$C_3H_5)_2$,- CON[CH$(CH_3)_2]_2$, -CON[C$(CH_3)_3]_2$, -NHCO$CH_3$, -NHCO$C_2H_5$, -NHCO$C_3H_7$, -NH-CO-cyclo-$C_3H_5$, -NHCO-CH$(CH_3)_2$, -NHCO-C$(CH_3)_3$, -NHCO-O$CH_3$, -NHCO-O$C_2H_5$, -NHCO-O$C_3H_7$, -NHCO-O-cyclo-$C_3H_5$, -NHCO-OCH$(CH_3)_2$, -NHCO-OC$(CH_3)_3$, -N$H_2$, -NH$CH_3$, -NH$C_2H_5$, -NH$C_3H_7$, -NH-cyclo-$C_3H_5$, -NHCH$(CH_3)_2$, -NHC$(CH_3)_3$, -N$(CH_3)_2$, -N$(C_2H_5)_2$, -N$(C_3H_7)_2$, -N(cyclo-$C_3H_5)_2$, -N[CH$(CH_3)_2]_2$, -N[C$(CH_3)_3]_2$, -$R^{10}$, -$R^{11}$,

, ,

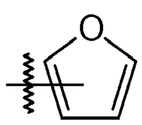 , 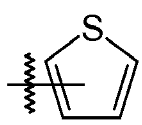 , 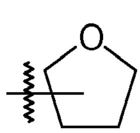 ,

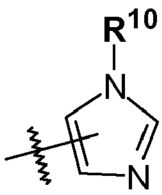,
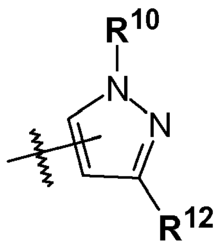,
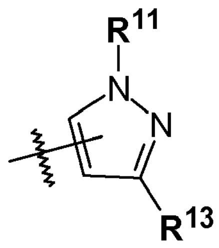,

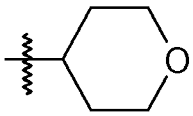,
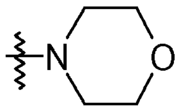,
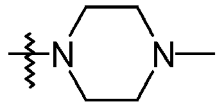,

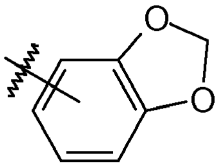,
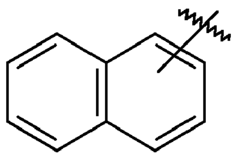,
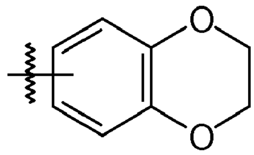,

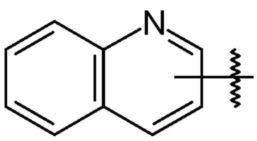,
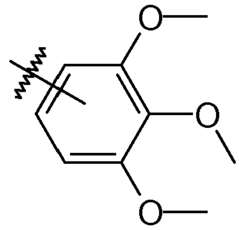,
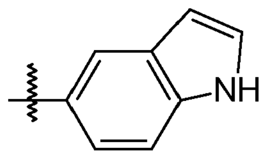,

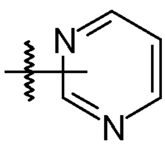,
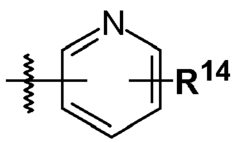,
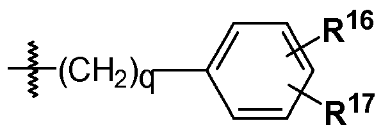,

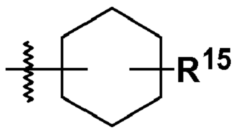;

$R^7$ and $R^9$ are independently of each other -H, $-CH_3$, $-C_2H_5$, $-C_3H_7$, $-CH(CH_3)^2$, $-C_4H_9$, $-CH_2-CH(CH_3)_2$, $-CH(CH_3)-C_2H_5$, $-C(CH_3)_3$;
$R^{14}$ and $R^{15}$ are independently of each other -H, $-NH_2$, -OH, -OMe;
$R^{16}$ and $R^{17}$ are independently of each other -H, -F, -Br, -Cl, -OH, -CN, $-R^{18}$, $-R^{19}$, $-OR^{18}$, $-OR^{19}$, $-CH_2OH$, $-CH_2NH_2$, $-CH_2CN$; $-CH_2N(R^{18})_2$, $-CH_2N(R^{19})_2$, $-CH_2NH(R^{18})$, $-CH_2NH(R^{19})$, $-O(CH_2)_3N(CH_3)_2$, $-SCH_3$, $-NH_2$, $-NH(R^{18})$, $-NH(R^{19})$, $-NR^{18}COR^{19}$, $-NHSO_2CH_3$, $-N(R^{18})_2$, $-N(R^{19})_2$, $-SO_2CH_3$, $-SO_2NH_2$, $-CH_2CO_2H$, $-C_2H_4CO_2H$, $-CH=CH-CO_2H$, $-COR^{20}$,

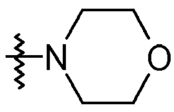 , 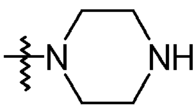 , 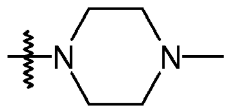 ;

$R^{20}$ is -OH, $-R^{21}$, $-OR^{21}$, $-NH_2$, $-NHR^{21}$, $-N(R^{21})_2$, $-NHC_2H_4OH$, $-NHC_2H_4OCH_3$, $-NH(CH_2)_rN(R^{21})_2$,

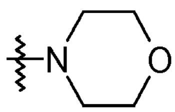 , 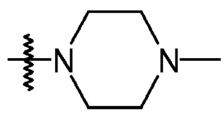 ;

$R^{10}$, $R^{11}$, $R^{12}$, $R^{13}$ $R^{18}$, $R^{19}$, and $R^{21}$ are independently of each other

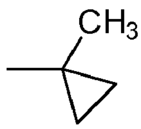

cyclo-$C_3H_5$, cyclo-$C_4H_7$, cyclo-$C_5H_9$, cyclo-$C_6H_{11}$, cyclo-$C_7H_{13}$, -H, $-CH_2-OCH_3$, $-C_2H_4-OCH_3$, $-C_3H_6OCH_3$, $-CH_2OC_2H_5$, $-C_2H_4-OC_2H_5$, $-C_3H_6OC_2H_5$, $CH_2OC_3H_7$, $C_2H_4-OC_3H_7$, $-C_3H_6-OC_3H_7$, $-CH_2$-O-cyclo-$C_3H_5$, $-C_2H_4$-O-cyclo-$C_3H_5$, $-C_3H_6$-O-cyclo-$C_3H_5$, $-CH_2-OCH(CH_3)_2$, $-C_2H_4-OCH(CH_3)_2$, $-C_3H_6OCH(CH_3)_2$, $-CH_2-OC(CH_3)_3$, $-C_2H_4-OC(CH_3)_3$, $-C_3H_6OC(CH_3)_3$, $-CH_2-OC_4H_9$, $C_2H_4-OC_4H_9$, $-C_3H_6OC_4H_9$, $-CH_2$-OPh, $-C_2H_4$-OPh, $-C_3H_6$OPh, $-CH_2-OCH_2$-Ph, $-C_2H_4-OCH_2$-Ph, $-C_3H_6OCH_2$-Ph, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_8H_{15}$, -Ph, $-CH_2$-Ph, $CH_2-CH_2$-Ph, -CH=CH-Ph, $-CPh_3$, $-CH_3$, $-C_2H_5$, $-C_3H_7$, $-CH(CH_3)^2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$,- $CH=C(CH_3)_2$, $-C(CH_3)=CH-CH_3$, -CH=CH-$CH=CH_2$, -C=CH, $-C=C-CH_3$, $-CH_2-C=CH$, $-C_2H_4-C\equiv CH$, $-CH_2-C\equiv C-CH_3$, $-C\equiv C-C_2H_5$, $-CH(CH_3)Ph$, $-C(CH_3)_2Ph$;
m, n, p, q and r are integer from 0 to 3;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

In yet another aspect of the prevent invention is related to compound of the general formula **(III)**

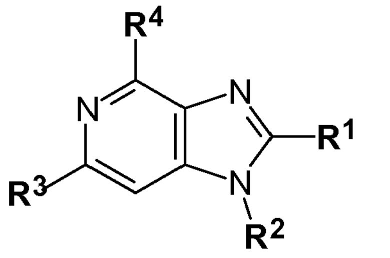

**(III)**

wherein
**R^1** represents $-(CH_2)_n-$**R**$^5$, or $-NH-(CH_2)_n-$**R**$^5$;
**R^2** represents -H, $-CH_3$, $-(CH_2)_2-O-CH_3$, $-(CH_2)_2-NHCOCH_3$,
$-CH_2$-cyclo-$C_3H_5$, -Ph, $-CH_2Ph$,

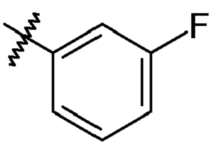 , 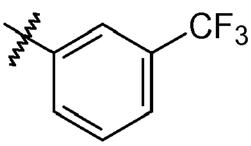 , 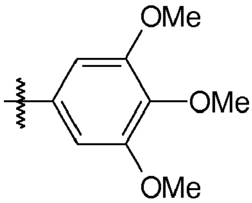 ,

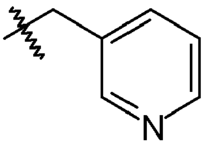 , 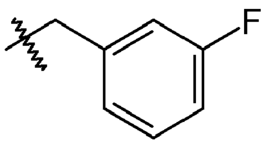 , 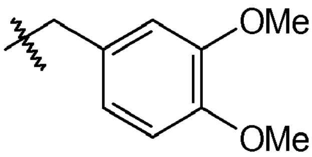 ,

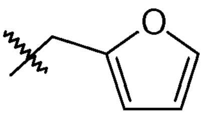 , 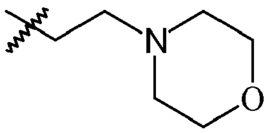 ;

$\mathbf{R^3}$ represents $-(CH_2)_m-\mathbf{R}^6$, or $-N\mathbf{R}^7((CH_2)_m-\mathbf{R}^6)$; $\mathbf{R}^4$ represents $-(CH_2)_p-\mathbf{R^8}$, or $-N\mathbf{R}^9((CH_2)_p-\mathbf{R}^8)$, wherein one of $\mathbf{R^3}$ and $\mathbf{R^4}$ represents -H;

$\mathbf{R^5}$, $\mathbf{R^6}$ and $\mathbf{R^8}$ are independently of each other -H, -F, -Cl, -Br, -I, -CN, $-NO_2$, $-NHCH_3$, $-N(CH_3)_2$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2-Ph$, $-OCPh_3$, -SH, $-SCH_3$, $-SC_2H_5$, $-COCH_3$, $-COC_2H_5$, $-COC_3H_7$, -CO-cyclo-$C_3H_5$, $-COCH(CH_3)_2$, $-COC(CH_3)_3$, -COOH, $-SO_3H$, $-OCF_3$, $-CH_2-OCF_3$, $-C_2H_4-OCF_3$, $-C_3H_6-OCF_3$, $-OC_2F_5$, $-COOCH_3$, $-COOC_2H_5$, $-COOC_3H_7$, -COO-cyclo-$C_3H_5$, $-COOCH(CH_3)_2$, $-COOC(CH_3)_3$, -OOC-$CH_3$, -OOC-$C_2H_5$, -OOC-$C_3H_7$, -OOC-cyclo-$C_3H_5$, -OOC-$CH(CH_3)_2$, -OOC-$C(CH_3)_3$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, -NHCO-$CH(CH_3)_2$, -NHCO-$C(CH_3)_3$, -NHCO-$OCH_3$, -NHCO-$OC_2H_5$, -NHCO-$OC_3H_7$, -NHCO-O-cyclo-$C_3H_5$, -NHCO-$OCH(CH_3)_2$, -NHCO-$OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $\mathbf{-R^{10}}$, $\mathbf{-R^{11}}$,

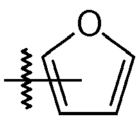 , 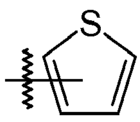 , 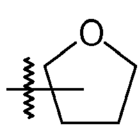 ,

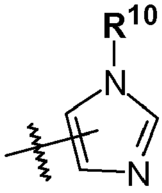 , 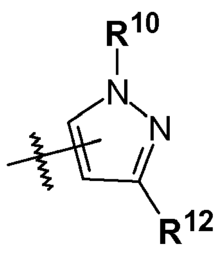 , 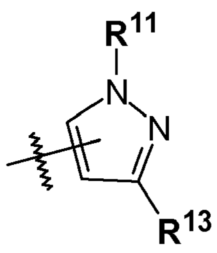 ,

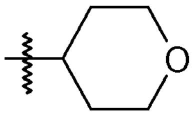, 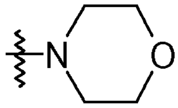, 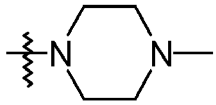,

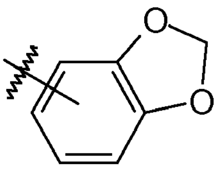, 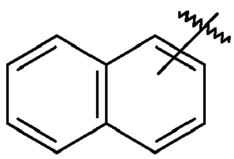, 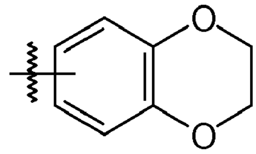,

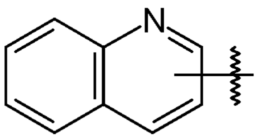, 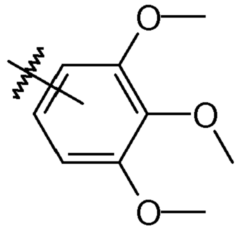, 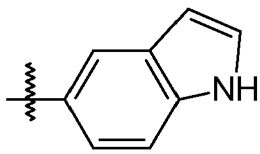,

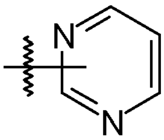, 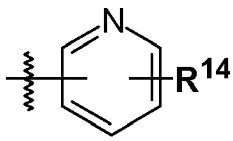, 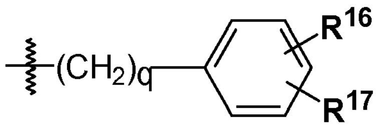,

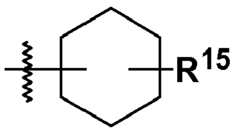;

**$R^7$** and **$R^9$** are independently of each other -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$;
**$R^{14}$** and **$R^{15}$** are independently of each other -H, $-NH_2$, -OH, -OMe;
**$R^{16}$** and **$R^{17}$** are independently of each other -H, -F, -Br, -Cl, -OH, -CN, **$-R^{18}$, $-R^{19}$,** $-OR^{18}$, $-OR^{19}$, $-CH_2OH$, $-CH_2NH_2$, $-CH_2CN$; $-CH_2N(R^{18})_2$, $-CH_2N(R^{19})_2$, $-CH_2NH(R^{18})$, $-CH_2NH(R^{19})$, $-O(CH_2)_3N(CH_3)_2$, $-SCH_3$, $-NH_2$, $-NH(R^{18})$, $-NH(R^{19})$, $-NR^{18}COR^{19}$, $-NHSO_2CH_3$, $-N(R^{18})_2$, $-N(R^{19})_2$, $-SO_2CH_3$, $-SO_2NH_2$, $-CH_2CO_2H$, $-C_2H_4CO_2H$, $-CH=CH-CO_2H$, $-COR^{20}$,

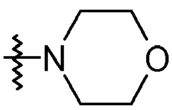, 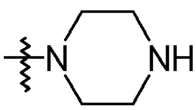, 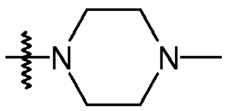;

**$R^{20}$** is -OH, **$-R^{21}$, $-OR^{21}$,** $-NH_2$, $-NHR^{21}$, $-N(R^{21})_2$, $-NHC_2H_4OH$, $-NHC_2H_4OCH_3$, $-NH(CH_2)_rN(R^{21})_2$,

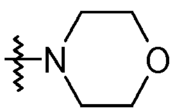, 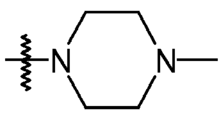;

**$R^{10}$, $R^{11}$, $R^{12}$, $R^{13}$ $R^{18}$, $R^{19}$**, and **$R^{21}$** are independently of each other

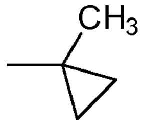

cyclo-$C_3H_5$, cyclo-$C_4H_7$, cyclo-$C_5H_9$, cyclo-$C_6H_{11}$, cyclo-$C_7H_{13}$, -H, -$CH_2$-$OCH_3$, -$C_2H_4$-$OCH_3$, -$C_3H_6OCH_3$, -$CH_2$-$OC_2H_5$, -$C_2H_4$-$OC_2H_5$, -$C_3H_6$-$OC_2H_5$, -$CH_2$-$OC_3H_7$, -$C_2H_4$-$OC_3H_7$, -$C_3H_6$-$OC_3H_7$, -$CH_2$-O-cyclo-$C_3H_5$, -$C_2H_4$-O-cyclo-$C_3H_5$, -$C_3H_6$-O-cyclo-$C_3H_5$, -$CH_2$-$OCH(CH_3)_2$, -$C_2H_4$-$OCH(CH_3)_2$, -$C_3H_6$-$OCH(CH_3)_2$, -$CH_2$-$OC(CH_3)_3$, -$C_2H_4$-$OC(CH_3)_3$, -$C_3H_6$-$OC(CH_3)_3$, -$CH_2$-$OC_4H_9$, -$C_2H_4$-$OC_4H_9$, -$C_3H_6$-$OC_4H_9$, -$CH_2$-OPh, -$C_2H_4$-OPh, -$C_3H_6$-OPh, -$CH_2$-$OCH_2$-Ph, -$C_2H_4$-$OCH_2$-Ph, -$C_3H_6$-$OCH_2$-Ph, -$CH_2F$, -$CHF_2$, -$CF_3$, -$CH_2Cl$, -$CH_2Br$, -$CH_2I$, -$CH_2$-$CH_2F$, -$CH_2$-$CHF_2$, -$CH_2$-$CF_3$, -$CH_2$-$CH_2Cl$, -$CH_2$-$CH_2Br$, -$CH_2$-$CH_2I$, cyclo-$C_8H_{15}$, -Ph, -$CH_2$-Ph, -$CH_2$-$CH_2$-Ph, -CH=CH-Ph, -$CPh_3$, -$CH_3$, -$C_2H_5$, -$C_3H_7$, -$CH(CH_3)_2$, -$C_4H_9$, -$CH_2$-$CH(CH_3)_2$, -$CH(CH_3)$-$C_2H_5$, -$C(CH_3)_3$, -$C_5H_{11}$, -$CH(CH_3)$-$C_3H_7$, -$CH_2$-$CH(CH_3)$-$C_2H_5$, -$CH(CH_3)$-$CH(CH_3)_2$, -$C(CH_3)_2$-$C_2H_5$, -$CH_2$-$C(CH_3)_3$, -$CH(C_2H_5)_2$, -$C_2H_4$-$CH(CH_3)_2$, -$C_6H_{13}$, -$C_7H_{15}$, -$C_8H_{17}$, -$C_3H_6$-$CH(CH_3)_2$, -$C_2H_4$-$CH(CH_3)$-$C_2H_5$, -$CH(CH_3)$-$C_4H_9$, -$CH_2$-$CH(CH_3)$-$C_3H_7$, -$CH(CH_3)$-$CH_2$-$CH(CH_3)_2$, -$CH(CH_3)$-$CH(CH_3)$-$C_2H_5$, -$CH_2$-$CH(CH_3)$-$CH(CH_3)_2$, -$CH_2$-$C(CH_3)_2$-$C_2H_5$, -$C(CH_3)_2$-$C_3H_7$, -$C(CH_3)_2$-$CH(CH_3)_2$, -$C_2H_4$-$C(CH_3)_3$, -$CH(CH_3)$-$C(CH_3)_3$, -$CH=CH_2$, -$CH_2$-$CH=CH_2$, -$C(CH_3)=CH_2$, -CH=CH-$CH_3$, -$C_2H_4$-$CH=CH_2$, -$CH_2$-CH=CH-$CH_3$, -CH=CH-$C_2H_5$, -$CH_2$-$C(CH_3)=CH_2$, -$CH(CH_3)$-CH=CH, -$CH=C(CH_3)_2$, -$C(CH_3)$=CH-$CH_3$, -CH=CH-$CH=CH_2$, -C≡CH, -C≡C-$CH_3$, -$CH_2$-C≡CH, -$C_2H_4$-C≡CH, -$CH_2$-C≡C-$CH_3$, -C≡C-$C_2H_5$, -$CH(CH_3)$Ph, -$C(CH_3)_2$Ph;

m, n, p, q and r are integer from 0 to 3;

and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

Preferred are compound of the general formula (**II**)

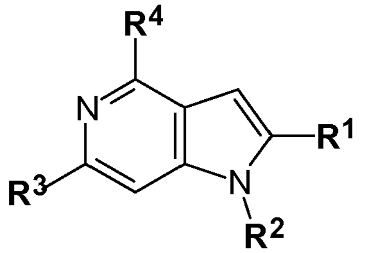

**(II)**

wherein

**$R^1$** represents -$(CH_2)_n$-**$R^5$**;

**$R^2$** represents -H, -$CH_3$, -$CH_2$Ph;

**$R^3$** represents -$(CH_2)_m$-**$R^6$**, or -N**$R^7$**($(CH_2)_m$-**$R^6$**);

**$R^4$** represents -$(CH_2)_p$-**$R^8$**, or -N**$R^9$**($(CH_2)_p$-**$R^8$**), wherein one of **$R^3$** and **$R^4$** represents -H;

**$R^5$, $R^6$** and **$R^8$** are independently of each other

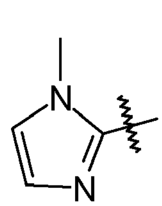 ,
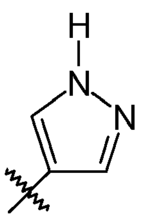 ,
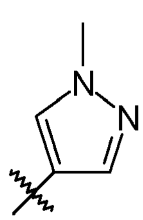 ,

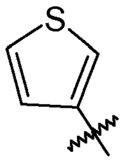, 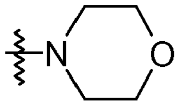, 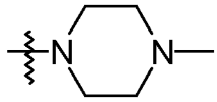,

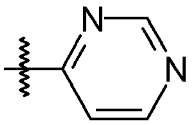, 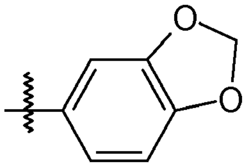, 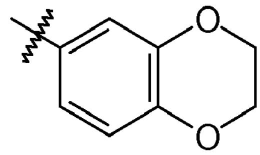,

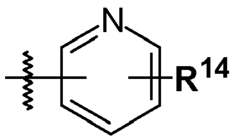, 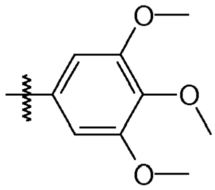, , 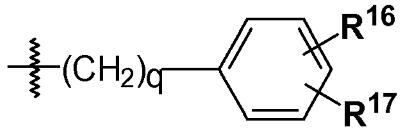 ;

$\mathbf{R^7}$ and $\mathbf{R^9}$ are independently of each other -H, $-CH_3$;
$\mathbf{R^{14}}$ is -H, -OMe;
$\mathbf{R^{16}}$ and $\mathbf{R^{17}}$ are independently of each other -H, -F, -Cl, -OH, -CN, $\mathbf{-R^{18}}$, $\mathbf{-R^{19}}$, $-O\mathbf{R^{18}}$, $-O\mathbf{R^{19}}$, $-SCH_3$, $-NH_2$,$-N\mathbf{R}^{18}CO\mathbf{R}^{19}$, $-N(\mathbf{R}^{18})_2$, $-N(\mathbf{R}^{19})_2$, $-NHSO_2CH_3$, $-SO_2CH_3$, $-CO\mathbf{R}^{20}$,

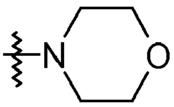, 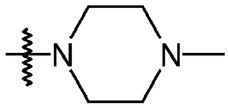 ;

$\mathbf{R^{20}}$ is $-\mathbf{R}^{21}$, $\mathbf{-OR^{21}}$, $-NH_2$, $-N(\mathbf{R^{21}})_2$;
$\mathbf{R^{18}}$, $\mathbf{R^{19}}$, and $\mathbf{R^{21}}$ are independently of each other -H, $-CF_3$, $-CH_3$, $-CH(CH_3)_2$ -Ph,;
m, n, p,and q are 0 or 1;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

Further preferred are compounds of the general formula **(III)**

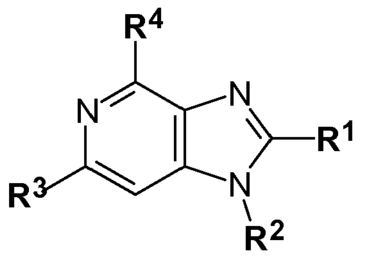

**(III)**

wherein
$\mathbf{R^1}$ represents $-(CH_2)_n-\mathbf{R}^5$, or $-NH-(CH_2)_n-\mathbf{R}^5$;
$\mathbf{R^2}$ represents -H, $-CH_3$, $-(CH_2)_2-O-CH_3$, $-(CH_2)_2-NHCOCH_3$, $-CH_2$-cyclo-$C_3H_5$, -Ph, $-CH_2Ph$,

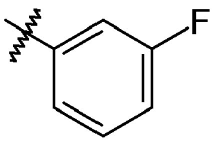 , 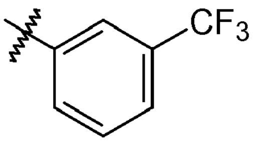 , 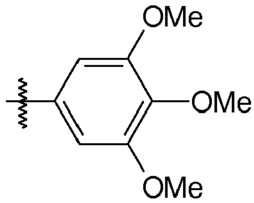 ,

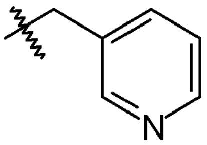 , 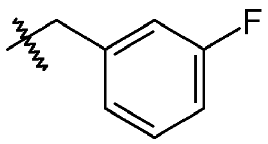 , 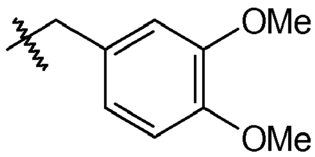 ,

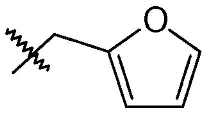 , 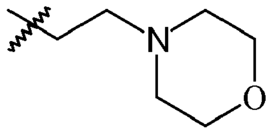 ;

$R^3$ represents $-(CH_2)_m-\mathbf{R}^6$, or $-N\mathbf{R}^7((CH_2)_m-\mathbf{R}^6)$;
$\mathbf{R}^4$ represents $-(CH_2)_p-\mathbf{R}^8$, or $-N\mathbf{R}^9((CH_2)_p-\mathbf{R}^8)$, wherein one of $\mathbf{R}^3$ and $\mathbf{R}^4$ represents -H;
$\mathbf{R}^5$, $\mathbf{R}^6$ and $\mathbf{R}^8$ are independently of each other -H, $-CH(CH_3)_2$, $-cyclo-C_3H_5$, -OH, $-OCH_3$, $-C(CH(CH_3)_2)CH_2OH$,

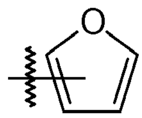 , 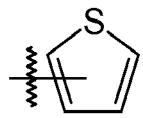 , 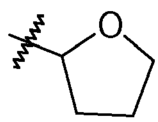 ,

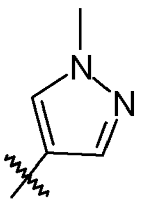 , 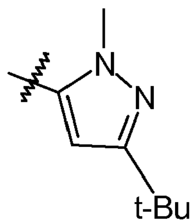 , 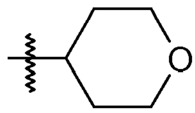 ,

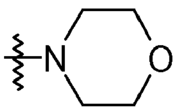 , 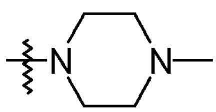 , 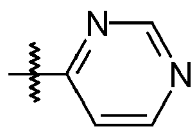 ,

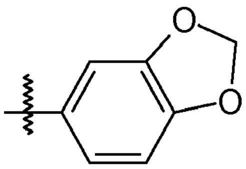 , 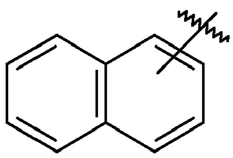 , 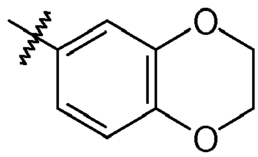 ,

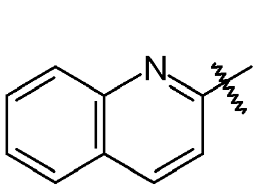 , 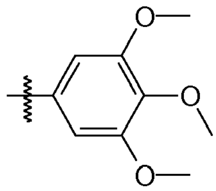 , 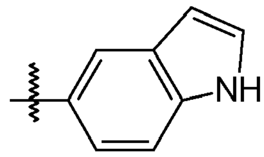 , ,

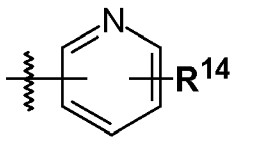 , 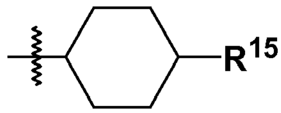 , 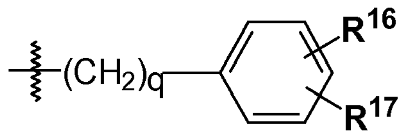 ;

**$R^7$** and **$R^9$** are independently of each other -H, $-CH_3$;
**$R^{14}$** and **$R^{15}$** are independently of each other -H, $-NH_2$, -OH, -OMe; **$R^{16}$** and **$R^{17}$** are independently of each other -H, -F, -Cl, -OH, -CN, **$-R^{18}$,**
**$-R^{19}$,** $-OR^{18}$, $-OR^{19}$, $-CH_2OH$, $-SCH_3$, $-NH_2$, $-NR^{18}COR^{19}$, $-NHSO_2CH_3$, $-N(R^{18})_2$, $-N(R^{19})_2$, $-SO_2CH_3$, $-COR^{20}$,

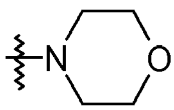 , 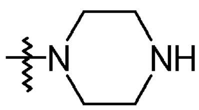 , 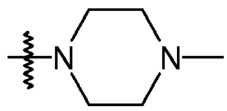 ;

**$R^{20}$** is -OH, **$-R^{21}$, $-OR^{21}$,** $-NH_2$, $-NHR^{21}$, $-N(R^{21})_2$, $-NHC_2H_4OH$, $-NHC_2H_4OCH_3$, $-NH(CH_2)_rN(R^{21})_2$;
**$R^{10}$**, **$R^{11}$, $R^{12}$**, **$R^{13}$, $R^{18}$, $R^{19}$**, and **$R^{21}$** are independently of each other -H, $-CH_3$, $-C_2H_5$, $-CH(CH_3)_2$, $-CF_3$, -Ph;
m, n, q and r are integer from 0 to 2, and
p is integer from 0 to 3;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

More preferred are compounds of the general formula **(IV)**

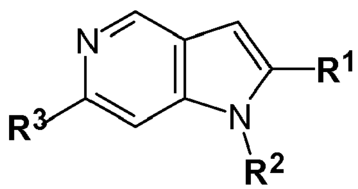

**(IV)**

wherein
**$R^1$** represents **$-R^5$;**
**$R^2$** represents -H, $-CH_3$, $-CH_2Ph$;
**$R^3$** represents **$-R^6$,** or $-NR^7R^6$;
**$R^5$** and **$R^6$** are independently of each other

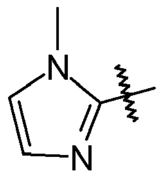 , 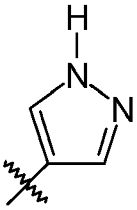 , 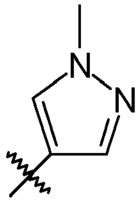 ,

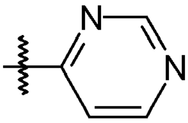 , 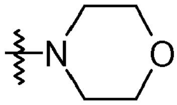 , 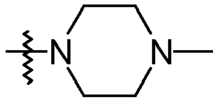 ,

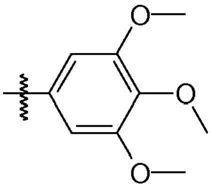 , 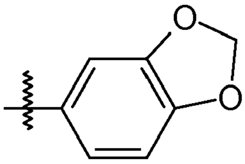 , 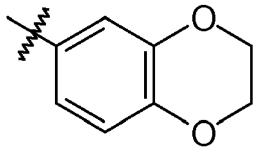 ,

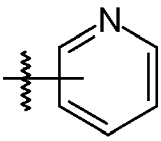 , 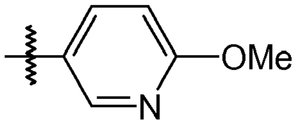 , 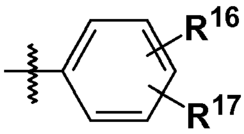 ;

**$R^7$** is -H, $-CH_3$;
**$R^{16}$** and **$R^{17}$** are independently of each other -H, -F, -Cl, -OH, -CN, $-NH_2$, $-CH_3$, $-CH(CH_3)_2$, $-CF_3$, $-OCH_3$, $-OCH(CH_3)_2$, $-OCF_3$, -OPh, $-SCH_3$, $-N(CH_3)_2$, $-NHCOCH_3$, $-NHSO_2CH_3$, $-N(CH_3)COCH_3$, $-SO_2CH_3$, $-COCH_3$, $-CONH_2$, $-CON(CH_3)_2$, $-CO_2CH_3$,

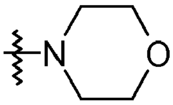 , 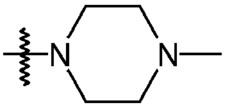 .

Also, more preferred are compounds of the general formula (V)

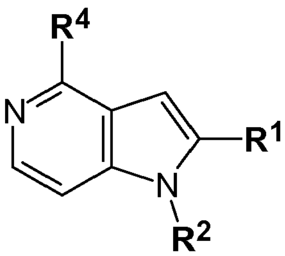

**(V)**

wherein
**$R^1$** represents **$-R^5$;**
**$R^2$** represents -H, $-CH_3$;

$R^4$ represents -$R^8$, or -NH-$R^8$;
$R^5$ and $R^8$ are independently of each other

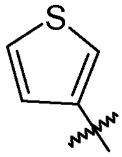 , 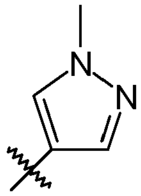 , 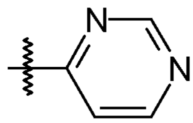 ,

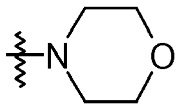 , 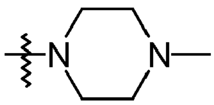 , 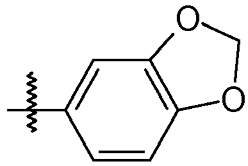 ,

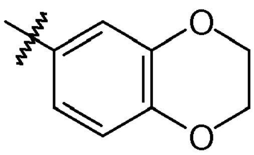 , 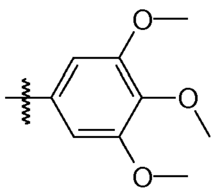 , 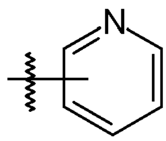 ,

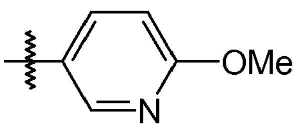 , 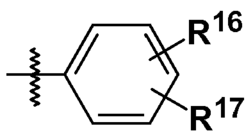 ;

$R^{16}$ and $R^{17}$ are independently of each other -H, -F, -Cl, -OH, -CN, $-CH_3$, $-CH(CH_3)_2$, $-CF_3$, $-OCH_3$, $-OCH(CH_3)_2$, $-OCF_3$, -OPh, $-SCH_3$, $-N(CH_3)_2$, $-NHCOCH_3$, $-NHSO_2CH_3$, $-SO_2CH_3$, $-COCH_3$, $-CONH_2$, $-CON(CH_3)_2$, $-CO_2CH_3$,

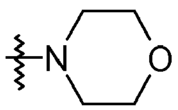 , 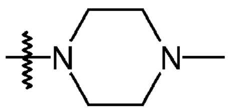 .

Also more preferred are compounds of the general formula **(VI)**

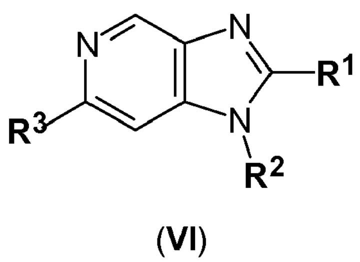

**(VI)**

wherein
$R^1$ represents $-(CH_2)_n-R^5$, or $-NH-(CH_2)_n-R^5$;
$R^2$ represents -H, $-CH_3$, $-(CH_2)_2-O-CH_3$, $-(CH_2)_2-NHCOCH_3$, $-CH_2$-cyclo-$C_3H_5$, -Ph, $-CH_2Ph$,

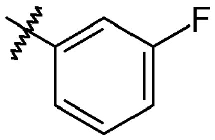, 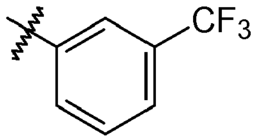, 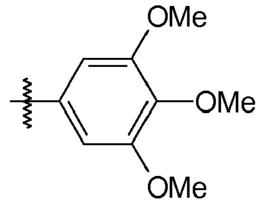,

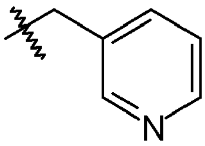, 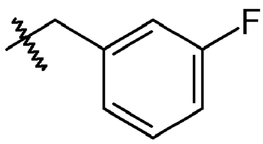, 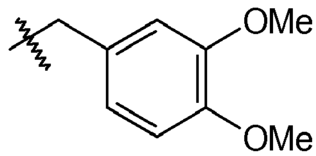,

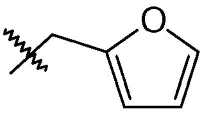, 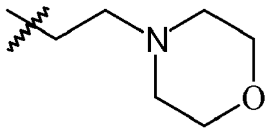;

$R^3$ represents $-(CH_2)_m-R^6$, or $-NH((CH_2)m-R^6)$;

$R^5$ and $R^6$ are independently of each other -H, $-CH(CH_3)_2$, -cyclo-$C_3H_5$, -OH, $-OCH_3$, $-C(CH(CH_3)_2)CH_2OH$,

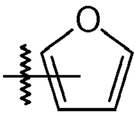, 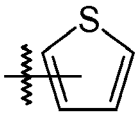, 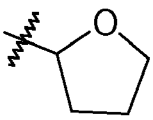,

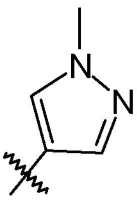, 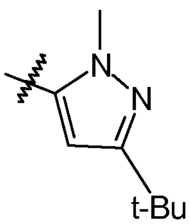, 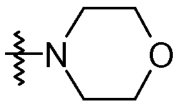,

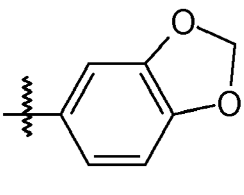, 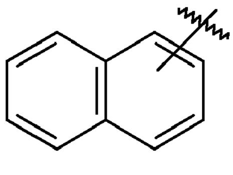, 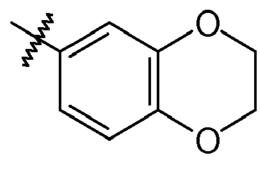,

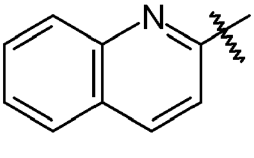, 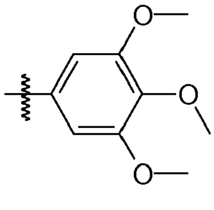, , 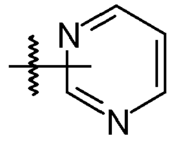,

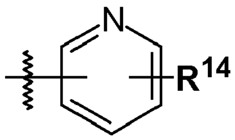
,
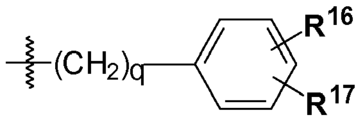
;

$R^{14}$ is -H, $-NH_2$, -OH, -OMe; $R^{16}$ and $R^{17}$ are independently of each other -H, -F, -Cl, -OH, -CN, $-CH_3$, $-CH(CH_3)_2$, $-CH_2OH$, $-CF_3$, $-OCH_3$, $-OCH(CH_3)_2$, $-OCF_3$, -OPh, $-SCH_3$, $-NH_2$, $-N(CH_3)_2$, $-NHCOCH_3$, $-NHSO_2CH_3$, $-SO_2CH_3$, $-COCH_3$, $-CO_2H$, $-CO_2CH_3$, $-CONH_2$, $-CONH(CH_3)$, $-CON(CH_3)_2$, $-CONHC_2H_4OH$, $-CONHC_2H_4OCH_3$,

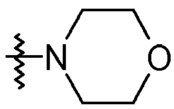
,
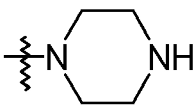
,
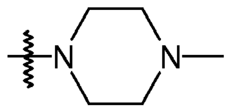
;

m, n and q are integer from 0 to 2;

Even more preferred are compounds of the general formula **(VII)**

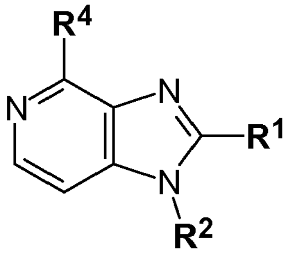

**(VII)**

wherein

**$R^1$** represents **$-R^5$;**

**$R^2$** represents -H, $-CH_3$, $-CH_2Ph$;

**$R^4$** represents $-(CH_2)_p$-**$R^8$**, or -N**$R^9$**($(CH_2)_p$-**$R^8$**);

**$R^5$** and **$R^8$** are independently of each other -H, $-CH(CH_3)_2$, -cyclo-$C_3H_5$, -OH, $-OCH_3$, $-C(CH(CH_3)_2)CH_2OH$,

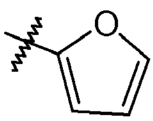
,
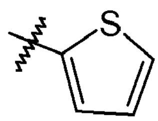
,
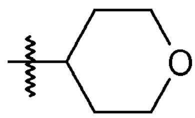
,

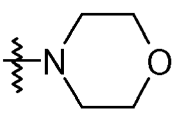
,
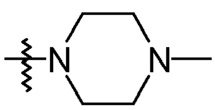
,
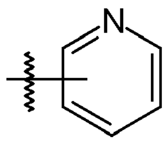
,

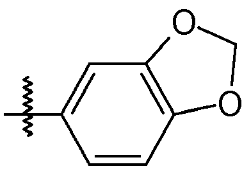
,
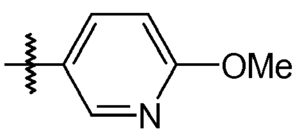
,
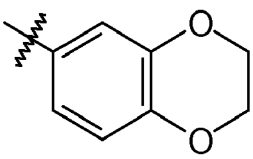
,

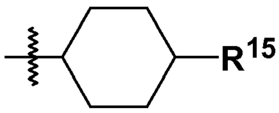 , 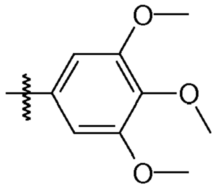 , , 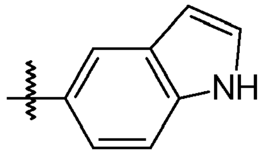 ,

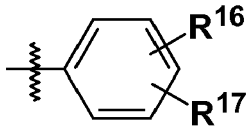 ;

**$R^9$** is -H, $-CH_3$;
**$R^{15}$** is -H, $-NH_2$, -OH;
**$R^{16}$** and **$R^{17}$** are independently of each other -H, -F, -Cl, -OH, $-CH_3$, $-CH(CH_3)_2$, $-CF_3$, $-OCH_3$, $-OCF_3$, -OPh, $-N(CH_3)_2$, $-NHSO_2CH_3$, $-SO_2CH_3$, $-CONH_2$, $-CONHC_2H_4OCH_3$, $-CONHC_2H_4N(C_2H_5)_2$;
p is integer from 0 to 3.

Most preferred compounds are selected from the group consisting of:

| compound | **name** |
|---|---|
| 1 | 2-(3-aminophenyl)-N-(6-methoxy-3-pyridyl)-1H-pyrrolo[3,2-c]pyridin-6-amine |
| 2 | 2-(3-aminophenyl)-N-(3-pyridyl)-1H-pyrrolo[3,2-c]pyridin-6-amine |
| 3 | 2-(3-aminophenyl)-N-[4-(4-methylpiperazin-1-yl)phenyl]-1H-pyrrolo[3,2-c]pyridin-6-amine |
| 4 | 6-(4-methylpiperazin-1-yl)-2-[3-(trifluoromethyl)phenyl]-1H-pyrrolo[3,2-c]pyridine |
| 5 | N-[3-[[2-[3-(trifluoromethyl)phenyl]-1H-pyrrolo[3,2-c]pyridin-6-yl]amino]phenyl]methanesulfonamide |
| 6 | 4-[[2-(3-fluorophenyl)-1H-pyrrolo[3,2-c]pyridin-6-yl]amino]benzamide |
| 7 | N-(3,4-dimethoxyphenyl)-2-(3-fluorophenyl)-1H-pyrrolo[3,2-c]pyridin-6-amine |
| 8 | 2-(3-fluorophenyl)-N-(6-methoxy-3-pyridyl)-1H-pyrrolo[3,2-c]pyridin-6-amine |
| 9 | N-(3-chloro-4-fluoro-phenyl)-2-(3-fluorophenyl)-1H-pyrrolo[3,2-c]pyridin-6-amine |
| 10 | 3-[[2-(3-fluorophenyl)-1H-pyrrolo[3,2-c]pyridin-6-yl]amino]phenol |
| 11 | 5-[[2-(3-fluorophenyl)-1H-pyrrolo[3,2-c]pyridin-6-yl]amino]-2-methoxy-phenol |
| 12 | 4-[2-(3-fluorophenyl)-1H-pyrrolo[3,2-c]pyridin-6-yl]morpholine |
| 13 | N-[3-[[2-(3-fluorophenyl)-1H-pyrrolo[3,2-c]pyridin-6-yl]amino]phenyl]methanesulfonamide |
| 14 | 4-[[2-(3-fluorophenyl)-1H-pyrrolo[3,2-c]pyridin-6-yl]amino]-N,N-dimethyl-benzamide |
| 15 | N-[3-[[2-(3-fluorophenyl)-1H-pyrrolo[3,2-c]pyridin-6-yl]amino]phenyl]acetamide |
| 16 | 2-(3-fluorophenyl)-N-(4-methylsulfonylphenyl)-1H-pyrrolo[3,2-c]pyridin-6-amine |
| 17 | 4-[[2-(3-fluorophenyl)-1-methyl-pyrrolo[3,2-c]pyridin-6-yl]amino]phenol |
| 18 | N,N-dimethyl-4-[6-(3,4,5-trimethoxyanilino)-1H-pyrrolo[3,2-c]pyridin-2-yl]benzamide |
| 19 | 4-[6-(3-methoxyanilino)-1H-pyrrolo[3,2-c]pyridin-2-yl]-N,N-dimethyl-benzamide |
| 20 | 4-[6-(2,4-dimethoxyanilino)-1H-pyrrolo[3,2-c]pyridin-2-yl]-N,N-dimethyl-benzamide |
| 21 | 4-[6-(3,4-dimethoxyanilino)-1H-pyrrolo[3,2-c]pyridin-2-yl]-N,N-dimethyl-benzamide |
| 22 | N,N-dimethyl-4-[6-(3-pyridylamino)-1H-pyrrolo[3,2-c]pyridin-2-yl]benzamide |
| 23 | 4-[6-(3-chloroanilino)-1H-pyrrolo[3,2-c]pyridin-2-yl]-N,N-dimethyl-benzamide |
| 24 | 4-(6-anilino-1 H-pyrrolo[3,2-c]pyridin-2-yl)-N,N-dimethyl-benzamide |

(continued)

| compound | name |
|---|---|
| 25 | N,N-dimethyl-4-[6-(4-phenoxyanilino)-1H-pyrrolo[3,2-c]pyridin-2-yl]benzamide |
| 26 | N,N-dimethyl-4-[6-[4-(4-methylpiperazin-1-yl)anilino]-1H-pyrrolo[3,2-c]pyridin-2-yl]benzamide |
| 27 | 4-[6-[3-(dimethylamino)anilino]-1H-pyrrolo[3,2-c]pyridin-2-yl]-N,N-dimethyl-benzamide |
| 28 | N,N-dimethyl-4-[6-(3-methylanilino)-1H-pyrrolo[3,2-c]pyridin-2-yl]benzamide |
| 29 | methyl 4-[[2-[4-(dimethylcarbamoyl)phenyl]-1H-pyrrolo[3,2-c]pyridin-6-yl]amino]benzoate |
| 30 | N,N-dimethyl-4-[6-(3-methylsulfonylanilino)-1H-pyrrolo[3,2-c]pyridin-2-yl]benzamide |
| 31 | 4-[6-(3-hydroxyanilino)-1H-pyrrolo[3,2-c]pyridin-2-yl]-N,N-dimethyl-benzamide |
| 32 | N,N-dimethyl-4-[6-[4-(trifluoromethyl)anilino]-1H-pyrrolo[3,2-c]pyridin-2-yl]benzamide |
| 33 | N,N-dimethyl-4-[6-[3-(trifluoromethoxy)anilino]-1H-pyrrolo[3,2-c]pyridin-2-yl]benzamide |
| 34 | N,N-dimethyl-4-[6-[4-(trifluoromethoxy)anilino]-1H-pyrrolo[3,2-c]pyridin-2-yl]benzamide |
| 35 | N,N-dimethyl-4-[6-(3-phenoxyanilino)-1H-pyrrolo[3,2-c]pyridin-2-yl]benzamide |
| 36 | 4-[6-(3-isopropylanilino)-1H-pyrrolo[3,2-c]pyridin-2-yl]-N,N-dimethyl-benzamide |
| 37 | 4-[6-(2-chloroanilino)-1H-pyrrolo[3,2-c]pyridin-2-yl]-N,N-dimethyl-benzamide |
| 38 | 4-[6-(4-isopropylanilino)-1H-pyrrolo[3,2-c]pyridin-2-yl]-N,N-dimethyl-benzamide |
| 39 | 4-[6-[3-(methanesulfonamido)anilino]-1H-pyrrolo[3,2-c]pyridin-2-yl]-N,N-dimethyl-benzamide |
| 40 | 4-[6-[4-(dimethylcarbamoyl)anilino]-1H-pyrrolo[3,2-c]pyridin-2-yl]-N,N-dimethyl-benzamide |
| 41 | 4-[6-(3-acetamidoanilino)-1H-pyrrolo[3,2-c]pyridin-2-yl]-N,N-dimethyl-benzamide |
| 42 | 4-[6-(3-acetylanilino)-1H-pyrrolo[3,2-c]pyridin-2-yl]-N,N-dimethyl-benzamide |
| 43 | N,N-dimethyl-4-[6-(4-methylsulfonylanilino)-1H-pyrrolo[3,2-c]pyridin-2-yl]benzamide |
| 44 | 4-[6-(3-isopropoxyanilino)-1H-pyrrolo[3,2-c]pyridin-2-yl]-N,N-dimethyl-benzamide |
| 45 | 4-[6-(3-methoxyanilino)-1-methyl-pyrrolo[3,2-c]pyridin-2-yl]-N,N-dimethyl-benzamide |
| 46 | 4-[6-(3,4-dimethoxyanilino)-1-methyl-pyrrolo[3,2-c]pyridin-2-yl]-N,N-dimethyl-benzamide |
| 47 | N,N-dimethyl-4-[1-methyl-6-(3-pyridylamino)pyrrolo[3,2-c]pyridin-2-yl]benzamide |
| 48 | 4-(6-anilino-1-methyl-pyrrolo[3,2-c]pyridin-2-yl)-N,N-dimethyl-benzamide |
| 49 | N,N-dimethyl-4-[1-methyl-6-[4-(4-methylpiperazin-1-yl)anilino]pyrrolo[3,2-c]pyridin-2-yl]benzamide |
| 50 | 4-[6-[3-(dimethylamino)anilino]-1-methyl-pyrrolo[3,2-c]pyridin-2-yl]-N,N-dimethyl-benzamide |
| 51 | N,N-dimethyl-4-[1-methyl-6-(2-pyridylamino)pyrrolo[3,2-c]pyridin-2-yl]benzamide |
| 52 | N,N-dimethyl-4-[1-methyl-6-(N-methylanilino)pyrrolo[3,2-c]pyridin-2-yl]benzamide |
| 53 | 4-[6-(3-hydroxyanilino)-1-methyl-pyrrolo[3,2-c]pyridin-2-yl]-N,N-dimethyl-benzamide |
| 54 | 4-[6-(3-hydroxy-4-methoxy-anilino)-1-methyl-pyrrolo[3,2-c]pyridin-2-yl]-N,N-dimethyl-benzamide |
| 55 | 4-[6-[3-(methanesulfonamido)anilino]-1-methyl-pyrrolo[3,2-c]pyridin-2-yl]-N,N-dimethyl-benzamide |
| 56 | 4-[6-(3-acetamidoanilino)-1-methyl-pyrrolo[3,2-c]pyridin-2-yl]-N,N-dimethyl-benzamide |
| 57 | 4-[6-(4-acetamidoanilino)-1-benzyl-pyrrolo[3,2-c]pyridin-2-yl]-N,N-dimethyl-benzamide |
| 58 | 4-[1-benzyl-6-(pyrimidin-4-ylamino)pyrrolo[3,2-c]pyridin-2-yl]-N,N-dimethyl-benzamide |
| 59 | 2-(4-dimethylaminophenyl)-1-methyl-N-(2-pyridyl)pyrrolo[3,2-c]pyridin-6-amine |
| 60 | 3-[[1-benzyl-2-(4-dimethylaminophenyl)pyrrolo[3,2-c]pyridin-6-yl]amino]phenol |
| 61 | 1-benzyl-2-(4-dimethylaminophenyl)-N-pyrimidin-4-yl-pyrrolo[3,2-c]pyridin-6-amine |
| 62 | 2-(2-pyridyl)-N-(3,4,5-trimethoxyphenyl)-1H-pyrrolo[3,2-c]pyridin-6-amine |

(continued)

| compound | name |
|---|---|
| 63 | N-(4-methoxy-2-methyl-phenyl)-2-(2-pyridyl)-1H-pyrrolo[3,2-c]pyridin-6-amine |
| 64 | N-(m-tolyl)-2-(2-pyridyl)-1H-pyrrolo[3,2-c]pyridin-6-amine |
| 65 | N-(4-methoxyphenyl)-2-(2-pyridyl)-1H-pyrrolo[3,2-c]pyridin-6-amine |
| 66 | 2-(2-pyridyl)-N-[3-(trifluoromethyl)phenyl]-1H-pyrrolo[3,2-c]pyridin-6-amine |
| 67 | methyl 4-[[2-(2-pyridyl)-1H-pyrrolo[3,2-c]pyridin-6-yl]amino]benzoate |
| 68 | 4-[[2-(2-pyridyl)-1H-pyrrolo[3,2-c]pyridin-6-yl]amino]benzonitrile |
| 69 | 2-(2-pyridyl)-N-[3-(trifluoromethoxy)phenyl]-1H-pyrrolo[3,2-c]pyridin-6-amine |
| 70 | N,N-dimethyl-4-[[2-(2-pyridyl)-1H-pyrrolo[3,2-c]pyridin-6-yl]amino]benzamide |
| 71 | N-(3-fluorophenyl)-2-(2-pyridyl)-1H-pyrrolo[3,2-c]pyridin-6-amine |
| 72 | N-(4-methylsulfonylphenyl)-2-(2-pyridyl)-1H-pyrrolo[3,2-c]pyridin-6-amine |
| 73 | N-(3-isopropoxyphenyl)-2-(2-pyridyl)-1H-pyrrolo[3,2-c]pyridin-6-amine |
| 74 | N-[4-[[2-(3-pyridyl)-1H-pyrrolo[3,2-c]pyridin-6-yl]amino]phenyl]acetamide |
| 75 | N-(6-methoxy-3-pyridyl)-2-(3-pyridyl)-1H-pyrrolo[3,2-c]pyridin-6-amine |
| 76 | 2-(3-pyridyl)-N-(4-pyridyl)-1H-pyrrolo[3,2-c]pyridin-6-amine |
| 77 | 4-[2-(3-pyridyl)-1H-pyrrolo[3,2-c]pyridin-6-yl]morpholine |
| 78 | N-(2-chlorophenyl)-2-(3-pyridyl)-1H-pyrrolo[3,2-c]pyridin-6-amine |
| 79 | N-[2-fluoro-5-(trifluoromethyl)phenyl]-2-(3-pyridyl)-1H-pyrrolo[3,2-c]pyridin-6-amine |
| 80 | N-[3-[[2-(3-pyridyl)-1H-pyrrolo[3,2-c]pyridin-6-yl]amino]phenyl]acetamide |
| 81 | N-(4-isopropoxyphenyl)-2-(3-pyridyl)-1H-pyrrolo[3,2-c]pyridin-6-amine |
| 82 | 1-methyl-2-(3-pyridyl)-N-(3,4,5-trimethoxyphenyl)pyrrolo[3,2-c]pyridin-6-amine |
| 83 | N-(2,4-dimethoxyphenyl)-1-methyl-2-(3-pyridyl)pyrrolo[3,2-c]pyridin-6-amine |
| 84 | N-(2,3-dihydro-1,4-benzodioxin-6-yl)-1-methyl-2-(3-pyridyl)pyrrolo[3,2-c]pyridin-6-amine |
| 85 | N-(3,4-dimethoxyphenyl)-1-methyl-2-(3-pyridyl)pyrrolo[3,2-c]pyridin-6-amine |
| 86 | N-(6-methoxy-3-pyridyl)-1-methyl-2-(3-pyridyl)pyrrolo[3,2-c]pyridin-6-amine |
| 87 | 1-methyl-N,2-bis(3-pyridyl)pyrrolo[3,2-c]pyridin-6-amine |
| 88 | 1-methyl-N-phenyl-2-(3-pyridyl)pyrrolo[3,2-c]pyridin-6-amine |
| 89 | N1,N1-dimethyl-N3-[1-methyl-2-(3-pyridyl)pyrrolo[3,2-c]pyridin-6-yl]benzene-1,3-diamine |
| 90 | 1-methyl-N-(m-tolyl)-2-(3-pyridyl)pyrrolo[3,2-c]pyridin-6-amine |
| 91 | N-(4-methoxyphenyl)-1-methyl-2-(3-pyridyl)pyrrolo[3,2-c]pyridin-6-amine |
| 92 | N-(4-fluorophenyl)-1-methyl-2-(3-pyridyl)pyrrolo[3,2-c]pyridin-6-amine |
| 93 | N-(1,3-benzodioxol-5-yl)-1-methyl-2-(3-pyridyl)pyrrolo[3,2-c]pyridin-6-amine |
| 94 | 4-[1-methyl-2-(3-pyridyl)pyrrolo[3,2-c]pyridin-6-yl]morpholine |
| 95 | N,N-dimethyl-4-[[1-methyl-2-(3-pyridyl)pyrrolo[3,2-c]pyridin-6-yl]amino]benzamide |
| 96 | N-[3-[[1-methyl-2-(3-pyridyl)pyrrolo[3,2-c]pyridin-6-yl]amino]phenyl]acetamide |
| 97 | 1-[3-[[1-methyl-2-(3-pyridyl)pyrrolo[3,2-c]pyridin-6-yl]amino]phenyl]ethanone |
| 98 | 1-benzyl-N-(2-pyridyl)-2-(3-pyridyl)pyrrolo[3,2-c]pyridin-6-amine |
| 99 | 1-benzyl-2-(3-pyridyl)-N-pyrimidin-4-yl-pyrrolo[3,2-c]pyridin-6-amine |
| 100 | 2-(3-methylimidazol-4-yl)-N-(3,4,5-trimethoxyphenyl)-1H-pyrrolo[3,2-c]pyridin-6-amine |

(continued)

| compound | name |
|---|---|
| 101 | N-(3-methoxyphenyl)-2-(3-methylimidazol-4-yl)-1H-pyrrolo[3,2-c]pyridin-6-amine |
| 102 | N-(3,4-dimethoxyphenyl)-2-(3-methylimidazol-4-yl)-1H-pyrrolo[3,2-c]pyridin-6-amine |
| 103 | N-(3-chlorophenyl)-2-(3-methylimidazol-4-yl)-1H-pyrrolo[3,2-c]pyridin-6-amine |
| 104 | N1,N1-dimethyl-N3-[2-(3-methylimidazol-4-yl)-1H-pyrrolo[3,2-c]pyridin-6-yl]benzene-1,3-diamine |
| 105 | 2-(3-methylimidazol-4-yl)-N-(m-tolyl)-1H-pyrrolo[3,2-c]pyridin-6-amine |
| 106 | N-(4-fluorophenyl)-2-(3-methylimidazol-4-yl)-1H-pyrrolo[3,2-c]pyridin-6-amine |
| 107 | 1-[3-[[2-(3-methylimidazol-4-yl)-1H-pyrrolo[3,2-c]pyridin-6-yl]amino]phenyl]ethanone |
| 108 | N-(3-fluorophenyl)-2-(3-methylimidazol-4-yl)-1H-pyrrolo[3,2-c]pyridin-6-amine |
| 109 | N-(4-isopropoxyphenyl)-2-(3-methylimidazol-4-yl)-1H-pyrrolo[3,2-c]pyridin-6-amine |
| 110 | N-(3-methoxyphenyl)-2-(1H-pyrazol-4-yl)-1H-pyrrolo[3,2-c]pyridin-6-amine |
| 111 | N-(3,4-dimethoxyphenyl)-2-(1H-pyrazol-4-yl)-1H-pyrrolo[3,2-c]pyridin-6-amine |
| 112 | N1,N1-dimethyl-N3-[2-(1H-pyrazol-4-yl)-1H-pyrrolo[3,2-c]pyridin-6-yl]benzene-1,3-diamine |
| 113 | N-(m-tolyl)-2-(1H-pyrazol-4-yl)-1H-pyrrolo[3,2-c]pyridin-6-amine |
| 114 | N-(4-fluorophenyl)-2-(1H-pyrazol-4-yl)-1H-pyrrolo[3,2-c]pyridin-6-amine |
| 115 | 2-(1H-pyrazol-4-yl)-N-(2-pyridyl)-1H-pyrrolo[3,2-c]pyridin-6-amine |
| 116 | N-[3-methoxy-5-(trifluoromethyl)phenyl]-2-(1H-pyrazol-4-yl)-1H-pyrrolo[3,2-c]pyridin-6-amine |
| 117 | N-[3-[[2-(1H-pyrazol-4-yl)-1H-pyrrolo[3,2-c]pyridin-6-yl]amino]phenyl]acetamide |
| 118 | 1-[3-[[2-(1 H-pyrazol-4-yl)-1H-pyrrolo[3,2-c]pyridin-6-yl]amino]phenyl]ethanone |
| 119 | N-(3-fluorophenyl)-2-(1H-pyrazol-4-yl)-1H-pyrrolo[3,2-c]pyridin-6-amine |
| 120 | N-(4-methylsulfonylphenyl)-2-(1H-pyrazol-4-yl)-1H-pyrrolo[3,2-c]pyridin-6-amine |
| 121 | N-[4-[[2-(1-methylpyrazol-4-yl)-1H-pyrrolo[3,2-c]pyridin-6-yl]amino]phenyl]acetamide |
| 122 | N-(2,3-dihydro-1,4-benzodioxin-6-yl)-2-(1-methylpyrazol-4-yl)-1H-pyrrolo[3,2-c]pyridin-6-amine |
| 123 | N-(3,4-dimethoxyphenyl)-2-(1-methylpyrazol-4-yl)-1H-pyrrolo[3,2-c]pyridin-6-amine |
| 124 | 1-[3-[[2-(1-methylpyrazol-4-yl)-1H-pyrrolo[3,2-c]pyridin-6-yl]amino]phenyl]ethanone |
| 125 | 1-methyl-2-(1-methylpyrazol-4-yl)-N-(3,4,5-trimethoxyphenyl)pyrrolo[3,2-c]pyridin-6-amine |
| 126 | N-[4-[[1-methyl-2-(1-methylpyrazol-4-yl)pyrrolo[3,2-c]pyridin-6-yl]amino]phenyl]acetamide |
| 127 | N-(3-methoxyphenyl)-1-methyl-2-(1-methylpyrazol-4-yl)pyrrolo[3,2-c]pyridin-6-amine |
| 128 | N-(2,3-dihydro-1,4-benzodioxin-6-yl)-1-methyl-2-(1-methylpyrazol-4-yl)pyrrolo[3,2-c]pyridin-6-amine |
| 129 | N-(3,4-dimethoxyphenyl)-1-methyl-2-(1-methylpyrazol-4-yl)pyrrolo[3,2-c]pyridin-6-amine |
| 130 | N-(6-methoxy-3-pyridyl)-1-methyl-2-(1-methylpyrazol-4-yl)pyrrolo[3,2-c]pyridin-6-amine |
| 131 | 1-methyl-2-(1-methylpyrazol-4-yl)-N-(3-pyridyl)pyrrolo[3,2-c]pyridin-6-amine |
| 132 | 1-methyl-2-(1-methylpyrazol-4-yl)-N-phenyl-pyrrolo[3,2-c]pyridin-6-amine |
| 133 | 1-methyl-N-[4-(4-methylpiperazin-1-yl)phenyl]-2-(1-methylpyrazol-4-yl)pyrrolo[3,2-c]pyridin-6-amine |
| 134 | N1,N1-dimethyl-N3-[1-methyl-2-(1-methylpyrazol-4-yl)pyrrolo[3,2-c]pyridin-6-yl]benzene-1,3-diamine |
| 135 | 1-methyl-2-(1-methylpyrazol-4-yl)-N-(4-pyridyl)pyrrolo[3,2-c]pyridin-6-amine |
| 136 | 1-methyl-2-(1-methylpyrazol-4-yl)-N-(m-tolyl)pyrrolo[3,2-c]pyridin-6-amine |
| 137 | N-(4-methoxyphenyl)-1-methyl-2-(1-methylpyrazol-4-yl)pyrrolo[3,2-c]pyridin-6-amine |
| 138 | N-(4-fluorophenyl)-1-methyl-2-(1-methylpyrazol-4-yl)pyrrolo[3,2-c]pyridin-6-amine |

(continued)

| compound | name |
|---|---|
| 139 | 1-methyl-2-(1-methylpyrazol-4-yl)-N-(2-pyridyl)pyrrolo[3,2-c]pyridin-6-amine |
| 140 | N,1-dimethyl-2-(1-methylpyrazol-4-yl)-N-phenyl-pyrrolo[3,2-c]pyridin-6-amine |
| 141 | methyl 4-[[1-methyl-2-(1-methylpyrazol-4-yl)pyrrolo[3,2-c]pyridin-6-yl]amino]benzoate |
| 142 | 4-[[1-methyl-2-(1-methylpyrazol-4-yl)pyrrolo[3,2-c]pyridin-6-yl]amino]benzonitrile |
| 143 | N-(1,3-benzodioxol-5-yl)-1-methyl-2-(1-methylpyrazol-4-yl)pyrrolo[3,2-c]pyridin-6-amine |
| 144 | 3-[[1-methyl-2-(1-methylpyrazol-4-yl)pyrrolo[3,2-c]pyridin-6-yl]amino]phenol |
| 145 | 4-[1-methyl-2-(1-methylpyrazol-4-yl)pyrrolo[3,2-c]pyridin-6-yl]morpholine |
| 146 | 1-methyl-2-(1-methylpyrazol-4-yl)-N-[3-(trifluoromethoxy)phenyl]pyrrolo[3,2-c]pyridin-6-amine |
| 147 | 1-methyl-2-(1-methylpyrazol-4-yl)-N-pyrimidin-4-yl-pyrrolo[3,2-c]pyridin-6-amine |
| 148 | N,N-dimethyl-4-[[1-methyl-2-(1-methylpyrazol-4-yl)pyrrolo[3,2-c]pyridin-6-yl]amino]benzamide |
| 149 | N-[3-[[1-methyl-2-(1-methylpyrazol-4-yl)pyrrolo[3,2-c]pyridin-6-yl]amino]phenyl]acetamide |
| 150 | 1-[3-[[1-methyl-2-(1-methylpyrazol-4-yl)pyrrolo[3,2-c]pyridin-6-yl]amino]phenyl]ethanone |
| 151 | N-(3-fluorophenyl)-1-methyl-2-(1-methylpyrazol-4-yl)pyrrolo[3,2-c]pyridin-6-amine |
| 152 | 1-methyl-2-(1-methylpyrazol-4-yl)-N-(4-methylsulfonylphenyl)pyrrolo[3,2-c]pyridin-6-amine |
| 153 | 1-methyl-2-(1-methylpyrazol-4-yl)-N-(4-methylsulfanylphenyl)pyrrolo[3,2-c]pyridin-6-amine |
| 154 | 1-benzyl-6-(4-methylpiperazin-1-yl)-2-(1-methylpyrazol-4-yl)pyrrolo[3,2-c]pyridine |
| 155 | 4-[1-benzyl-2-(1-methylpyrazol-4-yl)pyrrolo[3,2-c]pyridin-6-yl]morpholine |
| 156 | N-[3-[[1-benzyl-2-(1-methylpyrazol-4-yl)pyrrolo[3,2-c]pyridin-6-yl]amino]phenyl]methanesulfonamide |
| 157 | N-[3-[[1-benzyl-2-(1-methylpyrazol-4-yl)pyrrolo[3,2-c]pyridin-6-yl]amino]phenyl]acetamide |
| 158 | N-methyl-N-[3-[1-methyl-6-(3,4,5-trimethoxyanilino)pyrrolo[3,2-c]pyridin-2-yl]phenyl]acetamide |
| 159 | 4-[[2-[3-[acetyl(methyl)amino]phenyl]-1-methyl-pyrrolo[3,2-c]pyridin-6-yl]amino]benzamide |
| 160 | N-[3-[6-(3,4-dimethoxyanilino)-1-methyl-pyrrolo[3,2-c]pyridin-2-yl]phenyl]-N-methyl-acetamide |
| 161 | N-[3-[6-[(6-methoxy-3-pyridyl)amino]-1-methyl-pyrrolo[3,2-c]pyridin-2-yl]phenyl]-N-methyl-acetamide |
| 162 | N-methyl-N-[3-[1-methyl-6-(3-pyridylamino)pyrrolo[3,2-c]pyridin-2-yl]phenyl]acetamide |
| 163 | N-methyl-N-[3-[1-methyl-6-(4-morpholinoanilino)pyrrolo[3,2-c]pyridin-2-yl]phenyl]acetamide |
| 164 | N-methyl-N-[3-[1-methyl-6-[4-(4-methylpiperazin-1-yl)anilino]pyrrolo[3,2-c]pyridin-2-yl]phenyl]acetamide |
| 165 | N-[3-[6-[3-(dimethylamino)anilino]-1-methyl-pyrrolo[3,2-c]pyridin-2-yl]phenyl]-N-methyl-acetamide |
| 166 | N-[3-[6-(4-methoxyanilino)-1-methyl-pyrrolo[3,2-c]pyridin-2-yl]phenyl]-N-methyl-acetamide |
| 167 | N-methyl-N-[3-[1-methyl-6-(2-pyridylamino)pyrrolo[3,2-c]pyridin-2-yl]phenyl]acetamide |
| 168 | N-[3-[6-(4-cyanoanilino)-1-methyl-pyrrolo[3,2-c]pyridin-2-yl]phenyl]-N-methyl-acetamide |
| 169 | N-[3-[6-(1,3-benzodioxol-5-ylamino)-1-methyl-pyrrolo[3,2-c]pyridin-2-yl]phenyl]-N-methyl-acetamide |
| 170 | N-[3-[6-(3-hydroxyanilino)-1-methyl-pyrrolo[3,2-c]pyridin-2-yl]phenyl]-N-methyl-acetamide |
| 171 | N-methyl-N-[3-(1-methyl-6-morpholino-pyrrolo[3,2-c]pyridin-2-yl)phenyl]acetamide |
| 172 | 4-[[2-[3-[acetyl(methyl)amino]phenyl]-1-methyl-pyrrolo[3,2-c]pyridin-6-yl]amino]-N,N-dimethyl-benzamide |
| 173 | N-methyl-N-[3-[1-methyl-6-(4-methylsulfonylanilino)pyrrolo[3,2-c]pyridin-2-yl]phenyl]acetamide |
| 174 | 2-phenyl-N-(3,4,5-trimethoxyphenyl)-1H-pyrrolo[3,2-c]pyridin-6-amine |

(continued)

| compound | name |
|---|---|
| 175 | N-(3,4-dimethoxyphenyl)-2-phenyl-1 H-pyrrolo[3,2-c]pyridin-6-amine |
| 176 | N-(4-morpholinophenyl)-2-phenyl-1H-pyrrolo[3,2-c]pyridin-6-amine |
| 177 | N-[4-(4-methylpiperazin-1-yl)phenyl]-2-phenyl-1H-pyrrolo[3,2-c]pyridin-6-amine |
| 178 | 2-phenyl-N-(4-pyridyl)-1H-pyrrolo[3,2-c]pyridin-6-amine |
| 179 | N-(4-fluorophenyl)-2-phenyl-1H-pyrrolo[3,2-c]pyridin-6-amine |
| 180 | 3-[(2-phenyl-1H-pyrrolo[3,2-c]pyridin-6-yl)amino]phenol |
| 181 | 4-(2-phenyl-1H-pyrrolo[3,2-c]pyridin-6-yl)morpholine |
| 182 | N,N-dimethyl-4-[(2-phenyl-1H-pyrrolo[3,2-c]pyridin-6-yl)amino]benzamide |
| 183 | N-[3-[(2-phenyl-1H-pyrrolo[3,2-c]pyridin-6-yl)amino]phenyl]acetamide |
| 184 | 1-benzyl-2-phenyl-N-pyrimidin-4-yl-pyrrolo[3,2-c]pyridin-6-amine |
| 185 | 1-methyl-4-(4-methylpiperazin-1-yl)-2-[3-(trifluoromethyl)phenyl]pyrrolo[3,2-c]pyridine |
| 186 | 2-(3-fluorophenyl)-4-(4-methylpiperazin-1-yl)-1H-pyrrolo[3,2-c]pyridine |
| 187 | 4-[2-(3-fluorophenyl)-1H-pyrrolo[3,2-c]pyridin-4-yl]morpholine |
| 188 | N,N-dimethyl-4-(1-methyl-4-morpholino-pyrrolo[3,2-c]pyridin-2-yl)benzamide |
| 189 | N,N-dimethyl-4-(4-morpholino-1H-pyrrolo[3,2-c]pyridin-2-yl)aniline |
| 190 | 4-(4-methylpiperazin-1-yl)-2-(3-thienyl)-1H-pyrrolo[3,2-c]pyridine |
| 191 | 4-[2-(3-thienyl)-1H-pyrrolo[3,2-c]pyridin-4-yl]morpholine |
| 192 | 4-[2-(3,5-dimethoxyphenyl)-1-methyl-pyrrolo[3,2-c]pyridin-4-yl]morpholine |
| 193 | 4-[2-(2-methoxyphenyl)-1H-pyrrolo[3,2-c]pyridin-4-yl]morpholine |
| 194 | 2-(3-methoxyphenyl)-1-methyl-4-(4-methylpiperazin-1-yl)pyrrolo[3,2-c]pyridine |
| 195 | 4-[2-(3-chlorophenyl)-1-methyl-pyrrolo[3,2-c]pyridin-4-yl]morpholine |
| 196 | N-(3-pyridyl)-2-[3-(trifluoromethyl)phenyl]-1H-pyrrolo[3,2-c]pyridin-4-amine |
| 197 | N-(4-methylsulfonylphenyl)-2-[3-(trifluoromethyl)phenyl]-1H-pyrrolo[3,2-c]pyridin-4-amine |
| 198 | N-[4-[[2-(3-fluorophenyl)-1H-pyrrolo[3,2-c]pyridin-4-yl]amino]phenyl]acetamide |
| 199 | 4-[[2-(3-fluorophenyl)-1H-pyrrolo[3,2-c]pyridin-4-yl]amino]phenol |
| 200 | 2-(3-fluorophenyl)-N-(3-pyridyl)-1H-pyrrolo[3,2-c]pyridin-4-amine |
| 201 | 2-(3-fluorophenyl)-N-[4-(4-methylpiperazin-1-yl)phenyl]-1H-pyrrolo[3,2-c]pyridin-4-amine |
| 202 | 2-(3-fluorophenyl)-N-(3-methylsulfonylphenyl)-1H-pyrrolo[3,2-c]pyridin-4-amine |
| 203 | N-(1,3-benzodioxol-5-yl)-2-(3-fluorophenyl)-1H-pyrrolo[3,2-c]pyridin-4-amine |
| 204 | 5-[[2-(3-fluorophenyl)-1H-pyrrolo[3,2-c]pyridin-4-yl]amino]-2-methoxy-phenol |
| 205 | N-[3-[[2-(3-fluorophenyl)-1H-pyrrolo[3,2-c]pyridin-4-yl]amino]phenyl]methanesulfonamide |
| 206 | N-[3-[[2-(3-fluorophenyl)-1H-pyrrolo[3,2-c]pyridin-4-yl]amino]phenyl]acetamide |
| 207 | 2-(3-fluorophenyl)-N-(4-methylsulfonylphenyl)-1H-pyrrolo[3,2-c]pyridin-4-amine |
| 208 | N-(3,4-dimethoxyphenyl)-2-(3-fluorophenyl)-1-methyl-pyrrolo[3,2-c]pyridin-4-amine |
| 209 | 2-(3-fluorophenyl)-1-methyl-N-[4-(4-methylpiperazin-1-yl)phenyl]pyrrolo[3,2-c]pyridin-4-amine |
| 210 | N-[3-[[2-(3-fluorophenyl)-1-methyl-pyrrolo[3,2-c]pyridin-4-yl]amino]phenyl]acetamide |
| 211 | 4-[4-(4-acetamidoanilino)-1-methyl-pyrrolo[3,2-c]pyridin-2-yl]-N,N-dimethyl-benzamide |
| 212 | 4-[4-(3-methoxyanilino)-1-methyl-pyrrolo[3,2-c]pyridin-2-yl]-N,N-dimethyl-benzamide |

(continued)

| compound | **name** |
|---|---|
| 213 | 4-[4-(4-carbamoylanilino)-1-methyl-pyrrolo[3,2-c]pyridin-2-yl]-N,N-dimethyl-benzamide |
| 214 | 4-[4-(2,3-dihydro-1,4-benzodioxin-6-ylamino)-1-methyl-pyrrolo[3,2-c]pyridin-2-yl]-N,N-dimethyl-benzamide |
| 215 | 4-[4-(3,4-dimethoxyanilino)-1-methyl-pyrrolo[3,2-c]pyridin-2-yl]-N,N-dimethyl-benzamide |
| 216 | 4-[4-[(6-methoxy-3-pyridyl)amino]-1-methyl-pyrrolo[3,2-c]pyridin-2-yl]-N,N-dimethyl-benzamide |
| 217 | N,N-dimethyl-4-[1-methyl-4-(4-phenoxyanilino)pyrrolo[3,2-c]pyridin-2-yl]benzamide |
| 218 | 4-[4-[3-(dimethylamino)anilino]-1-methyl-pyrrolo[3,2-c]pyridin-2-yl]-N,N-dimethyl-benzamide |
| 219 | N,N-di methyl-4-[1-methyl-4-(2-pyridylamino)pyrrolo[3,2-c]pyridin-2-yl]benzamide |
| 220 | N,N-dimethyl-4-[1-methyl-4-(3-methoxy-5-(trifuloromethyl)phenylamino)pyrrolo[3,2-c]pyridin-2-yl]benzamide |
| 221 | 4-[4-(3-hydroxyanilino)-1-methyl-pyrrolo[3,2-c]pyridin-2-yl]-N,N-dimethyl-benzamide |
| 222 | N,N-dimethyl-4-[1-methyl-4-[3-(trifluoromethoxy)anilino]pyrrolo[3,2-c]pyridin-2-yl]benzamide |
| 223 | N,N-dimethyl-4-[1-methyl-4-(3-phenoxyanilino)pyrrolo[3,2-c]pyridin-2-yl]benzamide |
| 224 | 4-[4-(3-isopropylanilino)-1-methyl-pyrrolo[3,2-c]pyridin-2-yl]-N,N-dimethyl-benzamide |
| 225 | 4-[4-[3-(methanesulfonamido)anilino]-1-methyl-pyrrolo[3,2-c]pyridin-2-yl]-N,N-dimethyl-benzamide |
| 226 | 4-[[2-(4-dimethylaminophenyl)-1-methyl-pyrrolo[3,2-c]pyridin-4-yl]amino]phenol |
| 227 | 4-[[2-(4-dimethylaminophenyl)-1-methyl-pyrrolo[3,2-c]pyridin-4-yl]amino]benzamide |
| 228 | 5-[[2-(4-dimethylaminophenyl)-1-methyl-pyrrolo[3,2-c]pyridin-4-yl]amino]-2-methoxy-phenol |
| 229 | 2-(4-dimethylaminophenyl)-1-methyl-N-pyrimidin-4-yl-pyrrolo[3,2-c]pyridin-4-amine |
| 230 | 2-(4-dimethylaminophenyl)-1-methyl-N-(4-methylsulfonylphenyl)pyrrolo[3,2-c]pyridin-4-amine |
| 231 | N-[4-[[2-(3-pyridyl)-1H-pyrrolo[3,2-c]pyridin-4-yl]amino]phenyl]acetamide |
| 232 | N-(2,3-dihydro-1,4-benzodioxin-6-yl)-2-(3-pyridyl)-1H-pyrrolo[3,2-c]pyridin-4-amine |
| 233 | N-(6-methoxy-3-pyridyl)-2-(3-pyridyl)-1H-pyrrolo[3,2-c]pyridin-4-amine |
| 234 | N,2-bis(3-pyridyl)-1H-pyrrolo[3,2-c]pyridin-4-amine |
| 235 | N-[4-(4-methylpiperazin-1-yl)phenyl]-2-(3-pyridyl)-1H-pyrrolo[3,2-c]pyridin-4-amine |
| 236 | 2-methoxy-5-[[2-(3-pyridyl)-1H-pyrrolo[3,2-c]pyridin-4-yl]amino]phenol |
| 237 | 1-methyl-2-(3-pyridyl)-N-(3,4,5-trimethoxyphenyl)pyrrolo[3,2-c]pyridin-4-amine |
| 238 | N-(3,4-dimethoxyphenyl)-1-methyl-2-(3-pyridyl)pyrrolo[3,2-c]pyridin-4-amine |
| 239 | N1,N1-dimethyl-N3-[1-methyl-2-(3-pyridyl)pyrrolo[3,2-c]pyridin-4-yl]benzene-1,3-diamine |
| 240 | N-(4-methoxy-2-methyl-phenyl)-1-methyl-2-(3-pyridyl)pyrrolo[3,2-c]pyridin-4-amine |
| 241 | 1-methyl-N-(m-tolyl)-2-(3-pyridyl)pyrrolo[3,2-c]pyridin-4-amine |
| 242 | N-(4-fluorophenyl)-1-methyl-2-(3-pyridyl)pyrrolo[3,2-c]pyridin-4-amine |
| 243 | 1-methyl-2-(3-pyridyl)-N-[3-(trifluoromethyl)phenyl]pyrrolo[3,2-c]pyridin-4-amine |
| 244 | N-[3-[[1-methyl-2-(3-pyridyl)pyrrolo[3,2-c]pyridin-4-yl]amino]phenyl]methanesulfonamide |
| 245 | N-[3-[[1-methyl-2-(3-pyridyl)pyrrolo[3,2-c]pyridin-4-yl]amino]phenyl]acetamide |
| 246 | 2-(1-methylpyrazol-4-yl)-N-(4-methylsulfonylphenyl)-1H-pyrrolo[3,2-c]pyridin-4-amine |
| 247 | 1-methyl-2-(1-methylpyrazol-4-yl)-N-(3-pyridyl)pyrrolo[3,2-c]pyridin-4-amine |
| 248 | N-methyl-N-[3-[1-methyl-4-[4-(trifluoromethoxy)anilino]pyrrolo[3,2-c]pyridin-2-yl]phenyl]acetamide |

(continued)

| compound | name |
|---|---|
| 249 | N-[4-[[2-(3-thienyl)-1H-pyrrolo[3,2-c]pyridin-4-yl]amino]phenyl]acetamide |
| 250 | 4-[[2-(3-thienyl)-1H-pyrrolo[3,2-c]pyridin-4-yl]amino]benzamide |
| 251 | N-(2,4-dimethoxyphenyl)-2-(3-thienyl)-1H-pyrrolo[3,2-c]pyridin-4-amine |
| 252 | N-(2,3-dihydro-1,4-benzodioxin-6-yl)-2-(3-thienyl)-1H-pyrrolo[3,2-c]pyridin-4-amine |
| 253 | N-(3,4-dimethoxyphenyl)-2-(3-thienyl)-1H-pyrrolo[3,2-c]pyridin-4-amine |
| 254 | N-(6-methoxy-3-pyridyl)-2-(3-thienyl)-1H-pyrrolo[3,2-c]pyridin-4-amine |
| 255 | N-(3-pyridyl)-2-(3-thienyl)-1H-pyrrolo[3,2-c]pyridin-4-amine |
| 256 | N-(4-morpholinophenyl)-2-(3-thienyl)-1H-pyrrolo[3,2-c]pyridin-4-amine |
| 257 | N-[4-(4-methylpiperazin-1-yl)phenyl]-2-(3-thienyl)-1H-pyrrolo[3,2-c]pyridin-4-amine |
| 258 | N-(4-pyridyl)-2-(3-thienyl)-1H-pyrrolo[3,2-c]pyridin-4-amine |
| 259 | N-(4-methoxyphenyl)-2-(3-thienyl)-1H-pyrrolo[3,2-c]pyridin-4-amine |
| 260 | N-(2-pyridyl)-2-(3-thienyl)-1H-pyrrolo[3,2-c]pyridin-4-amine |
| 261 | methyl 4-[[2-(3-thienyl)-1H-pyrrolo[3,2-c]pyridin-4-yl]amino]benzoate |
| 262 | N-(3-methylsulfonylphenyl)-2-(3-thienyl)-1H-pyrrolo[3,2-c]pyridin-4-amine |
| 263 | N-(1,3-benzodioxol-5-yl)-2-(3-thienyl)-1H-pyrrolo[3,2-c]pyridin-4-amine |
| 264 | 3-[[2-(3-thienyl)-1H-pyrrolo[3,2-c]pyridin-4-yl]amino]phenol |
| 265 | N-[3-[[2-(3-thienyl)-1H-pyrrolo[3,2-c]pyridin-4-yl]amino]phenyl]methanesulfonamide |
| 266 | N-[3-[[2-(3-thienyl)-1H-pyrrolo[3,2-c]pyridin-4-yl]amino]phenyl]acetamide |
| 267 | 1-[3-[[2-(3-thienyl)-1H-pyrrolo[3,2-c]pyridin-4-yl]amino]phenyl]ethanone |
| 268 | N-(4-methylsulfonylphenyl)-2-(3-thienyl)-1H-pyrrolo[3,2-c]pyridin-4-amine |
| 269 | N-(4-isopropoxyphenyl)-2-(3-thienyl)-1H-pyrrolo[3,2-c]pyridin-4-amine |
| 270 | N-[4-[[1-methyl-2-(3-thienyl)pyrrolo[3,2-c]pyridin-4-yl]amino]phenyl]acetamide |
| 271 | 4-[[1-methyl-2-(3-thienyl)pyrrolo[3,2-c]pyridin-4-yl]amino]phenol |
| 272 | N-(2,4-dimethoxyphenyl)-1-methyl-2-(3-thienyl)pyrrolo[3,2-c]pyridin-4-amine |
| 273 | N-(2,3-dihydro-1,4-benzodioxin-6-yl)-1-methyl-2-(3-thienyl)pyrrolo[3,2-c]pyridin-4-amine |
| 274 | N-(3,4-dimethoxyphenyl)-1-methyl-2-(3-thienyl)pyrrolo[3,2-c]pyridin-4-amine |
| 275 | N-(6-methoxy-3-pyridyl)-1-methyl-2-(3-thienyl)pyrrolo[3,2-c]pyridin-4-amine |
| 276 | 1-methyl-N-(3-pyridyl)-2-(3-thienyl)pyrrolo[3,2-c]pyridin-4-amine |
| 277 | N1,N1-dimethyl-N3-[1-methyl-2-(3-thienyl)pyrrolo[3,2-c]pyridin-4-yl]benzene-1,3-diamine |
| 278 | 1-methyl-N-(4-pyridyl)-2-(3-thienyl)pyrrolo[3,2-c]pyridin-4-amine |
| 279 | N-(4-methoxyphenyl)-1-methyl-2-(3-thienyl)pyrrolo[3,2-c]pyridin-4-amine |
| 280 | 1-methyl-N-(2-pyridyl)-2-(3-thienyl)pyrrolo[3,2-c]pyridin-4-amine |
| 281 | 4-[[1-methyl-2-(3-thienyl)pyrrolo[3,2-c]pyridin-4-yl]amino]benzonitrile |
| 282 | 1-methyl-N-(3-methylsulfonylphenyl)-2-(3-thienyl)pyrrolo[3,2-c]pyridin-4-amine |
| 283 | N-(1,3-benzodioxol-5-yl)-1-methyl-2-(3-thienyl)pyrrolo[3,2-c]pyridin-4-amine |
| 284 | 2-methoxy-5-[[1-methyl-2-(3-thienyl)pyrrolo[3,2-c]pyridin-4-yl]amino]phenol |
| 285 | 1-methyl-N-pyrimidin-4-yl-2-(3-thienyl)pyrrolo[3,2-c]pyridin-4-amine |
| 286 | N-[3-[[1-methyl-2-(3-thienyl)pyrrolo[3,2-c]pyridin-4-yl]amino]phenyl]methanesulfonamide |

(continued)

| compound | name |
|---|---|
| 287 | N-[3-[[1-methyl-2-(3-thienyl)pyrrolo[3,2-c]pyridin-4-yl]amino]phenyl]acetamide |
| 288 | 1-[3-[[1-methyl-2-(3-thienyl)pyrrolo[3,2-c]pyridin-4-yl]amino]phenyl]ethanone |
| 289 | 1-methyl-N-(4-methylsulfonylphenyl)-2-(3-thienyl)pyrrolo[3,2-c]pyridin-4-amine |
| 290 | 4-[[2-(3,5-dimethoxyphenyl)-1H-pyrrolo[3,2-c]pyridin-4-yl]amino]benzamide |
| 291 | 2-(3,5-dimethoxyphenyl)-N-(4-pyridyl)-1H-pyrrolo[3,2-c]pyridin-4-amine |
| 292 | N-[3-[[2-(3,5-dimethoxyphenyl)-1H-pyrrolo[3,2-c]pyridin-4-yl]amino]phenyl]methanesulfonamide |
| 293 | 2-(3,5-dimethoxyphenyl)-N-(4-methylsulfonylphenyl)-1H-pyrrolo[3,2-c]pyridin-4-amine |
| 294 | 4-[[2-(2-methoxyphenyl)-1H-pyrrolo[3,2-c]pyridin-4-yl]amino]benzamide |
| 295 | 2-(2-methoxyphenyl)-N-(4-pyridyl)-1H-pyrrolo[3,2-c]pyridin-4-amine |
| 296 | N-(1,3-benzodioxol-5-yl)-2-(2-methoxyphenyl)-1H-pyrrolo[3,2-c]pyridin-4-amine |
| 297 | 3-[[2-(2-methoxyphenyl)-1H-pyrrolo[3,2-c]pyridin-4-yl]amino]phenol |
| 298 | N-[3-[[2-(2-methoxyphenyl)-1H-pyrrolo[3,2-c]pyridin-4-yl]amino]phenyl]methanesulfonamide |
| 299 | N-[3-[[2-(2-methoxyphenyl)-1H-pyrrolo[3,2-c]pyridin-4-yl]amino]phenyl]acetamide |
| 300 | 2-(2-methoxyphenyl)-N-(4-methylsulfonylphenyl)-1H-pyrrolo[3,2-c]pyridin-4-amine |
| 301 | N-[4-[[2-(2-methoxyphenyl)-1-methyl-pyrrolo[3,2-c]pyridin-4-yl]amino]phenyl]acetamide |
| 302 | N-(2,4-dimethoxyphenyl)-2-(2-methoxyphenyl)-1-methyl-pyrrolo[3,2-c]pyridin-4-amine |
| 303 | 2-(2-methoxyphenyl)-1-methyl-N-(3-pyridyl)pyrrolo[3,2-c]pyridin-4-amine |
| 304 | 2-(2-methoxyphenyl)-1-methyl-N-(2-pyridyl)pyrrolo[3,2-c]pyridin-4-amine |
| 305 | 2-(2-methoxyphenyl)-1-methyl-N-(4-methylsulfanylphenyl)pyrrolo[3,2-c]pyridin-4-amine |
| 306 | N-[4-[[2-(3-methoxyphenyl)-1H-pyrrolo[3,2-c]pyridin-4-yl]amino]phenyl]acetamide |
| 307 | 2-(3-methoxyphenyl)-N-(6-methoxy-3-pyridyl)-1H-pyrrolo[3,2-c]pyridin-4-amine |
| 308 | 2-(3-methoxyphenyl)-N-(3-pyridyl)-1H-pyrrolo[3,2-c]pyridin-4-amine |
| 309 | 2-(3-methoxyphenyl)-N-phenyl-1H-pyrrolo[3,2-c]pyridin-4-amine |
| 310 | 2-(3-methoxyphenyl)-N-[4-(4-methylpiperazin-1-yl)phenyl]-1H-pyrrolo[3,2-c]pyridin-4-amine |
| 311 | 2-(3-methoxyphenyl)-N-(3-methylsulfonylphenyl)-1H-pyrrolo[3,2-c]pyridin-4-amine |
| 312 | 2-methoxy-5-[[2-(3-methoxyphenyl)-1H-pyrrolo[3,2-c]pyridin-4-yl]amino]phenol |
| 313 | 4-[[2-(3-methoxyphenyl)-1H-pyrrolo[3,2-c]pyridin-4-yl]amino]-N,N-dimethyl-benzamide |
| 314 | N-(3-isopropoxyphenyl)-2-(3-methoxyphenyl)-1-methyl-pyrrolo[3,2-c]pyridin-4-amine |
| 315 | 2-(3-chlorophenyl)-N-(3-pyridyl)-1H-pyrrolo[3,2-c]pyridin-4-amine |
| 316 | 2-(3-chlorophenyl)-1-methyl-N-pyrimidin-4-yl-pyrrolo[3,2-c]pyridin-4-amine |
| 317 | 4-[(2-phenyl-1H-pyrrolo[3,2-c]pyridin-4-yl)amino]phenol |
| 318 | 4-[(2-phenyl-1H-pyrrolo[3,2-c]pyridin-4-yl)amino]benzamide |
| 319 | 2-phenyl-N-(3-pyridyl)-1H-pyrrolo[3,2-c]pyridin-4-amine |
| 320 | N-[4-(4-methylpiperazin-1-yl)phenyl]-2-phenyl-1H-pyrrolo[3,2-c]pyridin-4-amine |
| 321 | 2-phenyl-N-(2-pyridyl)-1H-pyrrolo[3,2-c]pyridin-4-amine |
| 322 | N,N-dimethyl-4-[(2-phenyl-1 H-pyrrolo[3,2-c]pyridin-4-yl)amino]benzamide |
| 323 | N-[3-[(2-phenyl-1 H-pyrrolo[3,2-c]pyridin-4-yl)amino]phenyl]acetamide |
| 324 | N-(4-methylsulfonylphenyl)-2-phenyl-1H-pyrrolo[3,2-c]pyridin-4-amine |

(continued)

| compound | name |
|---|---|
| 325 | 4-[(1-methyl-2-phenyl-pyrrolo[3,2-c]pyridin-4-yl)amino]benzamide |
| 326 | N-(3,4-dimethoxyphenyl)-1-methyl-2-phenyl-pyrrolo[3,2-c]pyridin-4-amine |
| 327 | 1-methyl-2-phenyl-N-(2-pyridyl)pyrrolo[3,2-c]pyridin-4-amine |
| 328 | 3-[(1-methyl-2-phenyl-pyrrolo[3,2-c]pyridin-4-yl)amino]phenol |
| 329 | 2-phenyl-N-(3,4,5-trimethoxyphenyl)-1H-imidazo[4,5-c]pyridin-4-amine |
| 330 | N-(3,4-dimethoxyphenyl)-2-phenyl-1H-imidazo[4,5-c]pyridin-4-amine |
| 331 | N1,N1-dimethyl-N3-(2-phenyl-1H-imidazo[4,5-c]pyridin-4-yl)benzene-1,3-diamine |
| 332 | 2-phenyl-N-[2-(2-pyridyl)ethyl]-1H-imidazo[4,5-c]pyridin-4-amine |
| 333 | N-(4-methoxyphenyl)-2-phenyl-1H-imidazo[4,5-c]pyridin-4-amine |
| 334 | 4-(4-methylpiperazin-1-yl)-2-phenyl-1H-imidazo[4,5-c]pyridine |
| 335 | 2-phenyl-N-(4-pyridylmethyl)-1H-imidazo[4,5-c]pyridin-4-amine |
| 336 | N-(3-methylsulfonylphenyl)-2-phenyl-1H-imidazo[4,5-c]pyridin-4-amine |
| 337 | 3-[(2-phenyl-1H-imidazo[4,5-c]pyridin-4-yl)amino]phenol |
| 338 | 4-(2-phenyl-1H-imidazo[4,5-c]pyridin-4-yl)morpholine |
| 339 | N-(cyclopropylmethyl)-2-phenyl-1H-imidazo[4,5-c]pyridin-4-amine |
| 340 | N-(3-methoxypropyl)-2-phenyl-1H-imidazo[4,5-c]pyridin-4-amine |
| 341 | 2-(3,4-dimethoxyphenyl)-N-(3-methoxyphenyl)-1H-imidazo[4,5-c]pyridin-4-amine |
| 342 | 2-(3,4-dimethoxyphenyl)-N-phenyl-1H-imidazo[4,5-c]pyridin-4-amine |
| 343 | N1-[2-(3,4-dimethoxyphenyl)-1H-imidazo[4,5-c]pyridin-4-yl]-N3,N3-dimethyl-benzene-1,3-diamine |
| 344 | 2-(3,4-dimethoxyphenyl)-N-isobutyl-1H-imidazo[4,5-c]pyridin-4-amine |
| 345 | 2-(3,4-dimethoxyphenyl)-N-(4-methoxyphenyl)-1H-imidazo[4,5-c]pyridin-4-amine |
| 346 | 2-(3,4-dimethoxyphenyl)-4-(4-methylpiperazin-1-yl)-1H-imidazo[4,5-c]pyridine |
| 347 | N-benzyl-2-(3,4-dimethoxyphenyl)-1H-imidazo[4,5-c]pyridin-4-amine |
| 348 | 4-[[2-(3,4-dimethoxyphenyl)-1H-imidazo[4,5-c]pyridin-4-yl]amino]cyclohexanol |
| 349 | 2-(3,4-dimethoxyphenyl)-N-methyl-N-phenyl-1H-imidazo[4,5-c]pyridin-4-amine |
| 350 | 2-(3,4-dimethoxyphenyl)-N-[(4-dimethylaminophenyl)methyl]-1H-imidazo[4,5-c]pyridin-4-amine |
| 351 | 2-(3,4-dimethoxyphenyl)-N-(3-methylsulfonylphenyl)-1H-imidazo[4,5-c]pyridin-4-amine |
| 352 | 3-[[2-(3,4-dimethoxyphenyl)-1H-imidazo[4,5-c]pyridin-4-yl]amino]phenol |
| 353 | 4-[2-(3,4-dimethoxyphenyl)-1H-imidazo[4,5-c]pyridin-4-yl]morpholine |
| 354 | N-(cyclopropylmethyl)-2-(3,4-dimethoxyphenyl)-1H-imidazo[4,5-c]pyridin-4-amine |
| 355 | N-(2-methoxyethyl)-2-[3-(trifluoromethyl)phenyl]-1H-imidazo[4,5-c]pyridin-4-amine |
| 356 | N-(4-methoxyphenyl)-2-[3-(trifluoromethyl)phenyl]-1H-imidazo[4,5-c]pyridin-4-amine |
| 357 | N-(2-morpholinoethyl)-2-[3-(trifluoromethyl)phenyl]-1H-imidazo[4,5-c]pyridin-4-amine |
| 358 | N-(3-methoxyphenyl)-2-(3-pyridyl)-1H-imidazo[4,5-c]pyridin-4-amine |
| 359 | N-(2-diethylaminoethyl)-4-[[2-(3-pyridyl)-1H-imidazo[4,5-c]pyridin-4-yl]amino]benzamide |
| 360 | N-benzyl-2-(3-pyridyl)-1H-imidazo[4,5-c]pyridin-4-amine |
| 361 | N-[[3-(dimethylamino)phenyl]methyl]-2-(3-pyridyl)-1H-imidazo[4,5-c]pyridin-4-amine |
| 362 | 3-[4-(4-methoxyanilino)-1H-imidazo[4,5-c]pyridin-2-yl]phenol |

(continued)

| compound | name |
|---|---|
| 363 | 3-(4-morpholino-1H-imidazo[4,5-c]pyridin-2-yl)phenol |
| 364 | 4-[4-(3,4,5-trimethoxyanilino)-1H-imidazo[4,5-c]pyridin-2-yl]phenol |
| 365 | 4-(4-anilino-1H-imidazo[4,5-c]pyridin-2-yl)phenol |
| 366 | N-[3-[[2-(4-hydroxyphenyl)-1H-imidazo[4,5-c]pyridin-4-yl]amino]phenyl]methanesulfonamide |
| 367 | 2-(3-methoxyphenyl)-N-(3,4,5-trimethoxyphenyl)-1H-imidazo[4,5-c]pyridin-4-amine |
| 368 | N,2-bis(3-methoxyphenyl)-1H-imidazo[4,5-c]pyridin-4-amine |
| 369 | N-(3,4-dimethoxyphenyl)-2-(3-methoxyphenyl)-1H-imidazo[4,5-c]pyridin-4-amine |
| 370 | 2-(3-methoxyphenyl)-N-phenyl-1H-imidazo[4,5-c]pyridin-4-amine |
| 371 | 2-(3-methoxyphenyl)-N-[2-(2-pyridyl)ethyl]-1H-imidazo[4,5-c]pyridin-4-amine |
| 372 | 2-(3-methoxyphenyl)-N-(4-methoxyphenyl)-1H-imidazo[4,5-c]pyridin-4-amine |
| 373 | 2-(3-methoxyphenyl)-N-[3-(trifluoromethyl)phenyl]-1H-imidazo[4,5-c]pyridin-4-amine |
| 374 | 3-[[2-(3-methoxyphenyl)-1H-imidazo[4,5-c]pyridin-4-yl]amino]phenol |
| 375 | 4-[2-(3-methoxyphenyl)-1H-imidazo[4,5-c]pyridin-4-yl]morpholine |
| 376 | N-(cyclopropylmethyl)-2-(3-methoxyphenyl)-1H-imidazo[4,5-c]pyridin-4-amine |
| 377 | 2-(p-tolyl)-N-(3,4,5-trimethoxyphenyl)-1H-imidazo[4,5-c]pyridin-4-amine |
| 378 | N-(3,4-dimethoxyphenyl)-2-(p-tolyl)-1H-imidazo[4,5-c]pyridin-4-amine |
| 379 | N-(6-methoxy-3-pyridyl)-2-(p-tolyl)-1H-imidazo[4,5-c]pyridin-4-amine |
| 380 | N-phenyl-2-(p-tolyl)-1H-imidazo[4,5-c]pyridin-4-amine |
| 381 | N-(2-diethylaminoethyl)-4-[[2-(p-tolyl)-1H-imidazo[4,5-c]pyridin-4-yl]amino]benzamide |
| 382 | N-(4-methoxyphenyl)-2-(p-tolyl)-1H-imidazo[4,5-c]pyridin-4-amine |
| 383 | 3-[[2-(p-tolyl)-1H-imidazo[4,5-c]pyridin-4-yl]amino]phenol |
| 384 | N-[3-[[2-(p-tolyl)-1H-imidazo[4,5-c]pyridin-4-yl]amino]phenyl]methanesulfonamide |
| 385 | N-(2-methoxyethyl)-3-[4-(3,4,5-trimethoxyanilino)-1H-imidazo[4,5-c]pyridin-2-yl]benzamide |
| 386 | 3-[4-(3-methoxyanilino)-1H-imidazo[4,5-c]pyridin-2-yl]-N-(2-methoxyethyl)benzamide |
| 387 | 3-(4-anilino-1H-imidazo[4,5-c]pyridin-2-yl)-N-(2-methoxyethyl)benzamide |
| 388 | 2-(3-isopropylphenyl)-4-(4-methylpiperazin-1-yl)-1H-imidazo[4,5-c]pyridine |
| 389 | 2-(4-methoxyphenyl)-N-(3,4,5-trimethoxyphenyl)-1H-imidazo[4,5-c]pyridin-4-amine |
| 390 | N-(3-methoxyphenyl)-2-(4-methoxyphenyl)-1H-imidazo[4,5-c]pyridin-4-amine |
| 391 | N-(2-methoxyethyl)-2-(4-methoxyphenyl)-1H-imidazo[4,5-c]pyridin-4-amine |
| 392 | N-(3,4-dimethoxyphenyl)-2-(4-methoxyphenyl)-1H-imidazo[4,5-c]pyridin-4-amine |
| 393 | 2-(4-methoxyphenyl)-N-phenyl-1H-imidazo[4,5-c]pyridin-4-amine |
| 394 | 2-(4-methoxyphenyl)-N-[2-(2-pyridyl)ethyl]-1H-imidazo[4,5-c]pyridin-4-amine |
| 395 | N,2-bis(4-methoxyphenyl)-1H-imidazo[4,5-c]pyridin-4-amine |
| 396 | 2-(4-methoxyphenyl)-4-(4-methylpiperazin-1-yl)-1H-imidazo[4,5-c]pyridine |
| 397 | 3-[[2-(4-methoxyphenyl)-1H-imidazo[4,5-c]pyridin-4-yl]amino]phenol |
| 398 | 2-methoxy-5-[[2-(4-methoxyphenyl)-1H-imidazo[4,5-c]pyridin-4-yl]amino]phenol |
| 399 | 4-[2-(4-methoxyphenyl)-1H-imidazo[4,5-c]pyridin-4-yl]morpholine |
| 400 | N-(cyclopropylmethyl)-2-(4-methoxyphenyl)-1H-imidazo[4,5-c]pyridin-4-amine |

(continued)

| compound | name |
|---|---|
| 401 | 2-(4-methoxyphenyl)-N-(3-methoxypropyl)-1H-imidazo[4,5-c]pyridin-4-amine |
| 402 | 2-(3-fluorophenyl)-N-(3,4,5-trimethoxyphenyl)-1H-imidazo[4,5-c]pyridin-4-amine |
| 403 | 4-[[2-(3-fluorophenyl)-1H-imidazo[4,5-c]pyridin-4-yl]amino]benzamide |
| 404 | N-(3,4-dimethoxyphenyl)-2-(3-fluorophenyl)-1H-imidazo[4,5-c]pyridin-4-amine |
| 405 | 2-(3-fluorophenyl)-N-(6-methoxy-3-pyridyl)-1H-imidazo[4,5-c]pyridin-4-amine |
| 406 | 2-(3-fluorophenyl)-N-[2-(2-pyridyl)ethyl]-1H-imidazo[4,5-c]pyridin-4-amine |
| 407 | 2-(3-fluorophenyl)-N-(4-methoxyphenyl)-1H-imidazo[4,5-c]pyridin-4-amine |
| 408 | 2-(3-fluorophenyl)-N-(3-phenoxyphenyl)-1H-imidazo[4,5-c]pyridin-4-amine |
| 409 | 2-(2,3-dihydro-1,4-benzodioxin-6-yl)-N-(3,4,5-trimethoxyphenyl)-1H-imidazo[4,5-c]pyridin-4-amine |
| 410 | 2-(2,3-dihydro-1,4-benzodioxin-6-yl)-N-(3-methoxyphenyl)-1H-imidazo[4,5-c]pyridin-4-amine |
| 411 | 2-(2,3-dihydro-1,4-benzodioxin-6-yl)-N-(3,4-dimethoxyphenyl)-1H-imidazo[4,5-c]pyridin-4-amine |
| 412 | 2-(2,3-dihydro-1,4-benzodioxin-6-yl)-N-phenyl-1H-imidazo[4,5-c]pyridin-4-amine |
| 413 | 2-(2,3-dihydro-1,4-benzodioxin-6-yl)-N-isobutyl-1H-imidazo[4,5-c]pyridin-4-amine |
| 414 | 2-(2,3-dihydro-1,4-benzodioxin-6-yl)-N-(4-methoxyphenyl)-1H-imidazo[4,5-c]pyridin-4-amine |
| 415 | 2-(2,3-dihydro-1,4-benzodioxin-6-yl)-4-(4-methylpiperazin-1-yl)-1H-imidazo[4,5-c]pyridine |
| 416 | 2-[[2-(2,3-dihydro-1,4-benzodioxin-6-yl)-1H-imidazo[4,5-c]pyridin-4-yl]amino]ethanol |
| 417 | 2-(2,3-dihydro-1,4-benzodioxin-6-yl)-N-(3-methylsulfonylphenyl)-1H-imidazo[4,5-c]pyridin-4-amine |
| 418 | 3-[[2-(2,3-dihydro-1,4-benzodioxin-6-yl)-1H-imidazo[4,5-c]pyridin-4-yl]amino]phenol |
| 419 | N-(cyclopropylmethyl)-2-(2,3-dihydro-1,4-benzodioxin-6-yl)-1H-imidazo[4,5-c]pyridin-4-amine |
| 420 | 2-(2,3-dihydro-1,4-benzodioxin-6-yl)-N-(3-methoxypropyl)-1H-imidazo[4,5-c]pyridin-4-amine |
| 421 | 2-(4-pyridyl)-N-(3,4,5-trimethoxyphenyl)-1H-imidazo[4,5-c]pyridin-4-amine |
| 422 | N-(3-methoxyphenyl)-2-(4-pyridyl)-1H-imidazo[4,5-c]pyridin-4-amine |
| 423 | N-(3,4-dimethoxyphenyl)-2-(4-pyridyl)-1H-imidazo[4,5-c]pyridin-4-amine |
| 424 | N-(3-chlorophenyl)-2-(4-pyridyl)-1H-imidazo[4,5-c]pyridin-4-amine |
| 425 | N-(2-diethylaminoethyl)-4-[[2-(4-pyridyl)-1H-imidazo[4,5-c]pyridin-4-yl]amino]benzamide |
| 426 | N1,N1-dimethyl-N3-[2-(4-pyridyl)-1H-imidazo[4,5-c]pyridin-4-yl]benzene-1,3-diamine |
| 427 | N-(4-methoxyphenyl)-2-(4-pyridyl)-1H-imidazo[4,5-c]pyridin-4-amine |
| 428 | N-benzyl-2-(4-pyridyl)-1H-imidazo[4,5-c]pyridin-4-amine |
| 429 | (2R)-3-methyl-2-[[2-(4-pyridyl)-1H-imidazo[4,5-c]pyridin-4-yl]amino]butan-1-ol |
| 430 | N-[(4-dimethylaminophenyl)methyl]-2-(4-pyridyl)-1H-imidazo[4,5-c]pyridin-4-amine |
| 431 | 3-[[2-(4-pyridyl)-1H-imidazo[4,5-c]pyridin-4-yl]amino]phenol |
| 432 | 2-methoxy-5-[[2-(4-pyridyl)-1H-imidazo[4,5-c]pyridin-4-yl]amino]phenol |
| 433 | N-[(4-chlorophenyl)methyl]-2-(4-pyridyl)-1H-imidazo[4,5-c]pyridin-4-amine |
| 434 | N-phenethyl-2-(4-pyridyl)-1H-imidazo[4,5-c]pyridin-4-amine |
| 435 | 2-(m-tolyl)-N-(3,4,5-trimethoxyphenyl)-1H-imidazo[4,5-c]pyridin-4-amine |
| 436 | N-(3,4-dimethoxyphenyl)-2-(m-tolyl)-1H-imidazo[4,5-c]pyridin-4-amine |
| 437 | 2-(m-tolyl)-N-[2-(2-pyridyl)ethyl]-1H-imidazo[4,5-c]pyridin-4-amine |
| 438 | 3-[[2-(m-tolyl)-1H-imidazo[4,5-c]pyridin-4-yl]amino]phenol |

(continued)

| compound | name |
|---|---|
| 439 | N-[3-[[2-(m-tolyl)-1H-imidazo[4,5-c]pyridin-4-yl]amino]phenyl]methanesulfonamide |
| 440 | 2-(3,4-difluorophenyl)-N-(3,4-dimethoxyphenyl)-1H-imidazo[4,5-c]pyridin-4-amine |
| 441 | 3-[[2-(3,4-difluorophenyl)-1H-imidazo[4,5-c]pyridin-4-yl]amino]phenol |
| 442 | 4-(4-methylpiperazin-1-yl)-2-[3-(trifluoromethoxy)phenyl]-1H-imidazo[4,5-c]pyridine |
| 443 | 4-[[2-[3-(trifluoromethoxy)phenyl]-1H-imidazo[4,5-c]pyridin-4-yl]amino]cyclohexanol |
| 444 | 2-methoxy-5-[[2-[3-(trifluoromethoxy)phenyl]-1H-imidazo[4,5-c]pyridin-4-yl]amino]phenol |
| 445 | N-(2-pyridylmethyl)-2-[3-(trifluoromethoxy)phenyl]-1H-imidazo[4,5-c]pyridin-4-amine |
| 446 | 4-[2-[3-(trifluoromethoxy)phenyl]-1H-imidazo[4,5-c]pyridin-4-yl]morpholine |
| 447 | N-(3,4-dimethoxyphenyl)-1-methyl-imidazo[4,5-c]pyridin-4-amine |
| 448 | N-(1H-indol-5-yl)-1-methyl-imidazo[4,5-c]pyridin-4-amine |
| 449 | 1-benzyl-N-cyclohexyl-imidazo[4,5-c]pyridin-4-amine |
| 450 | N1,N1-dimethyl-N3-(1-methylimidazo[4,5-c]pyridin-4-yl)benzene-1,3-diamine |
| 451 | 1-benzyl-N-(2-furylmethyl)imidazo[4,5-c]pyridin-4-amine |
| 452 | 1-benzyl-N-(2-thienylmethyl)imidazo[4,5-c]pyridin-4-amine |
| 453 | N-(4-methoxyphenyl)-1-methyl-imidazo[4,5-c]pyridin-4-amine |
| 454 | N-(1,3-benzodioxol-5-ylmethyl)-1-benzyl-imidazo[4,5-c]pyridin-4-amine |
| 455 | 1-benzyl-N-[(4-methoxyphenyl)methyl]imidazo[4,5-c]pyridin-4-amine |
| 456 | N-[(3,4-dimethoxyphenyl)methyl]-1-methyl-imidazo[4,5-c]pyridin-4-amine |
| 457 | 1-benzyl-N-(4-isopropylphenyl)imidazo[4,5-c]pyridin-4-amine |
| 458 | 2-(2-chlorophenyl)-N-(3,4-dimethoxyphenyl)-1H-imidazo[4,5-c]pyridin-4-amine |
| 459 | 2-(2-chlorophenyl)-N-[2-(4-pyridyl)ethyl]-1H-imidazo[4,5-c]pyridin-4-amine |
| 460 | 2-(2-chlorophenyl)-N-[(3,4,5-trimethoxyphenyl)methyl]-1H-imidazo[4,5-c]pyridin-4-amine |
| 461 | N-(3-chloro-4-fluoro-phenyl)-2-(2-chlorophenyl)-1H-imidazo[4,5-c]pyridin-4-amine |
| 462 | 2-(2-chlorophenyl)-N-[3-(trifluoromethyl)phenyl]-1H-imidazo[4,5-c]pyridin-4-amine |
| 463 | N-[3-[[2-(2-chlorophenyl)-1H-imidazo[4,5-c]pyridin-4-yl]amino]phenyl]methanesulfonamide |
| 464 | 2-(2-furyl)-N-(3-methoxyphenyl)-1H-imidazo[4,5-c]pyridin-4-amine |
| 465 | 2-(2-furyl)-N-(3-pyridyl)-1H-imidazo[4,5-c]pyridin-4-amine |
| 466 | 2-(2-furyl)-N-(4-methoxyphenyl)-1H-imidazo[4,5-c]pyridin-4-amine |
| 467 | 2-(2-fluoro-3-methoxy-phenyl)-N-(3,4,5-trimethoxyphenyl)-1H-imidazo[4,5-c]pyridin-4-amine |
| 468 | 2-(2-fluoro-3-methoxy-phenyl)-N-(3-methoxyphenyl)-1H-imidazo[4,5-c]pyridin-4-amine |
| 469 | N-(3,4-dimethoxyphenyl)-2-(2-fluoro-3-methoxy-phenyl)-1H-imidazo[4,5-c]pyridin-4-amine |
| 470 | 2-(2-fluoro-3-methoxy-phenyl)-N-(6-methoxy-3-pyridyl)-1H-imidazo[4,5-c]pyridin-4-amine |
| 471 | N-(4-chlorophenyl)-2-(2-fluoro-3-methoxy-phenyl)-1H-imidazo[4,5-c]pyridin-4-amine |
| 472 | 2-(2-fluoro-3-methoxy-phenyl)-N-phenyl-1H-imidazo[4,5-c]pyridin-4-amine |
| 473 | N-(2-diethylaminoethyl)-4-[[2-(2-fluoro-3-methoxy-phenyl)-1H-imidazo[4,5-c]pyridin-4-yl]amino]benzamide |
| 474 | N-(3-chloro-4-fluoro-phenyl)-2-(2-fluoro-3-methoxy-phenyl)-1H-imidazo[4,5-c]pyridin-4-amine |
| 475 | 2-(2-fluoro-3-methoxy-phenyl)-N-(4-methoxyphenyl)-1H-imidazo[4,5-c]pyridin-4-amine |

(continued)

| compound | name |
|---|---|
| 476 | N4-[2-(2-fluoro-3-methoxy-phenyl)-1H-imidazo[4,5-c]pyridin-4-yl]cyclohexane-1,4-diamine |
| 477 | 2-(2-fluoro-3-methoxy-phenyl)-N-(3-methylsulfonylphenyl)-1H-imidazo[4,5-c]pyridin-4-amine |
| 478 | 3-[[2-(2-fluoro-3-methoxy-phenyl)-1H-imidazo[4,5-c]pyridin-4-yl]amino]phenol |
| 479 | 5-[[2-(2-fluoro-3-methoxy-phenyl)-1H-imidazo[4,5-c]pyridin-4-yl]amino]-2-methoxy-phenol |
| 480 | 4-[2-(2-fluoro-3-methoxy-phenyl)-1H-imidazo[4,5-c]pyridin-4-yl]morpholine |
| 481 | 2-(2-fluoro-3-methoxy-phenyl)-N-(3-fluorophenyl)-1H-imidazo[4,5-c]pyridin-4-amine |
| 482 | N2-methyl-N6,1-bis(3,4,5-trimethoxyphenyl)imidazo[4,5-c]pyridine-2,6-diamine |
| 483 | N-[4-[[2-(methylamino)-1-(3,4,5-trimethoxyphenyl)imidazo[4,5-c]pyridin-6-yl]amino]phenyl]acetamide |
| 484 | N6-(3-methoxyphenyl)-N2-methyl-1-(3,4,5-trimethoxyphenyl)imidazo[4,5-c]pyridine-2,6-diamine |
| 485 | 4-[[2-(methylamino)-1-(3,4,5-trimethoxyphenyl)imidazo[4,5-c]pyridin-6-yl]amino]phenol |
| 486 | N6-(2,4-dimethoxyphenyl)-N2-methyl-1-(3,4,5-trimethoxyphenyl)imidazo[4,5-c]pyridine-2,6-diamine |
| 487 | N6-(2,3-dihydro-1,4-benzodioxin-6-yl)-N2-methyl-1-(3,4,5-trimethoxyphenyl)imidazo[4,5-c]pyridine-2,6-diamine |
| 488 | N6-(6-methoxy-3-pyridyl)-N2-methyl-1-(3,4,5-trimethoxyphenyl)imidazo[4,5-c]pyridine-2,6-diamine |
| 489 | N2-methyl-N6-[4-(4-methylpiperazin-1-yl)phenyl]-1-(3,4,5-trimethoxyphenyl)imidazo[4,5-c]pyridine-2,6-diamine |
| 490 | N6-(4-methoxyphenyl)-N2-methyl-1-(3,4,5-trimethoxyphenyl)imidazo[4,5-c]pyridine-2,6-diamine |
| 491 | 3-[[2-(methylamino)-1-(3,4,5-trimethoxyphenyl)imidazo[4,5-c]pyridin-6-yl]amino]phenol |
| 492 | N-[3-[[2-(methylamino)-1-(3,4,5-trimethoxyphenyl)imidazo[4,5-c]pyridin-6-yl]amino]phenyl]methanesulfonamide |
| 493 | N-[3-[[2-(methylamino)-1-(3,4,5-trimethoxyphenyl)imidazo[4,5-c]pyridin-6-yl]amino]phenyl]acetamide |
| 494 | N6-(3-fluorophenyl)-N2-methyl-1-(3,4,5-trimethoxyphenyl)imidazo[4,5-c]pyridine-2,6-diamine |
| 495 | N6-(3,4-dimethoxyphenyl)-1-(2-methoxyethyl)-N2-methyl-imidazo[4,5-c]pyridine-2,6-diamine |
| 496 | 1-(2-methoxyethyl)-N6-(4-methoxyphenyl)-N2-methyl-imidazo[4,5-c]pyridine-2,6-diamine |
| 497 | N6-(1,3-benzodioxol-5-yl)-1-(2-methoxyethyl)-N2-methyl-imidazo[4,5-c]pyridine-2,6-diamine |
| 498 | N6-(4-isopropylphenyl)-1-(2-methoxyethyl)-N2-methyl-imidazo[4,5-c]pyridine-2,6-diamine |
| 499 | 1-(2-methoxyethyl)-N2-methyl-N6-(4-methylsulfanylphenyl)imidazo[4,5-c]pyridine-2,6-diamine |
| 500 | N6-(4-isopropoxyphenyl)-1-(2-methoxyethyl)-N2-methyl-imidazo[4,5-c]pyridine-2,6-diamine |
| 501 | 6-(4-dimethylaminophenyl)-N-methyl-1-phenyl-imidazo[4,5-c]pyridin-2-amine |
| 502 | N2-methyl-1-phenyl-N6-(3,4,5-trimethoxyphenyl)imidazo[4,5-c]pyridine-2,6-diamine |
| 503 | N6-(3-methoxyphenyl)-N2-methyl-1-phenyl-imidazo[4,5-c]pyridine-2,6-diamine |
| 504 | 4-[[2-(methylamino)-1-phenyl-imidazo[4,5-c]pyridin-6-yl]amino]benzamide |
| 505 | N6-(2,3-dihydro-1,4-benzodioxin-6-yl)-N2-methyl-1-phenyl-imidazo[4,5-c]pyridine-2,6-diamine |
| 506 | N6-(3,4-dimethoxyphenyl)-N2-methyl-1-phenyl-imidazo[4,5-c]pyridine-2,6-diamine |
| 507 | N2-methyl-1-phenyl-N6-(3-pyridyl)imidazo[4,5-c]pyridine-2,6-diamine |
| 508 | N6-[3-(dimethylamino)phenyl]-N2-methyl-1-phenyl-imidazo[4,5-c]pyridine-2,6-diamine |
| 509 | N6-(4-methoxyphenyl)-N2-methyl-1-phenyl-imidazo[4,5-c]pyridine-2,6-diamine |
| 510 | [4-[2-(methylamino)-1-phenyl-imidazo[4,5-c]pyridin-6-yl]phenyl]methanol |
| 511 | methyl 4-[[2-(methylamino)-1-phenyl-imidazo[4,5-c]pyridin-6-yl]amino]benzoate |

(continued)

| compound | name |
|---|---|
| 512 | N6-(1,3-benzodioxol-5-yl)-N2-methyl-1-phenyl-imidazo[4,5-c]pyridine-2,6-diamine |
| 513 | 3-[[2-(methylamino)-1-phenyl-imidazo[4,5-c]pyridin-6-yl]amino]phenol |
| 514 | N-methyl-1-phenyl-6-(2-thienyl)imidazo[4,5-c]pyridin-2-amine |
| 515 | N,N-dimethyl-4-[[2-(methylamino)-1-phenyl-imidazo[4,5-c]pyridin-6-yl]amino]benzamide |
| 516 | N-[3-[[2-(methylamino)-1-phenyl-imidazo[4,5-c]pyridin-6-yl]amino]phenyl]acetamide |
| 517 | 1-[3-[[2-(methylamino)-1-phenyl-imidazo[4,5-c]pyridin-6-yl]amino]phenyl]ethanone |
| 518 | N2-methyl-N6-(4-methylsulfonylphenyl)-1-phenyl-imidazo[4,5-c]pyridine-2,6-diamine |
| 519 | 1-(2-furylmethyl)-N2-methyl-N6-(3,4,5-trimethoxyphenyl)imidazo[4,5-c]pyridine-2,6-diamine |
| 520 | N6-(3,4-dimethoxyphenyl)-1-(2-furylmethyl)-N2-methyl-imidazo[4,5-c]pyridine-2,6-diamine |
| 521 | 1-(2-furylmethyl)-N2-methyl-N6-(4-morpholinophenyl)imidazo[4,5-c]pyridine-2,6-diamine |
| 522 | N6-[3-(dimethylamino)phenyl]-1-(2-furylmethyl)-N2-methyl-imidazo[4,5-c]pyridine-2,6-diamine |
| 523 | 1-(2-furylmethyl)-N6-(4-methoxyphenyl)-N2-methyl-imidazo[4,5-c]pyridine-2,6-diamine |
| 524 | N6-(1,3-benzodioxol-5-yl)-1-(2-furylmethyl)-N2-methyl-imidazo[4,5-c]pyridine-2,6-diamine |
| 525 | 1-benzyl-6-(4-dimethylaminophenyl)-N-methyl-imidazo[4,5-c]pyridin-2-amine |
| 526 | 1-benzyl-N2-methyl-N6-(3,4,5-trimethoxyphenyl)imidazo[4,5-c]pyridine-2,6-diamine |
| 527 | N-[4-[[1-benzyl-2-(methylamino)imidazo[4,5-c]pyridin-6-yl]amino]phenyl]acetamide |
| 528 | 1-benzyl-N6-(2,3-dihydro-1,4-benzodioxin-6-yl)-N2-methyl-imidazo[4,5-c]pyridine-2,6-diamine |
| 529 | 1-benzyl-N-methyl-6-(2-phenoxyphenyl)imidazo[4,5-c]pyridin-2-amine |
| 530 | 1-benzyl-N2-methyl-N6-(m-tolyl)imidazo[4,5-c]pyridine-2,6-diamine |
| 531 | 1-benzyl-N6-(4-methoxyphenyl)-N2-methyl-imidazo[4,5-c]pyridine-2,6-diamine |
| 532 | 1-benzyl-6-(3-chlorophenyl)-N-methyl-imidazo[4,5-c]pyridin-2-amine |
| 533 | 3-[1-benzyl-2-(methylamino)imidazo[4,5-c]pyridin-6-yl]phenol |
| 534 | N6-(1,3-benzodioxol-5-yl)-1-benzyl-N2-methyl-imidazo[4,5-c]pyridine-2,6-diamine |
| 535 | 3-[[1-benzyl-2-(methylamino)imidazo[4,5-c]pyridin-6-yl]amino]phenol |
| 536 | 5-[[1-benzyl-2-(methylamino)imidazo[4,5-c]pyridin-6-yl]amino]-2-methoxy-phenol |
| 537 | 4-[[1-benzyl-2-(methylamino)imidazo[4,5-c]pyridin-6-yl]amino]-N,N-dimethyl-benzamide |
| 538 | N-[3-[[1-benzyl-2-(methylamino)imidazo[4,5-c]pyridin-6-yl]amino]phenyl]acetamide |
| 539 | N2-methyl-1-[3-(trifluoromethyl)phenyl]-N6-(3,4,5-trimethoxyphenyl)imidazo[4,5-c]pyridine-2,6-diamine |
| 540 | N-[4-[[2-(methylamino)-1-[3-(trifluoromethyl)phenyl]imidazo[4,5-c]pyridin-6-yl]amino]phenyl]acetamide |
| 541 | 4-[[2-(methylamino)-1-[3-(trifluoromethyl)phenyl]imidazo[4,5-c]pyridin-6-yl]amino]phenol |
| 542 | 4-[[2-(methylamino)-1-[3-(trifluoromethyl)phenyl]imidazo[4,5-c]pyridin-6-yl]amino]benzamide |
| 543 | N6-(2,4-dimethoxyphenyl)-N2-methyl-1-[3-(trifluoromethyl)phenyl]imidazo[4,5-c]pyridine-2,6-diamine |
| 544 | N6-(2,3-dihydro-1,4-benzodioxin-6-yl)-N2-methyl-1-[3-(trifluoromethyl)phenyl]imidazo[4,5-c]pyridine-2,6-diamine |
| 545 | N6-(3,4-dimethoxyphenyl)-N2-methyl-1-[3-(trifluoromethyl)phenyl]imidazo[4,5-c]pyridine-2,6-diamine |
| 546 | N6-(6-methoxy-3-pyridyl)-N2-methyl-1-[3-(trifluoromethyl)phenyl]imidazo[4,5-c]pyridine-2,6-diamine |
| 547 | N6-(4-methoxyphenyl)-N2-methyl-1-[3-(trifluoromethyl)phenyl]imidazo[4,5-c]pyridine-2,6-diamine |

(continued)

| compound | name |
|---|---|
| 548 | N2-methyl-N6-(2-pyridyl)-1-[3-(trifluoromethyl)phenyl]imidazo[4,5-c]pyridine-2,6-diamine |
| 549 | N6-(1,3-benzodioxol-5-yl)-N2-methyl-1-[3-(trifluoromethyl)phenyl]imidazo[4,5-c]pyridine-2,6-diamine |
| 550 | 3-[[2-(methylamino)-1-[3-(trifluoromethyl)phenyl]imidazo[4,5-c]pyridin-6-yl]amino]phenol |
| 551 | 2-methoxy-5-[[2-(methylamino)-1-[3-(trifluoromethyl)phenyl]imidazo[4,5-c]pyridin-6-yl]amino]phenol |
| 552 | N6-(3-methoxyphenyl)-N2-methyl-1-(2-morpholinoethyl)imidazo[4,5-c]pyridine-2,6-diamine |
| 553 | N6-(2,4-dimethoxyphenyl)-N2-methyl-1-(2-morpholinoethyl)imidazo[4,5-c]pyridine-2,6-diamine |
| 554 | N6-(2,3-dihydro-1,4-benzodioxin-6-yl)-N2-methyl-1-(2-morpholinoethyl)imidazo[4,5-c]pyridine-2,6-diamine |
| 555 | N6-(3,4-dimethoxyphenyl)-N2-methyl-1-(2-morpholinoethyl)imidazo[4,5-c]pyridine-2,6-diamine |
| 556 | N2-methyl-1-(2-morpholinoethyl)-N6-(m-tolyl)imidazo[4,5-c]pyridine-2,6-diamine |
| 557 | N6-(4-methoxyphenyl)-N2-methyl-1-(2-morpholinoethyl)imidazo[4,5-c]pyridine-2,6-diamine |
| 558 | 1-[3-[[2-(methylamino)-1-(2-morpholinoethyl)imidazo[4,5-c]pyridin-6-yl]amino]phenyl]ethanone |
| 559 | N2-methyl-1-(3-pyridylmethyl)-N6-(3,4,5-trimethoxyphenyl)imidazo[4,5-c]pyridine-2,6-diamine |
| 560 | N6-(3-methoxyphenyl)-N2-methyl-1-(3-pyridylmethyl)imidazo[4,5-c]pyridine-2,6-diamine |
| 561 | N6-(2,3-dihydro-1,4-benzodioxin-6-yl)-N2-methyl-1-(3-pyridylmethyl)imidazo[4,5-c]pyridine-2,6-diamine |
| 562 | N2-methyl-N6-phenyl-1-(3-pyridylmethyl)imidazo[4,5-c]pyridine-2,6-diamine |
| 563 | N-methyl-6-(3-pyridyl)-1-(3-pyridylmethyl)imidazo[4,5-c]pyridin-2-amine |
| 564 | N2-methyl-N6-(m-tolyl)-1-(3-pyridylmethyl)imidazo[4,5-c]pyridine-2,6-diamine |
| 565 | N6-(4-fluorophenyl)-N2-methyl-1-(3-pyridylmethyl)imidazo[4,5-c]pyridine-2,6-diamine |
| 566 | N-methyl-1-(3-pyridylmethyl)-6-(2-thienyl)imidazo[4,5-c]pyridin-2-amine |
| 567 | 1-(cyclopropylmethyl)-N2-methyl-N6-(3,4,5-trimethoxyphenyl)imidazo[4,5-c]pyridine-2,6-diamine |
| 568 | N-[4-[[1-(cyclopropylmethyl)-2-(methylamino)imidazo[4,5-c]pyridin-6-yl]amino]phenyl]acetamide |
| 569 | 1-(cyclopropylmethyl)-N6-(2,4-dimethoxyphenyl)-N2-methyl-imidazo[4,5-c]pyridine-2,6-diamine |
| 570 | 1-(cyclopropylmethyl)-N6-(2,3-dihydro-1,4-benzodioxin-6-yl)-N2-methyl-imidazo[4,5-c]pyridine-2,6-diamine |
| 571 | 1-(cyclopropylmethyl)-N2-methyl-N6-(4-morpholinophenyl)imidazo[4,5-c]pyridine-2,6-diamine |
| 572 | 1-(cyclopropylmethyl)-N2-methyl-N6-[4-(4-methylpiperazin-1-yl)phenyl]imidazo[4,5-c]pyridine-2,6-diamine |
| 573 | 1-(cyclopropylmethyl)-N6-[3-(dimethylamino)phenyl]-N2-methyl-imidazo[4,5-c]pyridine-2,6-diamine |
| 574 | N-[3-[[1-(cyclopropylmethyl)-2-(methylamino)imidazo[4,5-c]pyridin-6-yl]amino]phenyl]acetamide |
| 575 | 6-(4-dimethylaminophenyl)-1-methyl-N-(m-tolylmethyl)imidazo[4,5-c]pyridin-2-amine |
| 576 | N-[4-[[1-methyl-2-(m-tolylmethylamino)imidazo[4,5-c]pyridin-6-yl]amino]phenyl]acetamide |
| 577 | N6-(2,3-dihydro-1,4-benzodioxin-6-yl)-1-methyl-N2-(m-tolylmethyl)imidazo[4,5-c]pyridine-2,6-diamine |
| 578 | N6-(3,4-dimethoxyphenyl)-1-methyl-N2-(m-tolylmethyl)imidazo[4,5-c]pyridine-2,6-diamine |
| 579 | 6-(3,4-dimethoxyphenyl)-1-methyl-N-(m-tolylmethyl)imidazo[4,5-c]pyridin-2-amine |
| 580 | N-[3-[1-methyl-2-(m-tolylmethylamino)imidazo[4,5-c]pyridin-6-yl]phenyl]acetamide |
| 581 | 6-(4-chlorophenyl)-1-methyl-N-(m-tolylmethyl)imidazo[4,5-c]pyridin-2-amine |

(continued)

| compound | name |
|---|---|
| 582 | N6-(4-fluorophenyl)-1-methyl-N2-(m-tolylmethyl)imidazo[4,5-c]pyridine-2,6-diamine |
| 583 | 6-(3-furyl)-1-methyl-N-(m-tolylmethyl)imidazo[4,5-c]pyridin-2-amine |
| 584 | 1-methyl-N6-(3-methylsulfonylphenyl)-N2-(m-tolylmethyl)imidazo[4,5-c]pyridine-2,6-diamine |
| 585 | 3-[[1-methyl-2-(m-tolylmethylamino)imidazo[4,5-c]pyridin-6-yl]amino]phenol |
| 586 | 6-(2-methoxy-3-pyridyl)-1-methyl-N-(m-tolylmethyl)imidazo[4,5-c]pyridin-2-amine |
| 587 | N-[3-[[1-methyl-2-(m-tolylmethylamino)imidazo[4,5-c]pyridin-6-yl]amino]phenyl]methanesulfonamide |
| 588 | 1-methyl-6-phenyl-N-(3,4,5-trimethoxyphenyl)imidazo[4,5-c]pyridin-2-amine |
| 589 | 1-methyl-6-(3-pyridyl)-N-(3,4,5-trimethoxyphenyl)imidazo[4,5-c]pyridin-2-amine |
| 590 | 6-(6-amino-3-pyridyl)-1-methyl-N-[3-(trifluoromethyl)phenyl]imidazo[4,5-c]pyridin-2-amine |
| 591 | N-[2-(2,5-dimethoxyphenyl)ethyl]-1-methyl-6-(4-pyridyl)imidazo[4,5-c]pyridin-2-amine |
| 592 | N-[2-(2,5-dimethoxyphenyl)ethyl]-1-methyl-6-(3-thienyl)imidazo[4,5-c]pyridin-2-amine |
| 593 | N-[2-(2,5-dimethoxyphenyl)ethyl]-1-methyl-6-(3,4,5-trimethoxyphenyl)imidazo[4,5-c]pyridin-2-amine |
| 594 | 4-[2-[2-(2,5-dimethoxyphenyl)ethylamino]-1-methyl-imidazo[4,5-c]pyridin-6-yl]-2-methoxy-phenol |
| 595 | N-[2-(2,5-dimethoxyphenyl)ethyl]-1-methyl-6-(2-thienyl)imidazo[4,5-c]pyridin-2-amine |
| 596 | N-(cyclopropylmethyl)-1-methyl-6-(3-thienyl)imidazo[4,5-c]pyridin-2-amine |
| 597 | N-(cyclopropylmethyl)-1-methyl-6-(4-piperazin-1-ylphenyl)imidazo[4,5-c]pyridin-2-amine |
| 598 | N6-(1,3-benzodioxol-5-yl)-N2-(cyclopropylmethyl)-1-methyl-imidazo[4,5-c]pyridine-2,6-diamine |
| 599 | N2-[(3,4-dimethoxyphenyl)methyl]-1-methyl-N6-(3,4,5-trimethoxyphenyl)imidazo[4,5-c]pyridine-2,6-diamine |
| 600 | N-[4-[[2-[(3,4-dimethoxyphenyl)methylamino]-1-methyl-imidazo[4,5-c]pyridin-6-yl]amino]phenyl]acetamide |
| 601 | 4-[[2-[(3,4-dimethoxyphenyl)methylamino]-1-methyl-imidazo[4,5-c]pyridin-6-yl]amino]benzamide |
| 602 | N6-(2,4-dimethoxyphenyl)-N2-[(3,4-dimethoxyphenyl)methyl]-1-methyl-imidazo[4,5-c]pyridine-2,6-diamine |
| 603 | N6-(3,4-dimethoxyphenyl)-N2-[(3,4-dimethoxyphenyl)methyl]-1-methyl-imidazo[4,5-c]pyridine-2,6-diamine |
| 604 | N2-[(3,4-dimethoxyphenyl)methyl]-1-methyl-N6-phenyl-imidazo[4,5-c]pyridine-2,6-diamine |
| 605 | N2-[(3,4-dimethoxyphenyl)methyl]-1-methyl-N6-[4-(4-methylpiperazin-1-yl)phenyl]imidazo[4,5-c]pyridine-2,6-diamine |
| 606 | N2-[(3,4-dimethoxyphenyl)methyl]-N6-(4-methoxyphenyl)-1-methyl-imidazo[4,5-c]pyridine-2,6-diamine |
| 607 | N2-[(3,4-dimethoxyphenyl)methyl]-N6-(4-fluorophenyl)-1-methyl-imidazo[4,5-c]pyridine-2,6-diamine |
| 608 | N2-[(3,4-dimethoxyphenyl)methyl]-1-methyl-N6-(3-methylsulfonylphenyl)imidazo[4,5-c]pyridine-2,6-diamine |
| 609 | N2,1-dimethyl-N6-(3,4,5-trimethoxyphenyl)imidazo[4,5-c]pyridine-2,6-diamine |
| 610 | N6-(2,4-dimethoxyphenyl)-N2,1-dimethyl-imidazo[4,5-c]pyridine-2,6-diamine |
| 611 | N6-(4-fluorophenyl)-N2,1-dimethyl-imidazo[4,5-c]pyridine-2,6-diamine |
| 612 | 2-methoxy-4-[1-methyl-2-(methylamino)imidazo[4,5-c]pyridin-6-yl]phenol |
| 613 | N-(2-methoxyphenyl)-1-methyl-6-(3-pyridyl)imidazo[4,5-c]pyridin-2-amine |
| 614 | N-benzyl-1-methyl-6-(4-morpholinophenyl)imidazo[4,5-c]pyridin-2-amine |

(continued)

| compound | name |
|---|---|
| 615 | N-benzyl-6-(4-dimethylaminophenyl)-1-methyl-imidazo[4,5-c]pyridin-2-amine |
| 616 | N2-benzyl-1-methyl-N6-(3,4,5-trimethoxyphenyl)imidazo[4,5-c]pyridine-2,6-diamine |
| 617 | N-[4-[[2-(benzylamino)-1-methyl-imidazo[4,5-c]pyridin-6-yl]amino]phenyl]acetamide |
| 618 | N2-benzyl-N6-(6-methoxy-3-pyridyl)-1-methyl-imidazo[4,5-c]pyridine-2,6-diamine |
| 619 | N2-benzyl-N6-[3-(dimethylamino)phenyl]-1-methyl-imidazo[4,5-c]pyridine-2,6-diamine |
| 620 | N-benzyl-6-[3-(dimethylamino)phenyl]-1-methyl-imidazo[4,5-c]pyridin-2-amine |
| 621 | N2-benzyl-N6-(4-methoxyphenyl)-1-methyl-imidazo[4,5-c]pyridine-2,6-diamine |
| 622 | 3-[[2-(benzylamino)-1-methyl-imidazo[4,5-c]pyridin-6-yl]amino]phenol |
| 623 | N-benzyl-1-methyl-6-pyrimidin-5-yl-imidazo[4,5-c]pyridin-2-amine |
| 624 | N-benzyl-1-methyl-6-(1-methylpyrazol-4-yl)imidazo[4,5-c]pyridin-2-amine |
| 625 | 6-(6-amino-3-pyridyl)-N-benzyl-1-methyl-imidazo[4,5-c]pyridin-2-amine |
| 626 | 4-[[2-(benzylamino)-1-methyl-imidazo[4,5-c]pyridin-6-yl]amino]-N,N-dimethyl-benzamide |
| 627 | N-[3-[[2-(benzylamino)-1-methyl-imidazo[4,5-c]pyridin-6-yl]amino]phenyl]acetamide |
| 628 | 1-[3-[[2-(benzylamino)-1-methyl-imidazo[4,5-c]pyridin-6-yl]amino]phenyl]ethanone |
| 629 | N2-benzyl-N6-(3-fluorophenyl)-1-methyl-imidazo[4,5-c]pyridine-2,6-diamine |
| 630 | 1-methyl-6-(4-morpholinophenyl)-N-phenyl-imidazo[4,5-c]pyridin-2-amine |
| 631 | 6-(4-dimethylaminophenyl)-1-methyl-N-phenyl-imidazo[4,5-c]pyridin-2-amine |
| 632 | N6-(3,4-dimethoxyphenyl)-1-methyl-N2-phenyl-imidazo[4,5-c]pyridine-2,6-diamine |
| 633 | 1-methyl-N2-phenyl-N6-(3-pyridyl)imidazo[4,5-c]pyridine-2,6-diamine |
| 634 | 1-methyl-N2,N6-diphenyl-imidazo[4,5-c]pyridine-2,6-diamine |
| 635 | 1-methyl-6-(1-naphthyl)-N-phenyl-imidazo[4,5-c]pyridin-2-amine |
| 636 | 1-methyl-N2-phenyl-N6-(4-pyridyl)imidazo[4,5-c]pyridine-2,6-diamine |
| 637 | 1-methyl-N2-phenyl-N6-(2-pyridyl)imidazo[4,5-c]pyridine-2,6-diamine |
| 638 | 3-[(2-anilino-1-methyl-imidazo[4,5-c]pyridin-6-yl)amino]phenol |
| 639 | 1-methyl-N6-(4-methylsulfonylphenyl)-N2-phenyl-imidazo[4,5-c]pyridine-2,6-diamine |
| 640 | 1-methyl-6-(4-morpholinophenyl)-N-(tetrahydrofuran-2-ylmethyl)imidazo[4,5-c]pyridin-2-amine |
| 641 | 1-methyl-N2-(tetrahydrofuran-2-ylmethyl)-N6-(3,4,5-trimethoxyphenyl)imidazo[4,5-c]pyridine-2,6-diamine |
| 642 | N6-(3-chlorophenyl)-1-methyl-N2-(tetrahydrofuran-2-ylmethyl)imidazo[4,5-c]pyridine-2,6-diamine |
| 643 | N6-(3-chloro-4-fluoro-phenyl)-1-methyl-N2-(tetrahydrofuran-2-ylmethyl)imidazo[4,5-c]pyridine-2,6-diamine |
| 644 | 6-(2-methoxy-3-pyridyl)-1-methyl-N-(tetrahydrofuran-2-ylmethyl)imidazo[4,5-c]pyridin-2-amine |
| 645 | N2-(2-methoxyethyl)-1-methyl-N6-(3,4,5-trimethoxyphenyl)imidazo[4,5-c]pyridine-2,6-diamine |
| 646 | N-[4-[[2-(2-methoxyethylamino)-1-methyl-imidazo[4,5-c]pyridin-6-yl]amino]phenyl]acetamide |
| 647 | 6-[3-(dimethylamino)phenyl]-N-(2-methoxyethyl)-1-methyl-imidazo[4,5-c]pyridin-2-amine |
| 648 | N-(2-methoxyethyl)-1-methyl-6-(4-methylsulfonylphenyl)imidazo[4,5-c]pyridin-2-amine |
| 649 | methyl 4-[[2-(2-methoxyethylamino)-1-methyl-imidazo[4,5-c]pyridin-6-yl]amino]benzoate |
| 650 | N-(2-methoxyethyl)-6-(4-methoxy-2-methyl-phenyl)-1-methyl-imidazo[4,5-c]pyridin-2-amine |

(continued)

| compound | name |
|---|---|
| 651 | N-(2-methoxyethyl)-1-methyl-6-(1-methylpyrazol-4-yl)imidazo[4,5-c]pyridin-2-amine |
| 652 | 3-[2-(2-methoxyethylamino)-1-methyl-imidazo[4,5-c]pyridin-6-yl]-N-methyl-benzamide |
| 653 | N-[(2-fluorophenyl)methyl]-1-methyl-6-(4-morpholinophenyl)imidazo[4,5-c]pyridin-2-amine |
| 654 | 6-(4-dimethylaminophenyl)-N-[(2-fluorophenyl)methyl]-1-methyl-imidazo[4,5-c]pyridin-2-amine |
| 655 | [4-[2-[(2-fluorophenyl)methylamino]-1-methyl-imidazo[4,5-c]pyridin-6-yl]phenyl]methanol |
| 656 | 4-[2-[(2-fluorophenyl)methylamino]-1-methyl-imidazo[4,5-c]pyridin-6-yl]-2-methoxy-phenol |
| 657 | 6-(4-fluorophenyl)-N-[(2-fluorophenyl)methyl]-1-methyl-imidazo[4,5-c]pyridin-2-amine |
| 658 | N-[(2-fluorophenyl)methyl]-1-methyl-6-(2-thienyl)imidazo[4,5-c]pyridin-2-amine |
| 659 | 6-(3-aminophenyl)-1-[(3,4-dimethoxyphenyl)methyl]-N-methyl-imidazo[4,5-c]pyridin-2-amine |
| 660 | 1-[(3,4-dimethoxyphenyl)methyl]-N2-methyl-N6-(3,4,5-trimethoxyphenyl)imidazo[4,5-c]pyridine-2,6-diamine |
| 661 | N-[4-[[1-[(3,4-dimethoxyphenyl)methyl]-2-(methylamino)imidazo[4,5-c]pyridin-6-yl]amino]phenyl]acetamide |
| 662 | 1-[(3,4-dimethoxyphenyl)methyl]-N6-(3-methoxyphenyl)-N2-methyl-imidazo[4,5-c]pyridine-2,6-diamine |
| 663 | 4-[[1-[(3,4-dimethoxyphenyl)methyl]-2-(methylamino)imidazo[4,5-c]pyridin-6-yl]amino]phenol |
| 664 | 4-[[1-[(3,4-dimethoxyphenyl)methyl]-2-(methylamino)imidazo[4,5-c]pyridin-6-yl]amino]benzamide |
| 665 | N6-(3,4-dimethoxyphenyl)-1-[(3,4-dimethoxyphenyl)methyl]-N2-methyl-imidazo[4,5-c]pyridine-2,6-diamine |
| 666 | N6-(3-chlorophenyl)-1-[(3,4-dimethoxyphenyl)methyl]-N2-methyl-imidazo[4,5-c]pyridine-2,6-diamine |
| 667 | 1-[(3,4-dimethoxyphenyl)methyl]-N2-methyl-N6-phenyl-imidazo[4,5-c]pyridine-2,6-diamine |
| 668 | 1-[(3,4-dimethoxyphenyl)methyl]-N-methyl-6-(2-naphthyl)imidazo[4,5-c]pyridin-2-amine |
| 669 | 1-[(3,4-dimethoxyphenyl)methyl]-N-methyl-6-phenyl-imidazo[4,5-c]pyridin-2-amine |
| 670 | 1-[(3,4-dimethoxyphenyl)methyl]-N-methyl-6-(1-naphthyl)imidazo[4,5-c]pyridin-2-amine |
| 671 | 1-[(3,4-dimethoxyphenyl)methyl]-N2-methyl-N6-(m-tolyl)imidazo[4,5-c]pyridine-2,6-diamine |
| 672 | 1-[(3,4-dimethoxyphenyl)methyl]-6-(4-methoxyphenyl)-N-methyl-imidazo[4,5-c]pyridin-2-amine |
| 673 | 1-[3-[[1-[(3,4-dimethoxyphenyl)methyl]-2-(methylamino)imidazo[4,5-c]pyridin-6-yl]amino]phenyl]ethanone |
| 674 | 1-[(3,4-dimethoxyphenyl)methyl]-N6-(3-fluorophenyl)-N2-methyl-imidazo[4,5-c]pyridine-2,6-diamine |
| 675 | 1-[(3,4-dimethoxyphenyl)methyl]-N2-methyl-N6-(4-methylsulfanylphenyl)imidazo[4,5-c]pyridine-2,6-diamine |
| 676 | 1-[(3,4-dimethoxyphenyl)methyl]-N6-(3-isopropoxyphenyl)-N2-methyl-imidazo[4,5-c]pyridine-2,6-diamine |
| 677 | N-(2-methoxyethyl)-6-(2-naphthyl)-1H-imidazo[4,5-c]pyridin-2-amine |
| 678 | 6-(4-dimethylaminophenyl)-1-(3-fluorophenyl)-N-methyl-imidazo[4,5-c]pyridin-2-amine |
| 679 | 1-(3-fluorophenyl)-N2-methyl-N6-(3,4,5-trimethoxyphenyl)imidazo[4,5-c]pyridine-2,6-diamine |
| 680 | 1-(3-fluorophenyl)-N6-(3-methoxyphenyl)-N2-methyl-imidazo[4,5-c]pyridine-2,6-diamine |
| 681 | 4-[[1-(3-fluorophenyl)-2-(methylamino)imidazo[4,5-c]pyridin-6-yl]amino]benzamide |
| 682 | N6-[3-(dimethylamino)phenyl]-1-(3-fluorophenyl)-N2-methyl-imidazo[4,5-c]pyridine-2,6-diamine |
| 683 | 6-[3-(dimethylamino)phenyl]-1-(3-fluorophenyl)-N-methyl-imidazo[4,5-c]pyridin-2-amine |

(continued)

| compound | name |
|---|---|
| 684 | 1-(3-fluorophenyl)-N-methyl-6-(4-methylsulfonylphenyl)imidazo[4,5-c]pyridin-2-amine |
| 685 | 1-(3-fluorophenyl)-N6-(4-methoxyphenyl)-N2-methyl-imidazo[4,5-c]pyridine-2,6-diamine |
| 686 | 4-[[1-(3-fluorophenyl)-2-(methylamino)imidazo[4,5-c]pyridin-6-yl]amino]benzonitrile |
| 687 | 1-(3-fluorophenyl)-N-methyl-6-(2-thienyl)imidazo[4,5-c]pyridin-2-amine |
| 688 | N-[3-[[1-(3-fluorophenyl)-2-(methylamino)imidazo[4,5-c]pyridin-6-yl]amino]phenyl]acetamide |
| 689 | 1-[3-[[1-(3-fluorophenyl)-2-(methylamino)imidazo[4,5-c]pyridin-6-yl]amino]phenyl]ethanone |
| 690 | N-[3-(trifluoromethyl)phenyl]-1-(3,4,5-trimethoxyphenyl)imidazo[4,5-c]pyridin-6-amine |
| 691 | N1,N1-dimethyl-N3-(1-phenylimidazo[4,5-c]pyridin-6-yl)benzene-1,3-diamine |
| 692 | N-[4-[[1-(2-furylmethyl)imidazo[4,5-c]pyridin-6-yl]amino]phenyl]acetamide |
| 693 | 1-(2-furylmethyl)-N-(4-methoxyphenyl)imidazo[4,5-c]pyridin-6-amine |
| 694 | N-[4-[(1-benzylimidazo[4,5-c]pyridin-6-yl)amino]phenyl]acetamide |
| 695 | N-(1,3-benzodioxol-5-yl)-1-benzyl-imidazo[4,5-c]pyridin-6-amine |
| 696 | 1-(cyclopropylmethyl)-N-(4-methylsulfonylphenyl)imidazo[4,5-c]pyridin-6-amine |
| 697 | 1,2-dimethyl-N-(3,4,5-trimethoxyphenyl)imidazo[4,5-c]pyridin-6-amine |
| 698 | 1-[3-[(1,2-dimethylimidazo[4,5-c]pyridin-6-yl)amino]phenyl]ethanone |
| 699 | 2-benzyl-1-methyl-N-(3,4,5-trimethoxyphenyl)imidazo[4,5-c]pyridin-6-amine |
| 700 | N-[4-[(2-benzyl-1-methyl-imidazo[4,5-c]pyridin-6-yl)amino]phenyl]acetamide |
| 701 | 1-methyl-2-phenyl-N-(3,4,5-trimethoxyphenyl)imidazo[4,5-c]pyridin-6-amine |
| 702 | N-[4-[(1-methyl-2-phenyl-imidazo[4,5-c]pyridin-6-yl)amino]phenyl]acetamide |
| 703 | N-(2,4-dimethoxyphenyl)-1-methyl-2-phenyl-imidazo[4,5-c]pyridin-6-amine |
| 704 | N-(6-methoxy-3-pyridyl)-1-methyl-2-phenyl-imidazo[4,5-c]pyridin-6-amine |
| 705 | N-(4-methoxyphenyl)-1-methyl-2-phenyl-imidazo[4,5-c]pyridin-6-amine |
| 706 | N-(1,3-benzodioxol-5-yl)-1-methyl-2-phenyl-imidazo[4,5-c]pyridin-6-amine |
| 707 | 2-(2-furyl)-1-methyl-N-(3,4,5-trimethoxyphenyl)imidazo[4,5-c]pyridin-6-amine |
| 708 | N-[4-[[2-(2-furyl)-1-methyl-imidazo[4,5-c]pyridin-6-yl]amino]phenyl]acetamide |
| 709 | 2-(2-furyl)-1-methyl-N-(m-tolyl)imidazo[4,5-c]pyridin-6-amine |
| 710 | N-(2-chlorophenyl)-2-(2-furyl)-1-methyl-imidazo[4,5-c]pyridin-6-amine |
| 711 | 4-[[2-(2-furyl)-1-methyl-imidazo[4,5-c]pyridin-6-yl]amino]-N,N-dimethyl-benzamide |
| 712 | N-[3-[[2-(2-furyl)-1-methyl-imidazo[4,5-c]pyridin-6-yl]amino]phenyl]acetamide |
| 713 | N-[4-[[2-(3-fluorophenyl)-1-methyl-imidazo[4,5-c]pyridin-6-yl]amino]phenyl]acetamide |
| 714 | N-(1,3-benzodioxol-5-yl)-2-(3-fluorophenyl)-1-methyl-imidazo[4,5-c]pyridin-6-amine |
| 715 | 4-[[2-(3-fluorophenyl)-1-methyl-imidazo[4,5-c]pyridin-6-yl]amino]-N,N-dimethyl-benzamide |
| 716 | 2-(3,4-dimethoxyphenyl)-1-methyl-N-(3,4,5-trimethoxyphenyl)imidazo[4,5-c]pyridin-6-amine |
| 717 | N-[4-[[2-(3,4-dimethoxyphenyl)-1-methyl-imidazo[4,5-c]pyridin-6-yl]amino]phenyl]acetamide |
| 718 | 2-(3,4-dimethoxyphenyl)-N-(3-methoxyphenyl)-1-methyl-imidazo[4,5-c]pyridin-6-amine |
| 719 | 4-[[2-(3,4-dimethoxyphenyl)-1-methyl-imidazo[4,5-c]pyridin-6-yl]amino]benzamide |
| 720 | 2-(3,4-dimethoxyphenyl)-N-(4-methoxy-2-methyl-phenyl)-1-methyl-imidazo[4,5-c]pyridin-6-amine |
| 721 | 2-(3,4-dimethoxyphenyl)-N-(4-methoxyphenyl)-1-methyl-imidazo[4,5-c]pyridin-6-amine |

(continued)

| compound | name |
|---|---|
| 722 | N-(1,3-benzodioxol-5-yl)-2-(3,4-dimethoxyphenyl)-1-methyl-imidazo[4,5-c]pyridin-6-amine |
| 723 | 5-[[2-(3,4-dimethoxyphenyl)-1-methyl-imidazo[4,5-c]pyridin-6-yl]amino]-2-methoxy-phenol |
| 724 | 1-methyl-2-(4-pyridyl)-N-(3,4,5-trimethoxyphenyl)imidazo[4,5-c]pyridin-6-amine |
| 725 | 1-methyl-N-(3,4,5-trimethoxyphenyl)imidazo[4,5-c]pyridin-6-amine |
| 726 | N-[4-[(1-methylimidazo[4,5-c]pyridin-6-yl)amino]phenyl]acetamide |
| 727 | N-(2,4-dimethoxyphenyl)-1-methyl-imidazo[4,5-c]pyridin-6-amine |
| 728 | N-(4-methoxyphenyl)-1-methyl-imidazo[4,5-c]pyridin-6-amine |
| 729 | 1-methyl-2-(3-pyridylmethyl)-N-(3,4,5-trimethoxyphenyl)imidazo[4,5-c]pyridin-6-amine |
| 730 | N-[4-[[1-[(3,4-dimethoxyphenyl)methyl]imidazo[4,5-c]pyridin-6-yl]amino]phenyl]acetamide |
| 731 | N-(1,3-benzodioxol-5-yl)-1-[(3,4-dimethoxyphenyl)methyl]imidazo[4,5-c]pyridin-6-amine |
| 732 | 3-[1-(3,4,5-trimethoxyphenyl)imidazo[4,5-c]pyridin-6-yl]benzamide |
| 733 | 6-(3-fluorophenyl)-1-phenyl-imidazo[4,5-c]pyridine |
| 734 | 6-(3,4-dimethoxyphenyl)-1-[3-(trifluoromethyl)phenyl]imidazo[4,5-c]pyridine |
| 735 | 5-(1-methyl-2-phenyl-imidazo[4,5-c]pyridin-6-yl)pyridin-2-amine |
| 736 | 3-[2-(2-furyl)-1-methyl-imidazo[4,5-c]pyridin-6-yl]phenol |
| 737 | 1-[(3,4-dimethoxyphenyl)methyl]-6-(3-pyridyl)imidazo[4,5-c]pyridine |
| 738 | 1-[(3,4-dimethoxyphenyl)methyl]-6-(3,4,5-trimethoxyphenyl)imidazo[4,5-c]pyridine |
| 739 | 4-[1-[(3,4-dimethoxyphenyl)methyl]imidazo[4,5-c]pyridin-6-yl]-2-methoxy-phenol |
| 740 | 3-[1-(3-fluorophenyl)imidazo[4,5-c]pyridin-6-yl]-N,N-dimethyl-aniline |
| 741 | 1-phenylimidazo[4,5-c]pyridine-2-thiol |
| 742 | 2-(2-pyridyl)-1H-imidazo[4,5-c]pyridine |
| 743 | 2-(2-thienyl)-3H-imidazo[4,5-c]pyridine |
| 744 | 1-phenylimidazo[4,5-c]pyridine-2-thiol |
| 745 | 2-(methoxymethyl)-1-methyl-N-(3,4,5-trimethoxyphenyl)imidazo[4,5-c]pyridin-6-amine |
| 746 | 4-[2-(methylamino)-1-phenyl-imidazo[4,5-c]pyridin-6-yl]benzamide |
| 747 | 1-[3-[2-[(2-fluorophenyl)methylamino]-1-methyl-imidazo[4,5-c]pyridin-6-yl]phenyl]ethanone |
| 748 | 3-[2-(benzylamino)-1-methyl-imidazo[4,5-c]pyridin-6-yl]benzoic acid |
| 749 | N2,1-dimethyl-N6-(4-phenoxyphenyl)imidazo[4,5-c]pyridine-2,6-diamine |
| 750 | N2-(cyclopropylmethyl)-1-methyl-N6-(3-phenoxyphenyl)imidazo[4,5-c]pyridine-2,6-diamine |
| 751 | N-(2-methoxyethyl)-6-(6-methoxy-3-pyridyl)-1-methyl-imidazo[4,5-c]pyridin-2-amine |
| 752 | 1-phenyl-6-[3-(trifluoromethyl)phenyl]imidazo[4,5-c]pyridine |
| 753 | N6-(3-chloro-4-fluoro-phenyl)-N2-methyl-1-(3,4,5-trimethoxyphenyl)imidazo[4,5-c]pyridine-2,6-diamine |
| 754 | 1-[(3,4-dimethoxyphenyl)methyl]-6-(3-methoxyphenyl)imidazo[4,5-c]pyridine |
| 755 | 1-methyl-N2-(tetrahydrofuran-2-ylmethyl)-N6-[3-(trifluoromethoxy)phenyl]imidazo[4,5-c]pyridine-2,6-diamine |
| 756 | 2-(2-furyl)-1-methyl-6-[3-(trifluoromethoxy)phenyl]imidazo[4,5-c]pyridine |
| 757 | 6-(2-methoxyphenyl)-N-methyl-1-phenyl-imidazo[4,5-c]pyridin-2-amine |

(continued)

| compound | name |
|---|---|
| 758 | N-(cyclopropylmethyl)-1-methyl-6-(3-phenoxyphenyl)imidazo[4,5-c]pyridin-2-amine |
| 759 | 6-(1,3-benzodioxol-5-yl)-N-(2-methoxyethyl)-1-methyl-imidazo[4,5-c]pyridin-2-amine |
| 760 | 1-(cyclopropylmethyl)-N6-(4-methoxy-2-methyl-phenyl)-N2-methyl-imidazo[4,5-c]pyridine-2,6-diamine |
| 761 | N-[4-[1-(3-fluorophenyl)imidazo[4,5-c]pyridin-6-yl]phenyl]acetamide |
| 762 | 1-benzyl-N-methyl-6-[3-(trifluoromethoxy)phenyl]imidazo[4,5-c]pyridin-2-amine |
| 763 | N2-(cyclopropylmethyl)-1-methyl-N6-[4-(trifluoromethyl)phenyl]imidazo[4,5-c]pyridine-2,6-diamine |
| 764 | 2-(2-furyl)-6-(5-methoxy-3-pyridyl)-1-methyl-imidazo[4,5-c]pyridine |
| 765 | N2-methyl-1-(3-pyridylmethyl)-N6-[3-(trifluoromethyl)phenyl]imidazo[4,5-c]pyridine-2,6-diamine |
| 766 | 3-[1-benzyl-2-(methylamino)imidazo[4,5-c]pyridin-6-yl]-N-(2-hydroxyethyl)benzamide |
| 767 | 1-(2-furylmethyl)-N6-[3-methoxy-5-(trifluoromethyl)phenyl]-N2-methyl-imidazo[4,5-c]pyridine-2,6-diamine |
| 768 | N-[3-[1-(3-fluorophenyl)-2-(methylamino)imidazo[4,5-c]pyridin-6-yl]phenyl]methanesulfonamide |
| 769 | 6-(3-fluorophenyl)-1-methyl-N-(2-morpholinoethyl)imidazo[4,5-c]pyridin-2-amine |
| 770 | N-(2-methoxyethyl)-1-methyl-6-(o-tolyl)imidazo[4,5-c]pyridin-2-amine |
| 771 | N-(2-dimethylaminoethyl)-3-[1-(3-fluorophenyl)-2-(methylamino)imidazo[4,5-c]pyridin-6-yl]benzamide |
| 772 | N6-(3-isopropylphenyl)-1-(2-methoxyethyl)-N2-methyl-imidazo[4,5-c]pyridine-2,6-diamine |
| 773 | 1-(2-methoxyethyl)-N2-methyl-N6-[4-(trifluoromethoxy)phenyl]imidazo[4,5-c]pyridine-2,6-diamine |
| 774 | 2-(p-tolyl)-3H-imidazo[4,5-c]pyridine |
| 775 | 2-(4-fluorophenyl)-3H-imidazo[4,5-c]pyridine |
| 776 | N-(5-tert-butyl-2-methyl-pyrazol-3-yl)-1-(2-furylmethyl)imidazo[4,5-c]pyridin-6-amine |
| 777 | N6-[2-fluoro-5-(trifluoromethyl)phenyl]-1-(2-furylmethyl)-N2-methyl-imidazo[4,5-c]pyridine-2,6-diamine |
| 778 | 2-(1-methylimidazo[4,5-c]pyridin-2-yl)quinoline |
| 779 | 2-(4-fluorophenyl)-3H-imidazo[4,5-c]pyridine |
| 780 | 1-methylimidazo[4,5-c]pyridine-2-sulfonic acid |
| 781 | 6-(4-dimethylaminophenyl)-N-methyl-1-phenyl-imidazo[4,5-c]pyridin-2-amine |
| 782 | 6-(2-methoxyphenyl)-N-methyl-1-phenyl-imidazo[4,5-c]pyridin-2-amine |
| 783 | 6-[3-(dimethylamino)phenyl]-N-methyl-1-phenyl-imidazo[4,5-c]pyridin-2-amine |
| 784 | 6-(3-chloro-4-fluoro-phenyl)-N-methyl-1-phenyl-imidazo[4,5-c]pyridin-2-amine |
| 785 | [4-[2-(methylamino)-1-phenyl-imidazo[4,5-c]pyridin-6-yl]phenyl]methanol |
| 786 | 4-[2-(methylamino)-1-phenyl-imidazo[4,5-c]pyridin-6-yl]benzamide |
| 787 | N-methyl-1-phenyl-6-(2-thienyl)imidazo[4,5-c]pyridin-2-amine |
| 788 | 6-(2-methoxy-3-pyridyl)-N-methyl-1-phenyl-imidazo[4,5-c]pyridin-2-amine |
| 789 | 1-benzyl-6-(4-dimethylaminophenyl)-N-methyl-imidazo[4,5-c]pyridin-2-amine |
| 790 | 1-benzyl-N-methyl-6-(2-phenoxyphenyl)imidazo[4,5-c]pyridin-2-amine |
| 791 | 6-(1,3-benzodioxol-5-yl)-1-benzyl-N-methyl-imidazo[4,5-c]pyridin-2-amine |
| 792 | 1-benzyl-6-(4-chlorophenyl)-N-methyl-imidazo[4,5-c]pyridin-2-amine |

(continued)

| compound | name |
|---|---|
| 793 | 3-[1-benzyl-2-(methylamino)imidazo[4,5-c]pyridin-6-yl]-N-(2-dimethylaminoethyl)benzamide |
| 794 | 3-[1-benzyl-2-(methylamino)imidazo[4,5-c]pyridin-6-yl]-N-(2-hydroxyethyl)benzamide |
| 795 | 1-benzyl-6-(3-chlorophenyl)-N-methyl-imidazo[4,5-c]pyridin-2-amine |
| 796 | 3-[1-benzyl-2-(methylamino)imidazo[4,5-c]pyridin-6-yl]phenol |
| 797 | 4-[1-benzyl-2-(methylamino)imidazo[4,5-c]pyridin-6-yl]-2-methoxy-phenol |
| 798 | 1-benzyl-6-(3-furyl)-N-methyl-imidazo[4,5-c]pyridin-2-amine |
| 799 | N-[4-[1-benzyl-2-(methylamino)imidazo[4,5-c]pyridin-6-yl]phenyl]acetamide |
| 800 | N-methyl-6-(3-pyridyl)-1-(3-pyridylmethyl)imidazo[4,5-c]pyridin-2-amine |
| 801 | N-methyl-1-(3-pyridylmethyl)-6-(2-thienyl)imidazo[4,5-c]pyridin-2-amine |
| 802 | 6-(4-dimethylaminophenyl)-1-methyl-N-(m-tolylmethyl)imidazo[4,5-c]pyridin-2-amine |
| 803 | 6-(3,4-dimethoxyphenyl)-1-methyl-N-(m-tolylmethyl)imidazo[4,5-c]pyridin-2-amine |
| 804 | N-[3-[1-methyl-2-(m-tolylmethylamino)imidazo[4,5-c]pyridin-6-yl]phenyl]acetamide |
| 805 | 6-(4-chlorophenyl)-1-methyl-N-(m-tolylmethyl)imidazo[4,5-c]pyridin-2-amine |
| 806 | 1-methyl-6-(4-methylsulfonylphenyl)-N-(m-tolylmethyl)imidazo[4,5-c]pyridin-2-amine |
| 807 | 6-(4-methoxyphenyl)-1-methyl-N-(m-tolylmethyl)imidazo[4,5-c]pyridin-2-amine |
| 808 | 2-methoxy-4-[1-methyl-2-(m-tolylmethylamino)imidazo[4,5-c]pyridin-6-yl]phenol |
| 809 | 6-(3-furyl)-1-methyl-N-(m-tolylmethyl)imidazo[4,5-c]pyridin-2-amine |
| 810 | 6-(2-methoxy-3-pyridyl)-1-methyl-N-(m-tolylmethyl)imidazo[4,5-c]pyridin-2-amine |
| 811 | 6-(6-amino-3-pyridyl)-1-methyl-N-(m-tolylmethyl)imidazo[4,5-c]pyridin-2-amine |
| 812 | 1-methyl-6-phenyl-N-(3,4,5-trimethoxyphenyl)imidazo[4,5-c]pyridin-2-amine |
| 813 | 1-methyl-6-(3-pyridyl)-N-(3,4,5-trimethoxyphenyl)imidazo[4,5-c]pyridin-2-amine |
| 814 | 6-(3-fluorophenyl)-1-methyl-N-(3,4,5-trimethoxyphenyl)imidazo[4,5-c]pyridin-2-amine |
| 815 | 6-(6-amino-3-pyridyl)-1-methyl-N-[3-(trifluoromethyl)phenyl]imidazo[4,5-c]pyridin-2-amine |
| 816 | 6-(1,3-benzodioxol-5-yl)-N-[2-(2,5-dimethoxyphenyl)ethyl]-1-methyl-imidazo[4,5-c]pyridin-2-amine |
| 817 | N-[2-(2,5-dimethoxyphenyl)ethyl]-1-methyl-6-(4-pyridyl)imidazo[4,5-c]pyridin-2-amine |
| 818 | N-[2-(2,5-dimethoxyphenyl)ethyl]-1-methyl-6-(3-thienyl)imidazo[4,5-c]pyridin-2-amine |
| 819 | N-[2-(2,5-dimethoxyphenyl)ethyl]-1-methyl-6-(3,4,5-trimethoxyphenyl)imidazo[4,5-c]pyridin-2-amine |
| 820 | 3-[2-[2-(2,5-dimethoxyphenyl)ethylamino]-1-methyl-imidazo[4,5-c]pyridin-6-yl]phenol |
| 821 | 4-[2-[2-(2,5-dimethoxyphenyl)ethylamino]-1-methyl-imidazo[4,5-c]pyridin-6-yl]-2-methoxy-phenol |
| 822 | 3-[2-[2-(2,5-dimethoxyphenyl)ethylamino]-1-methyl-imidazo[4,5-c]pyridin-6-yl]benzamide |
| 823 | N-[2-(2,5-dimethoxyphenyl)ethyl]-6-(3-methoxyphenyl)-1-methyl-imidazo[4,5-c]pyridin-2-amine |
| 824 | N-[2-(2,5-dimethoxyphenyl)ethyl]-1-methyl-6-(2-thienyl)imidazo[4,5-c]pyridin-2-amine |
| 825 | N-[3-[2-[2-(2,5-dimethoxyphenyl)ethylamino]-1-methyl-imidazo[4,5-c]pyridin-6-yl]phenyl]methanesulfonamide |
| 826 | N-(cyclopropylmethyl)-1-methyl-6-phenyl-imidazo[4,5-c]pyridin-2-amine |
| 827 | N-(cyclopropylmethyl)-1-methyl-6-(3-thienyl)imidazo[4,5-c]pyridin-2-amine |
| 828 | N-(cyclopropylmethyl)-6-[3-(dimethylamino)phenyl]-1-methyl-imidazo[4,5-c]pyridin-2-amine |
| 829 | N-(cyclopropylmethyl)-1-methyl-6-(4-piperazin-1-ylphenyl)imidazo[4,5-c]pyridin-2-amine |

(continued)

| compound | name |
|---|---|
| 830 | [4-[1-methyl-2-(methylamino)imidazo[4,5-c]pyridin-6-yl]phenyl]methanol |
| 831 | N,1-dimethyl-6-[3-(trifluoromethyl)phenyl]imidazo[4,5-c]pyridin-2-amine |
| 832 | 2-methoxy-4-[1-methyl-2-(methylamino)imidazo[4,5-c]pyridin-6-yl]phenol |
| 833 | N-(2-methoxyphenyl)-1-methyl-6-(3-pyridyl)imidazo[4,5-c]pyridin-2-amine |
| 834 | N-benzyl-1-methyl-6-(4-morpholinophenyl)imidazo[4,5-c]pyridin-2-amine |
| 835 | N-benzyl-6-(4-dimethylaminophenyl)-1-methyl-imidazo[4,5-c]pyridin-2-amine |
| 836 | N-benzyl-1-methyl-6-phenyl-imidazo[4,5-c]pyridin-2-amine |
| 837 | N-benzyl-6-[3-(dimethylamino)phenyl]-1-methyl-imidazo[4,5-c]pyridin-2-amine |
| 838 | 3-[2-(benzylamino)-1-methyl-imidazo[4,5-c]pyridin-6-yl]benzoic acid |
| 839 | N-benzyl-6-(4-methoxyphenyl)-1-methyl-imidazo[4,5-c]pyridin-2-amine |
| 840 | N-benzyl-1-methyl-6-pyrimidin-5-yl-imidazo[4,5-c]pyridin-2-amine |
| 841 | N-benzyl-1-methyl-6-(2-thienyl)imidazo[4,5-c]pyridin-2-amine |
| 842 | N-benzyl-1-methyl-6-(1-methylpyrazol-4-yl)imidazo[4,5-c]pyridin-2-amine |
| 843 | 6-(6-amino-3-pyridyl)-N-benzyl-1-methyl-imidazo[4,5-c]pyridin-2-amine |
| 844 | 1-methyl-6-(4-morpholinophenyl)-N-phenyl-imidazo[4,5-c]pyridin-2-amine |
| 845 | 6-(4-dimethylaminophenyl)-1-methyl-N-phenyl-imidazo[4,5-c]pyridin-2-amine |
| 846 | 1-methyl-6-(1-naphthyl)-N-phenyl-imidazo[4,5-c]pyridin-2-amine |
| 847 | 6-(4-methoxyphenyl)-1-methyl-N-phenyl-imidazo[4,5-c]pyridin-2-amine |
| 848 | 6-(3-fluorophenyl)-1-methyl-N-(2-morpholinoethyl)imidazo[4,5-c]pyridin-2-amine |
| 849 | 1-methyl-6-(4-morpholinophenyl)-N-(tetrahydrofuran-2-ylmethyl)imidazo[4,5-c]pyridin-2-amine |
| 850 | 1-methyl-6-(2-naphthyl)-N-(tetrahydrofuran-2-ylmethyl)imidazo[4,5-c]pyridin-2-amine |
| 851 | 1-methyl-6-(3-phenoxyphenyl)-N-(tetrahydrofuran-2-ylmethyl)imidazo[4,5-c]pyridin-2-amine |
| 852 | 1-methyl-N-(tetrahydrofuran-2-ylmethyl)-6-(2-thienyl)imidazo[4,5-c]pyridin-2-amine |
| 853 | 6-(2-methoxy-3-pyridyl)-1-methyl-N-(tetrahydrofuran-2-ylmethyl)imidazo[4,5-c]pyridin-2-amine |
| 854 | 6-[3-(dimethylamino)phenyl]-N-(2-methoxyethyl)-1-methyl-imidazo[4,5-c]pyridin-2-amine |
| 855 | N-(2-methoxyethyl)-1-methyl-6-[3-(trifluoromethoxy)phenyl]imidazo[4,5-c]pyridin-2-amine |
| 856 | N-(2-methoxyethyl)-1-methyl-6-(4-methylsulfonylphenyl)imidazo[4,5-c]pyridin-2-amine |
| 857 | N-(2-methoxyethyl)-6-(6-methoxy-3-pyridyl)-1-methyl-imidazo[4,5-c]pyridin-2-amine |
| 858 | N-(2-methoxyethyl)-1-methyl-6-(o-tolyl)imidazo[4,5-c]pyridin-2-amine |
| 859 | N-(2-methoxyethyl)-6-(4-methoxy-2-methyl-phenyl)-1-methyl-imidazo[4,5-c]pyridin-2-amine |
| 860 | N-(2-methoxyethyl)-1-methyl-6-(1-methylpyrazol-4-yl)imidazo[4,5-c]pyridin-2-amine |
| 861 | 3-[2-(2-methoxyethylamino)-1-methyl-imidazo[4,5-c]pyridin-6-yl]-N-methyl-benzamide |
| 862 | 6-(3,4-dimethoxyphenyl)-1-methyl-N-(3-pyridylmethyl)imidazo[4,5-c]pyridin-2-amine |
| 863 | N-[(2-fluorophenyl)methyl]-1-methyl-6-(4-morpholinophenyl)imidazo[4,5-c]pyridin-2-amine |
| 864 | 6-(4-dimethylaminophenyl)-N-[(2-fluorophenyl)methyl]-1-methyl-imidazo[4,5-c]pyridin-2-amine |
| 865 | 6-(3,4-dimethoxyphenyl)-N-[(2-fluorophenyl)methyl]-1-methyl-imidazo[4,5-c]pyridin-2-amine |
| 866 | 6-(3-chlorophenyl)-N-[(2-fluorophenyl)methyl]-1-methyl-imidazo[4,5-c]pyridin-2-amine |
| 867 | [4-[2-[(2-fluorophenyl)methylamino]-1-methyl-imidazo[4,5-c]pyridin-6-yl]phenyl]methanol |

(continued)

| compound | name |
|---|---|
| 868 | [3-[2-[(2-fluorophenyl)methylamino]-1-methyl-imidazo[4,5-c]pyridin-6-yl]phenyl]methanol |
| 869 | 4-[2-[(2-fluorophenyl)methylamino]-1-methyl-imidazo[4,5-c]pyridin-6-yl]-2-methoxy-phenol |
| 870 | 6-(4-fluorophenyl)-N-[(2-fluorophenyl)methyl]-1-methyl-imidazo[4,5-c]pyridin-2-amine |
| 871 | 1-[3-[2-[(2-fluorophenyl)methylamino]-1-methyl-imidazo[4,5-c]pyridin-6-yl]phenyl]ethanone |
| 872 | N-[(2-fluorophenyl)methyl]-1-methyl-6-(2-thienyl)imidazo[4,5-c]pyridin-2-amine |
| 873 | 6-(3-aminophenyl)-1-[(3,4-dimethoxyphenyl)methyl]-N-methyl-imidazo[4,5-c]pyridin-2-amine |
| 874 | 1-[(3,4-dimethoxyphenyl)methyl]-N-methyl-6-(2-naphthyl)imidazo[4,5-c]pyridin-2-amine |
| 875 | 1-[(3,4-dimethoxyphenyl)methyl]-N-methyl-6-phenyl-imidazo[4,5-c]pyridin-2-amine |
| 876 | 1-[(3,4-dimethoxyphenyl)methyl]-N-methyl-6-(1-naphthyl)imidazo[4,5-c]pyridin-2-amine |
| 877 | 1-[(3,4-dimethoxyphenyl)methyl]-N-methyl-6-(3-thienyl)imidazo[4,5-c]pyridin-2-amine |
| 878 | 1-[(3,4-dimethoxyphenyl)methyl]-6-(4-methoxyphenyl)-N-methyl-imidazo[4,5-c]pyridin-2-amine |
| 879 | 4-[1-[(3,4-dimethoxyphenyl)methyl]-2-(methylamino)imidazo[4,5-c]pyridin-6-yl]-2-methoxy-phenol |
| 880 | 1-[(3,4-dimethoxyphenyl)methyl]-N-methyl-6-(2-thienyl)imidazo[4,5-c]pyridin-2-amine |
| 881 | 6-(5-methoxy-3-pyridyl)-N-(tetrahydrofuran-2-ylmethyl)-1H-imidazo[4,5-c]pyridin-2-amine |
| 882 | N-(2-methoxyethyl)-6-(2-naphthyl)-1H-imidazo[4,5-c]pyridin-2-amine |
| 883 | 6-(4-dimethylaminophenyl)-1-(3-fluorophenyl)-N-methyl-imidazo[4,5-c]pyridin-2-amine |
| 884 | 1-(3-fluorophenyl)-N-methyl-6-(1-naphthyl)imidazo[4,5-c]pyridin-2-amine |
| 885 | 6-(1,3-benzodioxol-5-yl)-1-(3-fluorophenyl)-N-methyl-imidazo[4,5-c]pyridin-2-amine |
| 886 | 6-[3-(dimethylamino)phenyl]-1-(3-fluorophenyl)-N-methyl-imidazo[4,5-c]pyridin-2-amine |
| 887 | 1-(3-fluorophenyl)-N-methyl-6-(4-methylsulfonylphenyl)imidazo[4,5-c]pyridin-2-amine |
| 888 | 4-[1-(3-fluorophenyl)-2-(methylamino)imidazo[4,5-c]pyridin-6-yl]-2-methoxy-phenol |
| 889 | 1-(3-fluorophenyl)-N-methyl-6-(2-thienyl)imidazo[4,5-c]pyridin-2-amine |

**Synthesis of compounds**

The inventive compound (I) according to the present invention can be prepared by methods known to one skilled in the art. The synthesis is preferably carried out according to the general synthetic routes, shown in schemes 1-4.

In the following schemes occurring abbreviations mean: AcOH (acetic acid); Boc (*tert*-butyl-oxycarbonyl); dba (dibenzylidenacetone); dppf (diphenylphosphino)ferrocene; DCM (dichloromethane); DIPEA (N-ethyl-N,N-diisopropylamine); DMAP (4-dimethylamino-pyridine); DMF (dimethylformamide); EDC (1-ethyl-3-(3-dimethylaminopropyl)carbodiimide)); Et (ethyl); MeOH (methanol); MeCN (acetonitrile); TFA (trifluoroacetic acid), Ph (phenyl), Ts (tosylate, p-toluenesulfonyl group), Ms (mesylate, methanesulfonyl group), TBHP (*tert*-butyl hydroperoxyde), PMB (para-methoxybenzyl); TFA (trifluoroacetic acid); PG (protecting group); X-phos (2-dicyclohexylphosphino-2',4',6'-triisopropylbiphenyl);

The compounds of general formula (I) presenting a pyrrolo[3,2-c] pyridine core can be synthesized by derivatization of pyrrolo[3,2-c]pyridine intermediates **4a*** and **4b*.** Said pyrrolo[3,2-c]pyridine intermediates can be accessed for example following the synthetic pathway depicted in **Scheme 1**, having as a key step a Sonogashira based cyclization. The synthesis begins from the commercially available 4-amino-pyridine derivatives **1a*** and **1b*,** which are subjected to iodination to afford iodinated intermediates **2a*** and **2b*.** Mesylation of the amine provides the intermediates **3a*** and **3b*,** suitable for Sonogashira coupling. At this step, a variety of 2-substitued pyrrolo[3,2-c]pyridine derivatives can be accessed by simply varying $R^1$ substituent on the coupling alkyne partner.

Conveniently, many terminal alkynes as these useful for obtaining intermediates **4a*** and **4b*** are commercially available. Moreover, additional terminal alkynes of general formula $R^1$-≡ can be easily prepared by a person skilled in the art following known synthetic procedures that are disclosed in patent or non-patent literature.

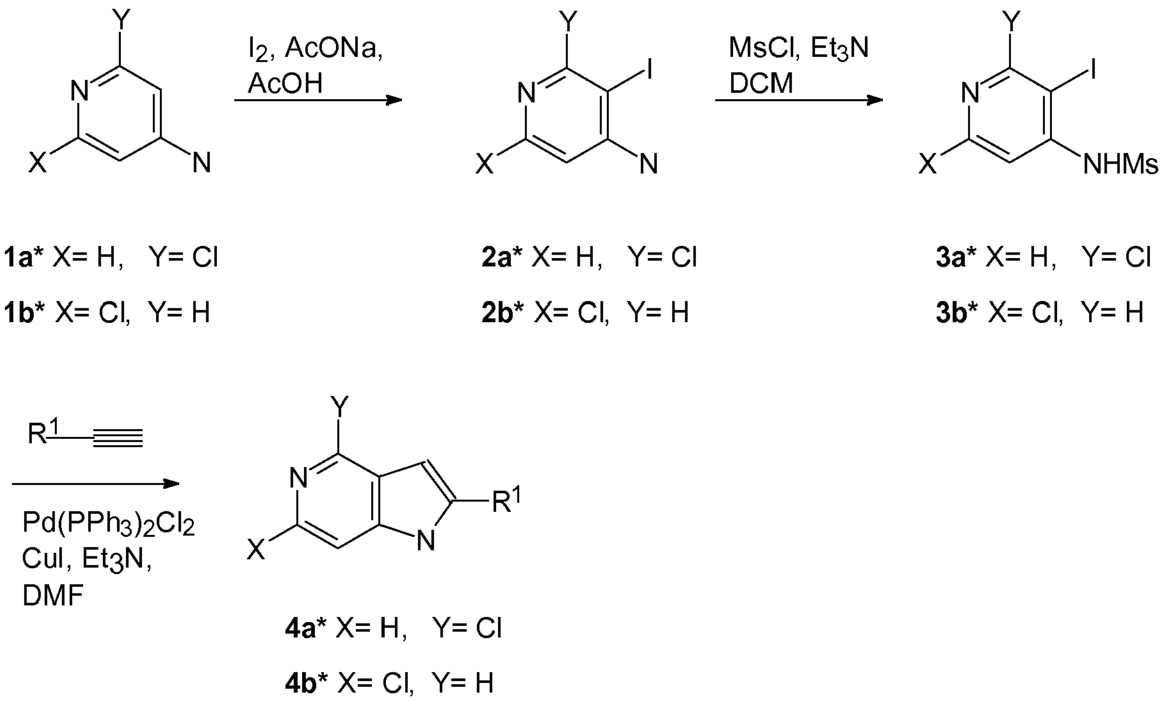


**1a*** X= H, Y= Cl

**1b*** X= Cl, Y= H

**2a*** X= H, Y= Cl

**2b*** X= Cl, Y= H

**3a*** X= H, Y= Cl

**3b*** X= Cl, Y= H

**4a*** X= H, Y= Cl

**4b*** X= Cl, Y= H

**Scheme 1:** Synthesis of pyrrolo[3,2-c]pyridine intermediates.

Once the pyrrolo[3,2-c]pyridine core was constructed and the substituent $R^1$ was installed, the synthesis of compounds of general formula (I) presenting a pyrrolo[3,2-c]pyridine core can be completed (see **Scheme 2)**. Simple amine protection, using for example alkyl-, aryl- or alkyl-aryl- halides in presence of a base, such as sodium hydride, installs the substituent $R^2$ at the position 2 of the pyrrolo[3,2-c]pyridine heterocycle. Conveniently, many alkyl-, aryl- or alkyl-arylhalides as these useful for protection of the amine **4a*** are commercially available. Moreover, additional many alkyl-, aryl- or alkyl-aryl- halides as these useful for protection of the amine **4a*** can be easily prepared by a person skilled in the art following known synthetic procedures that are disclosed in patent or non-patent literature. To access compounds of general formula (I), wherein $R^2$ = H amine intermediate **4a*** or its analogue **4b*** can be temporary protected with a suitable amine protecting group, such as Boc. Such a temporary protection involves, for example, treatment of amines **4a*** and **4b*** with $Boc_2O$ in presence of DMAP. However, the person skilled in the art of organic chemistry can use other alternative methods to temporary protect the amine group.

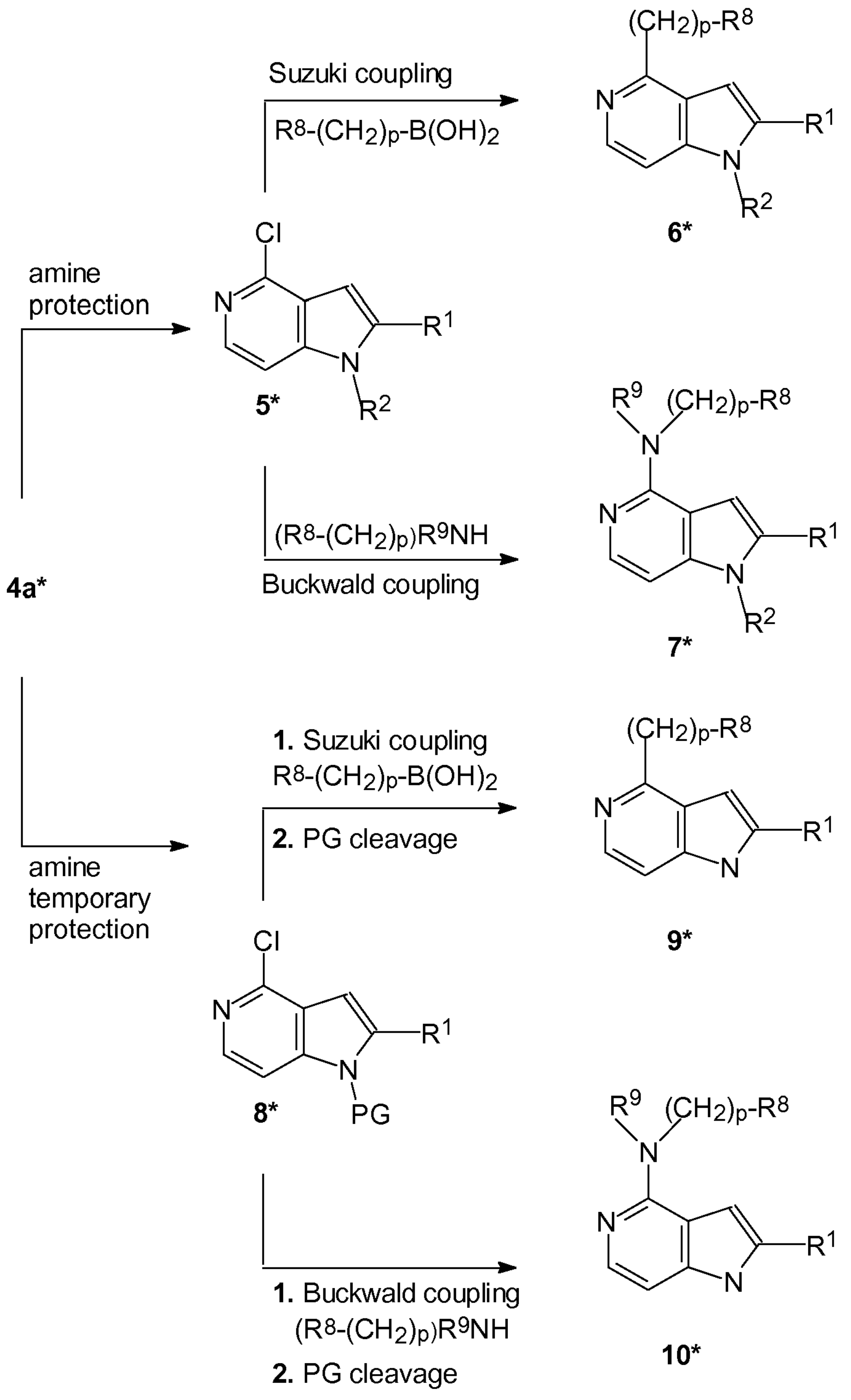


**Scheme 2**: Synthesis of pyrrolo[3,2-c]pyridine derivatives

The $R^4$ substituent, wherein $R^4$ is not H, can be introduced by subjecting the intermediates **5*** and **8*** to Buchwald or Suzuki coupling to provide after removal of temporary groups PG, the target compounds **6*, 7*, 9*** and **10*.** Buchwald coupling involves treatment of the halogenated (iodinated, chlorinated, brominated) or triflated pyrrolo[3,2-c]pyridine intermediate with a suitable amine, such as $(R^8$-$(CH_2)_p)R^9NH$, in presence of a palladium catalyst, such as $Pd_2(dba)_3$, a phosphine based ligand, such as X-phos and a base, such as $K_3PO_4$. The amines $(R^8$-$(CH_2)_p)R^9NH$ suitable for the coupling reaction are commercially available, or can be easily prepared by the person skilled in the art. Moreover, the person skilled in the art could envision other methods disclosed in patent or non-patent literature for introduction of the

amine moiety at the position 4 of the pyrrolo[3,2-c]pyridine derivative, including nucleophilic aromatic substitution, Mijita coupling and Ulmann coupling. Suzuki coupling requires treatment of the halogenated (iodinated, chlorinated, brominated) or triflated pyrrolo[3,2- c]pyridine with a boronic acid or ester, such as $R^8(CH_2)_pB(OH)_2$, in presence of a palladium catalyst, such as $Pd(dppf)_2Cl_2$ and a base. The boronic acids $R^8(CH_2)_pB(OH)_2$ suitable for the coupling reaction are commercially available, or can be easily prepared by the person skilled in the art. Eventually, the Suzuki coupling could be replaced by the person skilled in the art of organic chemistry by an alternative method, such as Humada coupling, Stille coupling and Himaya coupling. To provide the compounds of general formula (**I**) with a pyrrolo[3,2-c]pyridine and with $R^2$= H, the temporary amine protecting group at position 2 of the heterocycle is cleaved.

Following the same route, the pyrrolo[3,2-c]pyridine intermediate **4b*** could be converted to the compounds of general formula (**I**), presenting a pyrrolo[3,2-c]pyridine and wherein $R^4$= H.

The compounds of general formula (I), presenting a imidazo[4,5-c]pyridine core can be obtained by derivatization of intermediate **13*.** Intermediate **13*** can be synthesized according to the synthetic route depicted in **Scheme 3.** Moreover, the person skilled in the art could plan alternative synthetic routes to access intermediate **12*.** Commercially available 4-chloro-3-nitro-pyridine **11*** is converted *via* a 2-steps procedure, including oxidation and chlorination to dichloro-pyridine **12*,** which is further subjected to nucleophilic substitution with $R^2NH_2$, followed by reduction of the nitro group to provide expected intermediate **13*.** Conveniently the amines $R^2NH_2$ involved in the nucleophilic substitution are commercially available or can be easily prepared by the person skilled in the art.

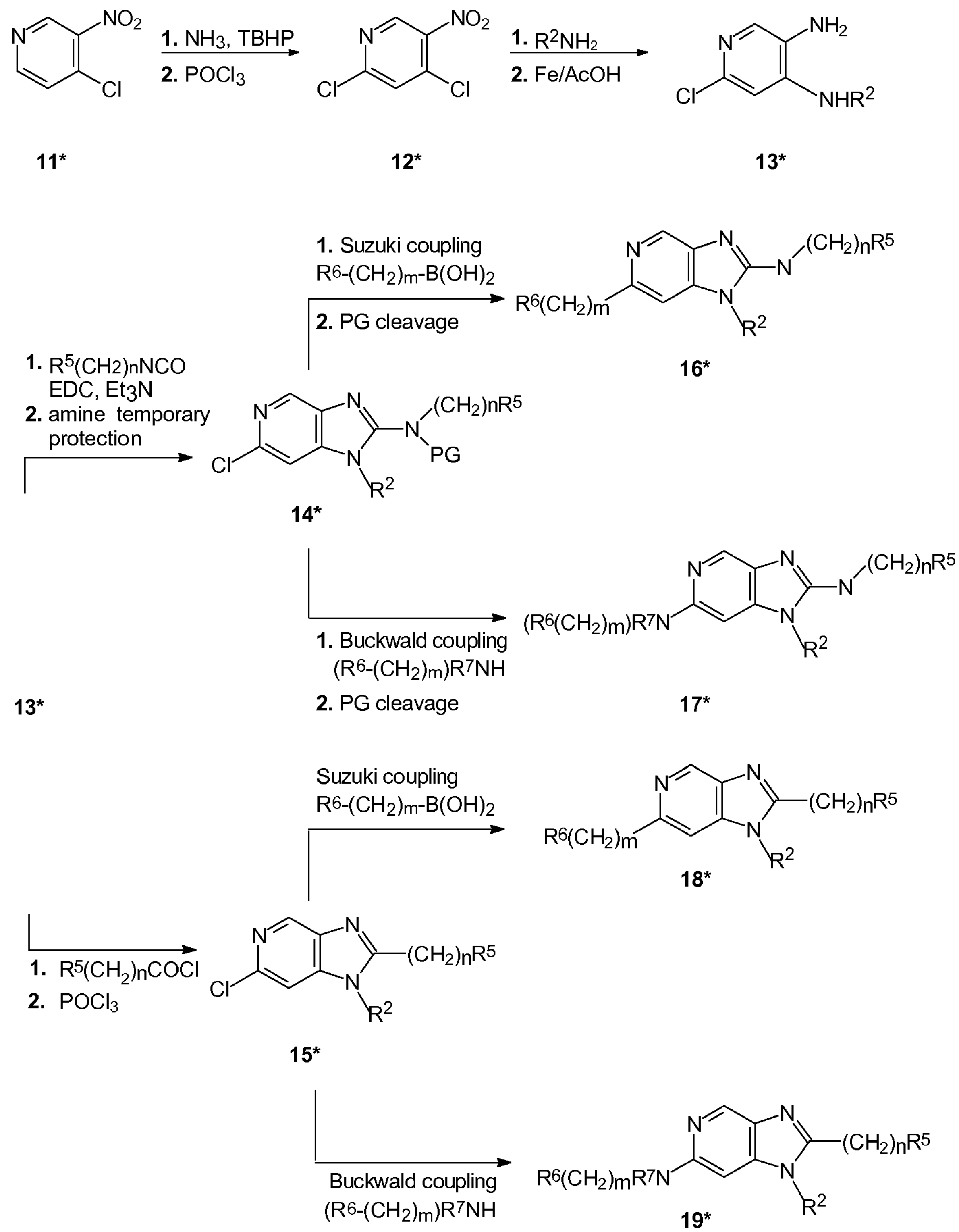


**Scheme 3**: Synthesis of imidazo[4,5-c]pyridine derivatives

Starting from intermediate **13***, the imidazo[4,5-c]pyridine core can be accessed either by treatment with isocyanates of general formula $R^5(CH_2)_nNCO$, followed by cyclization in presence of EDC and $Et_3N$, or by treatment with carboxyl chlorides of general formula $R^5(CH_2)_nCOCl$, followed by cyclization in presence of $POCl_3$. Both methods provide imidazo[4,5-c]pyridine derivatives substituted at position 2 with either an amine, or an alkyl, aryl or alkyl-aryl group. Conveniently, both the isocyanates of general formula $R^5(CH_2)_nNCO$ and the carboxyl chlorides of general formula $R^5(CH_2)$nCOCl useful for the cyclization are commercially available or can be easily prepared by the person skilled in

the art. As previously, chlorinated intermediates **14*** and **15*** can be easily converted to the compounds of general formula (I) presenting a imidazo[4,5-c]pyridine core and wherein $R^4$ = H by applying coupling methods such as Buchwald coupling with amines of general formula ($R^6$-$(CH_2)_m$)$R^7$NH or Suzuki coupling with boronic acids of general formula $R^6(CH_2)_mB(OH)_2$.

An alternative to access compounds of general formula (I) with a imidazo[4,5-c]pyridine core starts from commercially available 2,4-dichloro-3-nitro-pyridine **(Scheme 4).** After fluorination with KF, intermediate **21*** is subjected to nucleophilic substitution, followed by reduction of the nitro group to the corresponding amine. The nucleophilic substitution is performed with 4-methoxybenzyl amine or amines of general formula $R^2NH_2$ and has as a role the introduction on the molecule of the substituent at position 1. Conveniently the 4-methoxybenzyl amine or amines of general formula $R^2NH_2$ are commercially available or can be easily accessed by the person skilled in the art. Moreover, other methods can be envisioned by the person skilled in the art of organic chemistry to introduce the amine moiety on the pyridine heterocycle. The reduction of the nitro group to the corresponding amine can be performed by a variety of reduction methods familiar to the preson skilled in the art including treatment with ammonium formate on a Pd/C catalyst. As previously, treatment with aldehyde $R^1$CHO, followed by Suzuki coupling or amine coupling and when necessary cleavage of the PMB protective group with TFA, provide compounds of general formula (**I**) having a imidazo[4,5-c]pyridine core and a hydrogen as a $R^3$ substituent.

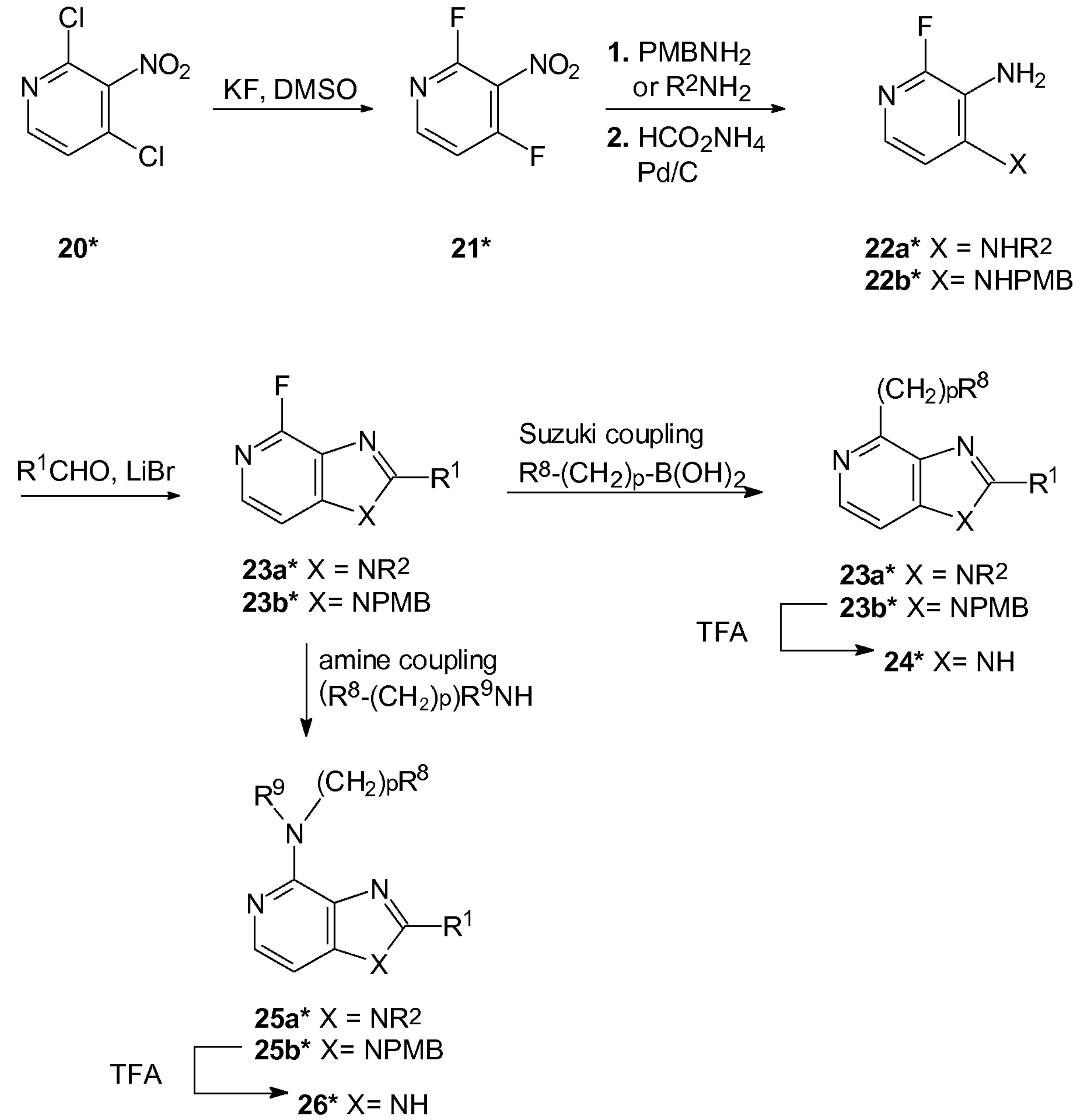


**20*** **21*** **22a*** X = $NHR^2$ **22b*** X= NHPMB

**23a*** X = $NR^2$ **23b*** X= NPMB

**23a*** X = $NR^2$ **23b*** X= NPMB **24*** X= NH

**25a*** X = $NR^2$ **25b*** X= NPMB **26*** X= NH

**Scheme 4:** Synthesis of imidazo[4,5-c]pyridine derivatives

**Indications**

Surprisingly it was found that the above-mentioned compounds of general formulae (**I**) - **(VII),** as well as pharmaceutical compositions comprising said compounds are assumed to be useful for the inhibition of GRK5.

Therefore, one aspect of the present invention is that the compounds according to the general formulae (I) - **(VII)** are suitable for use as inhibitor of GRK5. Inhibitors of GRK5 as used herein are defined as chemical compounds that inhibit the kinase activity of GRK5. GRK5 as used herein refers to the protein having accession number P34947 with UniProtKB/Swiss-Prot. Alternatives names of this protein are: G protein-coupled receptor kinase 5, EC = 2.7.11.16 and G protein-coupled receptor kinase GRK5.G protein-coupled receptor kinase 5.

The inventors used two different screening assays to search for new compounds being inhibitors of GRK5 and showing an effect in glucose dependent regulation of insulin production or release and/or for increase of glucose uptake. It could be determined that compounds of general formulae (I) - **(VII)** are most probably suitable as inhibitors of GRK5 and show an effect in glucose dependent regulation of insulin production or release and/or for increasing glucose uptake.

Different compound libraries with overall around 120000 compounds were tested using a GRK5 based assay (example 2) and an assay screening for effect to insulin production or release (phenotype screen; example 9). The hits of all three assays were further evaluated by investigation of their effects on insulin release and glucose uptake after compound treatment in C2C12 and Beta-TC6 cells.

Thus, the compounds of the present invention can be used for glucose dependent regulation of insulin production and/or release of insulin and/or for increase of glucose uptake. The compounds of general formulae (**I**) - **(VII)** are suitable for the treatment or prophylaxis of a metabolic disease. The present invention refers further to the use of at least one compound of general formulae (I) - **(VII)** for the manufacture of a pharmaceutical formulation for the prophylaxis and treatment of a metabolic disease.

The results of previous experiments of the inventors (cf. WO 2012/028335 A1) as well as prior art (see above) suggest that GRK5 is a suitable target for development of anti-diabetic drugs and active agents against obesity. According to the invention, the inventive compounds or the pharmaceutical composition of the invention can be used in treatment or prophylaxis of a metabolic disease. It is preferred that the metabolic disease according to the invention is selected from the group comprising or consisting of diabetes, obesity and impaired adipogenesis. Especially preferred the disease to be treated is diabetes mellitus type 2.

The compounds of the present invention are suitable for use as pharmaceutically active agent in medicine, particularly in human medicine, but also in veterinary medicine. The compounds of the present invention may be administered in a pharmaceutically effective amount by any suitable route to subjects in need thereof, e.g. parenterally, topically, rectally, nasally, buccally, vaginally, transdermally, by inhalation, by injection or infusion, by spray or via implanted reservoirs. Preferably, the compounds are administered orally or by injection or infusion, e.g. intravenously. For medical purposes, the compounds are preferably formulated as a pharmaceutical composition, which comprises at least one compound as described above, and pharmaceutically acceptable carriers, solvent or excipient. The present invention therefore refers also to pharmaceutical composition of at least one compound of the present invention and further comprising at least a second active agent being an anti-diabetic drug. It is thereby preferred that the anti-diabetic drug is selected from the group comprising or consisting of thiazolidinediones, metformin, sulfonylurea and other anti-diabetic drugs.

Sulfonylurea is preferred selected from the group comprising or consisting of carbutamide, acetohexamide, chlorpropamide, tolbutamide, tolazamide, glipizide, gliclazide, glibenclamide (glyburide), glibornuride, gliquidone, glisoxepide, glyclopyramide and glimepiride. Thiazolidinedione is preferred selected from the group comprising or consisting of rosiglitazone, pioglitazone and troglitazone.

The compounds of the general formulae (**I**) - **(VII)** can also be administered in form of their pharmaceutically active salts optionally using substantially nontoxic pharmaceutically acceptable carrier, excipients, adjuvants or diluents. The medications of the present invention are prepared in a conventional solid or liquid carrier or diluents and a conventional pharmaceutically-made adjuvant at suitable dosage level in a known way. These administratable forms, for example, include pills, tablets, film tablets, coated tablets, capsules, powders and deposits.

Within the disclosed methods the pharmaceutical compositions of the present invention, containing at least one compound of the general formulae (**I**) - **(VII)** or pharmaceutically acceptable salts thereof as an active ingredient, will typically be administered in a mixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The compounds of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semi-solid matrix. "Powders for constitution" refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates

such as magnesium stearate, calcium stearate or potassium stearate; stearic acid; high melting point waxes; and water soluble lubricants such as sodium chloride, sodium benzoate, sodium acetate, sodium oleate, polyethylene glycols and d'l-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Techniques for the formulation and administration of the compounds of the present invention may be found in "Remington's Pharmaceutical Sciences" Mack Publishing Co., Easton PA. A suitable composition comprising at least one compound of the invention and/or pharmaceutically acceptable salts thereof may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of a compound of the general formula (I) refers to that amount of the compound that results in an at least partial inhibition of GRK5. Toxicity and therapeutic efficacy of such compounds can be determined by standard pharmaceutical, pharmacological, and toxicological procedures in cell cultures or experimental animals for determining the LD50 (the dose lethal to 50% of the population) and the ED50 (the dose therapeutically effective in 50% of the population). The dose ratio between toxic and therapeutic effect is the therapeutic index and can be expressed as the ratio between LD50 and ED50. The dosage of the compound lies preferably within a range of circulating concentrations that include the ED50 with little or no toxicity. The actual amount of the composition administered will be dependent on the subject being treated, on the subject's weight, the severity of the affliction, the manner of administration and the judgment of the prescribing physician.

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice. However, those of skill in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## EXAMPLES

### General cell culture techniques:

Cell lines were routinely assayed for mycoplasma contamination and cultured at 95 % air, 5 % $CO_2$ and 37 °C in a Hera-Cell-150 incubator. Before seeding, the cell amount was determined using a Coulter Counter system (Coulter Electronics) and the corresponding cell amount for seeding was calculated. All cells were cultured according to the appropriate recommendations of ATCC protocols.

For analysis using the following assays, cells were seeded at different plate size and suitable cell-density. After 24 h the cells were washed with PBS and fresh media containing DMSO respectively the appropriate treatment with TKIs, peptides or chemicals, was replaced. Starving for insulin dependent examinations was performed in glucose- and FCS-free DMEM media for 4 h.

**Table 1:** Condition for the used cell lines:

| Cell line | Culture media | Initial cell amount 96-/12-/6-well |
|---|---|---|
| 3T3-L1 | DMEM 1.0 g/L glucose, 10 % (v/v) NCS, 2 mM L-glutamine, 1x Pen/Strep | 5x103/5x104/1 x105 |
| Beta TC-6 | DMEM 4.5 g/L glucose, 15 % (v/v) FCS, 2 mM L-glutamine, 1x Pen/Strep | 3x104/3x105/6 x105 |
| C2C12 | DMEM 4.5 g/L glucose, 10 % (v/v) FCS, 2 mM L-glutamine, 1x Pen/Strep | 3x103/3x104/6 x104 |
| RIN-5AH-T2B | RPMI-1640 4.5 g/L glucose, 10 % (v/v) FCS, 2 mM L-glutamine, 1x Pen/Strep | 3x104/3x105/6 x105 |

**Table 2**: Control compounds:

| Compound | Structure | Compound | Structure |
|---|---|---|---|
| **c1** | 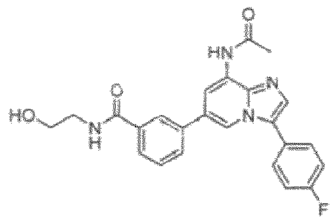 | **c3** | 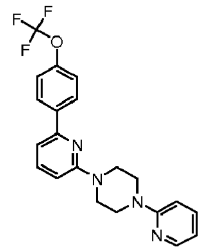 |
| **c2** | 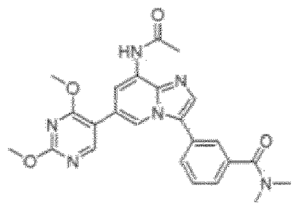 | **c4** | 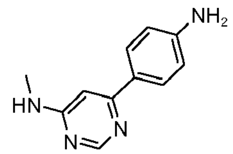 |
| Sunitinib (Sut, Sutent) | 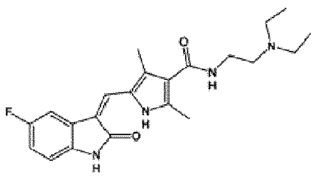 | Akt1 | 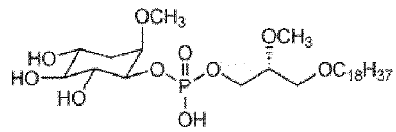 |
| Exendin-4 | His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-$NH_2$ | GLP-1 | His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-$NH_2$ |
| MEK | 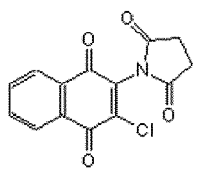 | | |

**Example 1. Cytotoxicity Assays**

Using a MTT (3-(4,5-dimethylthiazol-2-yl)-2,5-diphenyl tetrazolium bromide) approach, the potential citotoxic effect of the compounds of the present invention on the viability and proliferation of the beta-TC6 and C2C12 cells was

determined *in vitro* after a 72 h treatment.

For MTT transformation, 1/5 of total volume of a 5 mg/mL MTT stock solution was added to the cells as well as control wells and incubated at 37 °C, 5 % $CO_2$ (v/v) for 1 h. Then 1/2 of total volume of MTT-stop solution was added and plates were incubated overnight in the dark at 25 °C. The optical density (OD) was measured using a multiwell spectrophotometer at a wavelength of 570 nm.

**Example 2: High throughput screening based on GRK5 kinase activity assay**

Screening for possible inhibitory compounds of GRK5 was performed using the *ADP Glo™ Kinase Assay technology* (Promega) according to the protocol as describe below. As suitable substrate for full length GRK5 casein was identified. Increasing concentrations of GRK5 are incubated together with 100 μM ATP in the absence and presence of 10 μM casein in a reaction buffer. Within this incubation non-phosphorylated casein is converted into phosphorylated casein and ATP is converted into ADP. Thereafter nonconverted ATP is digested into AMP by addition of the ADP-Glo™ Reagent. Subsequently the ADP that is produced by GRK5 activity is phosphorylated back to ATP by addition of Promega Kinase Detection Reagent. These ATP levels serve as measure for the GRK5 activity and are quantified by a luciferase/luciferin reaction. A linear dependence between GRK5 concentration and luminescence signal can be observed. In the absence of casein increasing GRK5 concentration result only in a minor increase of luminescence demonstrating the specificity of the luminescence signal for GRK5 activity.

Km(ATP) was determined by measuring the GRK5 activity at increasing ATP concentrations. Km(ATP) was quantified to be 18.7 μM by fitting the GRK5 activity to the Michaelis Menten equation :

$$v\ (\text{GRK5 activity}) = (\text{Vmax} \times [\text{ATP}]) / (\text{Km(ATP)} + [\text{ATP}])$$

For GRK5 kinase activity assay, the following protocol was used: The assay was performed in: 384 well U bottom, PP, black, low volume (Corning, 3676) assay plates at reaction temperature of 25°C. The reaction buffer used was composed of: 20 mM MES pH 6.0, 1 mM DTT, 10 mM $MgCl_2$, 0.01 % Tween20 and the reaction volume was 6μL.

Firstly 4 μL 6/4 fold concentrated substrate and 6/4 fold concentrated ATP in 1 fold concentrated reaction buffer are added to each well of the assay plate. Subsequently 67 nL 1000 fold concentrated test compound in 100 % DMSO are added to each well except to C- and C+ wells using pin tool. Then 67 nL 100 % DMSO are pipetted to C- (no kinase, no compound) and C+ (no compound) wells using pin-tool and 2 μl reaction buffer are added to C- wells. 2 μl 6/2 fold concentrated full length GRK5 (Millipore, #14-714) in reaction buffer is added to each well except C- wells. After incubation for 120 min. at room temperature 6 μL ADP-Glo™ Reagent (ADP Glo™ Kinase Assay Kit: Promega, V9101) are added to each well to stop the kinase reaction and deplete the unconsumed ATP. After a second incubation for 40 min. at room temperature 12 μlL of Kinase Detection Reagent are added to convert ADP to ATP and start luciferase / luciferin reaction for detection of ATP. The final assay concentrations are 20 nM GRK5, 18.7 μM ATP and 10 μM of the substrate casein. Finally, after incubation for 40 min at room temperature the luminescence intensity was measured.

**Example 3.1: $IC_{50}$ determination of compounds inhibiting GRK5 by ADP Glo™ Kinase Assay technology (Promega)**

In order to determine the $IC_{50}$ values for all compounds ≥ 35 % inhibition, triplicates at 12 different concentrations with a dilution factor of three were used. The maximal compound assay concentration was set to 100 μM, representing the compound assay concentration used in the primary screen. For all assay plates, z-factor values were found to be significantly larger than 0.5, proving statistical relevance of the data.

The activity of the compounds was classified according to $IC_{50}$ for binding sites of Grk5 into the following ranges:

| | | |
|---|---|---|
| $IC_{50}\leq$ | 5 μM | ++++ |
| 5μM< $IC_{50}\leq$ | 20 μM | +++ |
| 20 μM< $IC_{50}\leq$ | 80 μM | ++ |
| 80 μM< $IC_{50}\leq$ | 120 μM | + |

**Example 3.2: $IC_{50}$ determination of compounds inhibiting GRK5 by Millipore KinaseProfiler™**

As alternative, $IC_{50}$ values are determined by a radiometric based filtration binding assay, namely Millipore KinaseProfiler™. GRK5 is incubated with 8 mM MOPS pH7.0, 0.2 mM EDTA, 2 mg/mL casein, 10 mM MgAcetate and [γ-$^{33}$P-APT] (specific activity approx. 500 cpm/pmol, concentration as required). The reaction is initiated by the addition

of the MgATP mix. After incubation for 40 minutes at room temperature, the reaction is stopped by the addition of 3 % phosphoric acid solution. 10 µL of the reaction is then spotted onto a P30 filtermat and washed three times for 5 minutes in 75 mM phosphoric acid and once in methanol prior to drying and scintillation counting.

The activity of the compounds was classified according to $IC_{50}$ for binding sites of GRK5 into the following ranges:

| | | |
|---|---|---|
| $IC_{50} \leq$ | 5 µM | ++++ |
| 5 µM< $IC_{50} \leq$ | 20 µM | +++ |
| 20 µM< $IC_{50} \leq$ | 80 µM | ++ |
| 80 µM< $IC_{50} \leq$ | 120 µM | + |

**Example 4: Release of insulin after treatment with the compounds of the example 2**

After having identified the most promising compounds acting as GRK5 inhibitors and determined the $IC_{50}$ values, the effect of the compounds of the example 2 on the release of insulin by beta-TC6 was determined. Cells were cultured overnight, washed with PBS and then treated with 5 µM of test compounds as well as non-inhibitor controls and Sunitinib as positive control, for 2 h in a high glucose (4.5 g/L) DMEM at 37°C and 5 % (v/v) $CO_2$. Release of insulin was detected using a rat/mouse insulin ELISA.

To order to measure the insulin released by beta-TC6 insulinoma (mouse) cells upon treatment with the compounds of the present invention, beta-TC6 insulinoma (mouse)cells were washed and incubated for 2 h in high (4.5 g/L) or low (1.125 g/L) glucose media at 37 °C. After incubation, the supernatant of the beta-TC6 insulinoma cells was diluted 1:20. The insulin release was measured with rat/mouse insulin ELISA (Merck Millipore Darmstadt, Cat. # EZRMI-13K) by following the manufacture protocol. The enzyme activity of the horseradish peroxidase of the immobilized biotinylated antibodies was monitored spectrophotometrically by the increased absorbency at 490 nm, and corrected by the absorbency at 610 nm. The results are summarized in Table 3 and show that compound 243 has a better effect on the insulin release by beta-TC6 cells than the positive control Sunitinib. Moreover, compound 6 induces a level similar to Sunitinib on the insulin release by beta-TC6 cells.

Table 3: Insulin release by beta-TC6 cells treated with the compounds of the present invention (the % values are in regard to the DMSO control, thus 30% means an increase of insulin release of 30% in regard to the physiological conditions where the insulin release is set at 0%)

| Compound | Insulin release by beta-TC6 cells at 5µM [%] |
|---|---|
| 392 | 30.52% |
| 305 | 20.24% |
| 6 | 81.25% |
| 3 | 15.43% |
| 475 | 48.72% |
| 243 | 142.26% |

**Example 5: Glucose uptake after treatment of C2C12 cells with compounds of the example 2**

To investigate the impact of the GRK5 inhibitors on glucose uptake, the fluorescent D-glucose analog 2-[*N*-(7-nitrobenz-2-oxa-1,3-diazol-4-yl)-amino]-2-deoxy-D-glucose (2-NBDG) was used. 2-NBDG is a fluorescently-labeled deoxyglucose analog that is commonly used to directly monitor glucose uptake by living cells. For 2-NBDG detection, cells were cultured for 1 h together with 5 µM of an inventive compound in glucose free media supplied with 100 µM 2-NBDG at 37 °C before collecting. Furthermore, as internal controls, samples of glucose free media, respectively glucose free media with test compound, were tested. Subsequently, cells were washed, trypsinized and harvested in ice-cold PBS before centrifuged at 1.6 x$10^3$ rpm at 4°C. Cells were analyzed by flow cytometry (FACS Calibur, BD Bioscience, respectively a BD Accuri® C6 Flow Cytometer). For evaluation, cells were gated using the side- and forward scatter SSC/FSC and quantified by excited at 488 nm and collected at 533 nM (FL1). The results are summarized in Table 4.

Table 4: 2-NBDG glucose uptake (%) by cells treated with the compounds of the present application

| Compound | 2-NBDG Glucose Uptake [%] |
|---|---|
| 305 | 50.81% |
| 6 | 45.35% |
| 3 | 38.51% |
| 137 | 69.73% |
| 243 | 48.87% |

**Example 6: Glucose mediated effect on insulin release by treatment of beta-TC6 cells with compounds of the example 2**

Hypoglycemia can cause impairment of cognitive function, motoric control or even consciousness. For safety reasons, it is important that the inventive compounds are glucose dependent and do not enhance the insulin release in low glucose environment. Therefore, the glucose dependent insulin release in beta-TC6 cells using media with 1.125 mM (20 mg/dL) glucose was verified. Cells were cultured for 24 h in a 96-well plate, washed with PBS and then appropriate media was replaced for 2 h at 37°C and 5 % (v/v) $CO_2$. Afterwards, the supernatant was used to detect the amount of insulin released in a rat/mouse insulin ELISA (Merck Millipore Darmstadt, Cat. # EZRMI-13K) by following the manufacture protocol. The enzyme activity of the horseradish peroxidase of the immobilized biotinylated antibodies was monitored spectrophotometrically by the increased absorbency at 490 nm, and corrected by the absorbency at 610 nm.

Treatment of beta-TC6 cells with compounds of the invention did not lead to increased insulin release like observed in high glucose media. Sunitinib seemed to decrease the insulin release (0.77 $\pm$ 0.03), whereas all the inventive compounds ranged close to the DMSO control.

**Example 7: Evaluation of insulin dependence of the inventive compounds**

To investigate if the 2-NBDG uptake of the inventive compound inhibitors of GRK5 are insulin dependent, 3T3-L pre-adipocytes were differentiated to matured adipocytes. Afterwards, cells were starved for 4 h before they were washed and glucose free media supplied with 100 $\mu$M 2-NBDG, in the presence and absence of 10 $\mu$g/ml insulin the test compound was added for 1 h at 37 °C. Furthermore, as internal controls, samples of glucose free media or glucose free media with compound were measured (data not shown). Fluorescence was analyzed by flow cytometer (FACS Calibur, BD Bioscience, respectively a BD Accuri® C6 Flow Cytometer). For evaluation, cells were gated using the side- and forward scatter SSC/FSC and quantified by excited at 488 and detected at 533 nm (FL1).

**Example 8: Comparison of the influence on insulin release of the inventive compounds and commercially available kinase inhibitors**

Based on literature references, several commercially available inhibitors were compared with compounds according to the invention. For comparison we chose the Akt1-Inhibitor II (Calbiochem #124008), which should lead to decreased insulin release, as well as the Map2K3-Inhibitor II (Calbiochem #444938), Exendin-4 (Sigma #E7144) and GLP-1 (Sigma #G8147), which should lead to an increased insulin release. Cells were cultured for 24 h before treated with the inventive compounds and control inhibitors for 2 h at 37 °C and 5 % (v/v) $CO_2$. Insulin in the supernatant was detected by mouse/rat insulin ELISA.

**Example 9: Insulin HTRF assay principle**

For further screening of compounds derived from compounds described above for their ability to regulate insulin release an insulin HTRF® (Homogeneous Time-Resolved Fluorescence; Cisbio International, France) was used. This assay is based on two antibodies against insulin binding to different epitopes of insulin. One of these antibodies is coupled to Europium (Ab-Eu-Cryptate) and the other one to the fluorophore XL665 (Ab-XL665). If the insulin is secreted into the supernatant of cell culture, both antibodies can bind to insulin and come therefore into close proximity, which allows upon excitation that energy transfer between (FRET) the long-life fluorescent donor Europium (cryptate) and the acceptors XL665. FRET increases proportionally with insulin concentration.

The automated assay protocol is as follows:

On day 1, 15k beta-TC6 cells per well were seeded for 24 or 48 h in 50 μL complete medium. On day 2 or respective on day 3, the medium is removed and cells are washed with 60 μL 1xPBS and 20 μL induction buffer is added using BioTek washer. Subsequently, 10 μL Sunitininb (5 -μM f.c.) or the test compound in induction buffer; 0.5% f.c. DMSO (CyBi-well) is transferred to the cells. The cells are then incubated at 37°C for 2, 3, 4h.

The HTRF assay is carried out using Greiner 384-well 784075 assay plates. First 10 μL supernatant is transferred from the cell plates in HTRF plates (CyBi-well). 10 μL of 0, 1, 2, and 4 ng/ml insulin standard controls in assay medium is used as a control. To each well 10 μL of combined 1:25 Ab-XL665 (Acc) and 1:20 Ab-Eu-Cryptate (WellMate) are added and incubated for 120 min at RT in the dark. Finally HTRF is measured on ViewLux ultra high throughput microplate imager (PerkinElmer).

The evaluation software DataFactoty derives relative activities Ar according to the following equation:

$$Ar = \frac{V - R_{(0\%\,control)}}{R_{(100\%control)} - R_{0\%control)}} \times 100$$

| | |
|---|---|
| Ar | relative activity in % |
| V | Raw data value of test well |
| $R(_{0\%\ control)}$ | Median raw data value of 0% control wells |
| $R_{(100\%\ control)}$ | Median raw data value of 100% control wells |

The activity of the compounds was classified according to their $A_r$ (relative activity) into the following ranges:

| | | |
|---|---|---|
| Ar ≥ | 100 % | ++++ |
| 50 % ≤ $A_r$ < | 100 % | +++ |
| 30 % ≤ Ar < | 50 % | ++ |
| 0 % < $A_r$ < | 30 % | + |

The relative activity values of the compounds of the present application are summarized in Table 5.

**Table 5. $A_r$ (relative activity) values of the inventive compounds**

| Compound | $A_r$ | Compound | $A_r$ |
|---|---|---|---|
| 1 | +++ | 14 | ++ |
| 2 | ++ | 15 | ++ |
| 3 | ++++ | 16 | ++ |
| 4 | ++ | 17 | +++ |
| 5 | + | 18 | +++ |
| 6 | +++ | 19 | +++ |
| 7 | +++ | 20 | +++ |
| 8 | ++ | 21 | +++ |
| 9 | + | 22 | ++++ |
| 10 | +++ | 23 | ++ |
| 11 | ++ | 24 | +++ |
| 12 | ++++ | 25 | + |
| 13 | +++ | 26 | +++ |
| 27 | +++ | 64 | +++ |

(continued)

| Compound | $A_r$ | Compound | $A_r$ |
|---|---|---|---|
| 28 | +++ | 65 | ++++ |
| 29 | ++ | 66 | ++ |
| 30 | +++ | 67 | ++ |
| 31 | ++++ | 68 | +++ |
| 32 | ++ | 69 | ++ |
| 33 | ++ | 70 | ++++ |
| 34 | + | 71 | +++ |
| 35 | + | 72 | ++++ |
| 36 | + | 73 | +++ |
| 37 | ++ | 74 | ++++ |
| 38 | + | 75 | ++++ |
| 39 | +++ | 76 | ++ |
| 40 | ++++ | 77 | +++ |
| 41 | ++++ | 78 | +++ |
| 42 | +++ | 79 | ++ |
| 43 | +++ | 80 | ++++ |
| 44 | ++ | 81 | ++ |
| 45 | ++ | 82 | +++ |
| 46 | +++ | 83 | +++ |
| 47 | +++ | 84 | +++ |
| 48 | +++ | 85 | +++ |
| 49 | +++ | 86 | +++ |
| 50 | +++ | 87 | +++ |
| 51 | ++ | 88 | ++ |
| 52 | ++ | 89 | +++ |
| 53 | +++ | 90 | ++ |
| 54 | +++ | 91 | +++ |
| 55 | +++ | 92 | + |
| 56 | +++ | 93 | +++ |
| 57 | ++ | 94 | ++ |
| 58 | ++ | 95 | +++ |
| 59 | + | 96 | +++ |
| 60 | + | 97 | ++ |
| 61 | + | 98 | ++ |
| 62 | ++++ | 99 | ++ |
| 63 | +++ | 100 | ++++ |
| 101 | +++ | 138 | +++ |
| 102 | ++++ | 139 | +++ |

(continued)

| Compound | $A_r$ | Compound | $A_r$ |
|---|---|---|---|
| 103 | ++ | 140 | ++ |
| 104 | +++ | 141 | +++ |
| 105 | +++ | 142 | +++ |
| 106 | +++ | 143 | ++++ |
| 107 | +++ | 144 | ++++ |
| 108 | +++ | 145 | ++++ |
| 109 | +++ | 146 | ++ |
| 110 | ++++ | 147 | +++ |
| 111 | ++++ | 148 | ++++ |
| 112 | +++ | 149 | ++++ |
| 113 | +++ | 150 | +++ |
| 114 | +++ | 151 | +++ |
| 115 | ++++ | 152 | ++++ |
| 116 | + | 153 | ++ |
| 117 | ++++ | 154 | ++ |
| 118 | ++++ | 155 | +++ |
| 119 | +++ | 156 | ++ |
| 120 | ++++ | 157 | +++ |
| 121 | ++++ | 158 | +++ |
| 122 | ++ | 159 | +++ |
| 123 | ++++ | 160 | +++ |
| 124 | ++++ | 161 | +++ |
| 125 | ++++ | 162 | +++ |
| 126 | +++ | 163 | +++ |
| 127 | +++ | 164 | +++ |
| 128 | +++ | 165 | ++++ |
| 129 | ++++ | 166 | ++ |
| 130 | ++++ | 167 | ++ |
| 131 | ++++ | 168 | ++ |
| 132 | +++ | 169 | ++ |
| 133 | +++ | 170 | +++ |
| 134 | +++ | 171 | +++ |
| 135 | +++ | 172 | +++ |
| 136 | +++ | 173 | ++ |
| 137 | +++ | 174 | +++ |
| 175 | +++ | 212 | ++ |
| 176 | +++ | 213 | +++ |
| 177 | +++ | 214 | +++ |

(continued)

| Compound | $A_r$ | Compound | $A_r$ |
|---|---|---|---|
| 178 | ++ | 215 | +++ |
| 179 | ++ | 216 | +++ |
| 180 | +++ | 217 | + |
| 181 | ++++ | 218 | +++ |
| 182 | +++ | 219 | ++ |
| 183 | +++ | 220 | + |
| 184 | ++ | 221 | ++ |
| 185 | + | 222 | + |
| 186 | ++ | 223 | + |
| 187 | ++ | 224 | + |
| 188 | + | 225 | ++ |
| 189 | ++++ | 226 | +++ |
| 190 | +++ | 227 | ++ |
| 191 | +++ | 228 | +++ |
| 192 | + | 229 | ++++ |
| 193 | +++ | 230 | ++ |
| 194 | + | 231 | ++++ |
| 195 | ++ | 232 | +++ |
| 196 | +++ | 233 | +++ |
| 197 | ++ | 234 | +++ |
| 198 | +++ | 235 | +++ |
| 199 | +++ | 236 | +++ |
| 200 | ++ | 237 | ++++ |
| 201 | ++ | 238 | ++ |
| 202 | ++ | 239 | ++ |
| 203 | ++ | 240 | + |
| 204 | +++ | 241 | + |
| 205 | +++ | 242 | + |
| 206 | +++ | 243 | + |
| 207 | +++ | 244 | +++ |
| 208 | ++ | 245 | +++ |
| 209 | +++ | 246 | ++++ |
| 210 | ++ | 247 | ++++ |
| 211 | ++ | 248 | + |
| 249 | ++++ | 294 | +++ |
| 250 | +++ | 295 | +++ |
| 251 | ++ | 296 | ++ |
| 252 | +++ | 297 | ++ |

(continued)

| Compound | $A_r$ | Compound | $A_r$ |
|---|---|---|---|
| 253 | +++ | 298 | +++ |
| 254 | +++ | 299 | +++ |
| 255 | +++ | 300 | +++ |
| 256 | +++ | 301 | +++ |
| 257 | ++ | 302 | +++ |
| 258 | ++++ | 303 | ++ |
| 259 | +++ | 304 | ++ |
| 260 | +++ | 305 | ++ |
| 261 | + | 306 | +++ |
| 262 | +++ | 307 | +++ |
| 263 | +++ | 308 | +++ |
| 264 | ++ | 309 | ++ |
| 265 | +++ | 310 | +++ |
| 266 | +++ | 311 | +++ |
| 267 | +++ | 312 | +++ |
| 268 | +++ | 313 | ++++ |
| 269 | + | 315 | ++ |
| 270 | +++ | 316 | ++ |
| 271 | +++ | 317 | +++ |
| 272 | +++ | 318 | ++++ |
| 273 | ++ | 319 | +++ |
| 274 | ++ | 320 | ++ |
| 275 | +++ | 321 | ++ |
| 276 | ++ | 322 | ++++ |
| 277 | ++ | 323 | +++ |
| 278 | +++ | 324 | +++ |
| 279 | ++ | 325 | ++ |
| 280 | ++ | 326 | ++ |
| 281 | + | 327 | +++ |
| 282 | ++ | 328 | ++ |
| 283 | ++ | 329 | +++ |
| 284 | +++ | 330 | ++++ |
| 285 | +++ | 331 | +++ |
| 286 | +++ | 332 | ++ |
| 287 | +++ | 333 | +++ |
| 288 | ++ | 334 | ++ |
| 289 | ++ | 335 | + |
| 290 | +++ | 336 | ++ |

(continued)

| Compound | $A_r$ | Compound | $A_r$ |
|---|---|---|---|
| 291 | +++ | 337 | ++ |
| 292 | ++ | 338 | ++ |
| 293 | +++ | 339 | ++ |
| 340 | ++ | 388 | ++ |
| 341 | +++ | 389 | +++ |
| 342 | +++ | 390 | ++ |
| 343 | +++ | 391 | ++ |
| 344 | ++ | 392 | +++ |
| 345 | ++++ | 393 | ++ |
| 346 | +++ | 394 | ++ |
| 347 | +++ | 395 | +++ |
| 348 | ++ | 396 | ++ |
| 349 | ++ | 397 | +++ |
| 350 | ++ | 398 | ++++ |
| 351 | +++ | 399 | ++ |
| 352 | ++++ | 400 | ++ |
| 353 | +++ | 401 | ++ |
| 354 | +++ | 402 | ++ |
| 355 | ++ | 403 | ++ |
| 356 | ++++ | 404 | ++++ |
| 357 | ++ | 405 | ++ |
| 358 | +++ | 406 | +++ |
| 359 | ++ | 407 | +++ |
| 360 | + | 408 | + |
| 361 | ++ | 409 | +++ |
| 362 | +++ | 410 | ++ |
| 363 | ++ | 411 | +++ |
| 364 | +++ | 412 | ++ |
| 365 | ++ | 413 | ++ |
| 366 | ++ | 414 | +++ |
| 367 | ++ | 415 | +++ |
| 368 | ++ | 416 | ++ |
| 369 | ++++ | 417 | ++ |
| 370 | ++ | 418 | +++ |
| 371 | ++ | 419 | +++ |
| 372 | +++ | 420 | ++ |
| 373 | + | 421 | +++ |
| 374 | +++ | 422 | ++ |

(continued)

| Compound | $A_r$ | Compound | $A_r$ |
|---|---|---|---|
| 375 | ++ | 423 | ++++ |
| 376 | ++ | 424 | ++ |
| 377 | ++ | 425 | +++ |
| 378 | +++ | 426 | +++ |
| 379 | +++ | 427 | +++ |
| 380 | ++ | 428 | ++ |
| 381 | +++ | 429 | + |
| 382 | ++ | 430 | +++ |
| 383 | +++ | 431 | +++ |
| 384 | ++ | 432 | ++++ |
| 385 | ++++ | 433 | ++ |
| 386 | +++ | 434 | ++ |
| 387 | ++ | 435 | +++ |
| 436 | +++ | 485 | +++ |
| 437 | ++ | 486 | ++ |
| 438 | ++ | 487 | +++ |
| 439 | ++ | 488 | +++ |
| 440 | ++ | 489 | +++ |
| 441 | ++ | 490 | +++ |
| 442 | ++ | 491 | +++ |
| 443 | ++ | 492 | ++ |
| 444 | ++ | 493 | +++ |
| 445 | ++ | 494 | ++ |
| 446 | ++ | 495 | ++ |
| 447 | +++ | 496 | +++ |
| 448 | +++ | 497 | +++ |
| 449 | +++ | 498 | ++ |
| 450 | ++ | 499 | +++ |
| 451 | + | 500 | +++ |
| 452 | ++ | 501 | ++++ |
| 453 | ++ | 502 | +++ |
| 454 | +++ | 503 | +++ |
| 455 | + | 504 | +++ |
| 457 | ++ | 505 | +++ |
| 458 | +++ | 506 | +++ |
| 459 | + | 507 | ++ |
| 460 | + | 508 | +++ |
| 461 | + | 509 | +++ |

(continued)

| Compound | $A_r$ | Compound | $A_r$ |
|---|---|---|---|
| 462 | + | 510 | ++ |
| 463 | ++ | 511 | ++ |
| 464 | ++ | 512 | +++ |
| 465 | + | 513 | +++ |
| 466 | +++ | 514 | ++ |
| 467 | ++++ | 515 | +++ |
| 468 | ++ | 516 | +++ |
| 469 | ++++ | 517 | +++ |
| 470 | ++ | 518 | +++ |
| 471 | + | 519 | +++ |
| 472 | +++ | 520 | +++ |
| 473 | ++++ | 521 | ++++ |
| 474 | ++ | 522 | ++ |
| 475 | +++ | 523 | +++ |
| 476 | + | 524 | +++ |
| 477 | ++ | 525 | ++ |
| 478 | +++ | 526 | +++ |
| 479 | ++++ | 527 | +++ |
| 480 | ++ | 528 | +++ |
| 481 | ++ | 529 | ++ |
| 482 | ++ | 530 | ++ |
| 483 | +++ | 531 | ++ |
| 484 | ++ | 532 | +++ |
| 533 | +++ | 581 | ++ |
| 534 | +++ | 582 | ++ |
| 535 | +++ | 583 | +++ |
| 536 | +++ | 584 | ++ |
| 537 | +++ | 585 | +++ |
| 538 | +++ | 586 | ++ |
| 539 | ++ | 587 | +++ |
| 540 | ++ | 588 | ++ |
| 541 | +++ | 589 | +++ |
| 542 | ++ | 590 | +++ |
| 543 | ++ | 591 | ++ |
| 544 | +++ | 592 | +++ |
| 545 | ++ | 593 | ++ |
| 546 | ++ | 594 | ++ |
| 547 | ++ | 595 | ++ |

(continued)

| Compound | $A_r$ | Compound | $A_r$ |
|---|---|---|---|
| 548 | ++ | 596 | +++ |
| 549 | ++ | 597 | +++ |
| 550 | ++ | 598 | ++ |
| 551 | +++ | 599 | +++ |
| 552 | ++ | 600 | +++ |
| 553 | ++ | 601 | +++ |
| 554 | +++ | 602 | ++ |
| 555 | ++ | 603 | +++ |
| 556 | ++ | 604 | ++ |
| 557 | +++ | 605 | +++ |
| 558 | +++ | 606 | +++ |
| 559 | +++ | 607 | +++ |
| 560 | +++ | 608 | ++ |
| 561 | ++++ | 609 | +++ |
| 562 | ++ | 610 | +++ |
| 563 | +++ | 611 | ++ |
| 564 | +++ | 612 | +++ |
| 565 | +++ | 613 | +++ |
| 566 | ++ | 614 | +++ |
| 567 | +++ | 615 | ++++ |
| 568 | +++ | 616 | +++ |
| 569 | ++ | 617 | +++ |
| 570 | ++ | 618 | +++ |
| 571 | +++ | 619 | +++ |
| 572 | +++ | 620 | ++++ |
| 573 | ++ | 621 | +++ |
| 574 | +++ | 622 | +++ |
| 575 | +++ | 623 | ++ |
| 576 | +++ | 624 | ++ |
| 577 | +++ | 625 | ++++ |
| 578 | +++ | 626 | ++ |
| 579 | ++ | 627 | +++ |
| 580 | ++ | 628 | ++ |
| 629 | ++ | 677 | +++ |
| 630 | +++ | 678 | ++++ |
| 631 | ++++ | 679 | +++ |
| 632 | +++ | 680 | +++ |
| 633 | +++ | 681 | +++ |

(continued)

| Compound | $A_r$ | Compound | $A_r$ |
|---|---|---|---|
| 634 | +++ | 682 | +++ |
| 635 | ++ | 683 | ++ |
| 636 | +++ | 684 | ++ |
| 637 | ++ | 685 | +++ |
| 638 | +++ | 686 | ++ |
| 639 | +++ | 687 | +++ |
| 640 | ++ | 688 | +++ |
| 641 | ++ | 689 | +++ |
| 642 | +++ | 690 | ++++ |
| 643 | ++ | 691 | ++ |
| 644 | +++ | 692 | ++ |
| 645 | ++ | 693 | +++ |
| 646 | +++ | 694 | +++ |
| 647 | +++ | 695 | +++ |
| 648 | ++ | 696 | ++ |
| 649 | +++ | 697 | +++ |
| 650 | ++ | 698 | ++ |
| 651 | +++ | 699 | +++ |
| 652 | +++ | 700 | +++ |
| 653 | +++ | 701 | +++ |
| 654 | ++++ | 702 | +++ |
| 655 | +++ | 703 | ++ |
| 656 | ++ | 704 | ++ |
| 657 | ++ | 705 | ++ |
| 658 | +++ | 706 | +++ |
| 659 | ++ | 707 | ++++ |
| 660 | +++ | 708 | ++++ |
| 661 | +++ | 709 | +++ |
| 662 | +++ | 710 | ++ |
| 663 | +++ | 711 | +++ |
| 664 | +++ | 712 | +++ |
| 665 | +++ | 713 | +++ |
| 666 | ++ | 714 | +++ |
| 667 | ++ | 715 | ++ |
| 668 | +++ | 716 | +++ |
| 669 | ++ | 717 | +++ |
| 670 | ++ | 718 | +++ |
| 671 | ++ | 719 | ++ |

(continued)

| Compound | $A_r$ | Compound | $A_r$ |
|---|---|---|---|
| 672 | ++ | 720 | ++ |
| 673 | +++ | 721 | +++ |
| 674 | ++ | 722 | ++ |
| 675 | +++ | 723 | ++++ |
| 676 | ++ | 724 | ++ |
| 725 | ++ | 773 | + |
| 726 | +++ | 774 | + |
| 727 | +++ | 775 | + |
| 728 | +++ | 776 | + |
| 729 | ++ | 777 | + |
| 730 | +++ | 778 | + |
| 731 | +++ | 779 | + |
| 732 | ++ | 781 | +++ |
| 733 | ++ | 782 | + |
| 734 | ++ | 783 | + |
| 735 | ++ | 784 | + |
| 736 | +++ | 785 | + |
| 737 | +++ | 786 | + |
| 738 | ++ | 787 | ++ |
| 739 | ++ | 788 | ++ |
| 740 | +++ | 789 | ++ |
| 741 | +++ | 790 | ++ |
| 742 | ++ | 791 | + |
| 743 | ++ | 792 | ++ |
| 744 | ++ | 793 | + |
| 745 | ++ | 794 | + |
| 746 | + | 795 | +++ |
| 747 | + | 796 | +++ |
| 748 | + | 797 | ++ |
| 749 | + | 798 | ++ |
| 750 | + | 799 | + |
| 751 | + | 800 | +++ |
| 752 | + | 801 | ++ |
| 753 | + | 802 | +++ |
| 754 | + | 803 | ++ |
| 755 | + | 804 | ++ |
| 756 | + | 805 | ++ |
| 757 | + | 806 | ++ |

(continued)

| Compound | $A_r$ | Compound | $A_r$ |
|---|---|---|---|
| 758 | + | 807 | ++ |
| 759 | + | 808 | ++ |
| 760 | + | 809 | +++ |
| 761 | + | 810 | ++ |
| 762 | + | 811 | ++ |
| 763 | + | 812 | ++ |
| 764 | + | 813 | +++ |
| 765 | + | 814 | + |
| 766 | + | 815 | +++ |
| 767 | + | 816 | + |
| 768 | + | 817 | ++ |
| 769 | + | 818 | +++ |
| 770 | + | 819 | ++ |
| 771 | + | 820 | ++ |
| 772 | + | 821 | ++ |
| 822 | + | 856 | ++ |
| 823 | ++ | 857 | + |
| 824 | ++ | 858 | + |
| 825 | + | 859 | ++ |
| 826 | ++ | 860 | +++ |
| 827 | +++ | 861 | +++ |
| 828 | ++ | 862 | + |
| 829 | +++ | 863 | +++ |
| 830 | ++ | 864 | ++++ |
| 831 | + | 865 | ++ |
| 832 | +++ | 866 | ++ |
| 833 | +++ | 867 | +++ |
| 834 | +++ | 868 | + |
| 835 | +++ | 869 | ++ |
| 836 | ++ | 870 | ++ |
| 837 | ++++ | 871 | + |
| 838 | + | 872 | +++ |
| 839 | ++ | 873 | ++ |
| 840 | ++ | 874 | +++ |
| 841 | ++ | 875 | ++ |
| 842 | ++ | 876 | ++ |
| 843 | ++++ | 877 | ++ |
| 844 | +++ | 878 | ++ |

(continued)

| Compound | $A_r$ | Compound | $A_r$ |
|---|---|---|---|
| 845 | ++++ | 879 | ++ |
| 846 | ++ | 880 | ++ |
| 847 | ++ | 881 | + |
| 848 | + | 882 | +++ |
| 849 | ++ | 883 | ++++ |
| 850 | ++ | 884 | ++ |
| 851 | ++ | 885 | + |
| 852 | ++ | 886 | ++ |
| 853 | +++ | 887 | ++ |
| 854 | +++ | 888 | ++ |
| 855 | ++ | 889 | +++ |
| N.D. (not determined) | | | |

**General chemical procedures**

All reagents were commercial grade and were used as received without further purification, unless otherwise specified. Reagent grade solvents were used in all other cases, unless otherwise specified, respectively. Silica gel generally used for column chromatography was Fisher silica gel (60A, 35-70 micron). Thin layer chromatography was carried out using pre-coated silica gel F-254 plates (thickness 0.25 mm). $^1$H-NMR spectra were recorded on a Bruker AM400 (400 MHz) spectrometer. The chemical shifts are expressed in ppm using the residual solvent as internal standard. Splitting patterns are designated as s (singlet), d (doublet), dd (double-doublet), t (triplet), q (quartet), quint (quintet), m (multiplet), and bs (broad singlet). Electrospray MS spectra were obtained on a Micromass platform LC-MS spectrometer (Column: Sunfire C18, 100A, 3.5 $\mu$m, 4.6 mm x 50 mm; gradient MeCN/$H_2O$: 5% MeCN (0.5 min), 5% to 95% MeCN (2.5 min), 95% MeCN (1.5 min); flowrate: 2.0 mL/min).

**Example 10: General synthetic procedure of compound (II)**

10.1 Synthesis of 2-chloro-5-iodo-pyridin-4-ylamine **2b***

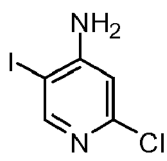

**2b***

2-Chloro-pyridin-4-ylamine **1b*** (3.2 g, 0.025 mol) and sodium acetate (4.1 g, 0.05 mol) were stirred in acetic acid (20 mL). A solution of iodine monochloride (4.1 g, 0.025 mol) in acetic acid (10 mL) was added and the reaction mixture was heated to 70 °C for approximately 3 h (NB: solution at ~50 °C, brown colour faded and precipitate formed as the reaction proceeded). The reaction mixture was cooled to room temperature, then poured onto water (700 mL), and extracted with EtOAc. The organic layer was carefully washed with a solution of $Na_2CO_3$ followed by a solution of $Na_2S_2O_3$, dried over $MgSO_4$ and concentrated *in vacuo.* The crude product was purified by column chromatography using 10 % EtOAc in DCM to yield 2-chloro-5-iodo-pyridin-4-ylamine **2b*** (2.60 g, 40 %). $^1$H NMR (400 MHz, $CDCl_3$) $\delta$(ppm): 8.34 (1 H, s), 6.63 (1 H, s), 4.78 (2 H, s).

10.2 Synthesis of *N*-(2-chloro-5-iodo-pyridin-4-yl)-methanesulfonamide **3b***

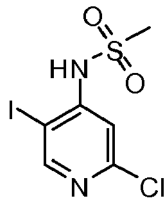

**3b***

2-Chloro-5-iodo-pyridin-4-ylamine **2b*** (5.1 g, 0.02 mol) and triethylamine (12.4 mL, 0.10 mol) were stirred in DCM (40 ml) and cooled in an ice water bath. Methanesulphonyl chloride (7.7 mL, 0.10 mol) in DCM (20 mL) was added dropwise and the reaction stirred at room temperature for 2 h. The reaction mixture was washed with water, dried over $MgSO_4$ and concentrated *in vacuo.* The crude product was purified by column chromatography using 5 % EtOAc in DCM to yield *N,N*-(2-chloro-5-iodo-pyridin-4-yl)-di-methylsulfonamide (4.87 g, 60 %). $^1$H NMR (400 MHz, $CDCl_3$) δ(ppm): 8.94-8.81 (1 H, m), 7.37 (1 H, t, J = 9.47 Hz), 3.56 (6 H, t, J = 9.37 Hz).
*N,N*-(2-chloro-5-iodo-pyridin-4-yl)-di-methylsulfonamide (4.87 g, 0.012 mol) and aqueous NaOH solution (10% solution, 25 ml) was stirred in THF (25 mL) at room temperature for 16 h. The reaction was concentrated *in vacuo* and the aqueous residue acidified to pH 4 using aqueous citric acid solution. The resulting precipitate was collected by filtration and dried to yield N-(2-chloro-5-iodo-pyridin-4-yl)-methanesulfonamide **3b*** (3.69 g, 93 %). $^1$H NMR (400 MHz, $CDCl_3$) δ(ppm): 8.59 (1 H, s), 7.54 (1 H, s), 7.08 (1 H, s), 3.20 (3 H, s).

10.3 Synthesis of 2-chloro-3-iodo-pyridin-4-ylamine **2a***

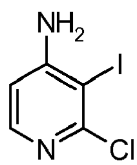

**2a***

2-Chloro-pyridin-4-ylamine **1a*** (3.2 g, 25 mmol) and sodium acetate (4.1 g, 50 mmol) were stirred in acetic acid (20 mL). A solution of iodine monochloride (4.1 g, 25 mmol) in acetic acid (10 mL) was added and the reaction mixture was heated to 70 °C for approximately three h (NB: reaction mixture became a solution at ~50 °C, the brown colour faded and a precipitate formed as the reaction proceeded). The reaction mixture was cooled to room temperature, then poured onto water (700 mL), and extracted with AcOEt (2 x 750 mL). The organic phase was carefully washed with a saturated aqueous solution of sodium carbonate (500 mL) followed by a 10% solution of sodium thiosulfate (500 mL), dried over $MgSO_4$ and concentrated *in vacuo.* The crude product was purified by column chromatography using 10% AcOEt in DCM to yield 2-chloro-3-iodo-pyridin-4-ylamine **2a*** (2.6 g, 41 %). $^1$H NMR (400 MHz, $CDCl_3$) δ (ppm): 7.88 (1 H, d, *J* = 5.5 Hz), 6.46 (1 H, d, *J* = 5.5 Hz), 4.96 (2 H, s).

10.4 Synthesis of *N*-(2-chloro-3-iodo-pyridin-4-yl)methanesulfonamide **3a***

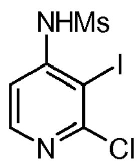

**3a***

2-Chloro-3-iodo-pyridin-4-ylamine **2a*** (5.1 g, 20 mmol) and triethylamine (14 mL, 100 mmol) were stirred in DCM (100 mL) and cooled in an ice water bath. Methanesulphonyl chloride (7.7 mL, 100 mmol) was added dropwise and stirred at room temperature for two h. The reaction mixture was washed with water (100 mL), dried over $MgSO_4$ and concentrated *in vacuo.* The crude product was purified by column chromatography using 1% AcOEt in DCM to yield *N*-methanesulfonamide-N-(2-chloro-3-iodo-pyridin-4-yl)-methanesulfonamide (4.2 g, 51 %). $^1$H NMR (400 MHz, DMSO) $\delta$(ppm): 8.53 (1 H, d, *J* = 5.1 Hz), 7.74 (1 H, d, *J* = 5.1 Hz), 3.70 (6 H, s).

*N*-methanesulfonamide-*N*-(2-chloro-3-iodo-pyridin-4-yl)-methanesulfonamide (4.2g, 10 mmol) and aqueous NaOH solution (10% solution, 25 mL) was stirred in THF (25 mL) at room temperature for 16 h. The reaction mixture was concentrated *in vacuo* and the aqueous residue acidified to pH 4 using an aqueous solution of citric acid. The resulting precipitate was collected by filtration and dried to yield *N*-(2-chloro-3-iodo-pyridin-4-yl)-methanesulfonamide 3a* (3.1 g, 92%). $^1$H NMR (400 Mhz, DMSO) $\delta$(ppm): 9.56 (1 H, s), 8.16 (1 H, d, *J* = 5.5 Hz), 7.30 (1 H, d, *J* = 5.5 Hz), 3.20 (3 H, s).

10.5 Synthesis of pyrrolo[3,2-c]pyridine intermediates

Synthesis of 6-chloro-2-$R^1$-1*H*-pyrrolo[3,2-c]pyridine **4b***

*N*-(2-chloro-5-iodo-pyridin-4-yl)-methanesulfonamide **3b*** (1.82 g, 5.5 mmol), $R^1$-ethylnyl (8.2 mmol), bis(triphenylphosphine)palladium(II) dichloride (0.18 g, 0.2 mmol), copper(I) iodide (0.04 g, 0.2 mmol) and triethylamine (3.1 mL, 25.0 mmol) in DMF (15 mL) were stirred under nitrogen and heated to 100 °C for 1-2 h (reaction monitored by LC-MS). The reaction was cooled to 50 °C and DBU (1.6 mL, 10.7 mmol) added, the reaction stirred at 50 °C for 30 minutes, then cooled to room temperature. The reaction mixture was then diluted with aqueous $NH_4Cl$ solution and EtOAc used to extract the product. The organic layer was dried over $MgSO_4$ and concentrated *in vacuo.* The residue was treated with water and $Et_2O$, and the resulting precipitate was collected by filtration and dried to yield 6-chloro-2-$R^1$-1*H*-pyrrolo[3,2-c]pyridine **4b*.**

Typical example: 6-chloro-2-phenyl-1*H*-pyrrolo[3,2-c]pyridine:

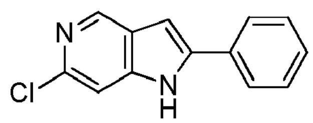

(0.72 g, 3.1 mmol, 57 %). $^1$H NMR (400 MHz, DMSO) $\delta$(ppm): 12.20 (1 H, s), 8.60 (1 H, s), 7.88 (2 H, d, *J* = 7.74 Hz), 7.52-7.44 (2 H, m), 7.40-7.33 (2 H, m), 7.06 (1 H, s).

Synthesis of 4-chloro-2-$R^1$-1H-pyrrolo[3,2-c]pyridine **4a***

N-(2-chloro-3-iodo-pyridin-4-yl)methanesulfonamide **3a*** (1.7 g, 5.0 mmol), $R^1$-ethynyl (5.5 mmol), bis(triphenylphosphine)palladium(II) dichloride (0.18 g, 0.2 mmol), copper(I) iodide (0.04 g, 0.2 mmol) and triethylamine (3.1 mL, 14 mmol) in dimethylformamide (15 mL) were stirred under nitrogen and heated to 100 oC for 1-2 hours (reaction monitored by LC-MS). The reaction was cooled to 50 oC and DBU (0.6 mL, 4 mmol) was added and the mixture was stirred at 50 oC for 30 minutes, then cooled to room temperature. The reaction mixture was diluted with an aqueous ammonium chloride solution (100 mL) and extracted with ethyl acetate (2 x 100 mL) and the combined organic extracts were dried over $MgSO_4$ and concentrated in vacuo*.* The residue was suspended in water and diethyl ether, the solid was collected by filtration and dried to yield 4-chloro-2-$R^1$-1H-pyrrolo[3,2-c]pyridine **4a*.**

Typical example: 4-chloro-2-phenyl-1 H-pyrrolo[3,2-c]pyridine.

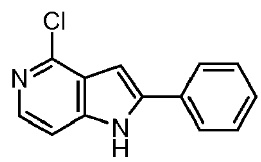

(0.46 g, 40%).$^{1}$H NMR (400 MHz, DMSO) δ (ppm): 12.39 (1 H, s), 8.04-7.92 (3 H, m), 7.55-7.36 (4 H, m), 7.05 (1 H, d, *J* = 2.1 Hz).

10.6 Protection of 1*H*-pyrrolo[3,2-c]pyridine intermediates **4a*** and **4b***

Typical example: Synthesis of 6-chloro-1-methyl-2-phenyl-1*H*-pyrrolo[3,2-*c*]pyridine

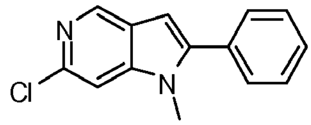

6-Chloro-2-phenyl-1*H*-pyrrolo[3,2-c]pyridine (5.25 g, 23.0 mmol) was stirred in DMF (100 mL), sodium hydride (0.97 g, 24.1 mmol) was added and the reaction stirred for 30 minutes after evolution of hydrogen had ceased. Methyl iodide (3.3 g, 23.0 mmol) was added and the reaction was stirred at room temperature for 30 minutes. The reaction was poured over water (500 mL) stirred for 30 minutes, and the resulting precipitate collected by filtration and dried. The crude product was purified by column chromatography using 10 % EtOAc in DCM to yield 6-chloro-1-methyl-2-phenyl-1*H*-pyrrolo[3,2-c]pyridine (3.79 g, 15.6 mmol, 68 %). $^{1}$H NMR (400 MHz, $CDCl_3$) δ(ppm): 8.65 (1 H, d, *J* = 0.86 Hz), 7.51-7.44 (5 H, m), 7.29 (1 H, s), 6.60 (1 H, d, *J* = 0.91 Hz), 3.71 (3 H, s).

Typical example: Synthesis of 4-(1-benzyl-6-chloro-1*H*-pyrrolo[3,2-c]pyridin-2-yl)-*N,N*-dimethyl-benzamide

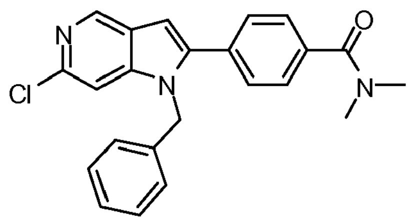

4-(6-chloro-1H-pyrrolo[3,2-c]pyridin-2-yl)-*N,N*-dimethyl-benzamide (12.0 g, 40.0 mmol) was stirred in DMF (160 mL), sodium hydride (1.6 g, 40.0 mmol) was added and the reaction stirred for 30 minutes after evolution of hydrogen had ceased. Benzyl bromide (6.8 g, 40.0 mmol) was added and the reaction was stirred at room temperature for 3 h. DMF was removed *in vacuo* and the residue was poured over water (500 mL) and the product extracted with EtOAc. The organic phases were dried over $MgSO_4$ and concentrated *in vacuo.* The crude product was purified by column chromatography using 50 % EtOAc in DCM to yield 4-(1-benzyl-6-chloro-1*H*-pyrrolo[3,2-c]pyridin-2-yl)-*N,N*-dimethyl-benzamide (4.6 g, 12.0 mmol, 30 %).$^{1}$H NMR (400 MHz, DMSO) δ(ppm): 8.69 (1 H, s), 7.65 (1 H, s), 7.54 (2 H, d, *J* = 8.06 Hz), 7.46 (2 H, d, *J* = 8.06 Hz), 7.23-7.13 (3 H, m), 6.87 (1 H, d, *J* = 0.85 Hz), 6.82 (2 H, d, *J* = 7.21 Hz), 5.53 (2 H, s), 2.97 (3 H, s), 2.89 (3 H, s).

Typical example: Synthesis of 4-chloro-1-methyl-2-phenyl-1*H*-pyrrolo[3,2-c]pyridine

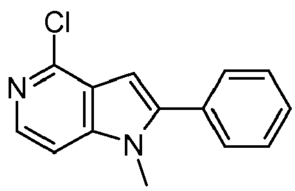

4-chloro-2-phenyl-1*H*-pyrrolo[3,2-c]pyridine (0.46 g, 2.0 mmol) was stirred in DMF (9 mL) then sodium hydride (0.08 g, 2.0 mmol) was added and the suspension was stirred for a further 30 minutes after evolution of hydrogen had ceased.

Methyl iodide (0.13 mL, 2.0 mmol) was added and the reaction was stirred at room temperature for 30 minutes. After this time, the mixture was poured onto water (25 mL) stirred for 30 minutes and the resulting precipitate was collected by filtration and dried. The crude product was purified by column chromatography using 10% AcOEt in DCM to yield 4-chloro-1-methyl-2-phenyl-1H-pyrrolo[3,2-c]pyridine (0.3 g, 62%). $^1H$ NMR (400 MHz, DMSO) δ (ppm): 8.06 (1 H, d, J = 5.8 Hz), 7.69-7.51 (6 H, m), 6.68 (1 H, d, J = 0.83 Hz), 3.80 (3 H, s).

10.7 Temporary protection of 1*H*-pyrrolo[3,2-c]pyridine intermediates **4a*** and **4b***

Typical example: Synthesis of 6-chloro-2-phenyl-1*H*-pyrrolo[3,2-*c*]pyridine-1-carboxylic acid *tert*-butyl ester

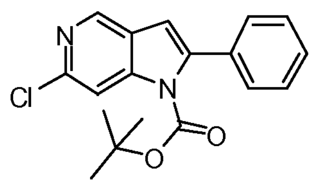

6-Chloro-2-phenyl-1*H*-pyrrolo[3,2-c]pyridine (4.0 g, 17.5 mmol) was suspended in DCM (75 mL), di-*tert*-butyl dicarbonate (3.8 g, 17.5 mmol) in DCM (20 mL) was added, followed by DMAP (0.1eq). After evolution of carbon dioxide had ceased the reaction mixture was absorbed onto silica and purified by column chromatography using 10 % EtOAc in DCM to yield 6-chloro-2-phenyl-1*H*-pyrrolo[3,2-c]pyridine-1-carboxylic acid *tert*-butyl ester (5.0 g, 15.2 mmol, 87%).$^1H$ NMR (400 MHz, $CDCl_3$) δ(ppm): 8.61 (1 H, d, *J* = 0.88 Hz), 8.13 (1 H, s), 7.45-7.37 (5 H, m), 6.58 (1 H, d, *J* = 0.84 Hz), 1.30 (9 H, s).

Typical example: Synthesis of 4-chloro-2-phenyl-1*H*-pyrrolo[3,2-*c*]pyridine-1-carboxylic acid *tert*-butyl ester

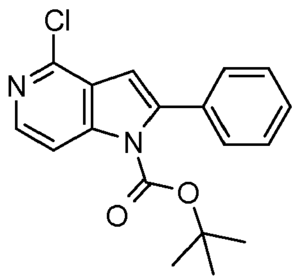

4-Chloro-2-phenyl-1H-pyrrolo[3,2-*c*]pyridine (3.0 g, 13 mmol) was suspended in DCM (25 mL) and di-*tert*-butyl dicarbonate (4.3 g, 20 mmol) in DCM(20 mL) was added, followed by DMAP (10 mol%). After evolution of carbon dioxide had ceased, a TLC indicated starting material was still present. Another portion of di-tert-butyl dicarbonate (4.3 g, 20 mmol) in DCM (20 mL) was added after which rapid gas evolution was again observed and the mixture became a clear solution.

The reaction mixture was absorbed onto silica gel and purified by column chromatography using 10% ethyl acetate in petroleum ether to yield 4-chloro-2-phenyl-1*H*-pyrrolo[3,2-c]pyridine-1-carboxylic acid *tert*-butyl ester (2.6 g, 7.7 mmol, 59%). $^1H$ NMR (400 MHz, $CDCl_3$) δ (ppm): 8.25 (1 H, d, *J* = 5.8 Hz), 8.01 (1 H, dd, *J* = 5.8, 0.8 Hz), 7.42 (5 H, m), 6.68 (1 H, d, *J* = 0.8 Hz), 1.31 (9 H, s).

10.8 **General** procedure for Buchwald-Hartwig reaction

The reaction vessels were charged with a solution of X-Phos (1.0 mL, 0.05 M solution in DCM, 0.05 mmol) then concentrated to dryness. 2-aryl-6-chloro-1-$R^2$-1*H*-pyrrolo[3,2-c]pyridine (1.0 mL, 0.4 M solution in 3 % $H_2O$/NMP, 0.4 mmol) was added, followed by the amine (0.5 mL, 2.4 M solution in 3 % $H_2O$/NMP, 1.2 mmol), $K_3PO_4$ (~190 mg, 0.9 mmol, weighed using a calibrated spatula) and $Pd_2dba_3$ (0.5 mL, 0.036 M solution in 3 % $H_2O$/NMP, 0.018 mmol). The reactions were stirred and heated to 140 °C for 16 h, then cooled to room temperature, diluted with $H_2O$ (4 mL), extracted using EtOAc (4 ml, followed by 2 ml) and concentrated in vacuo. The crude residues were dissolved in DMF and purified by reverse phase preparative HPLC.

Typical___example:___benzo[1,3]dioxol-5-yl-[2-(3-fluoro-phenyl)-1-methyl-1*H*-pyrrolo[3,2-c]pyridin-6-yl]-amine

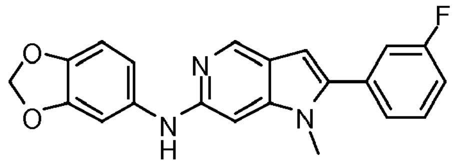

68.4 mg (47 %), $^1$H NMR (400 MHz, DMSO) δ (ppm): 8.58 (1 H, s), 8.48 (1 H, s), 7.56 (1 H, m), 7.49-7.43 (2 H, m), 7.35-7.25 (2 H, m), 6.93 (1 H, dd, *J* = 8.40, 2.16 Hz), 6.82 (1 H, d, *J* = 8.37 Hz), 6.74 (1 H, s), 6.64 (1 H, s), 5.95 (2 H, s), 3.64 (3 H, s).

Typical example: (1-benzyl-2-phenyl-1*H*-pyrrolo[3,2-c]pyridine-6-yl)-(4-morpholin-4-yl-phenyl)-amine

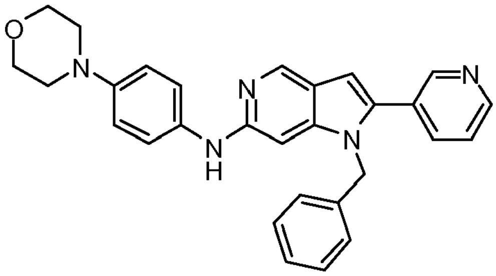

77.0mg (42 %), $^1$H NMR (400 MHz, DMSO) δ (ppm): 8.69 (1 H, d, *J* = 2.25 Hz), 8.59 (1 H, dd, *J* = 4.81, 1.61 Hz), 8.52 (1 H, d, *J* = 0.89 Hz), 8.42 (1 H, s), 7.89-7.85 (1 H, m), 7.50-7.46 (1 H, m), 7.37-7.19 (5 H, m), 6.94 (2 H, d, *J* = 7.49 Hz), 6.84 (2 H, d, *J* = 8.85 Hz), 6.76 (1 H, d, *J* = 0.81 Hz), 6.61 (1 H, s), 5.33 (2 H, s), 3.75-3.71 (4 H, m), 3.02-2.98 (4 H, m).

10.9 **General** procedure for Buchwald-Hartwig and deprotection reaction

The reaction vessels were charged with a solution of X-Phos (1.0 mL, 0.05 M solution in DCM, 0.05 mmol) then concentrated to dryness. 2-aryl-6-chloro-1*H*-pyrrolo[3,2-c]pyridine-1-carboxylic acid *tert*-butyl ester (1.0 mL, 0.3 M solution in 3 % $H_2O$/DMF, 0.3 mmol) was added, followed by the amine (0.38 mL, 2.4 M solution in 3 % $H_2O$/DMF, 0.9 mmol), $K_3PO_4$ (~140 mg, 0.7 mmol, weighed using a calibrated spatula) and $Pd_2dba_3$ (0.5 mL, 0.026 M solution in 3% $H_2O$/DMF, 0.013 mmol). The reactions were stirred and heated to 80 °C for 16 h, then cooled to room temperature, diluted with $H_2O$ (4 mL), extracted using EtOAc (4 mL, followed by 2 mL) and concentrated *in vacuo*. The crude residues were treated with DCM (2 mL) and TFA (1 mL) and left to stand for 16 h. The reactions were then concentrated *in vacuo,* dissolved in DMF and purified by reverse phase preparative HPLC.

Typical example: (2,4-dimethoxy-phenyl)-[2-(3-fluoro-phenyl)-1*H*-pyrrolo[3,2-c]pyridin-6-yl]-amine

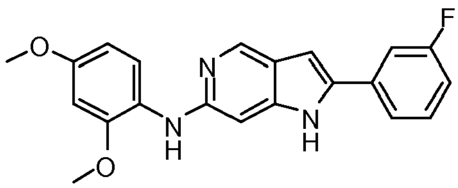

23.8 mg (16 %),$^1$H NMR (400 MHz, DMSO) δ (ppm): 12.32 (1 H, s), 9.04 (1 H, s), 8.54 (1 H, s), 7.83-7.77 (2 H, m), 7.67-7.60 (1 H, m), 7.38-7.30 (2 H, m), 7.26 (1 H, d, *J* = 1.87 Hz), 6.85 (1 H, d, *J* = 2.65 Hz), 6.77 (1 H, s), 6.71 (1 H, dd, *J* = 8.62, 2.66 Hz), 3.88 (6 H, d, *J* = 16.01 Hz).

**Example 11 General synthetic procedure of compound (III)**

11.1 Synthesis of 2,4-difluoro-3-nitropyridine **21***

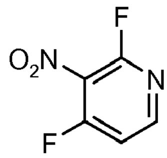

**21***

Potassium fluoride (90 g, 1.55 mol) was taken in a mixture toluene/DMSO (2/1, v/v, 1.35 L). The toluene was then removed by distillation under an inert atmosphere in order to remove any water present. The reaction mixture was cooled to 50 °C and 2,4-dichloro-3-nitropyridine **20*** was added (100 g, 0.52 mol). The mixture was then heated at 120 °C for 30 minutes, cooled to 20 °C, diluted with $Et_2O$ (3 L) and water (3 L). The organic layer was separated, washed with brine (500 mL), dried over anhydrous $Na_2SO_4$ and evaporated to furnish the crude product as a brown viscous liquid, which was used without further purification (55 g, 66%). HPLC: 80%; $^1$H-NMR (400 MHz, $CDCl_3$) $\delta$(ppm): 8.41 (t, *J* = 6.4 Hz, 1 H), 7.26 (m, 1 H).

11.2 Synthesis of 2-fluoro-*N*-(4-methoxybenzyl)pyridine-3,4-diamine **22b***

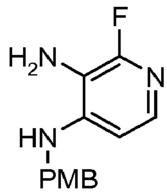

**22b***

A solution of 2,4-difluoro-3-nitropyridine **21*** (100 g, 0.63 mol) and triethylamine (0.63 mol) in THF (1 L) was cooled to -80 °C. After drop-wise addition of a solution of 4-methoxybenzylamine (77.6 g, 0.63 mol) in THF (400 mL), the mixture was stirred at -80 °C for one h, then slowly allowed to warm to room temperature. The reaction mixture was diluted with $Et_2O$ and partitioned with water. The organic layer was separated, washed with brine, dried over anhydrous $Na_2SO_4$, and evaporated to give intermediate protected amine as a yellow solid (101 g, 65%). $^1$H-NMR (400 MHz, $CDCl_3$): 8.24 (br s, 1 H), 7.85 (d, *J* = 6 Hz, 2H), 7.22 (d, *J* = 8.4 Hz, 2H), 6.91 (d, *J* = 3.6 Hz, 2H), 6.64 (d, *J* = 8.4 Hz, 1 H), 4.48 (d, *J* = 5.2 Hz, 2H), 3.82 (s, 1H).

To a suspension of (2-fluoro-3-nitro-pyridin-4-yl)-(4-methoxy-benzyl)amine (100 g, 0.38 mol) in methanol (700 mL) was added ammonium formate (1.13 mol) followed by 10% Pd/C (10 g, 50% $H_2O$) and the mixture was stirred at room temperature for 48 h (monitored by TLC). After this time, the mixture was filtered through celite and washed with methanol. The solvent was evaporated to obtain the crude product, which was purified by silica gel chromatography to afford compound **22b*** as a brown solid (64 g, 68%). $^1$H-NMR (400 MHz, $CDCl_3$): 7.54 (d, *J* = 5.6 Hz, 1 H), 7.27 (d, *J* = 6.8 Hz, 2H), 6.90 (d, *J* = 8.4 Hz, 2H), 6.43 (d, *J* = 5.6 Hz, 1 H), 4.56 (br s, 1 H), 4.32 (d, *J* = 5.2 Hz, 2H), 3.82 (s, 3H), 3.03 (br s, 2H).

11.3 Synthesis of 2-$R^1$-1*H*-imidazo[4,5-c]pyridine intermediates **23a*** and **23b***

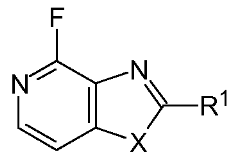

To solution of **22a*** or **22b*** (22 mmol) in dry DMF (or dry NMP); kept under a constant stream of nitrogen and at room temperature; was added the aldehyde $R^1CHO$ (25 mmol) and anhydrous lithium bromide (68 mmol). The mixture was stirred at room temperature for 15 minutes and then heated to 100 to 110 °C for 48 h. After this time, the mixture was cooled to room temperature, poured into ice cold water and extracted with AcOEt. The organic layer was separated, dried over sodium sulfate and evaporated to obtain crude product, which was purified by silica gel (60-120 mesh) chromatography using 17% AcOEt in hexane to afford the pure product.

Typical example: 4-fluoro-2-(3-fluorolphenyl)-1-(4-methoxybenzyl)-1*H*-imidazo[4,5-c]pyridine

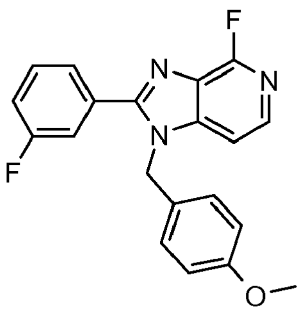

Isolated as a pale yellow pale yellow solid (30%); HPLC: 98.4%; LC-MS: 352 $(M+1)^+$; $^1$H-NMR (400 MHz, $CDCl_3$): 7.95 (d, *J* = 5.6 Hz, 1 H), 7.47 (m, 2H), 7.44 (m, 1 H), 7.23 (m, 1 H), 7.09 (dd, *J* = 2.4 Hz, 1 H), 6.97(d, *J* = 8.4 Hz, 2H), 6.87 (d, *J* = 8.8 Hz, 2H), 5.42 (s, 2H), 3.80 (s, 3H).

Typical example: 2-(2-chlorophenyl)-4-fluoro-1-(4-methoxybenzyl)-1*H*-imidazo[4,5-*c*]pyridine

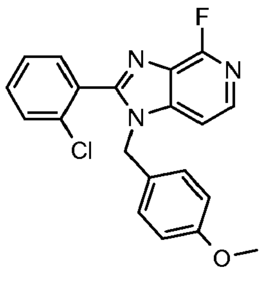

Isolated as a brown oil (25%); LC-MS: 368 $(M+1)^+$; HPLC: 92.2%; $^1$H-NMR (400 MHz, $CDCl_3$): 7.94 (d, J = 5.2 Hz, 1 H), 7.52 (m, 3H), 7.39 (m, 1 H), 7.08 (m, 1 H), 6.88 (d, J = 8.4 Hz, 2H), 6.7 (d, J = 8.8 Hz, 2H), 5.20 (s, 2H), 3.76 (s, 3H).

Typical example: 4-fluoro-2-(2-fluoro-3-methoxyphenyl)-1-(4-methoxybenzyl)-1*H*-imidazo[4,5-c]pyridine

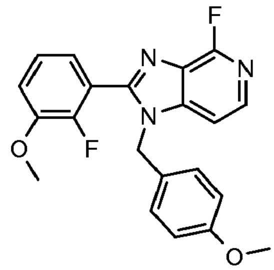

Isolated as an off-white solid (45%); HPLC: 97.7%; LC-MS: 382 $(M+1)^+$; $^1H$-NMR (400 MHz, $CDCl_3$): 7.9 (d, J = 5.6 Hz, 1 H), 7.23 (d, *J* = 8 Hz, 1 H), 7.19 (m, 2H), 7.01 (m, 1 H), 6.93 (d, *J* = 8.4 Hz), 6.79 (d, *J* = 8.4 Hz, 2H), 5.27 (s, 2H), 3.96 (s, 3H), 3.76 (s, 3H).

11.4 General procedure for aliphatic amines coupling

The reaction vessels were charged with the 4-fluoro-2-$R^1$-1-$R^3$-1H-imidazo[4,5-c]pyridine **23a*** or **23b*** (0.45 mmol), aliphatic amine (0.75 mmol) and triethylamine (0.75 mmol). Butanol (2.5 mL) was added and the reactions were heated to 115 °C for 24 h. The reactions were cooled to room temperature, NaOH solution (0.5 M, 2 mL) was added and the crude product extracted using AcOEt (5 mL). Organic phases were evaporated to yield the crude product. For final compounds the crude product was dissolved in DMF and purified by reverse phase preparative HPLC.

11.5 General procedure for aromatic amines coupling

The reaction vessels were charged with the 4-fluoro-2-$R^1$-1-$R^3$-1*H*-imidazo[4,5-c]pyridine **23a*** or **23b*** (0.45 mmol), aromatic amine (0.75 mmol) and a concentrated solution of HCl (0.75 mmol). Butanol (2.5 mL) was added and the reactions were heated to 115 °C for 24 h. The reactions were cooled to room temperature, NaOH solution (0.5 M, 2 mL) was added and the crude product was extracted using AcOEt (5 mL). Organic phases were evaporated to yield the crude product. For final compounds the crude product was dissolved in DMF and purified by reverse phase preparative HPLC.

11.6 General procedure for the removal of PMB protecting group

The crude products obtained according to procedures described in examples **7.4** and **7.5** starting from **23b*** were re-dissolved/suspended in TFA (2 mL) and shaken at 60 °C for 5 h. After solvents removal, a 4 M NaOH solution (2 mL) was added and the crude product extracted using AcOEt (5 mL). The organic phases were evaporated, the crude products were dissolved in DMF and purified by reverse phase preparative HPLC.

11.7 Synthesis of 2,4-dichloro-5-nitro-pyridin-2-ol **12***

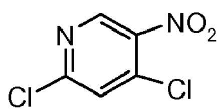

**12***

Ammonia (~80 mL) was condensed into a stirred -78 °C solution of THF (250 mL) under a nitrogen atmosphere. Potassium *tert*-butoxide (35.5 g, 316 mmol) was added to the mixture before allowing it to warm to -40 °C. Separately, a suspension of 4-chloro-3-nitropyridine (20 g, 126.2 mmol) in THF (100 mL) was cooled to 0 °C and a solution of *tert*-butyl hydroperoxide (6 M in decane, 21 mL, 126 mmol) was added over 10 minutes. The suspension was stirred for a further 5 minutes before adding the solution drop-wise to the ammonia mixture over 1 h. The reaction mixture was stirred for a further 1.5 h between -35 °C and -40 °C, before carefully quenching with saturated $NH_4Cl$ solution (25 mL). The reaction mixture was allowed to stir and vent overnight before the solvents were removed under vacuum with very careful vacuum regulation (~600 mbar initially). The crude solid was purified by trituration with ice cold saturated $NH_4Cl$

solution (50 mL), filtering onto a sinter. The solid was washed with ice cold water (2 x 25 mL) before drying under vacuum to give the intermediate phenol compound as a yellow solid (20.9 g, 95%). $^1$H NMR (400 MHz, DMSO) $\delta$(ppm): 8.8 (1 H, s), 7.0 (1 H, s), 5.9 (1 H, s). LC-MS: $R_t$=1.7 min, $[M-H]^-$ 175; $[M+H]^+$ 177.

4-Chloro-2-hydroxy-5-nitropyridine (17.2 g, 98.6 mmol) was suspended in toluene (150 mL) before careful addition of phosphorus oxychloride (28 mL, 300 mmol) over 20 minutes. The reaction mixture was heated to reflux for 6 h before cooling to 60 °C and stirring for a further 15 h. The reaction mixture was cooled to ambient temperature and then concentrated to dryness under vacuum to give a dark brown oil which was carefully basified at 0 °C with sat. $K_2CO_3$ (~60 mL). The mixture was extracted with AcOEt (3 x 200 mL). The combined organic phases were quickly washed with water and then brine before drying over $Na_2SO_4$. The solvents were removed under vacuum to give a dark brown oil which was purified by passing through a plug of silica, (eluting with petrol → 1:1, petrol: AcOEt) to give the product as a pale yellow powder (12.2 g, 64%). $^1$H NMR (400 MHz, $CDCl_3$) $\delta$ (ppm): 9.0 (1 H, s), 7.6 (1 H, s). LC-MS: $R_t$ = 2.44 min.

11.8 General procedure for (1-$R^2$-6-chloro-1*H*-imidazo[4,5-c]pyridin-2-yl)-$CH_2$-$R^7$-carbamic acid *tert*-butyl ester

11.8.1 Typical example: Synthesis of (1-methyl-6-chloro-1H-imidazo[4,5-c]pyridin-2-yl)methylcarbamic acid *tert*-butyl ester

Synthesis of 2-chloro-4-methylamine-5-nitropyridine

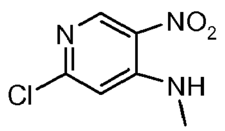

A mixture of methylamine hydrochloride (6.64 g, 98.4 mmol) and Hunig's base (17.1 mL, 98.4 mmol) in THF (50 mL) was added in portions at 0 °C to a solution of 2,4-dichloro-5-nitropyridine (10.5 g, 54.41 mmol) in THF (50 mL). After complete addition, the reaction mixture was stirred for further 30 minutes, then warmed to room temperature. The reaction mixture was poured into water (100 mL)and extracted AcOEt (3 x 150 mL). The organic layer was washed with water (80 mL) and brine (80 mL), dried over $Na_2SO_4$ and concentrated under vacuum to give a bright yellow powder (10.2 g, 99%). $^1$H NMR (400 MHz, $CDCl_3$) $\delta$ (ppm): 9.0 (1 H, s), 8.1 (1 H, bs), 6.7 (1 H, s), 3.0 (3H, d); LC-MS: $R_t$ = 2.12 min, $[M-H]^-$ 188; $[M+H]^+$ 190.

Synthesis of 2-chloro-4-methylamino-5-aminopyridine

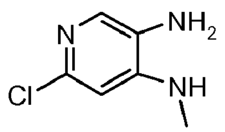

Iron powder (5 g, 89 mmol) in glacial acetic acid (100 mL) was stirred with high agitation *via* an overhead stirrer at 75-85 °C for ~12-20 minutes until a white coloration was observed. A suspension/solution of the 2-chloro-4-methylamine-5-nitropyridine (5 g, 26.67 mmol) in glacial acetic acid (100 mL) was added in small portions to the reaction mixture over 30 minutes. The reaction mixture was then stirred at 75 °C for a further h, then hot filtered through a pad of celite (wet with hot acetic acid), washed with hot acetic acid (50 mL) and AcOEt (100 mL). The solvents were removed under vacuum to give dark brown oil, which was basified at 0 °C with saturated $NaHCO_3$ solution (50 mL) and then extracted with AcOEt (3 x 50 mL). The combined organic layers were washed with brine (30 mL), dried over $Na_2SO_4$ and concentrated under vacuum to give a pale brown oil as the semi-pure product, which was used without further purification (4.12 g, 98%). $^1$H NMR (400 MHz, $CDCl_3$) $\delta$(ppm): 7.6 (1 H, s), 6.4 (1 H, s), 4.4 (1 H, bs), 3.0 (2H, bs), 2.85 (3H, d); LC-MS: $R_t$ = 2.0 min.

Synthesis of (6-chloro-1-methyl-1*H*-imidazo[4,5-*c*]pyridin-2-yl)-methyl-amine

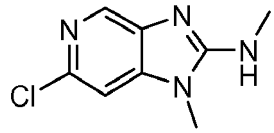

A solution of methylthioisocyanate (0.62 g, 8.46 mmol) and 2-chloro-4-methylamino-5-aminopyridine (1.00 g, 6.35 mmol) in $CHCl_3$ (12 mL) was heated at 50 °C for 12 h. The reaction mixture was cooled to room temperature, treated with EDC (1.46 g, 7.62 mmol) and $Et_3N$ (1.1 mL, 7.62 mmol) and stirred for additional 18 h. The reaction mixture was diluted with DCM (10 mL) and water (10 mL). After separation of the layers, the product as a white powder was precipitated from the aqueous layer by addition of DCM (20 mL). After filtration, the white powder was washed with water (10 mL) and dried under vacuum to give target product as a white solid (0.88 g, 71%). The organic layer was concentrated and purified by chromatography (AcOEt → 5% MeOH /AcOEt) to give a further amount of the product (0.12 g, ~10%). Overall yield: ~80%. $^1H$ NMR (400 MHz, DMSO) δ (ppm): 8.1 (1 H, s), 7.3 (1 H, s), 7.1 (1 H, bs), 3.5 (3H, s), 2.9 (3H, d). LC-MS. Rt = 1.25 min; $[M-H]^-$ 197; $[M+H]^+$ 199.

Synthesis of (1-methyl-6-chloro-1*H*-imidazo[4,5-c]pyridin-2-yl)methylcarbamic acid *tert*-butyl ester

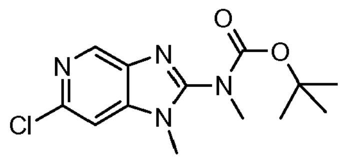

Di-*tert*-butyl dicarbonate (1.3 g, 5.9 mmol) was added in one portion to a solution of (1-methyl-6-chloro-1*H*-imidazo[4,5-c]pyridin-2-yl)-methyl-amine (1.1 g, 5.6 mmol) in pyridine (30 mL). DMAP (0.4 g, 3.2 mmol) was added and the reaction mixture was stirred for 18 h at room temperature. After solvents removal, the crude oil was triturated with ice cold *i*$Pr_2O$ to give a white solid, which was passed through a plug of silica (AcOEt/Petrol 1/1) to give the target product as a white solid (1.4 g, 80%). $^1H$ NMR (400 MHz, DMSO) δ(ppm): 8.67 (1 H, s), 7.8 (1 H, s), 3.63 (13H, s), 3.26 (3H, s), 1.4 (9H, s). LC-MS: $R_t$ = 2.36 min; $[M-H]^-$ 297; $[M+H]^+$ 299.

11.8.2 Typical example: Synthesis of (1-benzyl-6-chloro-1*H*-imidazo-[4,5-*c*]pyridin-2-yl)-methyl-carbamic acid *tert*-butyl ester

Synthesis of 2-chloro-4-benzylamine-5-nitropyridine

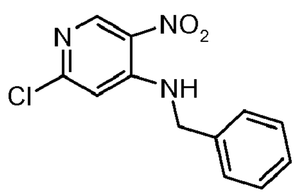

At 0 °C, benzylamine (12.9 mL, 118 mmol) was added drop-wise over 30 minutes to a solution of 2,4-dichloro-5-nitropyridine (20 g, 103 mmol) and $Et_3N$ (17.5 mL, 126 mmol) in THF (200 mL). After complete addition, the reaction mixture was stirred for further 30 minutes and then warmed to room temperature. The reaction mixture was poured into water (150 mL), extracted with AcOEt (500 mL). The aqueous phase was re-extracted with AcOEt (2 x 100 mL). The combined organic layers were washed with brine (150 mL) and dried over $Na_2SO_4$. The solvents were removed under vacuum to give the product as a pale orange powder (27.5 g, 100%). $^1H$ NMR (400 MHz, $CDCl_3$) δ (ppm): 9.05 (1 H, s), 8.5 (1 H, bs), 7.3 (5H, m), 6.7 (1 H, s), 4.5 (2H, d). LC-MS: $R_t$ = 2.7 min; $[M+H]^+$ 232.

Synthesis of 2-chloro-4-benzylamino-5-aminopyridine

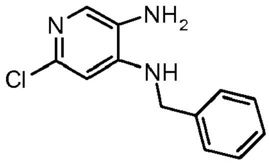

Iron powder (30 g, 535 mmol) in glacial acetic acid (300 mL) was stirred with high agitation *via* an overhead stirrer at 75-85 °C for ~12-20 minutes until a white coloration was observed. A suspension/solution of the 2-chloro-4-benzylamine-5-nitropyridine (27.5 g, 104 mmol) in glacial acetic acid (300 mL) was added in portions over 30 minutes at ~ 75 °C. After 30 minutes of stirring, the reaction mixture was hot filtered over a pad of celite, washed with hot acetic acid (150 mL) and then AcOEt (300 mL). The solvents were removed under vacuum to give a brown oil, which was carefully basified at 0 °C with saturated $NaHCO_3$ (200 mL) and then extracted with AcOEt (300 mL). During the extraction process, a precipitate formed, which was removed by filtering over a celite pad. The aqueous layer was re-extracted with AcOEt (2 x 300 mL) and the combined organic layers were washed with brine (120 mL), dried over $Na_2SO_4$ and concentrated *in vacuo* to give a pale brown oil as the product, which was used without further purification (24.5 g, ~100%). $^1H$ NMR (400 MHz, $CDCl_3$) $\delta$(ppm): 7.7 (1 H, s), 7.3 (5H, m), 6.5 (1 H, s), 4.65 (1 H, bs), 4.35 (2H, d), 3.0 (2H, bs). LC-MS: $R_t$ = 1.73 min; $[M-H]^-$ 234; $[M+H]^+$ 236.

Synthesis of (1-benzyl-6-chloro-1*H*-imidazo[4,5-*c*]pyridin-2-yl)-methyl-amine

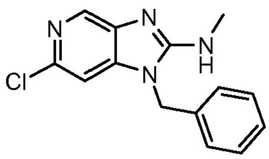

A solution of methylthioisocyanate (9.2 g, 125.9 mmol) and 2-chloro-4-benzylamino-5-aminopyridine (24.5 g, 104.9 mmol) in $CHCl_3$ (250 mL) was heated to 50 °C for 12 h. After cooling to room temperature, the reaction mixture was treated with EDC (24.1 g, 125.9 mmol), followed by $Et_3N$ (17.5 mL, 125.9 mmol) and stirred for additional 18 hours.

The reaction mixture was partitioned between $CHCl_3$ (200 mL) and water (150 mL). The aqueous layer was separated and re-extracted with $CHCl_3$ (2 x 150 mL) and the combined organic layers were washed with brine (100 mL). The organic layers were dried over $Na_2SO_4$, then concentrated to dryness to give a brown oil (~50 g), which was purified by column chromatography (60% AcOEt:petrol → 100% AcOEt, 5% MeOH) to give the product as a pale brown/pink solid. The product was further triturated with ice cold AcOEt to give the pure product as an off white solid (14.0 g, 49%). $^1H$ NMR (400 MHz, $CDCl_3$) $\delta$(ppm): 8.5 (1H, s); 7.35 (3H, m), 7.1 (2H, m), 7.0 (1 H, s), 5.05 (2H, s), 4.15 (1 H, bs), 3.05 (3H, d). LC-MS: $R_t$ = 2.15 min; $[M-H]^-$ 273; $[M+H]^+$ 275.

Synthesis of (1-benzyl-6-chloro-1*H*-imidazo[4,5-*c*]pyridin-2-yl)-methyl-carbamic acid *tert*-butyl ester

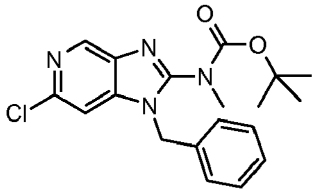

Di-*tert*-butyl dicarbonate (12.2 g, 55.9 mmol) was added in one portion to a solution of (1-benzyl-6-chloro-1*H*-imidazo[4,5-*c*]pyridin-2-yl)methylamine (14.0 g, 51.3 mmol) and DMAP (6.2 g, 50.7 mmol) in pyridine (100 mL). The

reaction mixture was stirred at room temperature for 18 h and then concentrated *in vacuo.* The crude oil was triturated with ice cold *i*$Pr_2O$ to give the target product as a white solid (19.0 g, ~99%). $^1H$ NMR (400 MHz, $CDCl_3$): 8.75 (1 H, s), 7.35 (3H, m), 7.15 (1 H, s), 7.05 (2H, m), 5.3 (1 H, s), 5.2 (2H, s), 3.15 (3H, s), 1.5 (9H, s). LC-MS: $R_t$ = 2.75 min; $[M-H]^-$ 373; $[M+H]^+$ 375.

11.8.3 Typical example: Synthesis of 6-chloro-1*H*-imidazo[4,5-*c*]pyridin-2-yl)-(2-benzyl)carbamic acid *tert*-butyl ester

Synthesis of (6-chloro-1*H*-imidazo[4,5-*c*]pyridin-2-yl)benzylamine

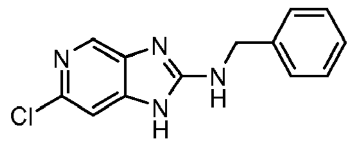

A solution of benzylthioisocyanate (7.0 mL, 53.1 mmol) and 6-chloro-pyridine-3,4-diamine (7.6 g, 53.1 mmol) in $CHCl_3$ (110 mL) was heated at 50 °C for 12 h. After cooling to ambient temperature, the reaction mixture was treated with EDC (12.2 g, 63.7 mmol) and $Et_3N$ (8.9 mL, 63.7 mmol). The reaction mixture was then stirred at room temperature for additional 18 h. $CHCl_3$ (110 mL) was added to the reaction mixture, followed by water (110 mL) and the organic phase was separated. The aqueous phase was re-extracted with $CHCl_3$ (2 x 150 mL) and the combined organic layers were washed with brine (50 mL). The organic layers were dried over $Na_2SO_4$, then concentrated in vacuo and purified by column chromatography on silica gel (AcOEt → 5% MeOH/AcOEt, then 90% DCM/MeOH) to give the product as a brown semi-solid. The solid was further purified by trituration with ice cold *i*$Pr_2O$ ether to give the product as an off white solid, which was used without further purification (3.00 g, 22%). LC-MS: $R_t$ = 2.31 min; $[M+H]^+$ 259/261.

Synthesis of (6-chloro-1*H*-imidazo[4,5-*c*]pyridin-2-yl)-(2-benzyl)carbamic acid *tert*-butyl ester

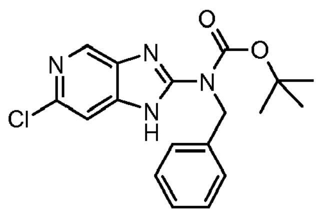

Di-*tert*-butyl dicarbonate (5.6 g, 25.5 mmol) was added in one portion to a solution of (6-chloro-1*H*-imidazo[4,5-c]pyridin-2-yl)-2-benzylamine (3.0 g, 11.6 mmol) and DMAP (0.9 g, 7.3 mmol) in pyridine (30 mL). The reaction mixture was stirred at room temperature for 18 hours, then concentrated *in vacuo* to give a crude oil, which was triturated with ice cold *i*$Pr_2O$her to give a crude semi-solid. Biotage column chromatography of the crude semi-solid (DCM → AcOEt, $R_f$(DCM/AcOEt = 1.1) = 0.7) to yield a pale yellow oil, which crystallized on standing to give an off white powder as the product (2.7 g, 65%). $^1H$ NMR (400 MHz, DMSO) δ(ppm): 12.6 (1 H, bs), 8.5 (1 H, bs), 7.5 (1 H, m), 7.3 (5.5H, m), 5.25 (2H, s), 1.4 (9H, s). LC-MS: $R_t$ = 3.33 min; $[M-H]^-$ 359; $[M+H]^+$ 361.

11.9 General procedure for (1-$R^2$-2-($(CH_2)_nR^5$)-6-chloro-1*H*-imidazo[4,5-*c*]pyridine **15***

11.9.1 Typical example: Synthesis of 6-chloro-1-methyl-2-phenyl-1*H*-imidazo[4,5-c]pyridine

Synthesis of *N*-(6-chloro-4-methylaminopyridin-3-yl)benzamide

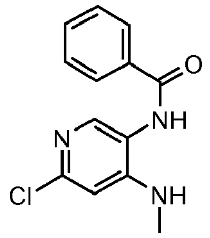

A solution of benzoyl chloride (3.4 g, 24.2 mmol) in DCM (25 mL) was added dropwise to a solution of 2-chloro-4-methylamino-5-aminopyridine (3.5 g, 22.2 mmol) and Hunig's base (3.4 g, 26.4 mmol) in DCM (75 mL). The mixture was stirred 15 minutes and concentrated *in vacuo.* Water was added to the residue causing heavy precipitation of beige solid. The beige solid was removed by filtration, washed with $NaHCO_3$ solution, followed by water, then dried under vacuum to give the product N-(6-chloro-4-methylamino-pyridin-3-yl)-benzamide as a beige solid (5.8 g, 100%). $^1H$ NMR (400 MHz, $CDCl_3$) δ (ppm): 8.40 (1 H, s), 7.92 (2 H, d, *J* = 7.63 Hz), 7.83 (1 H, s), 7.61-7.51 (1 H, m), 7.52-7.43 (2 H, m), 6.53 (1 H, s), 5.09 (1 H, d, *J* = 5.90 Hz), 2.85 (3 H, d, *J* = 4.93 Hz). LC-MS: $R_t$ = 2.03 min; $[M+H]^+$ 262.

Synthesis of 6-chloro-1-methyl-2-phenyl-1H-imidazo[4,5-*c*]pyridine

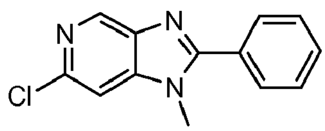

A solution of N-(6-Chloro-4-methylamino-pyridin-3-yl)-benzamide (5.8 g, 22.2 mmol) and $POCl_3$ (18 g, 111 mmol) in toluene (100 mL) was refluxed for 18 h. The reaction was cooled to ambient temperature and concentrated *in vacuo.* A solution of $NaHCO_3$ was added cautiously to the stirred residue causing heavy precipitation of an off-white solid. The solid was removed by filtration, washed with water, followed by $Et_2O$, then dried under vacuum to give the product 6-chloro-1-methyl-2-phenyl-1*H*-imidazo[4,5-*c*]pyridine as an off-white solid (5.0 g, 92%). $^1H$ NMR (400 MHz, DMSO) δ (ppm): 8.78 (1 H, d, *J* = 0.89 Hz), 7.92-7.88 (3 H, m), 7.63-7.58 (3 H, m), 3.90 (3 H, s). LC-MS: $R_t$ = 2.66 min; $[M+H]^+$ 244.

11.9.2 Typical example: Synthesis of 6-chloro-1-methyl-1*H*-imidazo[4,5-*c*]pyridine

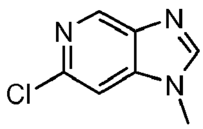

2-Chloro-4-methylamino-5-aminopyridine (3.0 g, 19.0 mmol), trimethyl ortho formate (50 mL) and formic acid (1.0 mL) were stirred at 100 °C for 3 h. The reaction was cooled to ambient temperature and the solvents removed under vacuum. The residue was poured into $NaHCO_3$ solution and then extracted with AcOEt. The combined organic layers were washed with water, followed by brine and then dried over $Na_2SO_4$. The solvents were removed under vacuum to give a brown solid, which was purified by column chromatography (30% AcOEt/DCM) to give the target product as a pale brown solid (2.7 g, 85%). $^1H$ NMR (400 MHz, DMSO) δ (ppm): 8.75 (1 H, d, *J* = 0.89 Hz), 8.39 (1 H, s), 7.84 (1 H, d, *J* = 0.90 Hz), 3.86 (3 H, s). LC-MS $R_t$ = 1.97 min; $[M+H]^+$ 168.

11.10 General procedure for the Suzuki reaction

All reactions were carried out in parallel and stock solutions of the following reagents were prepared: **15*** (0.5 M in DMF), boronic acid (0.72 M in DMF), $PPh_3$ (0.03 M in DMF), $Pd(OAc)_2$ (0.017 M in DMF) and $Na_2CO_3$ (0.6 M in $H_2O$). The reaction vessels were charged with the **15*** (0.6 mL, 0.3 mmol), followed by the boronic acid (0.5 mL, 0.36

mmol), $PPh_3$ (1 mL, 0.03 mmol) and $Na_2CO_3$ (0.9 mL, 0.45 mmol). After flushing with nitrogen for 10 seconds, an aliquot of $Pd(OAc)_2$ (0.9 mL, 0.015 mmol) was added and the reactions vessels were again flushed with nitrogen for 6 seconds before sealing. The reaction mixtures were heated with shaking at 80 °C block temperature for 18 h. After this time, the mixtures were allowed to cool to room temperature, then filtered through a 96 well plate Whatman filter unit (Whatman Unifilter, 0.45 μm polypropylene filter, long drip) into a 96 well plate, washing through with DMF (0.3 mL). The crude products were purified by reverse phase preparative HPLC.

11.11 General procedure for the Buchwald-Hartwig reaction

All reactions were carried out in parallel and stock solutions of the following reagents were prepared: **15*** (0.133 M in DMF : 5% Water), amine (2.222 M in DMF), $Pd_2(dba)_3$ (0.032 M in DMF), X-Phos (0.024 M in DCM).

The reaction vessels were charged with X-Phos (14 mg, 0.024 mmol) in DCM (1 mL) and then concentrated to dryness. The stock intermediates **15*** (3.0 mL, 0.4 mmol) were distributed across the reaction tubes followed by the relevant amine (0.27 mL, 0.6 mmol) and $K_3PO_4$ (140 mg, 0.6 mmol). After flushing with nitrogen for 10 seconds, an aliquot of $Pd_2(dba)_3$ (0.5 mL, 0.016 mmol) was added and the reactions vessels, which were again flushed with nitrogen for 6 seconds were sealed. The reaction mixtures were heated and stirred at 95 °C block temperature for 18 h. After this time, the mixtures were allowed to cool to room temperature and the crude reaction mixtures were filtered through a 96 well plate Whatman filter unit (Whatman Unifilter, 0.45 μm polypropylene filter, long drip) into a 96 well plate, washing through with DMF (0.3 mL). The crude products were purified by reverse phase preparative HPLC as outlined.

11.12 General procedure for the Suzuki coupling followed by deprotection

All reactions were carried out in parallel and stock solutions of the following reagents were prepared; **14*** wherein PG=Boc (0.2 M in DMF), boronic acid (0.5 M in DMF), $Pd(dppf)_2Cl_2$ (0.064 M in DMF) and $Cs_2CO_3$ (0.6 M in $H_2O$). The reaction vessels were charged with the **14*** (1 mL, 0.2 mmol), followed by the boronic acid (0.4 mL, 0.3 mmol) and $Cs_2CO_3$ (0.5 mL, 0.3 mmol). After flushing with nitrogen for 10 seconds, an aliquot of $Pd(dppf)_2Cl_2$ (0.25 mL, 0.016 mmol) was added and the reactions vessels were again flushed with nitrogen for 6 seconds before sealing. The reaction mixtures were heated with shaking at 80 °C block temperature for 18 h then cooled to room temperature and concentrated under reduced pressure by GeneVac (45 °C).

The residues were taken in DCM (2 mL), treated with TFA (0.75 mL) and shaked at room temperature for 18 h. The solvents were removed under reduced pressure by Genevac (Dry pure, 30 °C, 1 h; full vacuum 45 °C, 1 h) and the residues were then re-dissolved in DMF (1.1 mL). The crude reaction mixtures were filtered through a 96 well plate Whatman filter unit (Whatman Unifilter, 0.45 μm polypropylene filter, long drip) into a 96 well plate, washing through with DMF (0.3 mL). The crude products were purified by reverse phase preparative HPLC.

11.13 General procedure for the Buchwald-Hartwig reaction followed by deprotection

All reactions were carried out in parallel and stock solutions of the following reagents were prepared: **14*** wherein PG = Boc (0.133 M in DMF: Water (5%)), amine (2.222 M in DMF), $Pd_2(dba)_3$ (0.032 M in DMF), X-Phos (0.024 M in DCM). The reaction vessels were charged with X-Phos (14 mg, 0.024 mmol) in DCM (1 mL) and then concentrated to dryness. The stock intermediates **14*** (3.0 mL, 0.4 mmol) were distributed across the reaction tubes followed by the relevant amine (0.27 mL, 0.6 mmol) and $K_3PO_4$ (140 mg, 0.6 mmol). After flushing with nitrogen for 10 seconds, an aliquot of $Pd_2(dba)_3$ (0.5 mL, 0.016 mmol) was added and the reactions vessels, which were again flushed with nitrogen for 6 seconds were sealed. The reaction mixtures were heated and stirred at 95 °C block temperature for 18 h. After this time, the mixtures were allowed to cool to room temperature and concentrated under reduced pressure by GeneVac (45 °C).

The residues were taken in DCM (4 mL), treated with TFA (1.5 mL) and shaked at room temperature for 18 h. The solvents were removed under reduced pressure by Genevac (Dry pure, 30 °C, 1 h; full vacuum 45 °C, 1 h) and the residues were then re-dissolved in DMF (2.2 mL). The crude reaction mixtures were filtered through a 96 well plate Whatman filter unit (Whatman Unifilter, 0.45 μm polypropylene filter, long drip) into a 96 well plate, washing through with DMF (0.6 mL). The crude products were purified by reverse phase preparative HPLC.

**Example 12-1 Compound 1**

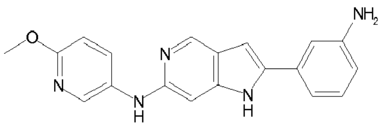

MW.: 331.14, LCMS-ESI (*m*/*z*) - found 332.14 [M+H]$^+$, 330.14 [M-H]$^-$

**Example 12-2 Compound 2**

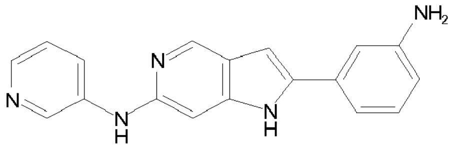

MW.: 301.13, LCMS-ESI (*m*/*z*) - found 302.13 [M+H]$^+$, 300.13 [M-H]$^-$

**Example 12-3 Compound 3**

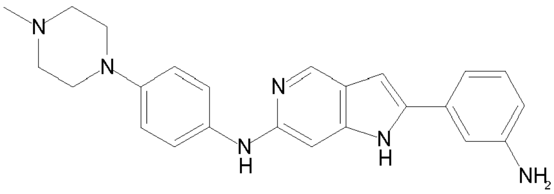

MW.: 398.22, LCMS-ESI (*m*/*z*) - found 399.22 [M+H]$^+$, 397.22 [M-H]$^-$

**Example 12-4 Compound 4**

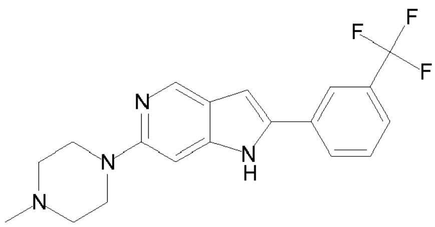

MW.: 360.16, LCMS-ESI (*m*/*z*) - found 361.15 [M+H]$^+$, 359,15 [M-H]$^-$

**Example 12-5 Compound 5**

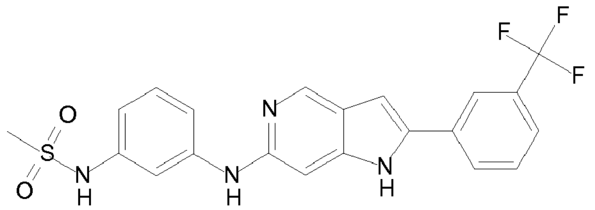

MW.: 446.10, LCMS-ESI (*m/z*) - found 447.10 $[M+H]^+$, 445.10 $[M-H]^-$

**Example 12-6 Compound 6**

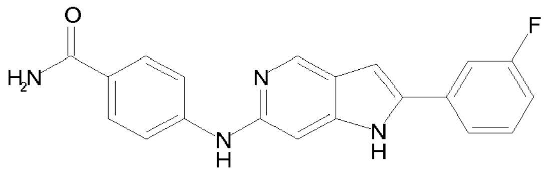

MW.: 346.12, LCMS-ESI (*m/z*) - found 347.12 $[M+H]^+$, 345.12 $[M-H]^-$

**Example 12-7 Compound 7**

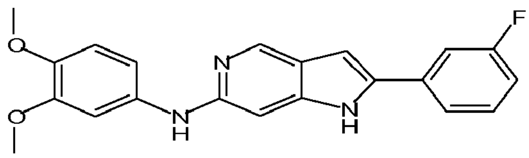

MW.: 363.14, LCMS-ESI (*m/z*) - found 364.13 $[M+H]^+$, 362.13 $[M-H]^-$

**Example 12-8 Compound 8**

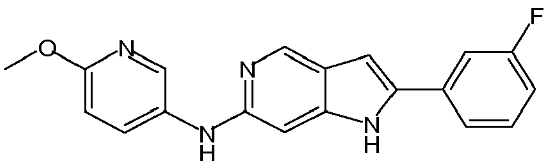

MW.: 334.12, LCMS-ESI (*m/z*) - found 335.12 $[M+H]^+$, 333.12 $[M-H]^-$

**Example 12-9 Compound** 9

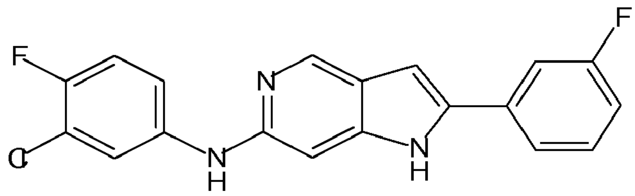

MW.: 355.07, LCMS-ESI (*m/z*) - found 356.06 $[M+H]^+$, 354.06 $[M-H]^-$

**Example 8-10 Compound 10**

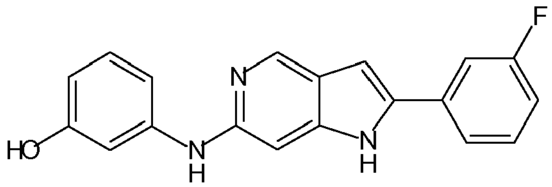

MW.: 319.11, LCMS-ESI (m/z) - found 320.11 $[M+H]^+$, 319.11 $[M-H]^-$

**Example 12-11 Compound 11**

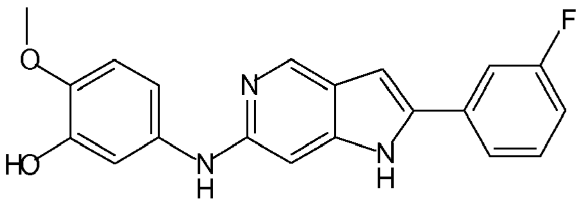

MW.: 349.12, LCMS-ESI (*m/z*) - found 350.12 $[M+H]^+$, 348.12 $[M-H]^-$

**Example 12-12 Compound** 12

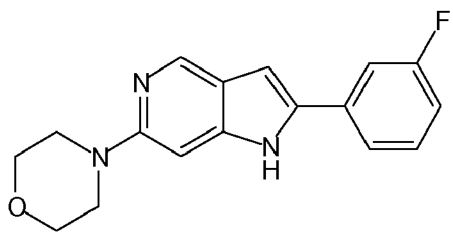

MW.: 297.13, LCMS-ESI (*m/z*) - found 298.12 $[M+H]^+$, 296.12 $[M-H]^-$

**Example 12-13 Compound 13**

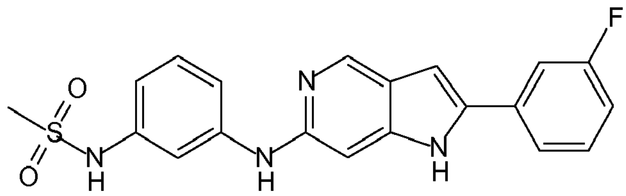

MW.: 396.11, LCMS-ESI (*m/z*) - found 397.10 $[M+H]^+$, 395.10 $[M-H]^-$

**Example 12-14 Compound 14**

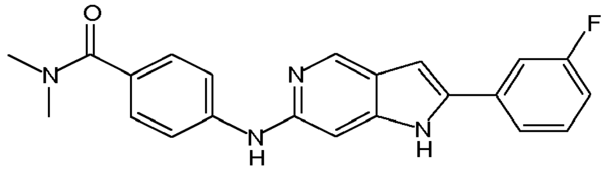

MW.: 374.15, LCMS-ESI (m/z) - found 375.15 $[M+H]^+$, 373.15 $[M-H]^-$

**Example 12-15 Compound 15**

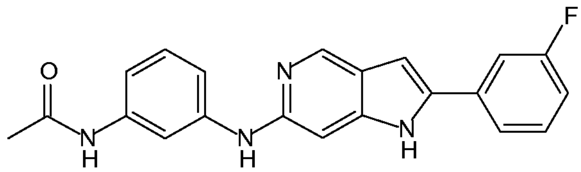

MW.: 360.14, LCMS-ESI (m/z) - found 361.13 $[M+H]^+$, 359.13 $[M-H]^-$

**Example 12-16 Compound 16**

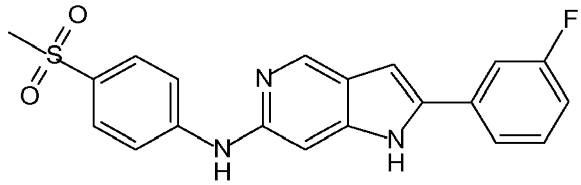

MW.: 381.09, LCMS-ESI (m/z) - found 382.09 $[M+H]^+$, 380.09 $[M-H]^-$

**Example 12-17 Compound 17**

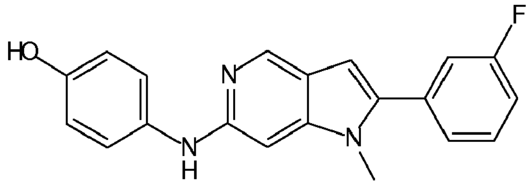

MW.: 333.13, LCMS-ESI (*m/z*) - found 334.13 $[M+H]^+$, 332.12 $[M-H]^-$

**Example 12-18 Compound 18**

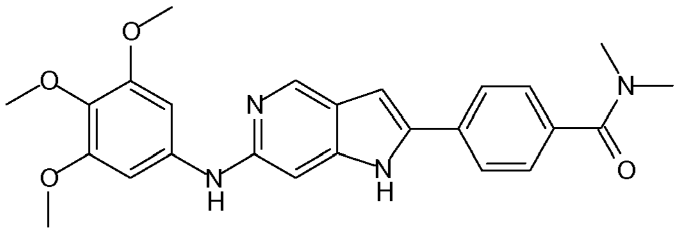

MW.: 446.20, LCMS-ESI (*m/z)* - found 447.19 $[M+H]^+$, 445.19 $[M-H]^-$

**Example 12-19 Compound 19**

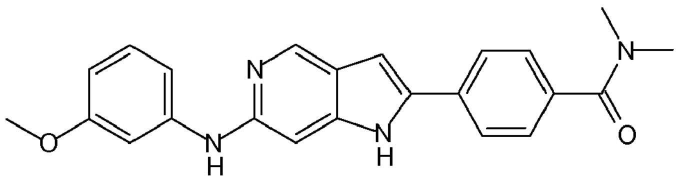

MW.: 386.17, LCMS-ESI (*m/z*) - found 387.17 $[M+H]^+$, 385.17 $[M-H]^-$

**Example 12-20 Compound 20**

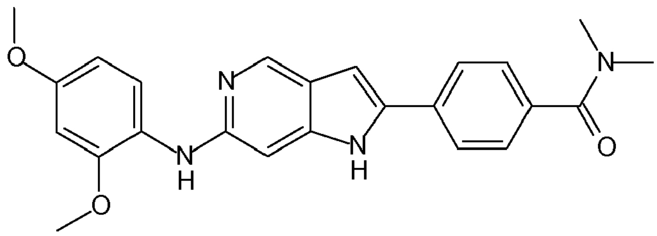

MW.:416.18, LCMS-ESI (m/z) - found 417.18 $[M+H]^+$, 415.18 $[M-H]^-$

**Example 12-21 Compound** 21

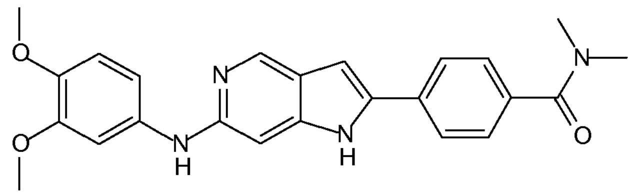

MW.: 416.18, LCMS-ESI (*m*/*z*) - found 417.18 $[M+H]^+$, 415.18 $[M-H]^-$

**Example 12-22 Compound 22**

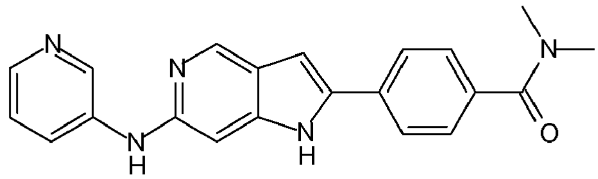

MW.: 357.16, LCMS-ESI (*m*/*z*) - found 358.15 $[M+H]^+$, 356.15 $[M-H]^-$

**Example 12-23 Compound 23**

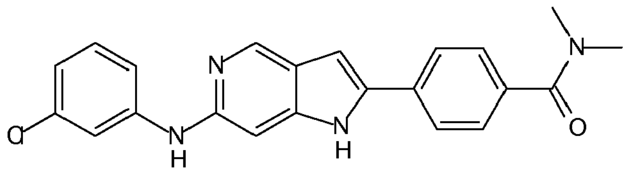

MW.: 390.12, LCMS-ESI (*m*/*z*) - found 391.12 $[M+H]^+$, 389.12 $[M-H]^-$

**Example 12-24 Compound 24**

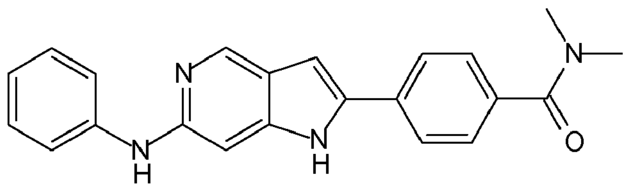

MW.: 356.16, LCMS-ESI (*m*/*z*) - found 357.16 $[M+H]^+$, 355.16 $[M-H]^-$

**Example 12-25 Compound 25**

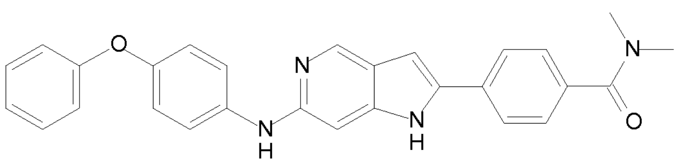

MW.: 448.19, LCMS-ESI (m/z) - found 449.19 $[M+H]^+$, 447.19 $[M-H]^-$

**Example 12-26 Compound 26**

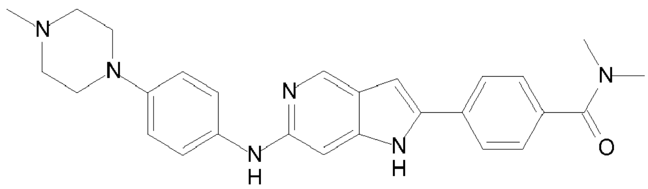

MW.: 454.25, LCMS-ESI (*m*/*z*) - found 455.24 $[M+H]^+$, 453.24 $[M-H]^-$

**Example 12-27 Compound 27**

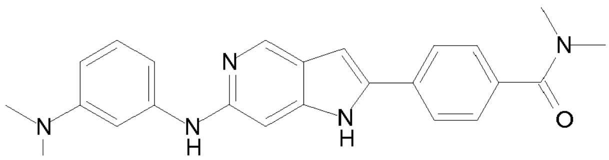

MW.: 399.21, LCMS-ESI (*m*/*z*) - found 400.2 $[M+H]^+$, 398.2 $[M-H]^-$

**Example 12-28 Compound 28**

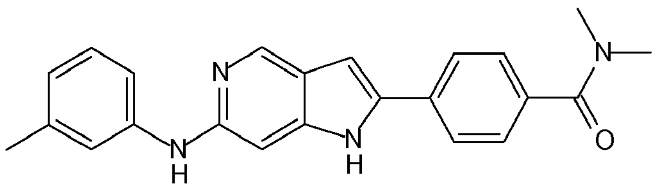

MW.: 370.18, LCMS-ESI (*m*/*z*) - found 371.17 $[M+H]^+$, 369.17 $[M-H]^-$

**Example 12-29 Compound 29**

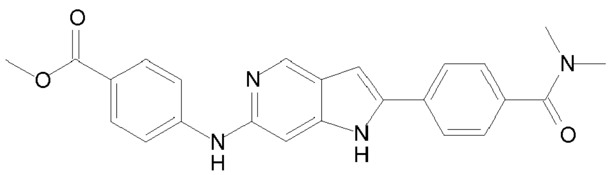

MW.: 414.17, LCMS-ESI (*m*/*z*) - found 415.16 $[M+H]^+$, 413.16 $[M-H]^-$

**Example 12-30 Compound 30**

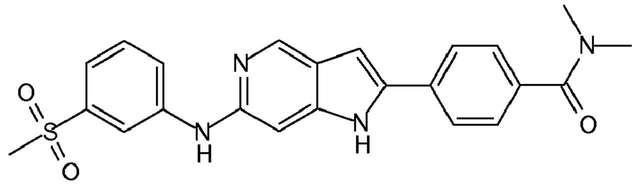

MW.: 434.14, LCMS-ESI (*m/z*) - found 435.14 $[M+H]^+$, 433.14 $[M-H]^-$

**Example 12-31 Compound 31**

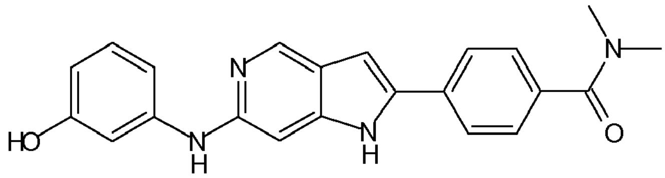

MW.: 372.16, LCMS-ESI (*m/z*) - found 373.15 $[M+H]^+$, 371.15 $[M-H]^-$

**Example 12-32 Compound 32**

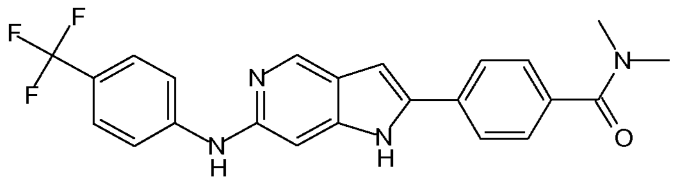

MW.: 424.15, LCMS-ESI (m/z) - found 425.15 $[M+H]^+$, 423.15 $[M-H]^-$

**Example 12-33 Compound 33**

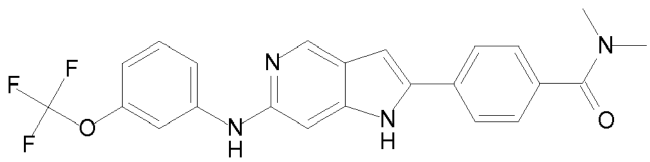

MW.: 440.15, LCMS-ESI (*m/z*) - found 441.14 $[M+H]^+$, 439.14 $[M-H]^-$

**Example 12-34 Compound** 34

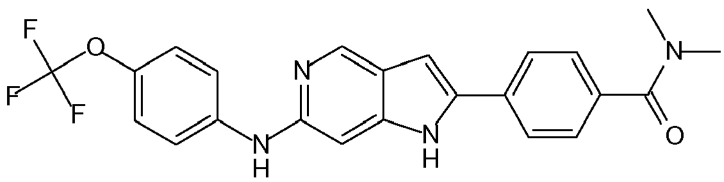

MW.: 440.15, LCMS-ESI (*m*/*z*) - found 441.14 $[M+H]^+$, 439.14 $[M-H]^-$

**Example 12-35 Compound 35**

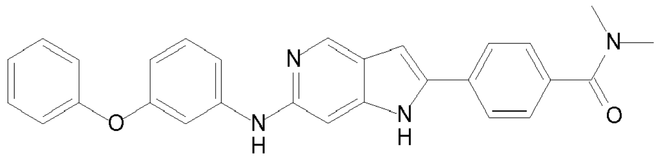

MW.: 448.19, LCMS-ESI (*m*/*z*) - found 449.19 $[M+H]^+$, 447.19 $[M-H]^-$

**Example 12-36 Compound** 36

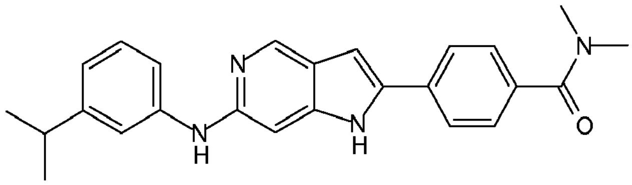

MW.: 398.21, LCMS-ESI (*m*/*z*) - found 399.21 $[M+H]^+$, 397.21 $[M-H]^-$

**Example 12-37 Compound 37**

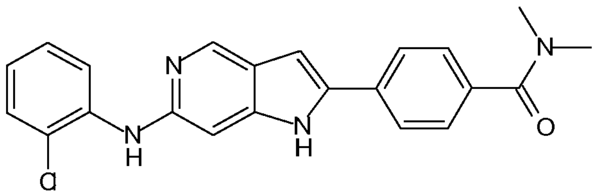

MW.: 390.12, LCMS-ESI (m/z) - found 391.12 $[M+H]^+$, 389.12 $[M-H]^-$

**Example 12-38 Compound 38**

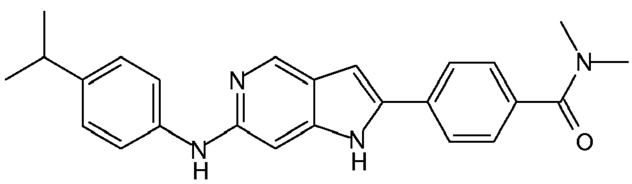

MW.: 398.21, LCMS-ESI (*m*/*z*) - found 399.21 $[M+H]^+$, 397.21 $[M-H]^-$

**Example 12-39 Compound 39**

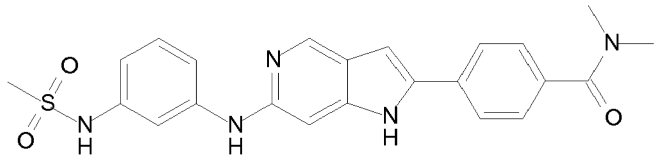

MW.: 449.15, LCMS-ESI (*m/z*) - found 450.15 $[M+H]^+$, 448.15 $[M-H]^-$

**Example 12-40 Compound 40**

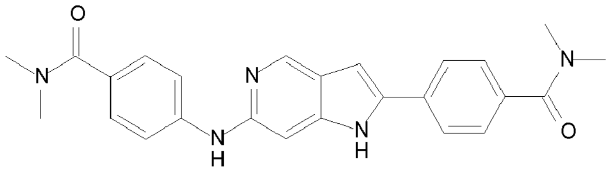

MW.: 427.20, LCMS-ESI (*m/z*) - found 428.20 $[M+H]^+$, 426.20 $[M-H]^-$

**Example 12-41 Compound 41**

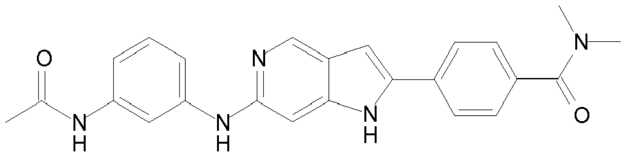

MW.: 413.19, LCMS-ESI (*m/z*) - found 414.18 $[M+H]^+$, 412.18 $[M-H]^-$

**Example 12-42 Compound 42**

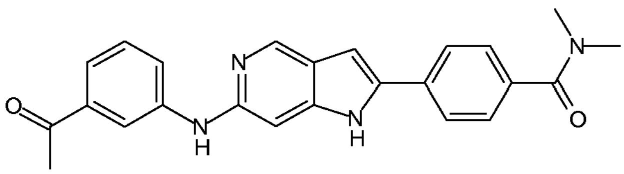

MW.: 398.17, LCMS-ESI (*m/z*) - found 399.17 $[M+H]^+$, 397.17 $[M-H]^-$

**Example 12-43 Compound 43**

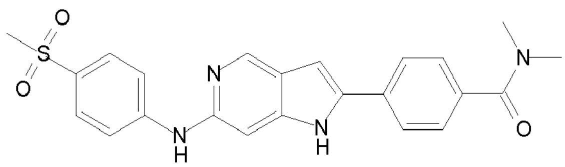

MW.: 434.14, LCMS-ESI (*m/z*) - found 435.14 $[M+H]^+$, 433.14 $[M-H]^-$

**Example 12-44 Compound 44**

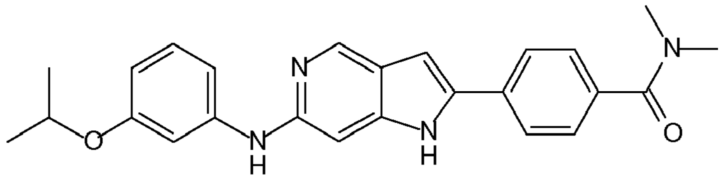

MW.: 414.21, LCMS-ESI (*m*/*z*) - found 415.20 $[M+H]^+$, 413.20 $[M-H]^-$

**Example 12-45 Compound 45**

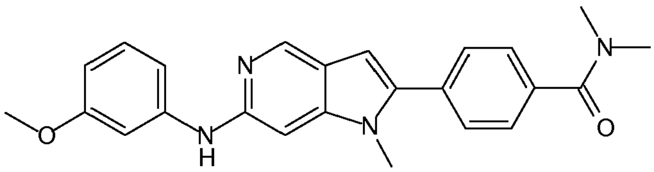

MW.: 400.19, LCMS-ESI (*m*/*z*) - found 401.19 $[M+H]^+$, 399.19 $[M-H]^-$

**Example 12-46 Compound 46**

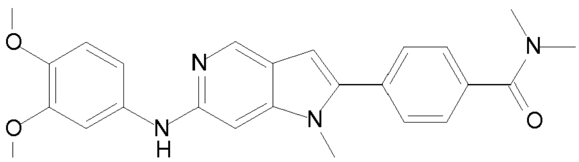

MW.: 430.20, LCMS-ESI (*m*/*z*) - found 431.20 $[M+H]^+$, 429.20 $[M-H]^-$

**Example 12-47 Compound** 47

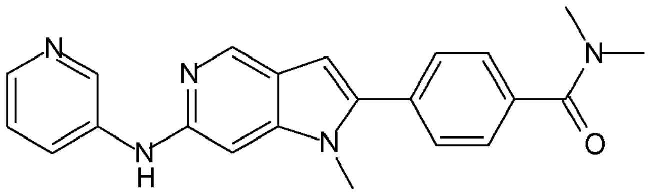

MW.: 371.17, LCMS-ESI (*m*/*z*) - found 372.17 $[M+H]^+$, 370.17 $[M-H]^-$

**Example 12-48 Compound 48**

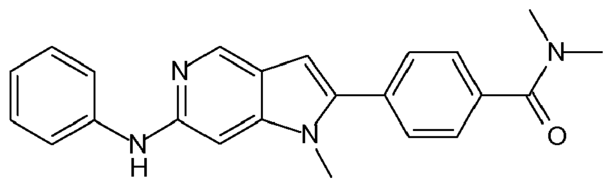

MW.: 370.18, LCMS-ESI (*m*/*z*) - found 371.17 $[M+H]^+$, 369.17 $[M-H]^-$

**Example 12-49 Compound 49**

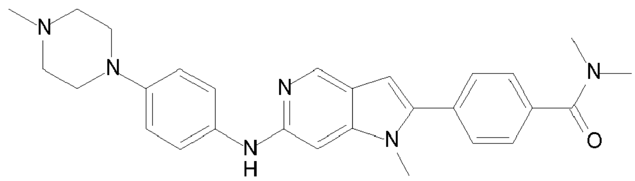

MW.: 468.26, LCMS-ESI (*m*/*z*) - found 469.26 $[M+H]^+$, 467.26 $[M-H]^-$

**Example 12-50 Compound 50**

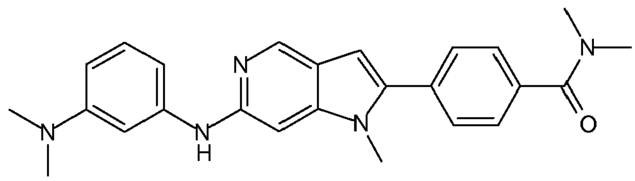

MW.: 413.22, LCMS-ESI (*m*/*z*) - found 414.22 $[M+H]^+$, 412.22 $[M-H]^-$

**Example 12-51 Compound 51**

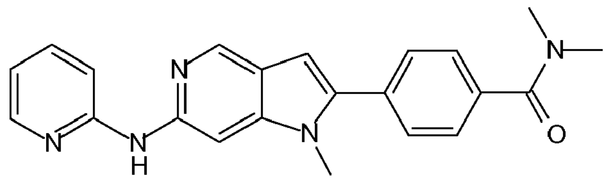

MW.: 371.17, LCMS-ESI (*m*/*z*) - found 372.17 $[M+H]^+$, 370.17 $[M-H]^-$

**Example 12-52 Compound 52**

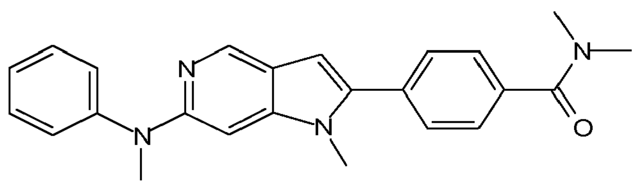

MW.: 384.19, LCMS-ESI (*m*/*z*) - found 385.19 $[M+H]^+$, 383.19 $[M-H]^-$

**Example 12-53 Compound 53**

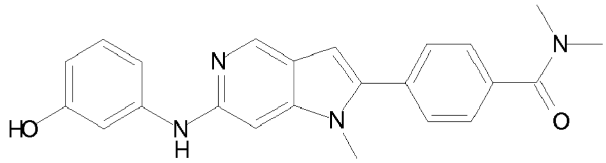

MW.: 386.17, LCMS-ESI (*m*/*z*) - found 387.17 $[M+H]^+$, 385.17 $[M-H]^-$

**Example 12-54 Compound 54**

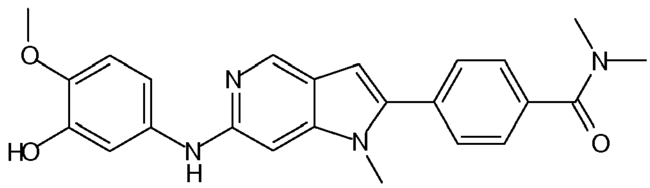

MW.:416.18, LCMS-ESI (*m*/*z*) - found 417.18 $[M+H]^+$, 415.18 $[M-H]^-$

**Example 12-55 Compound 55**

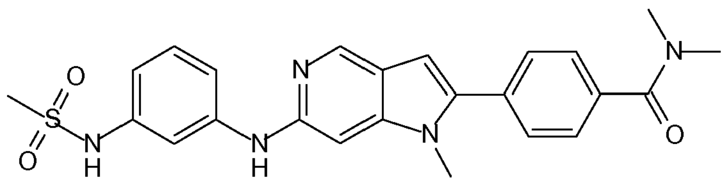

MW.: 463.17, LCMS-ESI (*m*/*z*) - found 464.16 $[M+H]^+$, 462.16 $[M-H]^-$

**Example 12-56 Compound 56**

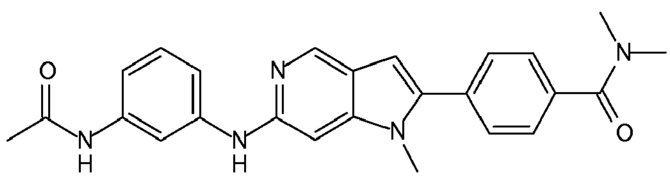

MW.: 427.20, LCMS-ESI (*m*/*z*) - found 428.20 $[M+H]^+$, 426.20 $[M-H]^-$

**Example 12-57 Compound 57**

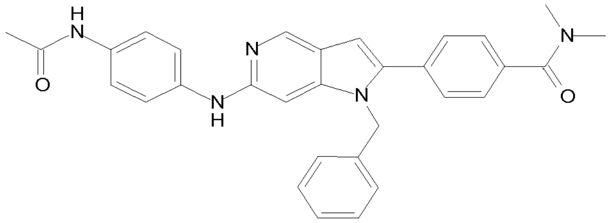

MW.: 503.23, LCMS-ESI (*m*/*z*) - found 504.20 $[M+H]^+$, 502.23 $[M-H]^-$

**Example 12-58 Compound 58**

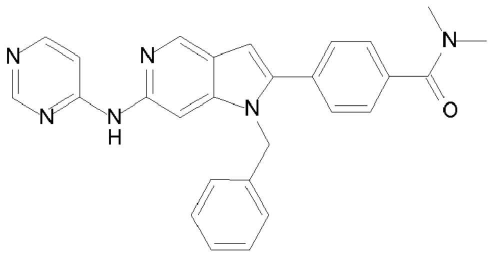

MW.: 448.20, LCMS-ESI (*m*/*z*) - found 449.20 $[M+H]^+$, 447.20 $[M-H]^-$

**Example 12-59 Compound** 59

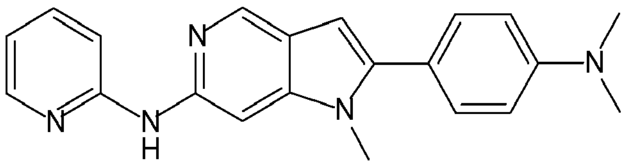

MW.: 343.18, LCMS-ESI (*m*/*z*) - found 344.18 $[M+H]^+$, 342.18 $[M-H]^-$

**Example 12-60 Compound 60**

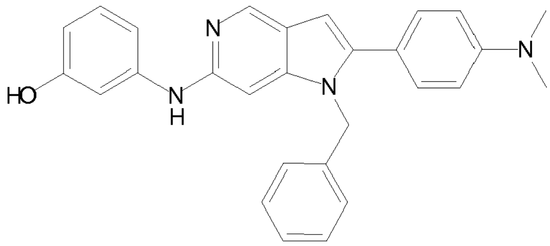

MW.: 434.21, LCMS-ESI (m/z) - found 435.21 $[M+H]^+$, 433.21 $[M-H]^-$

**Example 12-61 Compound 61**

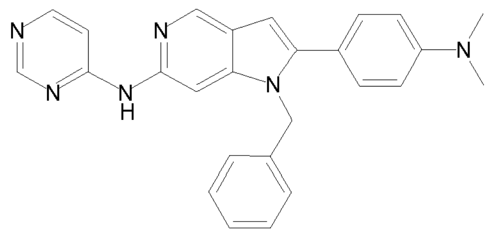

MW.: 420.21, LCMS-ESI (*m*/*z*) - found 421.20 $[M+H]^+$, 419.20 $[M-H]^-$

**Example 12-62 Compound 62**

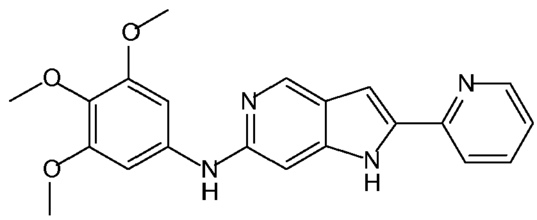

MW.: 376.15, LCMS-ESI (*m*/*z*) - found 377.15$[M+H]^+$, 375.15 $[M-H]^-$

**Example 12-63 Compound 63**

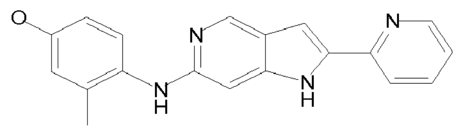

MW.: 330.15, LCMS-ESI (*m*/*z*) - found 331.14 $[M+H]^+$, 329.14 $[M-H]^-$

**Example 12-64 Compound 64**

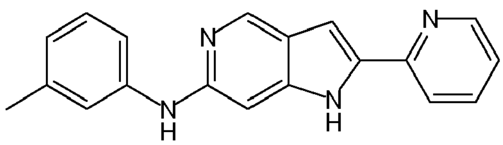

MW.: 300.14, LCMS-ESI (*m*/*z*) - found 301.13 $[M+H]^+$, 299.13 $[M-H]^-$

**Example 12-65 Compound 65**

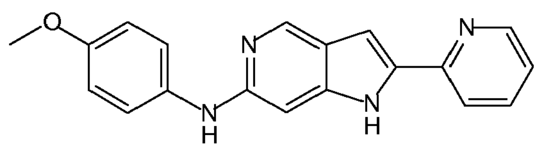

MW.: 316.13, LCMS-ESI ($m/z$) - found 317.13 $[M+H]^+$, 315.13 $[M-H]^-$

**Example 12-66 Compound 66**

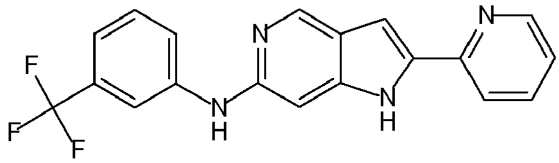

MW.: 354.11, LCMS-ESI ($m/z$) - found 355.10 $[M+H]^+$, 353.10 $[M-H]^-$

**Example 12-67 Compound 67**

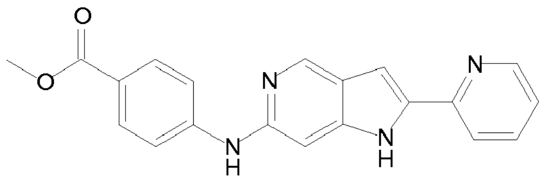

MW.: 344.13, LCMS-ESI ($m/z$) - found 345.12 $[M+H]^+$, 343.12 $[M-H]^-$

**Example 12-68 Compound 68**

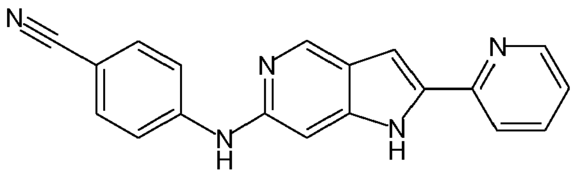

MW.: 311.12, LCMS-ESI ($m/z$) - found 312.11 $[M+H]^+$, 310.11 $[M-H]^-$

**Example 12-69 Compound 69**

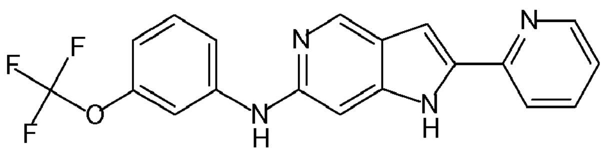

MW.: 370.10, LCMS-ESI ($m/z$) - found 371.10 $[M+H]^+$, 369.10 $[M-H]^-$

**Example 12-70 Compound 70**

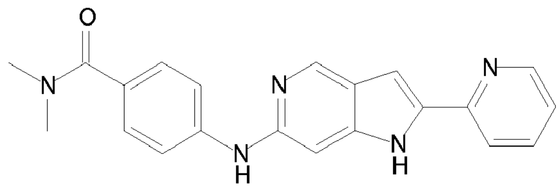

MW.: 357.16, LCMS-ESI (*m*/*z*) - found 358.16 [M+H]$^+$, 356.16 [M-H]$^-$

**Example 12-71 Compound 71**

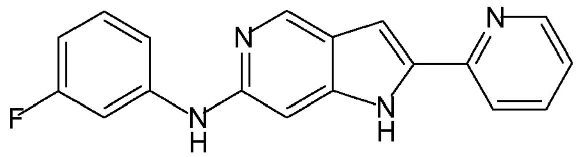

MW.: 304.11, LCMS-ESI (*m*/*z*) - found 305.11 [M+H]$^+$, 303.11 [M-H]$^-$

**Example 12-72 Compound 72**

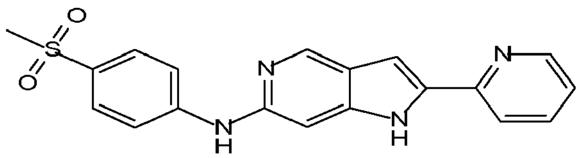

MW.: 364.10, LCMS-ESI (*m*/*z*) - found 365.09 [M+H]$^+$, 363.09 [M-H]$^-$

**Example 12-73 Compound 73**

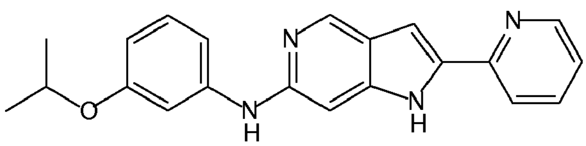

MW.: 344.16, LCMS-ESI (*m*/*z*) - found 345.16 [M+H]$^+$, 343.16 [M-H]$^-$

**Example 12-74 Compound 74**

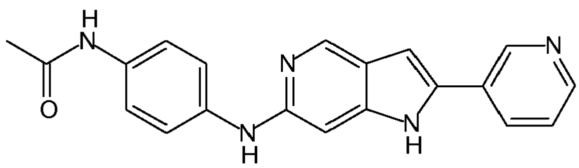

MW.: 343.14, LCMS-ESI (*m*/*z*) - found 344.14 [M+H]$^+$, 342.14 [M-H]$^-$

**Example 12-75 Compound 75**

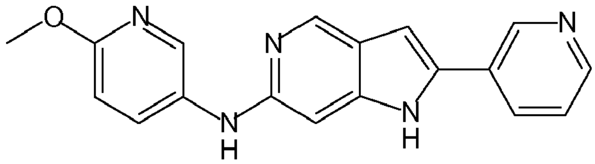

MW.: 317.13, LCMS-ESI (*m*/*z*) - found 318.12 $[M+H]^+$, 316.12 $[M-H]^-$

**Example 12-76 Compound 76**

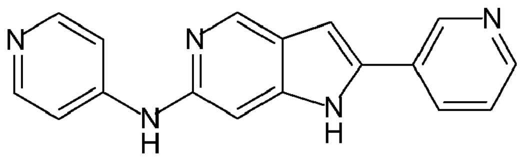

MW.: 287.12, LCMS-ESI (*m*/*z*) - found 288.12 $[M+H]^+$, 286.12 $[M-H]^-$

**Example** 12-77 Compound 77

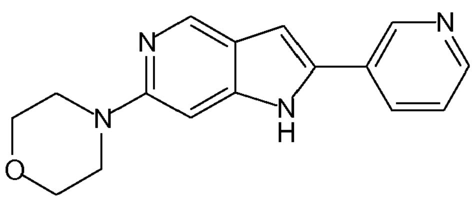

MW.: 280.13, LCMS-ESI (*m*/*z*) - found 281.13 $[M+H]^+$, 279.13 $[M-H]^-$

**Example** 12-78 Compound 78

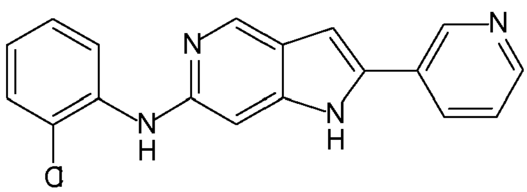

MW.: 320.08, LCMS-ESI (*m*/*z*) - found 321.08 $[M+H]^+$, 319.08 $[M-H]^-$

**Example** 12-79 Compound 79

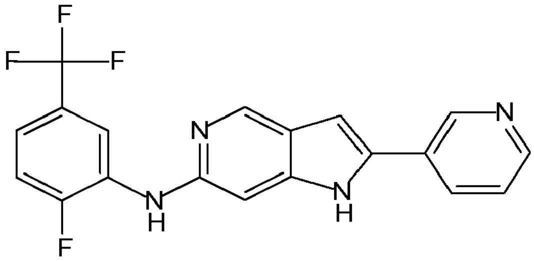

MW.: 372.10, LCMS-ESI (*m/z*) - found 373.10 $[M+H]^+$, 371.10 $[M-H]^-$

**Example** 12-80 Compound 80

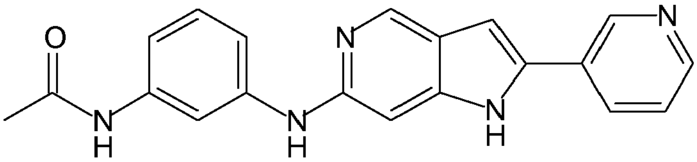

MW.: 343.14, LCMS-ESI (*m/z*) - found 344.14 $[M+H]^+$, 342.14 $[M-H]^-$

**Example** 12-81 Compound 81

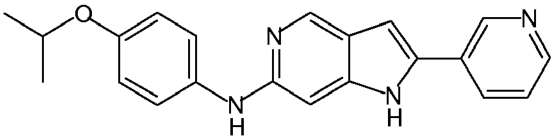

MW.: 344.16, LCMS-ESI (*m/z*) - found 345.16 $[M+H]^+$, 343.16 $[M-H]^-$

**Example** 12-82 Compound 82

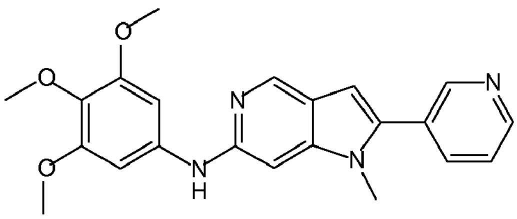

MW.: 390.17, LCMS-ESI (*m/z*) - found 391.16 $[M+H]^+$, 389.16 $[M-H]^-$

**Example** 12-83 Compound 83

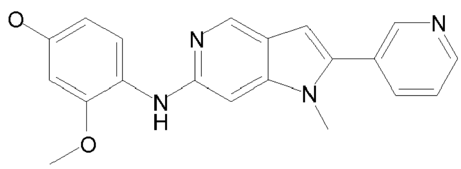

MW.: 360.16, LCMS-ESI (*m*/*z*) - found 361.15 $[M+H]^+$, 359.15 $[M-H]^-$

**Example** 12-84 Compound 84

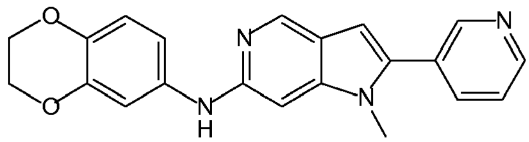

MW.: 358.14, LCMS-ESI (*m*/*z*) - found 359.14 $[M+H]^+$, 357.14 $[M-H]^-$

**Example** 12-85 Compound 85

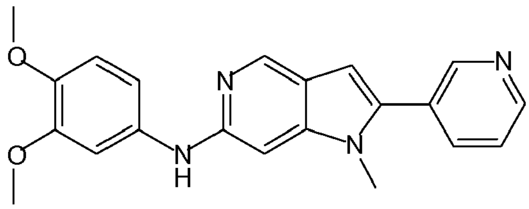

MW.: 360.16, LCMS-ESI (m/z) - found 361.15 $[M+H]^+$, 359.15 $[M-H]^-$

**Example** 12-86 Compound 86

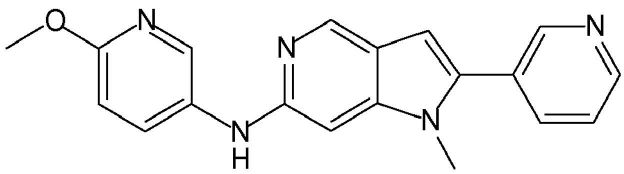

MW.: 331.14, LCMS-ESI (*m*/*z*) - found 332.14 $[M+H]^+$, 330.14 $[M-H]^-$

**Example 12-87 Compound 87**

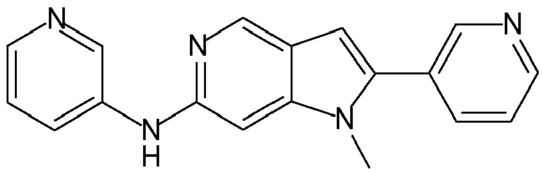

MW.: 301.13, LCMS-ESI (*m*/*z*) - found 302.12 $[M+H]^+$, 300.13 $[M-H]^-$

**Example 12-88 Compound 88**

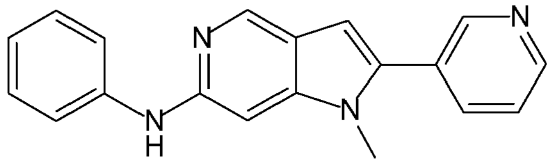

MW.: 300.14, LCMS-ESI (*m*/*z*) - found 301.13 $[M+H]^+$, 299.13 $[M-H]^-$

**Example 12-89 Compound 89**

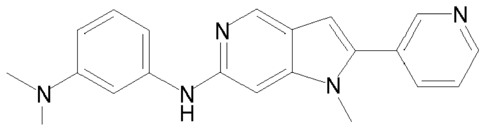

MW.: 343.18, LCMS-ESI (*m*/*z*) - found 344.18 $[M+H]^+$, 342.18 $[M-H]^-$

**Example 12-90 Compound** 90

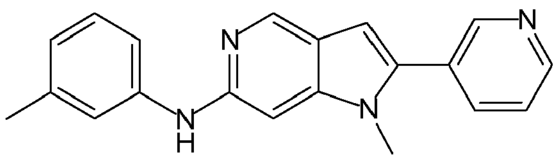

MW.: 314.15, LCMS-ESI (*m*/*z*) - found 315.15 $[M+H]^+$, 313.15 $[M-H]^-$

**Example 12-91 Compound 91**

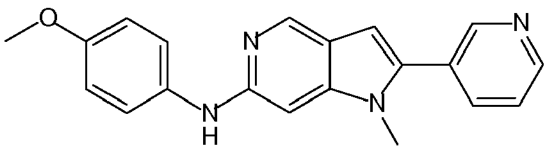

MW.: 330.15, LCMS-ESI (*m*/*z*) - found 331.14 $[M+H]^+$, 329.14 $[M-H]^-$

**Example 12-92 Compound 92**

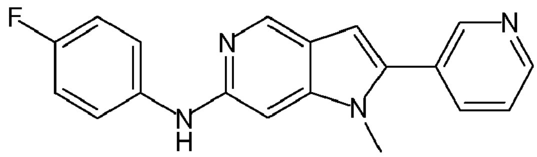

MW.: 318.13, LCMS-ESI (*m*/*z*) - found 319.12 $[M+H]^+$, 317.12 $[M-H]^-$

**Example 12-93 Compound 93**

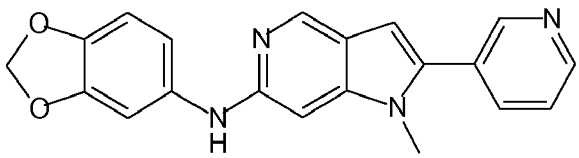

MW.: 344.13, LCMS-ESI (*m*/*z*) - found 345.12 $[M+H]^+$, 343.12 $[M-H]^-$

**Example 12-94 Compound 94**

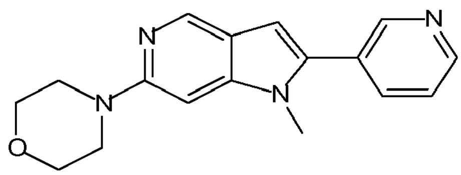

MW.: 294.15, LCMS-ESI (*m*/*z*) - found 295.14 $[M+H]^+$, 293.14 $[M-H]^-$

**Example 12-95 Compound 95**

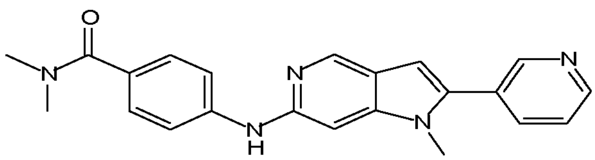

MW.: 371.17, LCMS-ESI (*m*/*z*) - found 372.17 $[M+H]^+$, 370.17 $[M-H]^-$

**Example 12-96 Compound 96**

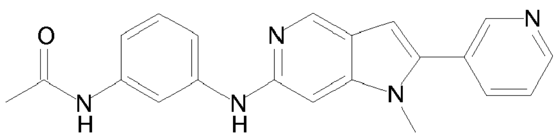

MW.: 357.16, LCMS-ESI (*m*/*z*) - found 358.15 $[M+H]^+$, 356.15 $[M-H]^-$

**Example 12-97 Compound 97**

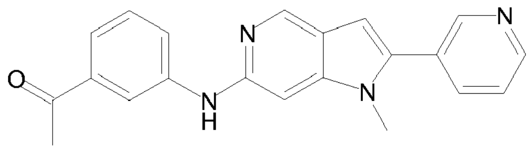

MW.: 342.15, LCMS-ESI (*m*/*z*) - found 343.14 $[M+H]^+$, 341.14 $[M-H]^-$

**Example 12-98 Compound 98**

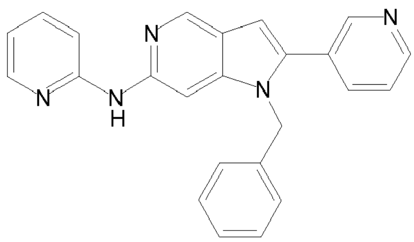

MW.: 377.16, LCMS-ESI (*m*/*z*) - found 378.16 $[M+H]^+$, 376.16 $[M-H]^-$

**Example 12-99 Compound 99**

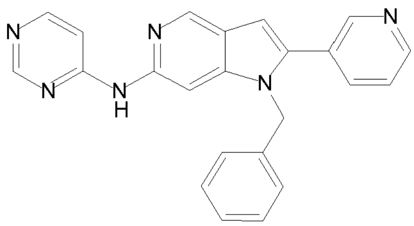

MW.: 378.16, LCMS-ESI (*m*/*z*) - found 379.15 $[M+H]^+$, 377.15 $[M-H]^-$

**Example 12-100 Compound 100**

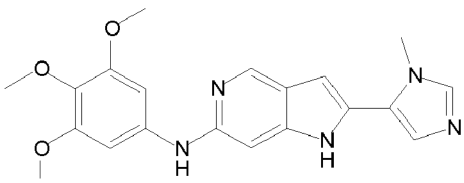

MW.: 379.16, LCMS-ESI (*m*/*z*) - found 380.16 $[M+H]^+$, 378.16 $[M-H]^-$

**Example 12-101 Compound 101**

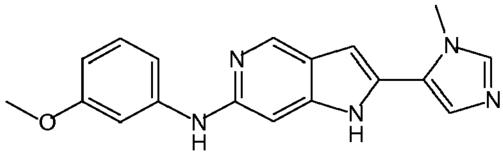

MW.: 319.14, LCMS-ESI (*m*/*z*) - found 320.14 [M+H]$^+$, 318.14 [M-H]$^-$

**Example 12-102 Compound 102**

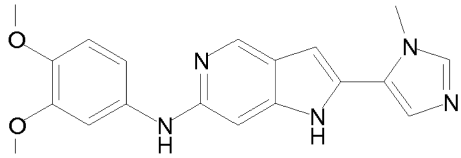

MW.: 349.15, LCMS-ESI (*m*/*z*) - found 350.15 [M+H]$^+$, 348.15 [M-H]$^-$

**Example 12-103 Compound 103**

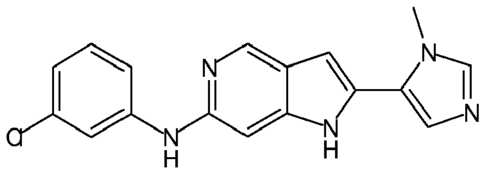

MW.: 323.09, LCMS-ESI (*m*/*z*) - found 324.09 [M+H]$^+$, 322.09 [M-H]$^-$

**Example 12-104 Compound 104**

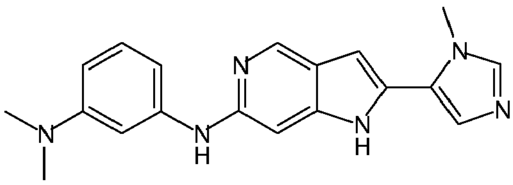

MW.: 332.17, LCMS-ESI (*m*/*z*) - found 333.17 [M+H]$^+$, 331.17 [M-H]$^-$

**Example 12-105 Compound 105**

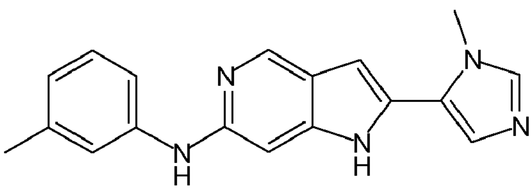

MW.: 303.15, LCMS-ESI (*m*/*z*) - found 304.14 $[M+H]^+$, 302.14 $[M-H]^-$

**Example 12-106 Compound 106**

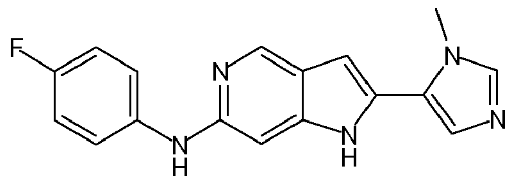

MW.: 307.12, LCMS-ESI (*m*/*z*) - found 308.12 $[M+H]^+$, 306.12 $[M-H]^-$

**Example 12-107 Compound 107**

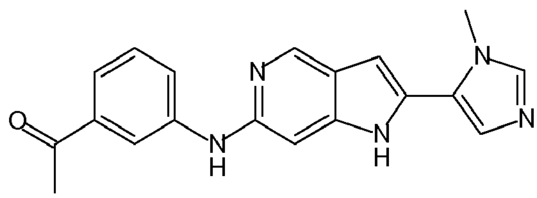

MW.: 331.14, LCMS-ESI (*m*/*z*) - found 332.14 $[M+H]^+$, 330.14 $[M-H]^-$

**Example 12-108 Compound 108**

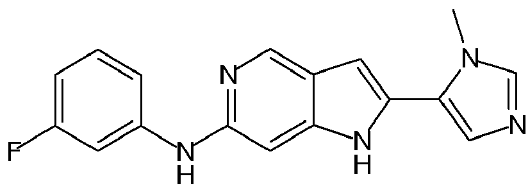

MW.: 307.12, LCMS-ESI (*m*/*z*) - found 308.12 $[M+H]^+$, 306.18 $[M-H]^-$

**Example 12-109 Compound 109**

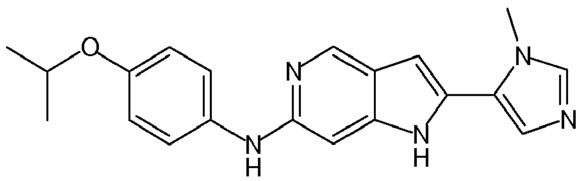

MW.: 347.17, LCMS-ESI (*m*/*z*) - found 348.17 $[M+H]^+$, 346.17 $[M-H]^-$

**Example 12-110 Compound 110**

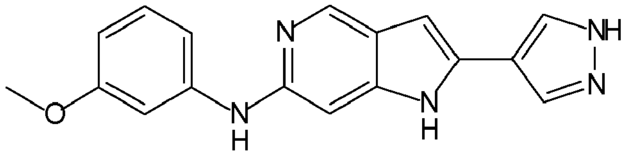

MW.: 305.13, LCMS-ESI (*m*/*z*) - found 306.13 $[M+H]^+$, 304.13 $[M-H]^-$

**Example 12-111 Compound 111**

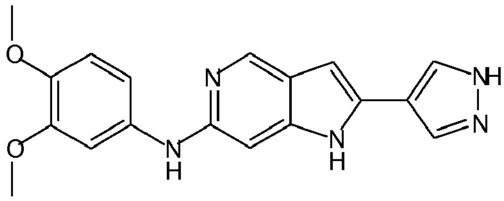

MW.: 335.14, LCMS-ESI (*m*/*z*) - found 336.13 $[M+H]^+$, 334.13 $[M-H]^-$

**Example 12-112 Compound 112**

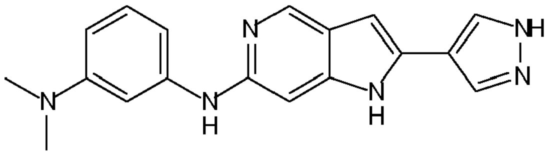

MW.: 318.16, LCMS-ESI (*m*/*z*) - found 319.15 $[M+H]^+$, 317.15 $[M-H]^-$

**Example 12-113 Compound 113**

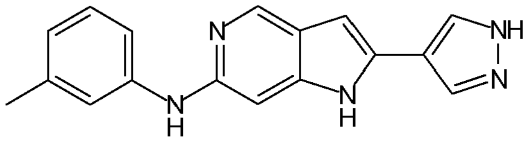

MW.: 289.13, LCMS-ESI (m/z) - found 290.13 $[M+H]^+$, 288.13 $[M-H]^-$

**Example 12-114 Compound 114**

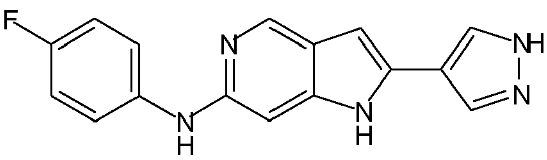

MW.: 293.11, LCMS-ESI (*m*/*z*) - found 294.10 $[M+H]^+$, 292.10 $[M-H]^-$

**Example 12-115 Compound 115**

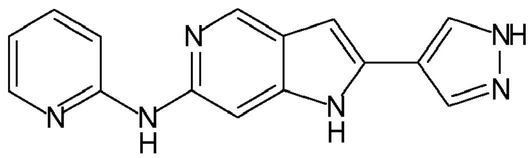

MW.: 276.11, LCMS-ESI (*m*/*z*) - found 277.11 $[M+H]^+$, 275.11 $[M-H]^-$

**Example 12-116 Compound 116**

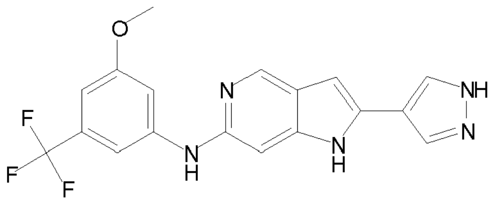

MW.: 373.12, LCMS-ESI (*m*/*z*) - found 374.11 $[M+H]^+$, 372.11 $[M-H]^-$

**Example 12-117 Compound 117**

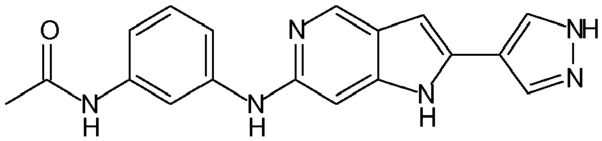

MW.: 332.14, LCMS-ESI (m/z) - found 333.13 $[M+H]^+$, 331.13 $[M-H]^-$

**Example** 12-118 Compound 118

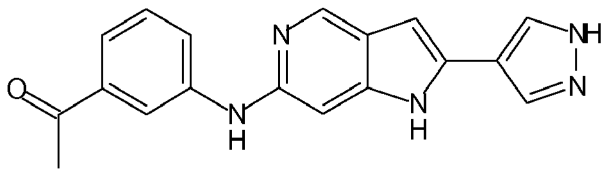

MW.: 317.13, LCMS-ESI (*m*/*z*) - found 318.12 $[M+H]^+$, 316.12 $[M-H]^-$

**Example 12-119 Compound 119**

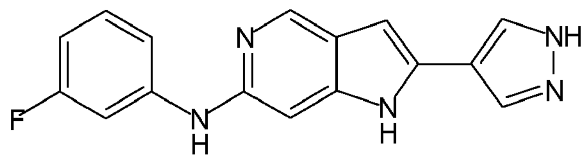

MW.: 293.11, LCMS-ESI (*m/z*) - found 294.10 $[M+H]^+$, 292.10 $[M-H]^-$

**Example 12-120 Compound 120**

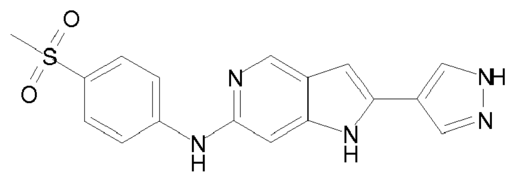

MW.: 353.09, LCMS-ESI (*m/z*) - found 354.09 $[M+H]^+$, 352.09 $[M-H]^-$

**Example 12-121 Compound 121**

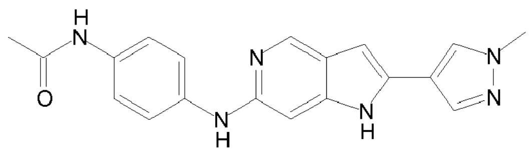

MW.: 346.15, LCMS-ESI (*m/z*) - found 347.15 $[M+H]^+$, 345.15 $[M-H]^-$

**Example 12-122 Compound 122**

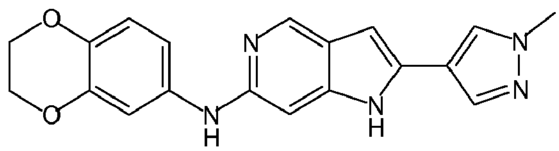

MW.: 347.14, LCMS-ESI (*m/z*) - found 348.13 $[M+H]^+$, 346.13 $[M-H]^-$

**Example 12-123 Compound 123**

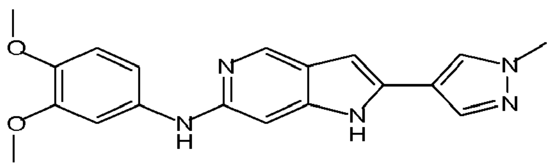

MW.: 349.15, LCMS-ESI (*m/z*) - found 350.15 $[M+H]^+$, 348.15 $[M-H]^-$

**Example 12-124 Compound 124**

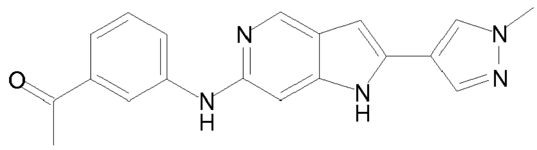

MW.: 331.14, LCMS-ESI (*m*/*z*) - found 332.14 $[M+H]^+$, 330.14 $[M-H]^-$

**Example 12-125 Compound 125**

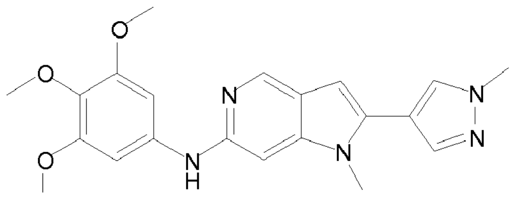

MW.: 393.18, LCMS-ESI (*m*/*z*) - found 394.18 $[M+H]^+$, 392.18 $[M-H]^-$

**Example 12-126 Compound 126**

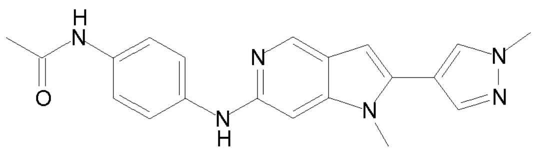

MW.: 360.17, LCMS-ESI (*m*/*z*) - found 361.17 $[M+H]^+$, 359.17 $[M-H]^-$

**Example 12-127 Compound 127**

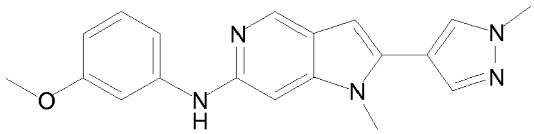

MW.: 333.16, LCMS-ESI (*m*/*z*) - found 334.15 $[M+H]^+$, 332.15 $[M-H]^-$

**Example 12-128 Compound 128**

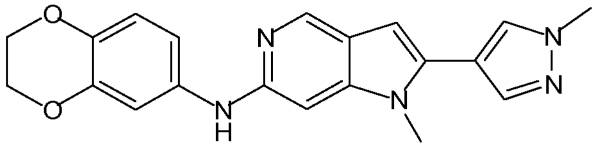

MW.: 361.15, LCMS-ESI (*m/z*) - found 362.15 $[M+H]^+$, 360.15 $[M-H]^-$

**Example 12-129 Compound 129**

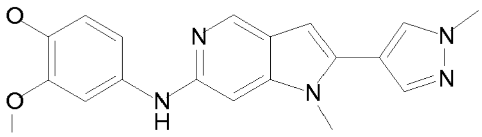

MW.: 363.17, LCMS-ESI (*m/z*) - found 364.17 $[M+H]^+$, 362.17 $[M-H]^-$

**Example 12-130 Compound 130**

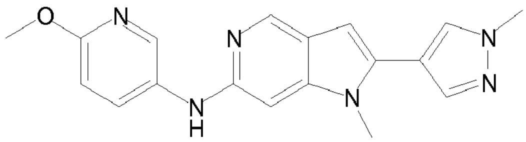

MW.: 334.15, LCMS-ESI (*m/z*) - found 335.15 $[M+H]^+$, 333.15 $[M-H]^-$

**Example 12-131 Compound 131**

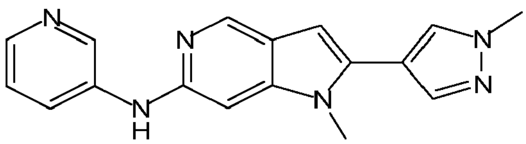

MW.: 304.14, LCMS-ESI (*m/z*) - found 305.14 $[M+H]^+$, 303.14 $[M-H]^-$

**Example 12-132 Compound 132**

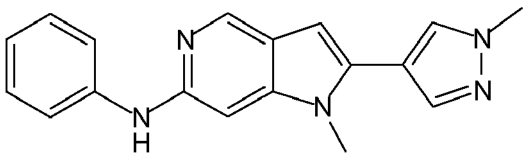

MW.: 303.15, LCMS-ESI (*m/z*) - found 304.14 $[M+H]^+$, 302.14 $[M-H]^-$

**Example 12-133 Compound 133**

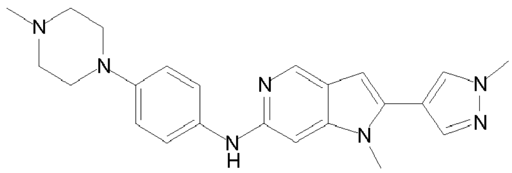

MW.: 401.23, LCMS-ESI (*m*/*z*) - found 402.23 $[M+H]^+$, 400.23 $[M-H]^-$

**Example 12-134 Compound 134**

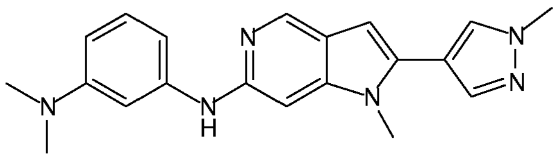

MW.: 346.19, LCMS-ESI (*m*/*z*) - found 347.19 $[M+H]^+$, 345.19 $[M-H]^-$

**Example 12-135 Compound 135**

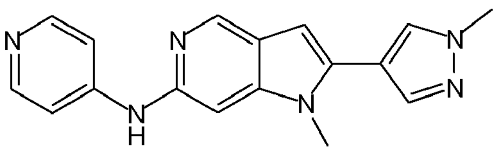

MW.: 304.14, LCMS-ESI (*m*/*z*) - found 305.14 $[M+H]^+$, 303.14 $[M-H]^-$

**Example 12-136 Compound 136**

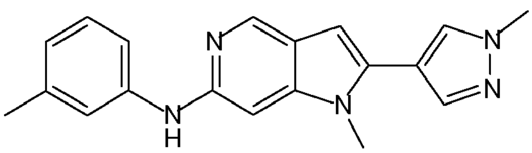

MW.: 317.16, LCMS-ESI (*m*/*z*) - found 318.16 $[M+H]^+$, 316.16 $[M-H]^-$

**Example 12-137 Compound 137**

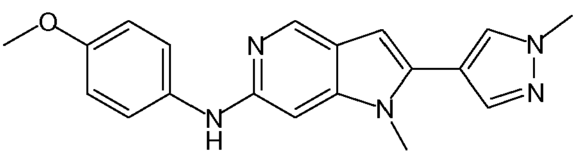

MW.: 333.16, LCMS-ESI ($m/z$) - found 334.15 $[M+H]^+$, 332.15 $[M-H]^-$

**Example 12-138 Compound 138**

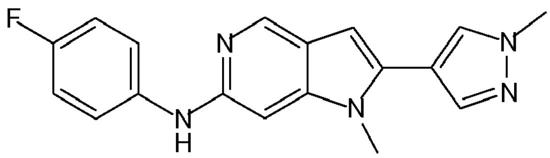

MW.: 321.14, LCMS-ESI ($m/z$) - found 322.13 $[M+H]^+$, 320.13 $[M-H]^-$

**Example 12-139 Compound 139**

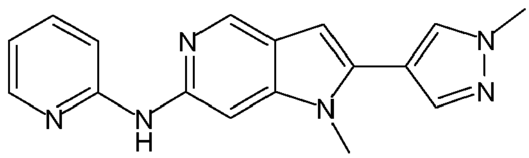

MW.: 304.14, LCMS-ESI ($m/z$) - found 305.14 $[M+H]^+$, 303.14 $[M-H]^-$

**Example 12-140 Compound 140**

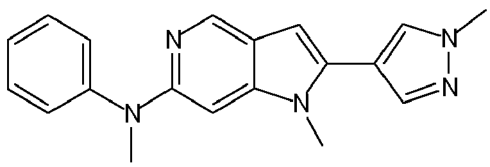

MW.: 317.16, LCMS-ESI ($m/z$) - found 318.16 $[M+H]^+$, 316.16 $[M-H]^-$

**Example 12-141 Compound 141**

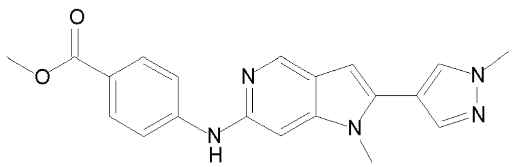

MW.: 361.15, LCMS-ESI ($m/z$) - found 362.15 $[M+H]^+$, 360.15 $[M-H]^-$

**Example 12-142 Compound 142**

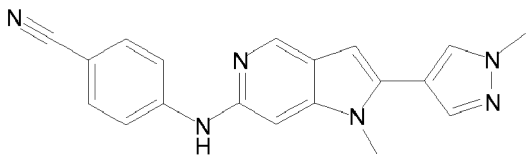

MW.: 328.14, LCMS-ESI ($m/z$) - found 329.14 $[M+H]^+$, 327.14 $[M-H]^-$

**Example 12-143 Compound 143**

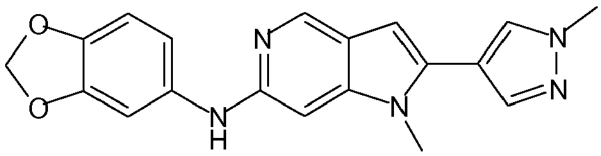

MW.: 347.14, LCMS-ESI ($m/z$) - found 348.13 $[M+H]^+$, 346.13 $[M-H]^-$

**Example 12-144 Compound 144**

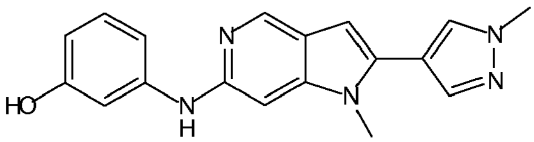

MW.: 319.14, LCMS-ESI ($m/z$) - found 320.14 $[M+H]^+$, 318.14 $[M-H]^-$

**Example 12-145 Compound 145**

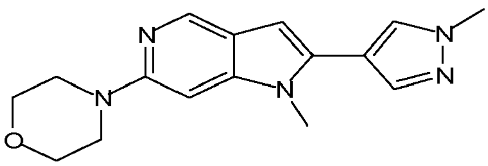

MW.: 297.16, LCMS-ESI ($m/z$) - found 298.15 $[M+H]^+$, 296.15 $[M-H]^-$

**Example 12-146 Compound 146**

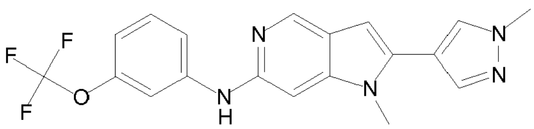

MW.: 387.13, LCMS-ESI ($m/z$) - found 388.13 $[M+H]^+$, 386.13 $[M-H]^-$

**Example 12-147 Compound 147**

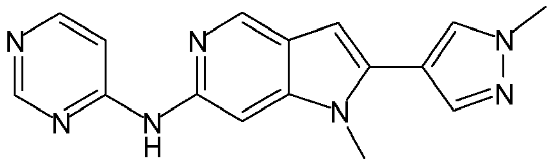

MW.: 305.14, LCMS-ESI (*m/z*) - found 306.13 $[M+H]^+$, 304.13 $[M-H]^-$

**Example 12-148 Compound 148**

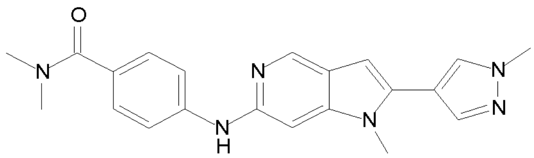

MW.: 374.19, LCMS-ESI (*m/z*) - found 375.18 $[M+H]^+$, 373.18 $[M-H]^-$

**Example 12-149 Compound 149**

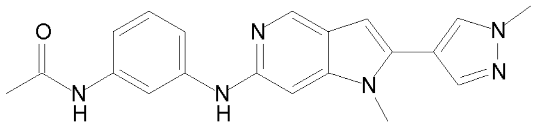

MW.: 360.17, LCMS-ESI (*m/z*) - found 361.17 $[M+H]^+$, 359.17 $[M-H]^-$

**Example 12-150 Compound 150**

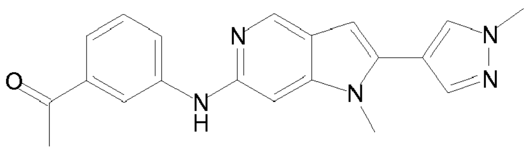

MW.: 345.16, LCMS-ESI (*m/z*) - found 346.15 $[M+H]^+$, 344.15 $[M-H]^-$

**Example 12-151 Compound 151**

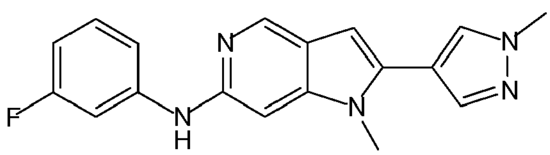

MW.: 321.14, LCMS-ESI (*m/z*) - found 322.13 $[M+H]^+$, 320.13 $[M-H]^-$

**Example 12-152 Compound 152**

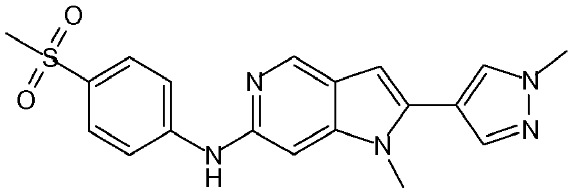

MW.:381.13, LCMS-ESI (*m/z*) - found 382.12 $[M+H]^+$, 380.12 $[M-H]^-$

**Example 12-153 Compound 153**

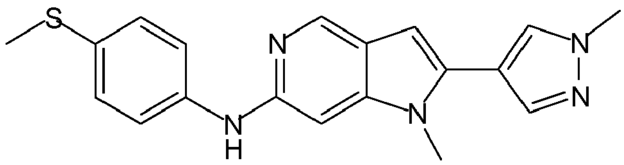

MW.: 349.14, LCMS-ESI (*m/z*) - found 350.13 $[M+H]^+$, 348.13 $[M-H]^-$

**Example 12-154 Compound 154**

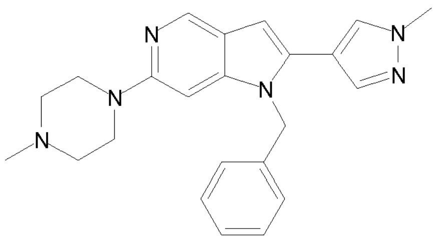

MW.: 386.22, LCMS-ESI (*m/z*) - found 387.22 $[M+H]^+$, 385.22 $[M-H]^-$

**Example 12-155 Compound 155**

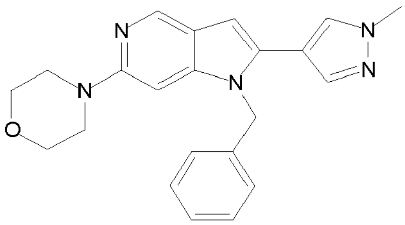

MW.: 373.19, LCMS-ESI (*m/z*) - found 374.19 $[M+H]^+$, 372.19 $[M-H]^-$

**Example 12-156 Compound 156**

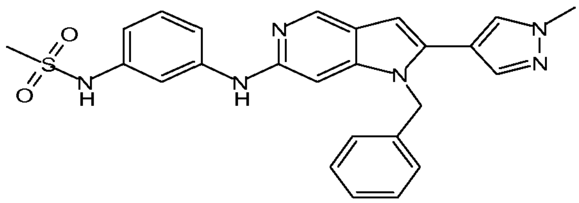

MW.: 472.17, LCMS-ESI (*m/z*) - found 473.16 $[M+H]^+$, 471.16 $[M-H]^-$

**Example 12-157 Compound 157**

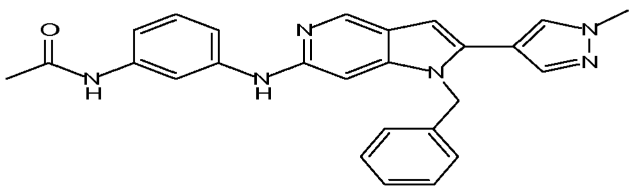

MW.: 436.20, LCMS-ESI (*m/z*) - found 437.20 $[M+H]^+$, 435.20 $[M-H]^-$

**Example 12-158 Compound 158**

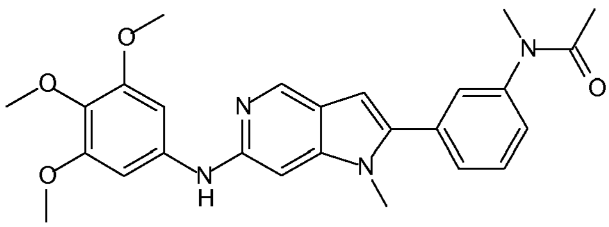

MW.: 460.21, LCMS-ESI (*m/z*) - found 461.21 $[M+H]^+$, 459.21 $[M-H]^-$

**Example 12-159 Compound 159**

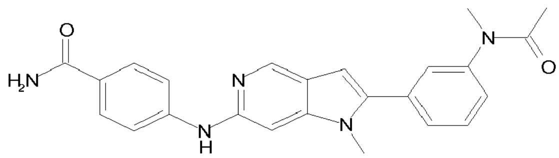

MW.: 413.19, LCMS-ESI (*m/z*) - found 414.18 $[M+H]^+$, 412.18 $[M-H]^-$

**Example 12-160 Compound 160**

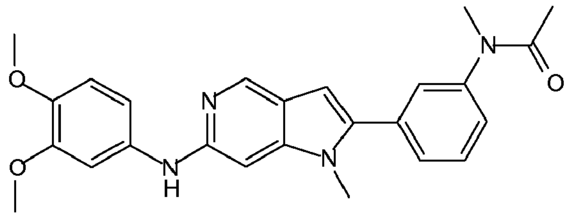

MW.: 430.20, LCMS-ESI (*m*/*z*) - found 431.20 $[M+H]^+$, 429.20 $[M-H]^-$

**Example 12-161 Compound 161**

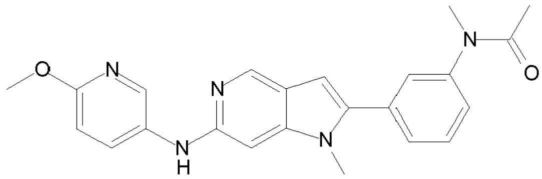

MW.: 401.19, LCMS-ESI (*m*/*z*) - found 402.18 $[M+H]^+$, 400.18 $[M-H]^-$

**Example 12-162 Compound 162**

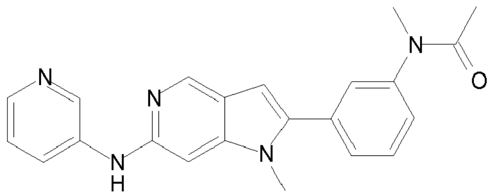

MW.: 371.17, LCMS-ESI (*m*/*z*) - found 372.17 $[M+H]^+$, 370.17 $[M-H]^-$

**Example 12-163 Compound 163**

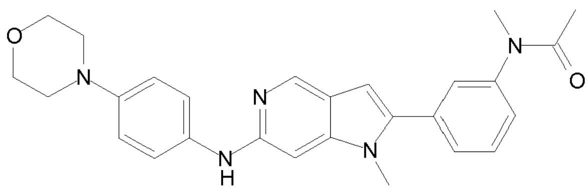

MW.: 455.23, LCMS-ESI (*m*/*z*) - found 456.23 $[M+H]^+$, 454.23 $[M-H]^-$

**Example 12-164 Compound 164**

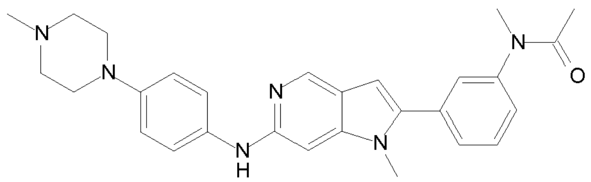

MW.: 468.26, LCMS-ESI (*m/z*) - found 469.26 $[M+H]^+$, 467.26 $[M-H]^-$

**Example 12-165 Compound 165**

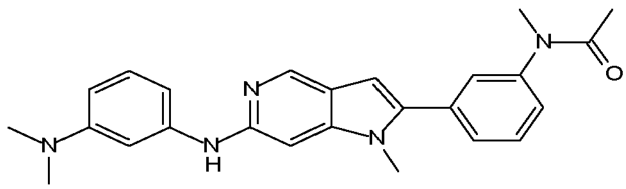

MW.: 413.22, LCMS-ESI (*m/z*) - found 414.22 $[M+H]^+$, 412.22 $[M-H]^-$

**Example 12-166 Compound 166**

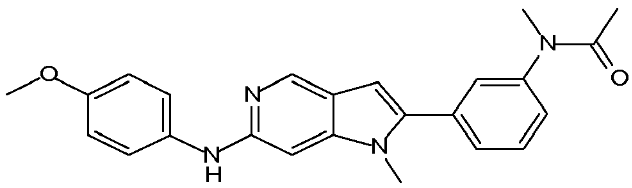

MW.: 400.19, LCMS-ESI (*m/z)* - found 401.19 $[M+H]^+$, 399.19 $[M-H]^-$

**Example 12-167 Compound 167**

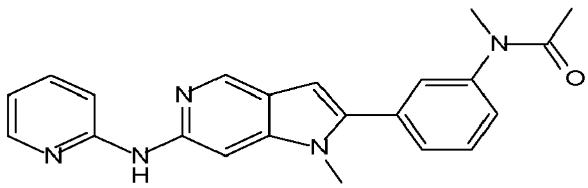

MW.: 371.17, LCMS-ESI (*m/z*) - found 372.17 $[M+H]^+$, 370.17 $[M-H]^-$

**Example** 12-168 **Compound 168**

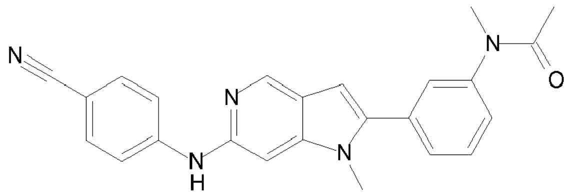

MW.: 395.17, LCMS-ESI (*m*/*z*) - found 396.17 $[M+H]^+$, 394.17 $[M-H]^-$

**Example 12-169 Compound 169**

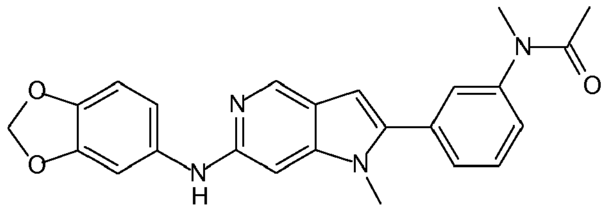

MW.: 414.17, LCMS-ESI (*m*/*z*) - found 415.16 $[M+H]^+$, 413.16 $[M-H]^-$

**Example 12-170 Compound 170**

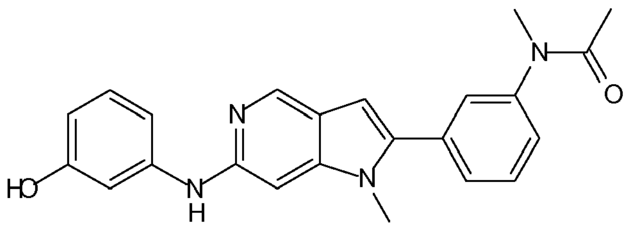

MW.: 386.17, LCMS-ESI (*m*/*z*) - found 387.17 $[M+H]^+$, 385.17 $[M-H]^-$

**Example 12-171 Compound 171**

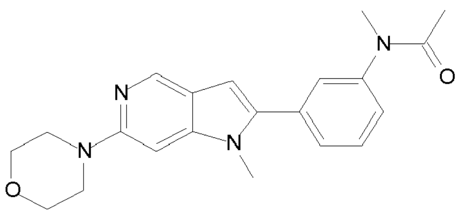

MW.: 364.19, LCMS-ESI (*m*/*z*) - found 365.19 $[M+H]^+$, 363.19 $[M-H]^-$

**Example 12-172 Compound 172**

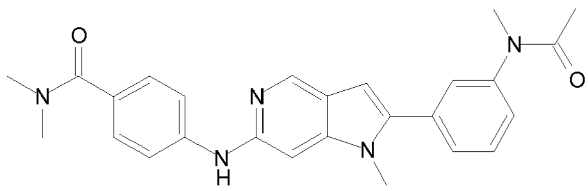

MW.: 441.22, LCMS-ESI (*m/z*) - found 442.21 $[M+H]^+$, 440.21 $[M-H]^-$

**Example 12-173 Compound 173**

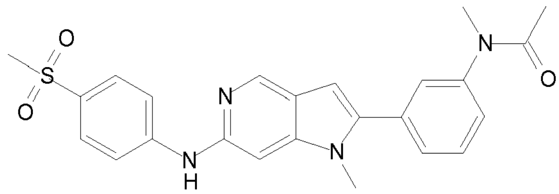

MW.: 448.16, LCMS-ESI (*m/z*) - found 449.15 $[M+H]^+$, 447.15 $[M-H]^-$

**Example 12-174 Compound 174**

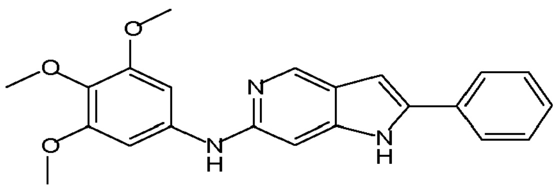

MW.: 375.16, LCMS-ESI (*m/z*) - found 376.15 $[M+H]^+$, 374.15 $[M-H]^-$

**Example 12-175 Compound 175**

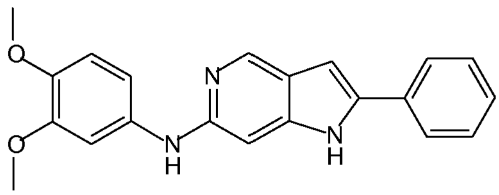

MW.: 345.15, LCMS-ESI (*m/z*) - found 346.14 $[M+H]^+$, 344.14 $[M-H]^-$

**Example 12-176 Compound 176**

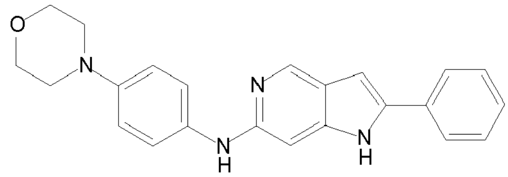

MW.: 370.18, LCMS-ESI (*m/z*) - found 371.17 $[M+H]^+$, 369.17 $[M-H]^-$

**Example 12-177 Compound 177**

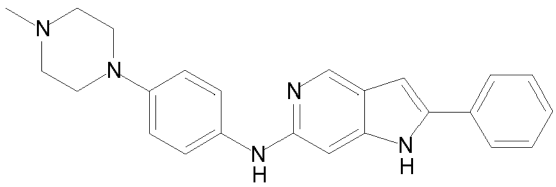

MW.: 383.21, LCMS-ESI (*m/z*) - found 384.21 $[M+H]^+$, 382.21 $[M-H]^-$

**Example 12-178 Compound 178**

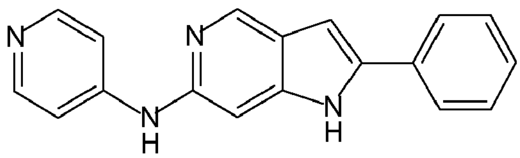

MW.: 286.12, LCMS-ESI (*m/z*) - found 287.12 $[M+H]^+$, 285.12 $[M-H]^-$

**Example 12-179 Compound 179**

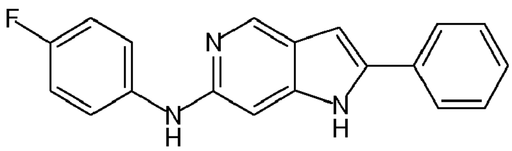

MW.: 303.12, LCMS-ESI (*m/z*) - found 304.12 $[M+H]^+$, 302.11 $[M-H]^-$

**Example 12-180 Compound 180**

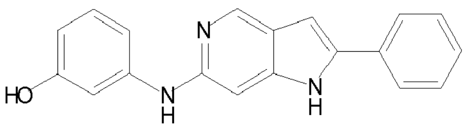

MW.: 301.12, LCMS-ESI (*m/z*) - found 302.12 $[M+H]^+$, 300.12 $[M-H]^-$

**Example 12-181 Compound 181**

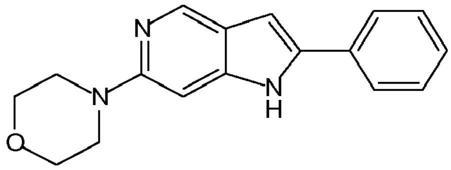

MW.: 279.14, LCMS-ESI (*m*/*z*) - found 280.13 $[M+H]^+$, 278.13 $[M-H]^-$

**Example 12-182 Compound 182**

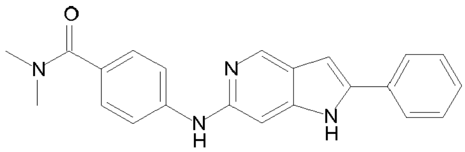

MW.: 356.16, LCMS-ESI (*m*/*z*) - found 357.16 $[M+H]^+$, 355.16 $[M-H]^-$

**Example 12-183 Compound 183**

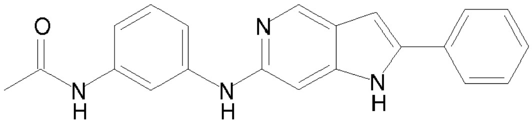

MW.: 342.15, LCMS-ESI (*m*/*z*) - found 343.14 $[M+H]^+$, 341.14 $[M-H]^-$

**Example 12-184 Compound 184**

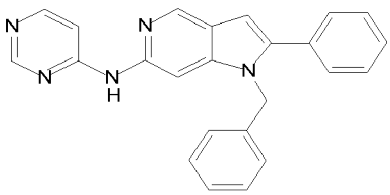

MW.: 377.16, LCMS-ESI (*m*/*z*) - found 378.16 $[M+H]^+$, 376.16 $[M-H]^-$

**Example 12-185 Compound 185**

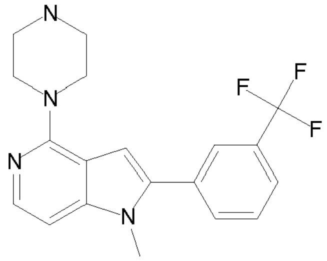

MW.: 374.17, LCMS-ESI (*m/z*) - found 375.17 $[M+H]^+$, 373.17 $[M-H]^-$

**Example 12-186 Compound 186**

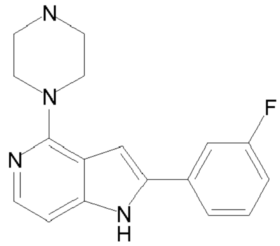

MW.: 310.16, LCMS-ESI (*m/z*) - found 311.15 $[M+H]^+$, 309.15 $[M-H]^-$

**Example 12-187 Compound 187**

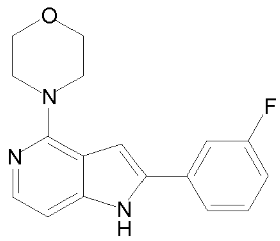

MW.: 297.13, LCMS-ESI (*m/z*) - found 298.12 $[M+H]^+$, 296.12 $[M-H]^-$

**Example 12-188 Compound 188**

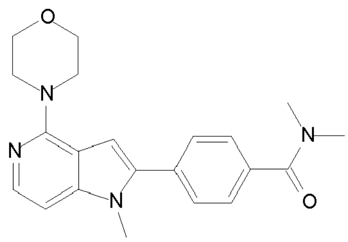

MW.: 364.19, LCMS-ESI (*m/z*) - found 365.19 $[M+H]^+$, 363.19 $[M-H]^-$

**Example 12-189 Compound 189**

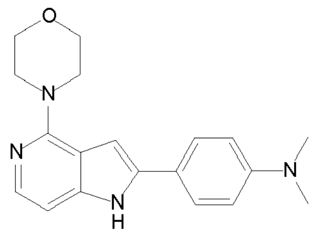

MW.: 322.18, LCMS-ESI (*m/z*) - found 323.17 $[M+H]^+$, 321.17 $[M-H]^-$

**Example 12-190 Compound 190**

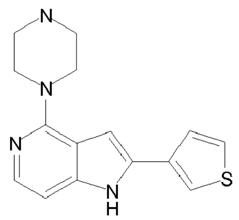

MW.: 298.13, LCMS-ESI (*m/z*) - found 299.12 $[M+H]^+$, 297.12 $[M-H]^-$

**Example 12-191 Compound 191**

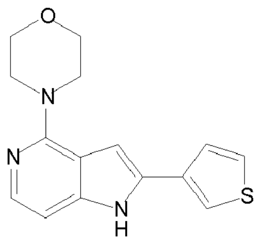

MW.: 285.09, LCMS-ESI (*m/z*) - found 286.09 $[M+H]^+$, 284.09 $[M-H]^-$

**Example 12-192 Compound 192**

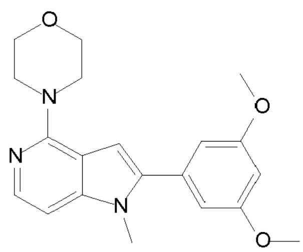

MW.: 353.17, LCMS-ESI (*m/z*) - found 354.17 $[M+H]^+$, 352.17 $[M-H]^-$

**Example 12-193 Compound 193**

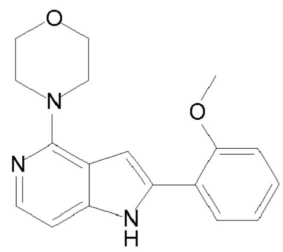

MW.: 342.15, LCMS-ESI (*m*/*z*) - found 343.14 $[M+H]^+$, 341.14 $[M-H]^-$

**Example 12-194 Compound 194**

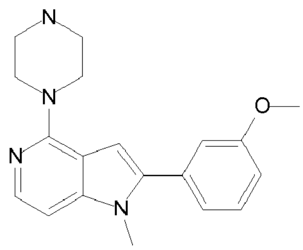

MW.: 336.19, LCMS-ESI (*m*/*z*) - found 337.19 $[M+H]^+$, 335.19 $[M-H]^-$

**Example 12-195 Compound 195**

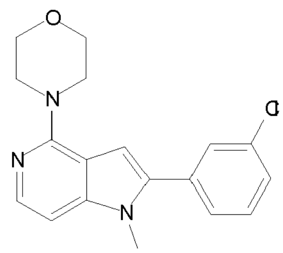

MW.: 327.11, LCMS-ESI (*m*/*z*) - found 328.11 $[M+H]^+$, 326.11 $[M-H]^-$

**Example 12-196 Compound 196**

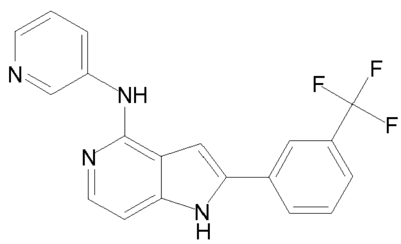

MW.: 354.11, LCMS-ESI (*m*/*z*) - found 355.10 $[M+H]^+$, 353.10 $[M-H]^-$

**Example 12-197 Compound 197**

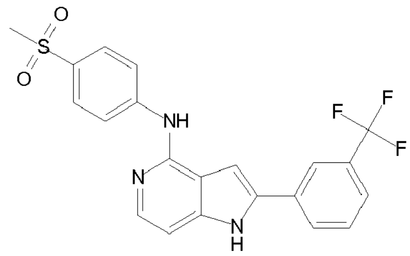

MW.: 431.09, LCMS-ESI (*m*/*z*) - found 432.09$[M+H]^+$, 430.09 $[M-H]^-$

**Example 12-198 Compound 198**

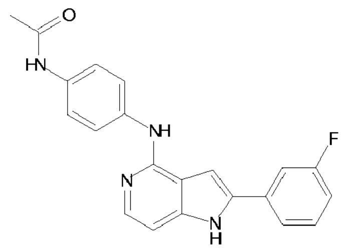

MW.: 360.14, LCMS-ESI (*m*/*z*) - found 361.13 $[M+H]^+$, 359.13 $[M-H]^-$

**Example 12-199 Compound 199**

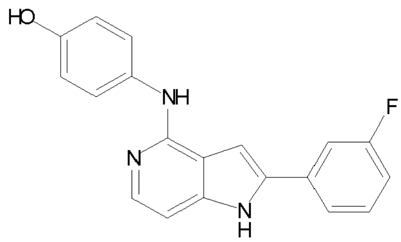

MW.: 319.11, LCMS-ESI (*m*/*z*) - found 320.11 $[M+H]^+$, 318.11 $[M-H]^-$

**Example 12-200 Compound 200**

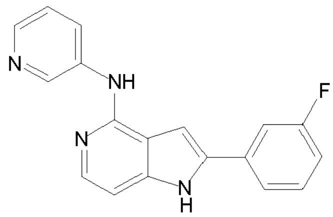

MW.: 304.11, LCMS-ESI (*m*/*z*) - found 305.11 $[M+H]^+$, 303.11 $[M-H]^-$

**Example 12-201 Compound 201**

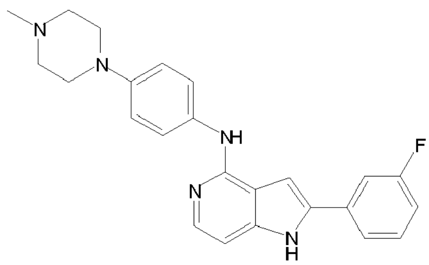

MW.: 401.20, LCMS-ESI (*m*/*z*) - found 402.2 $[M+H]^+$, 400.2 $[M-H]^-$

**Example 12-202 Compound 202**

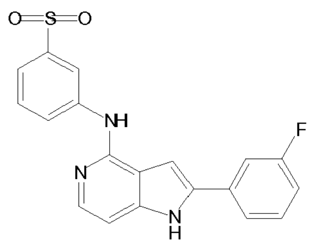

MW.: 381.09, LCMS-ESI (*m*/*z*) - found 382.09 $[M+H]^+$, 380.09 $[M-H]^-$

**Example 12-203 Compound 203**

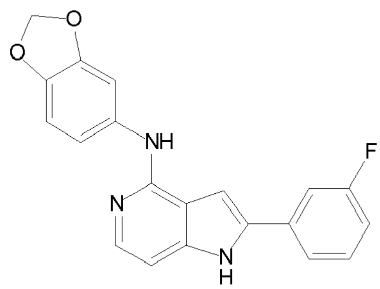

MW.: 347.11, LCMS-ESI (*m*/*z*) - found 348.10 $[M+H]^+$, 346.10 $[M-H]^-$

**Example 12-204 Compound 204**

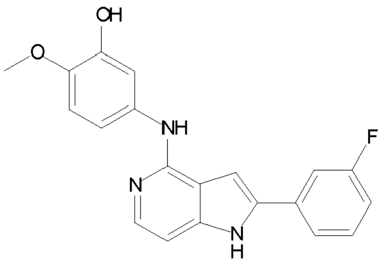

MW.: 349.12, LCMS-ESI (*m*/*z*) - found 350.12 $[M+H]^+$, 348.12 $[M-H]^-$

**Example 12-205 Compound 205**

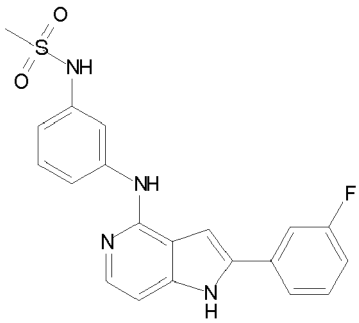

MW.: 396.11, LCMS-ESI (*m*/*z*) - found 397.1 $[M+H]^+$, 395.1 $[M-H]^-$

**Example 12-206 Compound 206**

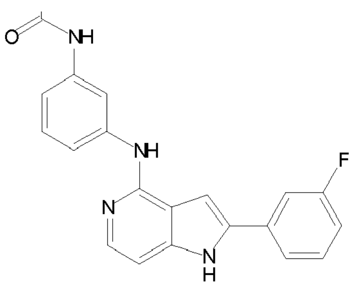

MW.: 360.14, LCMS-ESI (*m*/*z*) - found 361.13 $[M+H]^+$, 359.13 $[M-H]^-$

**Example 12-207 Compound 207**

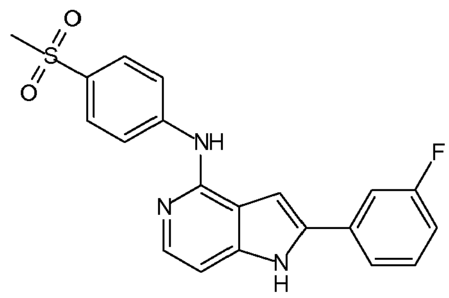

MW.: 381.09, LCMS-ESI (*m*/*z*) - found 382.09 $[M+H]^+$, 380.09 $[M-H]^-$

**Example 12-208 Compound 208**

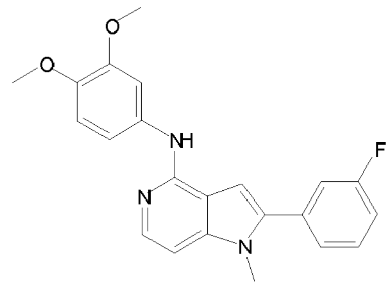

MW.: 377.15, LCMS-ESI (*m*/*z*) - found 378.15 $[M+H]^+$, 376.15 $[M-H]^-$

**Example 12-209 Compound 209**

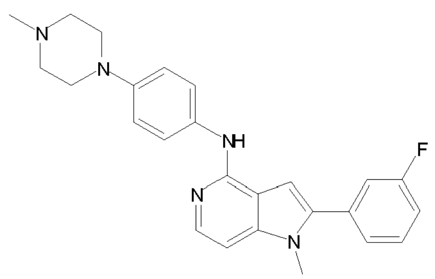

MW.: 415.22, LCMS-ESI (*m*/*z*) - found 416.21 $[M+H]^+$, 414.21 $[M-H]^-$

**Example 12-210 Compound 210**

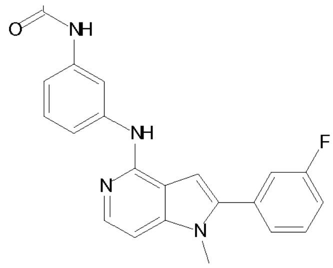

MW.: 374.15, LCMS-ESI (*m*/*z*) - found 375.15 $[M+H]^+$, 373.15 $[M-H]^-$

**Example 12-211 Compound 211**

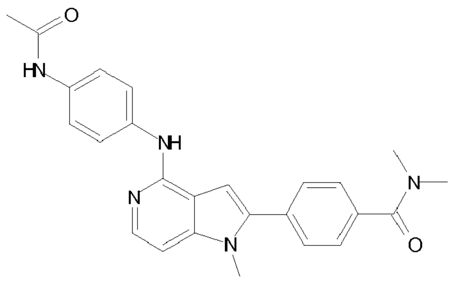

MW.: 427.20, LCMS-ESI (*m*/*z*) - found 428.2 $[M+H]^+$, 426.2 $[M-H]^-$

**Example 12-212 Compound 212**

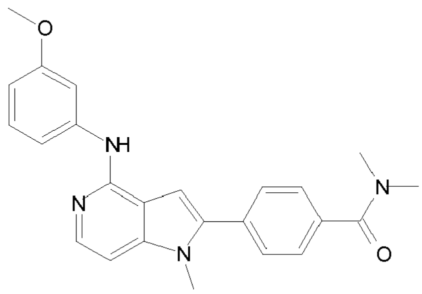

MW.: 400.19, LCMS-ESI (*m*/*z*) - found 401.19 $[M+H]^+$, 399.19 $[M-H]^-$

**Example 12-213 Compound 213**

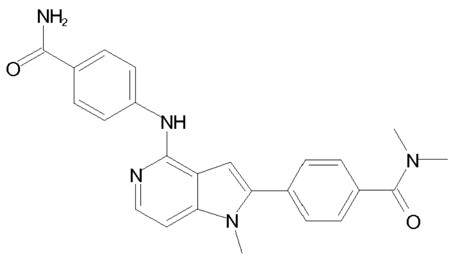

MW.: 413.19, LCMS-ESI (*m*/*z*) - found 414.18 $[M+H]^+$, 412.18 $[M-H]^-$

**Example 12-214 Compound 214**

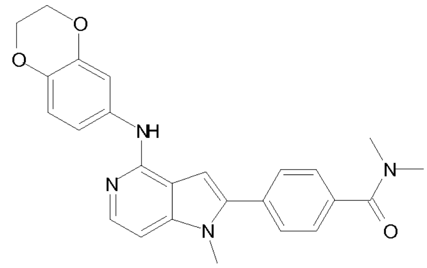

MW.: 428.18, LCMS-ESI (m/z) - found 429.18 $[M+H]^+$, 427.18 $[M-H]^-$

**Example 12-215 Compound 215**

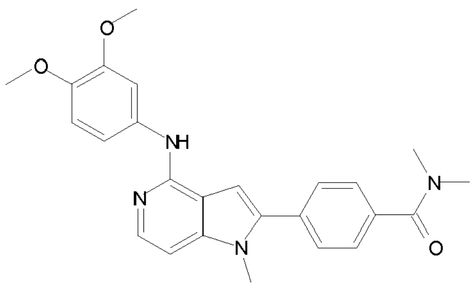

MW.: 430.20, LCMS-ESI (*m*/*z*) - found 431.2 $[M+H]^+$, 429.2 $[M-H]^-$

**Example 12-216 Compound 216**

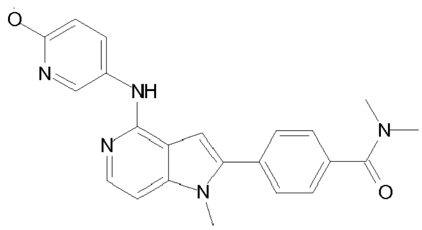

MW.: 401.19, LCMS-ESI (m/z) - found 402.18 $[M+H]^+$, 400.18 $[M-H]^-$

**Example 12-217 Compound 217**

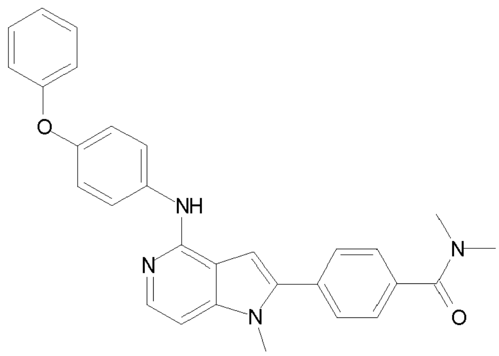

MW.: 462.21, LCMS-ESI (*m*/*z*) - found 463.2 $[M+H]^+$, 461.2 $[M-H]^-$

**Example 12-218 Compound 218**

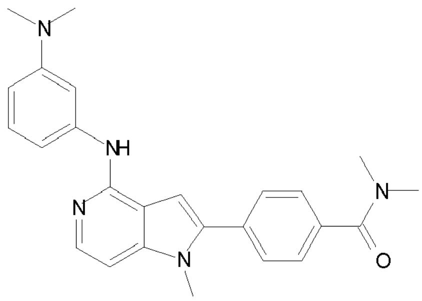

MW.: 413.22, LCMS-ESI (*m*/*z*) - found 414.22 $[M+H]^+$, 412.22 $[M-H]^-$

**Example 12-219 Compound 219**

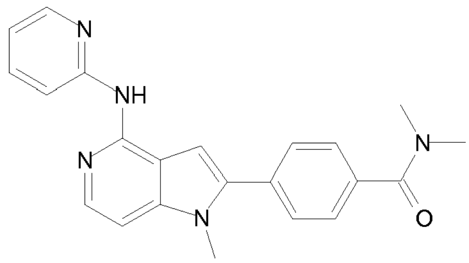

MW.: 371.17, LCMS-ESI (*m*/*z*) - found 372.17 $[M+H]^+$, 370.15 $[M-H]^-$

**Example 12-220 Compound 220**

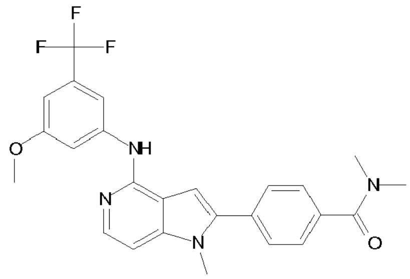

MW.: 468.18, LCMS-ESI (*m*/*z*) - found 469.17 $[M+H]^+$, 467.17 $[M-H]^-$

**Example 12-221 Compound 221**

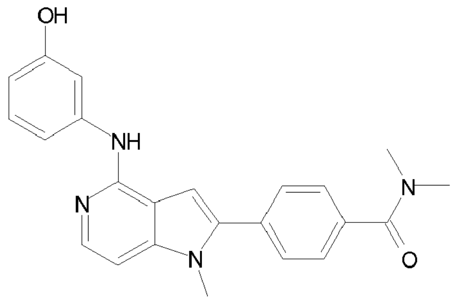

MW.: 386.17, LCMS-ESI (*m*/*z*) - found 387.17 $[M+H]^+$, 385.17 $[M-H]^-$

**Example 12-222 Compound 222**

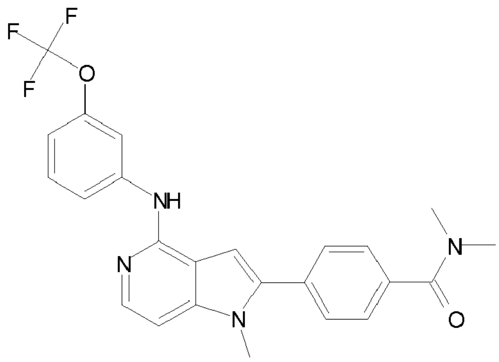

MW.: 454.16, LCMS-ESI (*m*/*z*) - found 455.16. $[M+H]^+$, 453.16 $[M-H]^-$

**Example 12-223 Compound 223**

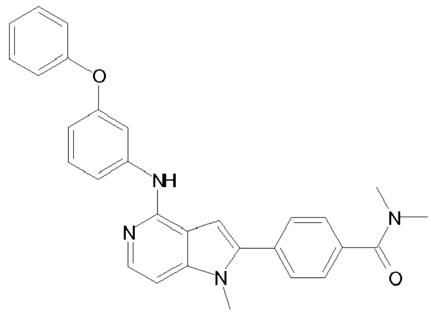

MW.: 462.21, LCMS-ESI ($m/z$) - found 463.2 $[M+H]^+$, 461.2 $[M-H]^-$

**Example 12-224 Compound 224**

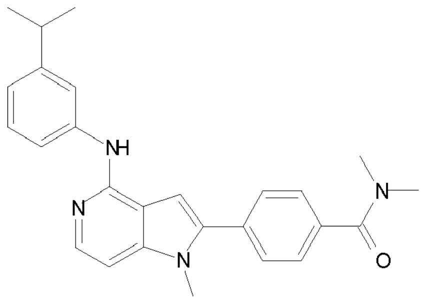

MW.: 412.23, LCMS-ESI ($m/z$) - found 413.22 $[M+H]^+$, 411.22 $[M-H]^-$

**Example 12-225 Compound 225**

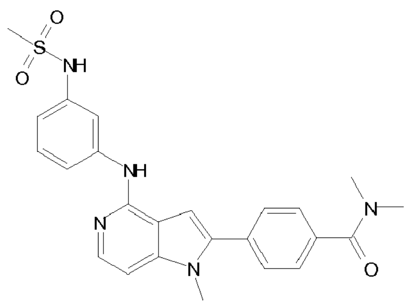

MW.: 463.17, LCMS-ESI ($m/z$) - found 464.16 $[M+H]^+$, 462.16 $[M-H]^-$

**Example 12-226 Compound 226**

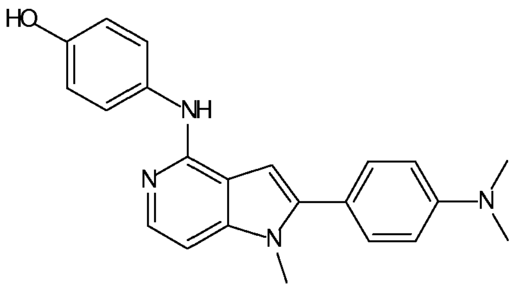

MW.: 358.18, LCMS-ESI (*m*/*z*) - found 359.17 $[M+H]^+$, 357.17 $[M-H]^-$

**Example 12-227 Compound 227**

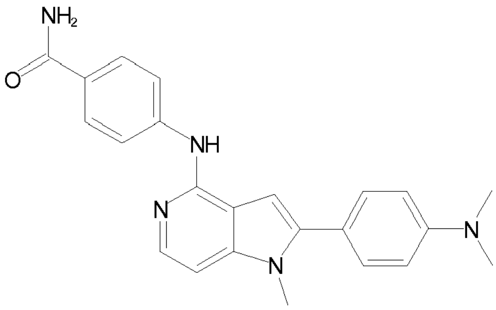

MW.: 385.19, LCMS-ESI (*m*/*z*) - found 386.19 $[M+H]^+$, 384.19 $[M-H]^-$

**Example 12-228 Compound 228**

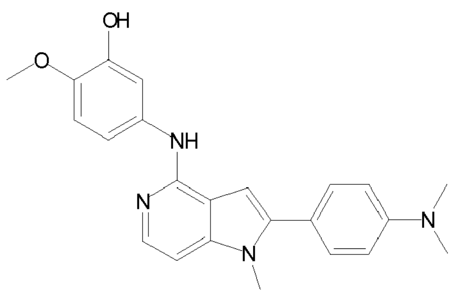

MW.: 388.19, LCMS-ESI (*m*/*z*) - found 389.19 $[M+H]^+$, 387.19 $[M-H]^-$

**Example 12-229 Compound 229**

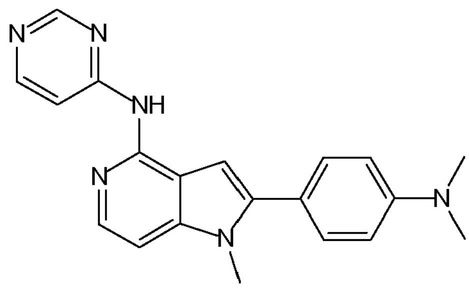

MW.: 344.17, LCMS-ESI (*m*/*z*) - found 345.17 $[M+H]^+$, 343.17 $[M-H]^-$

**Example 12-230 Compound 230**

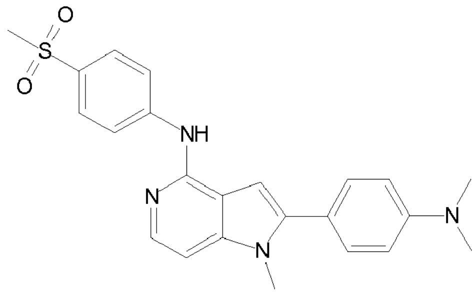

MW.: 420.16, LCMS-ESI (*m*/*z*) - found 421.16 $[M+H]^+$, 419.16 $[M-H]^-$

**Example 12-231 Compound 231**

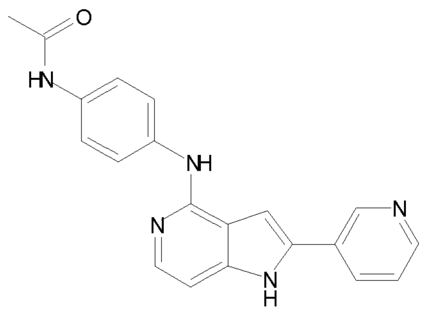

MW.: 343.14, LCMS-ESI (*m*/*z*) - found 344.14 $[M+H]^+$, 342.14 $[M-H]^-$

**Example 12-232 Compound 232**

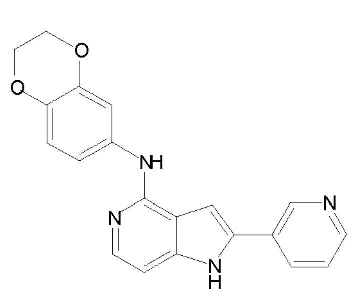

MW.: 344.13, LCMS-ESI (*m*/*z*) - found 345.12 $[M+H]^+$, 343.12 $[M-H]^-$

**Example 12-233 Compound 233**

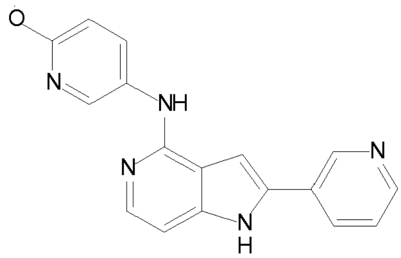

MW.: 317.13, LCMS-ESI (*m*/*z*) - found 318.12 $[M+H]^+$, 316.12 $[M-H]^-$

**Example 12-234 Compound 234**

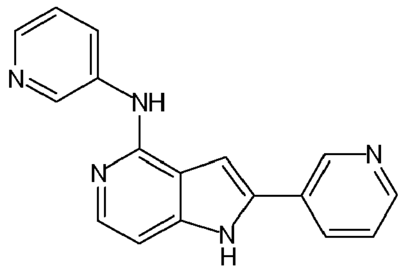

MW.: 287.12, LCMS-ESI (*m*/*z*) - found 288.11 $[M+H]^+$, 286.11 $[M-H]^-$

**Example 12-235 Compound 235**

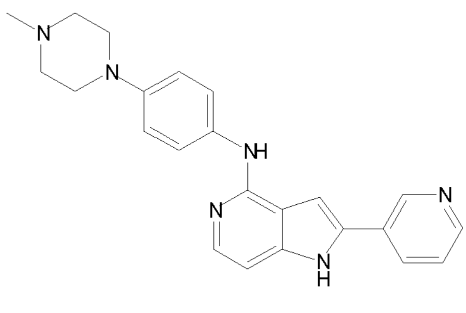

MW.: 384.21, LCMS-ESI ($m/z$) - found 385.2 $[M+H]^+$, 383.2 $[M-H]^-$

**Example 12-236 Compound 236**

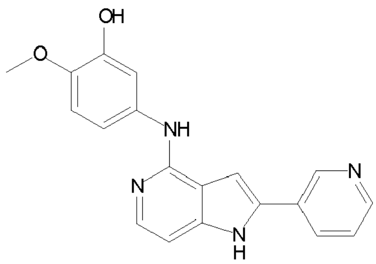

MW.: 332.13, LCMS-ESI ($m/z$) - found 333.12 $[M+H]^+$, 331.12 $[M-H]^-$

**Example 12-237 Compound 237**

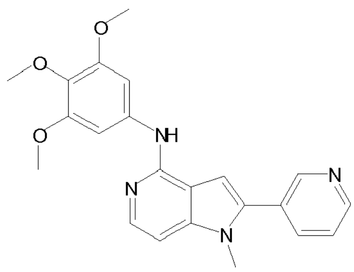

MW.: 390.17, LCMS-ESI ($m/z$) - found 391.16 $[M+H]^+$, 389.16 $[M-H]^-$

**Example 12-238 Compound 238**

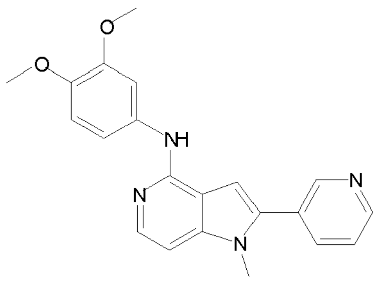

MW.: 360.16, LCMS-ESI ($m/z$) - found 361.15 $[M+H]^+$, 359.15 $[M-H]^-$

**Example 12-239 Compound 239**

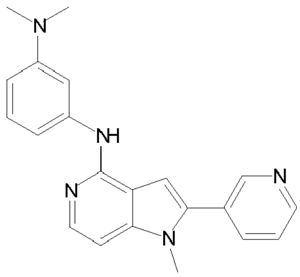

MW.: 343.18, LCMS-ESI (*m*/*z*) - found 344.18 $[M+H]^+$, 342.18 $[M-H]^-$

**Example 12-240 Compound 240**

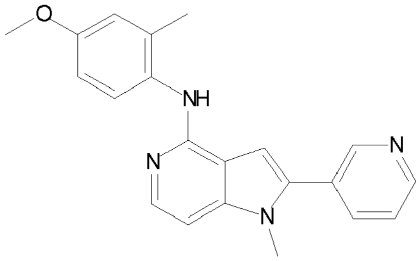

MW.: 344.16, LCMS-ESI (*m*/*z*) - found 345.16 $[M+H]^+$, 343.16 $[M-H]^-$

**Example 12-241 Compound 241**

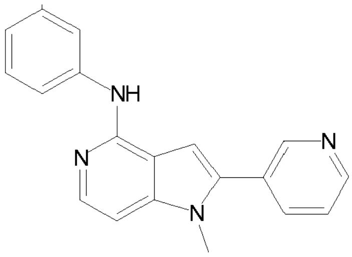

MW.: 314.15, LCMS-ESI (*m*/*z*) - found 315.15 $[M+H]^+$, 313.15 $[M-H]^-$

**Example 12-242 Compound 242**

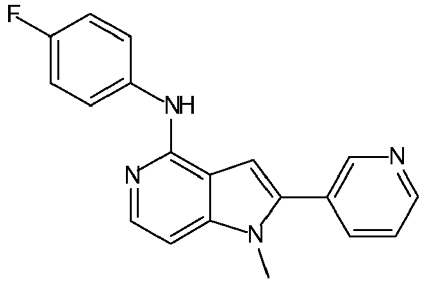

MW.: 318.13, LCMS-ESI ($m/z$) - found 319.13 $[M+H]^+$, 317.13 $[M-H]^-$

**Example 12-243 Compound 243**

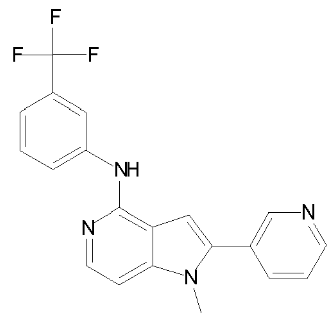

MW.: 368.12, LCMS-ESI ($m/z$) - found 369.12 $[M+H]^+$, 367.12 $[M-H]^-$

**Example 12-244 Compound 244**

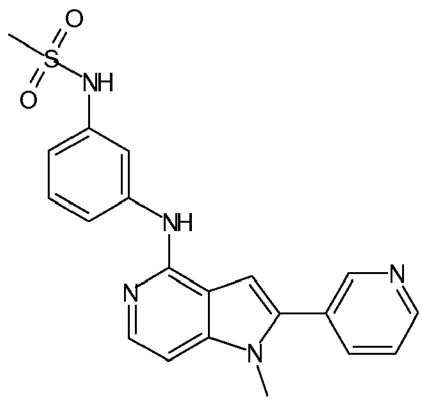

MW.: 393.13, LCMS-ESI ($m/z$) - found 394.12 $[M+H]^+$, 392.12 $[M-H]^-$

**Example 12-245 Compound 245**

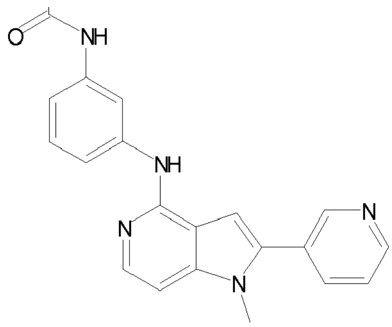

MW.: 357.16, LCMS-ESI ($m/z$) - found 358.15 $[M+H]^+$, 356.15 $[M-H]^-$

**Example 12-246 Compound 246**

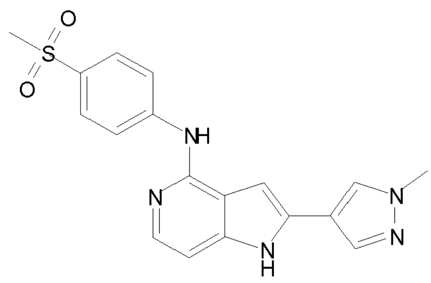

MW.: 367.11, LCMS-ESI (*m*/*z*) - found 368.11 $[M+H]^+$, 366.11 $[M-H]^-$

**Example 12-247 Compound 247**

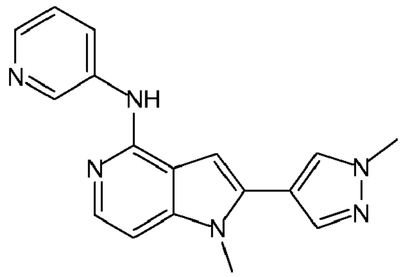

MW.: 304.14, LCMS-ESI (*m*/*z*) - found 305.14 $[M+H]^+$, 303.14 $[M-H]^-$

**Example 12-248 Compound 248**

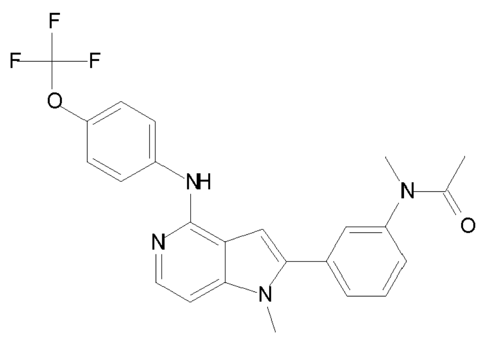

MW.: 454.16, LCMS-ESI (*m*/*z*) - found 455.16 $[M+H]^+$, 453.16 $[M-H]^-$

**Example 12-249 Compound 249**

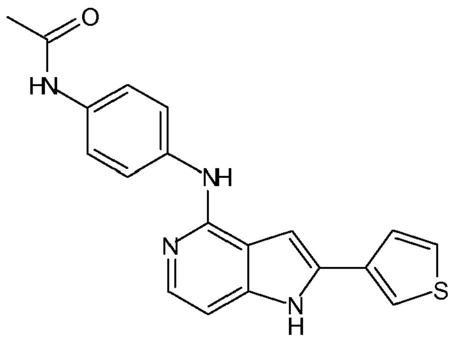

MW.: 348.10, LCMS-ESI ($m/z$) - found 349.10 $[M+H]^+$, 347.10 $[M-H]^-$

**Example 12-250 Compound 250**

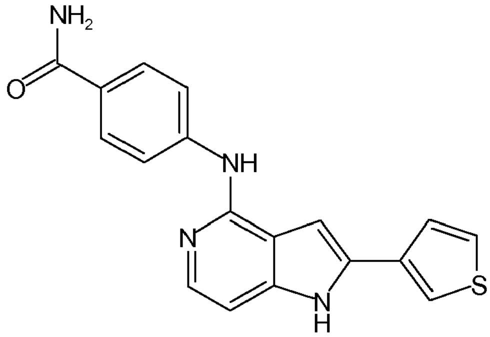

MW.: 334.09, LCMS-ESI ($m/z$) - found 335.08 $[M+H]^+$, 333.08 $[M-H]^-$

**Example 12-251 Compound 251**

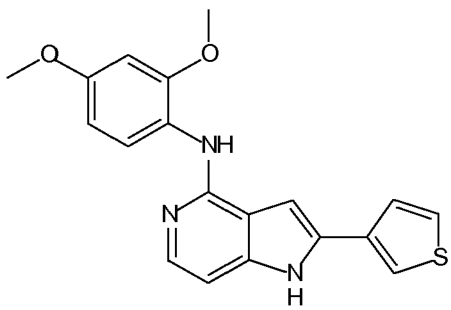

MW.: 351.10, LCMS-ESI ($m/z$) - found 352.10 $[M+H]^+$, 350.1 $[M-H]^-$

**Example 12-252 Compound 252**

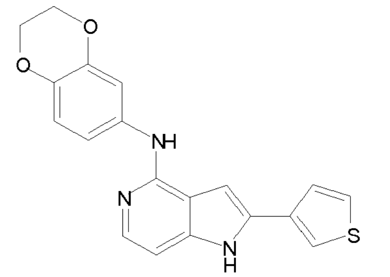

MW.: 349.09, LCMS-ESI (*m*/*z*) - found 350.08 $[M+H]^+$, 348.08 $[M-H]^-$

**Example 12-253 Compound 253**

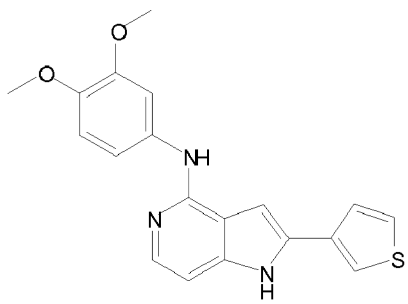

MW.: 351.10, LCMS-ESI (*m*/*z*) - found 352.10 $[M+H]^+$, 350.10 $[M-H]^-$

**Example 12-254 Compound 254**

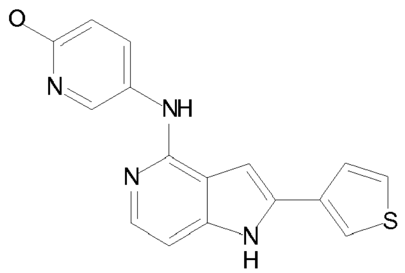

MW.: 322.09, LCMS-ESI (*m*/*z*) - found 323.08 $[M+H]^+$, 321.08 $[M-H]^-$

**Example 12-255 Compound 255**

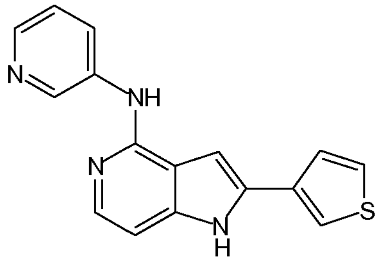

MW.: 292.08, LCMS-ESI ($m/z$) - found 293.07 $[M+H]^+$, 291.07 $[M-H]^-$

**Example 12-256 Compound 256**

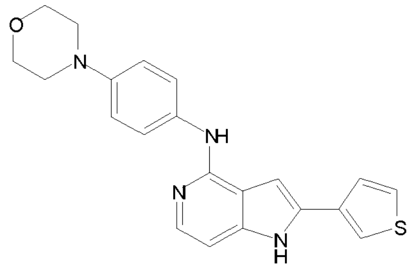

MW.: 376.14, LCMS-ESI ($m/z$) - found 377.13 $[M+H]^+$, 375.13 $[M-H]^-$

**Example 12-257 Compound 257**

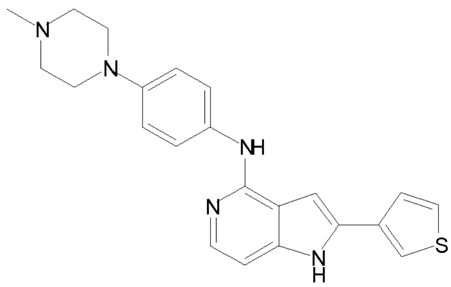

MW.: 389.17, LCMS-ESI ($m/z$) - found 390.16 $[M+H]^+$, 388.16 $[M-H]^-$

**Example 12-258 Compound 258**

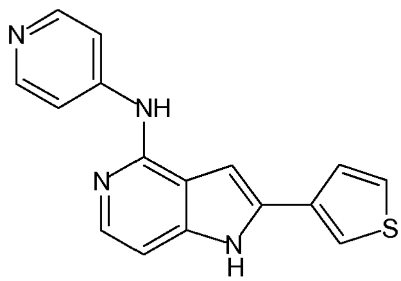

MW.: 292.08, LCMS-ESI ($m/z$) - found 293.07 $[M+H]^+$, 291.07 $[M-H]^-$

**Example 12-259 Compound 259**

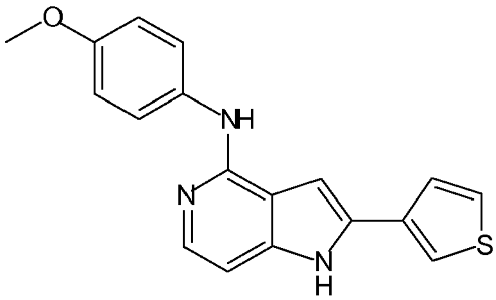

MW.: 321.09, LCMS-ESI (*m/z*) - found 322.09 $[M+H]^+$, 320.09 $[M-H]^-$

**Example 12-260 Compound 260**

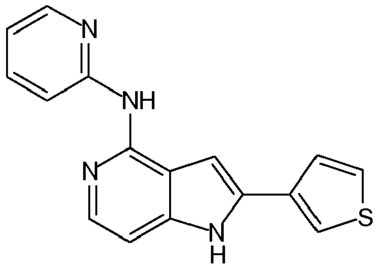

MW.: 292.08, LCMS-ESI (*m/z*) - found 293.07 $[M+H]^+$, 291.07 $[M-H]^-$

**Example 12-261 Compound 261**

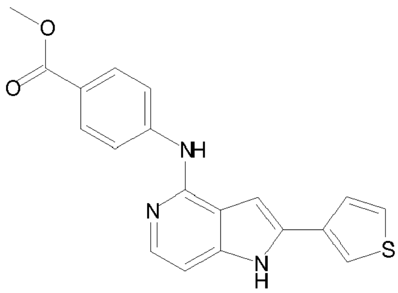

MW.: 349.09, LCMS-ESI (*m/z*) - found 350.08 $[M+H]^+$, 348.08 $[M-H]^-$

**Example 12-262 Compound 262**

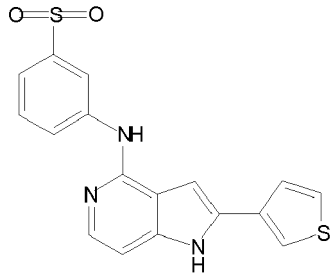

MW.: 369.06, LCMS-ESI (*m/z*) - found 370.06 $[M+H]^+$, 368.06 $[M-H]^-$

**Example 12-263 Compound 263**

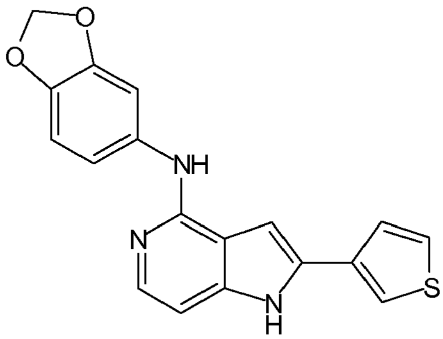

MW.: 335.07, LCMS-ESI (*m/z*) - found 336.07 $[M+H]^+$, 334.07 $[M-H]^-$

**Example 12-264 Compound 264**

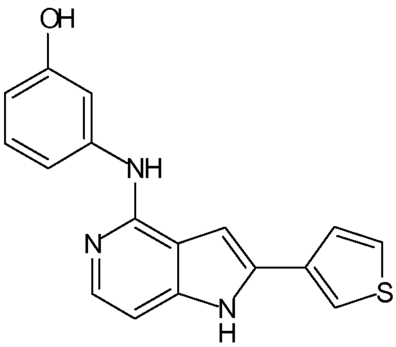

MW.: 307.08, LCMS-ESI (*m/z*) - found 308.07 $[M+H]^+$, 306.07 $[M-H]^-$

**Example 12-265 Compound 265**

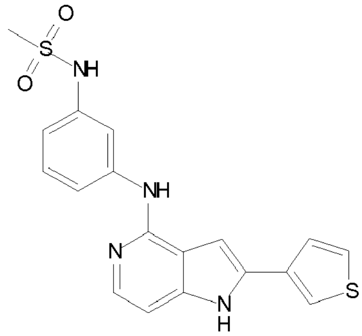

MW.: 384.07, LCMS-ESI (*m*/*z*) - found 385.07 $[M+H]^+$, 383.07 $[M-H]^-$

**Example 12-266 Compound 266**

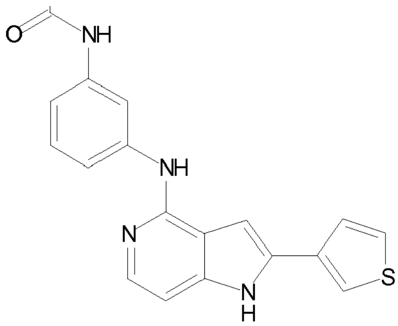

MW.: 348.10, LCMS-ESI (*m*/*z*) - found 349.1 $[M+H]^+$, 347.1 $[M-H]^-$

**Example 12-267 Compound 267**

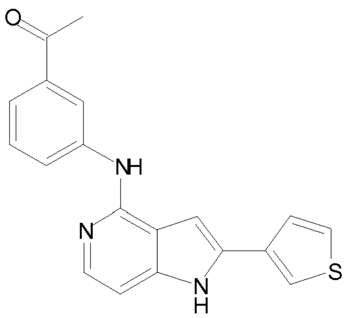

MW.: 333.09, LCMS-ESI (*m*/*z*) - found 334.09 $[M+H]^+$, 332.09 $[M-H]^-$

**Example 12-268 Compound 268**

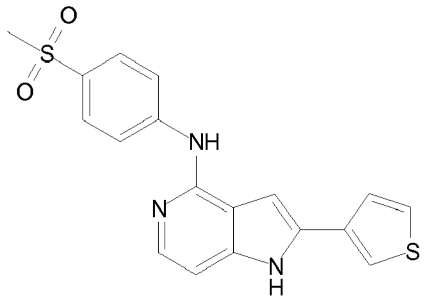

MW.: 369.06, LCMS-ESI (*m*/*z*) - found 370.06 $[M+H]^+$, 368.06 $[M-H]^-$

**Example 12-269 Compound 269**

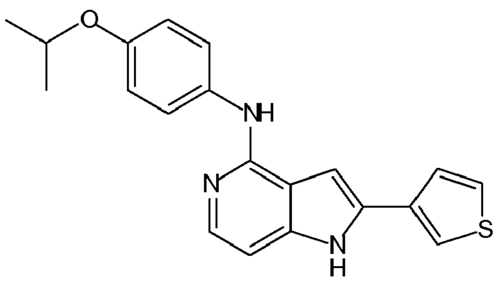

MW.: 349.12, LCMS-ESI (*m*/*z*) - found 350.12 $[M+H]^+$, 348.12 $[M-H]^-$

**Example 12-270 Compound 270**

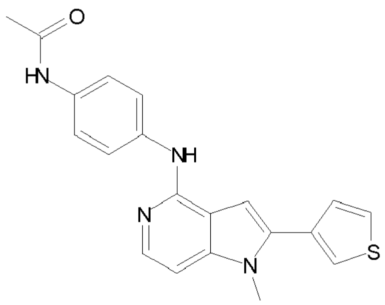

MW.: 362.12, LCMS-ESI (*m*/*z*) - found 363.12 $[M+H]^+$, 361.12 $[M-H]^-$

**Example 12-271 Compound 271**

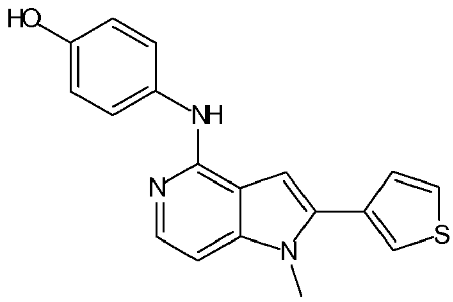

MW.: 321.09, LCMS-ESI (*m*/*z*) - found 322.09 $[M+H]^+$, 320.09 $[M-H]^-$

**Example 12-272 Compound 272**

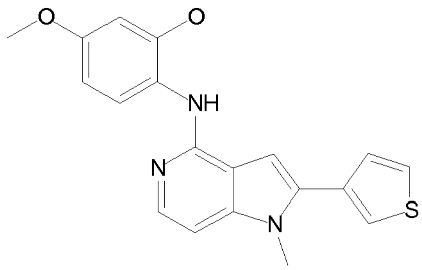

MW.: 365.12, LCMS-ESI (*m*/*z*) - found 366.12 $[M+H]^+$, 364.12 $[M-H]^-$

**Example 12-273 Compound 273**

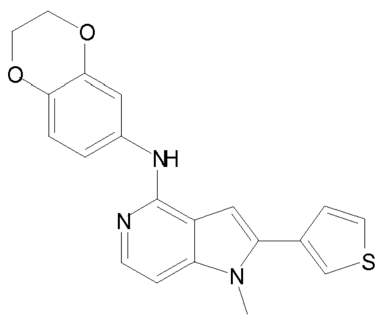

MW.: 363.10, LCMS-ESI (*m*/*z*) - found 364.10 $[M+H]^+$, 362.10 $[M-H]^-$

**Example 12-274 Compound 274**

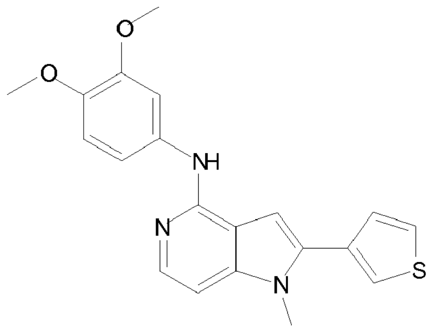

MW.: 365.12, LCMS-ESI (*m*/*z*) - found 366.12 $[M+H]^+$, 364.12 $[M-H]^-$

**Example 12-275 Compound 275**

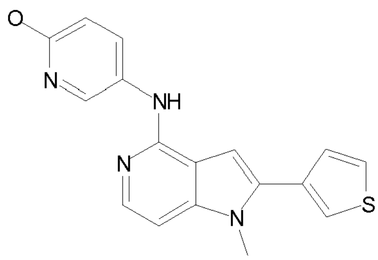

MW.: 336.10, LCMS-ESI (*m*/*z*) - found 337.10 $[M+H]^+$, 335.10 $[M-H]^-$

**Example 12-276 Compound 276**

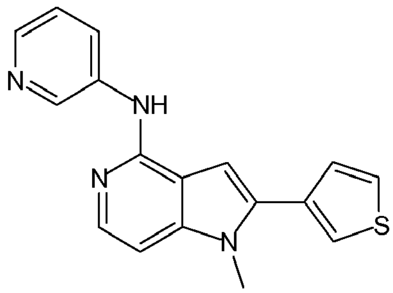

MW.: 306.09, LCMS-ESI (*m*/*z*) - found 307.09 $[M+H]^+$, 305.09 $[M-H]^-$

**Example 12-277 Compound 277**

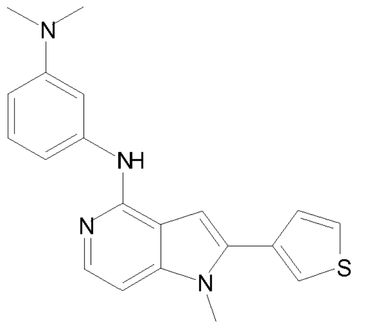

MW.: 348.14, LCMS-ESI (*m*/*z*) - found 349.14 $[M+H]^+$, 347.14 $[M-H]^-$

**Example 12-278 Compound 278**

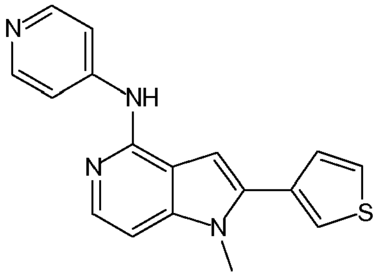

MW.: 306.09, LCMS-ESI (*m*/*z*) - found 307.09 $[M+H]^+$, 305.09 $[M-H]^-$

**Example 12-279 Compound 279**

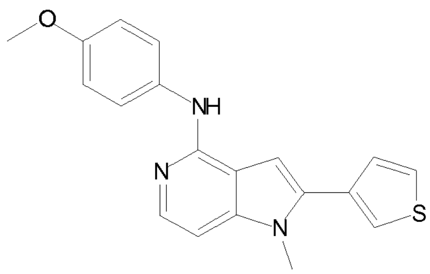

MW.: 335.11, LCMS-ESI (*m*/*z*) - found 336.1 $[M+H]^+$, 334.1 $[M-H]^-$

**Example 12-280 Compound 280**

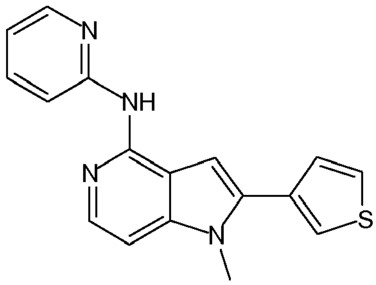

MW.: 306.09, LCMS-ESI (*m*/*z*) - found 307.09 $[M+H]^+$, 305.09 $[M-H]^-$

**Example 12-281 Compound 281**

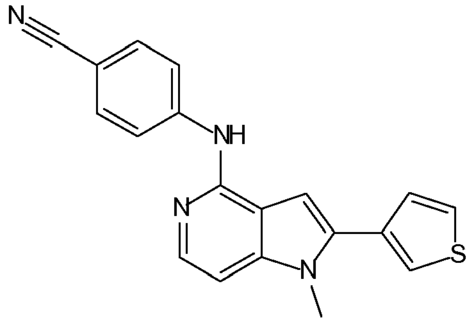

MW.: 330.09, LCMS-ESI (*m*/*z*) - found 331.09 $[M+H]^+$, 329.09 $[M-H]^-$

**Example 12-282 Compound 282**

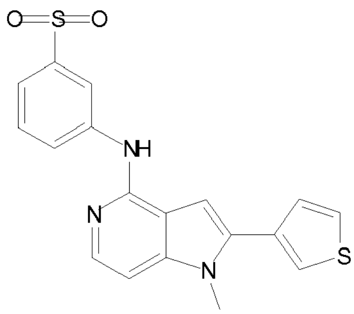

MW.: 383.08, LCMS-ESI (*m*/*z*) - found 384.02 $[M+H]^+$, 382.07 $[M-H]^-$

**Example 12-283 Compound 283**

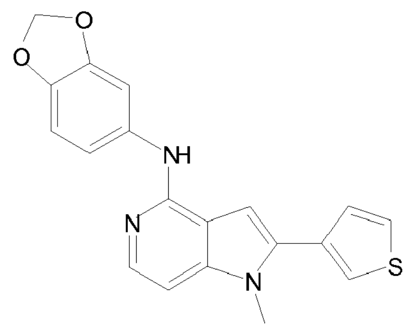

MW.: 349.09, LCMS-ESI (*m*/*z*) - found 350.08 $[M+H]^+$, 348.08 $[M-H]^-$

**Example 12-284 Compound 284**

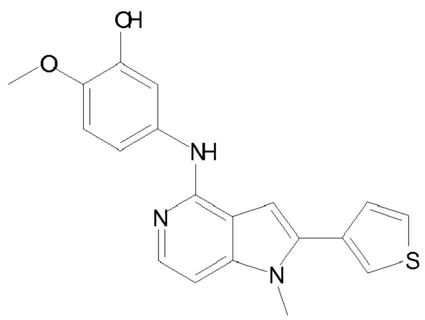

MW.: 351.10, LCMS-ESI (*m*/*z*) - found 352.10 $[M+H]^+$, 350.10 $[M-H]^-$

**Example 12-285 Compound 285**

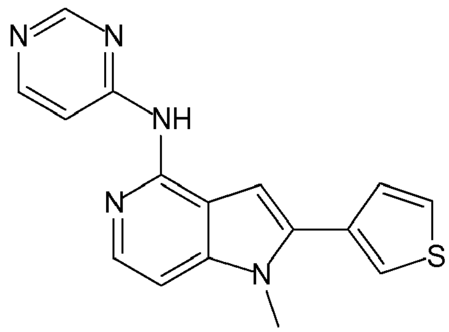

MW.: 307.09, LCMS-ESI (*m*/*z*) - found 308.08 $[M+H]^+$, 306.08 $[M-H]^-$

**Example 12-286 Compound 286**

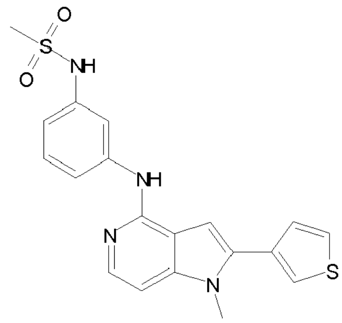

MW.: 398.08, LCMS-ESI (*m*/*z*) - found 399.08 $[M+H]^+$, 397.08 $[M-H]^-$

**Example 12-287 Compound 287**

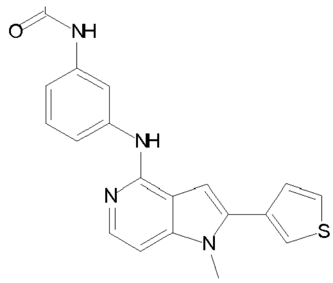

MW.: 362.12, LCMS-ESI (*m*/*z*) - found 363.12 $[M+H]^+$, 361.12 $[M-H]^-$

**Example 12-288 Compound 288**

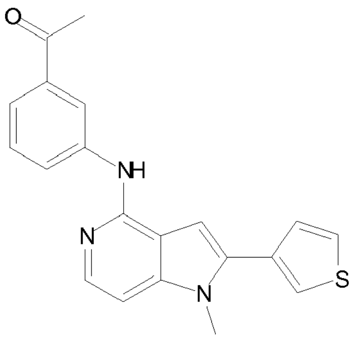

MW.: 347.11, LCMS-ESI (*m*/*z*) - found 348.10 $[M+H]^+$, 346.10 $[M-H]^-$

**Example 12-289 Compound 289**

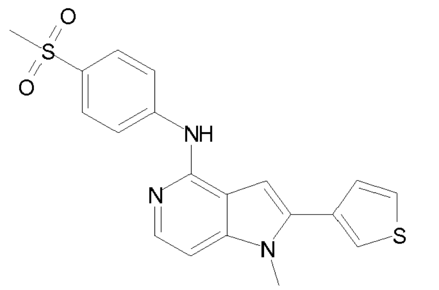

MW.: 383.08, LCMS-ESI (*m*/*z*) - found 384.07 $[M+H]^+$, 382.07 $[M-H]^-$

**Example 12-290 Compound 290**

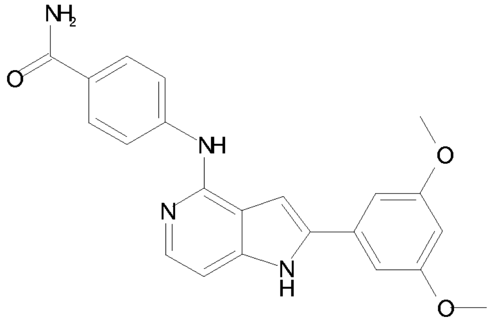

MW.: 388.15, LCMS-ESI (*m*/*z*) - found 389.15 $[M+H]^+$, 387.15 $[M-H]^-$

**Example 12-291 Compound 291**

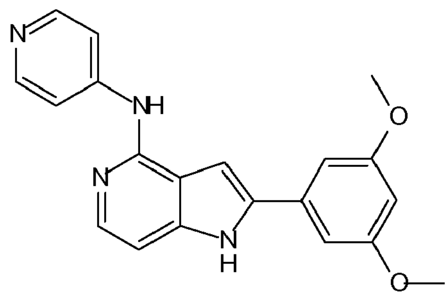

MW.: 346.14, LCMS-ESI (*m*/*z*) - found 347.14 $[M+H]^+$, 345.14 $[M-H]^-$

**Example 8-292 Compound 292**

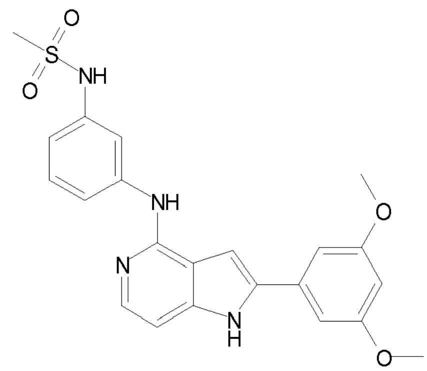

MW.: 438.14, LCMS-ESI (*m*/*z*) - found 439.13 $[M+H]^+$, 437.13 $[M-H]^-$

**Example 12-293 Compound 293**

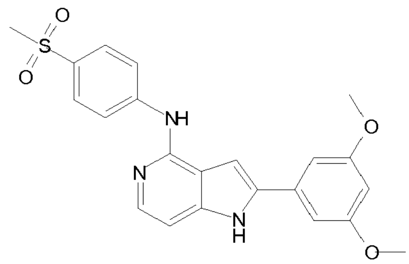

MW.: 423.13, LCMS-ESI (*m*/*z*) - found 424.13 $[M+H]^+$, 422.12 $[M-H]^-$

**Example 8-294 Compound 294**

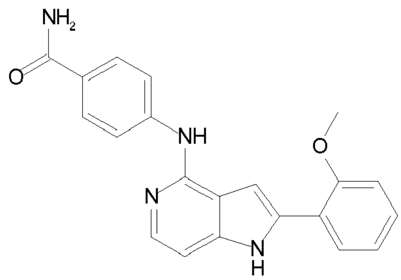

MW.: 358.14, LCMS-ESI (*m*/*z*) - found 359.14 $[M+H]^+$, 357.14 $[M-H]^-$

**Example 12-295 Compound 295**

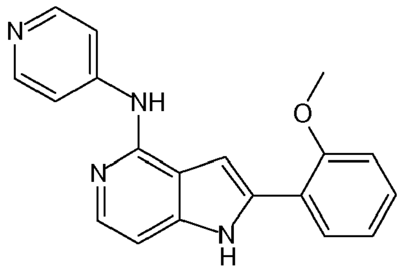

MW.: 316.13, LCMS-ESI (*m*/*z*) - found 317.13 $[M+H]^+$, 315.13 $[M-H]^-$

**Example 12-296 Compound 296**

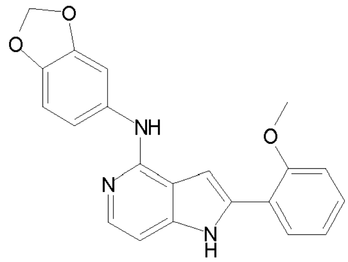

MW.: 359.13, LCMS-ESI (*m*/*z*) - found 360.12 $[M+H]^+$, 358.12 $[M-H]^-$

**Example 12-297 Compound 297**

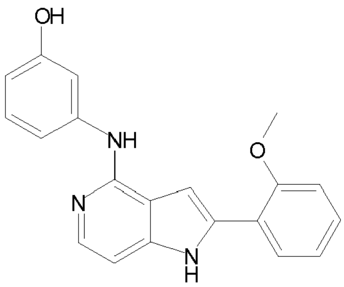

MW.: 331.13, LCMS-ESI (*m*/*z*) - found 332.13 $[M+H]^+$, 330.13 $[M-H]^-$

**Example 12-298 Compound 298**

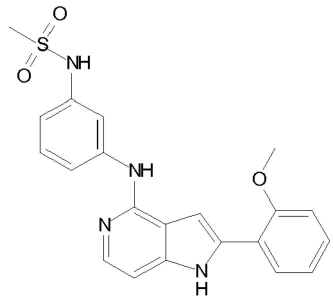

MW.: 408.13, LCMS-ESI (*m*/*z*) - found 409.12 $[M+H]^+$, 407.12 $[M-H]^-$

**Example 12-299 Compound 299**

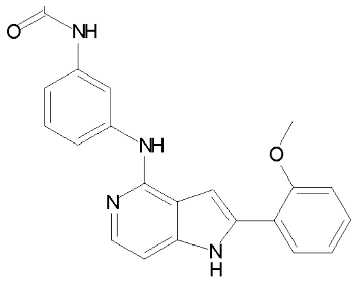

MW.: 372.16, LCMS-ESI (*m*/*z*) - found 373.15 $[M+H]^+$, 371.15 $[M-H]^-$

**Example 12-300 Compound 300**

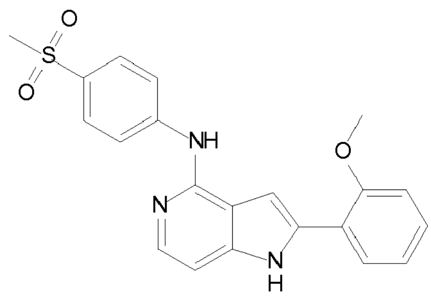

MW.: 393.11, LCMS-ESI (*m*/*z*) - found 394.11 $[M+H]^+$, 392.11 $[M-H]^-$

**Example 12-301 Compound 301**

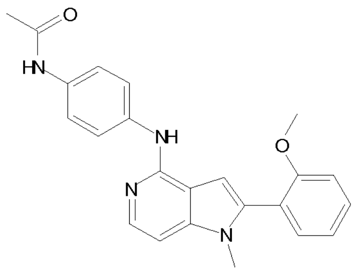

MW.: 386.17, LCMS-ESI (*m*/*z*) - found 387.17 $[M+H]^+$, 385.17 $[M-H]^-$

**Example 12-302 Compound 302**

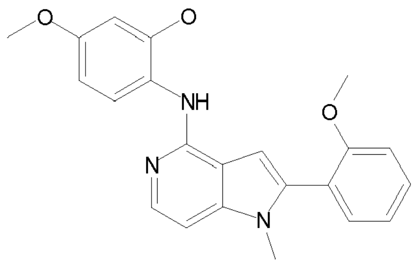

MW.: 389.17, LCMS-ESI (*m*/*z*) - found 390.17 $[M+H]^+$, 388.17 $[M-H]^-$

**Example 12-303 Compound 303**

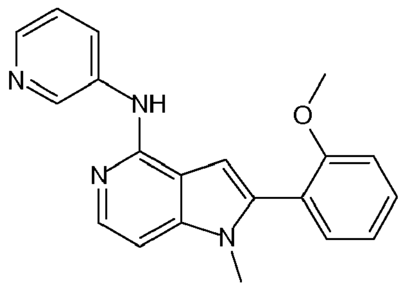

MW.: 330.15, LCMS-ESI (*m*/*z*) - found 331.14 $[M+H]^+$, 329.14 $[M-H]^-$

**Example 12-304 Compound 304**

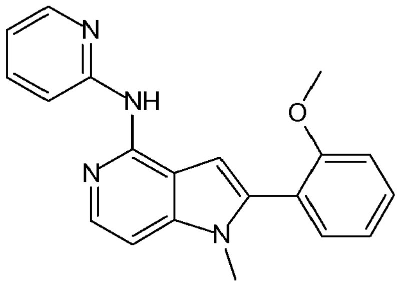

MW.: 330.15, LCMS-ESI (*m*/*z*) - found 331.14 $[M+H]^+$, 329.14 $[M-H]^-$

**Example 12-305 Compound 305**

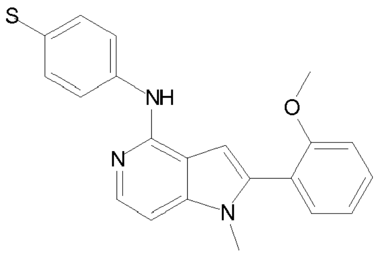

MW.: 375.14, LCMS-ESI (*m*/*z*) - found 376.14 $[M+H]^+$, 374.14 $[M-H]^-$

**Example 12-306 Compound 306**

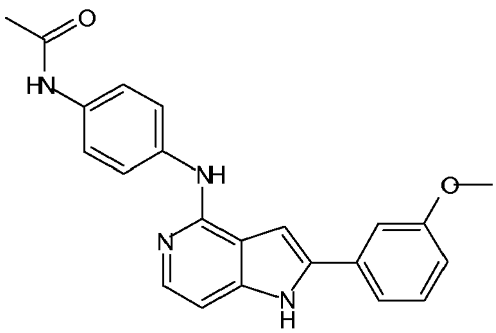

MW.: 372.16, LCMS-ESI (*m*/*z*) - found 373.15 $[M+H]^+$, 371.15 $[M-H]^-$

**Example 12-307 Compound 307**

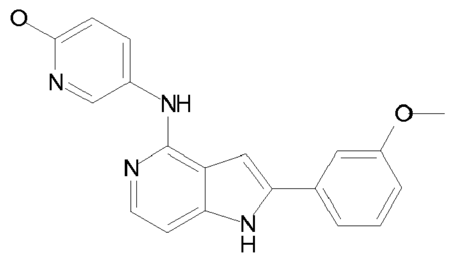

MW.: 346.14, LCMS-ESI (*m*/*z*) - found 347.14 $[M+H]^+$, 345.14 $[M-H]^-$

**Example 12-308 Compound 308**

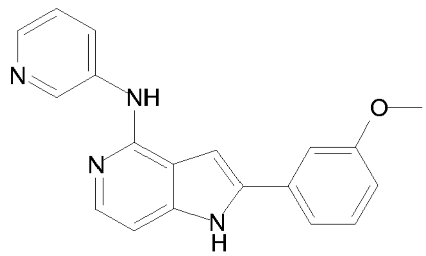

MW.: 316.13, LCMS-ESI (*m*/*z*) - found 317.13 $[M+H]^+$, 315.13 $[M-H]^-$

**Example 12-309 Compound 309**

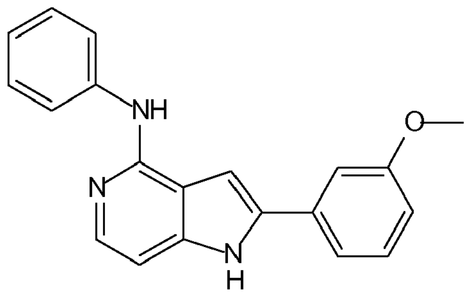

MW.: 315.14, LCMS-ESI (*m*/*z*) - found 316.13 $[M+H]^+$, 314.13 $[M-H]^-$

**Example 12-310 Compound 310**

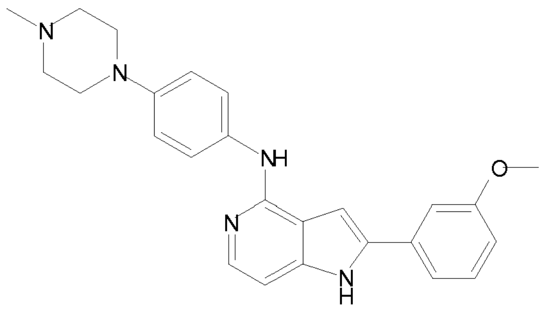

MW.: 413.22, LCMS-ESI (*m/z*) - found 414.22 $[M+H]^+$, 412.22 $[M-H]^-$

**Example 12-311 Compound 311**

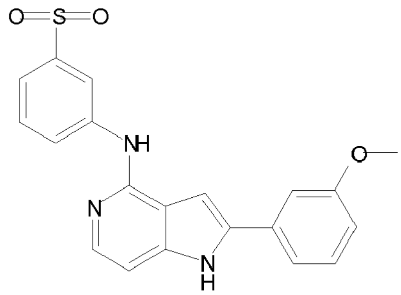

MW.: 393.11, LCMS-ESI (*m/z*) - found 394.11 $[M+H]^+$, 392.11 $[M-H]^-$

**Example 12-312 Compound 312**

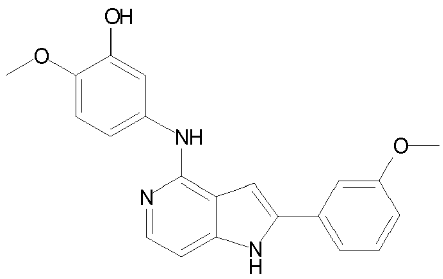

MW.: 361.14, LCMS-ESI (*m/z*) - found 362.14 $[M+H]^+$, 360.14 $[M-H]^-$

**Example 12-313 Compound 313**

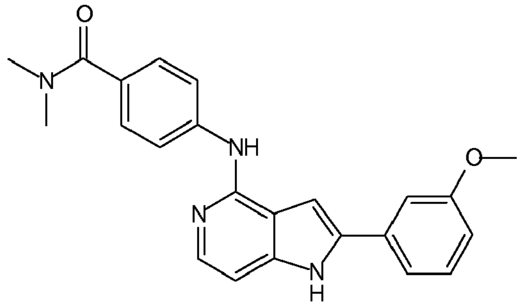

MW.: 386.17, LCMS-ESI (*m/z*) - found 387.17 $[M+H]^+$, 385.17 $[M-H]^-$

**Example 12-314 Compound 314**

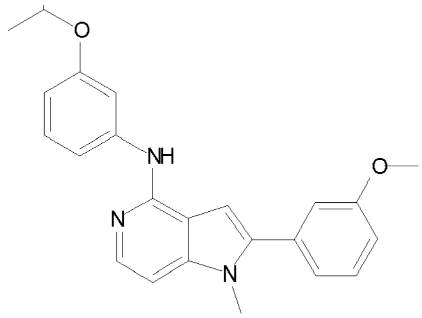

MW.: 387.19, LCMS-ESI (*m/z*) - found 388.19 $[M+H]^+$, 386.19 $[M-H]^-$

**Example 12-315 Compound 315**

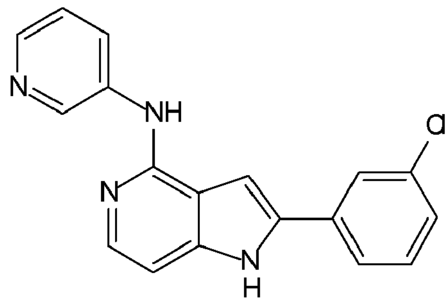

MW.: 320.08, LCMS-ESI (*m/z*) - found 321.08 $[M+H]^+$, 319.08 $[M-H]^-$

**Example 12-316 Compound 316**

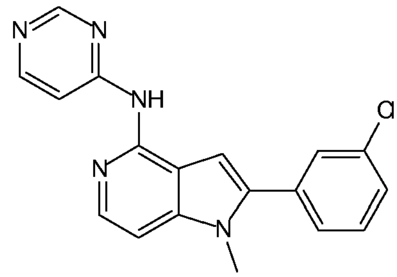

MW.: 335.09, LCMS-ESI (*m*/*z*) - found 336.09 $[M+H]^+$, 334.09 $[M-H]^-$

**Example 12-317 Compound 317**

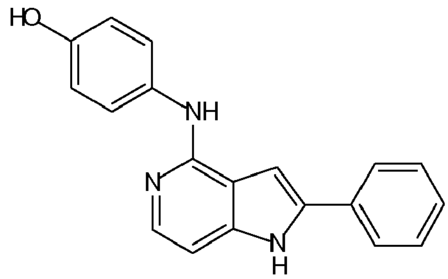

MW.: 301.12, LCMS-ESI (*m*/*z*) - found 302.12 $[M+H]^+$, 300.12 $[M-H]^-$

**Example 12-318 Compound 318**

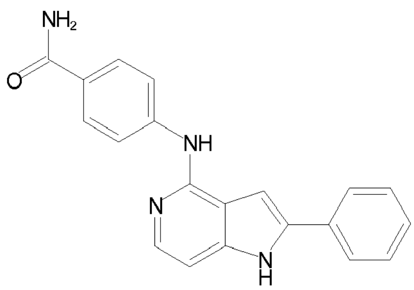

MW.: 328.13, LCMS-ESI (*m*/*z*) - found 329.13 $[M+H]^+$, 327.13 $[M-H]^-$

**Example 12-319 Compound 319**

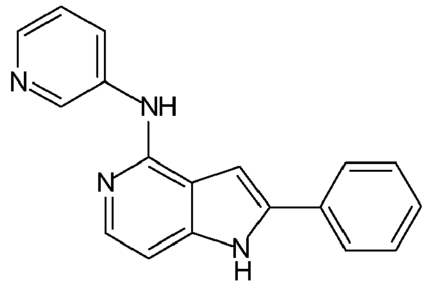

MW.: 286.12, LCMS-ESI (*m*/*z*) - found 287.12 $[M+H]^+$, 285.12 $[M-H]^-$

**Example 12-320 Compound 320**

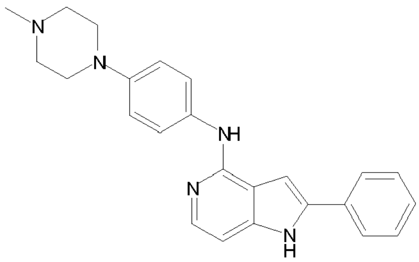

MW.: 383.21, LCMS-ESI (*m*/*z*) - found 384.21 $[M+H]^+$, 382.21 $[M-H]^-$

**Example 12-321 Compound 321**

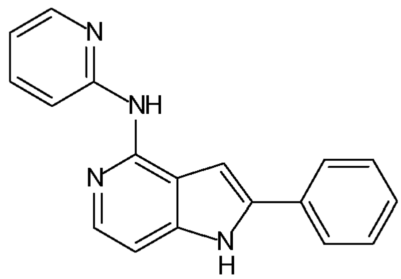

MW.: 286.12, LCMS-ESI (*m*/*z*) - found 287.12 $[M+H]^+$, 285.12 $[M-H]^-$

**Example 12-322 Compound 322**

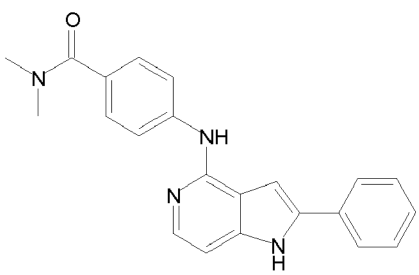

MW.: 356.16, LCMS-ESI (*m/z*) - found 357.16 $[M+H]^+$, 355.16 $[M-H]^-$

**Example 12-323 Compound 323**

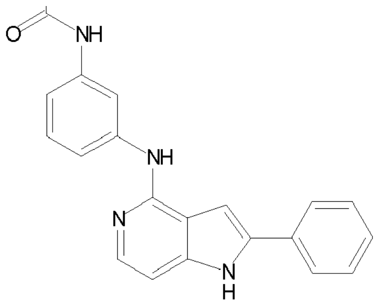

MW.: 342.15, LCMS-ESI (*m*/*z*) - found 343.15 $[M+H]^+$, 341.14 $[M-H]^-$

**Example 12-324 Compound 324**

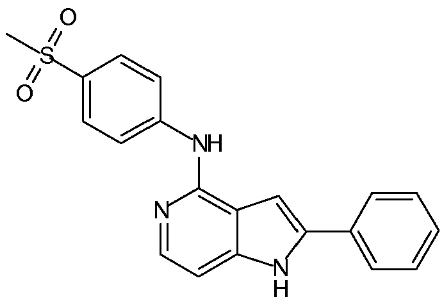

MW.: 363.10, LCMS-ESI (*m*/*z*) - found 364.1 $[M+H]^+$, 362.1 $[M-H]^-$

**Example 12-325 Compound 325**

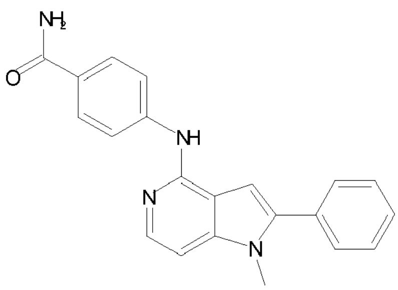

MW.: 342.15, LCMS-ESI (*m*/*z*) - found 343.14 $[M+H]^+$, 341.14 $[M-H]^-$

**Example 12-326 Compound 326**

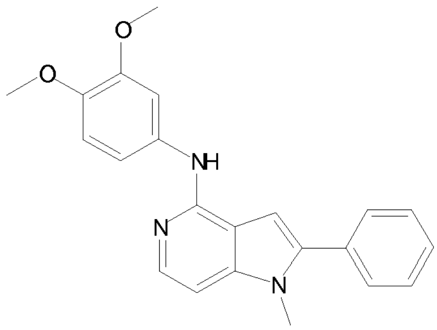

MW.: 359.16, LCMS-ESI (*m*/*z*) - found 360.16 $[M+H]^+$, 358.16 $[M-H]^-$

**Example 12-327 Compound 327**

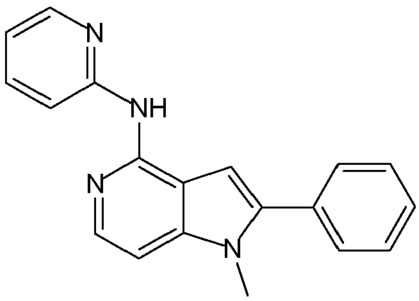

MW.: 300.14, LCMS-ESI (*m*/*z*) - found 301.13 $[M+H]^+$, 299.13 $[M-H]^-$

**Example 12-328 Compound 328**

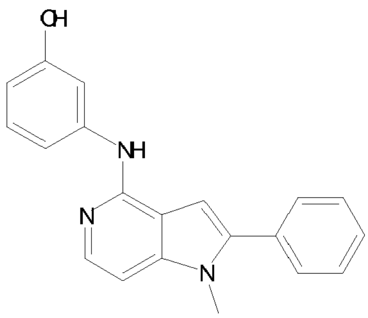

MW.: 315.14, LCMS-ESI (*m*/*z*) - found 316.13 $[M+H]^+$, 314.13 $[M-H]^-$

**Example 12-329 Compound 329**

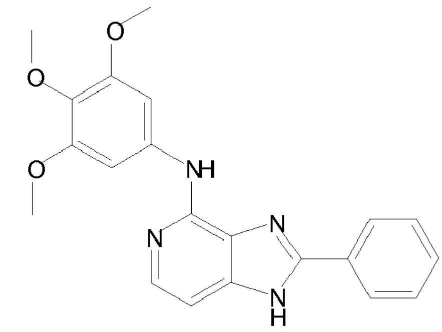

MW.: 376.15, LCMS-ESI ($m/z$) - found 377.15 $[M+H]^+$, 375.15 $[M-H]^-$

**Example 12-330 Compound 330**

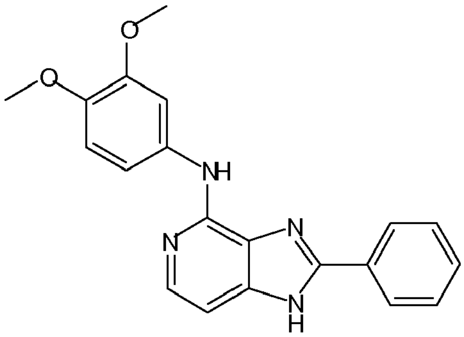

MW.: 346.14, LCMS-ESI ($m/z$) - found 347.14 $[M+H]^+$, 345.14 $[M-H]^-$

**Example 12-331 Compound 331**

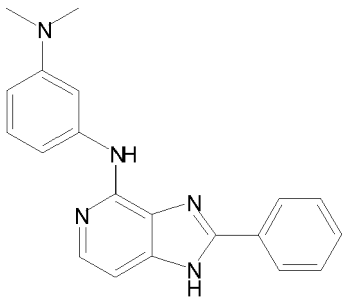

MW.: 329.16, LCMS-ESI ($m/z$) - found 330.16 $[M+H]^+$, 328.16 $[M-H]^-$

**Example 12-332 Compound 332**

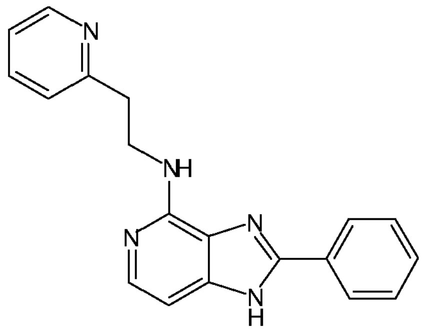

MW.: 315.15, LCMS-ESI (*m*/*z*) - found 316.14 $[M+H]^+$, 314.14 $[M-H]^-$

**Example 12-333 Compound 333**

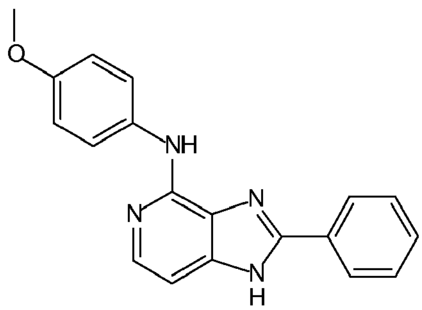

MW.: 316.13, LCMS-ESI (*m*/*z*) - found 317.13 $[M+H]^+$, 315.13 $[M-H]^-$

**Example 12-334 Compound 334**

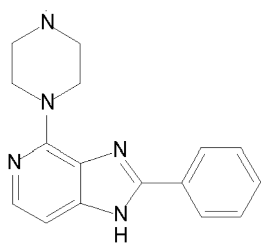

MW.: 293.16, LCMS-ESI (*m*/*z*) - found 294.16 $[M+H]^+$, 292.16 $[M-H]^-$

**Example 12-335 Compound 335**

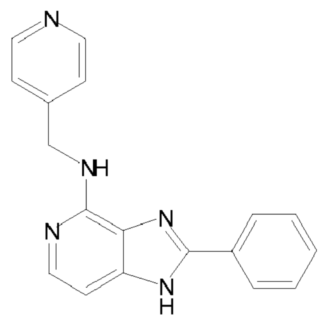

MW.: 301.13, LCMS-ESI (*m*/*z*) - found 302.13 $[M+H]^+$, 300.13 $[M-H]^-$

**Example 12-336 Compound 336**

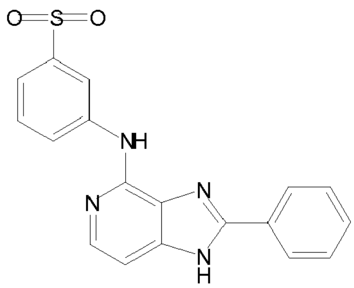

MW.: 364.10, LCMS-ESI (*m*/*z*) - found 365.09 $[M+H]^+$, 363.09 $[M-H]^-$

**Example 12-337 Compound 337**

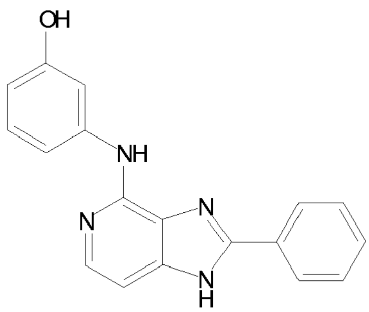

MW.: 302.12, LCMS-ESI (*m*/*z*) - found 303.12 $[M+H]^+$, 301.12 $[M-H]^-$

**Example 12-338 Compound 338**

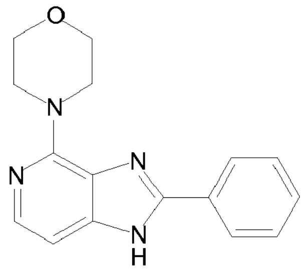

MW.: 280.13, LCMS-ESI (*m*/*z*) - found 281.13 $[M+H]^+$, 279.13 $[M-H]^-$

**Example 12-339 Compound 339**

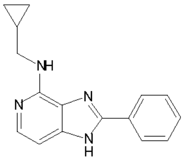

MW.: 264.14, LCMS-ESI (*m*/*z*) - found 265.13 $[M+H]^+$, 263.13 $[M-H]^-$

**Example 12-340 Compound 340**

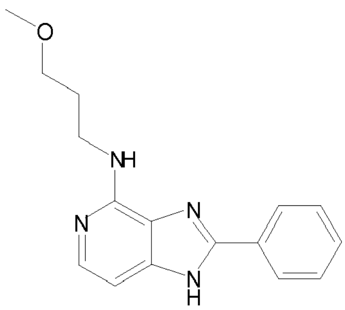

MW.: 282.15, LCMS-ESI (*m*/*z*) - found 283.15 $[M+H]^+$, 281.14 $[M-H]^-$

**Example 12-341 Compound 341**

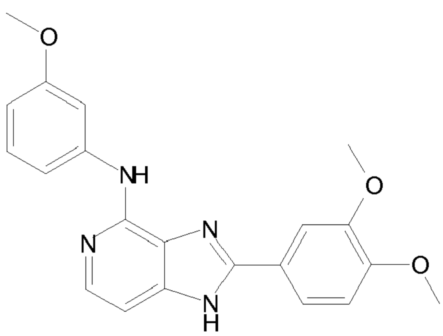

MW.: 376.15, LCMS-ESI (*m*/*z*) - found 377.15 $[M+H]^+$, 375.15 $[M-H]^-$

**Example 12-342 Compound 342**

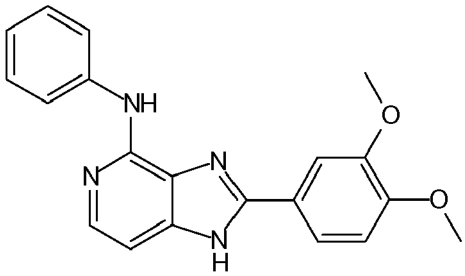

MW.: 346.14, LCMS-ESI (*m*/*z*) - found 347.14 $[M+H]^+$, 345.14 $[M-H]^-$

**Example 12-343 Compound 343**

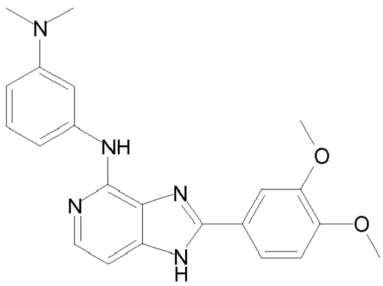

MW.: 389.19, LCMS-ESI (*m*/*z*) - found 390.19 $[M+H]^+$, 388.19 $[M-H]^-$

**Example 12-344 Compound 344**

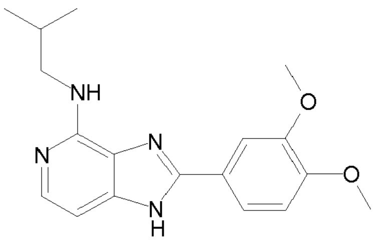

MW.: 326.17, LCMS-ESI (*m*/*z*) - found 327.17 $[M+H]^+$, 325.17 $[M-H]^-$

**Example 12-345 Compound 345**

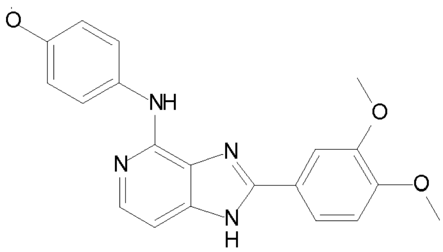

MW.: 376.15, LCMS-ESI (*m*/*z*) - found 377.15 [M+H]$^+$, 375.15 [M-H]$^-$

**Example 12-346 Compound 346**

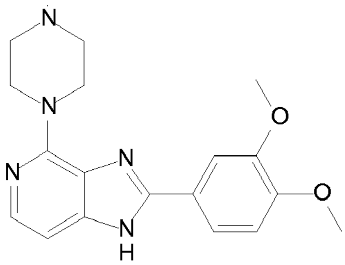

MW.: 353.19, LCMS-ESI (*m*/*z*) - found 354.18 [M+H]$^+$, 352.18 [M-H]$^-$

**Example 12-347 Compound 347**

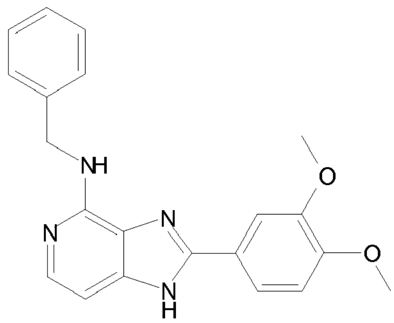

MW.: 360.16, LCMS-ESI (*m*/*z*) - found 361.15 [M+H]$^+$, 359.15 [M-H]$^-$

**Example 12-348 Compound 348**

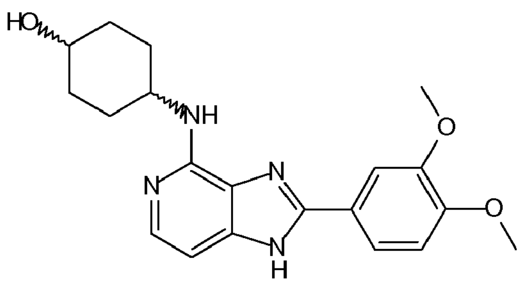

MW.: 368.18, LCMS-ESI (*m*/*z*) - found 369.18 $[M+H]^+$, 367.18 $[M-H]^-$

**Example 12-349 Compound 349**

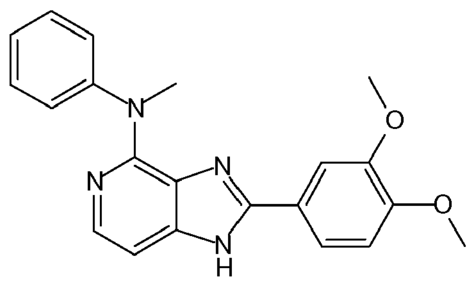

MW.: 360.16, LCMS-ESI (*m*/*z*) - found 361.15 $[M+H]^+$, 359.15 $[M-H]^-$

**Example 12-350 Compound 350**

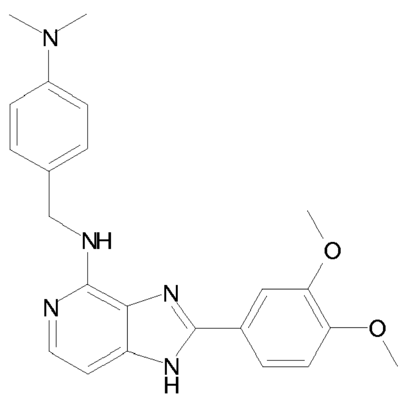

MW.: 403.20, LCMS-ESI (*m*/*z*) - found 404.20 $[M+H]^+$, 402.20 $[M-H]^-$

**Example** 12-351 Compound 351

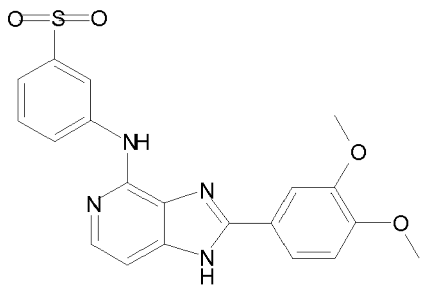

MW.: 424.12, LCMS-ESI (m/z) - found 425.12 $[M+H]^+$, 423.12 $[M-H]^-$

**Example** 12-352 Compound 352

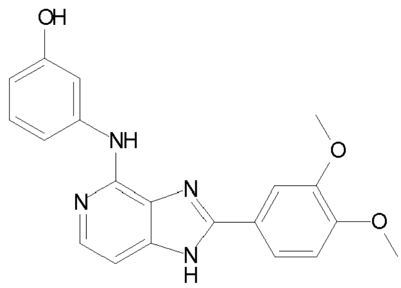

MW.: 362.14, LCMS-ESI (m/z) - found 363.13 $[M+H]^+$, 361.13 $[M-H]^-$

**Example** 12-353 Compound 353

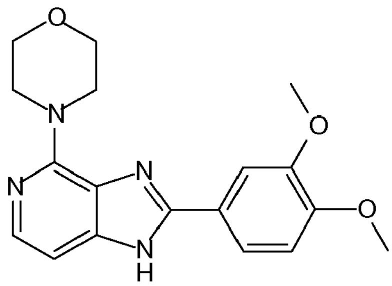

MW.: 340.15, LCMS-ESI (m/z) - found 341.15 $[M+H]^+$, 339.15 $[M-H]^-$

**Example** 12-354 Compound 354

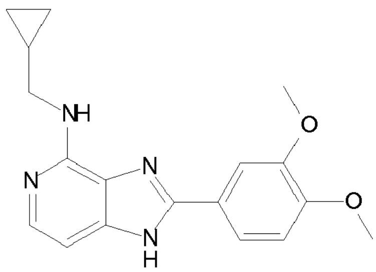

MW.: 324.16, LCMS-ESI (m/z) - found 325.15 $[M+H]^+$, 323.15 $[M-H]^-$

**Example** 12-355 Compound 355

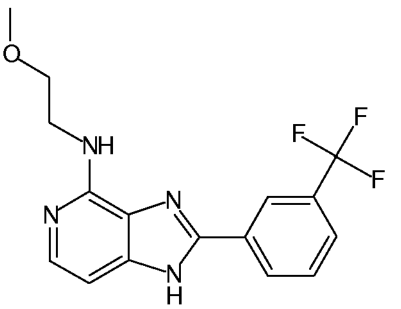

MW.: 336.12, LCMS-ESI (*m*/*z*) - found 337.12 $[M+H]^+$, 335.12 $[M-H]^-$

**Example 12-356 Compound 356**

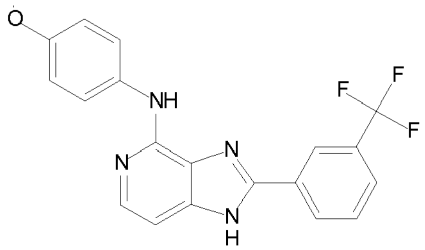

MW.: 384.12, LCMS-ESI (*m*/*z*) - found 385.12 $[M+H]^+$, 383.12 $[M-H]^-$

**Example 12-357 Compound 357**

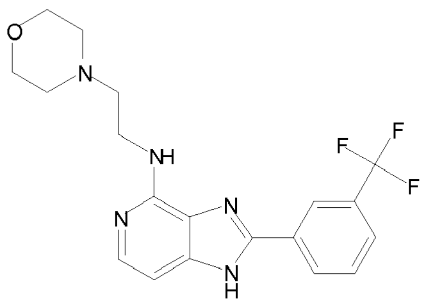

MW.: 391.16, LCMS-ESI (*m*/*z*) - found 392.16 $[M+H]^+$, 390.16 $[M-H]^-$

**Example 12-358 Compound 358**

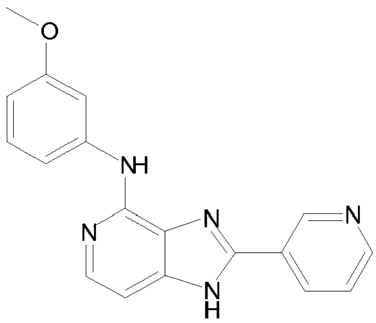

MW.: 317.13 LCMS-ESI (*m*/*z*) - found 318.132 [M+H]$^+$, 316.12 [M-H]$^-$

**Example 12-359 Compound 359**

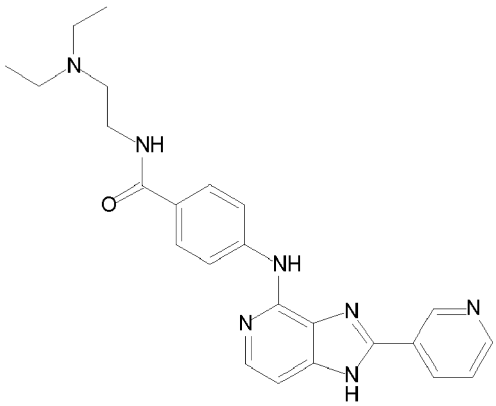

MW.: 429.23, LCMS-ESI (*m*/*z*) - found 430.22 [M+H]$^+$, 428.22 [M-H]$^-$

**Example 12-360 Compound 360**

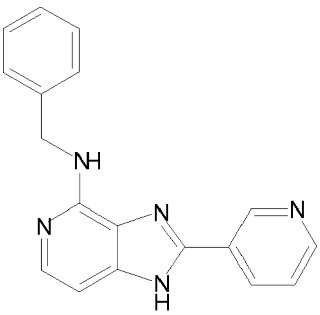

MW.: 301.13, LCMS-ESI (*m*/*z*) - found 302.13 [M+H]$^+$, 300.13 [M-H]$^-$

**Example 12-361 Compound 361**

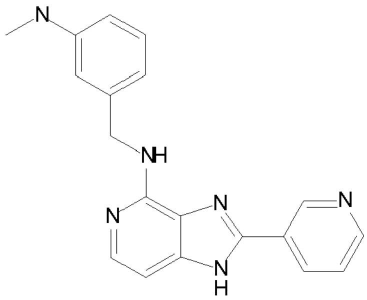

MW.: 344.17, LCMS-ESI (*m*/*z*) - found 345.17 $[M+H]^+$, 343.17 $[M-H]^-$

**Example 12-362 Compound 362**

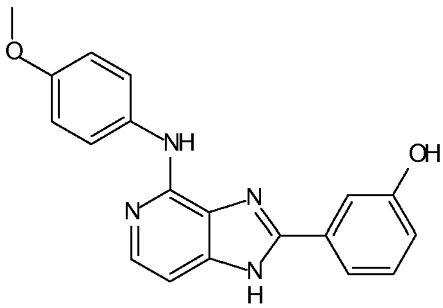

MW.: 332.13, LCMS-ESI (*m*/*z*) - found 333.12 $[M+H]^+$, 331.12 $[M-H]^-$

**Example 12-363 Compound 363**

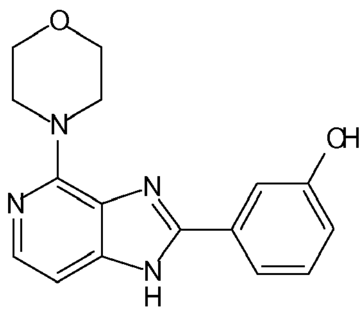

MW.: 296.13, LCMS-ESI (*m*/*z*) - found 297.12 $[M+H]^+$, 295.12 $[M-H]^-$

**Example 12-364 Compound 364**

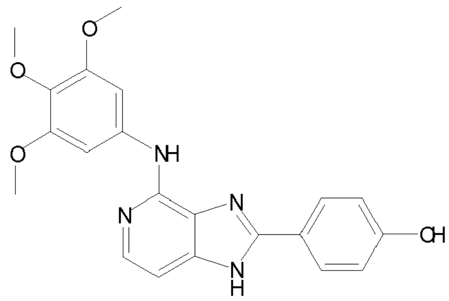

MW.: 392.15, LCMS-ESI (*m*/*z*) - found 393.14 $[M+H]^+$, 391.14 $[M-H]^-$

**Example 12-365 Compound 365**

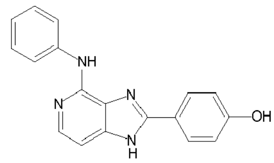

MW.: 302.12, LCMS-ESI (*m*/*z*) - found 303.11 $[M+H]^+$, 301.11 $[M-H]^-$

**Example 12-366 Compound 366**

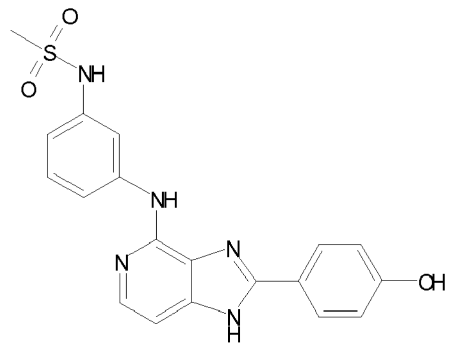

MW.: 395.11, LCMS-ESI (*m*/*z*) - found 396.1 $[M+H]^+$, 394.1 $[M-H]^-$

**Example 12-367 Compound 367**

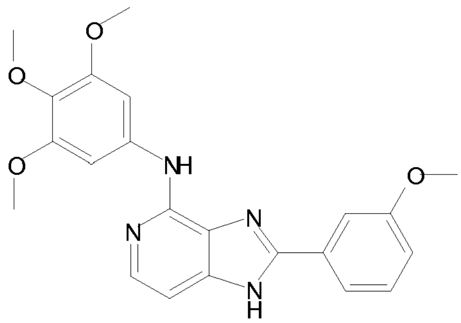

MW.: 406.16, LCMS-ESI (*m*/*z*) - found 407.16 $[M+H]^+$, 405.16 $[M-H]^-$

**Example 12-368 Compound 368**

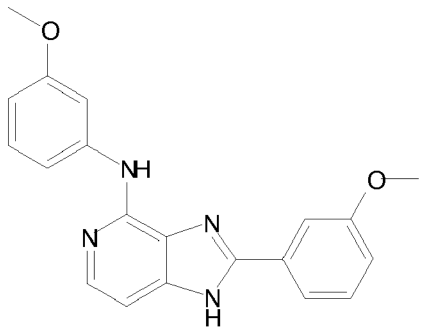

MW.: 346.14, LCMS-ESI (*m*/*z*) - found 347.13 $[M+H]^+$, 345.13 $[M-H]^-$

**Example 12-369 Compound 369**

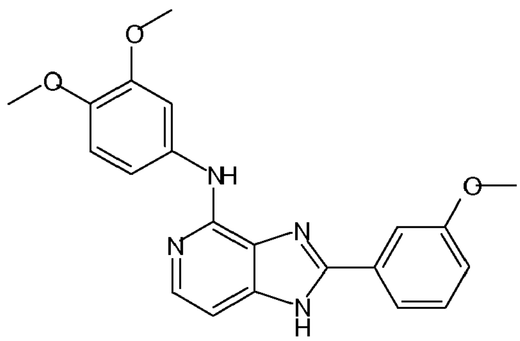

MW.: 376.15, LCMS-ESI (*m*/*z*) - found 377.15 $[M+H]^+$, 375.15 $[M-H]^-$

**Example 12-370 Compound 370**

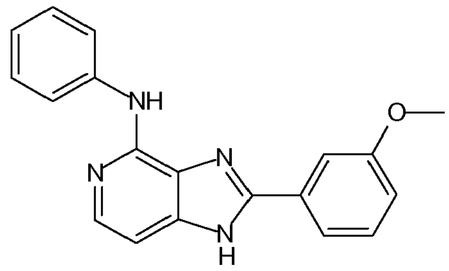

MW.: 316.13 LCMS-ESI (*m/z*) - found 317.13 $[M+H]^+$, 315.13 $[M-H]^-$

**Example 12-371 Compound 371**

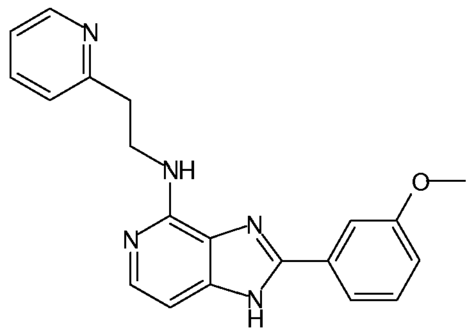

MW.: 345.16, LCMS-ESI (*m/z*) - found 346.15 $[M+H]^+$, 344.15 $[M-H]^-$

**Example 12-372 Compound 372**

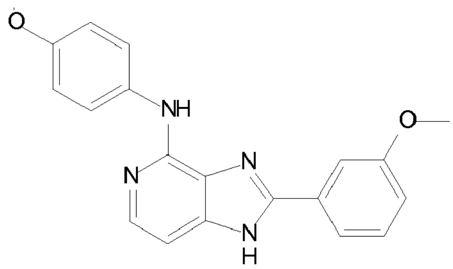

MW.: 346.14, LCMS-ESI (*m/z*) - found 347.14 $[M+H]^+$, 345.14 $[M-H]^-$

**Example 12-373 Compound 373**

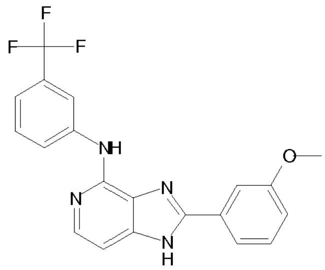

MW.: 384.12, LCMS-ESI (*m*/*z*) - found 385.12 $[M+H]^+$, 383.12 $[M-H]^-$

**Example 12-374 Compound 374**

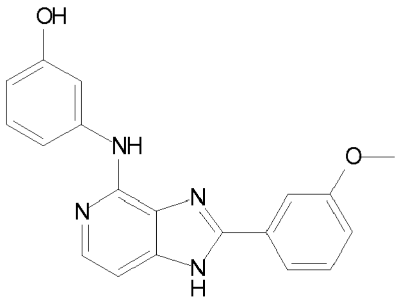

MW.: 332.13, LCMS-ESI (*m*/*z*) - found 333.12 $[M+H]^+$, 331.12 $[M-H]^-$

**Example 12-375 Compound 375**

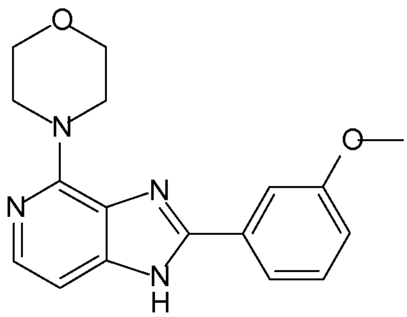

MW.: 310.14, LCMS-ESI (*m*/*z*) - found 311.14$[M+H]^+$, 309.14 $[M-H]^-$

**Example 12-376 Compound 376**

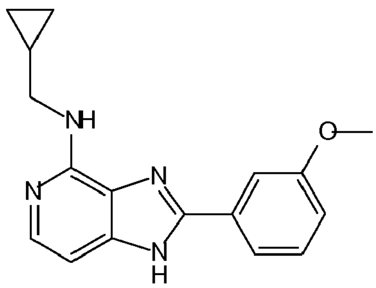

MW.: 294.15, LCMS-ESI (*m*/*z*) - found 295.14 $[M+H]^+$, 293.14 $[M-H]^-$

**Example 12-377 Compound 377**

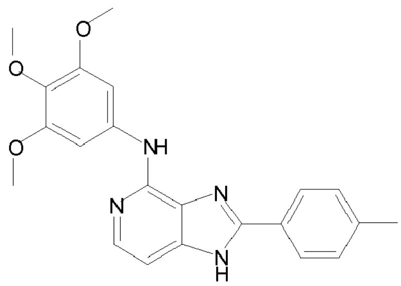

MW.: 390.17, LCMS-ESI (*m*/*z*) - found 391.16 $[M+H]^+$, 389.16 $[M-H]^-$

**Example 12-378 Compound 378**

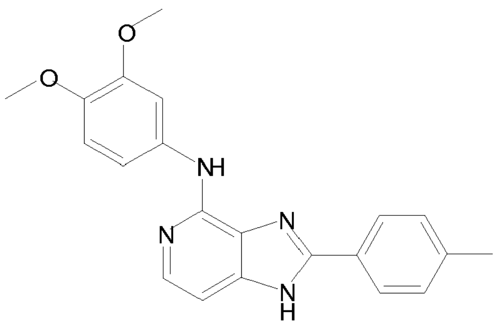

MW.: 360.16, LCMS-ESI (*m*/*z*) - found 361.15 $[M+H]^+$, 359.15 $[M-H]^-$

**Example 12-379 Compound 379**

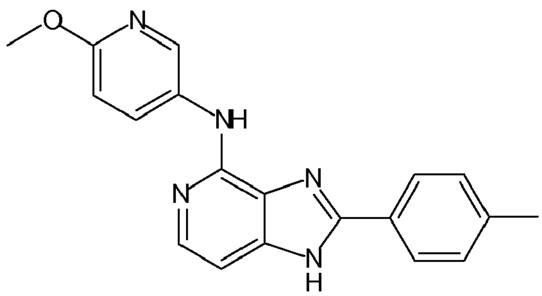

MW.: 331.14, LCMS-ESI (*m*/*z*) - found 332.14 $[M+H]^+$, 330.14 $[M-H]^-$

**Example 12-380 Compound 380**

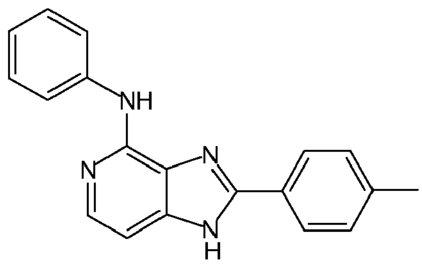

MW.: 300.14, LCMS-ESI (*m*/*z*) - found 301.13 $[M+H]^+$, 299.13 $[M-H]^-$

**Example 12-381 Compound 381**

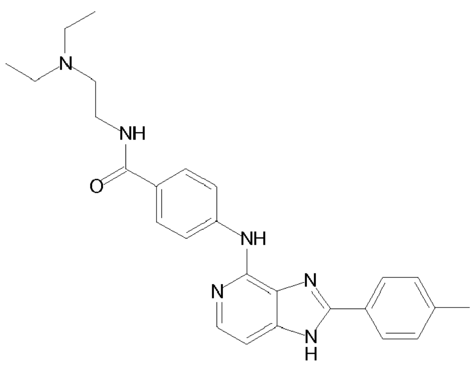

MW.: 442.25, LCMS-ESI (*m*/*z*) - found 443.24 $[M+H]^+$, 441.24 $[M-H]^-$

**Example 12-382 Compound 382**

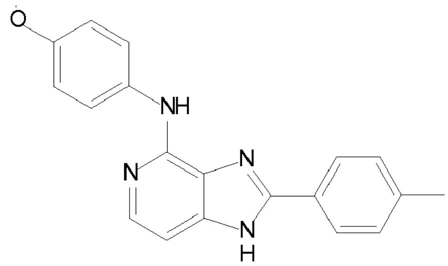

MW.: 330.15, LCMS-ESI (*m*/*z*) - found 331.14 $[M+H]^+$, 329.14 $[M-H]^-$

**Example 12-383 Compound 383**

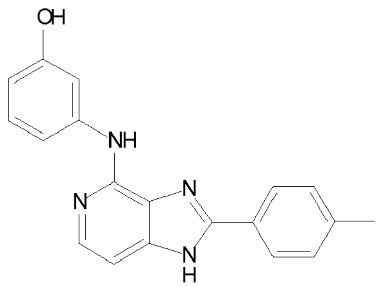

MW.: 316.13, LCMS-ESI (*m*/*z*) - found 317.13 $[M+H]^+$, 315.13 $[M-H]^-$

**Example 12-384 Compound 384**

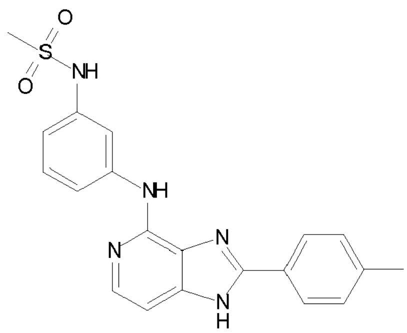

MW.: 393.13, LCMS-ESI (*m*/*z*) - found 394.12 $[M+H]^+$, 392.12 $[M-H]^-$

**Example 12-385 Compound 385**

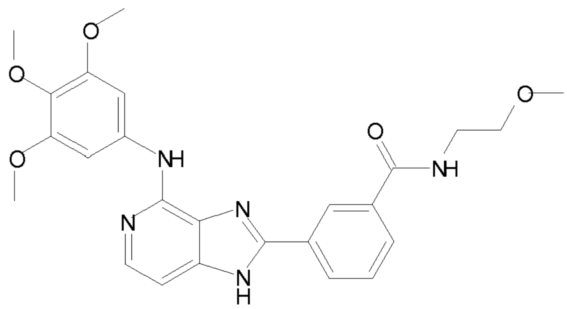

MW.: 477.20, LCMS-ESI (*m/z*) - found 478.20 $[M+H]^+$, 476.20 $[M-H]^-$

**Example 12-386 Compound 386**

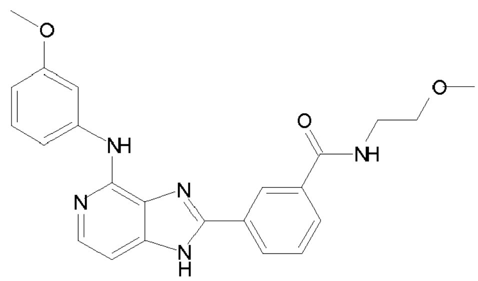

MW.: 417.18, LCMS-ESI (*m/z*) - found 418.18 $[M+H]^+$, 416.18 $[M-H]^-$

**Example 12-387 Compound 387**

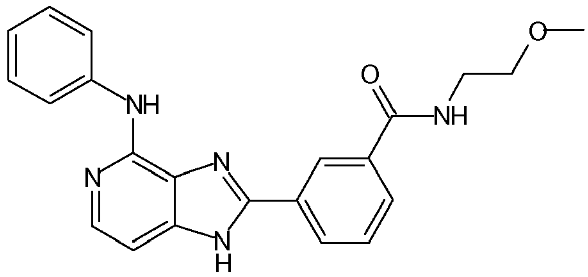

MW.: 387.17, LCMS-ESI (*m/z*) - found 388.17 $[M+H]^+$, 386.17 $[M-H]^-$

**Example 12-388 Compound 388**

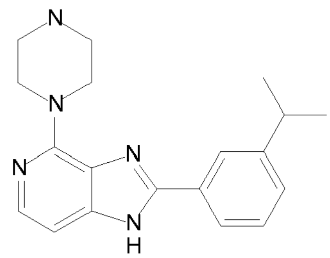

MW.: 355.21, LCMS-ESI (*m*/*z*) - found 336.12 $[M+H]^+$, 334.21 $[M-H]^-$

**Example 12-389 Compound 389**

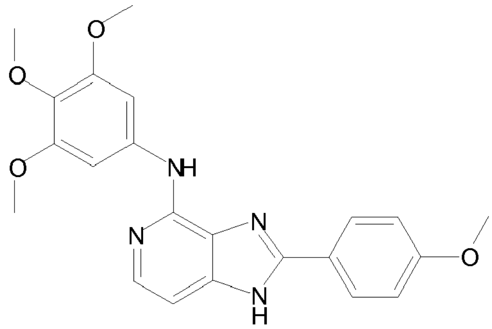

MW.: 406.16, LCMS-ESI (*m*/*z*) - found 407.16 $[M+H]^+$, 405.16 $[M-H]^-$

**Example 12-390 Compound 390**

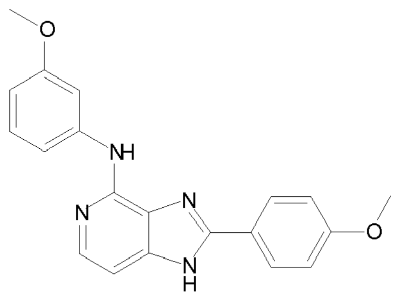

MW.: 346.14, LCMS-ESI (*m*/*z*) - found 347.14 $[M+H]^+$, 345.14 $[M-H]^-$

**Example 12-391 Compound 391**

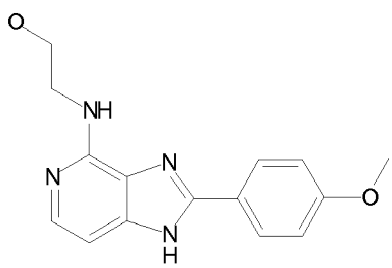

MW.: 298.14, LCMS-ESI (*m*/*z*) - found 299.14 $[M+H]^+$, 297.14 $[M-H]^-$

**Example 12-392 Compound 392**

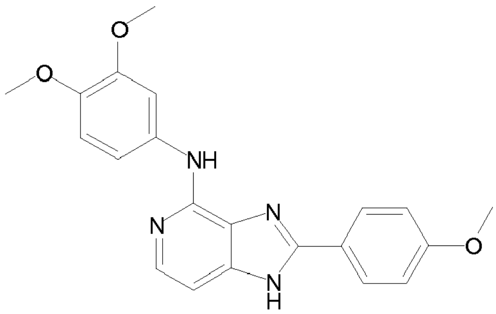

MW.: 376.15, LCMS-ESI (*m*/*z*) - found 377.15 $[M+H]^+$, 375.15 $[M-H]^-$

**Example 12-393 Compound 393**

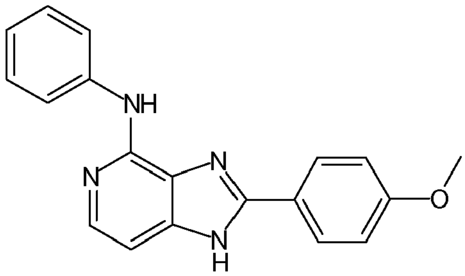

MW.: 316.13, LCMS-ESI (*m*/*z*) - found 317.13 $[M+H]^+$, 315.13 $[M-H]^-$

**Example 12-394 Compound 394**

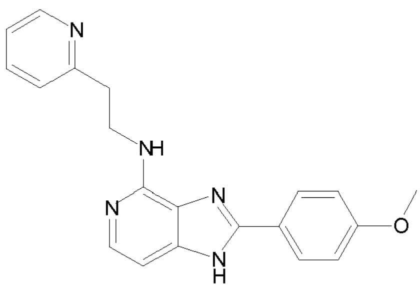

MW.: 354.16, LCMS-ESI (*m*/*z*) - found 346.15 $[M+H]^+$, 344.15 $[M-H]^-$

**Example 12-395 Compound 395**

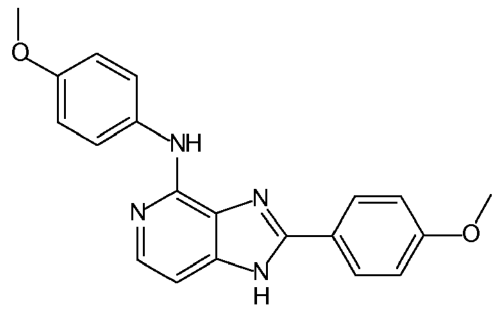

MW.: 346.14, LCMS-ESI (*m*/*z*) - found 347.14 $[M+H]^+$, 345.14 $[M-H]^-$

**Example 12-396 Compound 396**

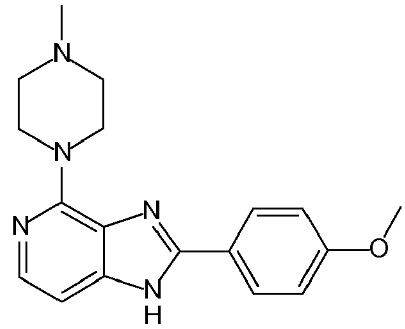

MW.: 323.17, LCMS-ESI (*m*/*z*) - found 324.17 $[M+H]^+$, 322.17 $[M-H]^-$

**Example 12-397 Compound 397**

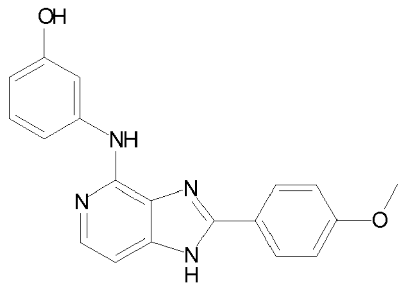

MW.: 332.13, LCMS-ESI (*m*/*z*) - found 333.12 $[M+H]^+$, 331.17 $[M-H]^-$

**Example 12-398 Compound 398**

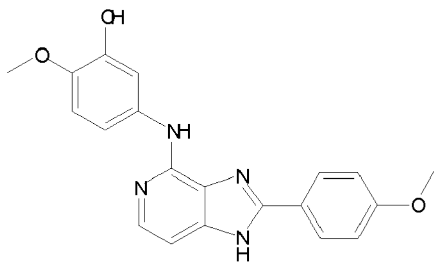

MW.: 362.14, LCMS-ESI (*m*/*z*) - found 363.13 $[M+H]^+$, 361.13 $[M-H]^-$

**Example 12-399 Compound 399**

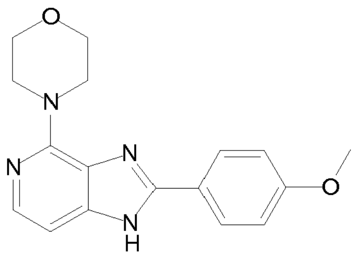

MW.: 310.14, LCMS-ESI (*m*/*z*) - found 311.14 $[M+H]^+$, 309.14 $[M-H]^-$

**Example 12-400 Compound 400**

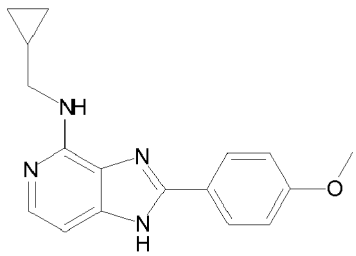

MW.: 294.15, LCMS-ESI (*m*/*z*) - found 295.14 $[M+H]^+$, 293.14 $[M-H]^-$

**Example 12-401 Compound 401**

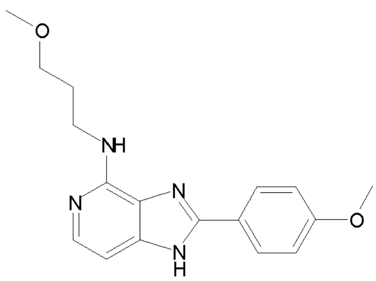

MW.: 312.16, LCMS-ESI (*m*/*z*) - found 313.15 $[M+H]^+$, 311.15 $[M-H]^-$

**Example 12-402 Compound 402**

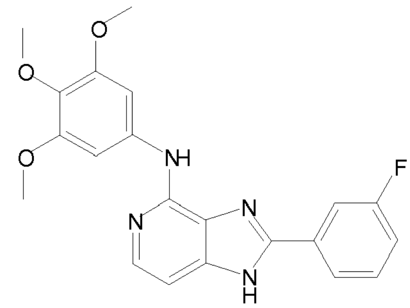

MW.: 394.14, LCMS-ESI (*m*/*z*) - found 395.14 $[M+H]^+$, 393.14 $[M-H]^-$

**Example 12-403 Compound 403**

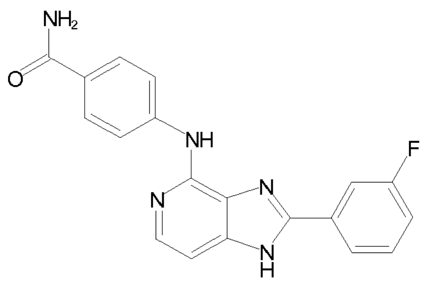

MW.: 347.12, LCMS-ESI (*m*/*z*) - found 348.11 $[M+H]^+$, 346.11 $[M-H]^-$

**Example 12-404 Compound 404**

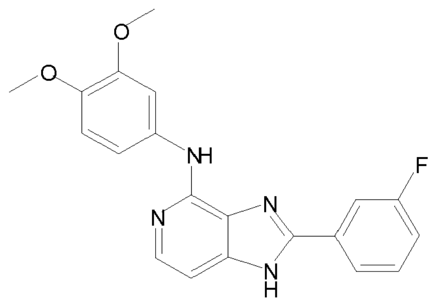

MW.: 364.13, LCMS-ESI (*m*/*z*) - found 365.13 $[M+H]^+$, 364.13 $[M-H]^-$

**Example 12-405 Compound 405**

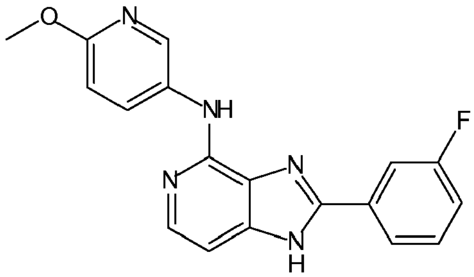

MW.: 335.12, LCMS-ESI (*m*/*z*) - found 336.11 $[M+H]^+$, 334.11 $[M-H]^-$

**Example 12-406 Compound 406**

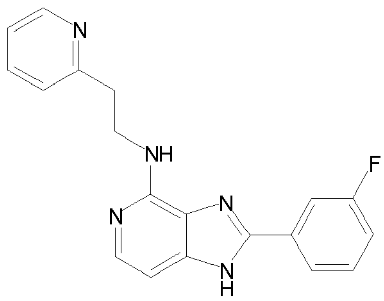

MW.: 333.14, LCMS-ESI (*m*/*z*) - found 334.13 $[M+H]^+$, 332.13 $[M-H]^-$

**Example 12-407 Compound 407**

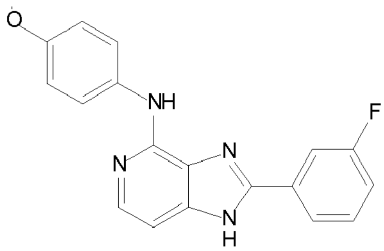

MW.: 334.12, LCMS-ESI (*m*/*z*) - found 335.12 $[M+H]^+$, 333.12 $[M-H]^-$

**Example 12-408 Compound 408**

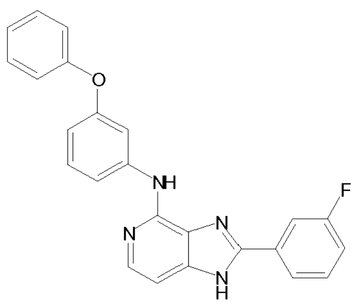

MW.: 396.14, LCMS-ESI (*m*/*z*) - found 397.13 $[M+H]^+$, 395.13 $[M-H]^-$

**Example 12-409 Compound 409**

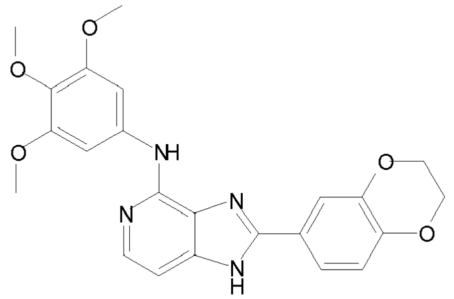

MW.: 434.16, LCMS-ESI (*m*/*z*) - found 435.15 $[M+H]^+$, 433.15 $[M-H]^-$

**Example 12-410 Compound 410**

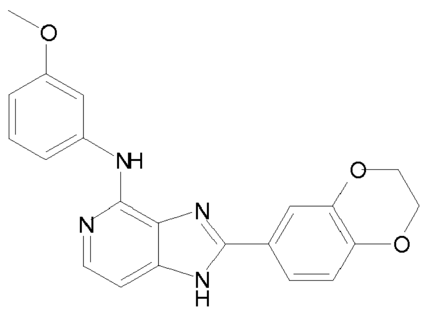

MW.: 374.14, LCMS-ESI (*m*/*z*) - found 375.13 $[M+H]^+$, 373.13 $[M-H]^-$

**Example 12-411 Compound 411**

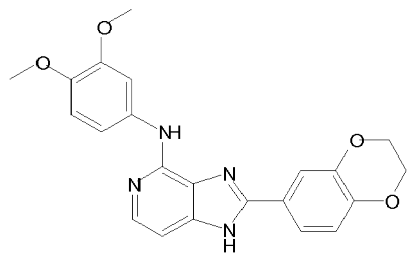

MW.: 404.15, LCMS-ESI (*m*/*z*) - found 405.14 $[M+H]^+$, 403.14 $[M-H]^-$

**Example 12-412 Compound 412**

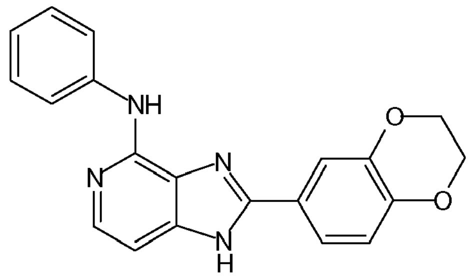

MW.: 344.13, LCMS-ESI (*m*/*z*) - found 345.12 $[M+H]^+$, 343.12 $[M-H]^-$

**Example 12-413 Compound 413**

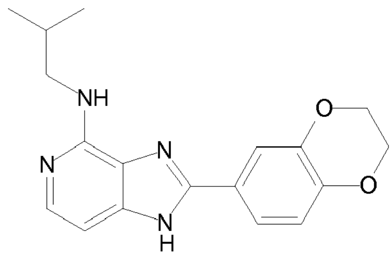

MW.: 324.16, LCMS-ESI (*m*/*z*) - found 325.15 $[M+H]^+$, 323.15 $[M-H]^-$

**Example 12-414 Compound 414**

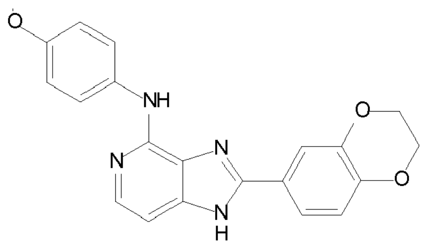

MW.: 374.14, LCMS-ESI (*m*/*z*) - found 375.13 $[M+H]^+$, 373.13 $[M-H]^-$

**Example 12-415 Compound 415**

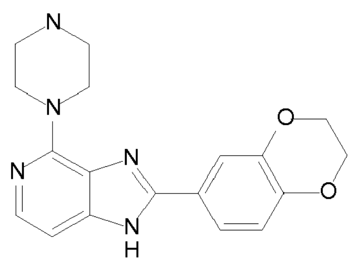

MW.: 351.17, LCMS-ESI (*m*/*z*) - found 352.17 $[M+H]^+$, 350.17 $[M-H]^-$

**Example 12-416 Compound 416**

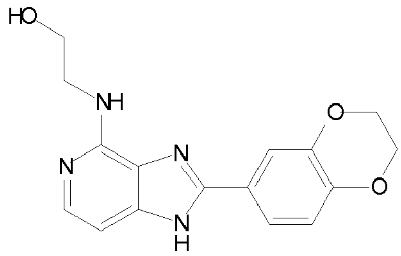

MW.: 312.12, LCMS-ESI (*m*/*z*) - found 313.12 $[M+H]^+$, 311.12 $[M-H]^-$

**Example 12-417 Compound 417**

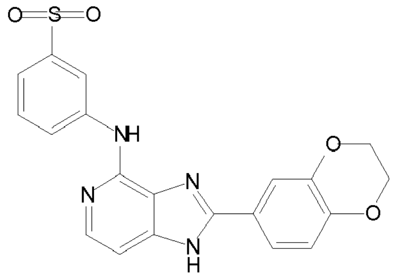

MW.: 422.10, LCMS-ESI (*m*/*z*) - found 423.10 $[M+H]^+$, 421.10 $[M-H]^-$

**Example 12-418 Compound 418**

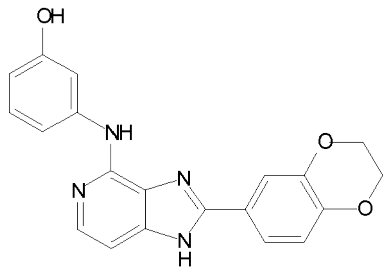

MW.: 360.12, LCMS-ESI (m/z) - found 361.12 $[M+H]^+$, 359.12 $[M-H]^-$

**Example 12-419 Compound 419**

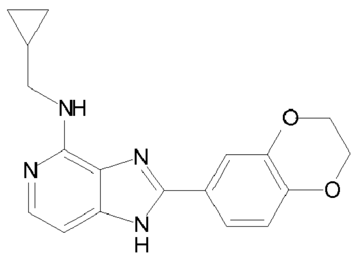

MW.: 322.14, LCMS-ESI *(m/z)* - found 323.14 $[M+H]^+$, 321.14 $[M-H]^-$

**Example 12-420 Compound 420**

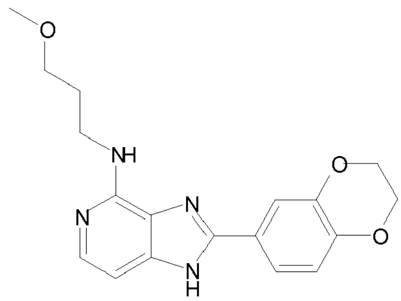

MW.: 340.15, LCMS-ESI (m/z) - found 341.15 $[M+H]^+$, 339.15 $[M-H]^-$

**Example 12-421 Compound 421**

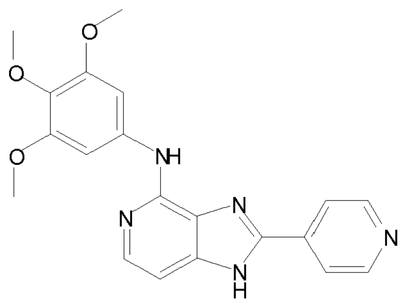

MW.: 377.15, LCMS-ESI (*m*/*z*) - found 378.14 $[M+H]^+$, 376.14$[M-H]^-$

**Example 12-422 Compound 422**

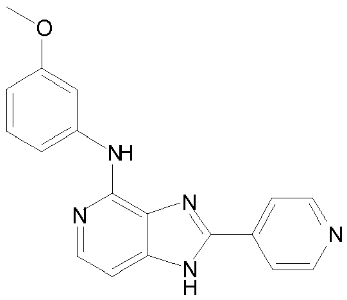

MW.: 317.13, LCMS-ESI (m/z) - found 318.12 $[M+H]^+$, 316.12 $[M-H]^-$

**Example 12-423 Compound 423**

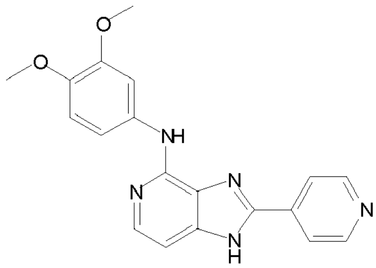

MW.: 347.14, LCMS-ESI (*m*/*z*) - found 348.13 $[M+H]^+$, 346.13 $[M-H]^-$

**Example 12-424 Compound 424**

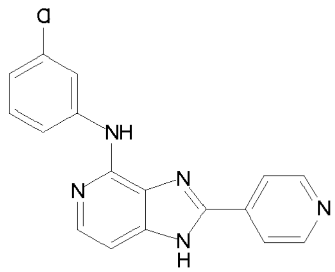

MW.: 321.08, LCMS-ESI (*m*/*z*) - found 322.07 $[M+H]^+$, 320.07 $[M-H]^-$

**Example 12-425 Compound 425**

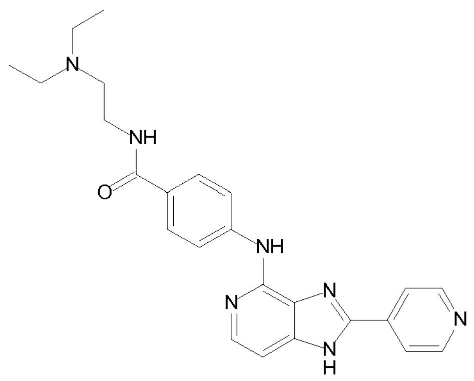

MW.: 429.23, LCMS-ESI (*m*/*z*) - found 430.22 $[M+H]^+$, 428.22 $[M-H]^-$

**Example 12-426 Compound 426**

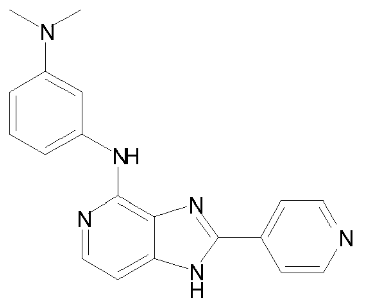

MW.: 330.16, LCMS-ESI (*m*/*z*) - found 331.15 $[M+H]^+$, 329.15 $[M-H]^-$

**Example 12-427 Compound 427**

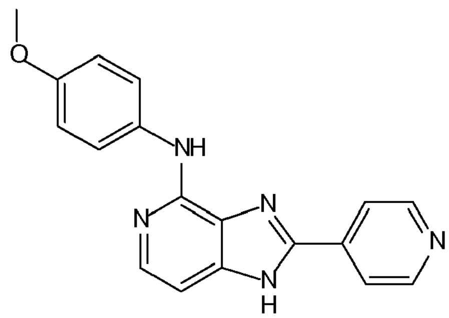

MW.: 317.13, LCMS-ESI (*m*/*z*) - found 318.12 $[M+H]^+$, 316.12 $[M-H]^-$

**Example 12-428 Compound 428**

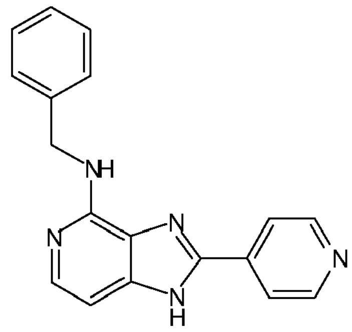

MW.: 301.13, LCMS-ESI (*m*/*z*) - found 302.13 $[M+H]^+$, 300.13 $[M-H]^-$

**Example 12-429 Compound 429**

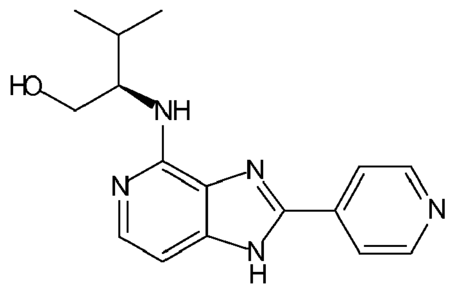

MW.: 297.16, LCMS-ESI (*m*/*z*) - found 298.15 $[M+H]^+$, 296.15 $[M-H]^-$

**Example 12-430 Compound 430**

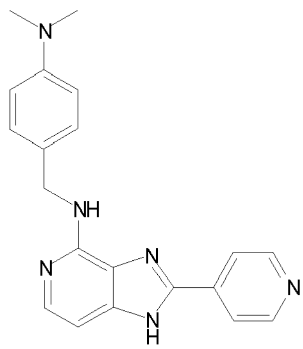

MW.: 344.17, LCMS-ESI (*m*/*z*) - found 345.17 $[M+H]^+$, 343.17 $[M-H]^-$

**Example 12-431 Compound 431**

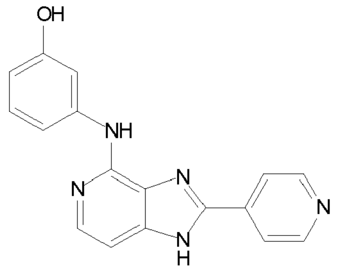

MW.: 303.11, LCMS-ESI (*m*/*z*) - found 304.11 $[M+H]^+$, 302.11 $[M-H]^-$

**Example 12-432 Compound 432**

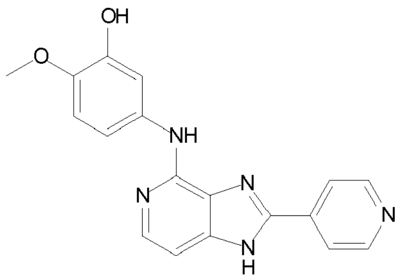

MW.: 333.12, LCMS-ESI (*m*/*z*) - found 334.12 $[M+H]^+$, 332.12 $[M-H]^-$

**Example 12-433 Compound 433**

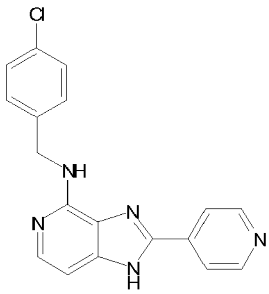

MW.: 335.09, LCMS-ESI (m/z) - found 336.09 $[M+H]^+$, 334.09 $[M-H]^-$

**Example 12-434 Compound 434**

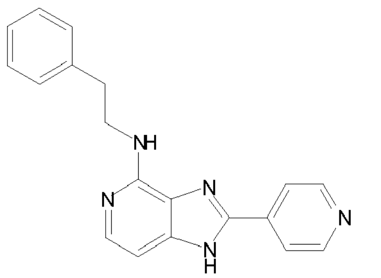

MW.: 315.15, LCMS-ESI (*m*/*z*) - found 316.14 $[M+H]^+$, 314.14 $[M-H]^-$

**Example 12-435 Compound 435**

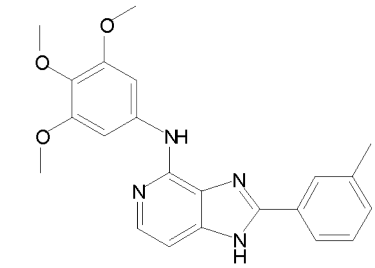

MW.: 390.17, LCMS-ESI (*m*/*z*) - found 391.16 $[M+H]^+$, 389.16 $[M-H]^-$

**Example 12-436 Compound 436**

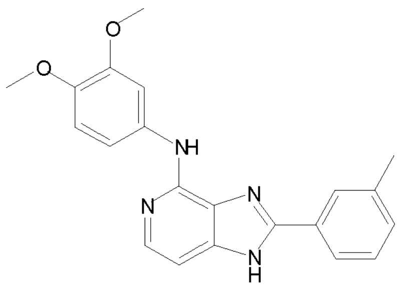

MW.: 360.16, LCMS-ESI (*m*/*z*) - found 361.15 $[M+H]^+$, 359.15 $[M-H]^-$

**Example 12-437 Compound 437**

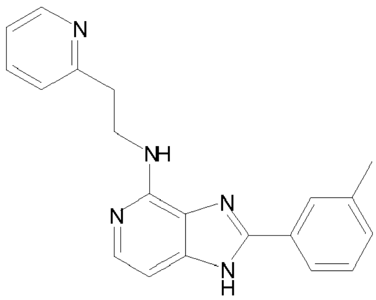

MW.: 329.16, LCMS-ESI (*m*/*z*) - found 330.16 $[M+H]^+$, 328.16 $[M-H]^-$

**Example 12-438 Compound 438**

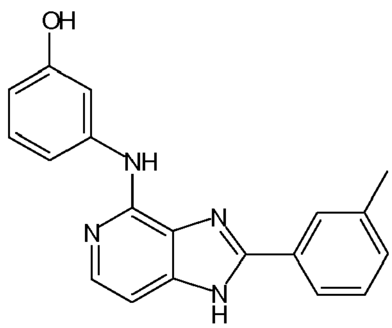

MW.: 316.13, LCMS-ESI (m/z) - found 317.13 $[M+H]^+$, 315.13 $[M-H]^-$

**Example 12-439 Compound 439**

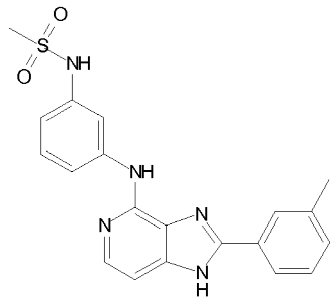

MW.: 393.13, LCMS-ESI (*m/z*) - found 394.12 $[M+H]^+$, 392.12 $[M-H]^-$

**Example 12-440 Compound 440**

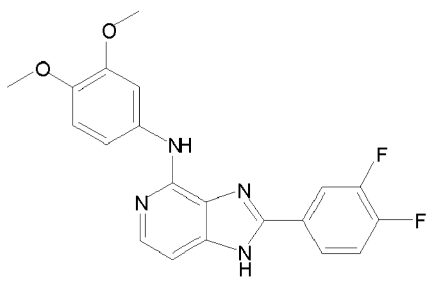

MW.: 382.12, LCMS-ESI (*m/z*) - found 383.12 $[M+H]^+$, 381.12 $[M-H]^-$

**Example 12-441 Compound 441**

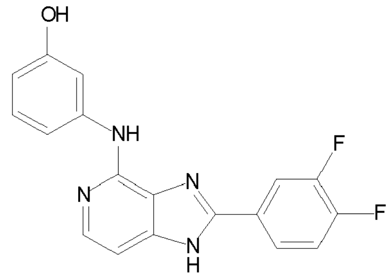

MW.: 338.10, LCMS-ESI (*m*/*z*) - found 339.09 $[M+H]^+$, 337.09 $[M-H]^-$

**Example 12-442 Compound 442**

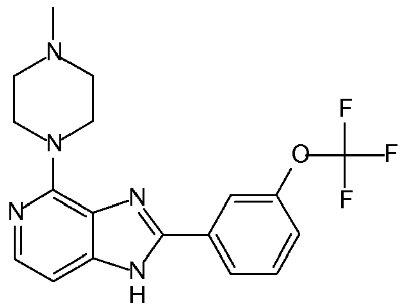

MW.: 377.15, LCMS-ESI (*m*/*z*) - found 378.14 $[M+H]^+$, 376.14 $[M-H]^-$

**Example 12-443 Compound 443**

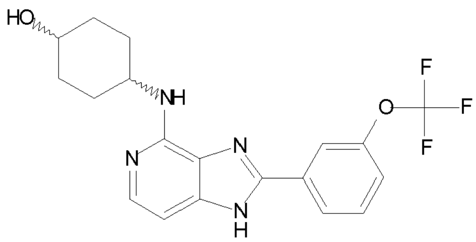

MW.: 392.15, LCMS-ESI (*m*/*z*) - found 393.15 $[M+H]^+$, 391.15 $[M-H]^-$

**Example 12-444 Compound 444**

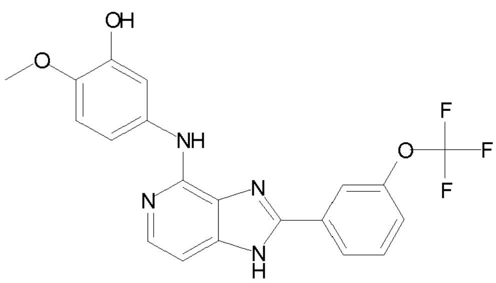

MW.: 416.11, LCMS-ESI (*m*/*z*) - found 417.11 $[M+H]^+$, 415.11 $[M-H]^-$

**Example 12-445 Compound 445**

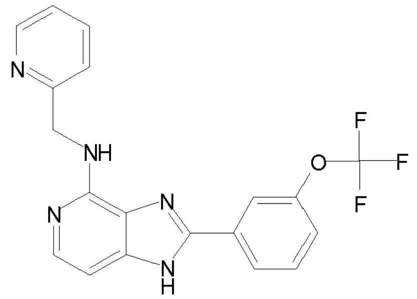

MW.: 385.12, LCMS-ESI (*m*/*z*) - found 386.11 $[M+H]^+$, 384.11 $[M-H]^-$

**Example 12-446 Compound 446**

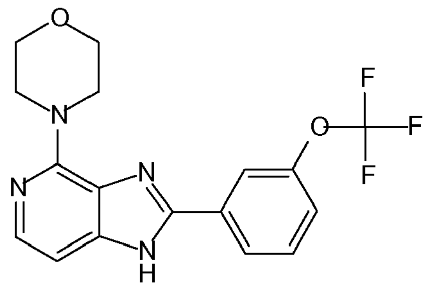

MW.: 364.11, LCMS-ESI (m/z) - found 365.11 $[M+H]^+$, 363.11 $[M-H]^-$

**Example 12-447 Compound 447**

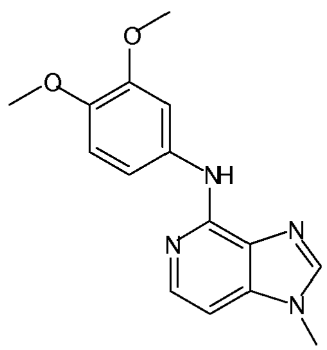

MW.: 284.13, LCMS-ESI (*m*/*z*) - found 285.12 $[M+H]^+$, 283.12 $[M-H]^-$

**Example 12-448 Compound 448**

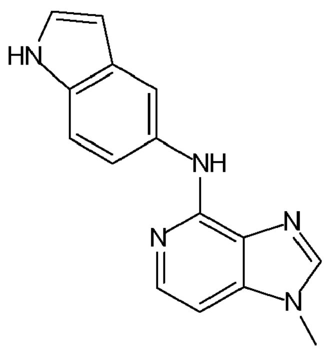

MW.: 263.12, LCMS-ESI (*m*/*z*) - found 264.11 $[M+H]^+$, 262.11 $[M-H]^-$

**Example 12-449 Compound 449**

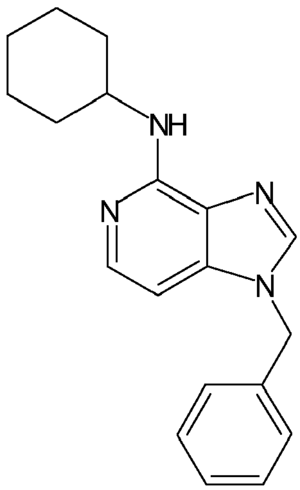

MW.: 306.18, LCMS-ESI (*m*/*z*) - found 307.18 $[M+H]^+$, 305.18 $[M-H]^-$

**Example 12-450 Compound 450**

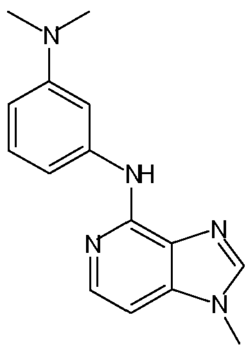

MW.: 267.15, LCMS-ESI (*m*/*z*) - found 268.14 $[M+H]^+$, 266.14 $[M-H]^-$

**Example 12-451 Compound 451**

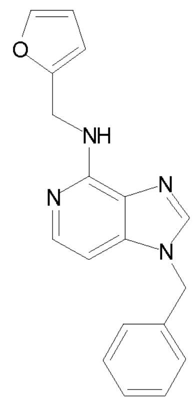

MW.: 304.13, LCMS-ESI (*m*/*z*) - found 305.13 $[M+H]^+$, 303.13 $[M-H]^-$

**Example 12-452 Compound 452**

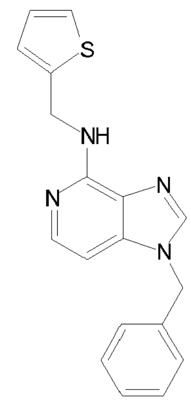

MW.: 320.11, LCMS-ESI (*m*/*z*) - found 321.11 $[M+H]^+$, 319.11 $[M-H]^-$

**Example 12-453 Compound 453**

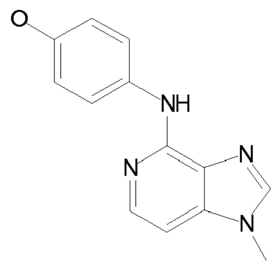

MW.: 254.12, LCMS-ESI (*m*/*z*) - found 255.11 $[M+H]^+$, 253.11 $[M-H]^-$

**Example 12-454 Compound 454**

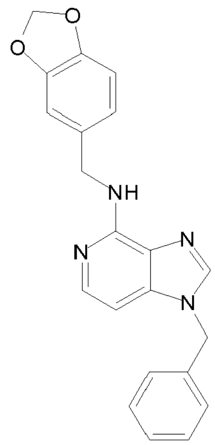

MW.: 358.14, LCMS-ESI (*m*/*z*) - found 359.14 $[M+H]^+$, 357.14 $[M-H]^-$

**Example 12-455 Compound 455**

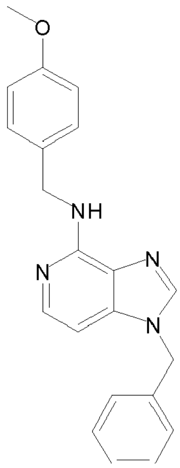

MW.: 344.16, LCMS-ESI (*m*/*z*) - found 345.16 $[M+H]^+$, 343.16 $[M-H]^-$

**Example 12-456 Compound 456**

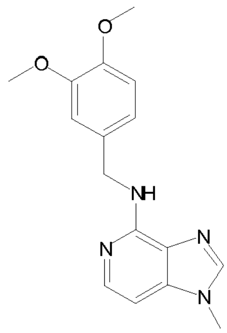

MW.: 298.14, LCMS-ESI (*m*/*z*) - found 299.14 $[M+H]^+$, 297.14 $[M-H]^-$

**Example 12-457 Compound 457**

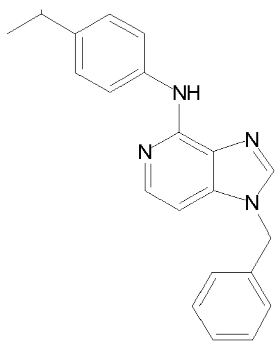

MW.: 342.18, LCMS-ESI (*m*/*z*) - found 343.18 $[M+H]^+$, 341.18 $[M-H]^-$

**Example 12-458 Compound 458**

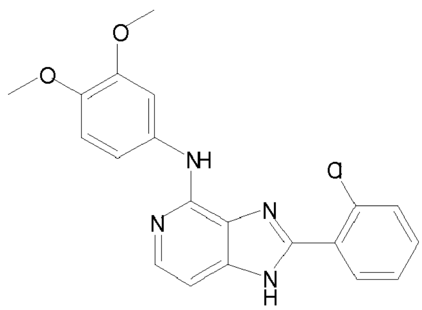

MW.: 380.10, LCMS-ESI (*m*/*z*) - found 381.10 $[M+H]^+$, 379.1 $[M-H]^-$

**Example 12-459 Compound 459**

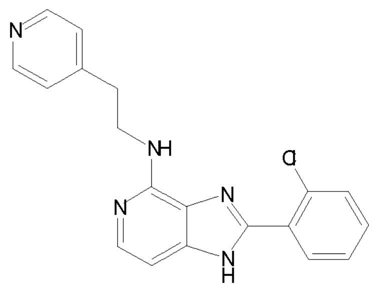

MW.: 349.11, LCMS-ESI (*m*/*z*) - found 350.10 $[M+H]^+$, 348.10 $[M-H]^-$

**Example 12-460 Compound 460**

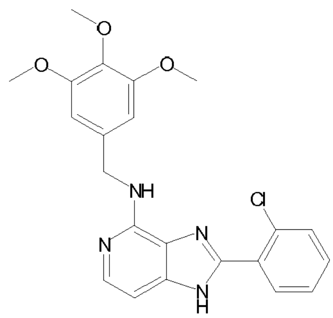

MW.: 330.16, LCMS-ESI (*m*/*z*) - found 331.15 $[M+H]^+$, 329.15 $[M-H]^-$

**Example 12-461 Compound 461**

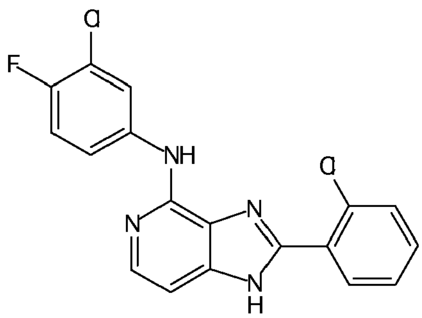

MW.: 372.03, LCMS-ESI (*m*/*z*) - found 373.03 $[M+H]^+$, 371.03 $[M-H]^-$

**Example 12-462 Compound 462**

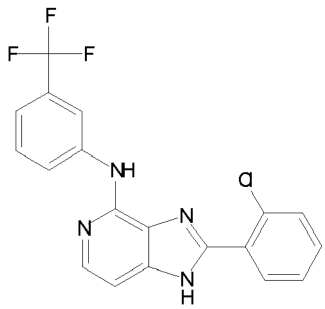

MW.: 388.07, LCMS-ESI (*m/z*) - found 389.07 $[M+H]^+$, 387.07 $[M-H]^-$

**Example 12-463 Compound 463**

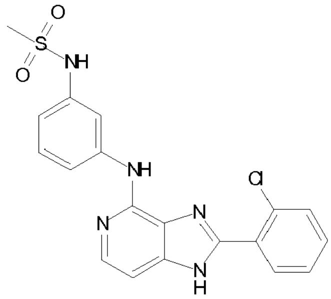

MW.: 413.07, LCMS-ESI (*m/z*) - found 414.07 $[M+H]^+$, 412.07 $[M-H]^-$

**Example 12-464 Compound 464**

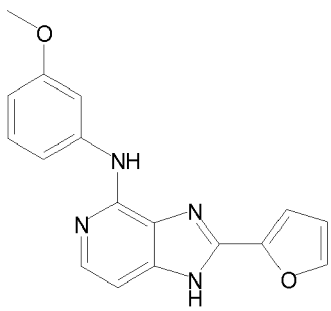

MW.: 306.11, LCMS-ESI (m/z) - found 307.11 $[M+H]^+$, 305.11 $[M-H]^-$

**Example 12-465 Compound 465**

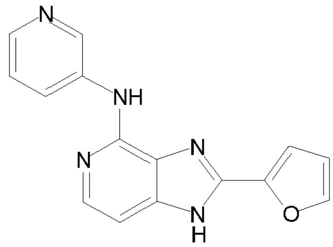

MW.: 277.10, LCMS-ESI (*m/z*) - found 278.09 $[M+H]^+$, 276.09 $[M-H]^-$

**Example 12-466 Compound** 466

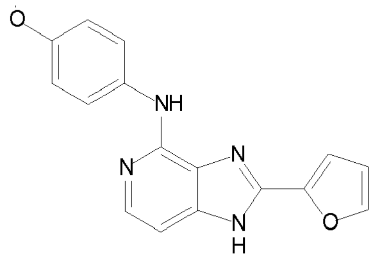

MW.: 306.11, LCMS-ESI (*m/z*) - found 307.11 $[M+H]^+$, 305.11 $[M-H]^-$

**Example 12-467 Compound 467**

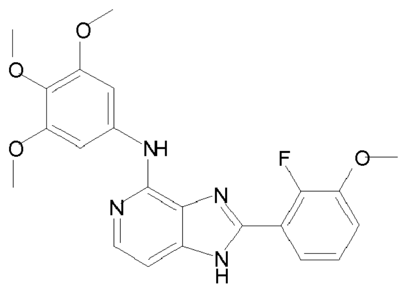

MW.: 424.15, LCMS-ESI (m/z) - found 425.15 $[M+H]^+$, 423.15 $[M-H]^-$

**Example 12-468 Compound 468**

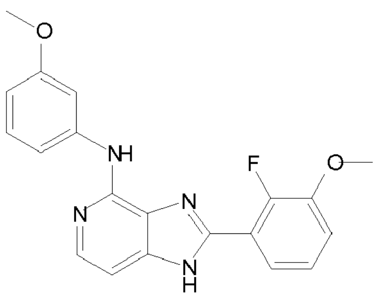

MW.: 364.13, LCMS-ESI (*m*/*z*) - found 365.13 $[M+H]^+$, 363.13 $[M-H]^-$

**Example 12-469 Compound 469**

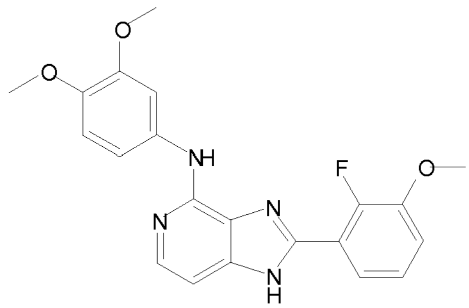

MW.: 394.14, LCMS-ESI (*m*/*z*) - found 395.14 $[M+H]^+$, 393.14 $[M-H]^-$

**Example 12-470 Compound 470**

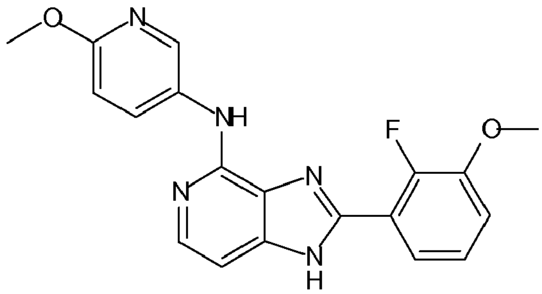

MW.: 365.13, LCMS-ESI (*m*/*z*) - found 366.12 $[M+H]^+$, 364.12 $[M-H]^-$

**Example 12-471 Compound 471**

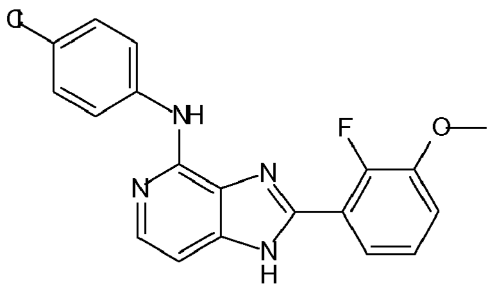

MW.: 368.08, LCMS-ESI (m/z) - found 369.08 $[M+H]^+$, 367.08 $[M-H]^-$

**Example 12-472 Compound 472**

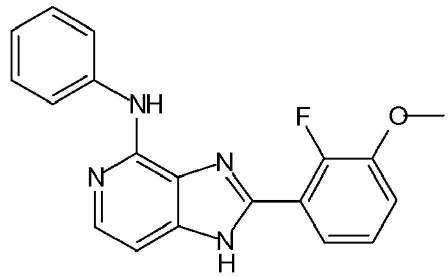

MW.: 334.12, LCMS-ESI (*m*/*z*) - found 335.12 $[M+H]^+$, 333.12 $[M-H]^-$

**Example 12-473 Compound 473**

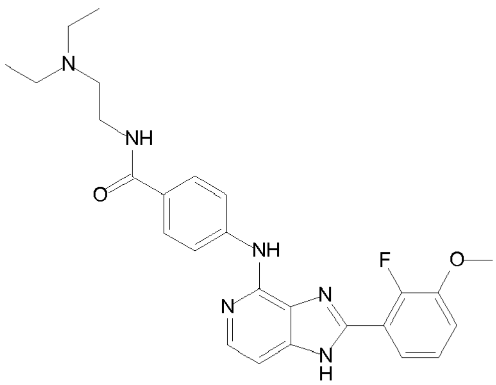

MW.: 476.23, LCMS-ESI (*m*/*z*) - found 477.23 $[M+H]^+$, 475.23 $[M-H]^-$

**Example 12-474 Compound 474**

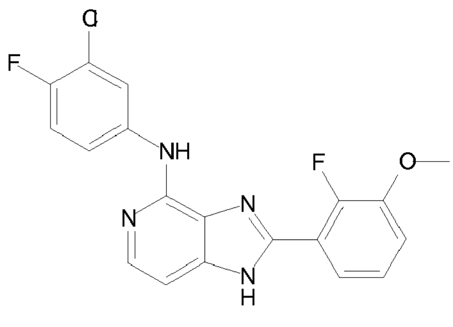

MW.: 386.07, LCMS-ESI (*m*/*z*) - found 387.07 $[M+H]^+$, 385.07 $[M-H]^-$

**Example 12-475 Compound 475**

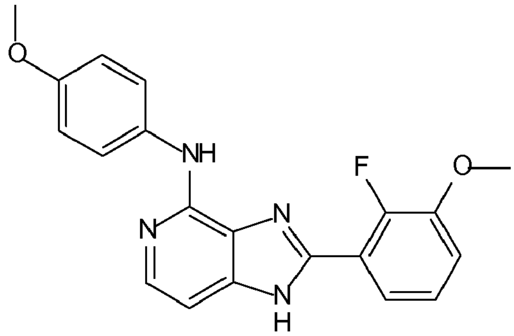

MW.: 364.13, LCMS-ESI (*m*/*z*) - found 365.13 $[M+H]^+$, 363.13 $[M-H]^-$

**Example 12-476 Compound 476**

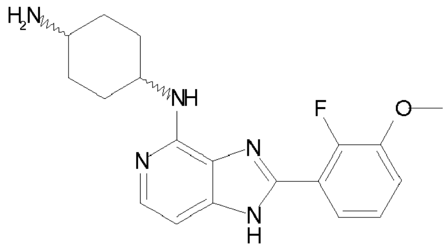

MW.: 355.18, LCMS-ESI (*m*/*z*) - found 356.18 $[M+H]^+$, 354.18 $[M-H]^-$

**Example 12-477 Compound 477**

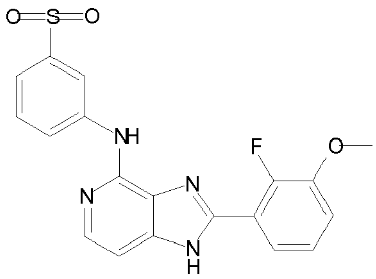

MW.: 412.10, LCMS-ESI (*m*/*z*) - found 413.10 $[M+H]^+$, 411.10 $[M-H]^-$

**Example 12-478 Compound 478**

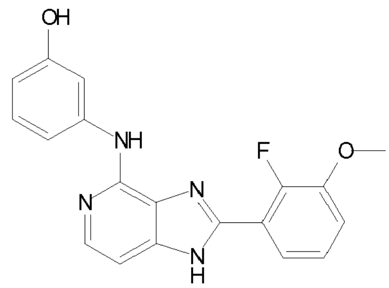

MW.: 350.12, LCMS-ESI (m/z) - found 351.11 $[M+H]^+$, 349.11 $[M-H]^-$

**Example 12-479 Compound 479**

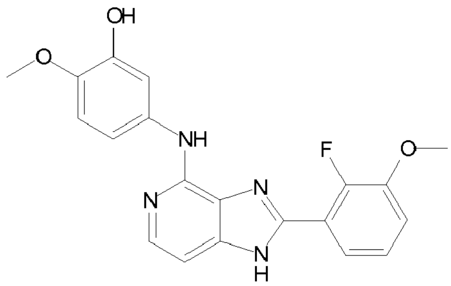

MW.: 380.13.16, LCMS-ESI (m/z) - found 381.12 $[M+H]^+$, 379.12 $[M-H]^-$

**Example 12-480 Compound 480**

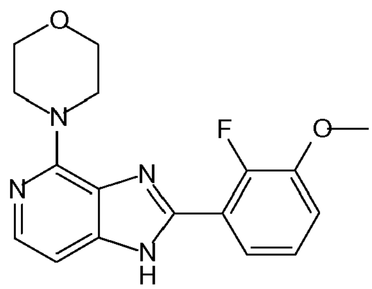

MW.: 328.13, LCMS-ESI (*m/z*) - found 329.13 $[M+H]^+$, 327.13 $[M-H]^-$

**Example 12-481 Compound 481**

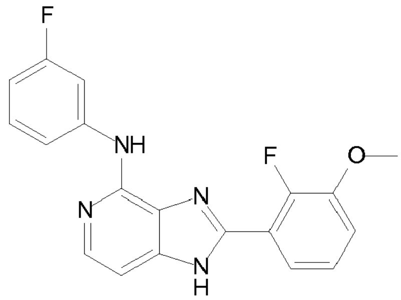

MW.: 352.11, LCMS-ESI (*m*/*z*) - found 353.11 $[M+H]^+$, 351.11 $[M-H]^-$

**Example 12-482 Compound 482**

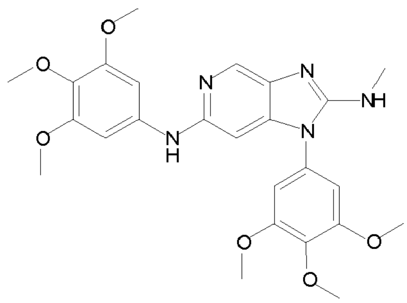

MW.: 495.21, LCMS-ESI (*m*/*z*) - found 496.21 $[M+H]^+$, 494.21 $[M-H]^-$

**Example 12-483 Compound 483**

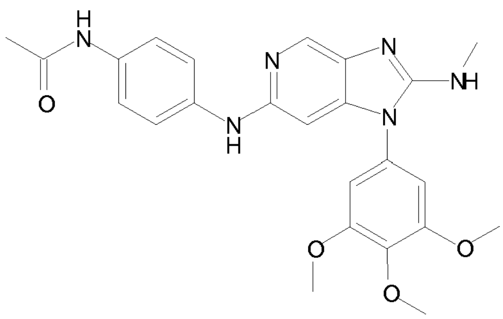

MW.: 462.2, LCMS-ESI (*m*/*z*) - found 463.2 $[M+H]^+$, 461.2 $[M-H]^-$

**Example 12-484 Compound 484**

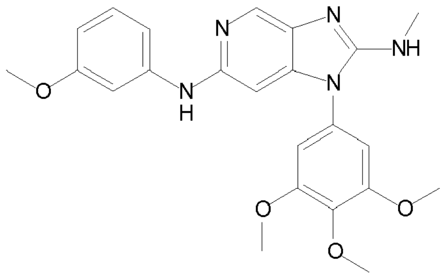

MW.: 435.19, LCMS-ESI (*m*/*z*) - found 436.19 $[M+H]^+$, 434.19 $[M-H]^-$

**Example 12-485 Compound 485**

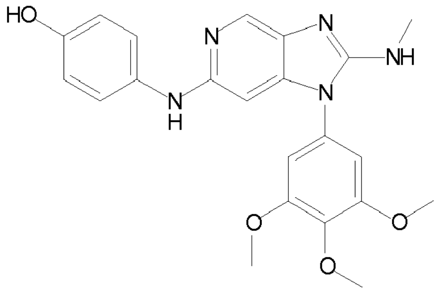

MW.: 421.17, LCMS-ESI (*m*/*z*) - found 422.17 $[M+H]^+$, 420.17 $[M-H]^-$

**Example 12-486 Compound 486**

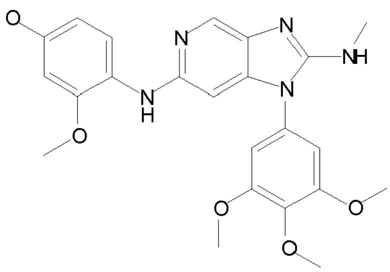

MW.: 465.20, LCMS-ESI (*m*/*z*) - found 466.2 $[M+H]^+$, 464.2 $[M-H]^-$

**Example 12-487 Compound 487**

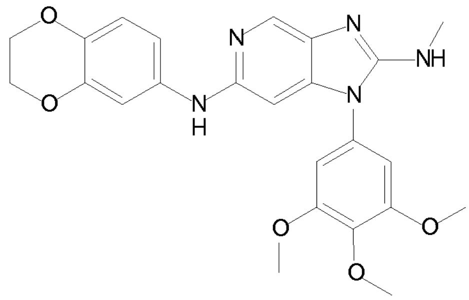

MW.: 463.19, LCMS-ESI (*m*/*z*) - found 464.18 $[M+H]^+$, 462.18 $[M-H]^-$

**Example 12-488 Compound 488**

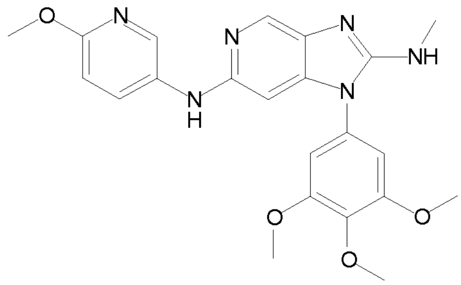

MW.: 436.19, LCMS-ESI (*m*/*z*) - found 437.18 $[M+H]^+$, 435.18 $[M-H]^-$

**Example 12-489 Compound 489**

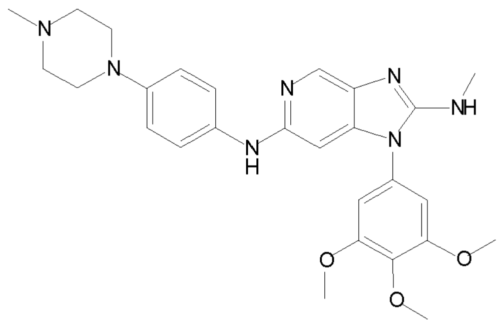

MW.: 503.26, LCMS-ESI (*m*/*z*) - found 504.26 $[M+H]^+$, 502.26 $[M-H]^-$

**Example 12-490 Compound 490**

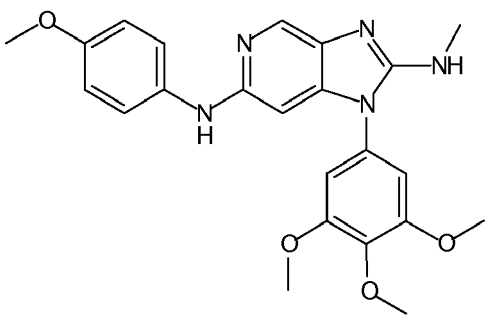

MW.: 435.19, LCMS-ESI (*m*/*z*) - found 436.19 $[M+H]^+$, 434.19 $[M-H]^-$

**Example 12-491 Compound 491**

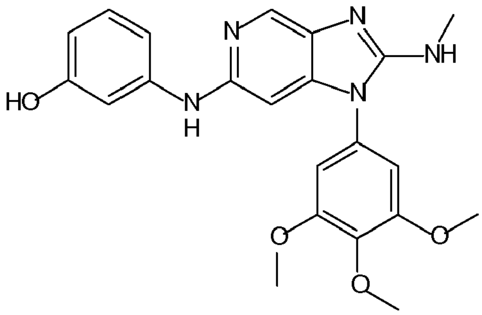

MW.: 421.17, LCMS-ESI (m/z) - found 422.17 $[M+H]^+$, 420.17 $[M-H]^-$

**Example 12-492 Compound 492**

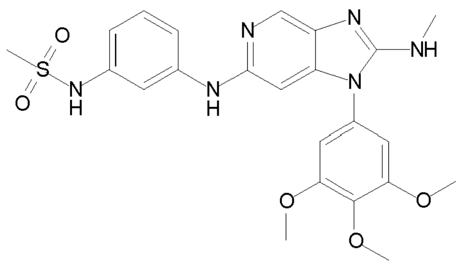

MW.: 498.17, LCMS-ESI (*m*/*z*) - found 499.16 $[M+H]^+$, 497.16 $[M-H]^-$

**Example 12-493 Compound 493**

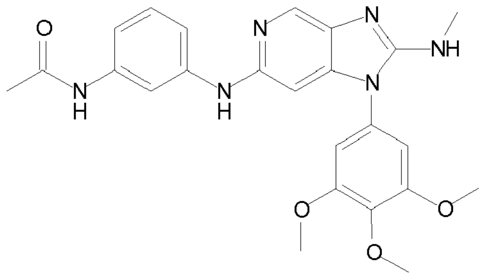

MW.: 462.20, LCMS-ESI (*m*/*z*) - found 463.20 $[M+H]^+$, 461.20 $[M-H]^-$

**Example 12-494 Compound 494**

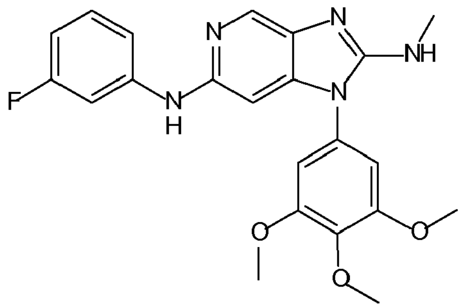

MW.: 423.17, LCMS-ESI (*m*/*z*) - found 424.17 $[M+H]^+$, 422.17 $[M-H]^-$

**Example 12-495 Compound 495**

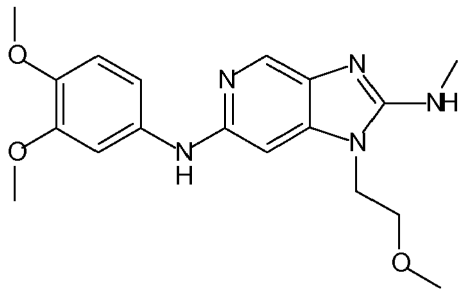

MW.: 357.18, LCMS-ESI (m/z) - found 358.18 $[M+H]^+$, 356.18 $[M-H]^-$

**Example 12-496 Compound 496**

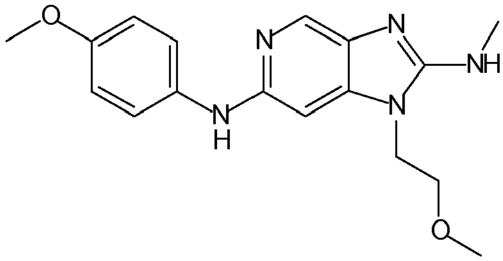

MW.: 327.17, LCMS-ESI (*m/z*) - found 328.17 [M+H]$^+$, 326.17 [M-H]$^-$

**Example 12-497 Compound 497**

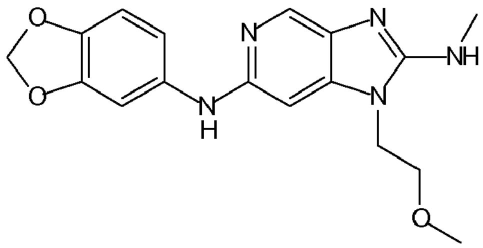

MW.: 341.15, LCMS-ESI (*m/z*) - found 342.14 [M+H]$^+$, 340.14 [M-H]$^-$

**Example 12-498 Compound 498**

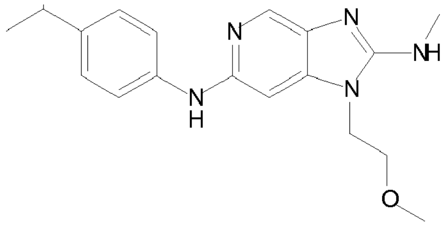

MW.: 339.21, LCMS-ESI (*m/z*) - found 340.2 [M+H]$^+$, 338.2 [M-H]$^-$

**Example 12-499 Compound 499**

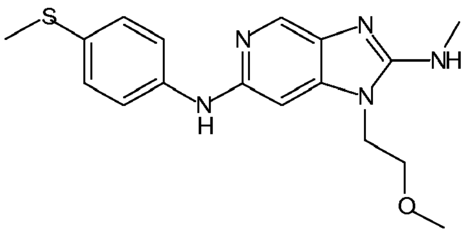

MW.: 343.15, LCMS-ESI (m/z) - found 344.14 [M+H]$^+$, 342.14 [M-H]$^-$

**Example** 12-500 Compound 500

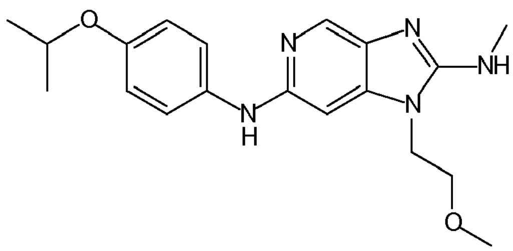

MW.: 355.20, LCMS-ESI (*m*/*z*) - found 356.20 $[M+H]^+$, 354.20 $[M-H]^-$

**Example 12-501 Compound 501**

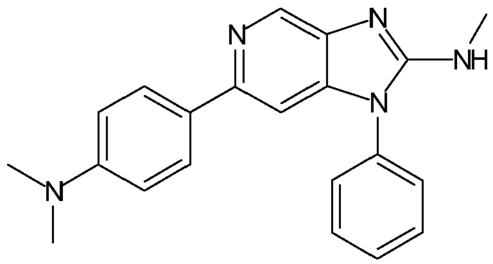

MW.: 343.18, LCMS-ESI (*m*/*z*) - found 344.18 $[M+H]^+$, 342.18 $[M-H]^-$

**Example 12-502 Compound 502**

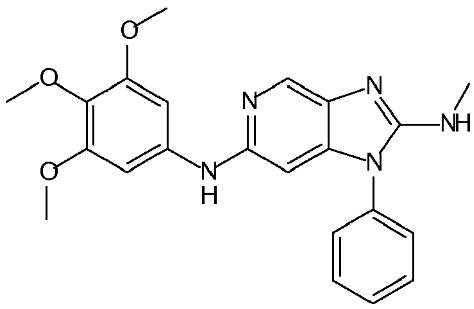

MW.: 405.18, LCMS-ESI (*m*/*z*) - found 406.18 $[M+H]^+$, 404.18 $[M-H]^-$

**Example 12-503 Compound 503**

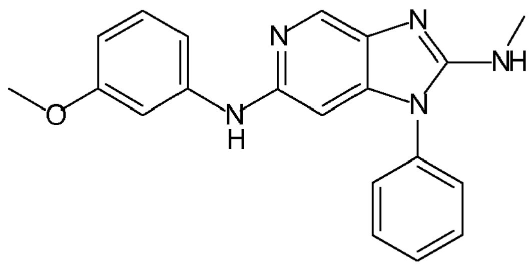

MW.: 345.16, LCMS-ESI (m/z) - found 346.15 $[M+H]^+$, 344.15 $[M-H]^-$

**Example 12-504 Compound 504**

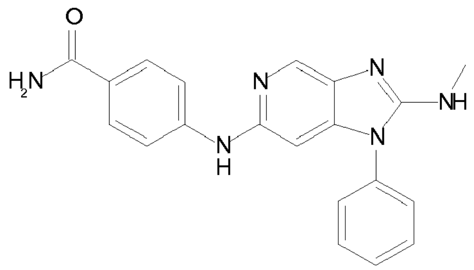

MW.: 358.15, LCMS-ESI (*m*/*z*) - found 359.15 $[M+H]^+$, 357.15 $[M-H]^-$

**Example 12-505 Compound 505**

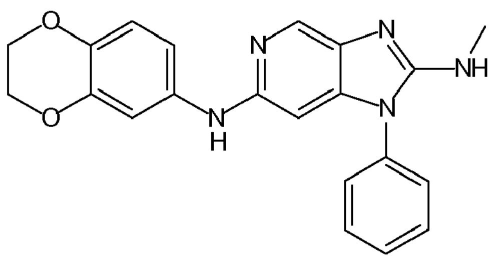

MW.: 373.15, LCMS-ESI (*m*/*z*) - found 374.15 $[M+H]^+$, 372.15 $[M-H]^-$

**Example 12-506 Compound 506**

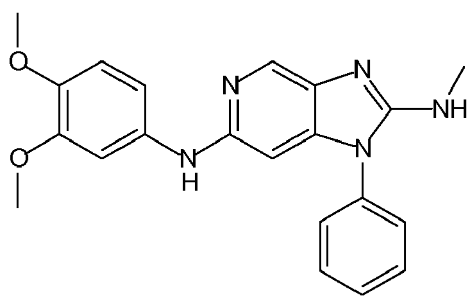

MW.: 375.17, LCMS-ESI ($m/z$) - found 376.17 $[M+H]^+$, 374.17 $[M-H]^-$

**Example 12-507 Compound 507**

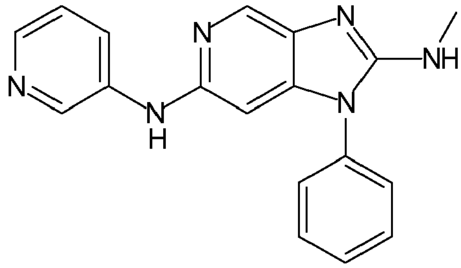

MW.: 316.14, LCMS-ESI ($m/z$) - found 317.14 $[M+H]^+$, 315.14 $[M-H]^-$

**Example 12-508 Compound 508**

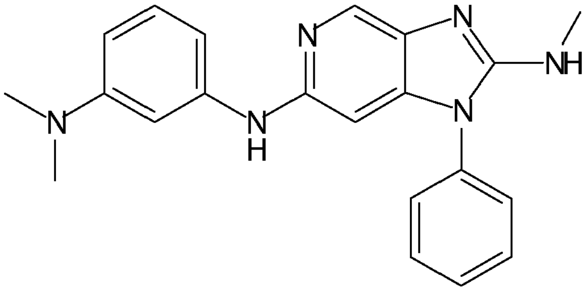

MW.: 358.19, LCMS-ESI ($m/z$) - found 359.19 $[M+H]^+$, 357.19 $[M-H]^-$

**Example 12-509 Compound 509**

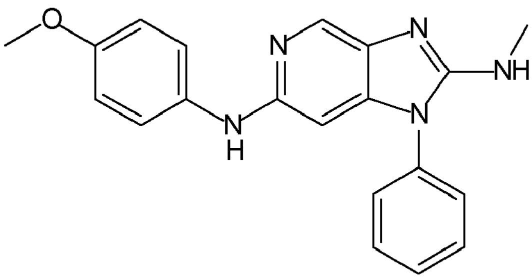

MW.: 345.16, LCMS-ESI ($m/z$) - found 346.15 $[M+H]^+$, 344.15 $[M-H]^-$

**Example 12-510 Compound 510**

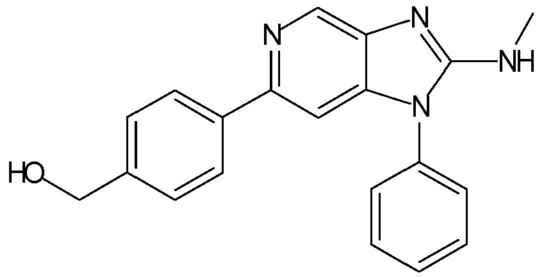

MW.: 330.15, LCMS-ESI (*m*/*z*) - found 331.14 $[M+H]^+$, 329.14 $[M-H]^-$

**Example 12-511 Compound 511**

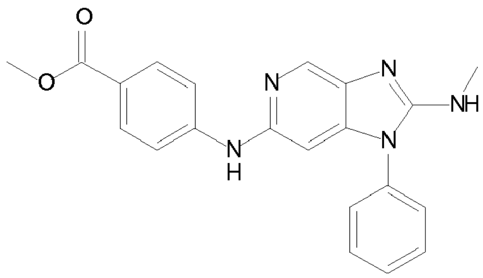

MW.: 373.15, LCMS-ESI (*m*/*z*) - found 374.15 $[M+H]^+$, 372.15 $[M-H]^-$

**Example 12-512 Compound 512**

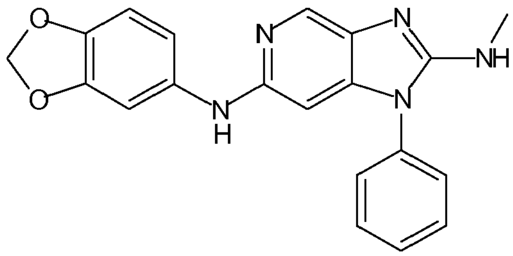

MW.: 359.14, LCMS-ESI (*m*/*z*) - found 360.13 $[M+H]^+$, 358.13 $[M-H]^-$

**Example 12-513 Compound 513**

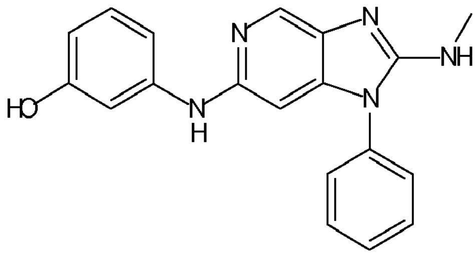

MW.: 331.14, LCMS-ESI (*m*/*z*) - found 332.14 $[M+H]^+$, 330.14 $[M-H]^-$

**Example 12-514 Compound 514**

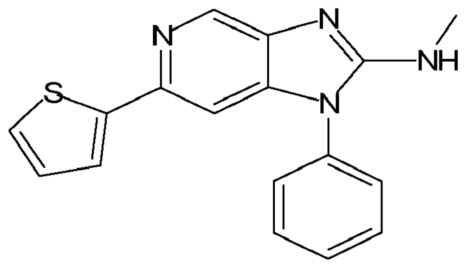

MW.: 306.09, LCMS-ESI (*m*/*z*) - found 307.09 $[M+H]^+$, 305.09 $[M-H]^-$

**Example 12-515 Compound 515**

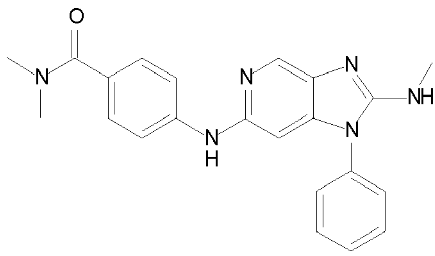

MW.: 386.19, LCMS-ESI (m/z) - found 387.18 $[M+H]^+$, 385.18 $[M-H]^-$

**Example 12-516 Compound 516**

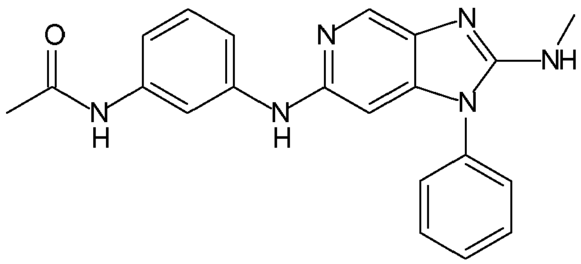

MW.: 372.17, LCMS-ESI (*m*/*z*) - found 373.17 $[M+H]^+$, 371.17 $[M-H]^-$

**Example 12-517 Compound 517**

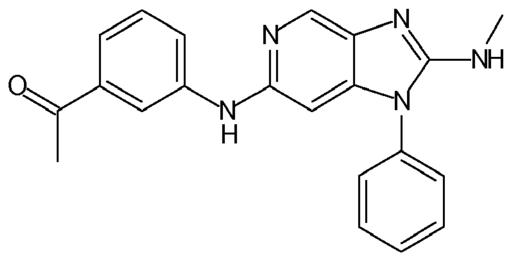

MW.: 357.16, LCMS-ESI (*m*/*z*) - found 358.15 $[M+H]^+$, 356.15 $[M-H]^-$

**Example 12-518 Compound 518**

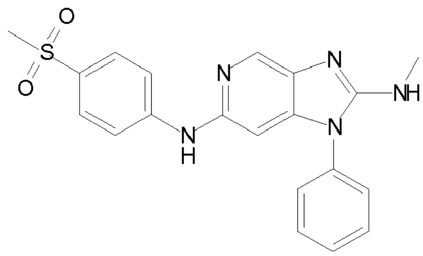

MW.: 393.13, LCMS-ESI (*m*/*z*) - found 394.12 $[M+H]^+$, 392.12 $[M-H]^-$

**Example 12-519 Compound 519**

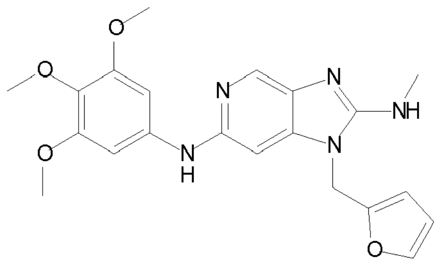

MW.: 409.17, LCMS-ESI (*m*/*z*) - found 410.17 $[M+H]^+$, 408.17 $[M-H]^-$

**Example 12-520 Compound 520**

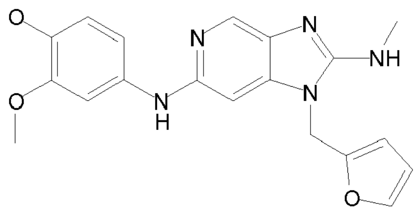

MW.: 379.16, LCMS-ESI (*m*/*z*) - found 380.16 $[M+H]^+$, 378.16 $[M-H]^-$

**Example 12-521 Compound 521**

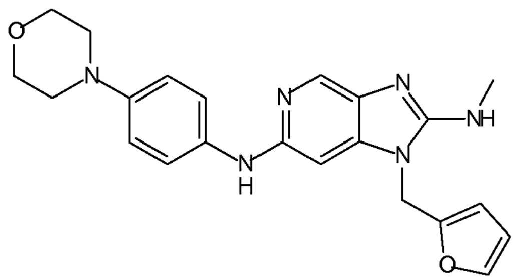

MW.: 404.20, LCMS-ESI (*m*/*z*) - found 405.19 $[M+H]^+$, 403.19 $[M-H]^-$

**Example 12-522 Compound 522**

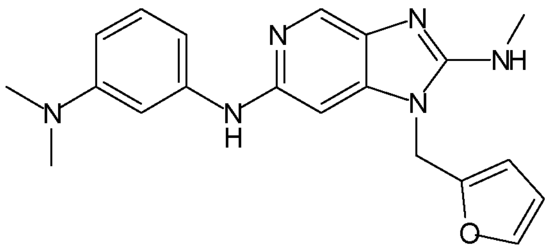

MW.: 362.19, LCMS-ESI (*m*/*z*) - found 363.18 $[M+H]^+$, 361.18 $[M-H]^-$

**Example 12-523 Compound 523**

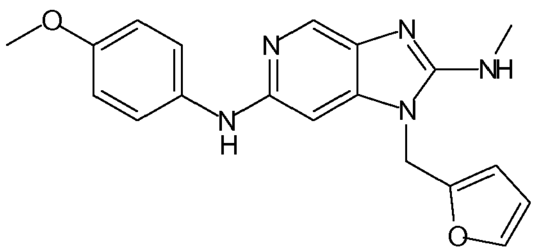

MW.: 349.15, LCMS-ESI (*m*/*z*) - found 350.15 $[M+H]^+$, 348.15 $[M-H]^-$

**Example 12-524 Compound 524**

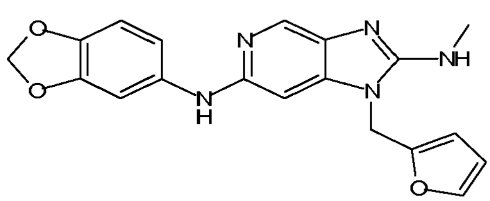

MW.: 363.13, LCMS-ESI (*m*/*z*) - found 364.13 $[M+H]^+$, 362.13 $[M-H]^-$

**Example 12-525 Compound 525**

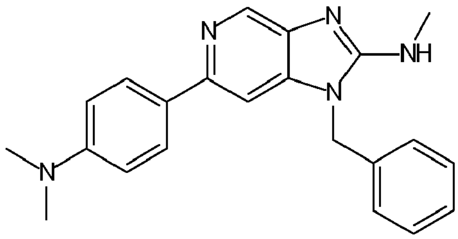

MW.: 357.20, LCMS-ESI (*m*/*z*) - found 358.20 $[M+H]^+$, 356.20 $[M-H]^-$

**Example 12-526 Compound 526**

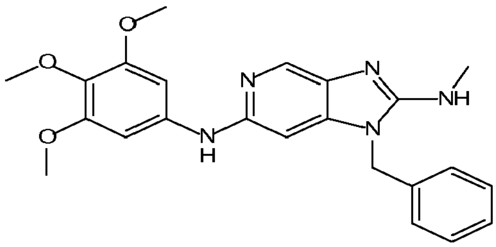

MW.: 419.20, LCMS-ESI (*m*/*z*) - found 420.19 $[M+H]^+$, 418.20 $[M-H]^-$

**Example 12-527 Compound 527**

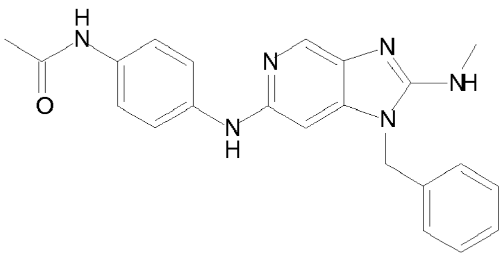

MW.: 386.19, LCMS-ESI (*m*/*z*) - found 387.19 $[M+H]^+$, 385.19 $[M-H]^-$

**Example 12-528 Compound 528**

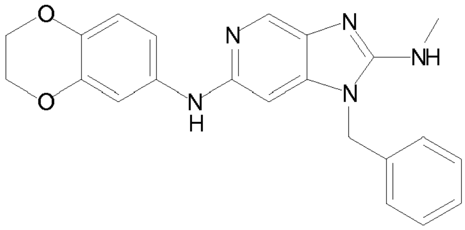

MW.: 387.17, LCMS-ESI (*m/z*) - found 388.17 $[M+H]^+$, 386.17 $[M-H]^-$

**Example 12-529 Compound 529**

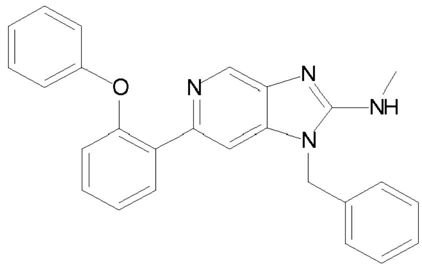

MW.: 406.18, LCMS-ESI (*m/z*) - found 407.17 $[M+H]^+$, 405.17 $[M-H]^-$

**Example 12-530 Compound 530**

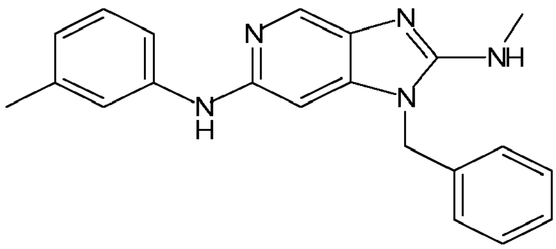

MW.: 343.18, LCMS-ESI (*m/z*) - found 344.18 $[M+H]^+$, 342.18 $[M-H]^-$

**Example 12-531 Compound 531**

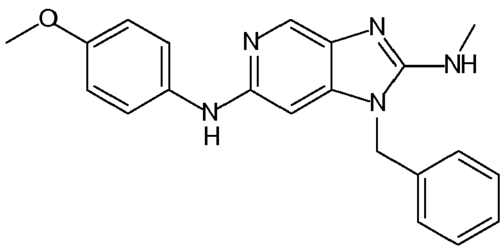

MW.: 359.17, LCMS-ESI (*m/z*) - found 360.17 $[M+H]^+$, 358.17 $[M-H]^-$

**Example 12-532 Compound 532**

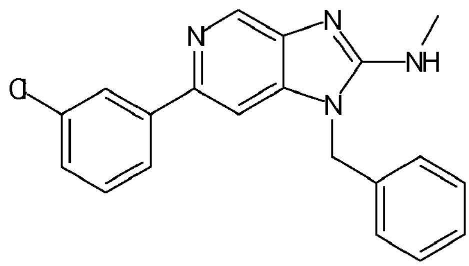

MW.: 348.11, LCMS-ESI (m/z) - found 349.11 $[M+H]^+$, 347.11 $[M-H]^-$

**Example** 12-533 Compound 533

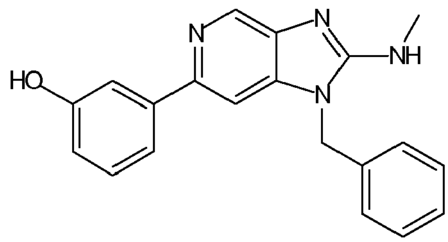

MW.: 330.15, LCMS-ESI (*m*/*z*) - found 331.14 $[M+H]^+$, 329.14 $[M-H]^-$

**Example 12-534 Compound 534**

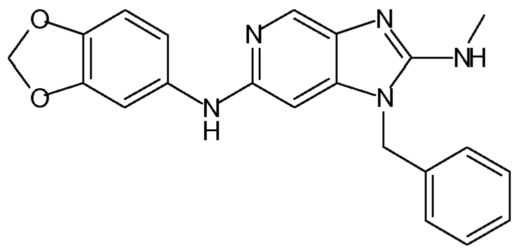

MW.: 373.15, LCMS-ESI (*m*/*z*) - found 374.15 $[M+H]^+$, 372.15 $[M-H]^-$

**Example 12-535 Compound 535**

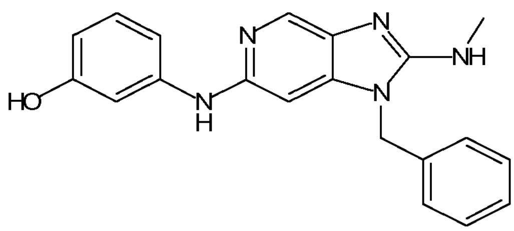

MW.: 345.16, LCMS-ESI (*m*/*z*) - found 346.15 $[M+H]^+$, 344.15 $[M-H]^-$

**Example 12-536 Compound 536**

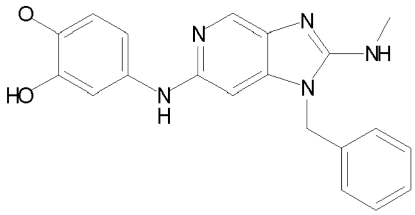

MW.: 375.17, LCMS-ESI (*m*/*z*) - found 376.17 $[M+H]^+$, 374.17 $[M-H]^-$

**Example 12-537 Compound 537**

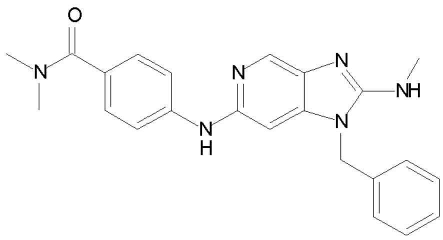

MW.: 400.2, LCMS-ESI (*m*/*z*) - found 401.2 $[M+H]^+$, 399.2 $[M-H]^-$

**Example 12-538 Compound 538**

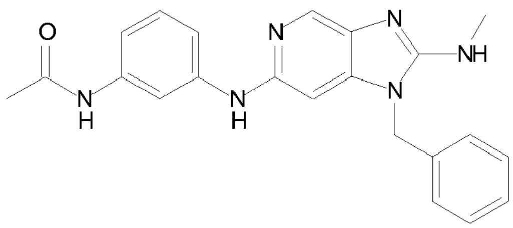

MW.: 386.19, LCMS-ESI (*m*/*z*) - found 387.18 $[M+H]^+$, 385.18 $[M-H]^-$

**Example 12-539 Compound 539**

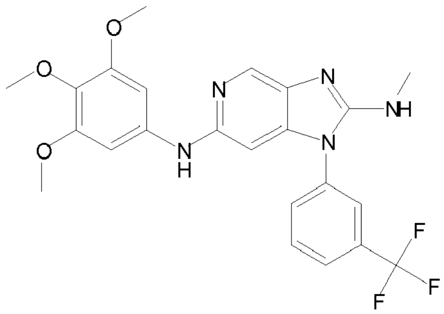

MW.: 473.17, LCMS-ESI (*m*/*z*) - found 474.16 $[M+H]^+$, 472.16 $[M-H]^-$

**Example 12-540 Compound 540**

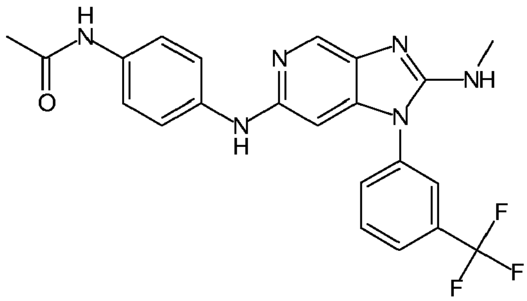

MW.: 440.16, LCMS-ESI (*m*/*z*) - found 441.15 $[M+H]^+$, 439.15 $[M-H]^-$

**Example 12-541 Compound 541**

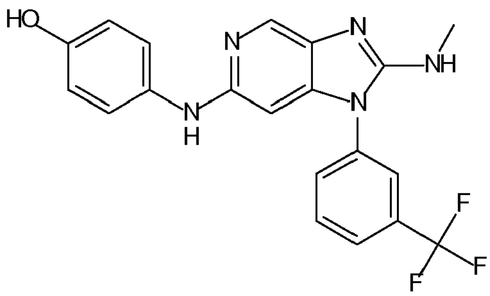

MW.: 399.13, LCMS-ESI (m/z) - found 400.13 $[M+H]^+$, 398.13 $[M-H]^-$

**Example 12-542 Compound 542**

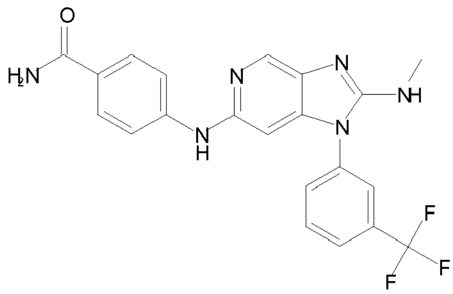

MW.: 426.14, LCMS-ESI (*m/z*) - found 427.14 $[M+H]^+$, 425.14 $[M-H]^-$

**Example 12-543 Compound 543**

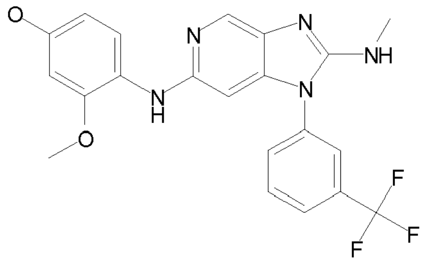

MW.: 443.16, LCMS-ESI (*m/z*) - found 444.15 $[M+H]^+$, 442.15 $[M-H]^-$

**Example 12-544 Compound 544**

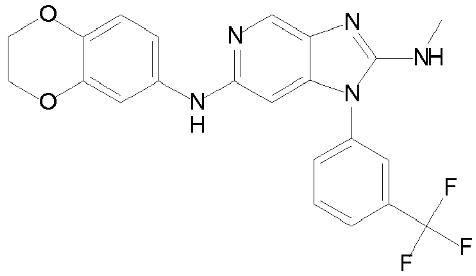

MW.: 441.14, LCMS-ESI (m/z) - found 442.14 $[M+H]^+$, 440.14 $[M-H]^-$

**Example 12-545 Compound 545**

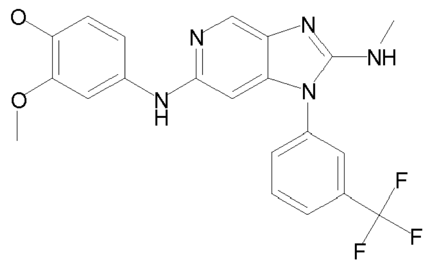

MW.: 443.16, LCMS-ESI (m/z) - found 444.15 [M+H]$^+$, 442.15 [M-H]$^-$

**Example 12-546 Compound 546**

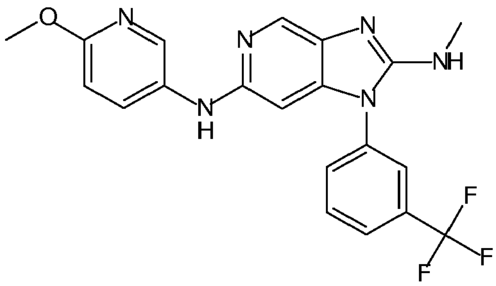

MW.: 414.14, LCMS-ESI (*m*/*z*) - found 415.14 [M+H]$^+$, 413.14 [M-H]$^-$

**Example 12-547 Compound 547**

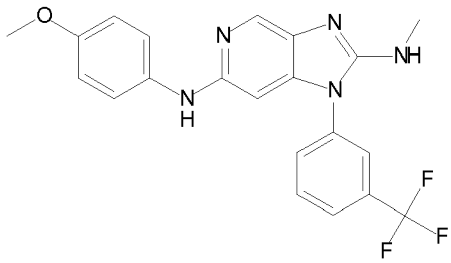

MW.: 413.15, LCMS-ESI (*m*/*z*) - found 414.14 [M+H]$^+$, 412.14 [M-H]$^-$

**Example 12-548 Compound 548**

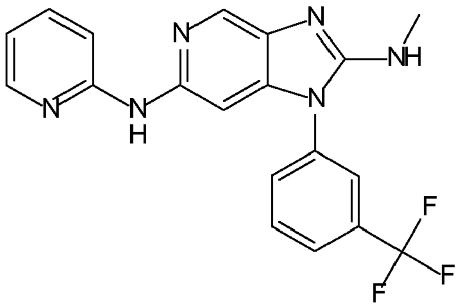

MW.: 384.13, LCMS-ESI (*m*/*z*) - found 385.13 [M+H]$^+$, 383.13 [M-H]$^-$

**Example 12-549 Compound 549**

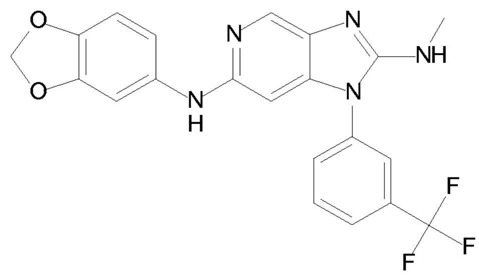

MW.: 427.13, LCMS-ESI (*m*/*z*) - found 428.13 $[M+H]^+$, 426.13 $[M-H]^-$

**Example 12-550 Compound 550**

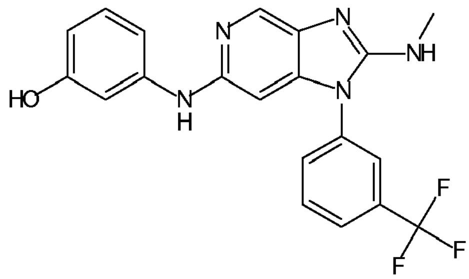

MW.: 399.13, LCMS-ESI (*m*/*z*) - found 400.13 $[M+H]^+$, 398.11 $[M-H]^-$

**Example 12-551 Compound 551**

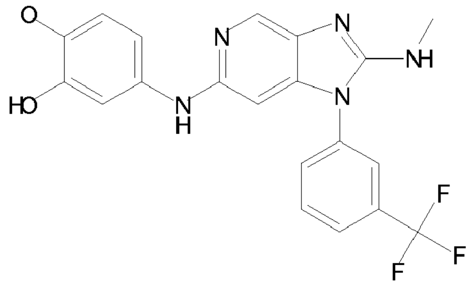

MW.: 429.14, LCMS-ESI (*m*/*z*) - found 430.14 $[M+H]^+$, 428.14 $[M-H]^-$

**Example 12-552 Compound 552**

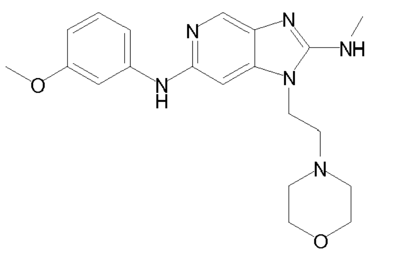

MW.: 382.21, LCMS-ESI (*m*/*z*) - found 383.21 $[M+H]^+$, 381.21 $[M-H]^-$

**Example 12-553 Compound 553**

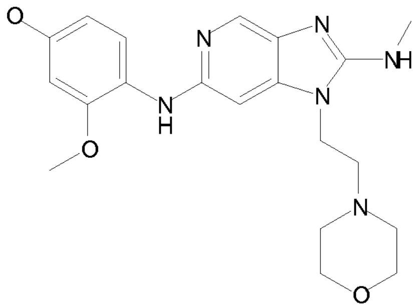

MW.: 412.22, LCMS-ESI (m/z) - found 413.22 $[M+H]^+$, 411.22 $[M-H]^-$

**Example 12-554 Compound 554**

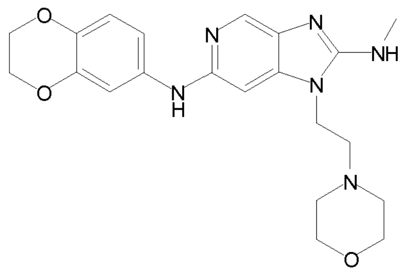

MW.: 410.21, LCMS-ESI (*m*/*z*) - found 411.20 $[M+H]^+$, 409.20 $[M-H]^-$

**Example 12-555 Compound 555**

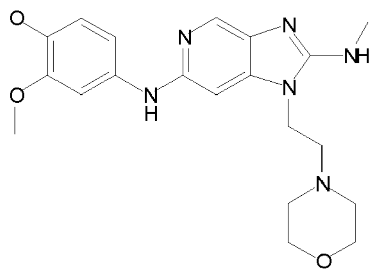

MW.: 412.22 LCMS-ESI (*m*/*z*) - found 413.22 $[M+H]^+$, 411.22 $[M-H]^-$

**Example 12-556 Compound 556**

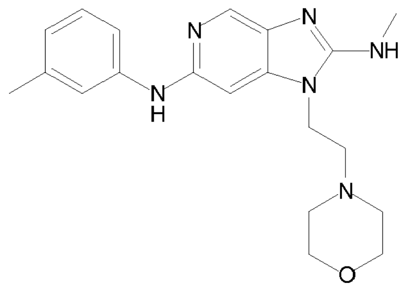

MW.: 366.22, LCMS-ESI (*m*/*z*) - found 367.21 $[M+H]^+$, 365.21 $[M-H]^-$

**Example 12-557 Compound 557**

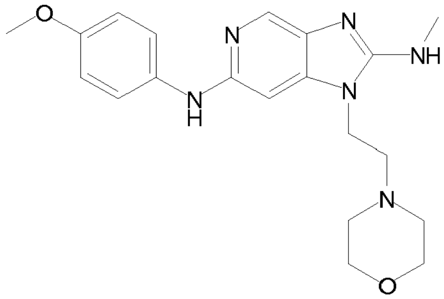

MW.: 382.21, LCMS-ESI (*m*/*z*) - found 383.21 $[M+H]^+$, 381.21 $[M-H]^-$

**Example 12-558 Compound 558**

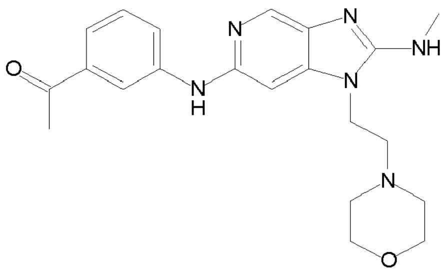

MW.: 394.21, LCMS-ESI (*m*/*z*) - found 395.21 $[M+H]^+$, 393.21 $[M-H]^-$

**Example 12-559 Compound 559**

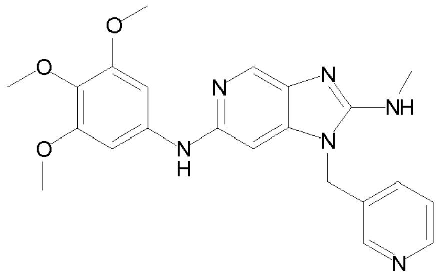

MW.: 420.19, LCMS-ESI (*m*/*z*) - found 421.19 $[M+H]^+$, 419.19 $[M-H]^-$

**Example 12-560 Compound 560**

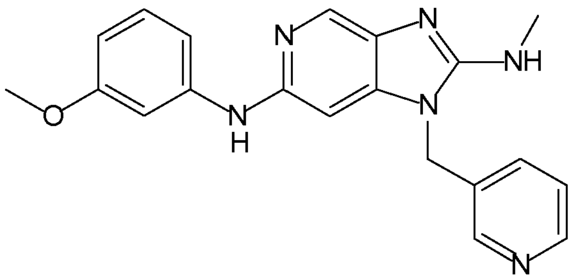

MW.: 360.17, LCMS-ESI (*m*/*z*) - found 361.17 $[M+H]^+$, 359.17 $[M-H]^-$

**Example 12-561 Compound 561**

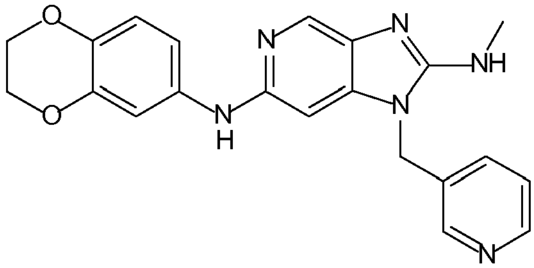

MW.: 388.16, LCMS-ESI (*m*/*z*) - found 389.16 $[M+H]^+$, 387.16 $[M-H]^-$

**Example** 12-562 Compound 562

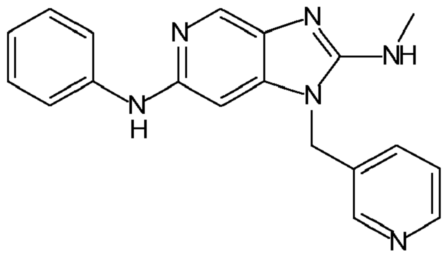

MW.: 330.16, LCMS-ESI (m/z) - found 331.15 [M+H]+, 329.15 [M-H]-

**Example 12-563 Compound 563**

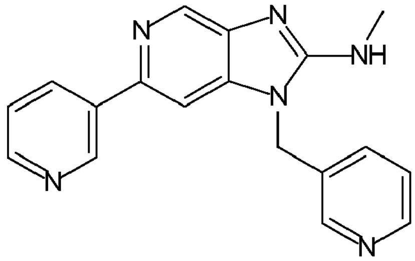

MW.: 316.14, LCMS-ESI (*m*/*z*) - found 317.14 [M+H]+, 315.14 [M-H]-

**Example 12-564 Compound 564**

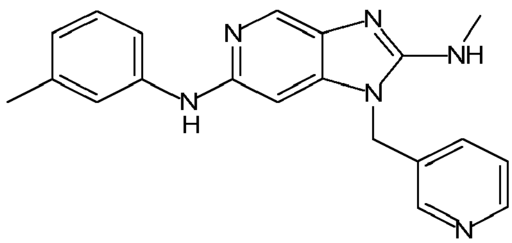

MW.: 344.17, LCMS-ESI (*m*/*z*) - found 345.17 [M+H]+, 343.17 [M-H]-

**Example 12-565 Compound 565**

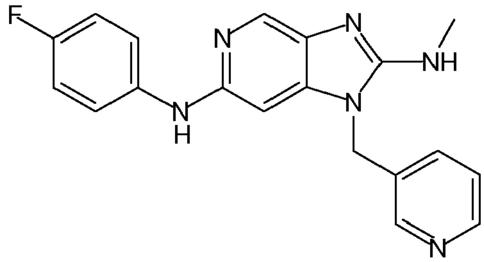

MW.: 348.15, LCMS-ESI (*m*/*z*) - found 349.15 [M+H]+, 347.15 [M-H]-

**Example 12-566 Compound 566**

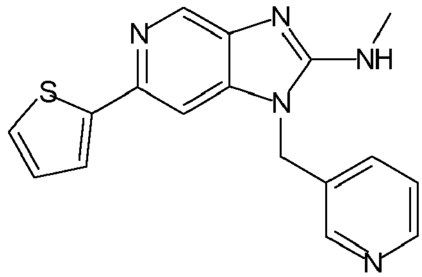

MW.: 321.10, LCMS-ESI (*m*/*z*) - found 322.10 $[M+H]^+$, 320.10 $[M-H]^-$

**Example 12-567 Compound 567**

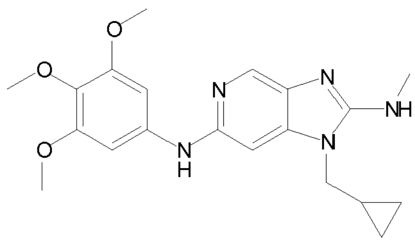

MW.: 383.20, LCMS-ESI (*m*/*z*) - found 384.20 $[M+H]^+$, 382.20 $[M-H]^-$

**Example 12-568 Compound 568**

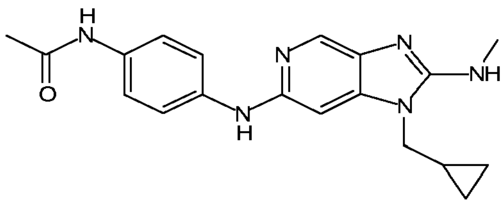

MW.: 350.19, LCMS-ESI (*m*/*z*) - found 351.18 $[M+H]^+$, 349.18 $[M-H]^-$

**Example 12-569 Compound 569**

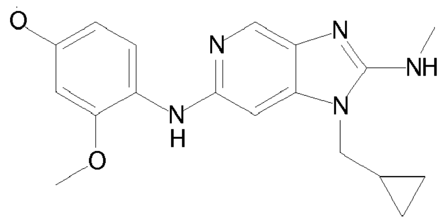

MW.: 353.19, LCMS-ESI (*m*/*z*) - found 354.18 $[M+H]^+$, 352.18 $[M-H]^-$

**Example 12-570 Compound 570**

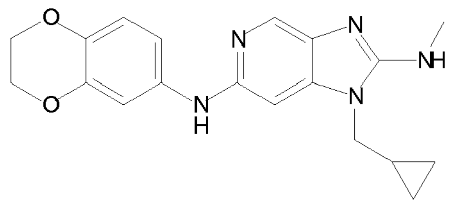

MW.: 351.17, LCMS-ESI (*m/z*) - found 352.17 $[M+H]^+$, 350.17 $[M-H]^-$

**Example 12-571 Compound 571**

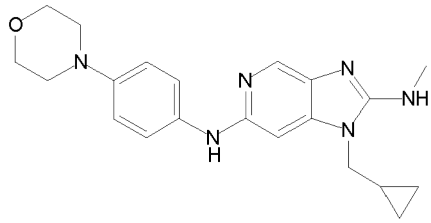

MW.: 378.22, LCMS-ESI (*m/z*) - found 379.21 $[M+H]^+$, 377.21 $[M-H]^-$

**Example 12-572 Compound 572**

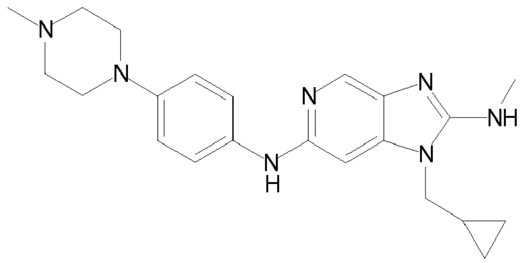

MW.: 391.25, LCMS-ESI (*m/z*) - found 392.24 $[M+H]^+$, 390.24 $[M-H]^-$

**Example 12-573 Compound 573**

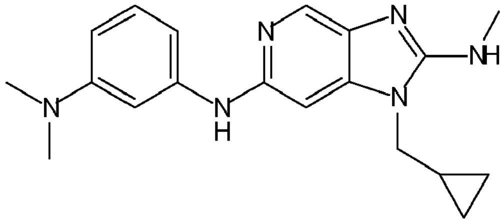

MW.: 336.21, LCMS-ESI (*m/z*) - found 337.20 $[M+H]^+$, 335.20 $[M-H]^-$

**Example 12-574 Compound 574**

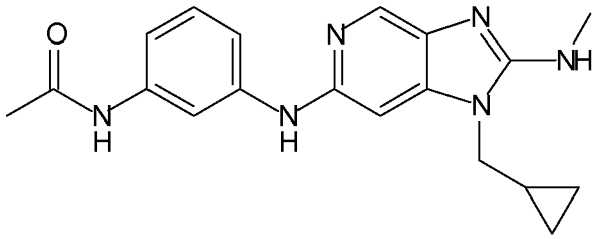

MW.: 350.19, LCMS-ESI (*m*/*z*) - found 351.18 $[M+H]^+$, 349.18 $[M-H]^-$

**Example 12-575 Compound 575**

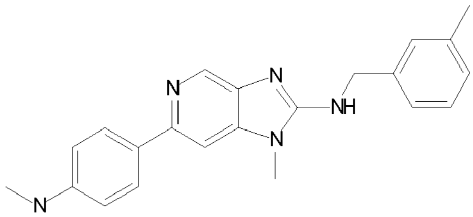

MW.: 371.21, LCMS-ESI (m/z) - found 372.21 $[M+H]^+$, 373.21 $[M-H]^-$

**Example 12-576 Compound 576**

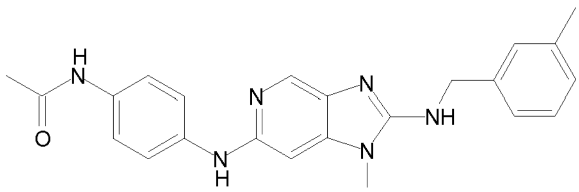

MW.: 400.20, LCMS-ESI (*m*/*z*) - found 401.20 $[M+H]^+$, 399.20 $[M-H]^-$

**Example 12-577 Compound 577**

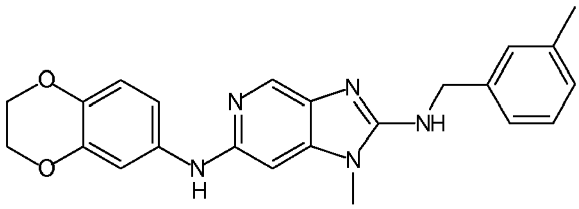

MW.: 401.19, LCMS-ESI (*m*/*z*) - found 402.18 $[M+H]^+$, 400.18 $[M-H]^-$

**Example 12-578 Compound 578**

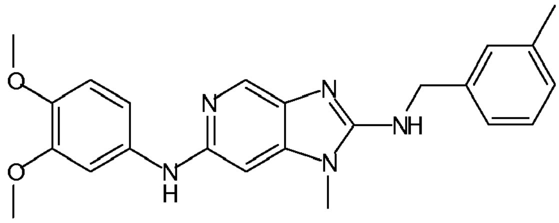

MW.: 403.20, LCMS-ESI (*m*/*z*) - found 404.20 $[M+H]^+$, 402.20 $[M-H]^-$

**Example 12-579 Compound 579**

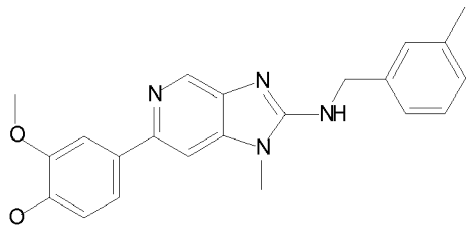

MW.: 388.19, LCMS-ESI (*m*/*z*) - found 389.19 $[M+H]^+$, 387.19 $[M-H]^-$

**Example 12-580 Compound 580**

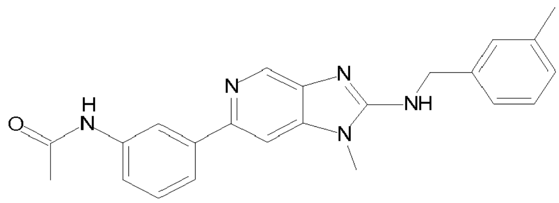

MW.: 385.19, LCMS-ESI (*m*/*z*) - found 386.19 $[M+H]^+$, 384.19 $[M-H]^-$

**Example 12-581 Compound 581**

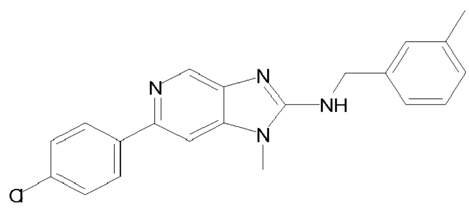

MW.: 362.13, LCMS-ESI (*m*/*z*) - found 363.13 $[M+H]^+$, 361.13 $[M-H]^-$

**Example 12-582 Compound 582**

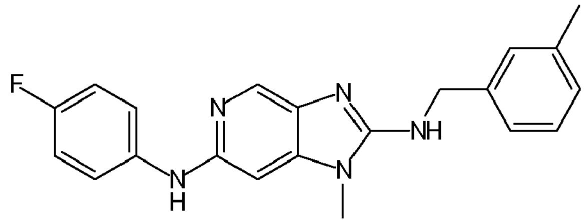

MW.: 361.17, LCMS-ESI (*m*/*z*) - found 362.17 $[M+H]^+$, 360.17 $[M-H]^-$

**Example 12-583 Compound 583**

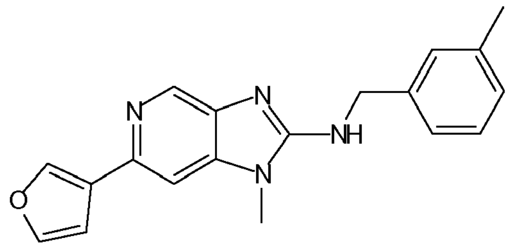

MW.: 318.15, LCMS-ESI (*m*/*z*) - found 319.14 $[M+H]^+$, 317.14 $[M-H]^-$

**Example 12-584 Compound 584**

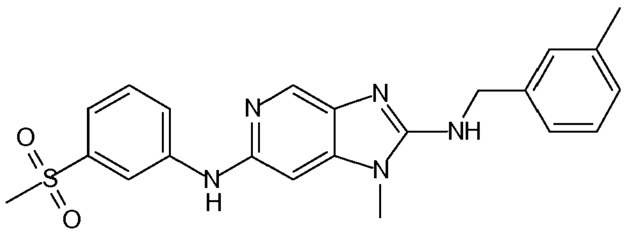

MW.: 421.16, LCMS-ESI (*m*/*z*) - found 422.15 $[M+H]^+$, 420.15 $[M-H]^-$

**Example 12-585 Compound 585**

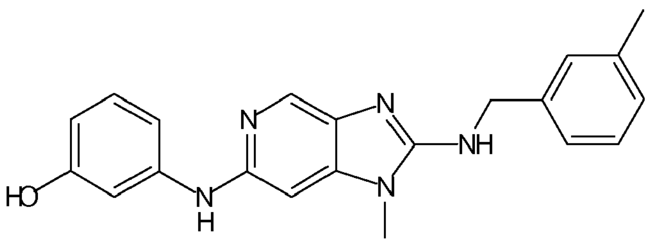

MW.: 359.17, LCMS-ESI ($m/z$) - found 360.17 $[M+H]^+$, 358.17 $[M-H]^-$

**Example 12-586 Compound 586**

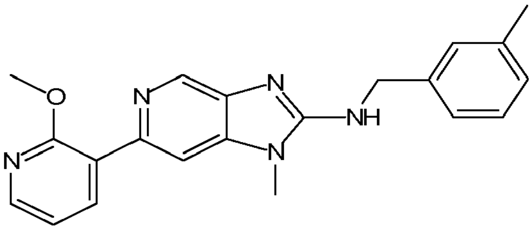

MW.: 359.17, LCMS-ESI ($m/z$) - found 360.17 $[M+H]^+$, 358.17 $[M-H]^-$

**Example 12-587 Compound 587**

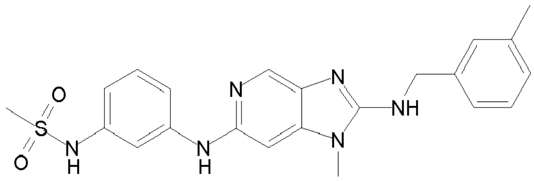

MW.: 436.17, LCMS-ESI ($m/z$) - found 437.16 $[M+H]^+$, 435.16 $[M-H]^-$

**Example 12-588 Compound 588**

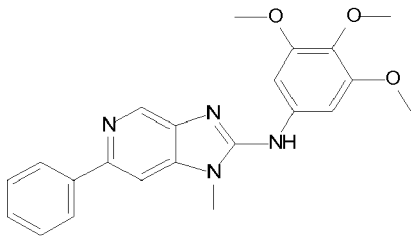

MW.: 390.17, LCMS-ESI ($m/z$) - found 391.16 $[M+H]^+$, 389.16 $[M-H]^-$

**Example 12-589 Compound 589**

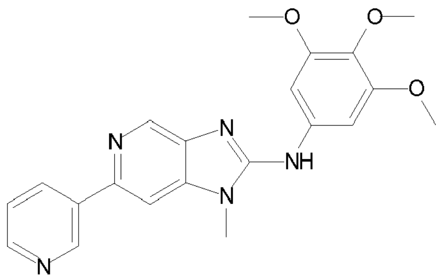

MW.: 391.16 LCMS-ESI (*m*/*z*) - found 392.16 $[M+H]^+$, 390.16 $[M-H]^-$

**Example 12-590 Compound 590**

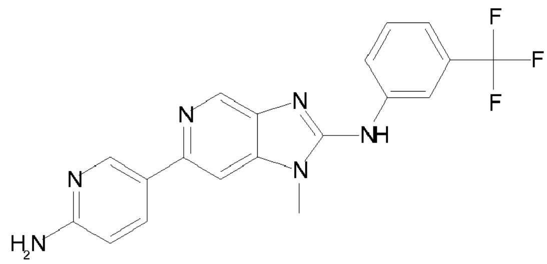

MW.: 384.13, LCMS-ESI (*m*/*z*) - found 385.13 $[M+H]^+$, 383.13 $[M-H]^-$

**Example 12-591 Compound 591**

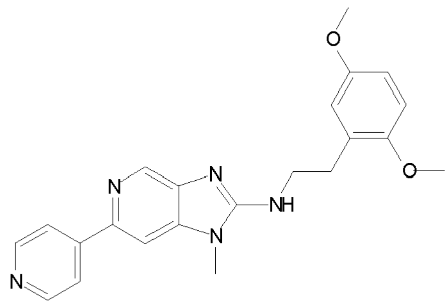

MW.: 389.19, LCMS-ESI (*m*/*z*) - found 390.19 $[M+H]^+$, 388.19 $[M-H]^-$

**Example 12-592 Compound 592**

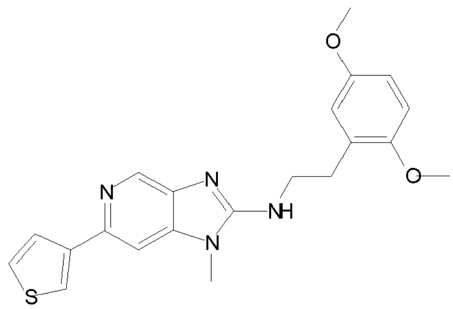

MW.: 394.15, LCMS-ESI (*m*/*z*) - found 395.14 $[M+H]^+$, 393.14 $[M-H]^-$

**Example 12-593 Compound 593**

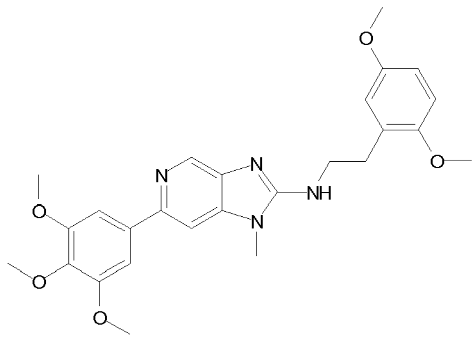

MW.: 478.22, LCMS-ESI (*m*/*z*) - found 479.22 $[M+H]^+$, 477.22 $[M-H]^-$

**Example 12-594 Compound 594**

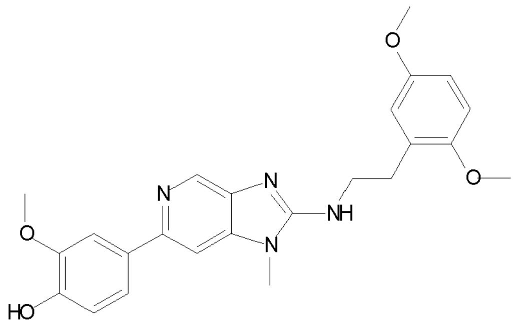

MW.: 434.20, LCMS-ESI (*m*/*z*) - found 435.19 $[M+H]^+$, 433.19 $[M-H]^-$

**Example 12-595 Compound 595**

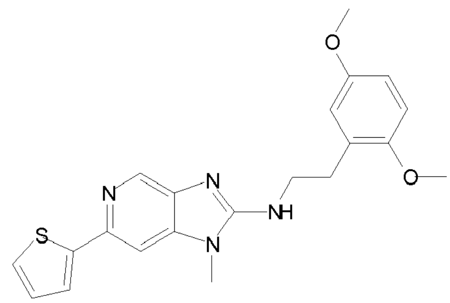

MW.: 394.15, LCMS-ESI (*m*/*z*) - found 395.14 $[M+H]^+$, 393.14 $[M-H]^-$

**Example 12-596 Compound 596**

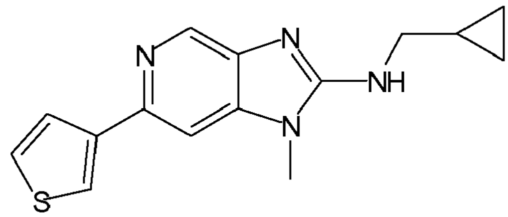

MW.: 284.11, LCMS-ESI (*m*/*z*) - found 285.11 $[M+H]^+$, 283.11 $[M-H]^-$

**Example 12-597 Compound 597**

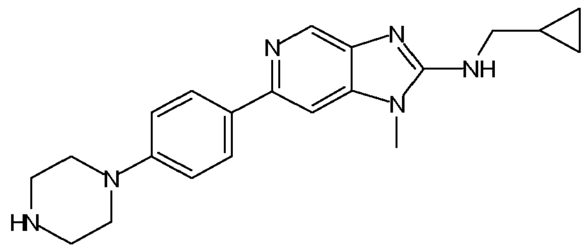

MW.: 366.22, LCMS-ESI (*m*/*z*) - found 363.22 $[M+H]^+$, 361.22 $[M-H]^-$

**Example 12-598 Compound 598**

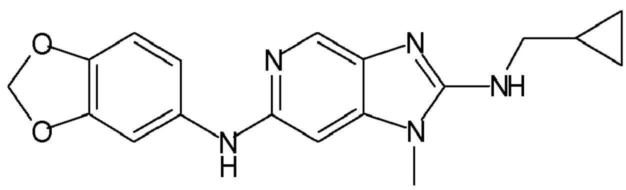

MW.: 337.15, LCMS-ESI (*m*/*z*) - found 338.15 $[M+H]^+$, 336.15 $[M-H]^-$

**Example 12-599 Compound 599**

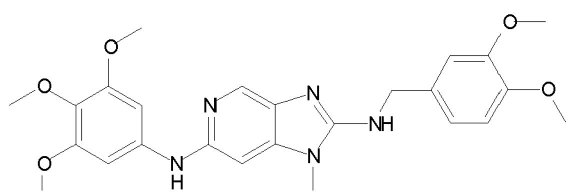

MW.: 479.22, LCMS-ESI (*m*/*z*) - found 480.21 $[M+H]^+$, 478.21 $[M-H]^-$

**Example 12-600 Compound 600**

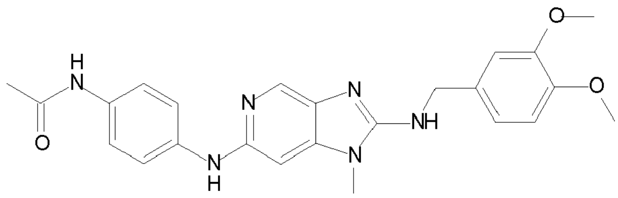

MW.: 446.21, LCMS-ESI (*m/z*) - found 447.20 $[M+H]^+$, 445.20 $[M-H]^-$

**Example 12-601 Compound 601**

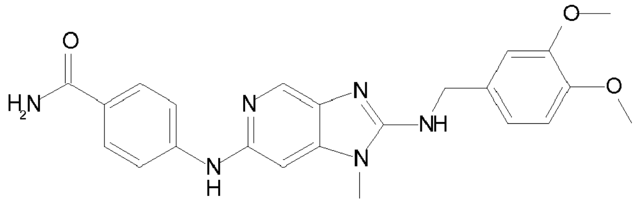

MW.: 432.19, LCMS-ESI (*m/z*) - found 433.19 $[M+H]^+$, 431.19 $[M-H]^-$

**Example 12-602 Compound 602**

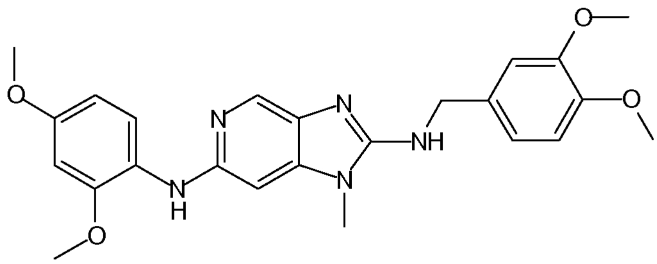

MW.: 449.21, LCMS-ESI (*m/z*) - found 450.2 $[M+H]^+$, 448.2 $[M-H]^-$

**Example 12-603 Compound 603**

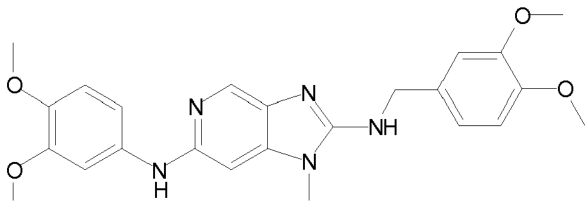

MW.: 449.21, LCMS-ESI (*m/z*) - found 450.20 $[M+H]^+$, 448.20 $[M-H]^-$

**Example 12-604 Compound 604**

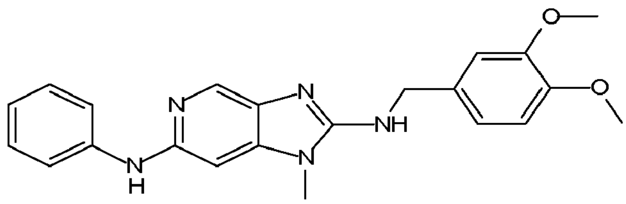

MW.: 389.19, LCMS-ESI (*m*/*z*) - found 390.18 $[M+H]^+$, 388.18 $[M-H]^-$

**Example 12-605 Compound 605**

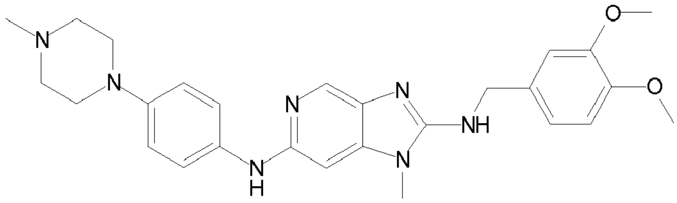

MW.: 487.27, LCMS-ESI (*m*/*z*) - found 488.27 $[M+H]^+$, 486.27 $[M-H]^-$

**Example 12-606 Compound 606**

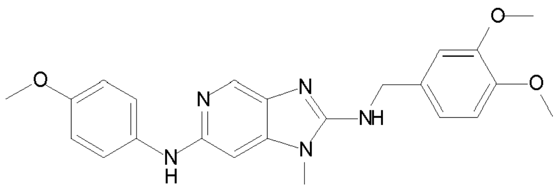

MW.: 419.20, LCMS-ESI (*m*/*z*) - found 420.19 $[M+H]^+$, 418.19 $[M-H]^-$

**Example 12-607 Compound 607**

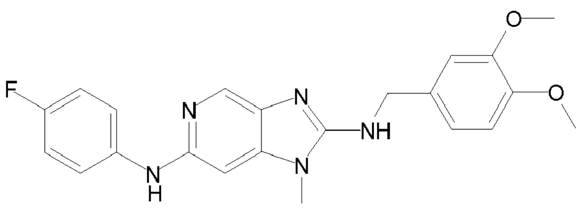

MW.: 407.18, LCMS-ESI (*m*/*z*) - found 408.17 $[M+H]^+$, 406.17 $[M-H]^-$

**Example 12-608 Compound 608**

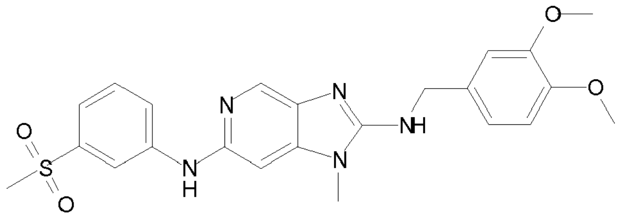

MW.: 434.20, LCMS-ESI (*m*/*z*) - found 435.19 [M+H]$^+$, 433.19 [M-H]$^-$

**Example 12-609 Compound 609**

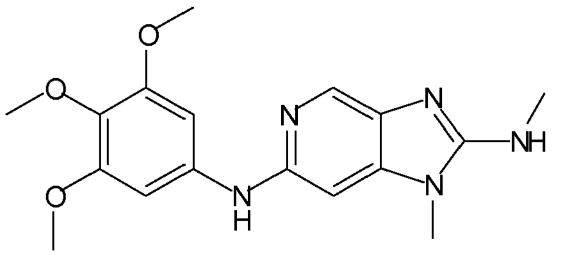

MW.: 343.16, LCMS-ESI (*m*/*z*) - found 344.16 [M+H]$^+$, 442.16 [M-H]$^-$

**Example 12-610 Compound 610**

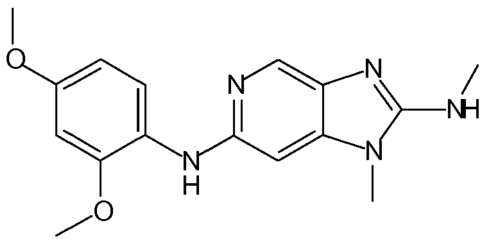

MW.: 313.15, LCMS-ESI (*m*/*z*) - found 314.15 [M+H]$^+$, 312.15 [M-H]$^-$

**Example 12-611** Compound **611**

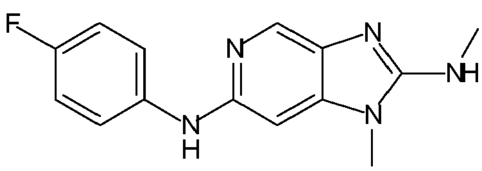

MW.: 271.12, LCMS-ESI (*m*/*z*) - found 272.12 [M+H]$^+$, 270.12 [M-H]$^-$

**Example 12-612 Compound 612**

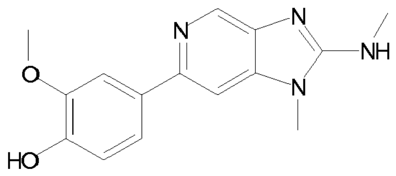

MW.: 284.13, LCMS-ESI (*m*/*z*) - found 285.12 $[M+H]^+$, 283.12 $[M-H]^-$

**Example 12-613 Compound 613**

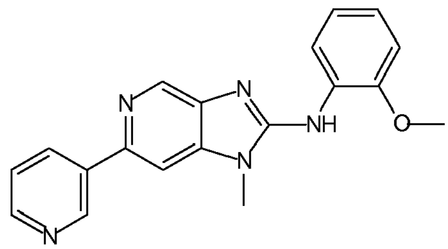

MW.: 331.14, LCMS-ESI (*m*/*z*) - found 332.14 $[M+H]^+$, 330.14 $[M-H]^-$

**Example 12-614 Compound 614**

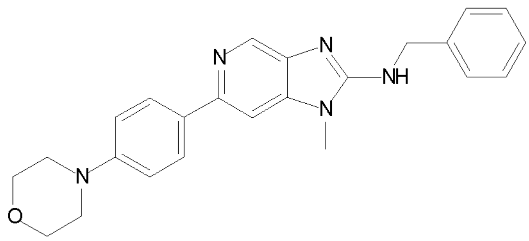

MW.: 399.21, LCMS-ESI (*m*/*z*) - found 400.2 $[M+H]^+$, 398.2 $[M-H]^-$

**Example 12-615 Compound 615**

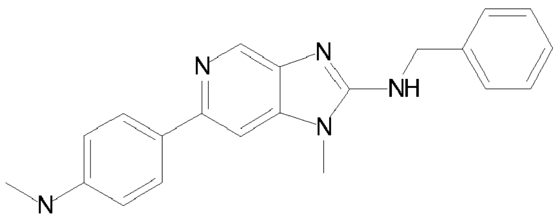

MW.: 357.20, LCMS-ESI (*m*/*z*) - found 358.19 $[M+H]^+$, 356.19 $[M-H]^-$

**Example 12-616 Compound 616**

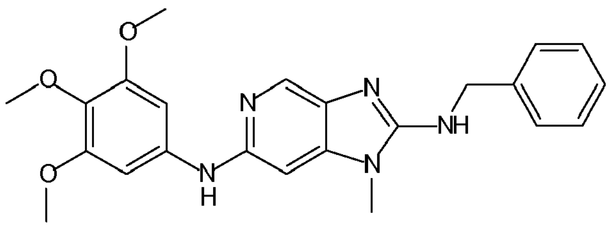

MW.: 419.20, LCMS-ESI (*m*/*z*) - found 420.19 $[M+H]^+$, 418.19 $[M-H]^-$

**Example 12-617 Compound** 617

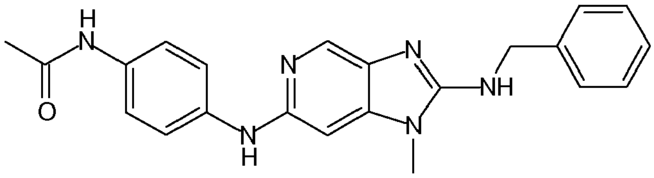

MW.: 386.19, LCMS-ESI (*m*/*z*) - found 387.18 $[M+H]^+$, 385.18 $[M-H]^-$

**Example 12-618 Compound 618**

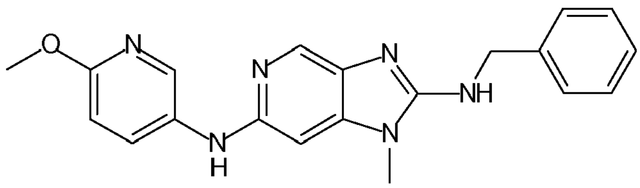

MW.: 360.17, LCMS-ESI (*m*/*z*) - found 361.17 $[M+H]^+$, 359.17 $[M-H]^-$

**Example 12-619 Compound 619**

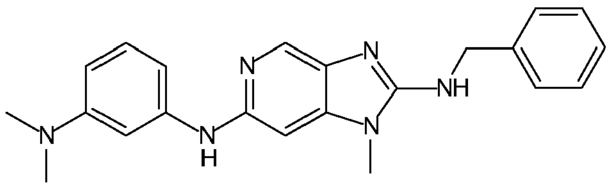

MW.: 372.21, LCMS-ESI (*m*/*z*) - found 373.20 $[M+H]^+$, 371.20 $[M-H]^-$

**Example 12-620 Compound 620**

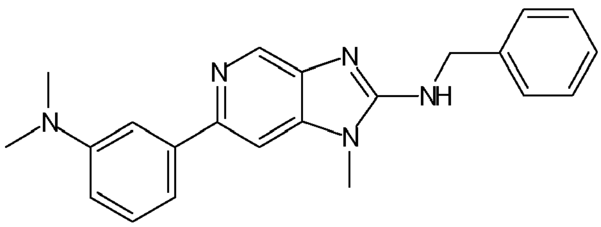

MW.: 357.20, LCMS-ESI (*m*/*z*) - found 358.19 $[M+H]^+$, 356.19 $[M-H]^-$

**Example 12-621 Compound 621**

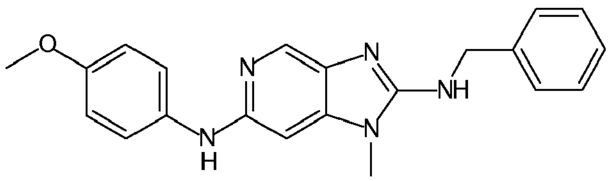

MW.: 359.17, LCMS-ESI (*m*/*z*) - found 360.17 $[M+H]^+$, 358.17 $[M-H]^-$

**Example 12-622 Compound 622**

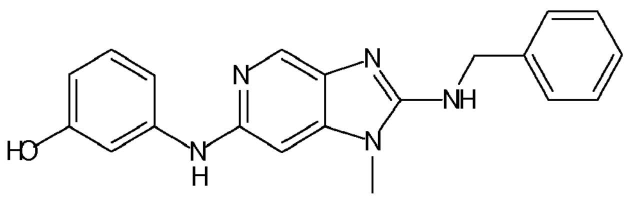

MW.: 345.16, LCMS-ESI (*m*/*z*) - found 345.15 $[M+H]^+$, 344.15 $[M-H]^-$

**Example 12-623 Compound 623**

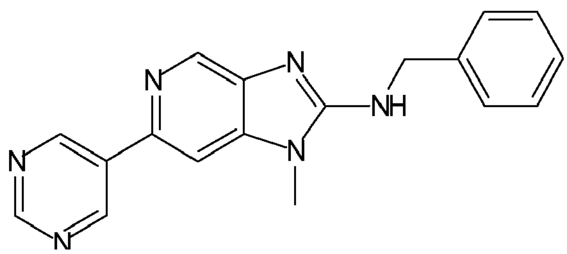

MW.: 316.14, LCMS-ESI (*m*/*z*) - found 317.14 $[M+H]^+$, 315.14 $[M-H]^-$

**Example 12-624 Compound 624**

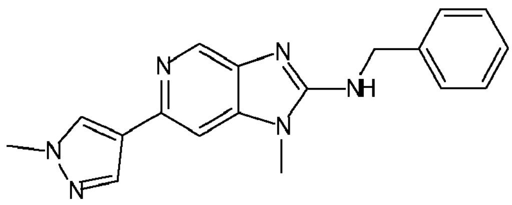

MW.: 318.16, LCMS-ESI (*m*/*z*) - found 319.15 $[M+H]^+$, 317.15 $[M-H]^-$

**Example 12-625 Compound 625**

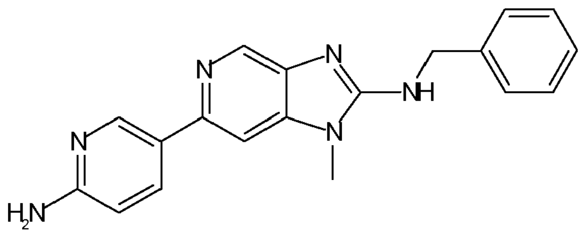

MW.: 330.16, LCMS-ESI (*m*/*z*) - found 331.15 $[M+H]^+$, 329.15 $[M-H]^-$

**Example 12-626 Compound 626**

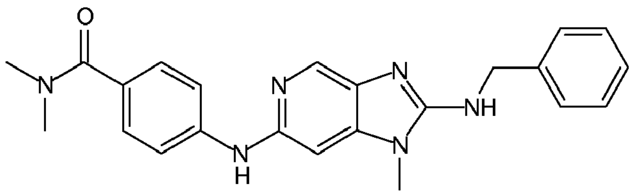

MW.: 400.20, LCMS-ESI (*m*/*z*) - found 401.20 $[M+H]^+$, 399.20 $[M-H]^-$

**Example 12-627 Compound 627**

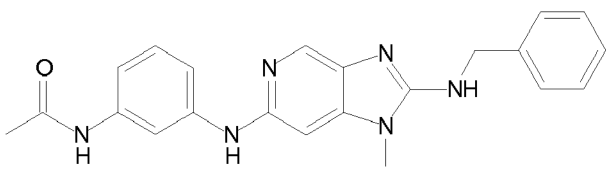

MW.: 386.19, LCMS-ESI (*m*/*z*) - found 387.18 $[M+H]^+$, 385.18 $[M-H]^-$

**Example 12-628 Compound 628**

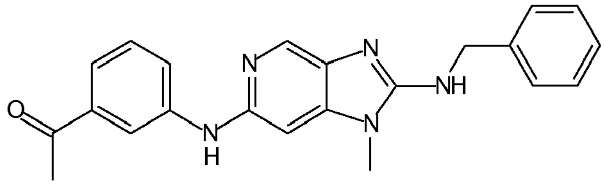

MW.: 371.17, LCMS-ESI (*m*/*z*) - found 372.17 $[M+H]^+$, 370.17 $[M-H]^-$

**Example 12-629 Compound 629**

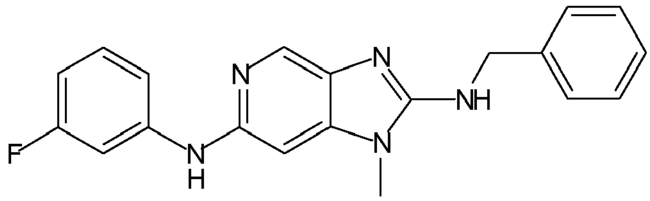

MW.: 347.15, LCMS-ESI (*m*/*z*) - found 348.15 $[M+H]^+$, 346.15 $[M-H]^-$

**Example 12-630 Compound 630**

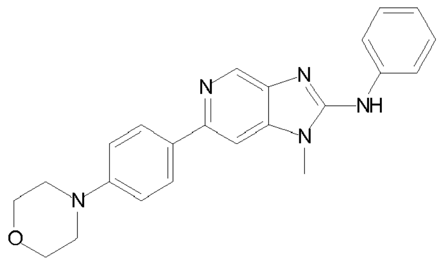

MW.: 385.19, LCMS-ESI (*m*/*z*) - found 386.19 $[M+H]^+$, 384.19 $[M-H]^-$

**Example 12-631 Compound 631**

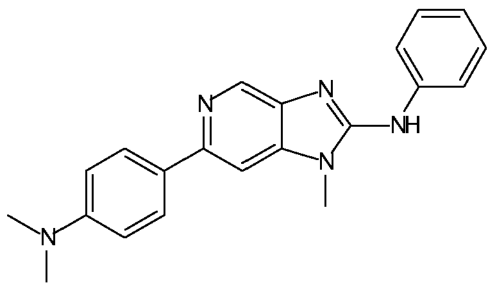

MW.: 343.18, LCMS-ESI (*m*/*z*) - found 344.18 $[M+H]^+$, 342.18 $[M-H]^-$

**Example 12-632 Compound 632**

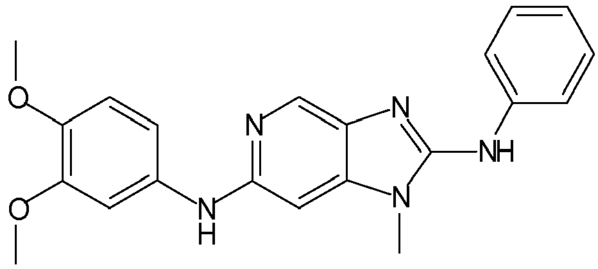

MW.: 375.17, LCMS-ESI (*m*/*z*) - found 376.17 $[M+H]^+$, 374.17 $[M-H]^-$

**Example 12-633 Compound 633**

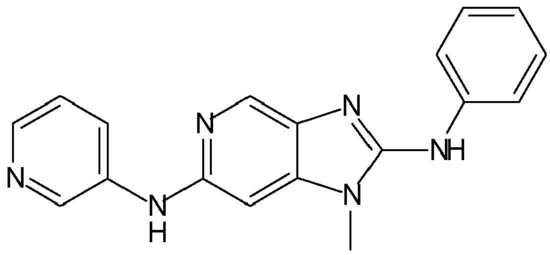

MW.: 316.14, LCMS-ESI (*m*/*z*) - found 317.14 $[M+H]^+$, 315.14 $[M-H]^-$

**Example 12-634 Compound 634**

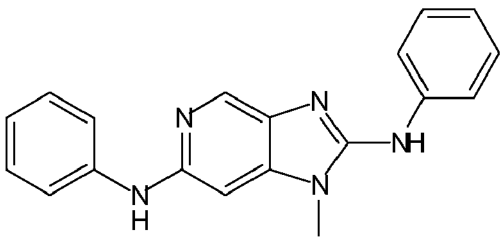

MW.: 315.15, LCMS-ESI (*m*/*z*) - found 316.14 $[M+H]^+$, 314.14 $[M-H]^-$

**Example 12-635 Compound 635**

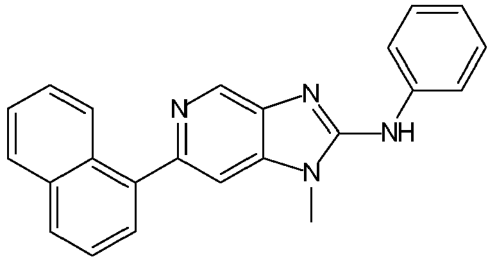

MW.: 350.15, LCMS-ESI (*m*/*z*) - found 351.15 $[M+H]^+$, 349.15 $[M-H]^-$

**Example 12-636 Compound 636**

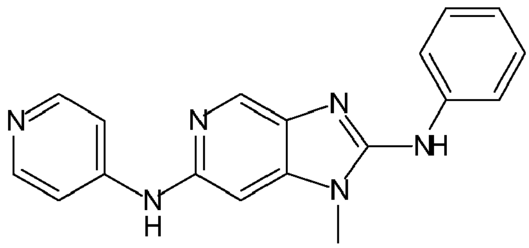

MW.: 316.14, LCMS-ESI (*m*/*z*) - found 317.14 $[M+H]^+$, 315.14 $[M-H]^-$

**Example 12-637 Compound 637**

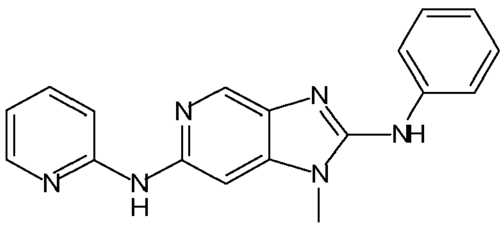

MW.: 316.14, LCMS-ESI (*m*/*z*) - found 317.14 $[M+H]^+$, 315.14 $[M-H]^-$

**Example 12-638 Compound 638**

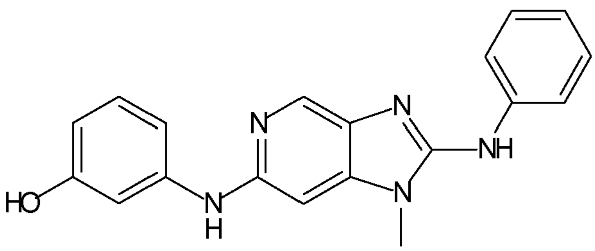

MW.: 331.14, LCMS-ESI (*m*/*z*) - found 332.14 $[M+H]^+$, 330.14 $[M-H]^-$

**Example 12-639 Compound 639**

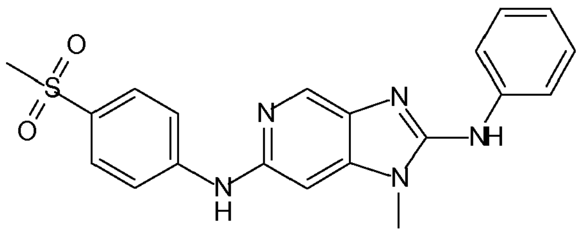

MW.: 393.13, LCMS-ESI (*m*/*z*) - found 394.13 $[M+H]^+$, 393.13 $[M-H]^-$

**Example 12-640 Compound 640**

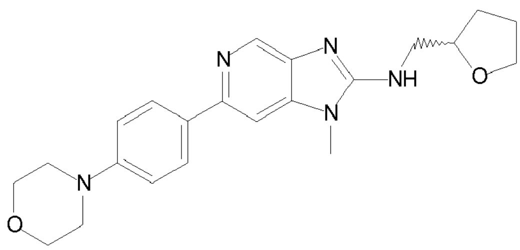

MW.: 393.22, LCMS-ESI (*m*/*z*) - found 394.22 $[M+H]^+$, 392.21 $[M-H]^-$

**Example 12-641 Compound 641**

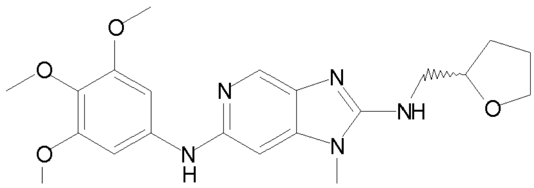

MW.: 413.21, LCMS-ESI (*m*/*z*) - found 414.20 $[M+H]^+$, 412.20 $[M-H]^-$

**Example 12-642 Compound 642**

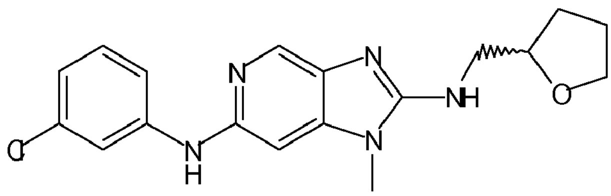

MW.: 357.14, LCMS-ESI (*m*/*z*) - found 358.14 $[M+H]^+$, 356.13 $[M-H]^-$

**Example 12-643 Compound 643**

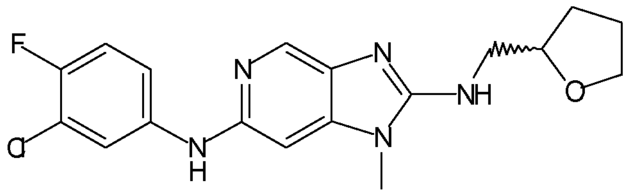

MW.: 375.13, LCMS-ESI (*m*/*z*) - found 376.12 $[M+H]^+$, 374.12 $[M-H]^-$

**Example 12-644 Compound 644**

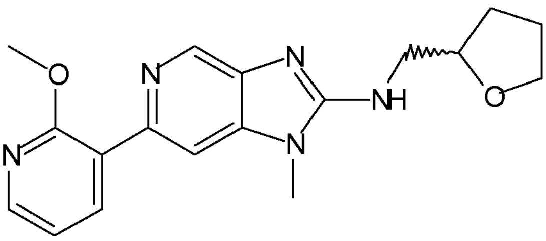

MW.: 339.17, LCMS-ESI (*m*/*z*) - found 340.17 $[M+H]^+$, 339.17 $[M-H]^-$

**Example 12-645 Compound 645**

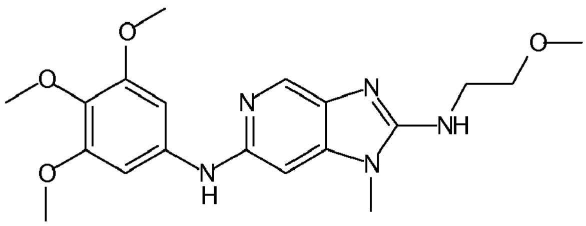

MW.: 387.19, LCMS-ESI (*m*/*z*) - found 388.19 $[M+H]^+$, 386.19 $[M-H]^-$

**Example 12-646 Compound 646**

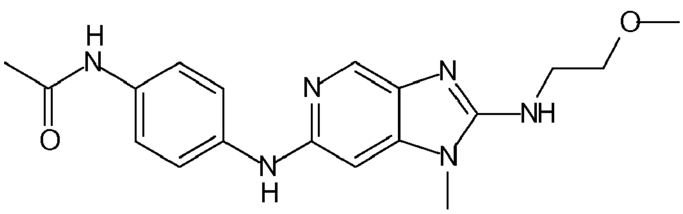

MW.: 354.18, LCMS-ESI (*m*/*z*) - found 355.18 $[M+H]^+$, 353.18 $[M-H]^-$

**Example 12-647 Compound 647**

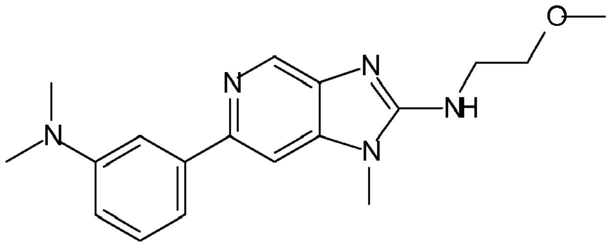

MW.: 325.19, LCMS-ESI (*m/z*) - found 326.19 $[M+H]^+$, 324.19 $[M-H]^-$

**Example 12-648 Compound 648**

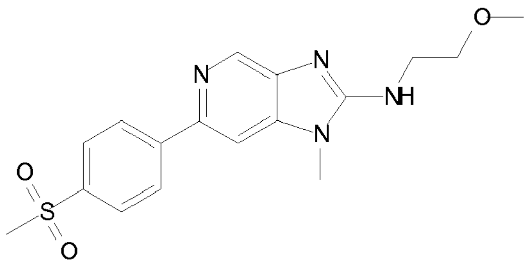

MW.: 360.13, LCMS-ESI (*m/z*) - found 361.12 $[M+H]^+$, 359.12 $[M-H]^-$

**Example 12-649 Compound 649**

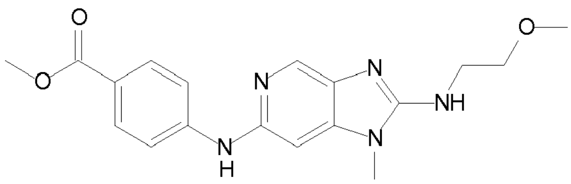

MW.: 355.16, LCMS-ESI (*m/z*) - found 356.16 $[M+H]^+$, 354.16 $[M-H]^-$

**Example 12-650 Compound 650**

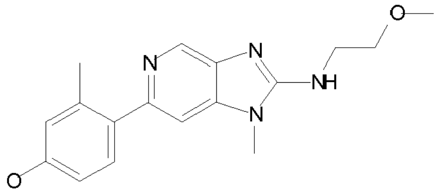

MW.: 326.17, LCMS-ESI (*m/z*) - found 327.17 $[M+H]^+$, 325.17 $[M-H]^-$

**Example 12-651 Compound 651**

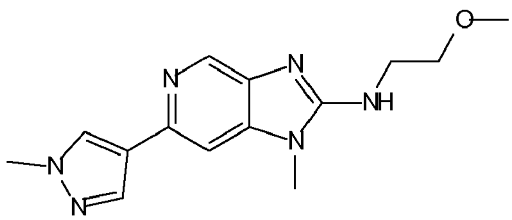

MW.: 286.15, LCMS-ESI ($m/z$) - found 287.15 $[M+H]^+$, 285.15 $[M-H]^-$

**Example 12-652 Compound 652**

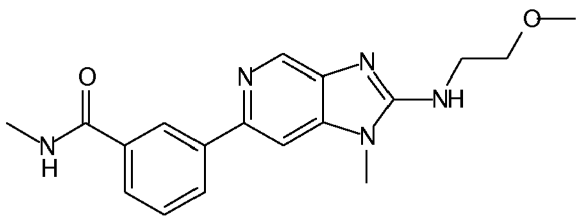

MW.: 339.17, LCMS-ESI ($m/z$) - found 340.17 $[M+H]^+$, 338.17 $[M-H]^-$

**Example 12-653 Compound 653**

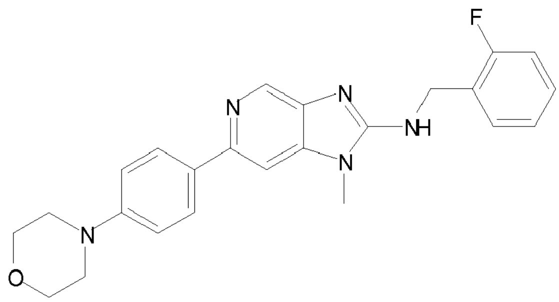

MW.: 417.20, LCMS-ESI ($m/z$) - found 418.19 $[M+H]^+$, 416.19 $[M-H]^-$

**Example 12-654 Compound 654**

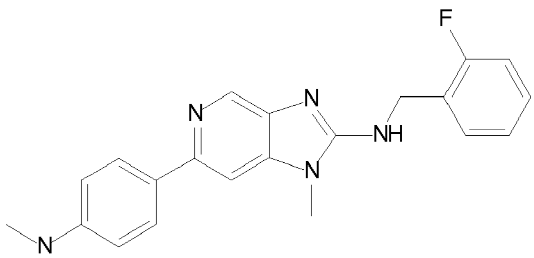

MW.: 375.19, LCMS-ESI ($m/z$) - found 376.18 $[M+H]^+$, 374.18 $[M-H]^-$

**Example 12-655 Compound 655**

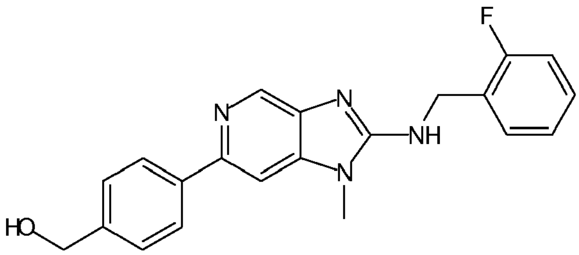

MW.: 362.15, LCMS-ESI ($m/z$) - found 363.15 $[M+H]^+$, 362.15 $[M-H]^-$

**Example 12-656 Compound 656**

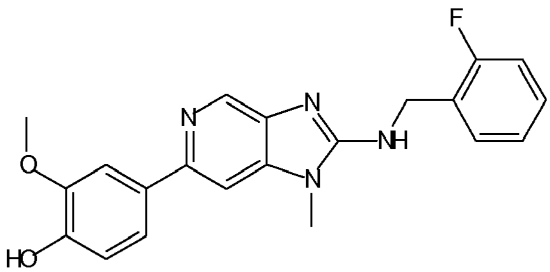

MW.: 378.15, LCMS-ESI ($m/z$) - found 379.14 $[M+H]^+$, 377.14 $[M-H]^-$

**Example 12-657 Compound 657**

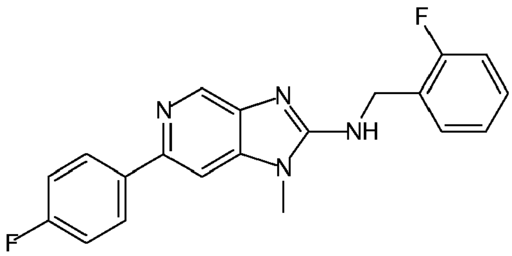

MW.: 350.13, LCMS-ESI ($m/z$) - found 351.13 $[M+H]^+$, 349.13 $[M-H]^-$

**Example 12-658 Compound 658**

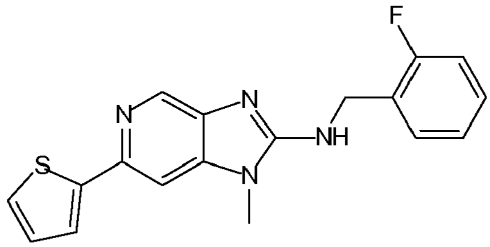

MW.: 338.10, LCMS-ESI (*m*/*z*) - found 339.10 [M+H]$^+$, 337.10 [M-H]$^-$

**Example 12-659 Compound 659**

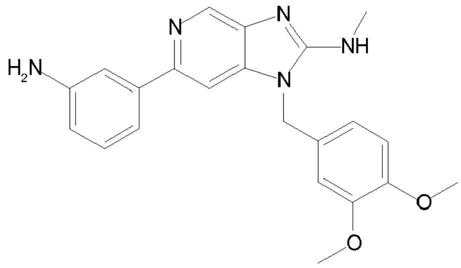

MW.: 389.19, LCMS-ESI (*m*/*z*) - found 390.18 [M+H]$^+$, 388.18 [M-H]$^-$

**Example 12-660 Compound 660**

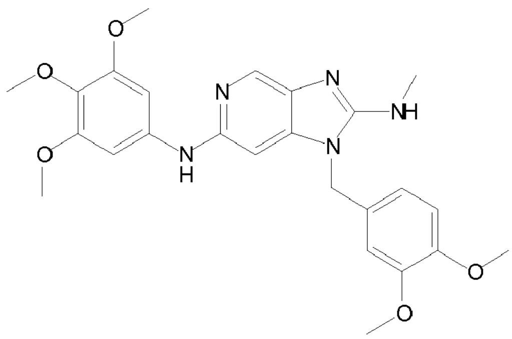

MW.: 479.22, LCMS-ESI (*m*/*z*) - found 480.21 [M+H]$^+$, 478.21 [M-H]$^-$

**Example 12-661 Compound 661**

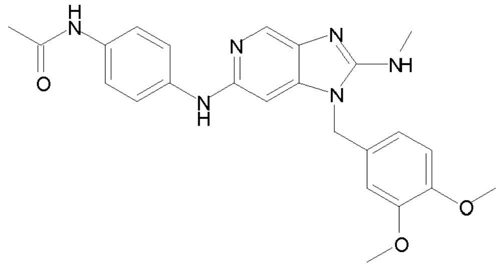

MW.: 446.21, LCMS-ESI (*m*/*z*) - found 447.20 $[M+H]^+$, 445.20 $[M-H]^-$

**Example 12-662 Compound 662**

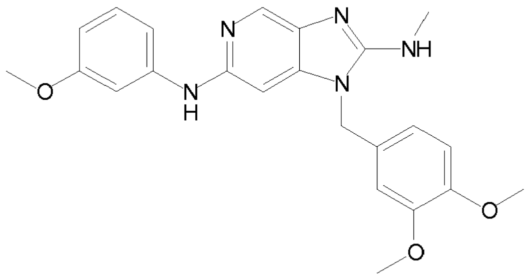

MW.: 419.20, LCMS-ESI (*m*/*z*) - found 420.19 $[M+H]^+$, 418.19 $[M-H]^-$

**Example 12-663 Compound 663**

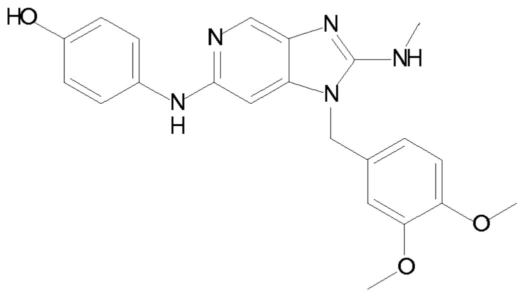

MW.: 405.18, LCMS-ESI (*m*/*z*) - found 406.18 $[M+H]^+$, 404.18 $[M-H]^-$

**Example 12-664 Compound 664**

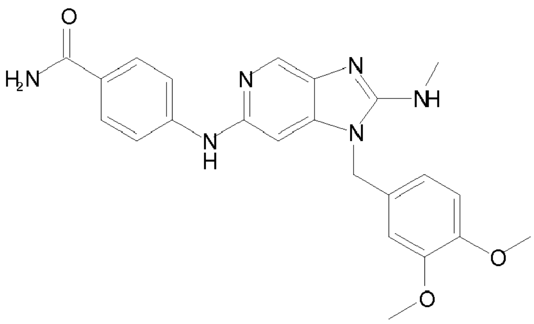

MW.: 432.19, LCMS-ESI (*m*/*z*) - found 433.19 $[M+H]^+$, 431.19 $[M-H]^-$

**Example 12-665 Compound 665**

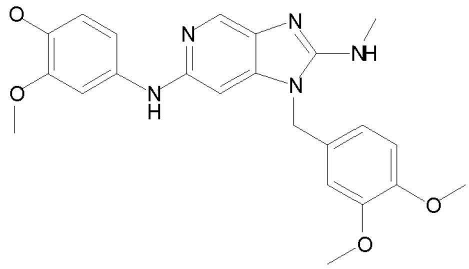

MW.: 449.21, LCMS-ESI (*m*/*z*) - found 450.20 $[M+H]^+$, 448.20 $[M-H]^-$

**Example 12-666 Compound 666**

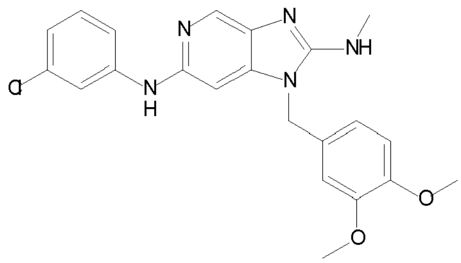

MW.: 423.15, LCMS-ESI (*m*/*z*) - found 424.14 $[M+H]^+$, 422.14 $[M-H]^-$

**Example 12-667 Compound 667**

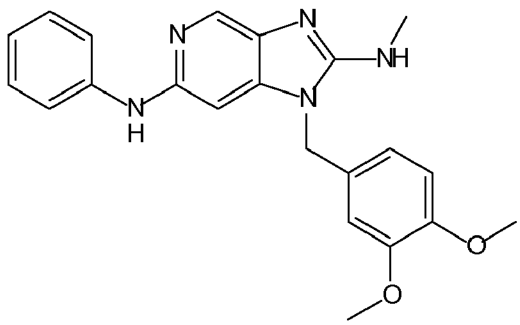

MW.: 389.19, LCMS-ESI (*m*/*z*) - found 390.18 $[M+H]^+$, 388.18 $[M-H]^-$

**Example 12-668 Compound 668**

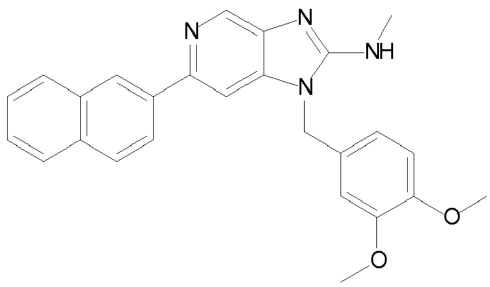

MW.: 424.19, LCMS-ESI (*m/z*) - found 425.19 $[M+H]^+$, 423.19 $[M-H]^-$

**Example 12-669 Compound 669**

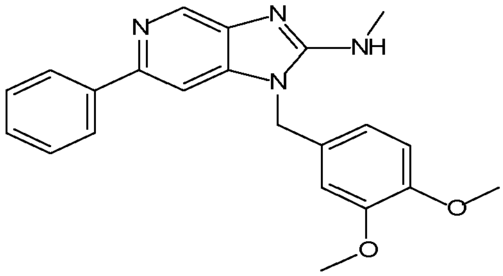

MW.: 374.17, LCMS-ESI (*m/z*) - found 375.17 $[M+H]^+$, 373.17 $[M-H]^-$

**Example 12-670 Compound 670**

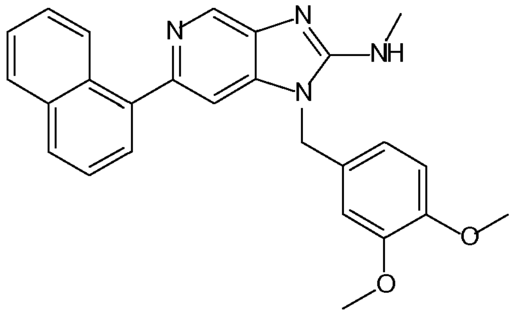

MW.: 424.19 LCMS-ESI (*m/z*) - found 425.19 $[M+H]^+$, 423.19 $[M-H]^-$

**Example 12-671 Compound 671**

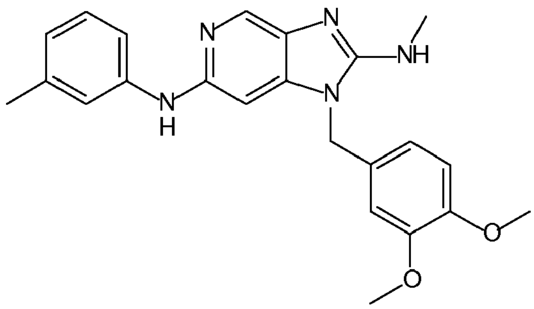

MW.: 403.20, LCMS-ESI (*m/z*) - found 404.20 $[M+H]^+$, 402.20 $[M-H]^-$

**Example 12-672 Compound 672**

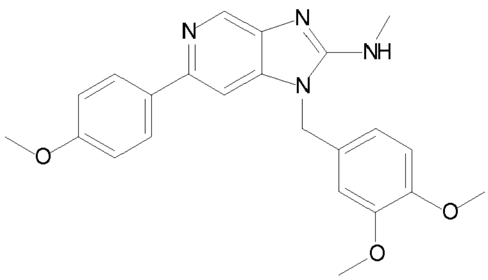

MW.: 404.18, LCMS-ESI (*m/z*) - found 405.18 $[M+H]^+$, 403.18 $[M-H]^-$

**Example 12-673 Compound 673**

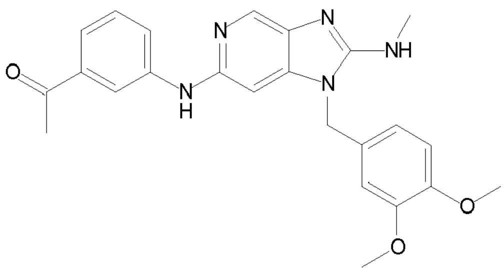

MW.: 431.20, LCMS-ESI (*m/z*) - found 432.30 $[M+H]^+$, 430.20 $[M-H]^-$

**Example 12-674 Compound 674**

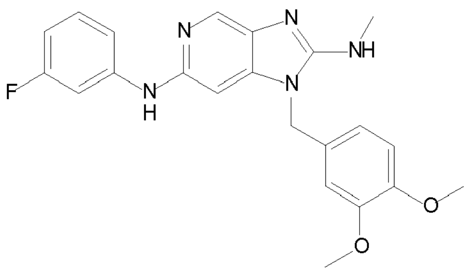

MW.: 407.18, LCMS-ESI (*m*/*z*) - found 408.17 [M+H]$^+$, 406.17 [M-H]$^-$

**Example 12-675 Compound 675**

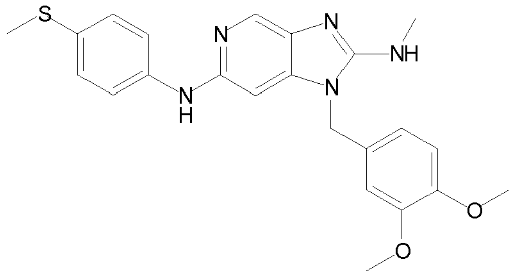

MW.: 435.17, LCMS-ESI (*m*/*z*) - found 436.17 [M+H]$^+$, 434.17 [M-H]$^-$

**Example 12-676 Compound 676**

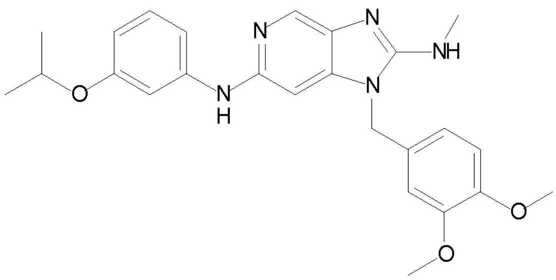

MW.: 447.23, LCMS-ESI (*m*/*z*) - found 448.22 [M+H]$^+$, 446.22 [M-H]$^-$

**Example 12-677 Compound 677**

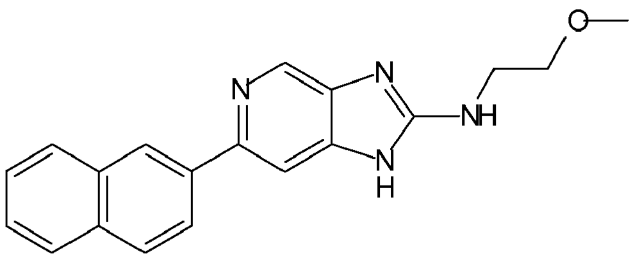

MW.: 318.15, LCMS-ESI (*m*/*z*) - found 319.14 [M+H]$^+$, 317.14 [M-H]$^-$

**Example 12-678 Compound 678**

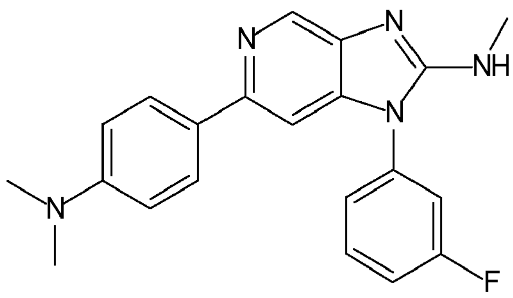

MW.: 361.17, LCMS-ESI (*m*/*z*) - found 362.17 $[M+H]^+$, 360.17 $[M-H]^-$

**Example 12-679 Compound 679**

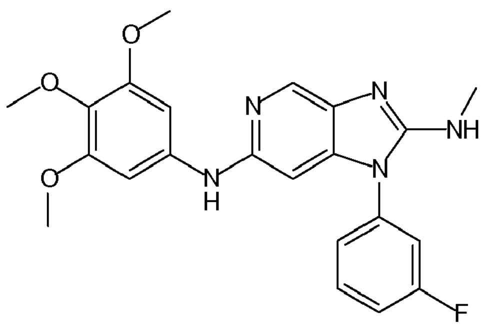

MW.: 423.17, LCMS-ESI (*m*/*z*) - found 424.17 $[M+H]^+$, 422.17 $[M-H]^-$

**Example 12-680 Compound 680**

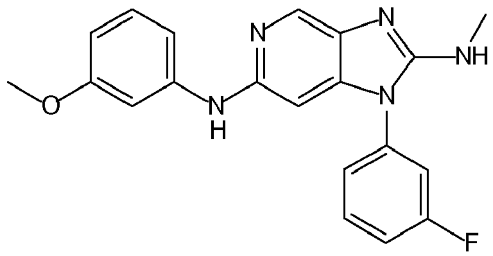

MW.: 363.15, LCMS-ESI (*m*/*z*) - found 364.15 $[M+H]^+$, 362.15 $[M-H]^-$

**Example 12-681 Compound 681**

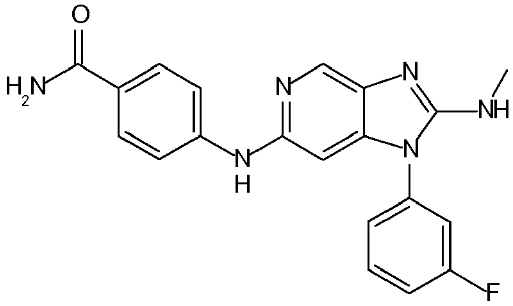

MW.: 376.14, LCMS-ESI (*m*/*z*) - found 377.14 $[M+H]^+$, 375.14 $[M-H]^-$

**Example 12-682 Compound 682**

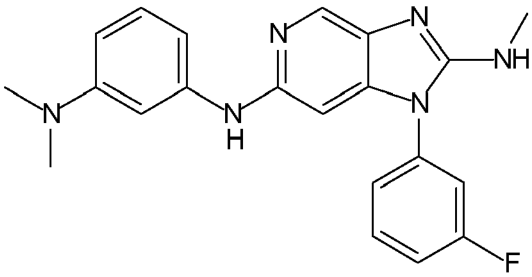

MW.: 368.14, LCMS-ESI (*m*/*z*) - found 369.14 $[M+H]^+$, 367.14 $[M-H]^-$

**Example 12-683 Compound 683**

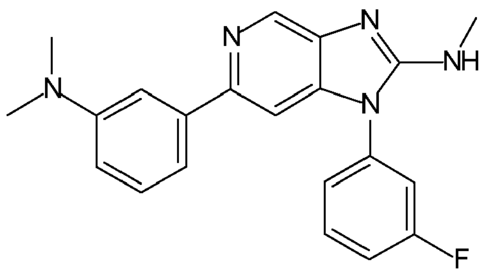

MW.: 361.17, LCMS-ESI (*m*/*z*) - found 362.17 $[M+H]^+$, 360.17 $[M-H]^-$

**Example 12-684 Compound 684**

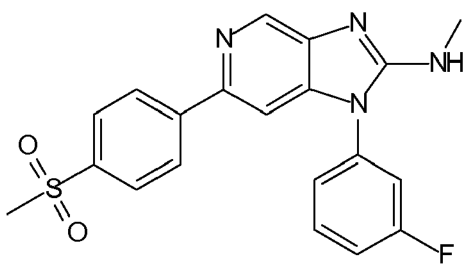

MW.: 396.11, LCMS-ESI (*m*/*z*) - found 397.11 $[M+H]^+$, 395.11 $[M-H]^-$

**Example 12-685 Compound 685**

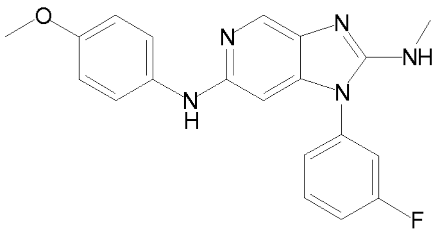

MW.: 363.15, LCMS-ESI (*m*/*z*) - found 364.15 $[M+H]^+$, 362.15 $[M-H]^-$

**Example 12-686 Compound 686**

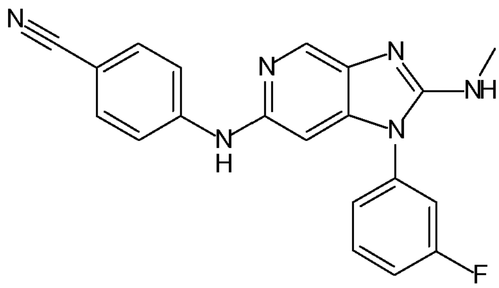

MW.: 358.13, LCMS-ESI (*m*/*z*) - found 359.13 $[M+H]^+$, 357.13 $[M-H]^-$

**Example 12-687 Compound 687**

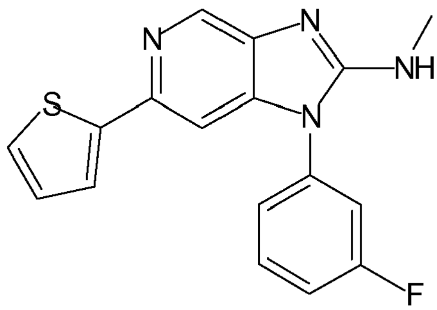

MW.: 324.08, LCMS-ESI (*m*/*z*) - found 325.08 $[M+H]^+$, 323.08 $[M-H]^-$

**Example 12-688 Compound 688**

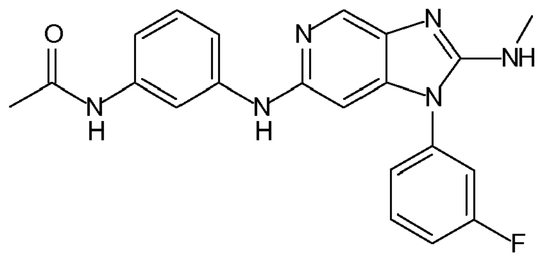

MW.: 390.16, LCMS-ESI (*m*/*z*) - found 391.16 $[M+H]^+$, 389.16 $[M-H]^-$

**Example 12-689 Compound 689**

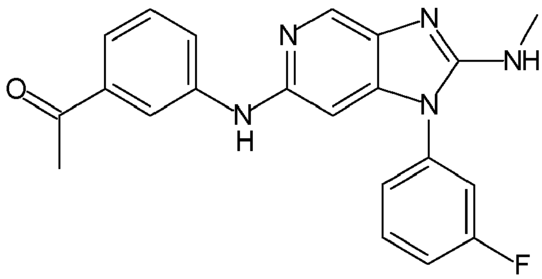

MW.: 375.15, LCMS-ESI (*m*/*z*) - found 376.15 $[M+H]^+$, 374.15 $[M-H]^-$

**Example 12-690 Compound 690**

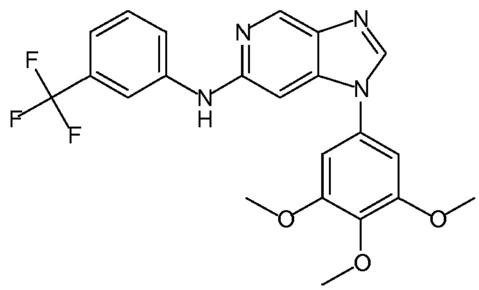

MW.: 444.14, LCMS-ESI (*m*/*z*) - found 445.14 $[M+H]^+$, 443.14 $[M-H]^-$

**Example 12-691 Compound 691**

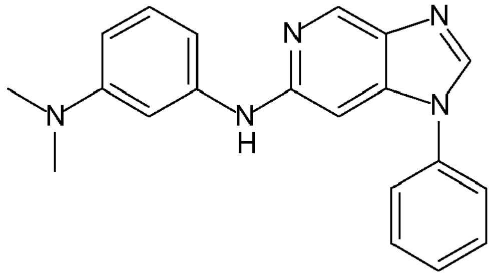

MW.: 329.16, LCMS-ESI ($m/z$) - found 330.16 $[M+H]^+$, 328.16 $[M-H]^-$

**Example 12-692 Compound 692**

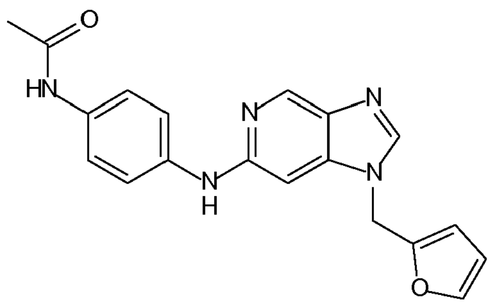

MW.: 347.14, LCMS-ESI ($m/z$) - found 348.13 $[M+H]^+$, 346.13 $[M-H]^-$

**Example 12-693 Compound 693**

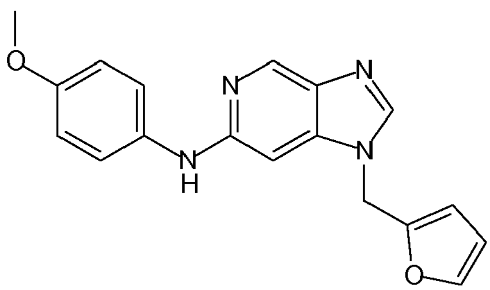

MW.: 320.13, LCMS-ESI ($m/z$) - found 321.12 $[M+H]^+$, 319.12 $[M-H]^-$

**Example 12-694 Compound 694**

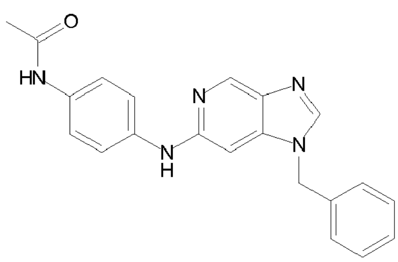

MW.: 357.16, LCMS-ESI ($m/z$) - found 358.16 $[M+H]^+$, 356.16 $[M-H]^-$

**Example 12-695 Compound 695**

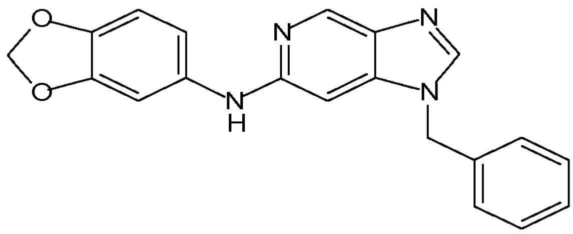

MW.: 344.13, LCMS-ESI (*m*/*z*) - found 345.12 $[M+H]^+$, 343.12 $[M-H]^-$

**Example 12-696 Compound 696**

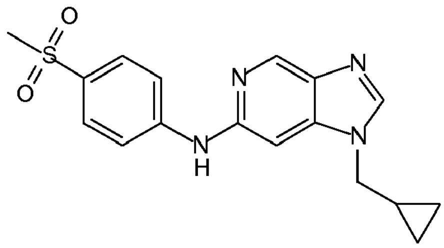

MW.: 342.12, LCMS-ESI (*m*/*z*) - found 343.11 $[M+H]^+$, 341.11 $[M-H]^-$

**Example 12-697 Compound 697**

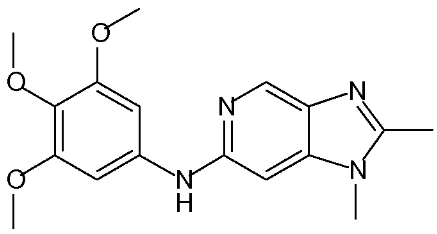

MW.: 328.15, LCMS-ESI (*m*/*z*) - found 329.15 $[M+H]^+$, 327.15 $[M-H]^-$

**Example 12-698 Compound 698**

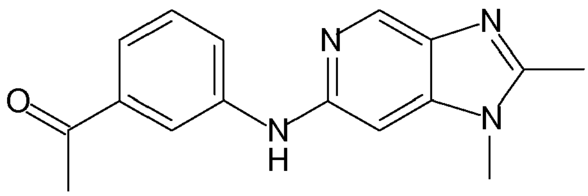

MW.: 280.13, LCMS-ESI (*m*/*z*) - found 281.13 $[M+H]^+$, 279.13 $[M-H]^-$

**Example 12-699 Compound 699**

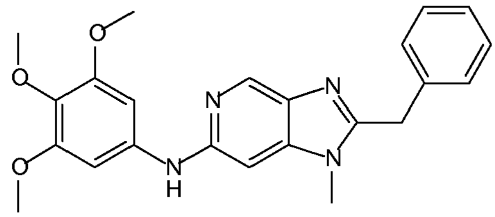

MW.: 404.18, LCMS-ESI (*m/z*) - found 405.18 [M+H]$^+$, 403.18 [M-H]$^-$

**Example 12-700 Compound 700**

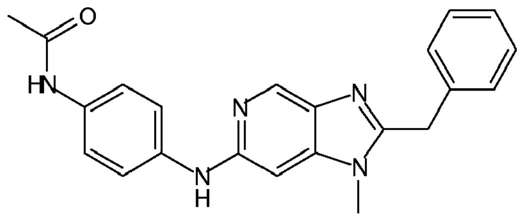

MW.: 371.17, LCMS-ESI (m/z) - found 372.17 [M+H]$^+$, 370.17 [M-H]$^-$

**Example 12-701 Compound 701**

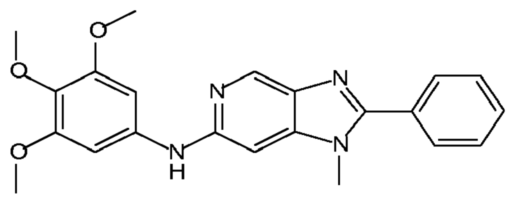

MW.: 390.17, LCMS-ESI (*m/z*) - found 391.16 [M+H]$^+$, 389.16 [M-H]$^-$

**Example 12-702 Compound 702**

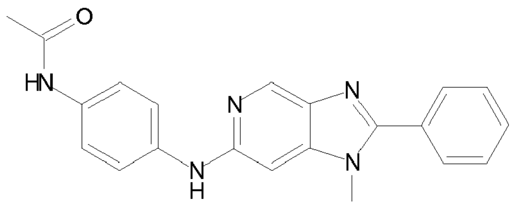

MW.: 357.16, LCMS-ESI (*m/z*) - found 358.15 [M+H]$^+$, 356.15 [M-H]$^-$

**Example 12-703 Compound 703**

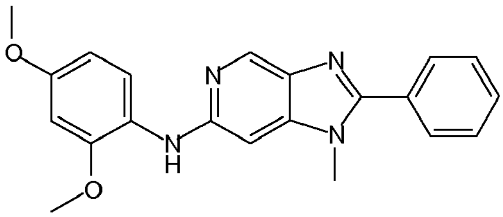

MW.: 360.16, LCMS-ESI (*m/z*) - found 361.15 [M+H]$^+$, 359.15 [M-H]$^-$

**Example 12-704 Compound 704**

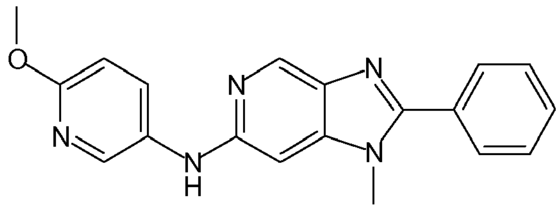

MW.: 331.14, LCMS-ESI (*m/z*) - found 332.14 [M+H]$^+$, 330.14 [M-H]$^-$

**Example 8-705 Compound 705**

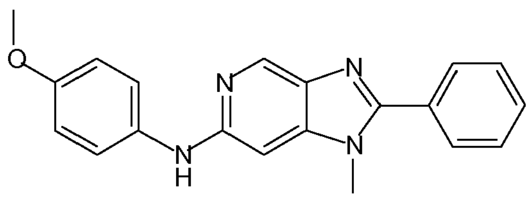

MW.: 330.15, LCMS-ESI (*m/z*) - found 331.14 [M+H]$^+$, 329.14 [M-H]$^-$

**Example 12-706 Compound 706**

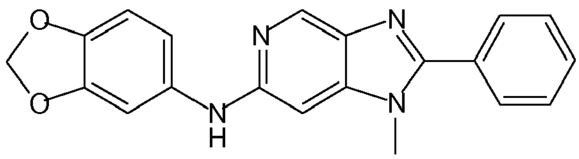

MW.: 344.13, LCMS-ESI (*m/z*) - found 345.12 [M+H]$^+$, 434.12 [M-H]$^-$

**Example 12-707 Compound 707**

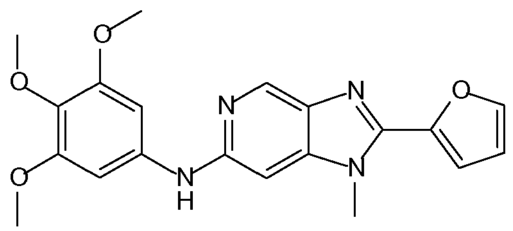

MW.: 380.15, LCMS-ESI (*m*/*z*) - found 381.14 $[M+H]^+$, 379.14 $[M-H]^-$

**Example 12-708 Compound 708**

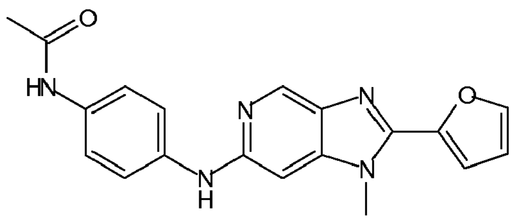

MW.: 347.14, LCMS-ESI (*m*/*z*) - found 348.13 $[M+H]^+$, 346.13 $[M-H]^-$

**Example 12-709 Compound 709**

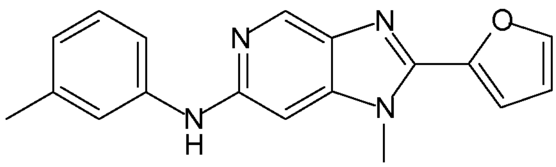

MW.: 304.13, LCMS-ESI (*m*/*z*) - found 305.13 $[M+H]^+$, 303.13 $[M-H]^-$

**Example 12-710 Compound 710**

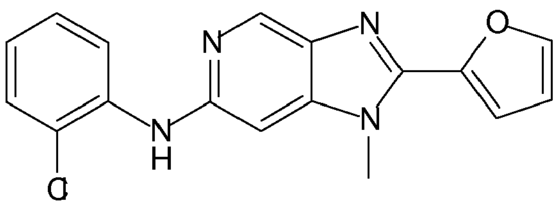

MW.: 324.08, LCMS-ESI (m/z) - found 325.07 $[M+H]^+$, 323.07 $[M-H]^-$

**Example 12-711 Compound 711**

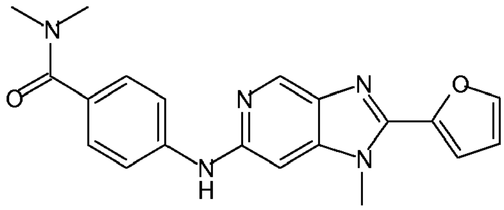

MW.: 361.15, LCMS-ESI (*m/z*) - found 362.15 $[M+H]^+$, 360.15 $[M-H]^-$

**Example 12-712 Compound 712**

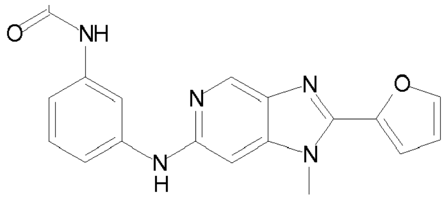

MW.: 347.14, LCMS-ESI (*m/z*) - found 348.13 $[M+H]^+$, 346.13 $[M-H]^-$

**Example 12-713 Compound 713**

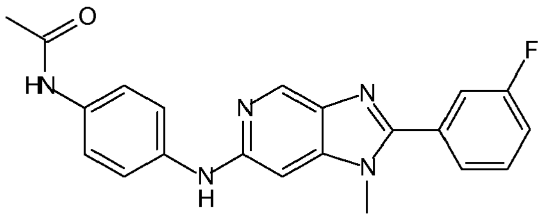

MW.: 423.17, LCMS-ESI (*m/z*) - found 424.17 $[M+H]^+$, 422.17 $[M-H]^-$

**Example 12-714 Compound 714**

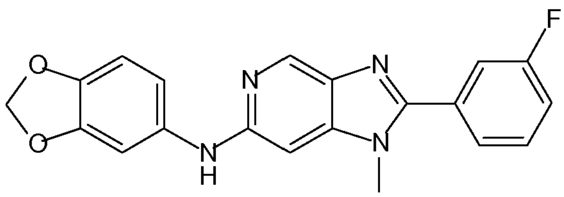

MW.: 362.12, LCMS-ESI (*m/z*) - found 363.11 $[M+H]^+$, 361.11 $[M-H]^-$

**Example 12-715 Compound 715**

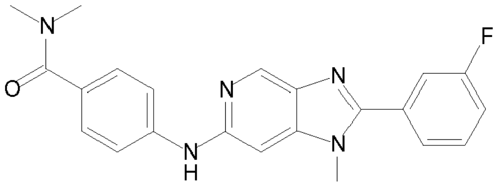

MW.: 389.17, LCMS-ESI (*m/z*) - found 390.16 $[M+H]^+$, 388.16 $[M-H]^-$

**Example 12-716 Compound 716**

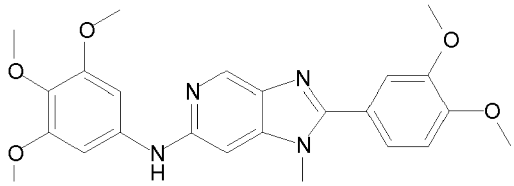

MW.: 450.19, LCMS-ESI (*m/z*) - found 451.19 $[M+H]^+$, 449.19 $[M-H]^-$

**Example 12-717 Compound 717**

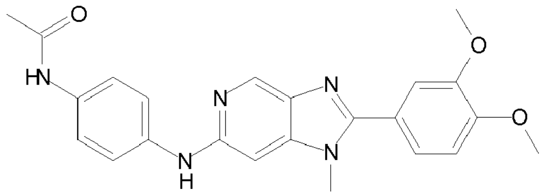

MW.: 417.18, LCMS-ESI (*m/z*) - found 418.18 $[M+H]^+$, 416.18 $[M-H]^-$

**Example 12-718 Compound 718**

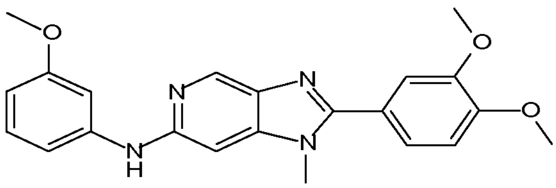

MW.: 390.17, LCMS-ESI (*m/z*) - found 391.16 $[M+H]^+$, 389.16 $[M-H]^-$

**Example 12-719 Compound 719**

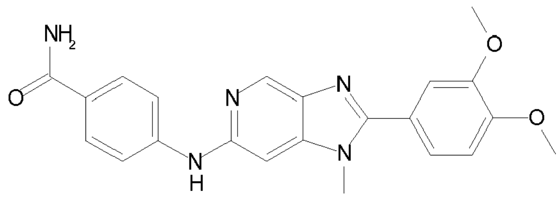

MW.: 403.16, LCMS-ESI (*m*/*z*) - found 404.16 $[M+H]^+$, 402.16 $[M-H]^-$

**Example 12-720 Compound 720**

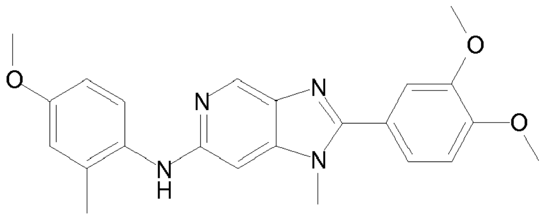

MW.: 404.18, LCMS-ESI (m/z) - found 405.18 $[M+H]^+$, 404.18 $[M-H]^-$

**Example 12-721 Compound 721**

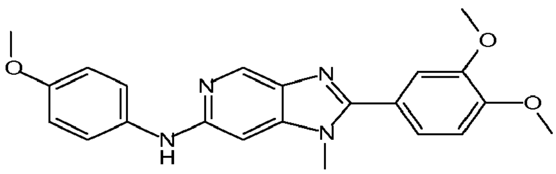

MW.: 390.17, LCMS-ESI (*m*/*z*) - found 391.16 $[M+H]^+$, 389.16 $[M-H]^-$

**Example 12-722 Compound 722**

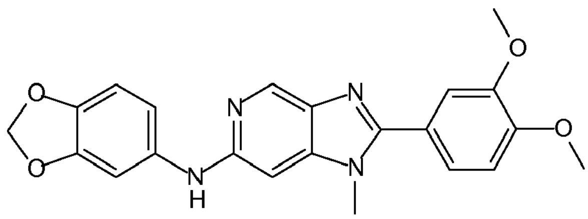

MW.: 404.15, LCMS-ESI (*m*/*z*) - found 405.14 $[M+H]^+$, 403.14 $[M-H]^-$

**Example 12-723 Compound 723**

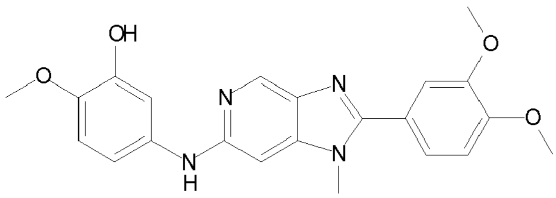

MW.: 406.16, LCMS-ESI (*m*/*z*) - found 407.16 $[M+H]^+$, 405.16 $[M-H]^-$

**Example 12-724 Compound 724**

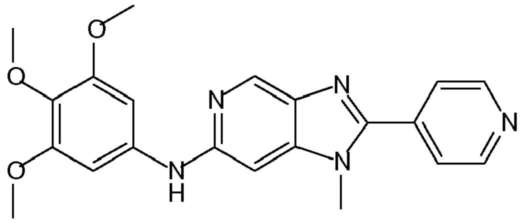

MW.: 391.16, LCMS-ESI (*m*/*z*) - found 392.16 $[M+H]^+$, 390.16 $[M-H]^-$

**Example 12-725 Compound 725**

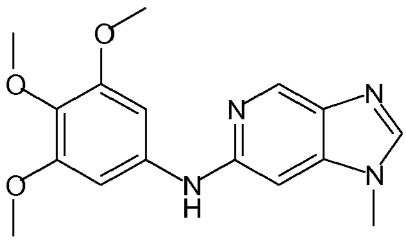

MW.: 314.14, LCMS-ESI (*m*/*z*) - found 315.13 $[M+H]^+$, 313.13 $[M-H]^-$

**Example 12-726 Compound 726**

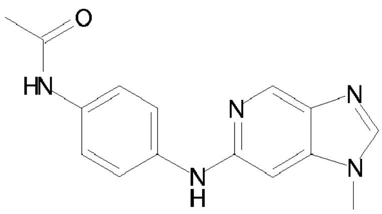

MW.: 281.13, LCMS-ESI (*m*/*z*) - found 282.12 $[M+H]^+$, 280.12 $[M-H]^-$

**Example 12-727 Compound 727**

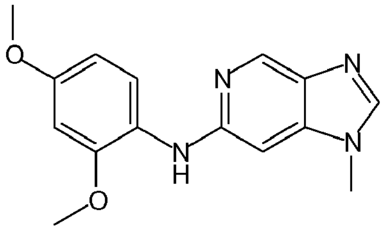

MW.: 284.13, LCMS-ESI (*m*/*z*) - found 285.12 [M+H]$^+$, 283.12 [M-H]$^-$

**Example 12-728 Compound 728**

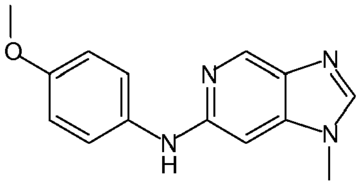

MW.: 254.12, LCMS-ESI (*m*/*z*) - found 255.11 [M+H]$^+$, 253.11 [M-H]$^-$

**Example 12-729 Compound 729**

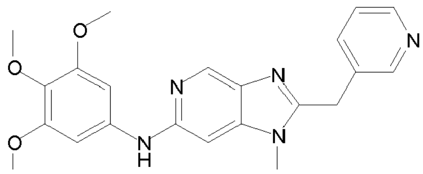

MW.: 405.18, LCMS-ESI (*m*/*z*) - found 406.18 [M+H]$^+$, 404.18 [M-H]$^-$

**Example 8-730 Compound 730**

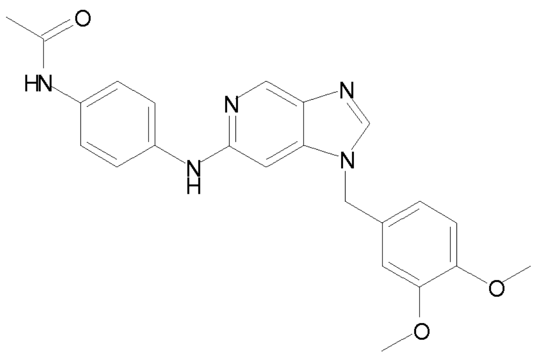

MW.: 417.18, LCMS-ESI (*m*/*z*) - found 418.18 [M+H]$^+$, 416.18 [M-H]$^-$

**Example 12-731 Compound 731**

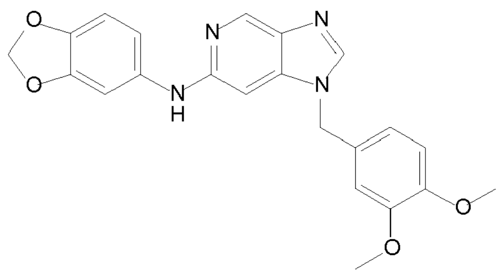

MW.: 404.15, LCMS-ESI ($m/z$) - found 405.14 $[M+H]^+$, 403.14 $[M-H]^-$

**Example 12-732 Compound 732**

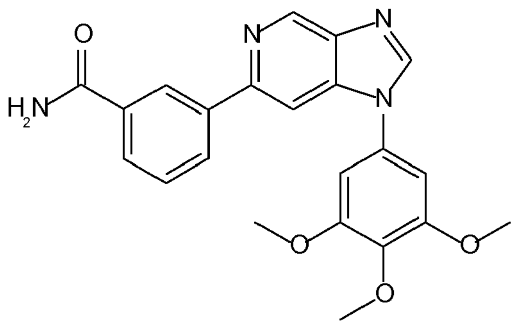

MW.: 404.15, LCMS-ESI ($m/z$) - found 405.14 $[M+H]^+$, 403.14 $[M-H]^-$

**Example 12-733 Compound 733**

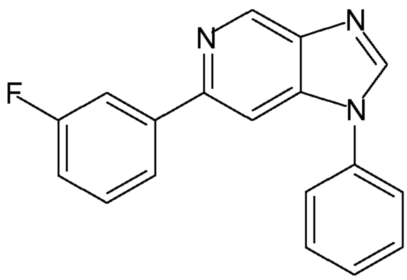

MW.: 289.10, LCMS-ESI ($m/z$) - found 290.10 $[M+H]^+$, 288.10 $[M-H]^-$

**Example 12-734 Compound 734**

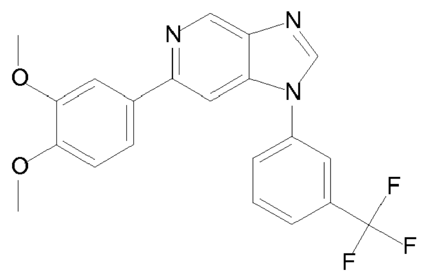

MW.: 399.12, LCMS-ESI (*m/z*) - found 400.11 $[M+H]^+$, 398.11 $[M-H]^-$

**Example 12-735 Compound 735**

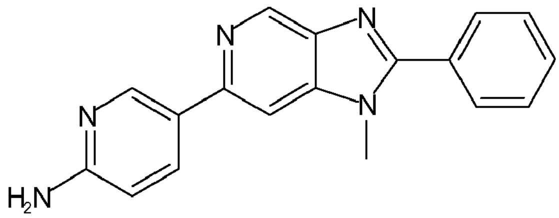

MW.: 301.13, LCMS-ESI (*m/z*) - found 302.13 $[M+H]^+$, 300.13 $[M-H]^-$

**Example 12-736 Compound 736**

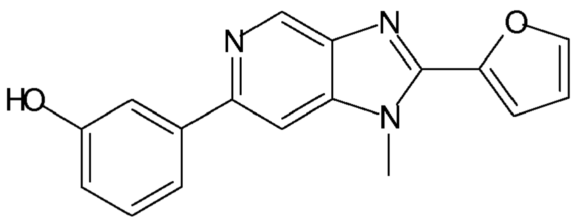

MW.: 291.10, LCMS-ESI (*m/z*) - found 292.10 $[M+H]^+$, 290.1 $[M-H]^-$

**Example 12-737 Compound 737**

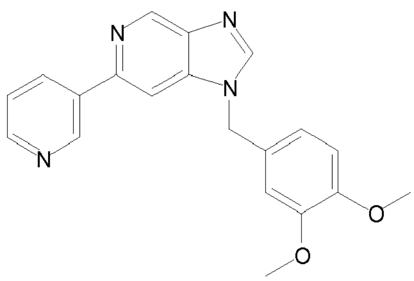

MW.: 346.14, LCMS-ESI (*m/z*) - found 347.14 $[M+H]^+$, 345.14 $[M-H]^-$

**Example 12-738 Compound 738**

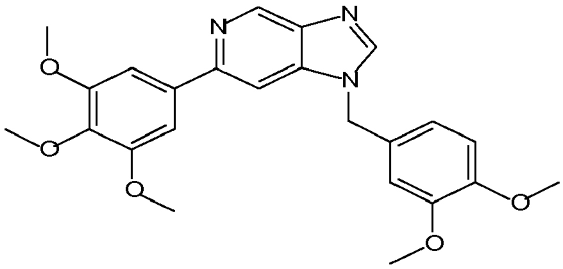

MW.: 435.18, LCMS-ESI (*m*/*z*) - found 436.17 $[M+H]^+$, 434.17 $[M-H]^-$

**Example 12-739 Compound 739**

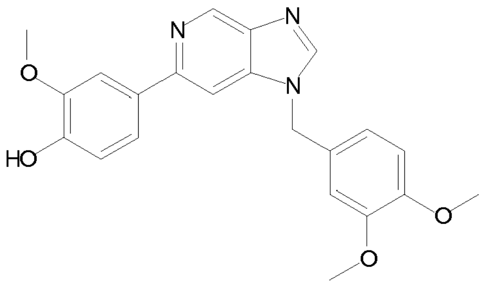

MW.: 391.15, LCMS-ESI (*m*/*z*) - found 392.15 $[M+H]^+$, 390.15 $[M-H]^-$

**Example 12-740 Compound 740**

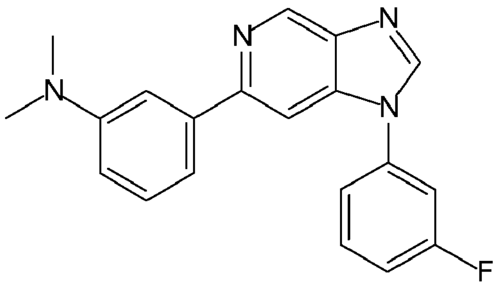

MW.: 332.14, LCMS-ESI (*m*/*z*) - found 333.14 $[M+H]^+$, 331.14 $[M-H]^-$

**Example 12-741 Compound 741**

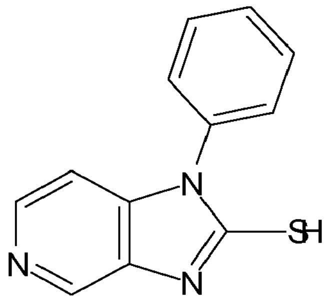

MW.: 227.29, LCMS-ESI (*m*/*z*) - found 228.29 $[M+H]^+$, 226.29 $[M-H]^-$

**Example 12-742 Compound 742**

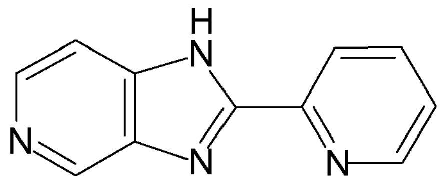

MW.: 196.213, LCMS-ESI (*m*/*z*) - found 197.21 $[M+H]^+$, 196.21 $[M-H]^-$

**Example 12-743 Compound 743**

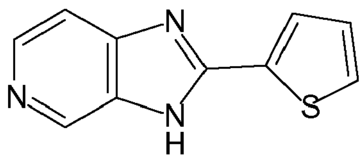

MW.: 201.251, LCMS-ESI (*m*/*z*) - found 202.25 $[M+H]^+$, 200.25 $[M-H]^-$

**Example 12-744 Compound 744**

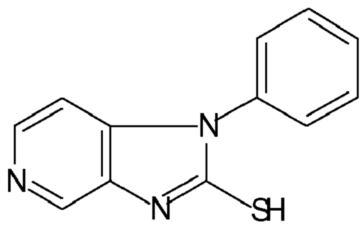

MW.: 227.28, LCMS-ESI (*m*/*z*) - found 228.28 $[M+H]^+$, 226.28 $[M-H]^-$

**Example 12-745 Compound 745**

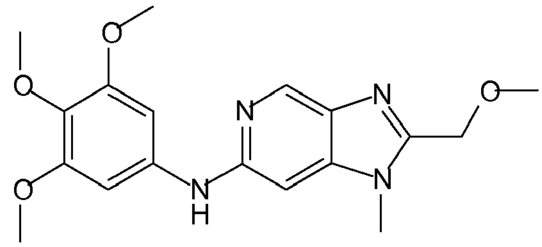

MW.: 358.16, LCMS-ESI (*m/z*) - found 359.16 $[M+H]^+$, 357.16 $[M-H]^-$

**Example 12-746 Compound 746**

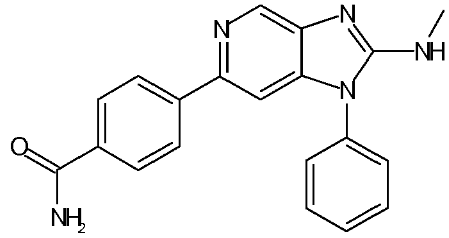

MW.: 343,14, LCMS-ESI (*m/z*) - found 344.14 $[M+H]^+$, 342.14 $[M-H]^-$

**Example 12-747 Compound 747**

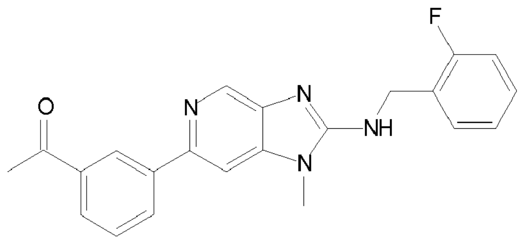

MW.: 374.15, LCMS-ESI (m/z) - found 375.15 $[M+H]^+$, 373.15 $[M-H]^-$

**Example 12-748 Compound 748**

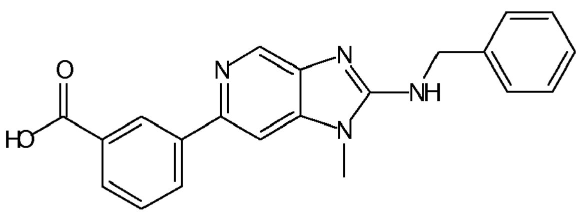

MW.: 358.14, LCMS-ESI (*m/z*) - found 359.14 $[M+H]^+$, 357.14 $[M-H]^-$

**Example 12-749 Compound 749**

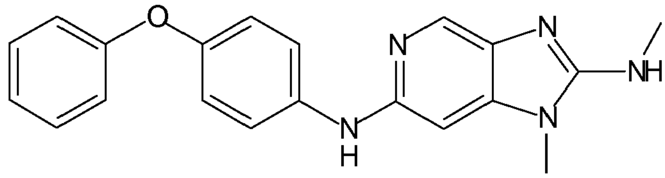

MW.: 345.16, LCMS-ESI (*m*/*z*) - found 346.15 $[M+H]^+$, 344.15 $[M-H]^-$

**Example 12-750 Compound 750**

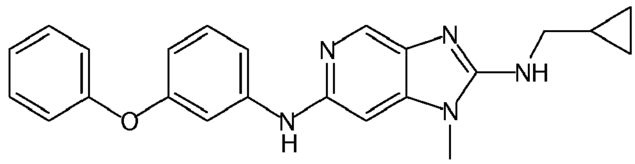

MW.: 385.19, LCMS-ESI (*m*/*z*) - found 386.19 $[M+H]^+$, 384.19 $[M-H]^-$

**Example 12-751 Compound 751**

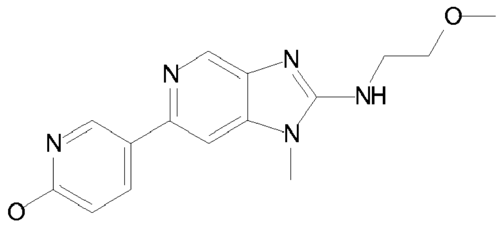

MW.: 313.15, LCMS-ESI (*m*/*z*) - found 314.15 $[M+H]^+$, 312.15 $[M-H]^-$

**Example 12-752 Compound 752**

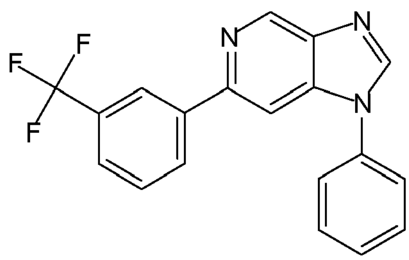

MW.: 339.10, LCMS-ESI (*m*/*z*) - found 340.10 $[M+H]^+$, 338.10 $[M-H]^-$

**Example 12-753 Compound 753**

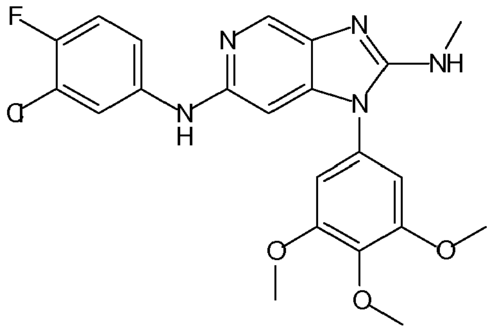

MW.: 457.13, LCMS-ESI (*m/z*) - found 458.13 $[M+H]^+$, 456.13 $[M-H]^-$

**Example 12-754 Compound 754**

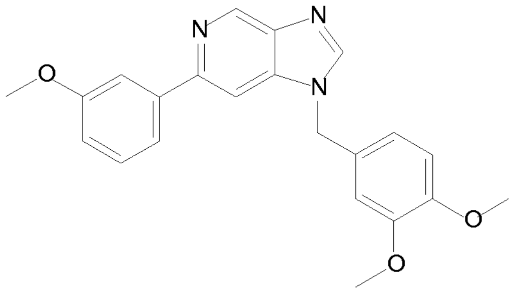

MW.: 375.16, LCMS-ESI (*m/z*) - found 376.15 $[M+H]^+$, 374.15 $[M-H]^-$

**Example 12-755 Compound 755**

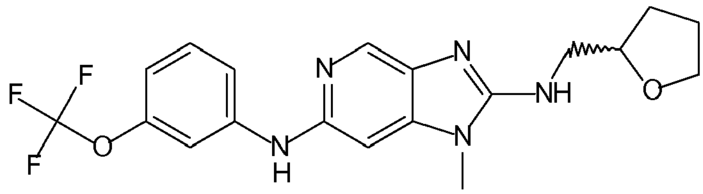

MW.: 407.16, LCMS-ESI (*m/z*) - found 408.15 $[M+H]^+$, 406.15 $[M-H]^-$

**Example 12-756 Compound 756**

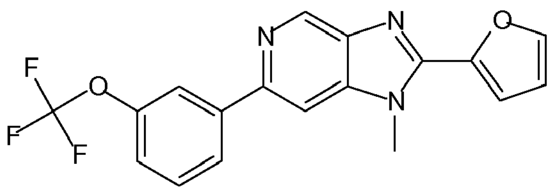

MW.: 359.09, LCMS-ESI (*m/z*) - found 360.08 $[M+H]^+$, 358.08 $[M-H]^-$

**Example 12-757 Compound 757**

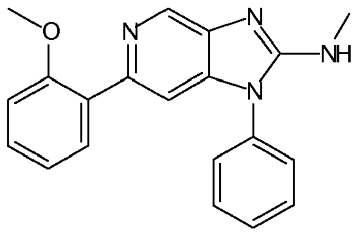

MW.: 330.15, LCMS-ESI (*m*/*z*) - found 331.14 $[M+H]^+$, 329.14 $[M-H]^-$

**Example** 12-758 Compound 758

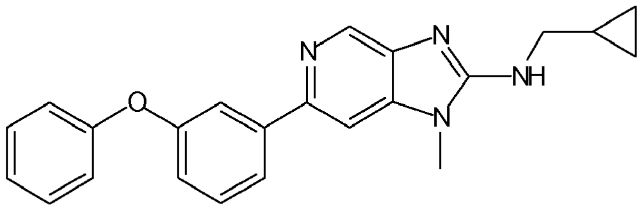

MW.: 370.18, LCMS-ESI (m/z) - found 371.17 $[M+H]^+$, 369.17 $[M-H]^-$

**Example** 12-759 Compound 759

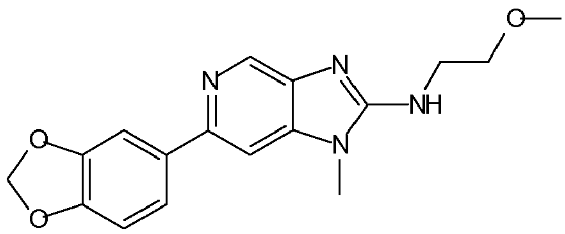

MW.: 326.14, LCMS-ESI (*m*/*z*) - found 327.13 $[M+H]^+$, 325.13 $[M-H]^-$

**Example** 12-760 Compound 760

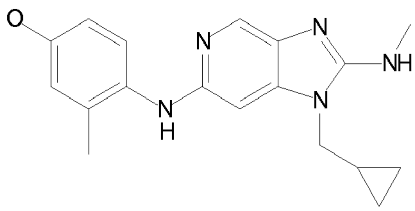

MW.: 337.19, LCMS-ESI (m/z) - found 338.19 $[M+H]^+$, 336.19 $[M-H]^-$

**Example** 12-761 Compound 761

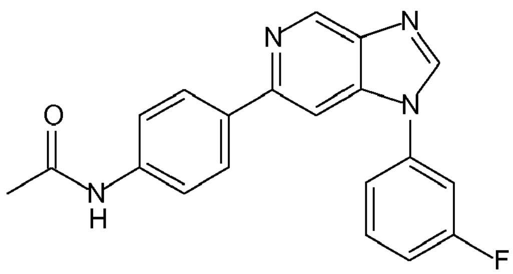

MW.: 337.19, LCMS-ESI (*m*/*z*) - found 338.19 $[M+H]^+$, 336.19 $[M-H]^-$

**Example 12-762 Compound 762**

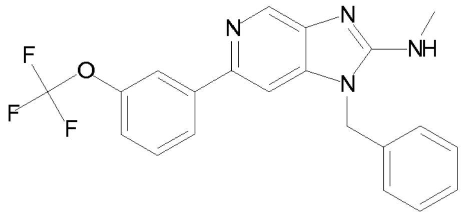

MW.: 398.14, LCMS-ESI (*m*/*z*) - found 399.13 $[M+H]^+$, 397.14 $[M-H]^-$

**Example 12-763 Compound 763**

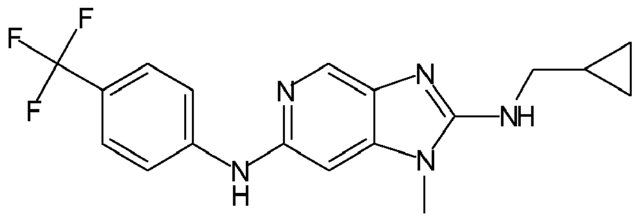

MW.: 361.15, LCMS-ESI (*m*/*z*) - found 362.15 $[M+H]^+$, 360.15 $[M-H]^-$

**Example 12-764 Compound 764**

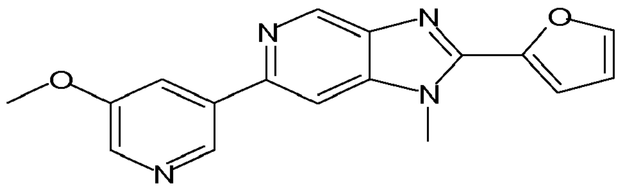

MW.: 306.11, LCMS-ESI (*m*/*z*) - found 307.11 $[M+H]^+$, 305.11 $[M-H]^-$

**Example 12-765 Compound 765**

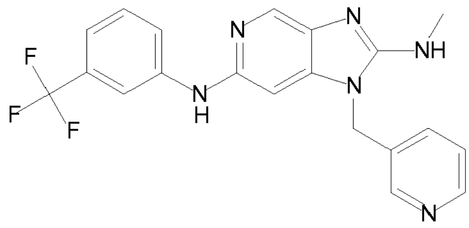

MW.: 398.15, LCMS-ESI (*m*/*z*) - found 399.14 $[M+H]^+$, 397.14 $[M-H]^-$

**Example 12-766 Compound 766**

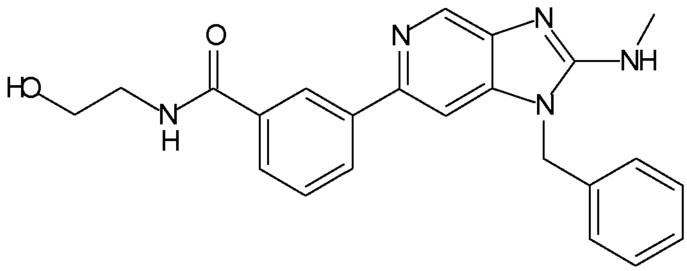

MW.: 401.19, LCMS-ESI (*m*/*z*) - found 402.18 $[M+H]^+$, 400.18 $[M-H]^-$

**Example 12-767 Compound 767**

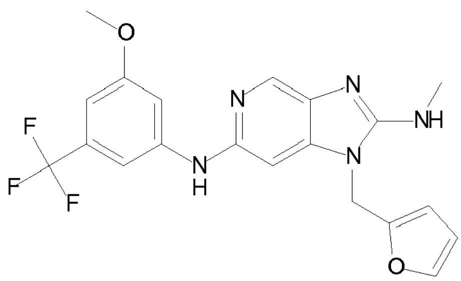

MW.: 417.14, LCMS-ESI (*m*/*z*) - found 418.14 $[M+H]^+$, 416.14 $[M-H]^-$

**Example 12-768 Compound 768**

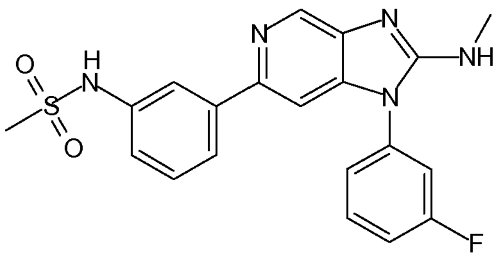

MW.: 411.12, LCMS-ESI (*m/z*) - found 412.11 $[M+H]^+$, 410.12 $[M-H]^-$

**Example 12-769 Compound 769**

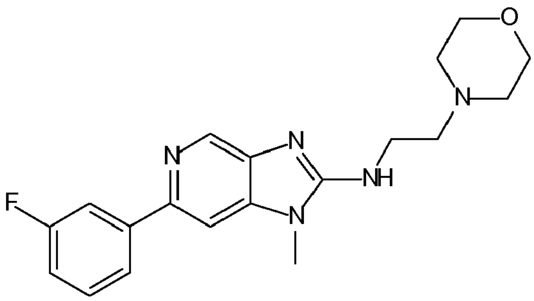

MW.: 355.18, LCMS-ESI (*m/z*) - found 356.18 $[M+H]^+$, 354.18 $[M-H]^-$

**Example 12-770 Compound 770**

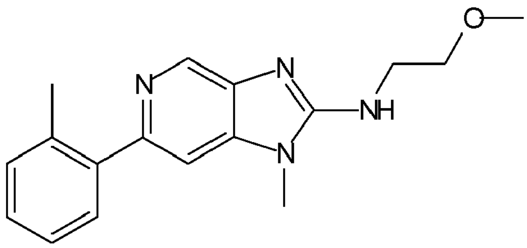

MW.: 296.16, LCMS-ESI (*m/z*) - found 297.16 $[M+H]^+$, 295.16 $[M-H]^-$

**Example 12-771 Compound 771**

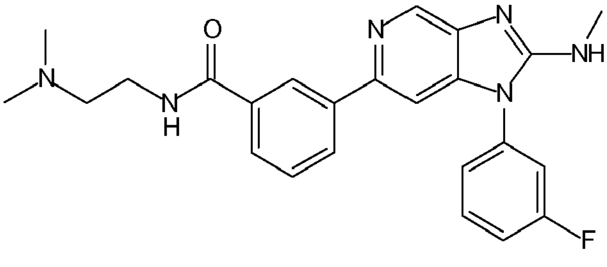

MW.: 432.21, LCMS-ESI ($m/z$) - found 433.20 $[M+H]^+$, 431.20 $[M-H]^-$

**Example 12-772 Compound 772**

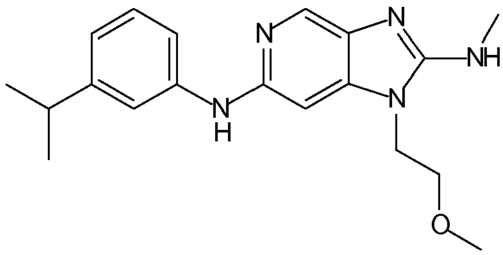

MW.: 339.21, LCMS-ESI ($m/z$) - found 340.20 $[M+H]^+$, 338.20 $[M-H]^-$

**Example 12-773 Compound 773**

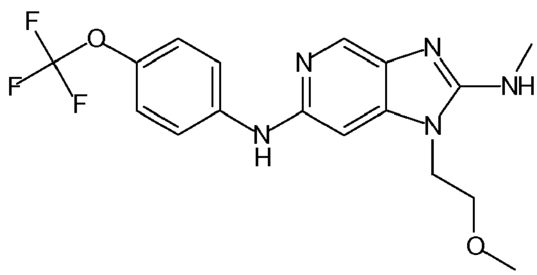

MW.: 381.14, LCMS-ESI ($m/z$) - found 382.14 $[M+H]^+$, 380.14 $[M-H]^-$

**Example 12-774 Compound 774**

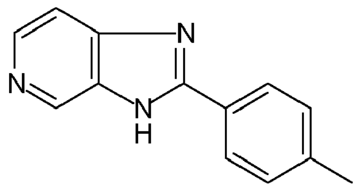

MW.: 209.252, LCMS-ESI ($m/z$) - found 210.25 $[M+H]^+$, 208.25$[M-H]^-$

**Example 12-775 Compound 775**

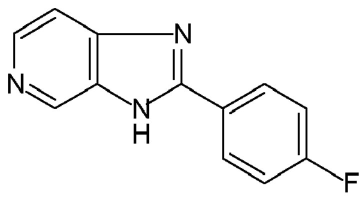

MW.: 213.215, LCMS-ESI (*m/z*) - found 214.21 $[M+H]^+$, 212.21 $[M-H]^-$

**Example 12-776 Compound 776**

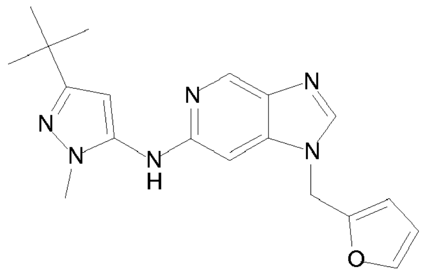

MW.: 350.19, LCMS-ESI (*m/z*) - found 351.18 $[M+H]^+$, 349.18 $[M-H]^-$

**Example 12-777 Compound 777**

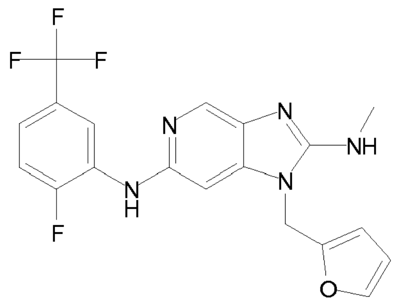

MW.: 405.12, LCMS-ESI (*m/z*) - found 406.12 $[M+H]^+$, 404.12 $[M-H]^-$

**Example 12-778 Compound 778**

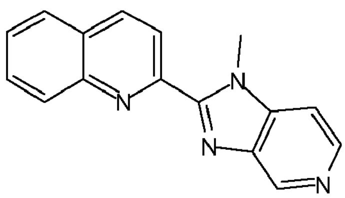

MW.: 260.3, LCMS-ESI (*m/z*) - found 261.3 $[M+H]^+$, 259.3 $[M-H]^-$

**Example 12-779 Compound 779**

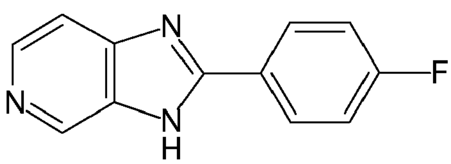

MW.: 213.215, LCMS-ESI (*m*/*z*) - found 214.21 [M+H]$^+$, 212.21 [M-H]$^-$

**Example 12-780 Compound 780**

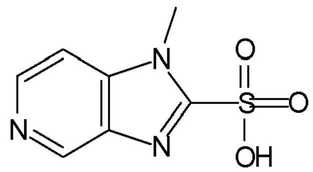

MW.: 213.215, LCMS-ESI (*m*/*z*) - found 214.21 [M+H]$^+$, 212.21 [M-H]$^-$

**Example 12-781 Compound 781**

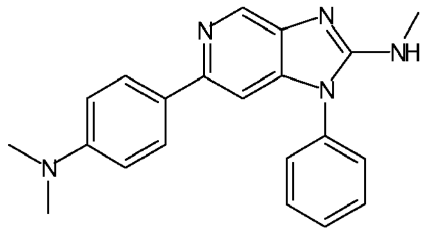

MW.: 343.18, LCMS-ESI (*m*/*z*) - found 344.18 [M+H]$^+$, 342.18 [M-H]$^-$

**Example 12-782 Compound 782**

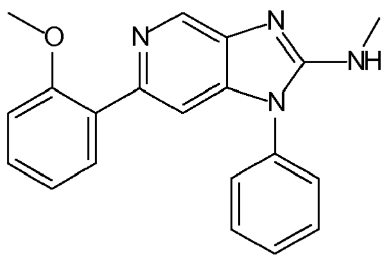

MW.: 330.15, LCMS-ESI (*m*/*z*) - found 331.14 [M+H]$^+$, 329.14 [M-H]$^-$

**Example 12-783 Compound 783**

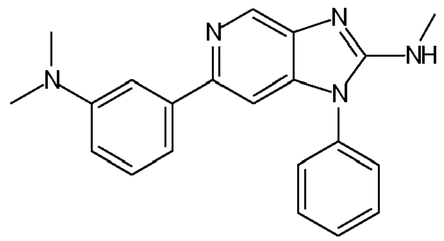

MW.: 343.18, LCMS-ESI (*m/z*) - found 344.18 $[M+H]^+$, 342.18 $[M-H]^-$

**Example 12-784 Compound 784**

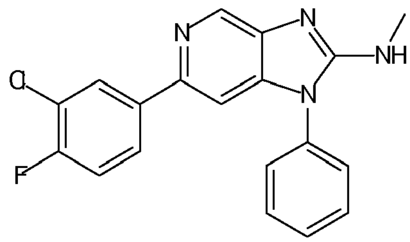

MW.: 352.09, LCMS-ESI (*m/z*) - found 353.08 $[M+H]^+$, 351.08 $[M-H]^-$

**Example 12-785 Compound 785**

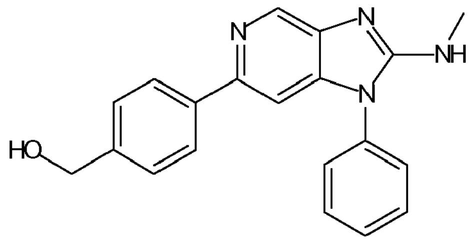

MW.: 330.15, LCMS-ESI (*m/z*) - found 331.14 $[M+H]^+$, 329.14 $[M-H]^-$

**Example 12-786 Compound 786**

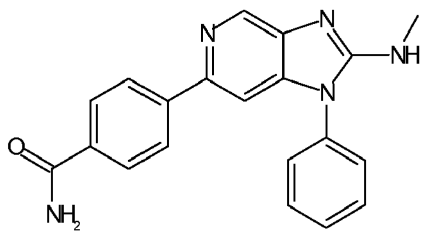

MW.: 343.14, LCMS-ESI (*m/z*) - found 344.14 $[M+H]^+$, 342.14 $[M-H]^-$

**Example 12-787 Compound 787**

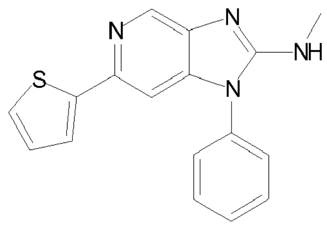

MW.: 306.09, LCMS-ESI (*m/z*) - found 307.09 $[M+H]^+$, 305.09 $[M-H]^-$

**Example 12-788 Compound 788**

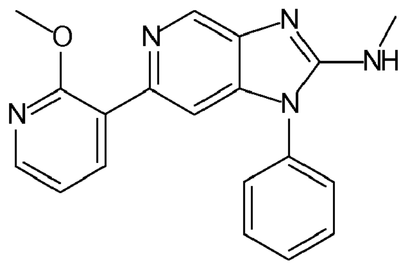

MW.: 331.14, LCMS-ESI (*m/z*) - found 332.14 $[M+H]^+$, 330.14 $[M-H]^-$

**Example 12-789 Compound 789**

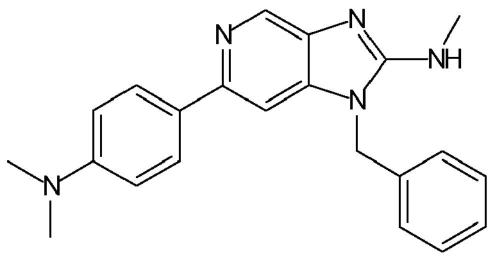

MW.: 357.20, LCMS-ESI (*m/z*) - found 358.19 $[M+H]^+$, 356.19 $[M-H]^-$

**Example 12-790 Compound 790**

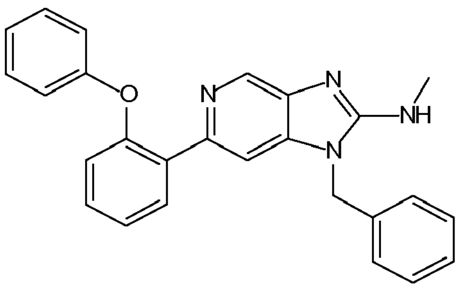

MW.: 406.18, LCMS-ESI (*m*/*z*) - found 407.17 $[M+H]^+$, 405.17 $[M-H]^-$

**Example 12-791 Compound 791**

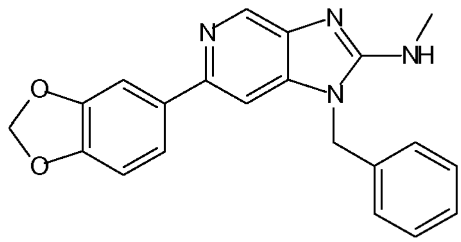

MW.: 358.14, LCMS-ESI (*m*/*z*) - found 359.14 $[M+H]^+$, 357.14 $[M-H]^-$

**Example 12-792 Compound 792**

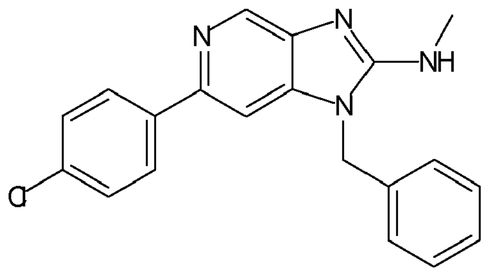

MW.: 348.11, LCMS-ESI (*m*/*z*) - found 349.11 $[M+H]^+$, 347.11 $[M-H]^-$

**Example 12-793 Compound 793**

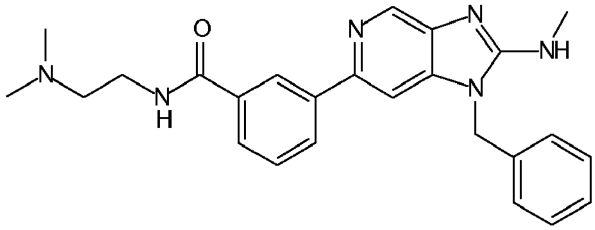

MW.: 428.23, LCMS-ESI (*m*/*z*) - found 429.23 $[M+H]^+$, 427.23 $[M-H]^-$

**Example 12-794 Compound 794**

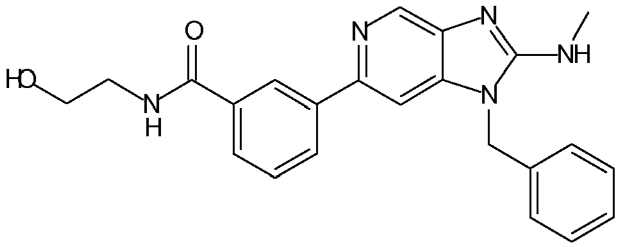

MW.: 401.19, LCMS-ESI (*m/z*) - found 402.18 $[M+H]^+$, 400.18 $[M-H]^-$

**Example 12-795 Compound 795**

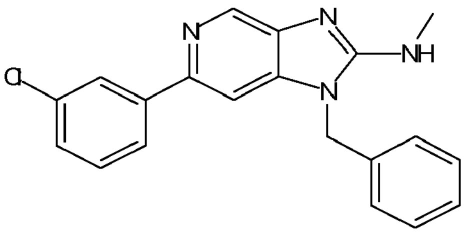

MW.: 348.11, LCMS-ESI (*m/z*) - found 349.11 $[M+H]^+$, 347.11 $[M-H]^-$

**Example 12-796 Compound 796**

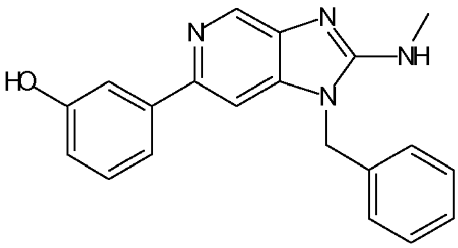

MW.: 330.15, LCMS-ESI (*m/z*) - found 331.14 $[M+H]^+$, 329.14 $[M-H]^-$

**Example 12-797 Compound 797**

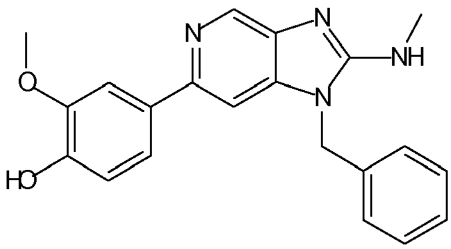

MW.: 360.16, LCMS-ESI (*m/z*) - found 361.15 $[M+H]^+$, 359.15 $[M-H]^-$

**Example 12-798 Compound 798**

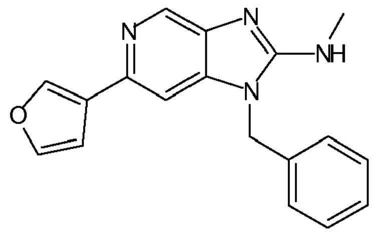

MW.: 304.13, LCMS-ESI (*m/z*) - found 305.13 $[M+H]^+$, 303.13 $[M-H]^-$

**Example 12-799 Compound 799**

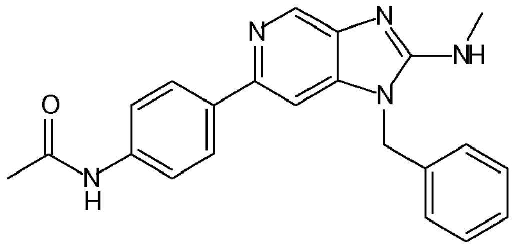

MW.: 371.17, LCMS-ESI (m/z) - found 372.17 $[M+H]^+$, 370.17 $[M-H]^-$

**Example 12-800 Compound 800**

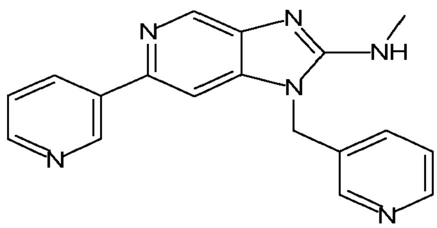

MW.: 316.14, LCMS-ESI (*m/z*) - found 317.14 $[M+H]^+$, 315.14 $[M-H]^-$

**Example 12-801 Compound 801**

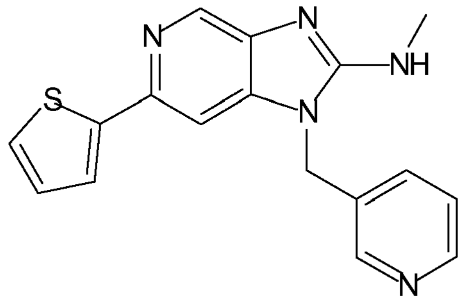

MW.: 321.10, LCMS-ESI (*m/z*) - found 322.10 $[M+H]^+$, 320.10 $[M-H]^-$

**Example 12-802 Compound 802**

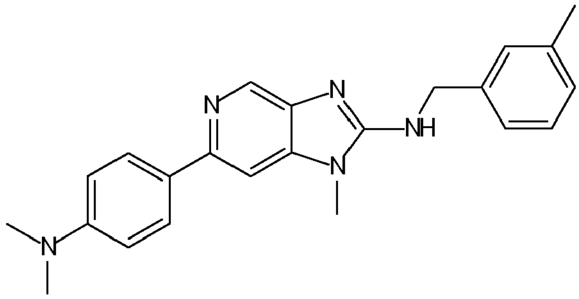

MW.: 371.21, LCMS-ESI (*m/z*) - found 372.21 $[M+H]^+$, 370.21 $[M-H]^-$

**Example 12-803 Compound 803**

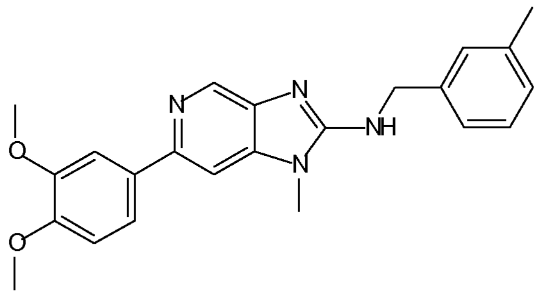

MW.: 388.19, LCMS-ESI (*m/z*) - found 389.19 $[M+H]^+$, 387.19 $[M-H]^-$

**Example 12-804 Compound 804**

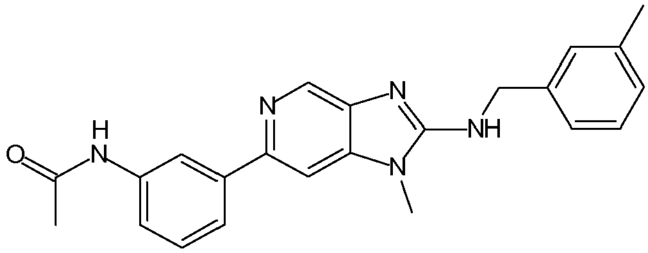

MW.: 385.19, LCMS-ESI (*m*/*z*) - found 386.19 $[M+H]^+$, 384.19 $[M-H]^-$

**Example 12-805 Compound 805**

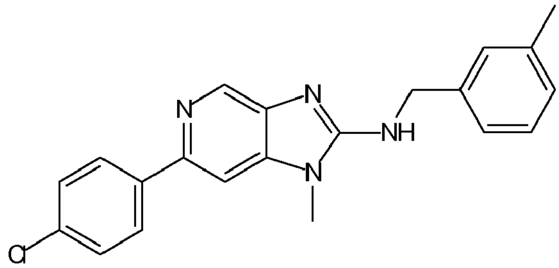

MW.: 362.13, LCMS-ESI (*m*/*z*) - found 363.13 $[M+H]^+$, 361.13 $[M-H]^-$

**Example 12-806 Compound 806**

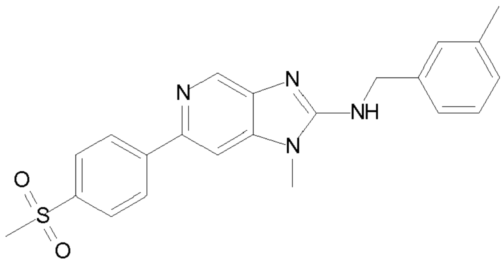

MW.: 406.15, LCMS-ESI (*m*/*z*) - found 407.14 $[M+H]^+$, 405.14 $[M-H]^-$

**Example 12-807 Compound 807**

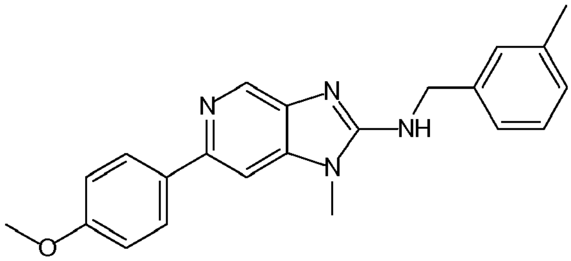

MW.: 358.18, LCMS-ESI (*m*/*z*) - found 359.17 $[M+H]^+$, 357.17 $[M-H]^-$

**Example 12-808 Compound 808**

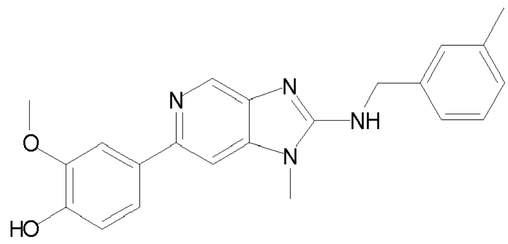

MW.: 374.17, LCMS-ESI (*m*/*z*) - found 375.17 $[M+H]^+$, 373.17 $[M-H]^-$

**Example 12-809 Compound 809**

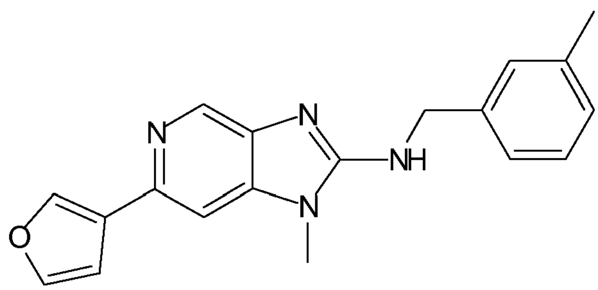

MW.: 318.15, LCMS-ESI (*m*/*z*) - found 319.14 $[M+H]^+$, 317.14 $[M-H]^-$

**Example 12-810 Compound 810**

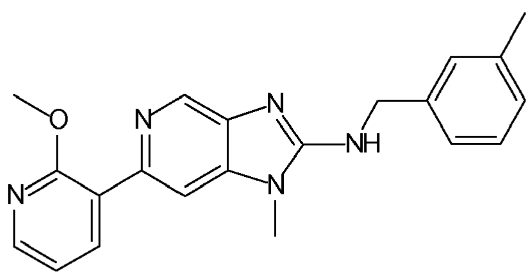

MW.: 359.17, LCMS-ESI (*m*/*z*) - found 360.17 $[M+H]^+$, 358.17 $[M-H]^-$

**Example 12-811 Compound 811**

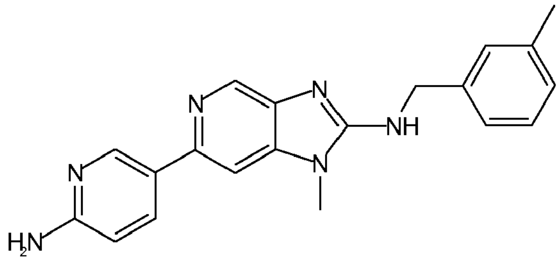

MW.: 344.17, LCMS-ESI (*m*/*z*) - found 345.17 $[M+H]^+$, 343.17 $[M-H]^-$

**Example 12-812 Compound 812**

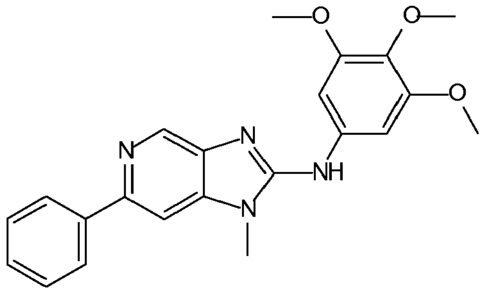

MW.: 390.17, LCMS-ESI (*m*/*z*) - found 391.16 $[M+H]^+$, 389.16 $[M-H]^-$

**Example 12-813 Compound 813**

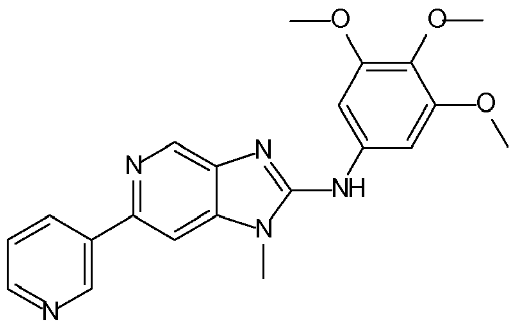

MW.: 391.16, LCMS-ESI (*m*/*z*) - found 392.16 $[M+H]^+$, 390.16 $[M-H]^-$

**Example 12-814 Compound 814**

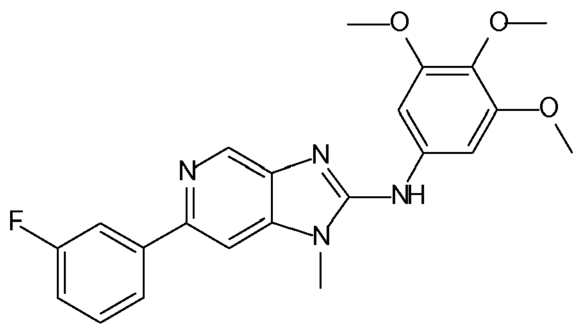

MW.: 408.16, LCMS-ESI (*m*/*z*) - found 409.16 $[M+H]^+$, 407.16 $[M-H]^-$

**Example 12-815 Compound 815**

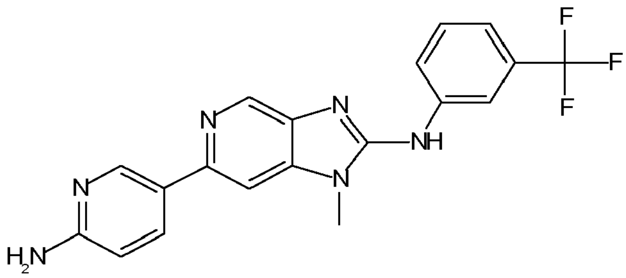

MW.: 384.13, LCMS-ESI (*m*/*z*) - found 385.13 $[M+H]^+$, 383.13 $[M-H]^-$

**Example 12-816 Compound 816**

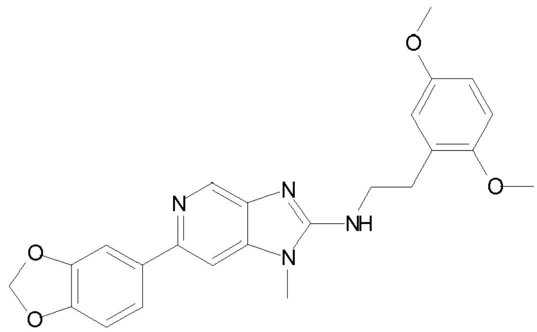

MW.: 432.18, LCMS-ESI (*m*/*z*) - found 433.18 $[M+H]^+$, 431.18 $[M-H]^-$

**Example 12-817 Compound 817**

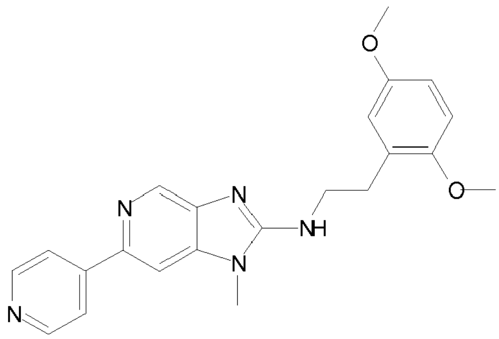

MW.: 389.19, LCMS-ESI (*m*/*z*) - found 390.18 $[M+H]^+$, 388.19 $[M-H]^-$

**Example 12-818 Compound 818**

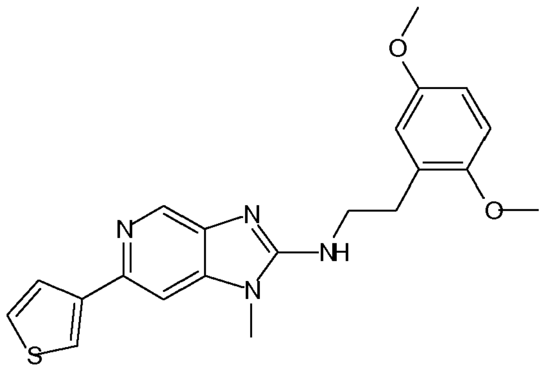

MW.: 394.15, LCMS-ESI (*m*/*z*) - found 395.14 $[M+H]^+$, 393.14 $[M-H]^-$

**Example 12-819 Compound 819**

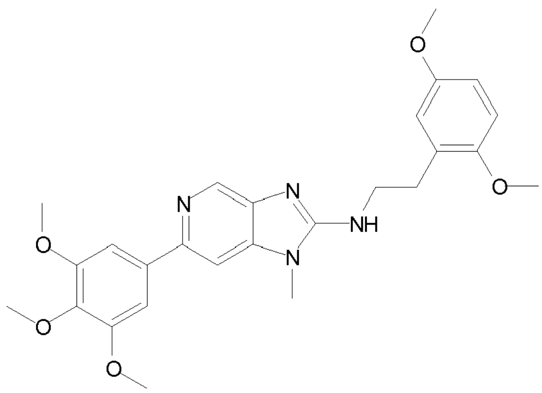

MW.: 478.22, LCMS-ESI (*m*/*z*) - found 479.22 $[M+H]^+$, 477.22 $[M-H]^-$

**Example 12-820 Compound 820**

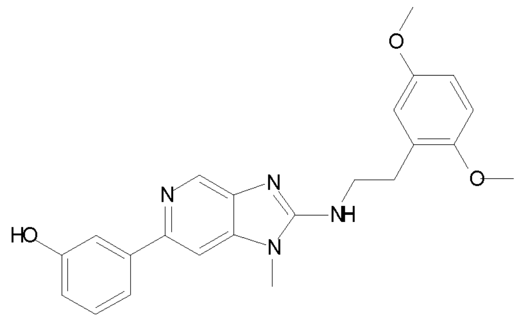

MW.: 404.18, LCMS-ESI (*m*/*z*) - found 405.18 $[M+H]^+$, 403.18 $[M-H]^-$

**Example 12-821 Compound 821**

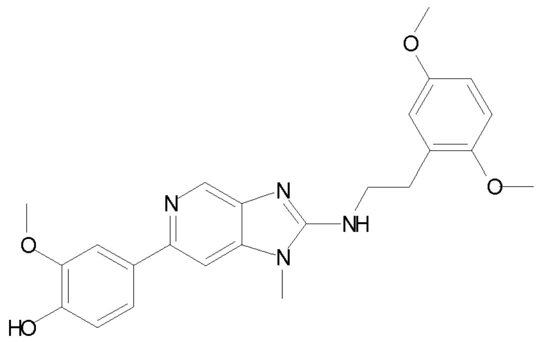

MW.: 434.20, LCMS-ESI (*m*/*z*) - found 435.19 $[M+H]^+$, 433.19 $[M-H]^-$

**Example 12-822 Compound 822**

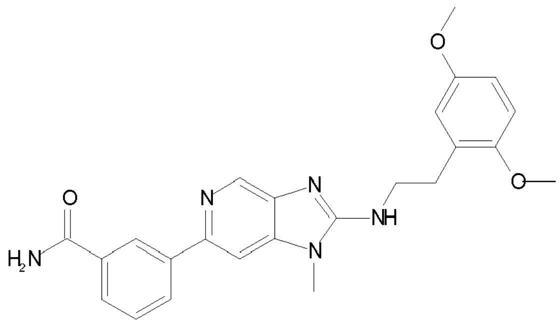

MW.: 431.20, LCMS-ESI (*m*/*z*) - found 432.19 $[M+H]^+$, 430.19 $[M-H]^-$

**Example 12-823 Compound 823**

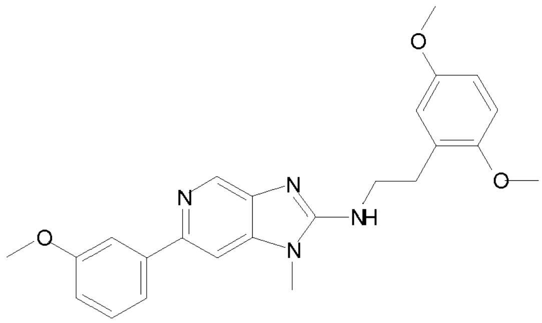

MW.: 418.20, LCMS-ESI (*m*/*z*) - found 419.20 $[M+H]^+$, 417.20 $[M-H]^-$

**Example 12-824 Compound 824**

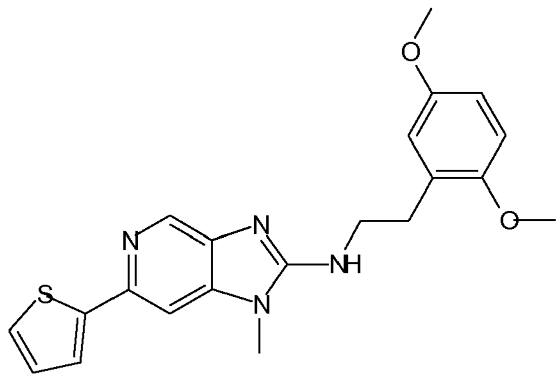

MW.: 394.15, LCMS-ESI (*m*/*z*) - found 395.14 $[M+H]^+$, 393.14 $[M-H]^-$

**Example 12-825 Compound 825**

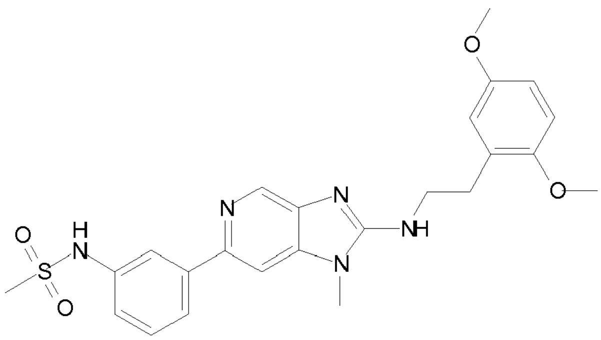

MW.: 481.18, LCMS-ESI (*m*/*z*) - found 482.17 $[M+H]^+$, 480.17 $[M-H]^-$

**Example 12-826 Compound 826**

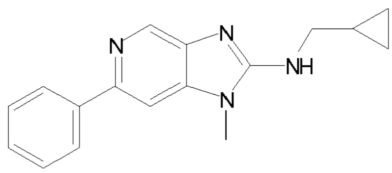

MW.: 278.15, LCMS-ESI (*m*/*z*) - found 279.15 $[M+H]^+$, 277.15 $[M-H]^-$

**Example 12-827 Compound 827**

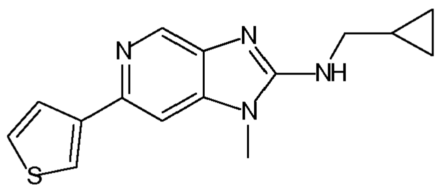

MW.: 284.11, LCMS-ESI (*m*/*z*) - found 285.11 $[M+H]^+$, 283.11 $[M-H]^-$

**Example 12-828 Compound 828**

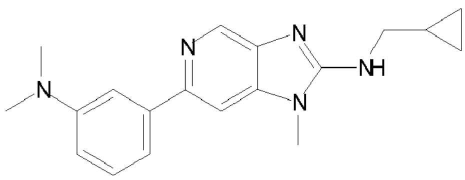

MW.: 321.20, LCMS-ESI (*m*/*z*) - found 322.19 $[M+H]^+$, 320.19 $[M-H]^-$

**Example 12-829 Compound 829**

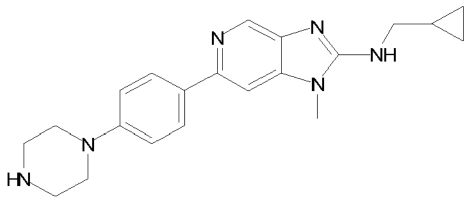

MW.: 362.22, LCMS-ESI (*m*/*z*) - found 363.22 $[M+H]^+$, 361.22 $[M-H]^-$

**Example 12-830 Compound 830**

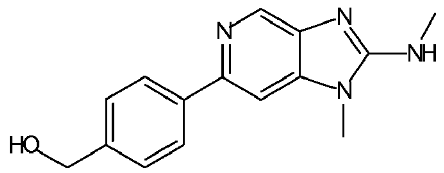

MW.: 268.13, LCMS-ESI (*m*/*z*) - found 269.13 $[M+H]^+$, 267.13 $[M-H]^-$

**Example 12-831 Compound 831**

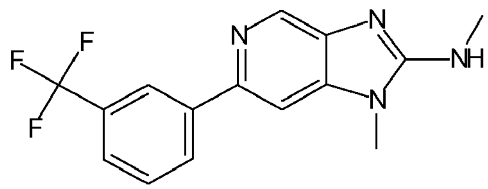

MW.: 306.11, LCMS-ESI (*m*/*z*) - found 307.10 $[M+H]^+$, 305.10 $[M-H]^-$

**Example 12-832 Compound 832**

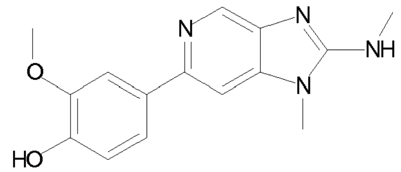

MW.: 284.13, LCMS-ESI (*m*/*z*) - found 285.12 $[M+H]^+$, 283.12 $[M-H]^-$

**Example 12-833 Compound 833**

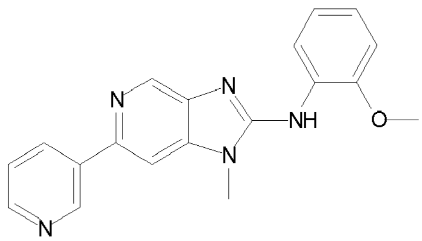

MW.: 331.14, LCMS-ESI (*m*/*z*) - found 332.14 $[M+H]^+$, 330.14 $[M-H]^-$

**Example 12-834 Compound 834**

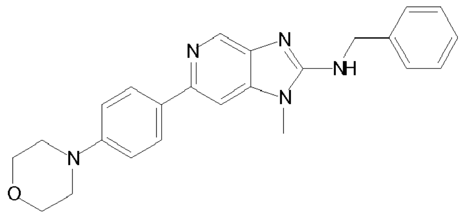

MW.: 399.21, LCMS-ESI (*m*/*z*) - found 400.20 $[M+H]^+$, 398.20 $[M-H]^-$

**Example 12-835 Compound 835**

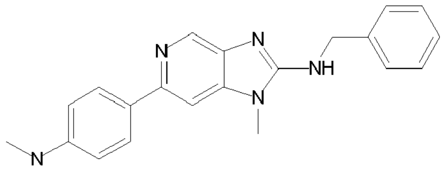

MW.: 357.20, LCMS-ESI (*m*/*z*) - found 358.19 $[M+H]^+$, 356.19 $[M-H]^-$

**Example 12-836 Compound 836**

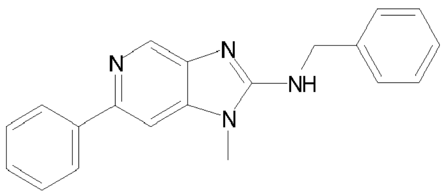

MW.: 314.15, LCMS-ESI (*m*/*z*) - found 315.15 $[M+H]^+$, 313.15 $[M-H]^-$

**Example 12-837 Compound 837**

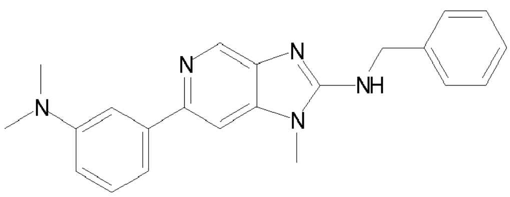

MW.: 357.20, LCMS-ESI (*m*/*z*) - found 358.19 $[M+H]^+$, 356.19 $[M-H]^-$

**Example 12-838 Compound 838**

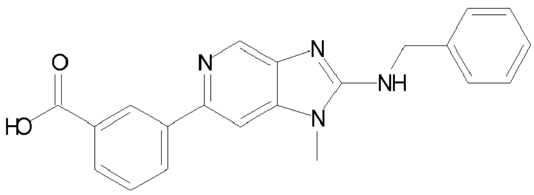

MW.: 358.14, LCMS-ESI ($m/z$) - found 359.14 $[M+H]^+$, 357.14 $[M-H]^-$

**Example 12-839 Compound 839**

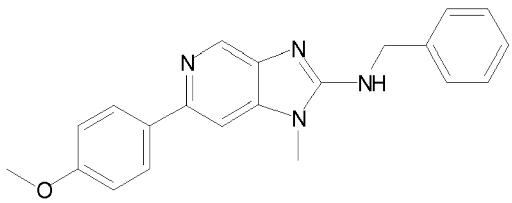

MW.: 344.16, LCMS-ESI ($m/z$) - found 345.16 $[M+H]^+$, 343.16 $[M-H]^-$

**Example 12-840 Compound 840**

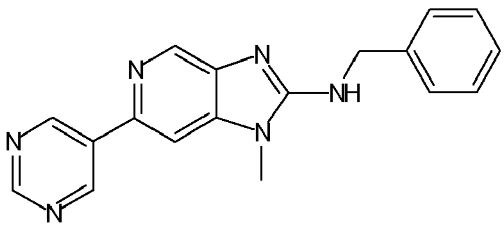

MW.: 316.14, LCMS-ESI ($m/z$) - found 317.14 $[M+H]^+$, 315.14 $[M-H]^-$

**Example 12-841 Compound 841**

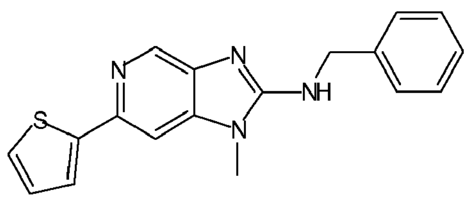

MW.: 320.11, LCMS-ESI ($m/z$) - found 321.11 $[M+H]^+$, 319.11 $[M-H]^-$

**Example 12-842 Compound 842**

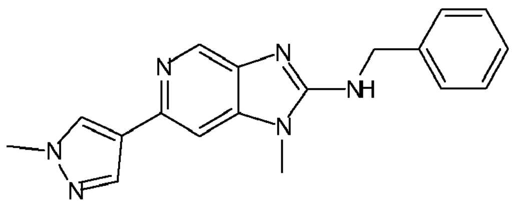

MW.: 318.16, LCMS-ESI (*m*/*z*) - found 319.15 $[M+H]^+$, 317.15 $[M-H]^-$

**Example 12-843 Compound 843**

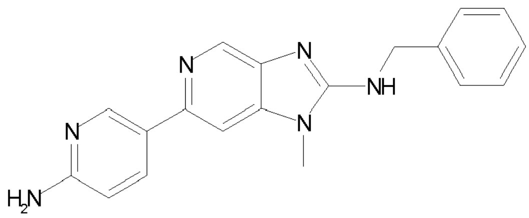

MW.: 330.16, LCMS-ESI (*m*/*z*) - found 331.15 $[M+H]^+$, 329.15 $[M-H]^-$

**Example 12-844 Compound 844**

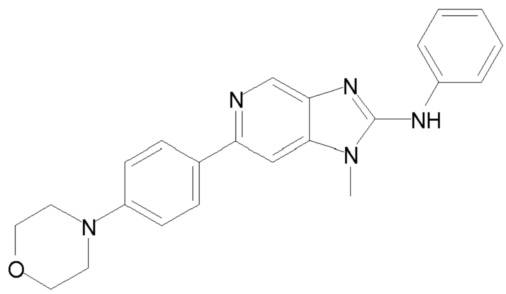

MW.: 385.19, LCMS-ESI (*m*/*z*) - found 386.19 $[M+H]^+$, 384.19 $[M-H]^-$

**Example 12-845 Compound 845**

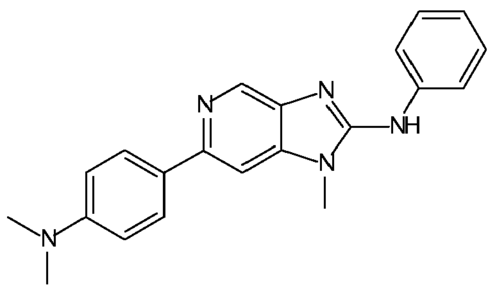

MW.: 343.18, LCMS-ESI (*m*/*z*) - found 344.18 $[M+H]^+$, 342.18 $[M-H]^-$

**Example 12-846 Compound 846**

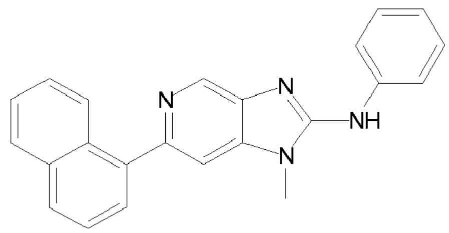

MW.: 350.15, LCMS-ESI (*m*/*z*) - found 351.15 $[M+H]^+$, 349.15 $[M-H]^-$

**Example 12-847 Compound 847**

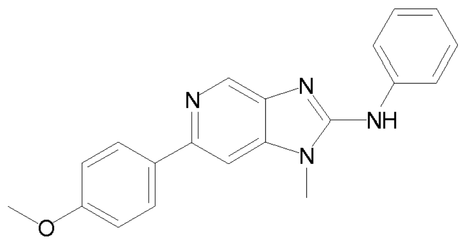

MW.: 330.15, LCMS-ESI (*m*/*z*) - found 331.14 $[M+H]^+$, 329.14 $[M-H]^-$

**Example 12-848 Compound 848**

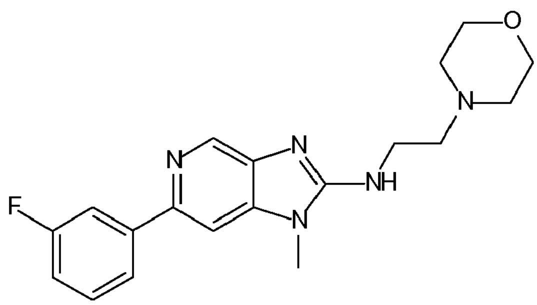

MW.: 355.18, LCMS-ESI (*m*/*z*) - found 356.18 $[M+H]^+$, 354.18 $[M-H]^-$

**Example 12-849 Compound 849**

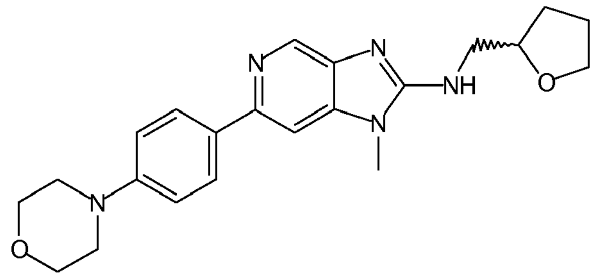

MW.: 393.22, LCMS-ESI (*m*/*z*) - found 394.21 $[M+H]^+$, 392.21 $[M-H]^-$

**Example 12-850 Compound 850**

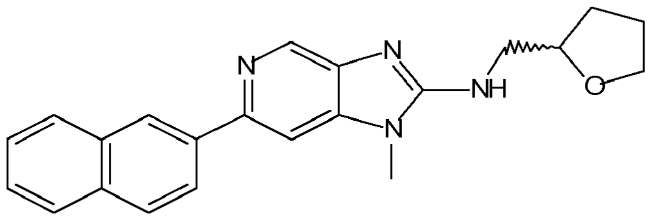

MW.: 358.18, LCMS-ESI (*m*/*z*) - found 359.18 $[M+H]^+$, 357.18 $[M-H]^-$

**Example 12-851 Compound 851**

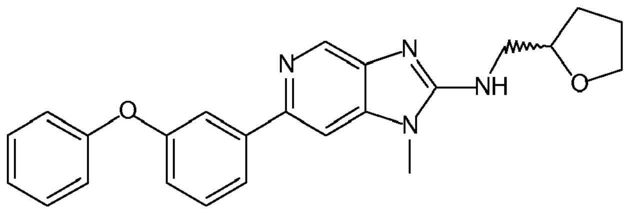

MW.: 400.19, LCMS-ESI (*m*/*z*) - found 401.19 $[M+H]^+$, 399.19 $[M-H]^-$

**Example 12-852 Compound 852**

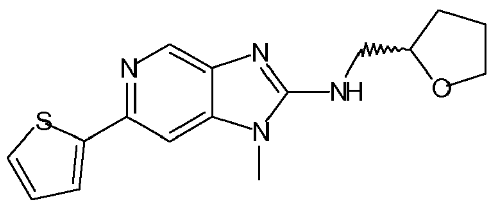

MW.: 314.12, LCMS-ESI (*m*/*z*) - found 315.12 $[M+H]^+$, 313.12 $[M-H]^-$

**Example 12-853 Compound 853**

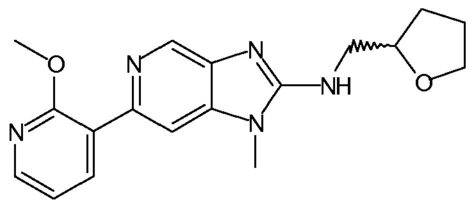

MW.: 339.17, LCMS-ESI (*m*/*z*) - found 340.17 $[M+H]^+$, 338.17 $[M-H]^-$

**Example 12-854 Compound 854**

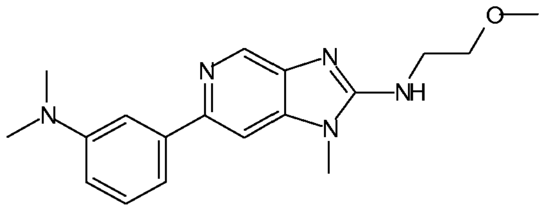

MW.: 325.19, LCMS-ESI (*m*/*z*) - found 326.19 $[M+H]^+$, 324.19 $[M-H]^-$

**Example 12-855 Compound 855**

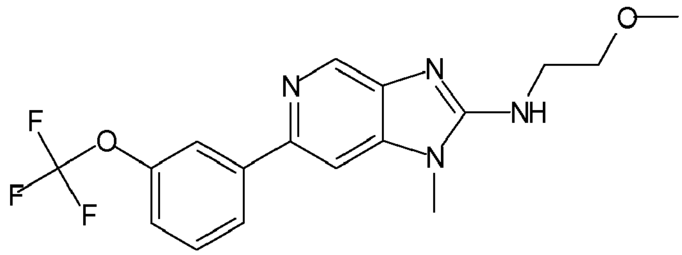

MW.: 366.13, LCMS-ESI (*m*/*z*) - found 367.13 $[M+H]^+$, 365.13 $[M-H]^-$

**Example 12-856 Compound 856**

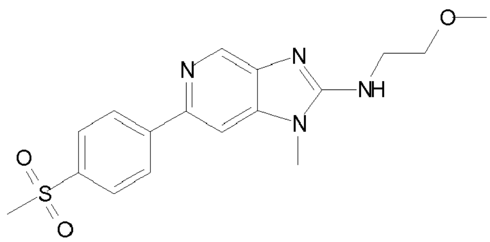

MW.: 360.13, LCMS-ESI (*m*/*z*) - found 361.13 $[M+H]^+$, 359.13 $[M-H]^-$

**Example 12-857 Compound 857**

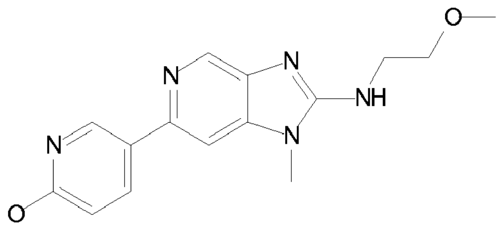

MW.: 313.15, LCMS-ESI (*m*/*z*) - found 314.15 $[M+H]^+$, 312.15 $[M-H]^-$

**Example 12-858 Compound 858**

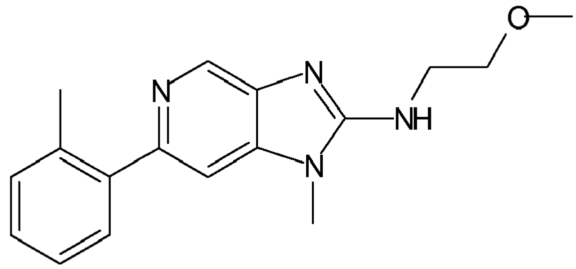

MW.: 296.16, LCMS-ESI (*m*/*z*) - found 297.16 $[M+H]^+$, 295.16 $[M-H]^-$

**Example 12-859 Compound 859**

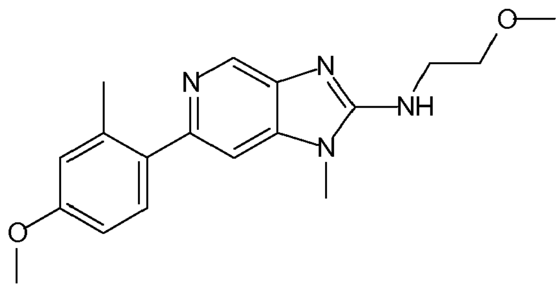

MW.: 326.17, LCMS-ESI (*m*/*z*) - found 327.17 $[M+H]^+$, 325.17 $[M-H]^-$

**Example 12-860 Compound 860**

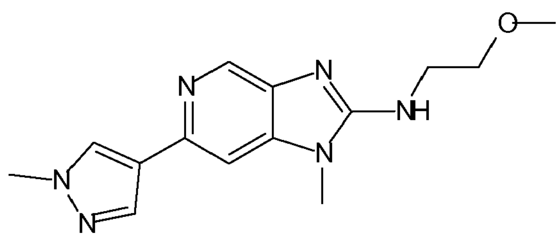

MW.: 286.15, LCMS-ESI (*m*/*z*) - found 287.15 $[M+H]^+$, 285.15 $[M-H]^-$

**Example 12-861 Compound 861**

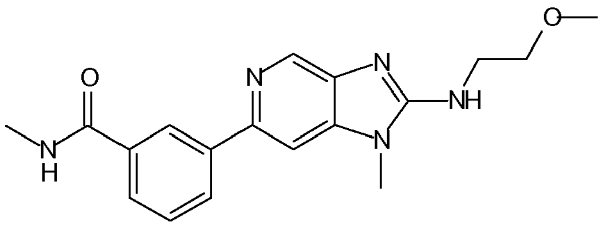

MW.: 339.17, LCMS-ESI ($m/z$) - found 340.17 $[M+H]^+$, 338.17 $[M-H]^-$

**Example 12-862 Compound 862**

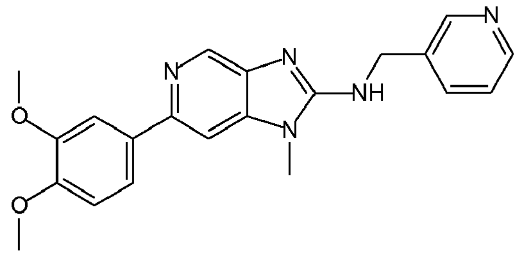

MW.: 375.17, LCMS-ESI ($m/z$) - found 376.17 $[M+H]^+$, 374.17 $[M-H]^-$

**Example 12-863 Compound 863**

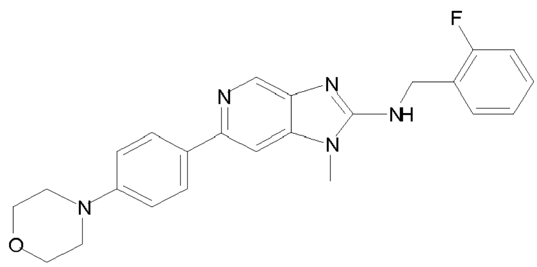

MW.: 417.20, LCMS-ESI ($m/z$) - found 418.19 $[M+H]^+$, 416.18 $[M-H]^-$

**Example 12-864 Compound 864**

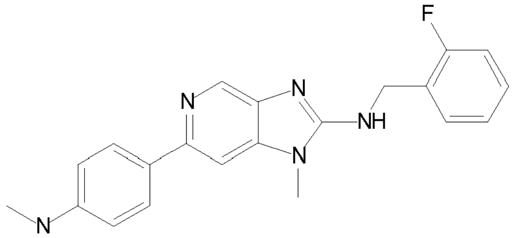

MW.: 375.19, LCMS-ESI (*m*/*z*) - found 376.18 $[M+H]^+$, 374.18 $[M-H]^-$

**Example 12-865 Compound 865**

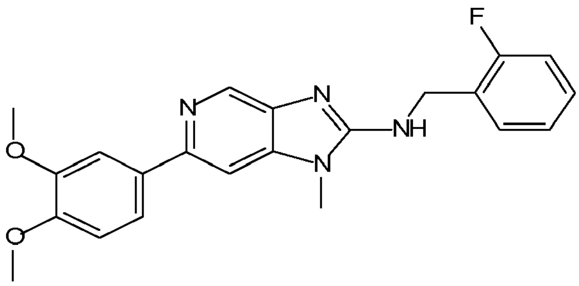

MW.: 392.16, LCMS-ESI (*m*/*z*) - found 393.16 $[M+H]^+$, 391.16 $[M-H]^-$

**Example 12-866 Compound 866**

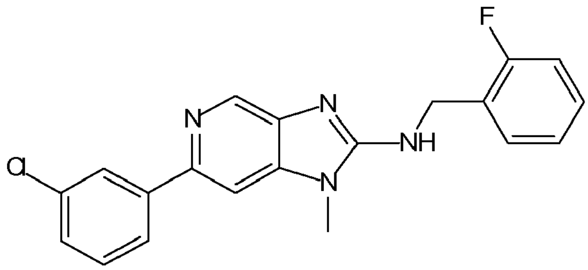

MW.: 366.10, LCMS-ESI (*m*/*z*) - found 367.10 $[M+H]^+$, 365.10 $[M-H]^-$

**Example 12-867 Compound 867**

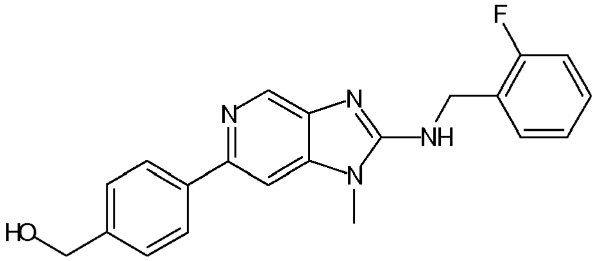

MW.: 362.15, LCMS-ESI ($m/z$) - found 363.15 $[M+H]^+$, 361.15 $[M-H]^-$

**Example 12-868 Compound 868**

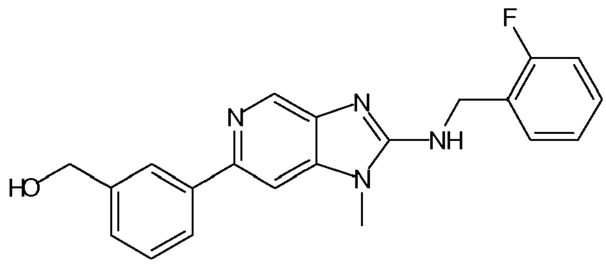

MW.: 362.15, LCMS-ESI ($m/z$) - found 363.15 $[M+H]^+$, 361.15 $[M-H]^-$

**Example 12-869 Compound 869**

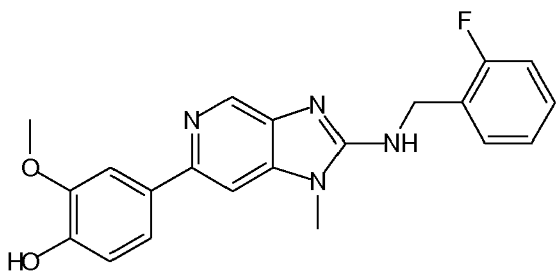

MW.: 378.15, LCMS-ESI ($m/z$) - found 379.14 $[M+H]^+$, 377.14 $[M-H]^-$

**Example 12-870 Compound 870**

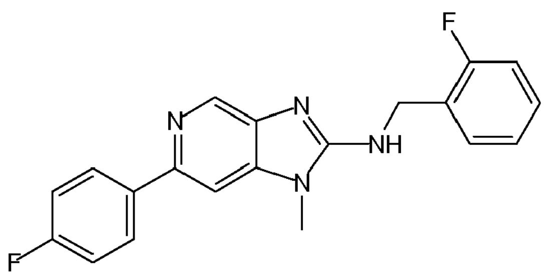

MW.: 350.13, LCMS-ESI ($m/z$) - found 351.13 $[M+H]^+$, 349.13 $[M-H]^-$

**Example 12-871 Compound 871**

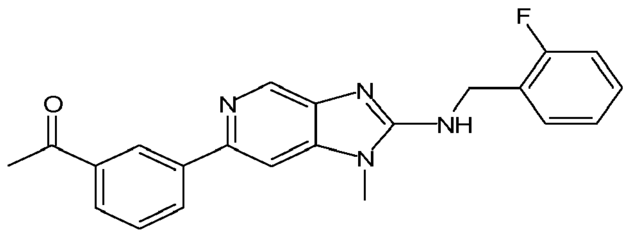

MW.: 374.15, LCMS-ESI (*m/z*) - found 375.15 $[M+H]^+$, 373.15 $[M-H]^-$

**Example 12-872 Compound 872**

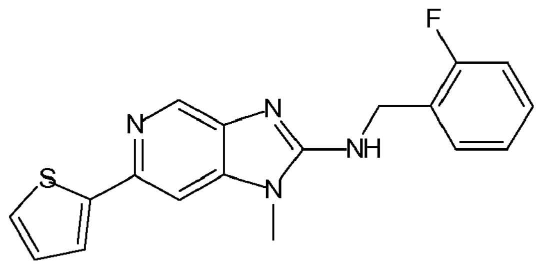

MW.: 338.10, LCMS-ESI (*m/z*) - found 339.10 $[M+H]^+$, 337.10 $[M-H]^-$

**Example 12-873 Compound 873**

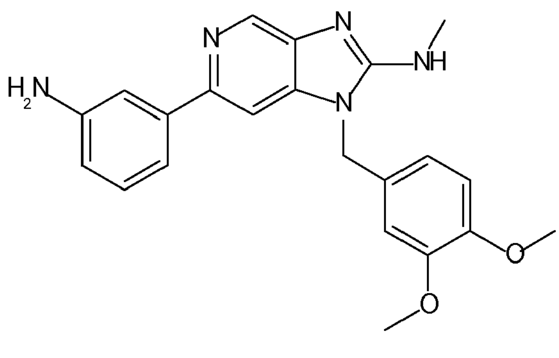

MW.: 389.19, LCMS-ESI (*m/z*) - found 390.19 $[M+H]^+$, 388.19 $[M-H]^-$

**Example 12-874 Compound 874**

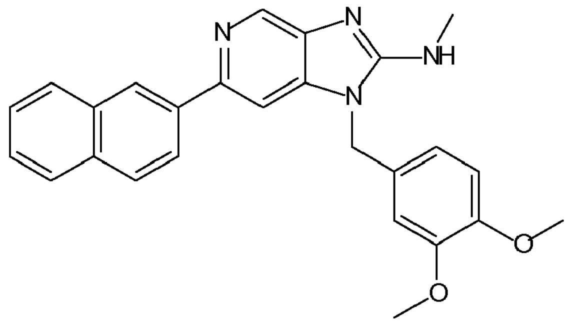

MW.: 424.19, LCMS-ESI ($m/z$) - found 425.19 $[M+H]^+$, 423.19 $[M-H]^-$

**Example 12-875 Compound 875**

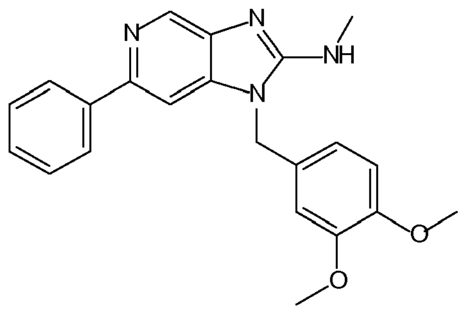

MW.: 374.17, LCMS-ESI ($m/z$) - found 375.17 $[M+H]^+$, 373.17 $[M-H]^-$

**Example 12-876 Compound 876**

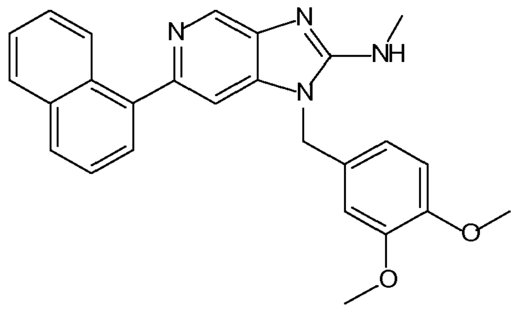

MW.: 424.19, LCMS-ESI ($m/z$) - found 425.19 $[M+H]^+$, 423.19 $[M-H]^-$

**Example 12-877 Compound 877**

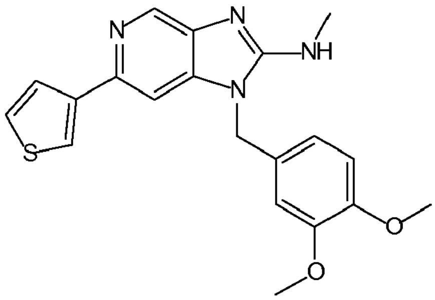

MW.: 380.13, LCMS-ESI (*m/z*) - found 381.13 $[M+H]^+$, 379.13 $[M-H]^-$

**Example 12-878 Compound 878**

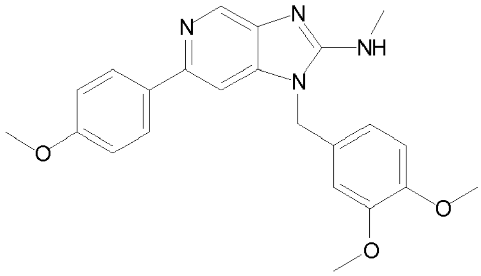

MW.: 404.18, LCMS-ESI (*m/z*) - found 405.18 $[M+H]^+$, 403.18 $[M-H]^-$

**Example 12-879 Compound 879**

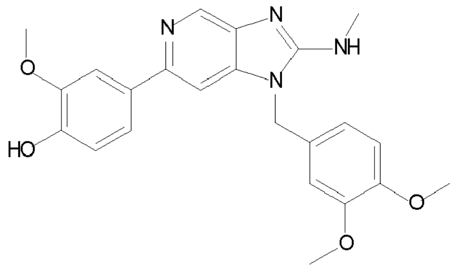

MW.: 420.18, LCMS-ESI (*m/z*) - found 421.18 $[M+H]^+$, 419.18 $[M-H]^-$

**Example 12-880 Compound 880**

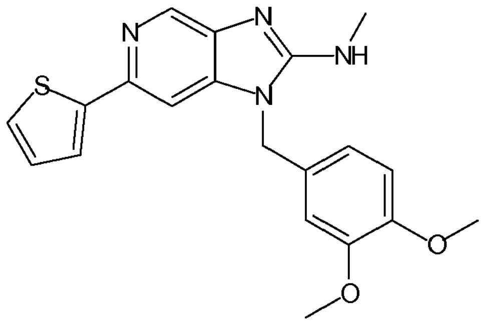

MW.: 380.13, LCMS-ESI (*m*/*z*) - found 381.13 $[M+H]^+$, 379.13 $[M-H]^-$

**Example 12-881 Compound 881**

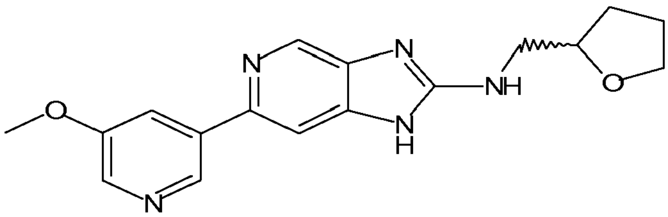

MW.: 325.15, LCMS-ESI (*m*/*z*) - found 326.15 $[M+H]^+$, 324.15 $[M-H]^-$

**Example 12-882 Compound 882**

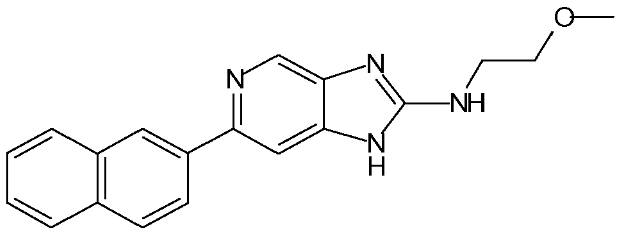

MW.: 318.15, LCMS-ESI (*m*/*z*) - found 319.14 $[M+H]^+$, 317.14 $[M-H]^-$

**Example 12-883 Compound 883**

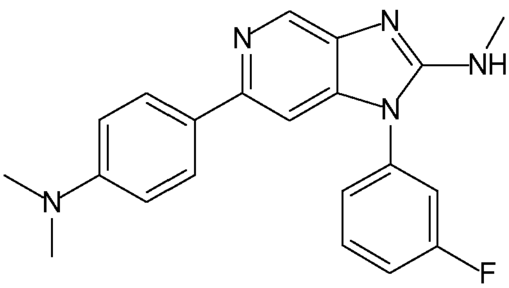

MW.: 361.17, LCMS-ESI (*m/z*) - found 362.17 $[M+H]^+$, 360.17 $[M-H]^-$

**Example 12-884 Compound 884**

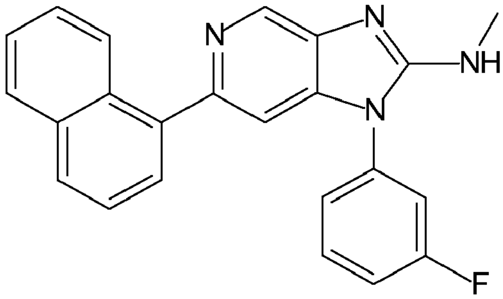

MW.: 368.14, LCMS-ESI (*m/z*) - found 369.14 $[M+H]^+$, 367.14 $[M-H]^-$

**Example 12-885 Compound 885**

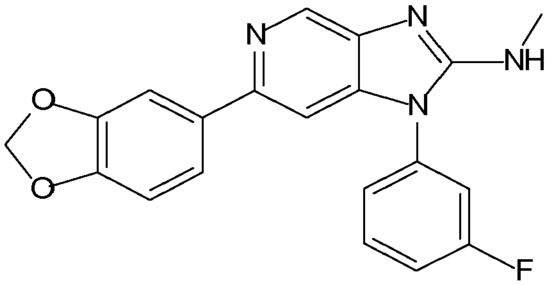

MW.: 362.12, LCMS-ESI (*m/z*) - found 363.11 $[M+H]^+$, 361.11 $[M-H]^-$

**Example 12-886 Compound 886**

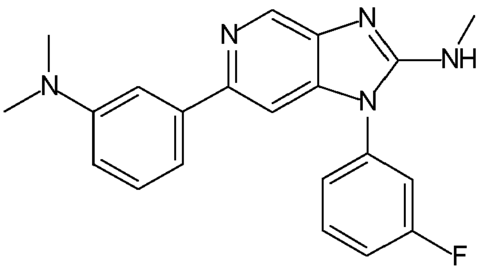

MW.: 361.17, LCMS-ESI (*m/z*) - found 362.17 $[M+H]^+$, 360.17 $[M-H]^-$

**Example 12-887 Compound 887**

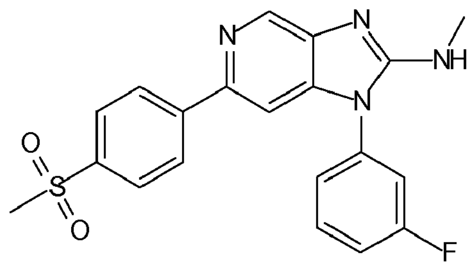

MW.: 396.11, LCMS-ESI (*m*/*z*) - found 397.10 $[M+H]^+$, 395.10 $[M-H]^-$

**Example 12-888 Compound 888**

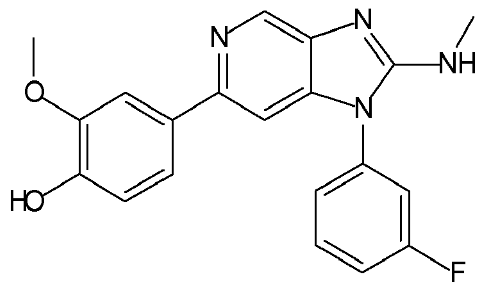

MW.: 364.13, LCMS-ESI (*m*/*z*) - found 365.13 $[M+H]^+$, 363.13 $[M-H]^-$

**Example 12-889 Compound 889**

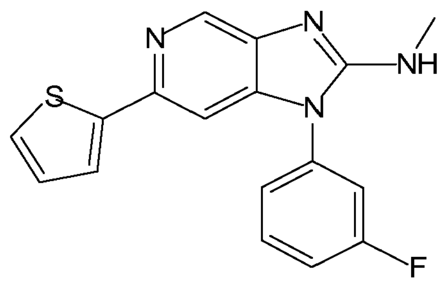

MW.: 324.08, LCMS-ESI (*m*/*z*) - found 325.08 $[M+H]^+$, 323.08 $[M-H]^-$

## Claims

**1.** Compound of the general formula (**I**)

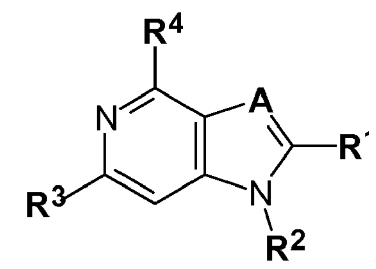

**(I)**

wherein

**A** represents C-H or N;

$\mathbf{R^1}$ represents $-(CH_2)_n-\mathbf{R^5}$ or $-NH-(CH_2)_n-\mathbf{R^5}$;

$\mathbf{R^2}$ represents -H, $-CH_3$, $-(CH_2)_k-O-CH_3$, $-(CH_2)_k-NHCOCH_3$, $-(CH_2)_k$-cyclo-$C_3H_5$, $-(CH_2)_k$-Ph, or $-(CH_2)_k-R^*$;

**R*** represents

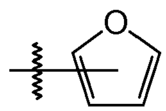,
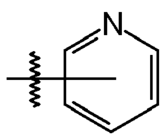,
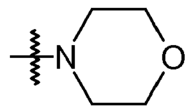,
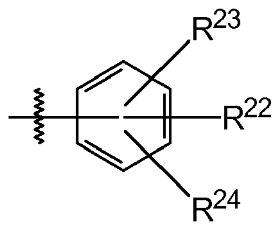;

$\mathbf{R^3}$ represents $-(CH_2)_m-\mathbf{R^6}$ or $-N\mathbf{R}^7((CH_2)_m-\mathbf{R^6})$;

$\mathbf{R^4}$ represents $-(CH_2)_p-\mathbf{R^8}$ or $-N\mathbf{R^9}((CH_2)_p-\mathbf{R^8})$, wherein one of $\mathbf{R^3}$ and $\mathbf{R^4}$ represents -H;

$\mathbf{R^5}$, $\mathbf{R^6}$ and $\mathbf{R^8}$ are independently of each other -H, -F, -Cl, -Br, -I, -CN, $-NO_2$, $-NHCH_3$, $-N(CH_3)_2$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_8-OH$, $-C_5H_{10}-OH$, $-C_6H_{12}-OH$, $-C_7H_{14}-OH$, $-C_8H_{16}-OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$,$-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-COCH_3$, $-COC_2H_5$, $-COC_3H_7$, -CO-cyclo-$C_3H_5$, $-COCH(CH_3)_2$, $-COC(CH_3)_3$, -COOH, $-SO_3H$, $-OCF_3$, $-CH_2-OCF_3$, $-C_2H_4-OCF_3$, $-C_3H_6OCF_3$, $-OC_2F_5$, $-COOCH_3$, $-COOC_2H_5$, $-COOC_3H_7$, -COO-cyclo-$C_3H_5$, $-COOCH(CH_3)_2$, $-COOC(CH_3)_3$, $-OOC-CH_3$, $-OOC-C_2H_5$, $-OOC-C_3H_7$, -OOC-cyclo-$C_3H_5$, $-OOC-CH(CH_3)_2$, $-OOC-C(CH_3)_3$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, -NHCO-O-cyclo-$C_3H_5$, $-NHCO-OCH(CH_3)_2$, $-NHCO-OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $\mathbf{-R^{10}}$, $\mathbf{-R^{11}}$,

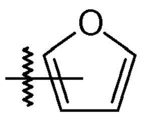,
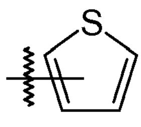,
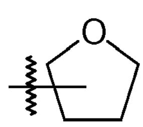,

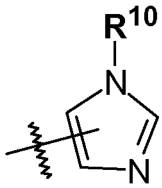 , 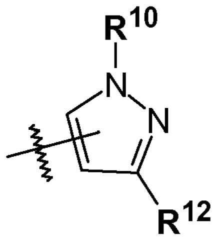 , 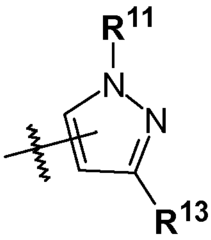 ,

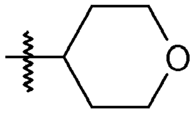 , 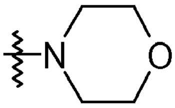 , 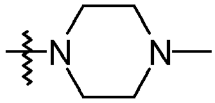 ,

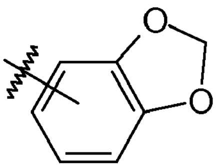 , 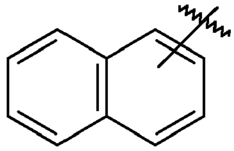 , 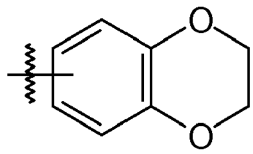 ,

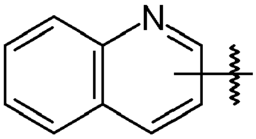 , 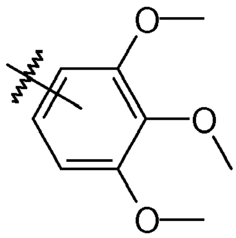 , 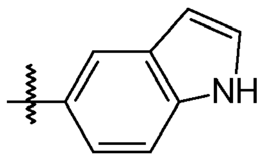 ,

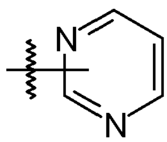 , 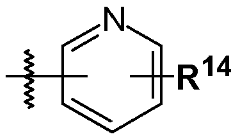 , 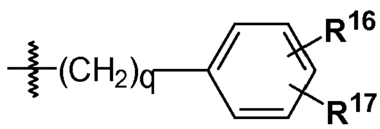 ,

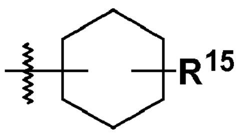 ;

**$R^7$** and **$R^9$** are independently of each other -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, or $-C(CH_3)_3$;
**$R^{14}$** and **$R^{15}$** are independently of each other -H, $-NH_2$, -OH, or -OMe; **$R^{16}$** and **$R^{17}$** are independently of each other -H, -F, -Br, -Cl, -OH, or -CN, -**$R^{18}$**, -**$R^{19}$**, -O**$R^{18}$**, -O**$R^{19}$**, $-CH_2OH$, $-CH_2NH_2$, $-CH_2CN$, $-CH_2N($**$R^{18}$**$)_2$, $-CH_2N($**$R^{19}$**$)_2$, $-CH_2NH($**$R^{18}$**$)$, $-CH_2NH($**$R^{19}$**$)$, $-O(CH_2)_3N(CH_3)_2$, $-SCH_3$, $-NH_2$, -NH(**$R^{18}$**), -NH(**$R^{19}$**), -N**$R^{18}$**CO**$R^{19}$**, $-NHSO_2CH_3$, -N(**$R^{18}$**)$_2$, -N(**$R^{19}$**)$_2$, $-SO_2CH_3$, $-SO_2NH_2$, $-CH_2CO_2H$, $-C_2H_4CO_2H$, $-CH=CH-CO_2H$, -CO**$R^{20}$**,

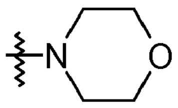, 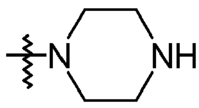, 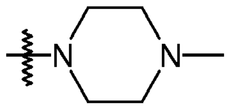;

$\mathbf{R^{20}}$ is -OH, $\mathbf{-R^{21}}$, $\mathbf{-OR^{21}}$, $-NH_2$, $-NH\mathbf{R^{21}}$, $-N(\mathbf{R^{21}})_2$, $-NHC_2H_4OH$, $-NHC_2H_4OCH_3$, $-NH(CH_2)_rN(\mathbf{R^{21}})_2$,

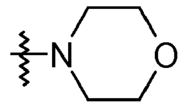, 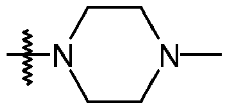;

$\mathbf{R^{10}}$, $\mathbf{R^{11}}$, $\mathbf{R^{12}}$, $\mathbf{R^{13}}$, $\mathbf{R^{18}}$, $\mathbf{R^{19}}$, and $\mathbf{R^{21}}$ are independently of each other

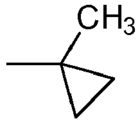

cyclo-$C_3H_5$, cyclo-$C_4H_7$, cyclo-$C_5H_9$, cyclo-$C_6H_{11}$, cyclo-$C_7H_{13}$, -H, $-CH_2-OCH_3$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2-OC_3H_7$, $-C_2H_4-OC_3H_7$, $-C_3H_6-OC_3H_7$,- $CH_2$-O-cyclo-$C_3H_5$, $-C_2H_4$-O-cyclo-$C_3H_5$, $-C_3H_6$-O-cyclo-$C_3H_5$, $-CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$, $-CH_2-OC_4H_9$, $-C_2H_4-OC_4H_9$, $-C_3H_6-OC_4H_9$, $-CH_2$-OPh, $-C_2H_4$-OPh, $-C_3H_6$-OPh, $-CH_2-OCH_2$-Ph, $-C_2H_4-OCH_2$-Ph, $-C_3H_6-OCH_2$-Ph, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_8H_{15}$, -Ph, $-CH_2$-Ph, $-CH_2-CH_2$-Ph, -CH=CH-Ph, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$,$-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-CH(CH_3)Ph$, or $-C(CH_3)_2Ph$;

$\mathbf{R^{22}}$, $\mathbf{R^{23}}$ and $\mathbf{R^{24}}$ represent independently of each other -H, -F, -Cl, -Br, $-OCH_3$, or $-CF_3$;

k is the integer 0, 1 or 2;

m, n, p, q and r are independently of each other an integer from 0 to 3; and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

**2.** Compound of according to claim 1 the general formula (**II**)

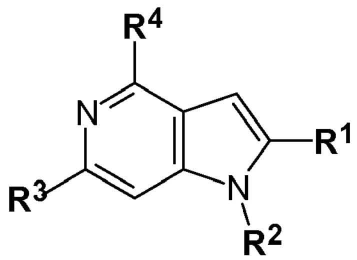

**(II)**

wherein

**$R^1$** represents $-(CH_2)_n-$**$R^5$**;
**$R^2$** represents -H, $-CH_3$, or $-CH_2Ph$;
**$R^3$** represents $-(CH_2)_m-$**$R^6$** or -N**$R^7$**($(CH_2)_m-$**$R^6$**);
**$R^4$** represents $-(CH_2)_p-$**$R^8$** or -N**$R^9$**($(CH_2)_p-$**$R^8$**), wherein one of **$R^3$** and **$R^4$** represents -H;
**$R^5$**, **$R^6$** and **$R^8$** are independently of each other

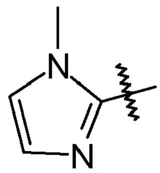 , 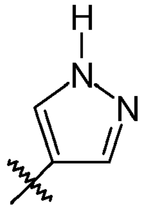 , 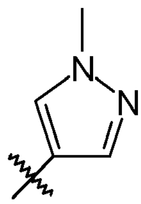 ,

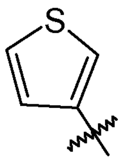 , 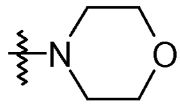 , 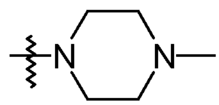 ,

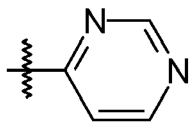 , 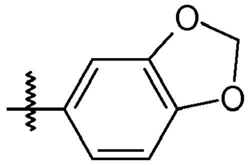 , 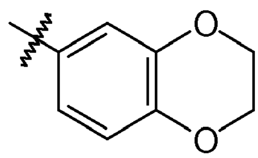 ,

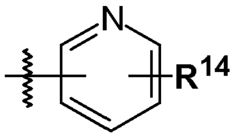 , 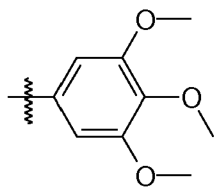 , , 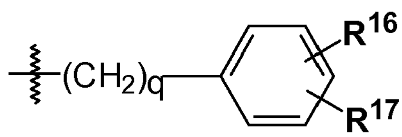 ;

**$R^7$** and **$R^9$** are independently of each other -H or $-CH_3$; **$R^{14}$** is -H or -OMe;
**$R^{16}$** and **$R^{17}$** are independently of each other -H, -F, -Cl, -OH, -CN, -**$R^{18}$**, -**$R^{19}$**, -O**$R^{18}$**, -O**$R^{19}$**, $-SCH_3$, $-NH_2$, -N**$R^{18}$**CO**$R^{19}$**, -N(**$R^{18}$**)$_2$, -N(**$R^{19}$**)$_2$, $-SO_2CH_3$, -CO**$R^{20}$**,

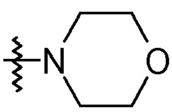 , 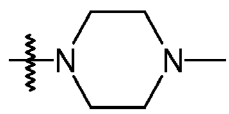 ;

**$R^{20}$** is -**$R^{21}$**, -O**$R^{21}$**, $-NH_2$, or -N(**$R^{21}$**)$_2$;
**$R^{18}$**, **$R^{19}$**, and **$R^{21}$** are independently of each other -H, $-CF_3$, $-CH_3$, $-CH(CH_3)_2$, or -Ph;
m, n, p,and q are independently of each other 0 or 1;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

**3.** Compound according to claim 1 of the general formula (**III**)

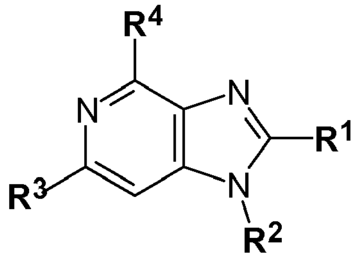

(III)

wherein

$R^1$ represents $-(CH_2)_n-R^5$ or $-NH-(CH_2)_n-R^5$;

$R^2$ represents -H, $-CH_3$, $-(CH_2)_2-O-CH_3$, $-(CH_2)_2-NHCOCH_3$, $-CH_2$-cyclo-$C_3H_5$, -Ph, $-CH_2Ph$,

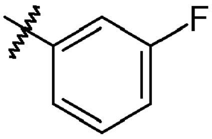 , 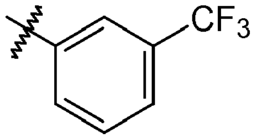 , 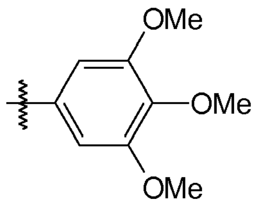 ,

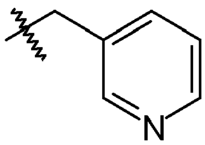 , 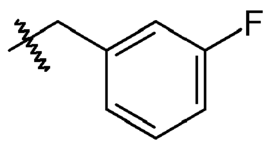 , 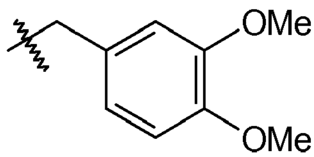 ,

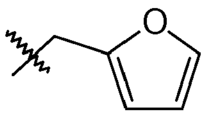 , 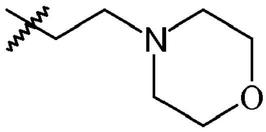 ;

$R^3$ represents $-(CH_2)_m-R^6$ or $-NR^7((CH_2)_m-R^6)$;

$R^4$ represents $-(CH_2)_p-R^8$ or $-NR^9((CH_2)_p-R^8)$, wherein one of $R^3$ and $R^4$ represents -H;

$R^5$, $R^6$ and $R^8$ are independently of each other -H, $-CH(CH_3)_2$, -cyclo-$C_3H_5$, -OH, $-OCH_3$, $-C(CH(CH_3)_2)CH_2OH$,

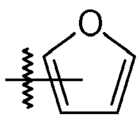 , 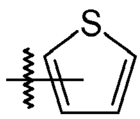 , 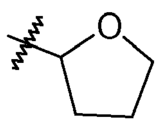 ,

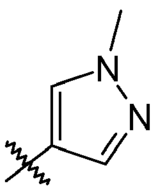 , 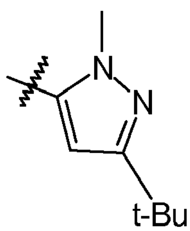 , 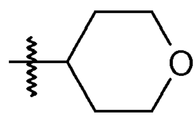 ,

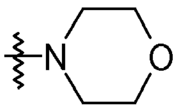 , 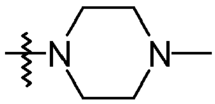 , 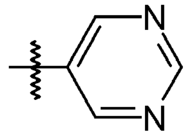 ,

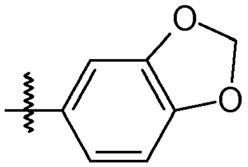 , 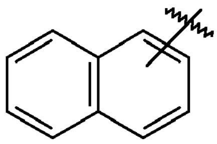 , 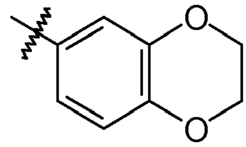 ,

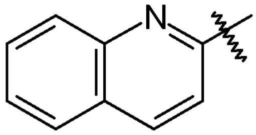 , 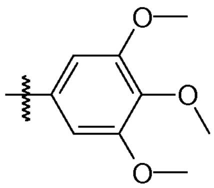 , , 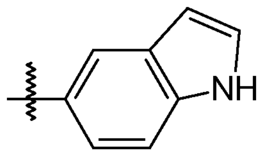 ,

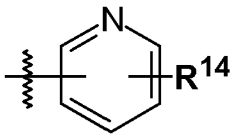 , 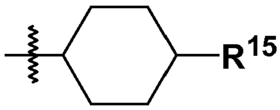 , 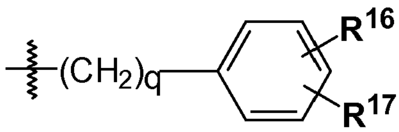 ;

**$R^7$** and **$R^9$** are independently of each other -H or $-CH_3$;
**$R^{14}$** and **$R^{15}$** are independently of each other -H, $-NH_2$, -OH, or -OMe; **$R^{16}$** and **$R^{17}$** are independently of each other -H, -F, -Cl, -OH, -CN, -**$R^{18}$**, -**$R^{19}$**, -O**$R^{18}$**, -O**$R^{19}$**, $-CH_2OH$, $-SCH_3$, $-NH_2$, -N**$R^{18}$**CO**$R^{19}$**, $-NHSO_2CH_3$, -N(**$R^{18}$**)$_2$, -N(**$R^{19}$**)$_2$, $-SO_2CH_3$, -CO**$R^{20}$**,

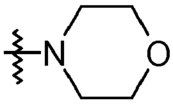 , 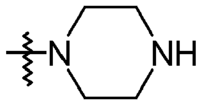 , 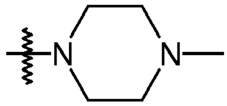 ;

**$R^{20}$** is -OH, -**$R^{21}$**, -O**$R^{21}$**, $-NH_2$, -NH**$R^{21}$**, -N(**$R^{21}$**)$_2$, $-NHC_2H_4OH$, $-NHC_2H_4OCH_3$, or $-NH(CH_2)_rN($**$R^{21}$**$)_2$;
**$R^{10}$**, **$R^{11}$**, **$R^{12}$**, **$R^{13}$**, **$R^{18}$**, **$R^{19}$**, and **$R^{21}$** are independently of each other -H, $-CH_3$, $-C_2H_5$, $-CH(CH_3)_2$, $-CF_3$, or -Ph;
m, n, q and r are independently of each other an integer from 0 to 2, and p is an integer from 0 to 3;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

**4.** Compound according to claim 1 selected from the group consisting of:

| compound | **name** |
|---|---|
| 1 | 2-(3-aminophenyl)-N-(6-methoxy-3-pyridyl)-1H-pyrrolo[3,2-c]pyridin-6-amine |
| 2 | 2-(3-aminophenyl)-N-(3-pyridyl)-1H-pyrrolo[3,2-c]pyridin-6-amine |
| 3 | 2-(3-aminophenyl)-N-[4-(4-methylpiperazin-1-yl)phenyl]-1H-pyrrolo[3,2-c]pyridin-6-amine |

(continued)

| compound | name |
|---|---|
| 4 | 6-(4-methylpiperazin-1-yl)-2-[3-(trifluoromethyl)phenyl]-1H-pyrrolo[3,2-c]pyridine |
| 5 | N-[3-[[2-[3-(trifluoromethyl)phenyl]-1H-pyrrolo[3,2-c]pyridin-6-yl]amino]phenyl]methanesulfonamide |
| 6 | 4-[[2-(3-fluorophenyl)-1H-pyrrolo[3,2-c]pyridin-6-yl]amino]benzamide |
| 7 | N-(3,4-dimethoxyphenyl)-2-(3-fluorophenyl)-1H-pyrrolo[3,2-c]pyridin-6-amine |
| 8 | 2-(3-fluorophenyl)-N-(6-methoxy-3-pyridyl)-1H-pyrrolo[3,2-c]pyridin-6-amine |
| 9 | N-(3-chloro-4-fluoro-phenyl)-2-(3-fluorophenyl)-1H-pyrrolo[3,2-c]pyridin-6-amine |
| 10 | 3-[[2-(3-fluorophenyl)-1H-pyrrolo[3,2-c]pyridin-6-yl]amino]phenol |
| 11 | 5-[[2-(3-fluorophenyl)-1H-pyrrolo[3,2-c]pyridin-6-yl]amino]-2-methoxyphenol |
| 12 | 4-[2-(3-fluorophenyl)-1H-pyrrolo[3,2-c]pyridin-6-yl]morpholine |
| 13 | N-[3-[[2-(3-fluorophenyl)-1H-pyrrolo[3,2-c]pyridin-6-yl]amino]phenyl]methanesulfonamide |
| 14 | 4-[[2-(3-fluorophenyl)-1H-pyrrolo[3,2-c]pyridin-6-yl]amino]-N,N-dimethyl-benzamide |
| 15 | N-[3-[[2-(3-fluorophenyl)-1H-pyrrolo[3,2-c]pyridin-6-yl]amino]phenyl]acetamide |
| 16 | 2-(3-fluorophenyl)-N-(4-methylsulfonylphenyl)-1H-pyrrolo[3,2-c]pyridin-6-amine |
| 17 | 4-[[2-(3-fluorophenyl)-1-methyl-pyrrolo[3,2-c]pyridin-6-yl]amino]phenol |
| 18 | N,N-dimethyl-4-[6-(3,4,5-trimethoxyanilino)-1H-pyrrolo[3,2-c]pyridin-2-yl]benzamide |
| 19 | 4-[6-(3-methoxyanilino)-1H-pyrrolo[3,2-c]pyridin-2-yl]-N,N-dimethyl-benzamide |
| 20 | 4-[6-(2,4-dimethoxyanilino)-1H-pyrrolo[3,2-c]pyridin-2-yl]-N,N-dimethyl-benzamide |
| 21 | 4-[6-(3,4-dimethoxyanilino)-1H-pyrrolo[3,2-c]pyridin-2-yl]-N,N-dimethyl-benzamide |
| 22 | N,N-dimethyl-4-[6-(3-pyridylamino)-1H-pyrrolo[3,2-c]pyridin-2-yl]benzamide |
| 23 | 4-[6-(3-chloroanilino)-1H-pyrrolo[3,2-c]pyridin-2-yl]-N,N-dimethyl-benzamide |
| 24 | 4-(6-anilino-1 H-pyrrolo[3,2-c]pyridin-2-yl)-N,N-dimethyl-benzamide |
| 25 | N,N-dimethyl-4-[6-(4-phenoxyanilino)-1H-pyrrolo[3,2-c]pyridin-2-yl]benzamide |
| 26 | N,N-dimethyl-4-[6-[4-(4-methylpiperazin-1-yl)anilino]-1H-pyrrolo[3,2-c]pyridin-2-yl]benzamide |
| 27 | 4-[6-[3-(dimethylamino)anilino]-1H-pyrrolo[3,2-c]pyridin-2-yl]-N,N-dimethyl-benzamide |
| 28 | N,N-dimethyl-4-[6-(3-methylanilino)-1H-pyrrolo[3,2-c]pyridin-2-yl]benzamide |
| 29 | methyl 4-[[2-[4-(dimethylcarbamoyl)phenyl]-1H-pyrrolo[3,2-c]pyridin-6-yl]amino]benzoate |
| 30 | N,N-dimethyl-4-[6-(3-methylsulfonylanilino)-1H-pyrrolo[3,2-c]pyridin-2-yl]benzamide |
| 31 | 4-[6-(3-hydroxyanilino)-1H-pyrrolo[3,2-c]pyridin-2-yl]-N,N-dimethyl-benzamide |
| 32 | N,N-dimethyl-4-[6-[4-(trifluoromethyl)anilino]-1H-pyrrolo[3,2-c]pyridin-2-yl]benzamide |
| 33 | N,N-dimethyl-4-[6-[3-(trifluoromethoxy)anilino]-1H-pyrrolo[3,2-c]pyridin-2-yl]benzamide |
| 34 | N,N-dimethyl-4-[6-[4-(trifluoromethoxy)anilino]-1H-pyrrolo[3,2-c]pyridin-2-yl]benzamide |
| 35 | N,N-dimethyl-4-[6-(3-phenoxyanilino)-1H-pyrrolo[3,2-c]pyridin-2-yl]benzamide |
| 36 | 4-[6-(3-isopropylanilino)-1H-pyrrolo[3,2-c]pyridin-2-yl]-N,N-dimethyl-benzamide |
| 37 | 4-[6-(2-chloroanilino)-1H-pyrrolo[3,2-c]pyridin-2-yl]-N,N-dimethyl-benzamide |
| 38 | 4-[6-(4-isopropylanilino)-1H-pyrrolo[3,2-c]pyridin-2-yl]-N,N-dimethyl-benzamide |
| 39 | 4-[6-[3-(methanesulfonamido)anilino]-1H-pyrrolo[3,2-c]pyridin-2-yl]-N,N-dimethyl-benzamide |
| 40 | 4-[6-[4-(dimethylcarbamoyl)anilino]-1H-pyrrolo[3,2-c]pyridin-2-yl]-N,N-dimethyl-benzamide |
| 41 | 4-[6-(3-acetamidoanilino)-1H-pyrrolo[3,2-c]pyridin-2-yl]-N,N-dimethyl-benzamide |

(continued)

| compound | name |
|---|---|
| 42 | 4-[6-(3-acetylanilino)-1H-pyrrolo[3,2-c]pyridin-2-yl]-N,N-dimethyl-benzamide |
| 43 | N,N-dimethyl-4-[6-(4-methylsulfonylanilino)-1H-pyrrolo[3,2-c]pyridin-2-yl]benzamide |
| 44 | 4-[6-(3-isopropoxyanilino)-1H-pyrrolo[3,2-c]pyridin-2-yl]-N,N-dimethyl-benzamide |
| 45 | 4-[6-(3-methoxyanilino)-1-methyl-pyrrolo[3,2-c]pyridin-2-yl]-N,N-dimethyl-benzamide |
| 46 | 4-[6-(3,4-dimethoxyanilino)-1-methyl-pyrrolo[3,2-c]pyridin-2-yl]-N,N-dimethyl-benzamide |
| 47 | N, N-d i methyl-4-[1-methyl-6-(3-pyridylam ino)pyrrolo[3,2-c] pyrid in-2-yl]benzamide |
| 48 | 4-(6-anilino-1-methyl-pyrrolo[3,2-c]pyridin-2-yl)-N,N-dimethyl-benzamide |
| 49 | N,N-dimethyl-4-[1-methyl-6-[4-(4-methylpiperazin-1-yl)anilino]pyrrolo[3,2-c]pyridin-2-yl]benzamide |
| 50 | 4-[6-[3-(dimethylamino)anilino]-1-methyl-pyrrolo[3,2-c]pyridin-2-yl]-N,N-dimethyl-benzamide |
| 51 | N, N-d i methyl-4-[1-methyl-6-(2-pyridylam ino)pyrrolo[3,2-c] pyrid in-2-yl]benzamide |
| 52 | N, N-d i methyl-4-[1-methyl-6-(N-methylan il ino)pyrrolo[3,2-c] pyrid in-2-yl]benzamide |
| 53 | 4-[6-(3-hydroxyanilino)-1-methyl-pyrrolo[3,2-c]pyridin-2-yl]-N,N-dimethyl-benzamide |
| 54 | 4-[6-(3-hydroxy-4-methoxy-anilino)-1-methyl-pyrrolo[3,2-c]pyridin-2-yl]-N,N-dimethyl-benzamide |
| 55 | 4-[6-[3-(methanesulfonamido)anilino]-1-methyl-pyrrolo[3,2-c]pyridin-2-yl]-N,N-dimethyl-benzamide |
| 56 | 4-[6-(3-acetamidoanilino)-1-methyl-pyrrolo[3,2-c]pyridin-2-yl]-N,N-dimethyl-benzamide |
| 57 | 4-[6-(4-acetamidoanilino)-1-benzyl-pyrrolo[3,2-c]pyridin-2-yl]-N,N-dimethyl-benzamide |
| 58 | 4-[1-benzyl-6-(pyrim id in-4-ylam i no)pyrrolo[3,2-c] pyrid in-2-yl]-N, N-dimethyl-benzamide |
| 59 | 2-(4-dimethylaminophenyl)-1-methyl-N-(2-pyridyl)pyrrolo[3,2-c]pyridin-6-amine |
| 60 | 3-[[1-benzyl-2-(4-dimethylaminophenyl)pyrrolo[3,2-c]pyridin-6-yl]amino]phenol |
| 61 | 1-benzyl-2-(4-dimethylaminophenyl)-N-pyrimidin-4-yl-pyrrolo[3,2-c]pyridin-6-amine |
| 62 | 2-(2-pyridyl)-N-(3,4,5-trimethoxyphenyl)-1H-pyrrolo[3,2-c]pyridin-6-amine |
| 63 | N-(4-methoxy-2-methyl-phenyl)-2-(2-pyridyl)-1H-pyrrolo[3,2-c]pyridin-6-amine |
| 64 | N-(m-tolyl)-2-(2-pyridyl)-1H-pyrrolo[3,2-c]pyridin-6-amine |
| 65 | N-(4-methoxyphenyl)-2-(2-pyridyl)-1H-pyrrolo[3,2-c]pyridin-6-amine |
| 66 | 2-(2-pyridyl)-N-[3-(trifluoromethyl)phenyl]-1H-pyrrolo[3,2-c]pyridin-6-amine |
| 67 | methyl 4-[[2-(2-pyridyl)-1H-pyrrolo[3,2-c]pyridin-6-yl]amino]benzoate |
| 68 | 4-[[2-(2-pyridyl)-1H-pyrrolo[3,2-c]pyridin-6-yl]amino]benzonitrile |
| 69 | 2-(2-pyridyl)-N-[3-(trifluoromethoxy)phenyl]-1H-pyrrolo[3,2-c]pyridin-6-amine |
| 70 | N,N-dimethyl-4-[[2-(2-pyridyl)-1H-pyrrolo[3,2-c]pyridin-6-yl]amino]benzamide |
| 71 | N-(3-fluorophenyl)-2-(2-pyridyl)-1H-pyrrolo[3,2-c]pyridin-6-amine |
| 72 | N-(4-methylsulfonylphenyl)-2-(2-pyridyl)-1H-pyrrolo[3,2-c]pyridin-6-amine |
| 73 | N-(3-isopropoxyphenyl)-2-(2-pyridyl)-1H-pyrrolo[3,2-c]pyridin-6-amine |
| 74 | N-[4-[[2-(3-pyridyl)-1H-pyrrolo[3,2-c]pyridin-6-yl]amino]phenyl]acetamide |
| 75 | N-(6-methoxy-3-pyridyl)-2-(3-pyridyl)-1H-pyrrolo[3,2-c]pyridin-6-amine |
| 76 | 2-(3-pyridyl)-N-(4-pyridyl)-1H-pyrrolo[3,2-c]pyridin-6-amine |
| 77 | 4-[2-(3-pyridyl)-1H-pyrrolo[3,2-c]pyridin-6-yl]morpholine |
| 78 | N-(2-chlorophenyl)-2-(3-pyridyl)-1H-pyrrolo[3,2-c]pyridin-6-amine |
| 79 | N-[2-fluoro-5-(trifluoromethyl)phenyl]-2-(3-pyridyl)-1H-pyrrolo[3,2-c]pyridin-6-amine |

(continued)

| compound | name |
|---|---|
| 80 | N-[3-[[2-(3-pyridyl)-1H-pyrrolo[3,2-c]pyridin-6-yl]amino]phenyl]acetamide |
| 81 | N-(4-isopropoxyphenyl)-2-(3-pyridyl)-1H-pyrrolo[3,2-c]pyridin-6-amine |
| 82 | 1-methyl-2-(3-pyridyl)-N-(3,4,5-trimethoxyphenyl)pyrrolo[3,2-c]pyridin-6-amine |
| 83 | N-(2,4-dimethoxyphenyl)-1-methyl-2-(3-pyridyl)pyrrolo[3,2-c]pyridin-6-amine |
| 84 | N-(2,3-dihydro-1,4-benzodioxin-6-yl)-1-methyl-2-(3-pyridyl)pyrrolo[3,2-c]pyridin-6-amine |
| 85 | N-(3,4-dimethoxyphenyl)-1-methyl-2-(3-pyridyl)pyrrolo[3,2-c]pyridin-6-amine |
| 86 | N-(6-methoxy-3-pyridyl)-1-methyl-2-(3-pyridyl)pyrrolo[3,2-c]pyridin-6-amine |
| 87 | 1-methyl-N,2-bis(3-pyridyl)pyrrolo[3,2-c]pyridin-6-amine |
| 88 | 1-methyl-N-phenyl-2-(3-pyridyl)pyrrolo[3,2-c]pyridin-6-amine |
| 89 | N1,N1-dimethyl-N3-[1-methyl-2-(3-pyridyl)pyrrolo[3,2-c]pyridin-6-yl]benzene-1,3-diamine |
| 90 | 1-methyl-N-(m-tolyl)-2-(3-pyridyl)pyrrolo[3,2-c]pyridin-6-amine |
| 91 | N-(4-methoxyphenyl)-1-methyl-2-(3-pyridyl)pyrrolo[3,2-c]pyridin-6-amine |
| 92 | N-(4-fluorophenyl)-1-methyl-2-(3-pyridyl)pyrrolo[3,2-c]pyridin-6-amine |
| 93 | N-(1,3-benzodioxol-5-yl)-1-methyl-2-(3-pyridyl)pyrrolo[3,2-c]pyridin-6-amine |
| 94 | 4-[1-methyl-2-(3-pyridyl)pyrrolo[3,2-c]pyridin-6-yl]morpholine |
| 95 | N,N-dimethyl-4-[[1-methyl-2-(3-pyridyl)pyrrolo[3,2-c]pyridin-6-yl]amino]benzamide |
| 96 | N-[3-[[1-methyl-2-(3-pyridyl)pyrrolo[3,2-c]pyridin-6-yl]amino]phenyl]acetamide |
| 97 | 1-[3-[[1-methyl-2-(3-pyridyl)pyrrolo[3,2-c]pyridin-6-yl]amino]phenyl]ethanone |
| 98 | 1-benzyl-N-(2-pyridyl)-2-(3-pyridyl)pyrrolo[3,2-c]pyridin-6-amine |
| 99 | 1-benzyl-2-(3-pyridyl)-N-pyrimidin-4-yl-pyrrolo[3,2-c]pyridin-6-amine |
| 100 | 2-(3-methylimidazol-4-yl)-N-(3,4,5-trimethoxyphenyl)-1H-pyrrolo[3,2-c]pyridin-6-amine |
| 101 | N-(3-methoxyphenyl)-2-(3-methylimidazol-4-yl)-1H-pyrrolo[3,2-c]pyridin-6-amine |
| 102 | N-(3,4-dimethoxyphenyl)-2-(3-methylimidazol-4-yl)-1H-pyrrolo[3,2-c]pyridin-6-amine |
| 103 | N-(3-chlorophenyl)-2-(3-methylimidazol-4-yl)-1H-pyrrolo[3,2-c]pyrid in-6-amine |
| 104 | N1,N1-dimethyl-N3-[2-(3-methylimidazol-4-yl)-1H-pyrrolo[3,2-c]pyridin-6-yl]benzene-1,3-diamine |
| 105 | 2-(3-methylimidazol-4-yl)-N-(m-tolyl)-1H-pyrrolo[3,2-c]pyridin-6-amine |
| 106 | N-(4-fluorophenyl)-2-(3-methylimidazol-4-yl)-1H-pyrrolo[3,2-c]pyridin-6-amine |
| 107 | 1-[3-[[2-(3-methylimidazol-4-yl)-1H-pyrrolo[3,2-c]pyridin-6-yl]amino]phenyl]ethanone |
| 108 | N-(3-fluorophenyl)-2-(3-methylimidazol-4-yl)-1H-pyrrolo[3,2-c]pyridin-6-amine |
| 109 | N-(4-isopropoxyphenyl)-2-(3-methylimidazol-4-yl)-1H-pyrrolo[3,2-c]pyridin-6-amine |
| 110 | N-(3-methoxyphenyl)-2-(1H-pyrazol-4-yl)-1H-pyrrolo[3,2-c]pyridin-6-amine |
| 111 | N-(3,4-dimethoxyphenyl)-2-(1H-pyrazol-4-yl)-1H-pyrrolo[3,2-c]pyridin-6-amine |
| 112 | N1,N1-dimethyl-N3-[2-(1H-pyrazol-4-yl)-1H-pyrrolo[3,2-c]pyridin-6-yl]benzene-1,3-diamine |
| 113 | N-(m-tolyl)-2-(1H-pyrazol-4-yl)-1H-pyrrolo[3,2-c]pyridin-6-amine |
| 114 | N-(4-fluorophenyl)-2-(1H-pyrazol-4-yl)-1H-pyrrolo[3,2-c]pyridin-6-amine |
| 115 | 2-(1H-pyrazol-4-yl)-N-(2-pyridyl)-1H-pyrrolo[3,2-c]pyridin-6-amine |
| 116 | N-[3-methoxy-5-(trifluoromethyl)phenyl]-2-(1H-pyrazol-4-yl)-1H-pyrrolo[3,2-c]pyridin-6-amine |
| 117 | N-[3-[[2-(1H-pyrazol-4-yl)-1H-pyrrolo[3,2-c]pyridin-6-yl]amino]phenyl]acetamide |

(continued)

| compound | name |
|---|---|
| 118 | 1-[3-[[2-(1 H-pyrazol-4-yl)-1H-pyrrolo[3,2-c]pyridin-6-yl]amino]phenyl]ethanone |
| 119 | N-(3-fluorophenyl)-2-(1H-pyrazol-4-yl)-1H-pyrrolo[3,2-c]pyridin-6-amine |
| 120 | N-(4-methylsulfonylphenyl)-2-(1H-pyrazol-4-yl)-1H-pyrrolo[3,2-c]pyridin-6-amine |
| 121 | N-[4-[[2-(1-methylpyrazol-4-yl)-1H-pyrrolo[3,2-c]pyridin-6-yl]amino]phenyl]acetamide |
| 122 | N-(2,3-dihydro-1,4-benzodioxin-6-yl)-2-(1-methylpyrazol-4-yl)-1H-pyrrolo[3,2-c]pyridin-6-amine |
| 123 | N-(3,4-dimethoxyphenyl)-2-(1-methylpyrazol-4-yl)-1H-pyrrolo[3,2-c]pyridin-6-amine |
| 124 | 1-[3-[[2-(1-methylpyrazol-4-yl)-1H-pyrrolo[3,2-c]pyridin-6-yl]amino]phenyl]ethanone |
| 125 | 1-methyl-2-(1-methylpyrazol-4-yl)-N-(3,4,5-trimethoxyphenyl)pyrrolo[3,2-c]pyridin-6-amine |
| 126 | N-[4-[[1-methyl-2-(1-methylpyrazol-4-yl)pyrrolo[3,2-c]pyridin-6-yl]amino]phenyl]acetamide |
| 127 | N-(3-methoxyphenyl)-1-methyl-2-(1-methylpyrazol-4-yl)pyrrolo[3,2-c]pyridin-6-amine |
| 128 | N-(2,3-dihydro-1,4-benzodioxin-6-yl)-1-methyl-2-(1-methylpyrazol-4-yl)pyrrolo[3,2-c]pyridin-6-amine |
| 129 | N-(3,4-dimethoxyphenyl)-1-methyl-2-(1-methylpyrazol-4-yl)pyrrolo[3,2-c]pyridin-6-amine |
| 130 | N-(6-methoxy-3-pyridyl)-1-methyl-2-(1-methylpyrazol-4-yl)pyrrolo[3,2-c]pyridin-6-amine |
| 131 | 1-methyl-2-(1-methylpyrazol-4-yl)-N-(3-pyridyl)pyrrolo[3,2-c]pyridin-6-amine |
| 132 | 1-methyl-2-(1-methylpyrazol-4-yl)-N-phenyl-pyrrolo[3,2-c]pyridin-6-amine |
| 133 | 1-methyl-N-[4-(4-methylpiperazin-1-yl)phenyl]-2-(1-methylpyrazol-4-yl)pyrrolo[3,2-c]pyridin-6-amine |
| 134 | N1,N1-dimethyl-N3-[1-methyl-2-(1-methylpyrazol-4-yl)pyrrolo[3,2-c]pyridin-6-yl]benzene-1,3-diamine |
| 135 | 1-methyl-2-(1-methylpyrazol-4-yl)-N-(4-pyridyl)pyrrolo[3,2-c]pyridin-6-amine |
| 136 | 1-methyl-2-(1-methylpyrazol-4-yl)-N-(m-tolyl)pyrrolo[3,2-c]pyridin-6-amine |
| 137 | N-(4-methoxyphenyl)-1-methyl-2-(1-methylpyrazol-4-yl)pyrrolo[3,2-c]pyridin-6-amine |
| 138 | N-(4-fluorophenyl)-1-methyl-2-(1-methylpyrazol-4-yl)pyrrolo[3,2-c]pyridin-6-amine |
| 139 | 1-methyl-2-(1-methylpyrazol-4-yl)-N-(2-pyridyl)pyrrolo[3,2-c]pyridin-6-amine |
| 140 | N,1-dimethyl-2-(1-methylpyrazol-4-yl)-N-phenyl-pyrrolo[3,2-c]pyridin-6-amine |
| 141 | methyl 4-[[1-methyl-2-(1-methylpyrazol-4-yl)pyrrolo[3,2-c]pyridin-6-yl]amino]benzoate |
| 142 | 4-[[1-methyl-2-(1-methylpyrazol-4-yl)pyrrolo[3,2-c]pyridin-6-yl]amino]benzonitrile |
| 143 | N-(1,3-benzodioxol-5-yl)-1-methyl-2-(1-methylpyrazol-4-yl)pyrrolo[3,2-c]pyridin-6-amine |
| 144 | 3-[[1-methyl-2-(1-methylpyrazol-4-yl)pyrrolo[3,2-c]pyridin-6-yl]amino]phenol |
| 145 | 4-[1-methyl-2-(1-methylpyrazol-4-yl)pyrrolo[3,2-c]pyridin-6-yl]morpholine |
| 146 | 1-methyl-2-(1-methylpyrazol-4-yl)-N-[3-(trifluoromethoxy)phenyl]pyrrolo[3,2-c]pyridin-6-amine |
| 147 | 1-methyl-2-(1-methylpyrazol-4-yl)-N-pyrimidin-4-yl-pyrrolo[3,2-c]pyridin-6-amine |
| 148 | N,N-dimethyl-4-[[1-methyl-2-(1-methylpyrazol-4-yl)pyrrolo[3,2-c]pyridin-6-yl]amino]benzamide |
| 149 | N-[3-[[1-methyl-2-(1-methylpyrazol-4-yl)pyrrolo[3,2-c]pyridin-6-yl]amino]phenyl]acetamide |
| 150 | 1-[3-[[1-methyl-2-(1-methylpyrazol-4-yl)pyrrolo[3,2-c]pyridin-6-yl]amino]phenyl]ethanone |
| 151 | N-(3-fluorophenyl)-1-methyl-2-(1-methylpyrazol-4-yl)pyrrolo[3,2-c]pyridin-6-amine |
| 152 | 1-methyl-2-(1-methylpyrazol-4-yl)-N-(4-methylsulfonylphenyl)pyrrolo[3,2-c]pyridin-6-amine |
| 153 | 1-methyl-2-(1-methylpyrazol-4-yl)-N-(4-methylsulfanylphenyl)pyrrolo[3,2-c]pyridin-6-amine |
| 154 | 1-benzyl-6-(4-methylpiperazin-1-yl)-2-(1-methylpyrazol-4-yl)pyrrolo[3,2-c]pyridine |
| 155 | 4-[1-benzyl-2-(1-methylpyrazol-4-yl)pyrrolo[3,2-c]pyridin-6-yl]morpholine |

(continued)

| compound | name |
|---|---|
| 156 | N-[3-[[1-benzyl-2-(1-methylpyrazol-4-yl)pyrrolo[3,2-c]pyridin-6-yl]amino]phenyl]methanesulfonamide |
| 157 | N-[3-[[1-benzyl-2-(1-methylpyrazol-4-yl)pyrrolo[3,2-c]pyridin-6-yl]amino]phenyl]acetamide |
| 158 | N-methyl-N-[3-[1-methyl-6-(3,4,5-trimethoxyanilino)pyrrolo[3,2-c]pyridin-2-yl]phenyl]acetamide |
| 159 | 4-[[2-[3-[acetyl(methyl)amino]phenyl]-1-methyl-pyrrolo[3,2-c]pyridin-6-yl]amino]benzamide |
| 160 | N-[3-[6-(3,4-dimethoxyanilino)-1-methyl-pyrrolo[3,2-c]pyridin-2-yl]phenyl]-N-methyl-acetamide |
| 161 | N-[3-[6-[(6-methoxy-3-pyridyl)amino]-1-methyl-pyrrolo[3,2-c]pyridin-2-yl]phenyl]-N-methyl-acetamide |
| 162 | N-methyl-N-[3-[1-methyl-6-(3-pyridylam ino)pyrrolo[3,2-c] pyrid in-2-yl]phenyl]acetamide |
| 163 | N-methyl-N-[3-[1-methyl-6-(4-morpholinoanilino)pyrrolo[3,2-c]pyridin-2-yl]phenyl]acetamide |
| 164 | N-methyl-N-[3-[1-methyl-6-[4-(4-methylpiperazin-1-yl)anilino]pyrrolo[3,2-c]pyridin-2-yl]phenyl]acetamide |
| 165 | N-[3-[6-[3-(dimethylamino)anilino]-1-methyl-pyrrolo[3,2-c]pyridin-2-yl]phenyl]-N-methyl-acetamide |
| 166 | N-[3-[6-(4-methoxyanilino)-1-methyl-pyrrolo[3,2-c]pyridin-2-yl]phenyl]-N-methyl-acetamide |
| 167 | N-methyl-N-[3-[1-methyl-6-(2-pyridylam ino)pyrrolo[3,2-c] pyrid in-2-yl]phenyl]acetamide |
| 168 | N-[3-[6-(4-cyanoanilino)-1-methyl-pyrrolo[3,2-c]pyridin-2-yl]phenyl]-N-methyl-acetamide |
| 169 | N-[3-[6-(1,3-benzodioxol-5-ylamino)-1-methyl-pyrrolo[3,2-c]pyridin-2-yl] phenyl]-N-methyl-acetam ide |
| 170 | N-[3-[6-(3-hydroxyanilino)-1-methyl-pyrrolo[3,2-c]pyridin-2-yl]phenyl]-N-methyl-acetamide |
| 171 | N-methyl-N-[3-(1-methyl-6-morpholino-pyrrolo[3,2-c]pyridin-2-yl)phenyl]acetamide |
| 172 | 4-[[2-[3-[acetyl(methyl)amino]phenyl]-1-methyl-pyrrolo[3,2-c]pyridin-6-yl]amino]-N,N-dimethyl-benzamide |
| 173 | N-methyl-N-[3-[1-methyl-6-(4-methylsulfonylanilino)pyrrolo[3,2-c]pyridin-2-yl]phenyl]acetamide |
| 174 | 2-phenyl-N-(3,4,5-trimethoxyphenyl)-1H-pyrrolo[3,2-c]pyridin-6-amine |
| 175 | N-(3,4-dimethoxyphenyl)-2-phenyl-1 H-pyrrolo[3,2-c]pyridin-6-amine |
| 176 | N-(4-morpholinophenyl)-2-phenyl-1H-pyrrolo[3,2-c]pyridin-6-amine |
| 177 | N-[4-(4-methylpiperazin-1-yl)phenyl]-2-phenyl-1H-pyrrolo[3,2-c]pyridin-6-amine |
| 178 | 2-phenyl-N-(4-pyridyl)-1H-pyrrolo[3,2-c]pyridin-6-amine |
| 179 | N-(4-fluorophenyl)-2-phenyl-1-H-pyrrolo[3,2-c]pyridin-6-amine |
| 180 | 3-[(2-phenyl-1-H-pyrrolo[3,2-c]pyridin-6-yl)amino]phenol |
| 181 | 4-(2-phenyl-1-H-pyrrolo[3,2-c]pyridin-6-yl)morpholine |
| 182 | N,N-dimethyl-4-[(2-phenyl-1H-pyrrolo[3,2-c]pyridin-6-yl)amino]benzamide |
| 183 | N-[3-[(2-phenyl-1 H-pyrrolo[3,2-c]pyridin-6-yl)amino]phenyl]acetamide |
| 184 | 1-benzyl-2-phenyl-N-pyrimidin-4-yl-pyrrolo[3,2-c]pyridin-6-amine |
| 185 | 1-methyl-4-(4-methylpiperazin-1-yl)-2-[3-(trifluoromethyl)phenyl]pyrrolo[3,2-c]pyridine |
| 186 | 2-(3-fluorophenyl)-4-(4-methylpiperazin-1-yl)-1H-pyrrolo[3,2-c]pyridine |
| 187 | 4-[2-(3-fluorophenyl)-1H-pyrrolo[3,2-c]pyridin-4-yl]morpholine |
| 188 | N,N-dimethyl-4-(1-methyl-4-morpholino-pyrrolo[3,2-c]pyridin-2-yl)benzamide |
| 189 | N,N-dimethyl-4-(4-morpholino-1 H-pyrrolo[3,2-c]pyridin-2-yl)aniline |
| 190 | 4-(4-methylpiperazin-1-yl)-2-(3-thienyl)-1H-pyrrolo[3,2-c]pyridine |
| 191 | 4-[2-(3-thienyl)-1H-pyrrolo[3,2-c]pyridin-4-yl]morpholine |

(continued)

| compound | name |
|---|---|
| 192 | 4-[2-(3,5-dimethoxyphenyl)-1-methyl-pyrrolo[3,2-c]pyridin-4-yl]morpholine |
| 193 | 4-[2-(2-methoxyphenyl)-1H-pyrrolo[3,2-c]pyridin-4-yl]morpholine |
| 194 | 2-(3-methoxyphenyl)-1-methyl-4-(4-methylpiperazin-1-yl)pyrrolo[3,2-c]pyridine |
| 195 | 4-[2-(3-chlorophenyl)-1-methyl-pyrrolo[3,2-c]pyridin-4-yl]morpholine |
| 196 | N-(3-pyridyl)-2-[3-(trifluoromethyl)phenyl]-1H-pyrrolo[3,2-c]pyridin-4-amine |
| 197 | N-(4-methylsulfonylphenyl)-2-[3-(trifluoromethyl)phenyl]-1H-pyrrolo[3,2-c]pyridin-4-amine |
| 198 | N-[4-[[2-(3-fluorophenyl)-1H-pyrrolo[3,2-c]pyridin-4-yl]amino]phenyl]acetamide |
| 199 | 4-[[2-(3-fluorophenyl)-1H-pyrrolo[3,2-c]pyridin-4-yl]amino]phenol |
| 200 | 2-(3-fluorophenyl)-N-(3-pyridyl)-1H-pyrrolo[3,2-c]pyridin-4-amine |
| 201 | 2-(3-fluorophenyl)-N-[4-(4-methylpiperazin-1-yl)phenyl]-1H-pyrrolo[3,2-c]pyridin-4-amine |
| 202 | 2-(3-fluorophenyl)-N-(3-methylsulfonylphenyl)-1H-pyrrolo[3,2-c]pyridin-4-amine |
| 203 | N-(1,3-benzodioxol-5-yl)-2-(3-fluorophenyl)-1H-pyrrolo[3,2-c]pyridin-4-amine |
| 204 | 5-[[2-(3-fluorophenyl)-1H-pyrrolo[3,2-c]pyridin-4-yl]amino]-2-methoxyphenol |
| 205 | N-[3-[[2-(3-fluorophenyl)-1H-pyrrolo[3,2-c]pyridin-4-yl]amino]phenyl]methanesulfonamide |
| 206 | N-[3-[[2-(3-fluorophenyl)-1H-pyrrolo[3,2-c]pyridin-4-yl]amino]phenyl]acetamide |
| 207 | 2-(3-fluorophenyl)-N-(4-methylsulfonylphenyl)-1H-pyrrolo[3,2-c]pyridin-4-amine |
| 208 | N-(3,4-dimethoxyphenyl)-2-(3-fluorophenyl)-1-methyl-pyrrolo[3,2-c]pyridin-4-amine |
| 209 | 2-(3-fluorophenyl)-1-methyl-N-[4-(4-methylpiperazin-1-yl)phenyl]pyrrolo[3,2-c]pyridin-4-amine |
| 210 | N-[3-[[2-(3-fluorophenyl)-1-methyl-pyrrolo[3,2-c]pyridin-4-yl]amino]phenyl]acetamide |
| 211 | 4-[4-(4-acetamidoanilino)-1-methyl-pyrrolo[3,2-c]pyridin-2-yl]-N,N-dimethyl-benzamide |
| 212 | 4-[4-(3-methoxyanilino)-1-methyl-pyrrolo[3,2-c]pyridin-2-yl]-N,N-dimethyl-benzamide |
| 213 | 4-[4-(4-carbamoylanilino)-1-methyl-pyrrolo[3,2-c]pyridin-2-yl]-N,N-dimethyl-benzamide |
| 214 | 4-[4-(2,3-dihydro-1,4-benzodioxin-6-ylamino)-1-methyl-pyrrolo[3,2-c]pyridin-2-yl]-N,N-dimethyl-benzamide |
| 215 | 4-[4-(3,4-dimethoxyanilino)-1-methyl-pyrrolo[3,2-c]pyridin-2-yl]-N,N-dimethyl-benzamide |
| 216 | 4-[4-[(6-methoxy-3-pyridyl)amino]-1-methyl-pyrrolo[3,2-c]pyridin-2-yl]-N,N-dimethyl-benzamide |
| 217 | N,N-dimethyl-4-[1-methyl-4-(4-phenoxyanilino)pyrrolo[3,2-c]pyridin-2-yl]benzamide |
| 218 | 4-[4-[3-(dimethylamino)anilino]-1-methyl-pyrrolo[3,2-c]pyridin-2-yl]-N,N-dimethyl-benzamide |
| 219 | N,N-dimethyl-4-[1-methyl-4-(2-pyridylam ino)pyrrolo[3,2-c]pyridin-2-yl]benzamide |
| 220 | N,N-dimethyl-4-[1-methyl-4-(3-methoxy-5-(trifuloromethyl)phenylamino)pyrrolo[3,2-c]pyridin-2-yl]benzamide |
| 221 | 4-[4-(3-hydroxyanilino)-1-methyl-pyrrolo[3,2-c]pyridin-2-yl]-N,N-dimethyl-benzamide |
| 222 | N,N-dimethyl-4-[1-methyl-4-[3-(trifluoromethoxy)anilino]pyrrolo[3,2-c]pyridin-2-yl]benzamide |
| 223 | N,N-dimethyl-4-[1-methyl-4-(3-phenoxyanilino)pyrrolo[3,2-c]pyridin-2-yl]benzamide |
| 224 | 4-[4-(3-isopropylanilino)-1-methyl-pyrrolo[3,2-c]pyridin-2-yl]-N,N-dimethyl-benzamide |
| 225 | 4-[4-[3-(methanesulfonamido)anilino]-1-methyl-pyrrolo[3,2-c]pyridin-2-yl]-N, N-dimethyl-benzamide |
| 226 | 4-[[2-(4-dimethylaminophenyl)-1-methyl-pyrrolo[3,2-c]pyridin-4-yl]amino]phenol |
| 227 | 4-[[2-(4-dimethylaminophenyl)-1-methyl-pyrrolo[3,2-c]pyridin-4-yl]amino]benzamide |

(continued)

| compound | name |
|---|---|
| 228 | 5-[[2-(4-dimethylaminophenyl)-1-methyl-pyrrolo[3,2-c]pyridin-4-yl]amino]-2-methoxy-phenol |
| 229 | 2-(4-dimethylaminophenyl)-1-methyl-N-pyrimidin-4-yl-pyrrolo[3,2-c]pyridin-4-amine |
| 230 | 2-(4-dimethylaminophenyl)-1-methyl-N-(4-methylsulfonylphenyl)pyrrolo[3,2-c]pyridin-4-amine |
| 231 | N-[4-[[2-(3-pyridyl)-1H-pyrrolo[3,2-c]pyridin-4-yl]amino]phenyl]acetamide |
| 232 | N-(2,3-dihydro-1,4-benzodioxin-6-yl)-2-(3-pyridyl)-1H-pyrrolo[3,2-c]pyridin-4-amine |
| 233 | N-(6-methoxy-3-pyridyl)-2-(3-pyridyl)-1H-pyrrolo[3,2-c]pyridin-4-amine |
| 234 | N,2-bis(3-pyridyl)-1H-pyrrolo[3,2-c]pyridin-4-amine |
| 235 | N-[4-(4-methylpiperazin-1-yl)phenyl]-2-(3-pyridyl)-1H-pyrrolo[3,2-c]pyridin-4-amine |
| 236 | 2-methoxy-5-[[2-(3-pyridyl)-1H-pyrrolo[3,2-c]pyridin-4-yl]amino]phenol |
| 237 | 1-methyl-2-(3-pyridyl)-N-(3,4,5-trimethoxyphenyl)pyrrolo[3,2-c]pyridin-4-amine |
| 238 | N-(3,4-dimethoxyphenyl)-1-methyl-2-(3-pyridyl)pyrrolo[3,2-c]pyridin-4-amine |
| 239 | N1,N1-dimethyl-N3-[1-methyl-2-(3-pyridyl)pyrrolo[3,2-c]pyridin-4-yl]benzene-1,3-diamine |
| 240 | N-(4-methoxy-2-methyl-phenyl)-1-methyl-2-(3-pyridyl)pyrrolo[3,2-c]pyridin-4-amine |
| 241 | 1-methyl-N-(m-tolyl)-2-(3-pyridyl)pyrrolo[3,2-c]pyridin-4-amine |
| 242 | N-(4-fluorophenyl)-1-methyl-2-(3-pyridyl)pyrrolo[3,2-c]pyridin-4-amine |
| 243 | 1-methyl-2-(3-pyridyl)-N-[3-(trifluoromethyl)phenyl]pyrrolo[3,2-c]pyridin-4-amine |
| 244 | N-[3-[[1-methyl-2-(3-pyridyl)pyrrolo[3,2-c]pyridin-4-yl]amino]phenyl]methanesulfonamide |
| 245 | N-[3-[[1-methyl-2-(3-pyridyl)pyrrolo[3,2-c]pyridin-4-yl]amino]phenyl]acetamide |
| 246 | 2-(1-methylpyrazol-4-yl)-N-(4-methylsulfonylphenyl)-1H-pyrrolo[3,2-c]pyridin-4-amine |
| 247 | 1-methyl-2-(1-methylpyrazol-4-yl)-N-(3-pyridyl)pyrrolo[3,2-c]pyridin-4-amine |
| 248 | N-methyl-N-[3-[1-methyl-4-[4-(trifluoromethoxy)anilino]pyrrolo[3,2-c]pyridin-2-yl]phenyl]acetamide |
| 249 | N-[4-[[2-(3-thienyl)-1H-pyrrolo[3,2-c]pyridin-4-yl]amino]phenyl]acetamide |
| 250 | 4-[[2-(3-thienyl)-1H-pyrrolo[3,2-c]pyridin-4-yl]amino]benzamide |
| 251 | N-(2,4-dimethoxyphenyl)-2-(3-thienyl)-1H-pyrrolo[3,2-c]pyridin-4-amine |
| 252 | N-(2,3-dihydro-1,4-benzodioxin-6-yl)-2-(3-thienyl)-1H-pyrrolo[3,2-c]pyridin-4-amine |
| 253 | N-(3,4-dimethoxyphenyl)-2-(3-thienyl)-1H-pyrrolo[3,2-c]pyridin-4-amine |
| 254 | N-(6-methoxy-3-pyridyl)-2-(3-thienyl)-1H-pyrrolo[3,2-c]pyridin-4-amine |
| 255 | N-(3-pyridyl)-2-(3-thienyl)-1H-pyrrolo[3,2-c]pyridin-4-amine |
| 256 | N-(4-morpholinophenyl)-2-(3-thienyl)-1H-pyrrolo[3,2-c]pyridin-4-amine |
| 257 | N-[4-(4-methylpiperazin-1-yl)phenyl]-2-(3-thienyl)-1H-pyrrolo[3,2-c]pyridin-4-amine |
| 258 | N-(4-pyridyl)-2-(3-thienyl)-1H-pyrrolo[3,2-c]pyridin-4-amine |
| 259 | N-(4-methoxyphenyl)-2-(3-thienyl)-1H-pyrrolo[3,2-c]pyridin-4-amine |
| 260 | N-(2-pyridyl)-2-(3-thienyl)-1H-pyrrolo[3,2-c]pyridin-4-amine |
| 261 | methyl 4-[[2-(3-thienyl)-1H-pyrrolo[3,2-c]pyridin-4-yl]amino]benzoate |
| 262 | N-(3-methylsulfonylphenyl)-2-(3-thienyl)-1H-pyrrolo[3,2-c]pyridin-4-amine |
| 263 | N-(1,3-benzodioxol-5-yl)-2-(3-thienyl)-1H-pyrrolo[3,2-c]pyridin-4-amine |
| 264 | 3-[[2-(3-thienyl)-1H-pyrrolo[3,2-c]pyridin-4-yl]amino]phenol |
| 265 | N-[3-[[2-(3-thienyl)-1H-pyrrolo[3,2-c]pyridin-4-yl]amino]phenyl]methanesulfonamide |

(continued)

| compound | name |
|---|---|
| 266 | N-[3-[[2-(3-thienyl)-1H-pyrrolo[3,2-c]pyridin-4-yl]amino]phenyl]acetamide |
| 267 | 1-[3-[[2-(3-thienyl)-1H-pyrrolo[3,2-c]pyridin-4-yl]amino]phenyl]ethanone |
| 268 | N-(4-methylsulfonylphenyl)-2-(3-thienyl)-1H-pyrrolo[3,2-c]pyrid in-4-amine |
| 269 | N-(4-isopropoxyphenyl)-2-(3-thienyl)-1H-pyrrolo[3,2-c]pyridin-4-amine |
| 270 | N-[4-[[1-methyl-2-(3-thienyl)pyrrolo[3,2-c]pyridin-4-yl]amino]phenyl]acetamide |
| 271 | 4-[[1-methyl-2-(3-thienyl)pyrrolo[3,2-c]pyridin-4-yl]amino]phenol |
| 272 | N-(2,4-dimethoxyphenyl)-1-methyl-2-(3-thienyl)pyrrolo[3,2-c]pyridin-4-amine |
| 273 | N-(2,3-dihydro-1,4-benzodioxin-6-yl)-1-methyl-2-(3-thienyl)pyrrolo[3,2-c]pyridin-4-amine |
| 274 | N-(3,4-dimethoxyphenyl)-1-methyl-2-(3-thienyl)pyrrolo[3,2-c]pyridin-4-amine |
| 275 | N-(6-methoxy-3-pyridyl)-1-methyl-2-(3-thienyl)pyrrolo[3,2-c]pyridin-4-amine |
| 276 | 1-methyl-N-(3-pyridyl)-2-(3-thienyl)pyrrolo[3,2-c]pyridin-4-amine |
| 277 | N1,N1-dimethyl-N3-[1-methyl-2-(3-thienyl)pyrrolo[3,2-c]pyridin-4-yl]benzene-1,3-diamine |
| 278 | 1-methyl-N-(4-pyridyl)-2-(3-thienyl)pyrrolo[3,2-c]pyridin-4-amine |
| 279 | N-(4-methoxyphenyl)-1-methyl-2-(3-thienyl)pyrrolo[3,2-c]pyridin-4-amine |
| 280 | 1-methyl-N-(2-pyridyl)-2-(3-thienyl)pyrrolo[3,2-c]pyridin-4-amine |
| 281 | 4-[[1-methyl-2-(3-thienyl)pyrrolo[3,2-c]pyridin-4-yl]amino]benzonitrile |
| 282 | 1-methyl-N-(3-methylsulfonylphenyl)-2-(3-thienyl)pyrrolo[3,2-c]pyridin-4-amine |
| 283 | N-(1,3-benzodioxol-5-yl)-1-methyl-2-(3-thienyl)pyrrolo[3,2-c]pyridin-4-amine |
| 284 | 2-methoxy-5-[[1-methyl-2-(3-thienyl)pyrrolo[3,2-c]pyridin-4-yl]amino]phenol |
| 285 | 1-methyl-N-pyrimidin-4-yl-2-(3-thienyl)pyrrolo[3,2-c]pyridin-4-amine |
| 286 | N-[3-[[1-methyl-2-(3-thienyl)pyrrolo[3,2-c]pyridin-4-yl]amino]phenyl]methanesulfonamide |
| 287 | N-[3-[[1-methyl-2-(3-thienyl)pyrrolo[3,2-c]pyridin-4-yl]amino]phenyl]acetamide |
| 288 | 1-[3-[[1-methyl-2-(3-thienyl)pyrrolo[3,2-c]pyridin-4-yl]amino]phenyl]ethanone |
| 289 | 1-methyl-N-(4-methylsulfonylphenyl)-2-(3-thienyl)pyrrolo[3,2-c]pyridin-4-amine |
| 290 | 4-[[2-(3,5-dimethoxyphenyl)-1H-pyrrolo[3,2-c]pyridin-4-yl]amino]benzamide |
| 291 | 2-(3,5-dimethoxyphenyl)-N-(4-pyridyl)-1H-pyrrolo[3,2-c]pyridin-4-amine |
| 292 | N-[3-[[2-(3,5-dimethoxyphenyl)-1H-pyrrolo[3,2-c]pyridin-4-yl]amino]phenyl]methanesulfonamide |
| 293 | 2-(3,5-dimethoxyphenyl)-N-(4-methylsulfonylphenyl)-1H-pyrrolo[3,2-c]pyridin-4-amine |
| 294 | 4-[[2-(2-methoxyphenyl)-1H-pyrrolo[3,2-c]pyridin-4-yl]amino]benzamide |
| 295 | 2-(2-methoxyphenyl)-N-(4-pyridyl)-1H-pyrrolo[3,2-c]pyridin-4-amine |
| 296 | N-(1,3-benzodioxol-5-yl)-2-(2-methoxyphenyl)-1H-pyrrolo[3,2-c]pyridin-4-amine |
| 297 | 3-[[2-(2-methoxyphenyl)-1H-pyrrolo[3,2-c]pyridin-4-yl]amino]phenol |
| 298 | N-[3-[[2-(2-methoxyphenyl)-1H-pyrrolo[3,2-c]pyridin-4-yl]amino]phenyl]methanesulfonamide |
| 299 | N-[3-[[2-(2-methoxyphenyl)-1H-pyrrolo[3,2-c]pyridin-4-yl]amino]phenyl]acetamide |
| 300 | 2-(2-methoxyphenyl)-N-(4-methylsulfonylphenyl)-1H-pyrrolo[3,2-c]pyridin-4-amine |
| 301 | N-[4-[[2-(2-methoxyphenyl)-1-methyl-pyrrolo[3,2-c]pyridin-4-yl]amino]phenyl]acetamide |
| 302 | N-(2,4-dimethoxyphenyl)-2-(2-methoxyphenyl)-1-methyl-pyrrolo[3,2-c]pyridin-4-amine |
| 303 | 2-(2-methoxyphenyl)-1-methyl-N-(3-pyridyl)pyrrolo[3,2-c]pyridin-4-amine |

(continued)

| compound | name |
|---|---|
| 304 | 2-(2-methoxyphenyl)-1-methyl-N-(2-pyridyl)pyrrolo[3,2-c]pyridin-4-amine |
| 305 | 2-(2-methoxyphenyl)-1-methyl-N-(4-methylsulfanylphenyl)pyrrolo[3,2-c]pyridin-4-amine |
| 306 | N-[4-[[2-(3-methoxyphenyl)-1H-pyrrolo[3,2-c]pyridin-4-yl]amino]phenyl]acetamide |
| 307 | 2-(3-methoxyphenyl)-N-(6-methoxy-3-pyridyl)-1H-pyrrolo[3,2-c]pyridin-4-amine |
| 308 | 2-(3-methoxyphenyl)-N-(3-pyridyl)-1H-pyrrolo[3,2-c]pyridin-4-amine |
| 309 | 2-(3-methoxyphenyl)-N-phenyl-1H-pyrrolo[3,2-c]pyridin-4-amine |
| 310 | 2-(3-methoxyphenyl)-N-[4-(4-methylpiperazin-1-yl)phenyl]-1H-pyrrolo[3,2-c]pyridin-4-amine |
| 311 | 2-(3-methoxyphenyl)-N-(3-methylsulfonylphenyl)-1H-pyrrolo[3,2-c]pyridin-4-amine |
| 312 | 2-methoxy-5-[[2-(3-methoxyphenyl)-1H-pyrrolo[3,2-c]pyridin-4-yl]amino]phenol |
| 313 | 4-[[2-(3-methoxyphenyl)-1H-pyrrolo[3,2-c]pyridin-4-yl]amino]-N,N-dimethyl-benzamide |
| 314 | N-(3-isopropoxyphenyl)-2-(3-methoxyphenyl)-1-methyl-pyrrolo[3,2-c]pyridin-4-amine |
| 315 | 2-(3-chlorophenyl)-N-(3-pyridyl)-1H-pyrrolo[3,2-c]pyridin-4-amine |
| 316 | 2-(3-chlorophenyl)-1-methyl-N-pyrimidin-4-yl-pyrrolo[3,2-c]pyridin-4-amine |
| 317 | 4-[(2-phenyl-1 H-pyrrolo[3,2-c]pyridin-4-yl)amino]phenol |
| 318 | 4-[(2-phenyl-1H-pyrrolo[3,2-c]pyridin-4-yl)amino]benzamide |
| 319 | 2-phenyl-N-(3-pyridyl)-1H-pyrrolo[3,2-c]pyridin-4-amine |
| 320 | N-[4-(4-methylpiperazin-1-yl)phenyl]-2-phenyl-1H-pyrrolo[3,2-c]pyridin-4-amine |
| 321 | 2-phenyl-N-(2-pyridyl)-1H-pyrrolo[3,2-c]pyridin-4-amine |
| 322 | N,N-dimethyl-4-[(2-phenyl-1H-pyrrolo[3,2-c]pyridin-4-yl)amino]benzamide |
| 323 | N-[3-[(2-phenyl-1H-pyrrolo[3,2-c]pyridin-4-yl)amino]phenyl]acetamide |
| 324 | N-(4-methylsulfonylphenyl)-2-phenyl-1H-pyrrolo[3,2-c]pyridin-4-amine |
| 325 | 4-[(1-methyl-2-phenyl-pyrrolo[3,2-c]pyridin-4-yl)amino]benzamide |
| 326 | N-(3,4-dimethoxyphenyl)-1-methyl-2-phenyl-pyrrolo[3,2-c]pyridin-4-amine |
| 327 | 1-methyl-2-phenyl-N-(2-pyridyl)pyrrolo[3,2-c]pyridin-4-amine |
| 328 | 3-[(1-methyl-2-phenyl-pyrrolo[3,2-c]pyridin-4-yl)amino]phenol |
| 329 | 2-phenyl-N-(3,4,5-trimethoxyphenyl)-1H-imidazo[4,5-c]pyridin-4-amine |
| 330 | N-(3,4-dimethoxyphenyl)-2-phenyl-1H-imidazo[4,5-c]pyridin-4-amine |
| 331 | N1,N1-dimethyl-N3-(2-phenyl-1H-imidazo[4,5-c]pyridin-4-yl)benzene-1,3-diamine |
| 332 | 2-phenyl-N-[2-(2-pyridyl)ethyl]-1H-imidazo[4,5-c]pyridin-4-amine |
| 333 | N-(4-methoxyphenyl)-2-phenyl-1H-imidazo[4,5-c]pyridin-4-amine |
| 334 | 4-(4-methylpiperazin-1-yl)-2-phenyl-1H-imidazo[4,5-c]pyridine |
| 335 | 2-phenyl-N-(4-pyridylmethyl)-1H-imidazo[4,5-c]pyridin-4-amine |
| 336 | N-(3-methylsulfonylphenyl)-2-phenyl-1H-imidazo[4,5-c]pyridin-4-amine |
| 337 | 3-[(2-phenyl-1H-imidazo[4,5-c]pyridin-4-yl)amino]phenol |
| 338 | 4-(2-phenyl-1H-imidazo[4,5-c]pyridin-4-yl)morpholine |
| 339 | N-(cyclopropylmethyl)-2-phenyl-1H-imidazo[4,5-c]pyridin-4-amine |
| 340 | N-(3-methoxypropyl)-2-phenyl-1H-imidazo[4,5-c]pyridin-4-amine |
| 341 | 2-(3,4-dimethoxyphenyl)-N-(3-methoxyphenyl)-1H-imidazo[4,5-c]pyridin-4-amine |

(continued)

| compound | name |
|---|---|
| 342 | 2-(3,4-dimethoxyphenyl)-N-phenyl-1H-imidazo[4,5-c]pyridin-4-amine |
| 343 | N1-[2-(3,4-dimethoxyphenyl)-1H-imidazo[4,5-c]pyridin-4-yl]-N3,N3-dimethyl-benzene-1,3-diamine |
| 344 | 2-(3,4-dimethoxyphenyl)-N-isobutyl-1H-imidazo[4,5-c]pyridin-4-amine |
| 345 | 2-(3,4-dimethoxyphenyl)-N-(4-methoxyphenyl)-1H-imidazo[4,5-c]pyridin-4-amine |
| 346 | 2-(3,4-dimethoxyphenyl)-4-(4-methylpiperazin-1-yl)-1H-imidazo[4,5-c]pyridine |
| 347 | N-benzyl-2-(3,4-dimethoxyphenyl)-1H-imidazo[4,5-c]pyridin-4-amine |
| 348 | 4-[[2-(3,4-dimethoxyphenyl)-1H-imidazo[4,5-c]pyridin-4-yl]amino]cyclohexanol |
| 349 | 2-(3,4-dimethoxyphenyl)-N-methyl-N-phenyl-1H-imidazo[4,5-c]pyridin-4-amine |
| 350 | 2-(3,4-dimethoxyphenyl)-N-[(4-dimethylaminophenyl)methyl]-1H-imidazo[4,5-c]pyridin-4-amine |
| 351 | 2-(3,4-dimethoxyphenyl)-N-(3-methylsulfonylphenyl)-1H-imidazo[4,5-c]pyridin-4-amine |
| 352 | 3-[[2-(3,4-dimethoxyphenyl)-1H-imidazo[4,5-c]pyridin-4-yl]amino]phenol |
| 353 | 4-[2-(3,4-dimethoxyphenyl)-1H-imidazo[4,5-c]pyridin-4-yl]morpholine |
| 354 | N-(cyclopropylmethyl)-2-(3,4-dimethoxyphenyl)-1H-imidazo[4,5-c]pyridin-4-amine |
| 355 | N-(2-methoxyethyl)-2-[3-(trifluoromethyl)phenyl]-1H-imidazo[4,5-c]pyridin-4-amine |
| 356 | N-(4-methoxyphenyl)-2-[3-(trifluoromethyl)phenyl]-1H-imidazo[4,5-c]pyridin-4-amine |
| 357 | N-(2-morpholinoethyl)-2-[3-(trifluoromethyl)phenyl]-1H-imidazo[4,5-c]pyridin-4-amine |
| 358 | N-(3-methoxyphenyl)-2-(3-pyridyl)-1H-imidazo[4,5-c]pyridin-4-amine |
| 359 | N-(2-diethylaminoethyl)-4-[[2-(3-pyridyl)-1H-imidazo[4,5-c]pyridin-4-yl]amino]benzamide |
| 360 | N-benzyl-2-(3-pyridyl)-1H-imidazo[4,5-c]pyridin-4-amine |
| 361 | N-[[3-(dimethylamino)phenyl]methyl]-2-(3-pyridyl)-1H-imidazo[4,5-c]pyridin-4-amine |
| 362 | 3-[4-(4-methoxyanilino)-1H-imidazo[4,5-c]pyridin-2-yl]phenol |
| 363 | 3-(4-morpholino-1H-imidazo[4,5-c]pyridin-2-yl)phenol |
| 364 | 4-[4-(3,4,5-trimethoxyanilino)-1H-imidazo[4,5-c]pyridin-2-yl]phenol |
| 365 | 4-(4-anilino-1H-imidazo[4,5-c]pyridin-2-yl)phenol |
| 366 | N-[3-[[2-(4-hydroxyphenyl)-1H-imidazo[4,5-c]pyridin-4-yl]amino]phenyl]methanesulfonamide |
| 367 | 2-(3-methoxyphenyl)-N-(3,4,5-trimethoxyphenyl)-1H-imidazo[4,5-c]pyridin-4-amine |
| 368 | N,2-bis(3-methoxyphenyl)-1H-imidazo[4,5-c]pyridin-4-amine |
| 369 | N-(3,4-dimethoxyphenyl)-2-(3-methoxyphenyl)-1H-imidazo[4,5-c]pyridin-4-amine |
| 370 | 2-(3-methoxyphenyl)-N-phenyl-1H-imidazo[4,5-c]pyridin-4-amine |
| 371 | 2-(3-methoxyphenyl)-N-[2-(2-pyridyl)ethyl]-1H-imidazo[4,5-c]pyridin-4-amine |
| 372 | 2-(3-methoxyphenyl)-N-(4-methoxyphenyl)-1H-imidazo[4,5-c]pyridin-4-amine |
| 373 | 2-(3-methoxyphenyl)-N-[3-(trifluoromethyl)phenyl]-1H-imidazo[4,5-c]pyridin-4-amine |
| 374 | 3-[[2-(3-methoxyphenyl)-1H-imidazo[4,5-c]pyridin-4-yl]amino]phenol |
| 375 | 4-[2-(3-methoxyphenyl)-1H-imidazo[4,5-c]pyridin-4-yl]morpholine |
| 376 | N-(cyclopropylmethyl)-2-(3-methoxyphenyl)-1H-imidazo[4,5-c]pyridin-4-amine |
| 377 | 2-(p-tolyl)-N-(3,4,5-trimethoxyphenyl)-1H-imidazo[4,5-c]pyridin-4-amine |
| 378 | N-(3,4-dimethoxyphenyl)-2-(p-tolyl)-1H-imidazo[4,5-c]pyridin-4-amine |
| 379 | N-(6-methoxy-3-pyridyl)-2-(p-tolyl)-1H-imidazo[4,5-c]pyridin-4-amine |

(continued)

| compound | name |
|---|---|
| 380 | N-phenyl-2-(p-tolyl)-1H-imidazo[4,5-c]pyridin-4-amine |
| 381 | N-(2-diethylaminoethyl)-4-[[2-(p-tolyl)-1H-imidazo[4,5-c]pyridin-4-yl]amino]benzamide |
| 382 | N-(4-methoxyphenyl)-2-(p-tolyl)-1H-imidazo[4,5-c]pyridin-4-amine |
| 383 | 3-[[2-(p-tolyl)-1H-imidazo[4,5-c]pyridin-4-yl]amino]phenol |
| 384 | N-[3-[[2-(p-tolyl)-1H-imidazo[4,5-c]pyridin-4-yl]amino]phenyl]methanesulfonamide |
| 385 | N-(2-methoxyethyl)-3-[4-(3,4,5-trimethoxyanilino)-1H-imidazo[4,5-c]pyridin-2-yl]benzamide |
| 386 | 3-[4-(3-methoxyanilino)-1H-imidazo[4,5-c]pyridin-2-yl]-N-(2-methoxyethyl)benzamide |
| 387 | 3-(4-anilino-1H-imidazo[4,5-c]pyridin-2-yl)-N-(2-methoxyethyl)benzamide |
| 388 | 2-(3-isopropylphenyl)-4-(4-methylpiperazin-1-yl)-1H-imidazo[4,5-c]pyridine |
| 389 | 2-(4-methoxyphenyl)-N-(3,4,5-trimethoxyphenyl)-1H-imidazo[4,5-c]pyridin-4-amine |
| 390 | N-(3-methoxyphenyl)-2-(4-methoxyphenyl)-1H-imidazo[4,5-c]pyridin-4-amine |
| 391 | N-(2-methoxyethyl)-2-(4-methoxyphenyl)-1H-imidazo[4,5-c]pyridin-4-amine |
| 392 | N-(3,4-dimethoxyphenyl)-2-(4-methoxyphenyl)-1H-imidazo[4,5-c]pyridin-4-amine |
| 393 | 2-(4-methoxyphenyl)-N-phenyl-1H-imidazo[4,5-c]pyridin-4-amine |
| 394 | 2-(4-methoxyphenyl)-N-[2-(2-pyridyl)ethyl]-1H-imidazo[4,5-c]pyridin-4-amine |
| 395 | N,2-bis(4-methoxyphenyl)-1H-imidazo[4,5-c]pyridin-4-amine |
| 396 | 2-(4-methoxyphenyl)-4-(4-methylpiperazin-1-yl)-1H-imidazo[4,5-c]pyridine |
| 397 | 3-[[2-(4-methoxyphenyl)-1H-imidazo[4,5-c]pyridin-4-yl]amino]phenol |
| 398 | 2-methoxy-5-[[2-(4-methoxyphenyl)-1H-imidazo[4,5-c]pyridin-4-yl]amino]phenol |
| 399 | 4-[2-(4-methoxyphenyl)-1H-imidazo[4,5-c]pyridin-4-yl]morpholine |
| 400 | N-(cyclopropylmethyl)-2-(4-methoxyphenyl)-1H-imidazo[4,5-c]pyridin-4-amine |
| 401 | 2-(4-methoxyphenyl)-N-(3-methoxypropyl)-1H-imidazo[4,5-c]pyridin-4-amine |
| 402 | 2-(3-fluorophenyl)-N-(3,4,5-trimethoxyphenyl)-1H-imidazo[4,5-c]pyridin-4-amine |
| 403 | 4-[[2-(3-fluorophenyl)-1H-imidazo[4,5-c]pyridin-4-yl]amino]benzamide |
| 404 | N-(3,4-dimethoxyphenyl)-2-(3-fluorophenyl)-1H-imidazo[4,5-c]pyridin-4-amine |
| 405 | 2-(3-fluorophenyl)-N-(6-methoxy-3-pyridyl)-1H-imidazo[4,5-c]pyridin-4-amine |
| 406 | 2-(3-fluorophenyl)-N-[2-(2-pyridyl)ethyl]-1H-imidazo[4,5-c]pyridin-4-amine |
| 407 | 2-(3-fluorophenyl)-N-(4-methoxyphenyl)-1H-imidazo[4,5-c]pyridin-4-amine |
| 408 | 2-(3-fluorophenyl)-N-(3-phenoxyphenyl)-1H-imidazo[4,5-c]pyridin-4-amine |
| 409 | 2-(2,3-dihydro-1,4-benzodioxin-6-yl)-N-(3,4,5-trimethoxyphenyl)-1H-imidazo[4,5-c]pyridin-4-amine |
| 410 | 2-(2,3-dihydro-1,4-benzodioxin-6-yl)-N-(3-methoxyphenyl)-1H-imidazo[4,5-c]pyridin-4-amine |
| 411 | 2-(2,3-dihydro-1,4-benzodioxin-6-yl)-N-(3,4-dimethoxyphenyl)-1H-imidazo[4,5-c]pyridin-4-amine |
| 412 | 2-(2,3-dihydro-1,4-benzodioxin-6-yl)-N-phenyl-1H-imidazo[4,5-c]pyridin-4-amine |
| 413 | 2-(2,3-dihydro-1,4-benzodioxin-6-yl)-N-isobutyl-1H-imidazo[4,5-c]pyridin-4-amine |
| 414 | 2-(2,3-dihydro-1,4-benzodioxin-6-yl)-N-(4-methoxyphenyl)-1H-imidazo[4,5-c]pyridin-4-amine |
| 415 | 2-(2,3-dihydro-1,4-benzodioxin-6-yl)-4-(4-methylpiperazin-1-yl)-1H-imidazo[4,5-c]pyridine |
| 416 | 2-[[2-(2,3-dihydro-1,4-benzodioxin-6-yl)-1H-imidazo[4,5-c]pyridin-4-yl]amino]ethanol |
| 417 | 2-(2,3-dihydro-1,4-benzodioxin-6-yl)-N-(3-methylsulfonylphenyl)-1H-imidazo[4,5-c]pyridin-4-amine |

(continued)

| compound | name |
|---|---|
| 418 | 3-[[2-(2,3-dihydro-1,4-benzodioxin-6-yl)-1H-imidazo[4,5-c]pyridin-4-yl]amino]phenol |
| 419 | N-(cyclopropylmethyl)-2-(2,3-dihydro-1,4-benzodioxin-6-yl)-1H-imidazo[4,5-c]pyridin-4-amine |
| 420 | 2-(2,3-dihydro-1,4-benzodioxin-6-yl)-N-(3-methoxypropyl)-1H-imidazo[4,5-c]pyridin-4-amine |
| 421 | 2-(4-pyridyl)-N-(3,4,5-trimethoxyphenyl)-1H-imidazo[4,5-c]pyridin-4-amine |
| 422 | N-(3-methoxyphenyl)-2-(4-pyridyl)-1H-imidazo[4,5-c]pyridin-4-amine |
| 423 | N-(3,4-dimethoxyphenyl)-2-(4-pyridyl)-1H-imidazo[4,5-c]pyridin-4-amine |
| 424 | N-(3-chlorophenyl)-2-(4-pyridyl)-1H-imidazo[4,5-c]pyridin-4-amine |
| 425 | N-(2-diethylaminoethyl)-4-[[2-(4-pyridyl)-1H-imidazo[4,5-c]pyridin-4-yl]amino]benzamide |
| 426 | N1,N1-dimethyl-N3-[2-(4-pyridyl)-1H-imidazo[4,5-c]pyridin-4-yl]benzene-1,3-diamine |
| 427 | N-(4-methoxyphenyl)-2-(4-pyridyl)-1H-imidazo[4,5-c]pyridin-4-amine |
| 428 | N-benzyl-2-(4-pyridyl)-1H-imidazo[4,5-c]pyridin-4-amine |
| 429 | (2R)-3-methyl-2-[[2-(4-pyridyl)-1H-imidazo[4,5-c]pyridin-4-yl]amino]butan-1-ol |
| 430 | N-[(4-dimethylaminophenyl)methyl]-2-(4-pyridyl)-1H-imidazo[4,5-c]pyridin-4-amine |
| 431 | 3-[[2-(4-pyridyl)-1H-imidazo[4,5-c]pyridin-4-yl]amino]phenol |
| 432 | 2-methoxy-5-[[2-(4-pyridyl)-1H-imidazo[4,5-c]pyridin-4-yl]amino]phenol |
| 433 | N-[(4-chlorophenyl)methyl]-2-(4-pyridyl)-1H-imidazo[4,5-c]pyridin-4-amine |
| 434 | N-phenethyl-2-(4-pyridyl)-1H-imidazo[4,5-c]pyridin-4-amine |
| 435 | 2-(m-tolyl)-N-(3,4,5-trimethoxyphenyl)-1H-imidazo[4,5-c]pyridin-4-amine |
| 436 | N-(3,4-dimethoxyphenyl)-2-(m-tolyl)-1H-imidazo[4,5-c]pyridin-4-amine |
| 437 | 2-(m-tolyl)-N-[2-(2-pyridyl)ethyl]-1H-imidazo[4,5-c]pyridin-4-amine |
| 438 | 3-[[2-(m-tolyl)-1H-imidazo[4,5-c]pyridin-4-yl]amino]phenol |
| 439 | N-[3-[[2-(m-tolyl)-1H-imidazo[4,5-c]pyridin-4-yl]amino]phenyl]methanesulfonamide |
| 440 | 2-(3,4-difluorophenyl)-N-(3,4-dimethoxyphenyl)-1H-imidazo[4,5-c]pyridin-4-amine |
| 441 | 3-[[2-(3,4-difluorophenyl)-1H-imidazo[4,5-c]pyridin-4-yl]amino]phenol |
| 442 | 4-(4-methylpiperazin-1-yl)-2-[3-(trifluoromethoxy)phenyl]-1H-imidazo[4,5-c]pyridine |
| 443 | 4-[[2-[3-(trifluoromethoxy)phenyl]-1H-imidazo[4,5-c]pyridin-4-yl]amino]cyclohexanol |
| 444 | 2-methoxy-5-[[2-[3-(trifluoromethoxy)phenyl]-1H-imidazo[4,5-c]pyridin-4-yl]amino]phenol |
| 445 | N-(2-pyridylmethyl)-2-[3-(trifluoromethoxy)phenyl]-1H-imidazo[4,5-c]pyridin-4-amine |
| 446 | 4-[2-[3-(trifluoromethoxy)phenyl]-1H-imidazo[4,5-c]pyridin-4-yl]morpholine |
| 447 | N-(3,4-dimethoxyphenyl)-1-methyl-imidazo[4,5-c]pyridin-4-amine |
| 448 | N-(1H-indol-5-yl)-1-methyl-imidazo[4,5-c]pyridin-4-amine |
| 449 | 1-benzyl-N-cyclohexyl-imidazo[4,5-c]pyridin-4-amine |
| 450 | N1,N1-dimethyl-N3-(1-methylimidazo[4,5-c]pyridin-4-yl)benzene-1,3-diamine |
| 451 | 1-benzyl-N-(2-furylmethyl)imidazo[4,5-c]pyridin-4-amine |
| 452 | 1-benzyl-N-(2-thienylmethyl)imidazo[4,5-c]pyridin-4-amine |
| 453 | N-(4-methoxyphenyl)-1-methyl-imidazo[4,5-c]pyridin-4-amine |
| 454 | N-(1,3-benzodioxol-5-ylmethyl)-1-benzyl-imidazo[4,5-c]pyridin-4-amine |
| 455 | 1-benzyl-N-[(4-methoxyphenyl)methyl]imidazo[4,5-c]pyridin-4-amine |

(continued)

| compound | **name** |
|---|---|
| 456 | N-[(3,4-dimethoxyphenyl)methyl]-1-methyl-imidazo[4,5-c]pyridin-4-amine |
| 457 | 1-benzyl-N-(4-isopropylphenyl)imidazo[4,5-c]pyridin-4-amine |
| 458 | 2-(2-chlorophenyl)-N-(3,4-dimethoxyphenyl)-1H-imidazo[4,5-c]pyridin-4-amine |
| 459 | 2-(2-chlorophenyl)-N-[2-(4-pyridyl)ethyl]-1H-imidazo[4,5-c]pyridin-4-amine |
| 460 | 2-(2-chlorophenyl)-N-[(3,4,5-trimethoxyphenyl)methyl]-1H-imidazo[4,5-c]pyridin-4-amine |
| 461 | N-(3-chloro-4-fluoro-phenyl)-2-(2-chlorophenyl)-1H-imidazo[4,5-c]pyridin-4-amine |
| 462 | 2-(2-chlorophenyl)-N-[3-(trifluoromethyl)phenyl]-1H-imidazo[4,5-c]pyridin-4-amine |
| 463 | N-[3-[[2-(2-chlorophenyl)-1H-imidazo[4,5-c]pyridin-4-yl]amino]phenyl]methanesulfonamide |
| 464 | 2-(2-furyl)-N-(3-methoxyphenyl)-1H-imidazo[4,5-c]pyridin-4-amine |
| 465 | 2-(2-furyl)-N-(3-pyridyl)-1H-imidazo[4,5-c]pyridin-4-amine |
| 466 | 2-(2-furyl)-N-(4-methoxyphenyl)-1H-imidazo[4,5-c]pyridin-4-amine |
| 467 | 2-(2-fluoro-3-methoxy-phenyl)-N-(3,4,5-trimethoxyphenyl)-1H-imidazo[4,5-c]pyridin-4-amine |
| 468 | 2-(2-fluoro-3-methoxy-phenyl)-N-(3-methoxyphenyl)-1H-imidazo[4,5-c]pyridin-4-amine |
| 469 | N-(3,4-dimethoxyphenyl)-2-(2-fluoro-3-methoxy-phenyl)-1H-imidazo[4,5-c]pyridin-4-amine |
| 470 | 2-(2-fluoro-3-methoxy-phenyl)-N-(6-methoxy-3-pyridyl)-1H-imidazo[4,5-c]pyridin-4-amine |
| 471 | N-(4-chlorophenyl)-2-(2-fluoro-3-methoxy-phenyl)-1H-imidazo[4,5-c]pyridin-4-amine |
| 472 | 2-(2-fluoro-3-methoxy-phenyl)-N-phenyl-1H-imidazo[4,5-c]pyridin-4-amine |
| 473 | N-(2-diethylaminoethyl)-4-[[2-(2-fluoro-3-methoxy-phenyl)-1H-imidazo[4,5-c]pyridin-4-yl]amino]benzamide |
| 474 | N-(3-chloro-4-fluoro-phenyl)-2-(2-fluoro-3-methoxy-phenyl)-1H-imidazo[4,5-c]pyridin-4-amine |
| 475 | 2-(2-fluoro-3-methoxy-phenyl)-N-(4-methoxyphenyl)-1H-imidazo[4,5-c]pyridin-4-amine |
| 476 | N4-[2-(2-fluoro-3-methoxy-phenyl)-1H-imidazo[4,5-c]pyridin-4-yl]cyclohexane-1,4-diamine |
| 477 | 2-(2-fluoro-3-methoxy-phenyl)-N-(3-methylsulfonylphenyl)-1H-imidazo[4,5-c]pyridin-4-amine |
| 478 | 3-[[2-(2-fluoro-3-methoxy-phenyl)-1H-imidazo[4,5-c]pyridin-4-yl]amino]phenol |
| 479 | 5-[[2-(2-fluoro-3-methoxy-phenyl)-1H-imidazo[4,5-c]pyridin-4-yl]amino]-2-methoxy-phenol |
| 480 | 4-[2-(2-fluoro-3-methoxy-phenyl)-1H-imidazo[4,5-c]pyridin-4-yl]morpholine |
| 481 | 2-(2-fluoro-3-methoxy-phenyl)-N-(3-fluorophenyl)-1H-imidazo[4,5-c]pyridin-4-amine |
| 482 | N2-methyl-N6,1-bis(3,4,5-trimethoxyphenyl)imidazo[4,5-c]pyridine-2,6-diamine |
| 483 | N-[4-[[2-(methylamino)-1-(3,4,5-trimethoxyphenyl)imidazo[4,5-c]pyridin-6-yl]amino]phenyl]acetamide |
| 484 | N6-(3-methoxyphenyl)-N2-methyl-1-(3,4,5-trimethoxyphenyl)imidazo[4,5-c]pyridine-2,6-diamine |
| 485 | 4-[[2-(methylamino)-1-(3,4,5-trimethoxyphenyl)imidazo[4,5-c]pyridin-6-yl]amino]phenol |
| 486 | N6-(2,4-dimethoxyphenyl)-N2-methyl-1-(3,4,5-trimethoxyphenyl)imidazo[4,5-c]pyridine-2,6-diamine |
| 487 | N6-(2,3-dihydro-1,4-benzodioxin-6-yl)-N2-methyl-1-(3,4,5-trimethoxyphenyl)imidazo[4,5-c]pyridine-2,6-diamine |
| 488 | N6-(6-methoxy-3-pyridyl)-N2-methyl-1-(3,4,5-trimethoxyphenyl)imidazo[4,5-c]pyridine-2,6-diamine |
| 489 | N2-methyl-N6-[4-(4-methylpiperazin-1-yl)phenyl]-1-(3,4,5-trimethoxyphenyl)imidazo[4,5-c]pyridine-2,6-diamine |
| 490 | N6-(4-methoxyphenyl)-N2-methyl-1-(3,4,5-trimethoxyphenyl)imidazo[4,5-c]pyridine-2,6-diamine |
| 491 | 3-[[2-(methylamino)-1-(3,4,5-trimethoxyphenyl)imidazo[4,5-c]pyridin-6-yl]amino]phenol |

(continued)

| compound | name |
|---|---|
| 492 | N-[3-[[2-(methylamino)-1-(3,4,5-trimethoxyphenyl)imidazo[4,5-c]pyridin-6-yl]amino]phenyl]methanesulfonamide |
| 493 | N-[3-[[2-(methylamino)-1-(3,4,5-trimethoxyphenyl)imidazo[4,5-c]pyridin-6-yl]amino]phenyl]acetamide |
| 494 | N6-(3-fluorophenyl)-N2-methyl-1-(3,4,5-trimethoxyphenyl)imidazo[4,5-c]pyridine-2,6-diamine |
| 495 | N6-(3,4-dimethoxyphenyl)-1-(2-methoxyethyl)-N2-methyl-imidazo[4,5-c]pyridine-2,6-diamine |
| 496 | 1-(2-methoxyethyl)-N6-(4-methoxyphenyl)-N2-methyl-imidazo[4,5-c]pyridine-2,6-diamine |
| 497 | N6-(1,3-benzodioxol-5-yl)-1-(2-methoxyethyl)-N2-methyl-imidazo[4,5-c]pyridine-2,6-diamine |
| 498 | N6-(4-isopropylphenyl)-1-(2-methoxyethyl)-N2-methyl-imidazo[4,5-c]pyridine-2,6-diamine |
| 499 | 1-(2-methoxyethyl)-N2-methyl-N6-(4-methylsulfanylphenyl)imidazo[4,5-c]pyridine-2,6-diamine |
| 500 | N6-(4-isopropoxyphenyl)-1-(2-methoxyethyl)-N2-methyl-imidazo[4,5-c]pyridine-2,6-diamine |
| 501 | 6-(4-dimethylaminophenyl)-N-methyl-1-phenyl-imidazo[4,5-c]pyridin-2-amine |
| 502 | N2-methyl-1-phenyl-N6-(3,4,5-trimethoxyphenyl)imidazo[4,5-c]pyridine-2,6-diamine |
| 503 | N6-(3-methoxyphenyl)-N2-methyl-1-phenyl-imidazo[4,5-c]pyridine-2,6-diamine |
| 504 | 4-[[2-(methylamino)-1-phenyl-imidazo[4,5-c]pyridin-6-yl]amino]benzamide |
| 505 | N6-(2,3-dihydro-1,4-benzodioxin-6-yl)-N2-methyl-1-phenyl-imidazo[4,5-c]pyridine-2,6-diamine |
| 506 | N6-(3,4-dimethoxyphenyl)-N2-methyl-1-phenyl-imidazo[4,5-c]pyridine-2,6-diamine |
| 507 | N2-methyl-1-phenyl-N6-(3-pyridyl)imidazo[4,5-c]pyridine-2,6-diamine |
| 508 | N6-[3-(dimethylamino)phenyl]-N2-methyl-1-phenyl-imidazo[4,5-c]pyridine-2,6-diamine |
| 509 | N6-(4-methoxyphenyl)-N2-methyl-1-phenyl-imidazo[4,5-c]pyridine-2,6-diamine |
| 510 | [4-[2-(methylamino)-1-phenyl-imidazo[4,5-c]pyridin-6-yl]phenyl]methanol |
| 511 | methyl 4-[[2-(methylamino)-1-phenyl-imidazo[4,5-c]pyridin-6-yl]amino]benzoate |
| 512 | N6-(1,3-benzodioxol-5-yl)-N2-methyl-1-phenyl-imidazo[4,5-c]pyridine-2,6-diamine |
| 513 | 3-[[2-(methylamino)-1-phenyl-imidazo[4,5-c]pyridin-6-yl]amino]phenol |
| 514 | N-methyl-1-phenyl-6-(2-thienyl)imidazo[4,5-c]pyridin-2-amine |
| 515 | N,N-dimethyl-4-[[2-(methylamino)-1-phenyl-imidazo[4,5-c]pyridin-6-yl]amino]benzamide |
| 516 | N-[3-[[2-(methylamino)-1-phenyl-imidazo[4,5-c]pyridin-6-yl]amino]phenyl]acetamide |
| 517 | 1-[3-[[2-(methylamino)-1-phenyl-imidazo[4,5-c]pyridin-6-yl]amino]phenyl]ethanone |
| 518 | N2-methyl-N6-(4-methylsulfonylphenyl)-1-phenyl-imidazo[4,5-c]pyridine-2,6-diamine |
| 519 | 1-(2-furylmethyl)-N2-methyl-N6-(3,4,5-trimethoxyphenyl)imidazo[4,5-c]pyridine-2,6-diamine |
| 520 | N6-(3,4-dimethoxyphenyl)-1-(2-furylmethyl)-N2-methyl-imidazo[4,5-c]pyridine-2,6-diamine |
| 521 | 1-(2-furylmethyl)-N2-methyl-N6-(4-morpholinophenyl)imidazo[4,5-c]pyridine-2,6-diamine |
| 522 | N6-[3-(dimethylamino)phenyl]-1-(2-furylmethyl)-N2-methyl-imidazo[4,5-c]pyridine-2,6-diamine |
| 523 | 1-(2-furylmethyl)-N6-(4-methoxyphenyl)-N2-methyl-imidazo[4,5-c]pyridine-2,6-diamine |
| 524 | N6-(1,3-benzodioxol-5-yl)-1-(2-furylmethyl)-N2-methyl-imidazo[4,5-c]pyridine-2,6-diamine |
| 525 | 1-benzyl-6-(4-dimethylaminophenyl)-N-methyl-imidazo[4,5-c]pyridin-2-amine |
| 526 | 1-benzyl-N2-methyl-N6-(3,4,5-trimethoxyphenyl)imidazo[4,5-c]pyridine-2,6-diamine |
| 527 | N-[4-[[1-benzyl-2-(methylamino)imidazo[4,5-c]pyridin-6-yl]amino]phenyl]acetamide |
| 528 | 1-benzyl-N6-(2,3-dihydro-1,4-benzodioxin-6-yl)-N2-methyl-imidazo[4,5-c]pyridine-2,6-diamine |

(continued)

| compound | name |
|---|---|
| 529 | 1-benzyl-N-methyl-6-(2-phenoxyphenyl)imidazo[4,5-c]pyridin-2-amine |
| 530 | 1-benzyl-N2-methyl-N6-(m-tolyl)imidazo[4,5-c]pyridine-2,6-diamine |
| 531 | 1-benzyl-N6-(4-methoxyphenyl)-N2-methyl-imidazo[4,5-c]pyridine-2,6-diamine |
| 532 | 1-benzyl-6-(3-chlorophenyl)-N-methyl-imidazo[4,5-c]pyridin-2-amine |
| 533 | 3-[1-benzyl-2-(methylamino)imidazo[4,5-c]pyridin-6-yl]phenol |
| 534 | N6-(1,3-benzodioxol-5-yl)-1-benzyl-N2-methyl-imidazo[4,5-c]pyridine-2,6-diamine |
| 535 | 3-[[1-benzyl-2-(methylamino)imidazo[4,5-c]pyridin-6-yl]amino]phenol |
| 536 | 5-[[1-benzyl-2-(methylamino)imidazo[4,5-c]pyridin-6-yl]amino]-2-methoxy-phenol |
| 537 | 4-[[1-benzyl-2-(methylamino)imidazo[4,5-c]pyridin-6-yl]amino]-N,N-dimethyl-benzamide |
| 538 | N-[3-[[1-benzyl-2-(methylamino)imidazo[4,5-c]pyridin-6-yl]amino]phenyl]acetamide |
| 539 | N2-methyl-1-[3-(trifluoromethyl)phenyl]-N6-(3,4,5-trimethoxyphenyl)imidazo[4,5-c]pyridine-2,6-diamine |
| 540 | N-[4-[[2-(methylamino)-1-[3-(trifluoromethyl)phenyl]imidazo[4,5-c]pyridin-6-yl]amino]phenyl]acetamide |
| 541 | 4-[[2-(methylamino)-1-[3-(trifluoromethyl)phenyl]imidazo[4,5-c]pyridin-6-yl]amino]phenol |
| 542 | 4-[[2-(methylamino)-1-[3-(trifluoromethyl)phenyl]imidazo[4,5-c]pyridin-6-yl]amino]benzamide |
| 543 | N6-(2,4-dimethoxyphenyl)-N2-methyl-1-[3-(trifluoromethyl)phenyl]imidazo[4,5-c]pyridine-2,6-diamine |
| 544 | N6-(2,3-dihydro-1,4-benzodioxin-6-yl)-N2-methyl-1-[3-(trifluoromethyl)phenyl]imidazo[4,5-c]pyridine-2,6-diamine |
| 545 | N6-(3,4-dimethoxyphenyl)-N2-methyl-1-[3-(trifluoromethyl)phenyl]imidazo[4,5-c]pyridine-2,6-diamine |
| 546 | N6-(6-methoxy-3-pyridyl)-N2-methyl-1-[3-(trifluoromethyl)phenyl]imidazo[4,5-c]pyridine-2,6-diamine |
| 547 | N6-(4-methoxyphenyl)-N2-methyl-1-[3-(trifluoromethyl)phenyl]imidazo[4,5-c]pyridine-2,6-diamine |
| 548 | N2-methyl-N6-(2-pyridyl)-1-[3-(trifluoromethyl)phenyl]imidazo[4,5-c]pyridine-2,6-diamine |
| 549 | N6-(1,3-benzodioxol-5-yl)-N2-methyl-1-[3-(trifluoromethyl)phenyl]imidazo[4,5-c]pyridine-2,6-diamine |
| 550 | 3-[[2-(methylamino)-1-[3-(trifluoromethyl)phenyl]imidazo[4,5-c]pyridin-6-yl]amino]phenol |
| 551 | 2-methoxy-5-[[2-(methylamino)-1-[3-(trifluoromethyl)phenyl]imidazo[4,5-c]pyridin-6-yl]amino]phenol |
| 552 | N6-(3-methoxyphenyl)-N2-methyl-1-(2-morpholinoethyl)imidazo[4,5-c]pyridine-2,6-diamine |
| 553 | N6-(2,4-dimethoxyphenyl)-N2-methyl-1-(2-morpholinoethyl)imidazo[4,5-c]pyridine-2,6-diamine |
| 554 | N6-(2,3-dihydro-1,4-benzodioxin-6-yl)-N2-methyl-1-(2-morpholinoethyl)imidazo[4,5-c]pyridine-2,6-diamine |
| 555 | N6-(3,4-dimethoxyphenyl)-N2-methyl-1-(2-morpholinoethyl)imidazo[4,5-c]pyridine-2,6-diamine |
| 556 | N2-methyl-1-(2-morpholinoethyl)-N6-(m-tolyl)imidazo[4,5-c]pyridine-2,6-diamine |
| 557 | N6-(4-methoxyphenyl)-N2-methyl-1-(2-morpholinoethyl)imidazo[4,5-c]pyridine-2,6-diamine |
| 558 | 1-[3-[[2-(methylamino)-1-(2-morpholinoethyl)imidazo[4,5-c]pyridin-6-yl]amino]phenyl]ethanone |
| 559 | N2-methyl-1-(3-pyridylmethyl)-N6-(3,4,5-trimethoxyphenyl)imidazo[4,5-c]pyridine-2,6-diamine |
| 560 | N6-(3-methoxyphenyl)-N2-methyl-1-(3-pyridylmethyl)imidazo[4,5-c]pyridine-2,6-diamine |
| 561 | N6-(2,3-dihydro-1,4-benzodioxin-6-yl)-N2-methyl-1-(3-pyridylmethyl)imidazo[4,5-c]pyridine-2,6-diamine |
| 562 | N2-methyl-N6-phenyl-1-(3-pyridylmethyl)imidazo[4,5-c]pyridine-2,6-diamine |

(continued)

| compound | name |
|---|---|
| 563 | N-methyl-6-(3-pyridyl)-1-(3-pyridylmethyl)imidazo[4,5-c]pyridin-2-amine |
| 564 | N2-methyl-N6-(m-tolyl)-1-(3-pyridylmethyl)imidazo[4,5-c]pyridine-2,6-diamine |
| 565 | N6-(4-fluorophenyl)-N2-methyl-1-(3-pyridylmethyl)imidazo[4,5-c]pyridine-2,6-diamine |
| 566 | N-methyl-1-(3-pyridylmethyl)-6-(2-thienyl)imidazo[4,5-c]pyridin-2-amine |
| 567 | 1-(cyclopropylmethyl)-N2-methyl-N6-(3,4,5-trimethoxyphenyl)imidazo[4,5-c]pyridine-2,6-diamine |
| 568 | N-[4-[[1-(cyclopropylmethyl)-2-(methylamino)imidazo[4,5-c]pyridin-6-yl]amino]phenyl]acetamide |
| 569 | 1-(cyclopropylmethyl)-N6-(2,4-dimethoxyphenyl)-N2-methyl-imidazo[4,5-c]pyridine-2,6-diamine |
| 570 | 1-(cyclopropylmethyl)-N6-(2,3-dihydro-1,4-benzodioxin-6-yl)-N2-methyl-imidazo[4,5-c]pyridine-2,6-diamine |
| 571 | 1-(cyclopropylmethyl)-N2-methyl-N6-(4-morpholinophenyl)imidazo[4,5-c]pyridine-2,6-diamine |
| 572 | 1-(cyclopropylmethyl)-N2-methyl-N6-[4-(4-methylpiperazin-1-yl)phenyl]imidazo[4,5-c]pyridine-2,6-diamine |
| 573 | 1-(cyclopropylmethyl)-N6-[3-(dimethylamino)phenyl]-N2-methyl-imidazo[4,5-c]pyridine-2,6-diamine |
| 574 | N-[3-[[1-(cyclopropylmethyl)-2-(methylamino)imidazo[4,5-c]pyridin-6-yl]amino]phenyl]acetamide |
| 575 | 6-(4-dimethylaminophenyl)-1-methyl-N-(m-tolylmethyl)imidazo[4,5-c]pyridin-2-amine |
| 576 | N-[4-[[1-methyl-2-(m-tolylmethylamino)imidazo[4,5-c]pyridin-6-yl]amino]phenyl]acetamide |
| 577 | N6-(2,3-dihydro-1,4-benzodioxin-6-yl)-1-methyl-N2-(m-tolylmethyl)imidazo[4,5-c]pyridine-2,6-diamine |
| 578 | N6-(3,4-dimethoxyphenyl)-1-methyl-N2-(m-tolylmethyl)imidazo[4,5-c]pyridine-2,6-diamine |
| 579 | 6-(3,4-dimethoxyphenyl)-1-methyl-N-(m-tolylmethyl)imidazo[4,5-c]pyridin-2-amine |
| 580 | N-[3-[1-methyl-2-(m-tolylmethylamino)imidazo[4,5-c]pyridin-6-yl]phenyl]acetamide |
| 581 | 6-(4-chlorophenyl)-1-methyl-N-(m-tolylmethyl)imidazo[4,5-c]pyridin-2-amine |
| 582 | N6-(4-fluorophenyl)-1-methyl-N2-(m-tolylmethyl)imidazo[4,5-c]pyridine-2,6-diamine |
| 583 | 6-(3-furyl)-1-methyl-N-(m-tolylmethyl)imidazo[4,5-c]pyridin-2-amine |
| 584 | 1-methyl-N6-(3-methylsulfonylphenyl)-N2-(m-tolylmethyl)imidazo[4,5-c]pyridine-2,6-diamine |
| 585 | 3-[[1-methyl-2-(m-tolylmethylamino)imidazo[4,5-c]pyridin-6-yl]amino]phenol |
| 586 | 6-(2-methoxy-3-pyridyl)-1-methyl-N-(m-tolylmethyl)imidazo[4,5-c]pyridin-2-amine |
| 587 | N-[3-[[1-methyl-2-(m-tolylmethylamino)imidazo[4,5-c]pyridin-6-yl]amino]phenyl]methanesulfonamide |
| 588 | 1-methyl-6-phenyl-N-(3,4,5-trimethoxyphenyl)imidazo[4,5-c]pyridin-2-amine |
| 589 | 1-methyl-6-(3-pyridyl)-N-(3,4,5-trimethoxyphenyl)imidazo[4,5-c]pyridin-2-amine |
| 590 | 6-(6-amino-3-pyridyl)-1-methyl-N-[3-(trifluoromethyl)phenyl]imidazo[4,5-c]pyridin-2-amine |
| 591 | N-[2-(2,5-dimethoxyphenyl)ethyl]-1-methyl-6-(4-pyridyl)imidazo[4,5-c]pyridin-2-amine |
| 592 | N-[2-(2,5-dimethoxyphenyl)ethyl]-1-methyl-6-(3-thienyl)imidazo[4,5-c]pyridin-2-amine |
| 593 | N-[2-(2,5-dimethoxyphenyl)ethyl]-1-methyl-6-(3,4,5-trimethoxyphenyl)imidazo[4,5-c]pyridin-2-amine |
| 594 | 4-[2-[2-(2,5-dimethoxyphenyl)ethylamino]-1-methyl-imidazo[4,5-c]pyridin-6-yl]-2-methoxy-phenol |
| 595 | N-[2-(2,5-dimethoxyphenyl)ethyl]-1-methyl-6-(2-thienyl)imidazo[4,5-c]pyridin-2-amine |
| 596 | N-(cyclopropylmethyl)-1-methyl-6-(3-thienyl)imidazo[4,5-c]pyridin-2-amine |
| 597 | N-(cyclopropylmethyl)-1-methyl-6-(4-piperazin-1-ylphenyl)imidazo[4,5-c]pyridin-2-amine |
| 598 | N6-(1,3-benzodioxol-5-yl)-N2-(cyclopropylmethyl)-1-methyl-imidazo[4,5-c]pyridine-2,6-diamine |

(continued)

| compound | name |
|---|---|
| 599 | N2-[(3,4-dimethoxyphenyl)methyl]-1-methyl-N6-(3,4,5-trimethoxyphenyl)imidazo[4,5-c]pyridine-2,6-diamine |
| 600 | N-[4-[[2-[(3,4-dimethoxyphenyl)methylamino]-1-methyl-imidazo[4,5-c]pyridin-6-yl]amino]phenyl]acetamide |
| 601 | 4-[[2-[(3,4-dimethoxyphenyl)methylamino]-1-methyl-imidazo[4,5-c]pyridin-6-yl]amino]benzamide |
| 602 | N6-(2,4-dimethoxyphenyl)-N2-[(3,4-dimethoxyphenyl)methyl]-1-methyl-imidazo[4,5-c]pyridine-2,6-diamine |
| 603 | N6-(3,4-dimethoxyphenyl)-N2-[(3,4-dimethoxyphenyl)methyl]-1-methyl-imidazo[4,5-c]pyridine-2,6-diamine |
| 604 | N2-[(3,4-dimethoxyphenyl)methyl]-1-methyl-N6-phenyl-imidazo[4,5-c]pyridine-2,6-diamine |
| 605 | N2-[(3,4-dimethoxyphenyl)methyl]-1-methyl-N6-[4-(4-methylpiperazin-1-yl)phenyl]imidazo[4,5-c]pyridine-2,6-diamine |
| 606 | N2-[(3,4-dimethoxyphenyl)methyl]-N6-(4-methoxyphenyl)-1-methyl-imidazo[4,5-c]pyridine-2,6-diamine |
| 607 | N2-[(3,4-dimethoxyphenyl)methyl]-N6-(4-fluorophenyl)-1-methyl-imidazo[4,5-c]pyridine-2,6-diamine |
| 608 | N2-[(3,4-dimethoxyphenyl)methyl]-1-methyl-N6-(3-methylsulfonylphenyl)imidazo[4,5-c]pyridine-2,6-diamine |
| 609 | N2,1-dimethyl-N6-(3,4,5-trimethoxyphenyl)imidazo[4,5-c]pyridine-2,6-diamine |
| 610 | N6-(2,4-dimethoxyphenyl)-N2,1-dimethyl-imidazo[4,5-c]pyridine-2,6-diamine |
| 611 | N6-(4-fluorophenyl)-N2,1-dimethyl-imidazo[4,5-c]pyridine-2,6-diamine |
| 612 | 2-methoxy-4-[1-methyl-2-(methylamino)imidazo[4,5-c]pyridin-6-yl]phenol |
| 613 | N-(2-methoxyphenyl)-1-methyl-6-(3-pyridyl)imidazo[4,5-c]pyridin-2-amine |
| 614 | N-benzyl-1-methyl-6-(4-morpholinophenyl)imidazo[4,5-c]pyridin-2-amine |
| 615 | N-benzyl-6-(4-dimethylaminophenyl)-1-methyl-imidazo[4,5-c]pyridin-2-amine |
| 616 | N2-benzyl-1-methyl-N6-(3,4,5-trimethoxyphenyl)imidazo[4,5-c]pyridine-2,6-diamine |
| 617 | N-[4-[[2-(benzylamino)-1-methyl-imidazo[4,5-c]pyridin-6-yl]amino]phenyl]acetamide |
| 618 | N2-benzyl-N6-(6-methoxy-3-pyridyl)-1-methyl-imidazo[4,5-c]pyridine-2,6-diamine |
| 619 | N2-benzyl-N6-[3-(dimethylamino)phenyl]-1-methyl-imidazo[4,5-c]pyridine-2,6-diamine |
| 620 | N-benzyl-6-[3-(dimethylamino)phenyl]-1-methyl-imidazo[4,5-c]pyridin-2-amine |
| 621 | N2-benzyl-N6-(4-methoxyphenyl)-1-methyl-imidazo[4,5-c]pyridine-2,6-diamine |
| 622 | 3-[[2-(benzylamino)-1-methyl-imidazo[4,5-c]pyridin-6-yl]amino]phenol |
| 623 | N-benzyl-1-methyl-6-pyrimidin-5-yl-imidazo[4,5-c]pyridin-2-amine |
| 624 | N-benzyl-1-methyl-6-(1-methylpyrazol-4-yl)imidazo[4,5-c]pyridin-2-amine |
| 625 | 6-(6-amino-3-pyridyl)-N-benzyl-1-methyl-imidazo[4,5-c]pyridin-2-amine |
| 626 | 4-[[2-(benzylamino)-1-methyl-imidazo[4,5-c]pyridin-6-yl]amino]-N,N-dimethyl-benzamide |
| 627 | N-[3-[[2-(benzylamino)-1-methyl-imidazo[4,5-c]pyridin-6-yl]amino]phenyl]acetamide |
| 628 | 1-[3-[[2-(benzylamino)-1-methyl-imidazo[4,5-c]pyridin-6-yl]amino]phenyl]ethanone |
| 629 | N2-benzyl-N6-(3-fluorophenyl)-1-methyl-imidazo[4,5-c]pyridine-2,6-diamine |
| 630 | 1-methyl-6-(4-morpholinophenyl)-N-phenyl-imidazo[4,5-c]pyridin-2-amine |
| 631 | 6-(4-dimethylaminophenyl)-1-methyl-N-phenyl-imidazo[4,5-c]pyridin-2-amine |

(continued)

| compound | name |
|---|---|
| 632 | N6-(3,4-dimethoxyphenyl)-1-methyl-N2-phenyl-imidazo[4,5-c]pyridine-2,6-diamine |
| 633 | 1-methyl-N2-phenyl-N6-(3-pyridyl)imidazo[4,5-c]pyridine-2,6-diamine |
| 634 | 1-methyl-N2,N6-diphenyl-imidazo[4,5-c]pyridine-2,6-diamine |
| 635 | 1-methyl-6-(1-naphthyl)-N-phenyl-imidazo[4,5-c]pyridin-2-amine |
| 636 | 1-methyl-N2-phenyl-N6-(4-pyridyl)imidazo[4,5-c]pyridine-2,6-diamine |
| 637 | 1-methyl-N2-phenyl-N6-(2-pyridyl)imidazo[4,5-c]pyridine-2,6-diamine |
| 638 | 3-[(2-anilino-1-methyl-imidazo[4,5-c]pyridin-6-yl)amino]phenol |
| 639 | 1-methyl-N6-(4-methylsulfonylphenyl)-N2-phenyl-imidazo[4,5-c]pyridine-2,6-diamine |
| 640 | 1-methyl-6-(4-morpholinophenyl)-N-(tetrahydrofuran-2-ylmethyl)imidazo[4,5-c]pyridin-2-amine |
| 641 | 1-methyl-N2-(tetrahydrofuran-2-ylmethyl)-N6-(3,4,5-trimethoxyphenyl)imidazo[4,5-c]pyridine-2,6-diamine |
| 642 | N6-(3-chlorophenyl)-1-methyl-N2-(tetrahydrofuran-2-ylmethyl)imidazo[4,5-c]pyridine-2,6-diamine |
| 643 | N6-(3-chloro-4-fluoro-phenyl)-1-methyl-N2-(tetrahydrofuran-2-ylmethyl)imidazo[4,5-c]pyridine-2,6-diamine |
| 644 | 6-(2-methoxy-3-pyridyl)-1-methyl-N-(tetrahydrofuran-2-ylmethyl)imidazo[4,5-c]pyridin-2-amine |
| 645 | N2-(2-methoxyethyl)-1-methyl-N6-(3,4,5-trimethoxyphenyl)imidazo[4,5-c]pyridine-2,6-diamine |
| 646 | N-[4-[[2-(2-methoxyethylamino)-1-methyl-imidazo[4,5-c]pyridin-6-yl]amino]phenyl]acetamide |
| 647 | 6-[3-(dimethylamino)phenyl]-N-(2-methoxyethyl)-1-methyl-imidazo[4,5-c]pyridin-2-amine |
| 648 | N-(2-methoxyethyl)-1-methyl-6-(4-methylsulfonylphenyl)imidazo[4,5-c]pyridin-2-amine |
| 649 | methyl 4-[[2-(2-methoxyethylamino)-1-methyl-imidazo[4,5-c]pyridin-6-yl]amino]benzoate |
| 650 | N-(2-methoxyethyl)-6-(4-methoxy-2-methyl-phenyl)-1-methyl-imidazo[4,5-c]pyridin-2-amine |
| 651 | N-(2-methoxyethyl)-1-methyl-6-(1-methylpyrazol-4-yl)imidazo[4,5-c]pyridin-2-amine |
| 652 | 3-[2-(2-methoxyethylamino)-1-methyl-imidazo[4,5-c]pyridin-6-yl]-N-methyl-benzamide |
| 653 | N-[(2-fluorophenyl)methyl]-1-methyl-6-(4-morpholinophenyl)imidazo[4,5-c]pyridin-2-amine |
| 654 | 6-(4-dimethylaminophenyl)-N-[(2-fluorophenyl)methyl]-1-methyl-imidazo[4,5-c]pyridin-2-amine |
| 655 | [4-[2-[(2-fluorophenyl)methylamino]-1-methyl-imidazo[4,5-c]pyridin-6-yl]phenyl]methanol |
| 656 | 4-[2-[(2-fluorophenyl)methylamino]-1-methyl-imidazo[4,5-c]pyridin-6-yl]-2-methoxy-phenol |
| 657 | 6-(4-fluorophenyl)-N-[(2-fluorophenyl)methyl]-1-methyl-imidazo[4,5-c]pyridin-2-amine |
| 658 | N-[(2-fluorophenyl)methyl]-1-methyl-6-(2-thienyl)imidazo[4,5-c]pyridin-2-amine |
| 659 | 6-(3-aminophenyl)-1-[(3,4-dimethoxyphenyl)methyl]-N-methyl-imidazo[4,5-c]pyridin-2-amine |
| 660 | 1-[(3,4-dimethoxyphenyl)methyl]-N2-methyl-N6-(3,4,5-trimethoxyphenyl)imidazo[4,5-c]pyridine-2,6-diamine |
| 661 | N-[4-[[1-[(3,4-dimethoxyphenyl)methyl]-2-(methylamino)imidazo[4,5-c]pyridin-6-yl]amino]phenyl]acetamide |
| 662 | 1-[(3,4-dimethoxyphenyl)methyl]-N6-(3-methoxyphenyl)-N2-methyl-imidazo[4,5-c]pyridine-2,6-diamine |
| 663 | 4-[[1-[(3,4-dimethoxyphenyl)methyl]-2-(methylamino)imidazo[4,5-c]pyridin-6-yl]amino]phenol |
| 664 | 4-[[1-[(3,4-dimethoxyphenyl)methyl]-2-(methylamino)imidazo[4,5-c]pyridin-6-yl]amino]benzamide |

(continued)

| compound | name |
|---|---|
| 665 | N6-(3,4-dimethoxyphenyl)-1-[(3,4-dimethoxyphenyl)methyl]-N2-methyl-imidazo[4,5-c]pyridine-2,6-diamine |
| 666 | N6-(3-chlorophenyl)-1-[(3,4-dimethoxyphenyl)methyl]-N2-methyl-imidazo[4,5-c]pyridine-2,6-diamine |
| 667 | 1-[(3,4-dimethoxyphenyl)methyl]-N2-methyl-N6-phenyl-imidazo[4,5-c]pyridine-2,6-diamine |
| 668 | 1-[(3,4-dimethoxyphenyl)methyl]-N-methyl-6-(2-naphthyl)imidazo[4,5-c]pyridin-2-amine |
| 669 | 1-[(3,4-dimethoxyphenyl)methyl]-N-methyl-6-phenyl-imidazo[4,5-c]pyridin-2-amine |
| 670 | 1-[(3,4-dimethoxyphenyl)methyl]-N-methyl-6-(1-naphthyl)imidazo[4,5-c]pyridin-2-amine |
| 671 | 1-[(3,4-dimethoxyphenyl)methyl]-N2-methyl-N6-(m-tolyl)imidazo[4,5-c]pyridine-2,6-diamine |
| 672 | 1-[(3,4-dimethoxyphenyl)methyl]-6-(4-methoxyphenyl)-N-methyl-imidazo[4,5-c]pyridin-2-amine |
| 673 | 1-[3-[[1-[(3,4-dimethoxyphenyl)methyl]-2-(methylamino)imidazo[4,5-c]pyridin-6-yl]amino]phenyl]ethanone |
| 674 | 1-[(3,4-dimethoxyphenyl)methyl]-N6-(3-fluorophenyl)-N2-methyl-imidazo[4,5-c]pyridine-2,6-diamine |
| 675 | 1-[(3,4-dimethoxyphenyl)methyl]-N2-methyl-N6-(4-methylsulfanylphenyl)imidazo[4,5-c]pyridine-2,6-diamine |
| 676 | 1-[(3,4-dimethoxyphenyl)methyl]-N6-(3-isopropoxyphenyl)-N2-methyl-imidazo[4,5-c]pyridine-2,6-diamine |
| 677 | N-(2-methoxyethyl)-6-(2-naphthyl)-1H-imidazo[4,5-c]pyridin-2-amine |
| 678 | 6-(4-dimethylaminophenyl)-1-(3-fluorophenyl)-N-methyl-imidazo[4,5-c]pyridin-2-amine |
| 679 | 1-(3-fluorophenyl)-N2-methyl-N6-(3,4,5-trimethoxyphenyl)imidazo[4,5-c]pyridine-2,6-diamine |
| 680 | 1-(3-fluorophenyl)-N6-(3-methoxyphenyl)-N2-methyl-imidazo[4,5-c]pyridine-2,6-diamine |
| 681 | 4-[[1-(3-fluorophenyl)-2-(methylamino)imidazo[4,5-c]pyridin-6-yl]amino]benzamide |
| 682 | N6-[3-(dimethylamino)phenyl]-1-(3-fluorophenyl)-N2-methyl-imidazo[4,5-c]pyridine-2,6-diamine |
| 683 | 6-[3-(dimethylamino)phenyl]-1-(3-fluorophenyl)-N-methyl-imidazo[4,5-c]pyridin-2-amine |
| 684 | 1-(3-fluorophenyl)-N-methyl-6-(4-methylsulfonylphenyl)imidazo[4,5-c]pyridin-2-amine |
| 685 | 1-(3-fluorophenyl)-N6-(4-methoxyphenyl)-N2-methyl-imidazo[4,5-c]pyridine-2,6-diamine |
| 686 | 4-[[1-(3-fluorophenyl)-2-(methylamino)imidazo[4,5-c]pyridin-6-yl]amino]benzonitrile |
| 687 | 1-(3-fluorophenyl)-N-methyl-6-(2-thienyl)imidazo[4,5-c]pyridin-2-amine |
| 688 | N-[3-[[1-(3-fluorophenyl)-2-(methylamino)imidazo[4,5-c]pyridin-6-yl]amino]phenyl]acetamide |
| 689 | 1-[3-[[1-(3-fluorophenyl)-2-(methylamino)imidazo[4,5-c]pyridin-6-yl]amino]phenyl]ethanone |
| 690 | N-[3-(trifluoromethyl)phenyl]-1-(3,4,5-trimethoxyphenyl)imidazo[4,5-c]pyridin-6-amine |
| 691 | N1,N1-dimethyl-N3-(1-phenylimidazo[4,5-c]pyridin-6-yl)benzene-1,3-diamine |
| 692 | N-[4-[[1-(2-furylmethyl)imidazo[4,5-c]pyridin-6-yl]amino]phenyl]acetamide |
| 693 | 1-(2-furylmethyl)-N-(4-methoxyphenyl)imidazo[4,5-c]pyridin-6-amine |
| 694 | N-[4-[(1-benzylimidazo[4,5-c]pyridin-6-yl)amino]phenyl]acetamide |
| 695 | N-(1,3-benzodioxol-5-yl)-1-benzyl-imidazo[4,5-c]pyridin-6-amine |
| 696 | 1-(cyclopropylmethyl)-N-(4-methylsulfonylphenyl)imidazo[4,5-c]pyridin-6-amine |
| 697 | 1,2-dimethyl-N-(3,4,5-trimethoxyphenyl)imidazo[4,5-c]pyridin-6-amine |
| 698 | 1-[3-[(1,2-dimethylimidazo[4,5-c]pyridin-6-yl)amino]phenyl]ethanone |
| 699 | 2-benzyl-1-methyl-N-(3,4,5-trimethoxyphenyl)imidazo[4,5-c]pyridin-6-amine |

(continued)

| compound | name |
|---|---|
| 700 | N-[4-[(2-benzyl-1-methyl-imidazo[4,5-c]pyridin-6-yl)amino]phenyl]acetamide |
| 701 | 1-methyl-2-phenyl-N-(3,4,5-trimethoxyphenyl)imidazo[4,5-c]pyridin-6-amine |
| 702 | N-[4-[(1-methyl-2-phenyl-imidazo[4,5-c]pyridin-6-yl)amino]phenyl]acetamide |
| 703 | N-(2,4-dimethoxyphenyl)-1-methyl-2-phenyl-imidazo[4,5-c]pyridin-6-amine |
| 704 | N-(6-methoxy-3-pyridyl)-1-methyl-2-phenyl-imidazo[4,5-c]pyridin-6-amine |
| 705 | N-(4-methoxyphenyl)-1-methyl-2-phenyl-imidazo[4,5-c]pyridin-6-amine |
| 706 | N-(1,3-benzodioxol-5-yl)-1-methyl-2-phenyl-imidazo[4,5-c]pyridin-6-amine |
| 707 | 2-(2-furyl)-1-methyl-N-(3,4,5-trimethoxyphenyl)imidazo[4,5-c]pyridin-6-amine |
| 708 | N-[4-[[2-(2-furyl)-1-methyl-imidazo[4,5-c]pyridin-6-yl]amino]phenyl]acetamide |
| 709 | 2-(2-furyl)-1-methyl-N-(m-tolyl)imidazo[4,5-c]pyridin-6-amine |
| 710 | N-(2-chlorophenyl)-2-(2-furyl)-1-methyl-imidazo[4,5-c]pyridin-6-amine |
| 711 | 4-[[2-(2-furyl)-1-methyl-imidazo[4,5-c]pyridin-6-yl]amino]-N,N-dimethyl-benzamide |
| 712 | N-[3-[[2-(2-furyl)-1-methyl-imidazo[4,5-c]pyridin-6-yl]amino]phenyl]acetamide |
| 713 | N-[4-[[2-(3-fluorophenyl)-1-methyl-imidazo[4,5-c]pyridin-6-yl]amino]phenyl]acetamide |
| 714 | N-(1,3-benzodioxol-5-yl)-2-(3-fluorophenyl)-1-methyl-imidazo[4,5-c]pyridin-6-amine |
| 715 | 4-[[2-(3-fluorophenyl)-1-methyl-imidazo[4,5-c]pyridin-6-yl]amino]-N,N-dimethyl-benzamide |
| 716 | 2-(3,4-dimethoxyphenyl)-1-methyl-N-(3,4,5-trimethoxyphenyl)imidazo[4,5-c]pyridin-6-amine |
| 717 | N-[4-[[2-(3,4-dimethoxyphenyl)-1-methyl-imidazo[4,5-c]pyridin-6-yl]amino]phenyl]acetamide |
| 718 | 2-(3,4-dimethoxyphenyl)-N-(3-methoxyphenyl)-1-methyl-imidazo[4,5-c]pyridin-6-amine |
| 719 | 4-[[2-(3,4-dimethoxyphenyl)-1-methyl-imidazo[4,5-c]pyridin-6-yl]amino]benzamide |
| 720 | 2-(3,4-dimethoxyphenyl)-N-(4-methoxy-2-methyl-phenyl)-1-methyl-imidazo[4,5-c]pyridin-6-amine |
| 721 | 2-(3,4-dimethoxyphenyl)-N-(4-methoxyphenyl)-1-methyl-imidazo[4,5-c]pyridin-6-amine |
| 722 | N-(1,3-benzodioxol-5-yl)-2-(3,4-dimethoxyphenyl)-1-methyl-imidazo[4,5-c]pyridin-6-amine |
| 723 | 5-[[2-(3,4-dimethoxyphenyl)-1-methyl-imidazo[4,5-c]pyridin-6-yl]amino]-2-methoxy-phenol |
| 724 | 1-methyl-2-(4-pyridyl)-N-(3,4,5-trimethoxyphenyl)imidazo[4,5-c]pyridin-6-amine |
| 725 | 1-methyl-N-(3,4,5-trimethoxyphenyl)imidazo[4,5-c]pyridin-6-amine |
| 726 | N-[4-[(1-methylimidazo[4,5-c]pyridin-6-yl)amino]phenyl]acetamide |
| 727 | N-(2,4-dimethoxyphenyl)-1-methyl-imidazo[4,5-c]pyridin-6-amine |
| 728 | N-(4-methoxyphenyl)-1-methyl-imidazo[4,5-c]pyridin-6-amine |
| 729 | 1-methyl-2-(3-pyridylmethyl)-N-(3,4,5-trimethoxyphenyl)imidazo[4,5-c]pyridin-6-amine |
| 730 | N-[4-[[1-[(3,4-dimethoxyphenyl)methyl]imidazo[4,5-c]pyridin-6-yl]amino]phenyl]acetamide |
| 731 | N-(1,3-benzodioxol-5-yl)-1-[(3,4-dimethoxyphenyl)methyl]imidazo[4,5-c]pyridin-6-amine |
| 732 | 3-[1-(3,4,5-trimethoxyphenyl)imidazo[4,5-c]pyridin-6-yl]benzamide |
| 733 | 6-(3-fluorophenyl)-1-phenyl-imidazo[4,5-c]pyridine |
| 734 | 6-(3,4-dimethoxyphenyl)-1-[3-(trifluoromethyl)phenyl]imidazo[4,5-c]pyridine |
| 735 | 5-(1-methyl-2-phenyl-imidazo[4,5-c]pyridin-6-yl)pyridin-2-amine |
| 736 | 3-[2-(2-furyl)-1-methyl-imidazo[4,5-c]pyridin-6-yl]phenol |
| 737 | 1-[(3,4-dimethoxyphenyl)methyl]-6-(3-pyridyl)imidazo[4,5-c]pyridine |

(continued)

| compound | name |
|---|---|
| 738 | 1-[(3,4-dimethoxyphenyl)methyl]-6-(3,4,5-trimethoxyphenyl)imidazo[4,5-c]pyridine |
| 739 | 4-[1-[(3,4-dimethoxyphenyl)methyl]imidazo[4,5-c]pyridin-6-yl]-2-methoxy-phenol |
| 740 | 3-[1-(3-fluorophenyl)imidazo[4,5-c]pyridin-6-yl]-N,N-dimethyl-aniline |
| 741 | 1-phenylimidazo[4,5-c]pyridine-2-thiol |
| 742 | 2-(2-pyridyl)-1H-imidazo[4,5-c]pyridine |
| 743 | 2-(2-thienyl)-3H-imidazo[4,5-c]pyridine |
| 744 | 1-phenylimidazo[4,5-c]pyridine-2-thiol |
| 745 | 2-(methoxymethyl)-1-methyl-N-(3,4,5-trimethoxyphenyl)imidazo[4,5-c]pyridin-6-amine |
| 746 | 4-[2-(methylamino)-1-phenyl-imidazo[4,5-c]pyridin-6-yl]benzamide |
| 747 | 1-[3-[2-[(2-fluorophenyl)methylamino]-1-methyl-imidazo[4,5-c]pyridin-6-yl]phenyl]ethanone |
| 748 | 3-[2-(benzylamino)-1-methyl-imidazo[4,5-c]pyridin-6-yl]benzoic acid |
| 749 | N2,1-dimethyl-N6-(4-phenoxyphenyl)imidazo[4,5-c]pyridine-2,6-diamine |
| 750 | N2-(cyclopropylmethyl)-1-methyl-N6-(3-phenoxyphenyl)imidazo[4,5-c]pyridine-2,6-diamine |
| 751 | N-(2-methoxyethyl)-6-(6-methoxy-3-pyridyl)-1-methyl-imidazo[4,5-c]pyridin-2-amine |
| 752 | 1-phenyl-6-[3-(trifluoromethyl)phenyl]imidazo[4,5-c]pyridine |
| 753 | N6-(3-chloro-4-fluoro-phenyl)-N2-methyl-1-(3,4,5-trimethoxyphenyl)imidazo[4,5-c]pyridine-2,6-diamine |
| 754 | 1-[(3,4-dimethoxyphenyl)methyl]-6-(3-methoxyphenyl)imidazo[4,5-c]pyridine |
| 755 | 1-methyl-N2-(tetrahydrofuran-2-ylmethyl)-N6-[3-(trifluoromethoxy)phenyl]imidazo[4,5-c]pyridine-2,6-diamine |
| 756 | 2-(2-furyl)-1-methyl-6-[3-(trifluoromethoxy)phenyl]imidazo[4,5-c]pyridine |
| 757 | 6-(2-methoxyphenyl)-N-methyl-1-phenyl-imidazo[4,5-c]pyridin-2-amine |
| 758 | N-(cyclopropylmethyl)-1-methyl-6-(3-phenoxyphenyl)imidazo[4,5-c]pyridin-2-amine |
| 759 | 6-(1,3-benzodioxol-5-yl)-N-(2-methoxyethyl)-1-methyl-imidazo[4,5-c]pyridin-2-amine |
| 760 | 1-(cyclopropylmethyl)-N6-(4-methoxy-2-methyl-phenyl)-N2-methyl-imidazo[4,5-c]pyridine-2,6-diamine |
| 761 | N-[4-[1-(3-fluorophenyl)imidazo[4,5-c]pyridin-6-yl]phenyl]acetamide |
| 762 | 1-benzyl-N-methyl-6-[3-(trifluoromethoxy)phenyl]imidazo[4,5-c]pyridin-2-amine |
| 763 | N2-(cyclopropylmethyl)-1-methyl-N6-[4-(trifluoromethyl)phenyl]imidazo[4,5-c]pyridine-2,6-diamine |
| 764 | 2-(2-furyl)-6-(5-methoxy-3-pyridyl)-1-methyl-imidazo[4,5-c]pyridine |
| 765 | N2-methyl-1-(3-pyridylmethyl)-N6-[3-(trifluoromethyl)phenyl]imidazo[4,5-c]pyridine-2,6-diamine |
| 766 | 3-[1-benzyl-2-(methylamino)imidazo[4,5-c]pyridin-6-yl]-N-(2-hydroxyethyl)benzamide |
| 767 | 1-(2-furylmethyl)-N6-[3-methoxy-5-(trifluoromethyl)phenyl]-N2-methyl-imidazo[4,5-c]pyridine-2,6-diamine |
| 768 | N-[3-[1-(3-fluorophenyl)-2-(methylamino)imidazo[4,5-c]pyridin-6-yl]phenyl]methanesulfonamide |
| 769 | 6-(3-fluorophenyl)-1-methyl-N-(2-morpholinoethyl)imidazo[4,5-c]pyridin-2-amine |
| 770 | N-(2-methoxyethyl)-1-methyl-6-(o-tolyl)imidazo[4,5-c]pyridin-2-amine |
| 771 | N-(2-dimethylaminoethyl)-3-[1-(3-fluorophenyl)-2-(methylamino)imidazo[4,5-c]pyridin-6-yl]benzamide |

(continued)

| compound | name |
|---|---|
| 772 | N6-(3-isopropylphenyl)-1-(2-methoxyethyl)-N2-methyl-imidazo[4,5-c]pyridine-2,6-diamine |
| 773 | 1-(2-methoxyethyl)-N2-methyl-N6-[4-(trifluoromethoxy)phenyl]imidazo[4,5-c]pyridine-2,6-diamine |
| 774 | 2-(p-tolyl)-3H-imidazo[4,5-c]pyridine |
| 775 | 2-(4-fluorophenyl)-3H-imidazo[4,5-c]pyridine |
| 776 | N-(5-tert-butyl-2-methyl-pyrazol-3-yl)-1-(2-furylmethyl)imidazo[4,5-c]pyridin-6-amine |
| 777 | N6-[2-fluoro-5-(trifluoromethyl)phenyl]-1-(2-furylmethyl)-N2-methyl-imidazo[4,5-c]pyridine-2,6-diamine |
| 778 | 2-(1-methylimidazo[4,5-c]pyridin-2-yl)quinoline |
| 779 | 2-(4-fluorophenyl)-3H-imidazo[4,5-c]pyridine |
| 780 | 1-methylimidazo[4,5-c]pyridine-2-sulfonic acid |
| 781 | 6-(4-dimethylaminophenyl)-N-methyl-1-phenyl-imidazo[4,5-c]pyridin-2-amine |
| 782 | 6-(2-methoxyphenyl)-N-methyl-1-phenyl-imidazo[4,5-c]pyridin-2-amine |
| 783 | 6-[3-(dimethylamino)phenyl]-N-methyl-1-phenyl-imidazo[4,5-c]pyridin-2-amine |
| 784 | 6-(3-chloro-4-fluoro-phenyl)-N-methyl-1-phenyl-imidazo[4,5-c]pyridin-2-amine |
| 785 | [4-[2-(methylamino)-1-phenyl-imidazo[4,5-c]pyridin-6-yl]phenyl]methanol |
| 786 | 4-[2-(methylamino)-1-phenyl-imidazo[4,5-c]pyridin-6-yl]benzamide |
| 787 | N-methyl-1-phenyl-6-(2-thienyl)imidazo[4,5-c]pyridin-2-amine |
| 788 | 6-(2-methoxy-3-pyridyl)-N-methyl-1-phenyl-imidazo[4,5-c]pyridin-2-amine |
| 789 | 1-benzyl-6-(4-dimethylaminophenyl)-N-methyl-imidazo[4,5-c]pyridin-2-amine |
| 790 | 1-benzyl-N-methyl-6-(2-phenoxyphenyl)imidazo[4,5-c]pyridin-2-amine |
| 791 | 6-(1,3-benzodioxol-5-yl)-1-benzyl-N-methyl-imidazo[4,5-c]pyridin-2-amine |
| 792 | 1-benzyl-6-(4-chlorophenyl)-N-methyl-imidazo[4,5-c]pyridin-2-amine |
| 793 | 3-[1-benzyl-2-(methylamino)imidazo[4,5-c]pyridin-6-yl]-N-(2-dimethylaminoethyl)benzamide |
| 794 | 3-[1-benzyl-2-(methylamino)imidazo[4,5-c]pyridin-6-yl]-N-(2-hydroxyethyl)benzamide |
| 795 | 1-benzyl-6-(3-chlorophenyl)-N-methyl-imidazo[4,5-c]pyridin-2-amine |
| 796 | 3-[1-benzyl-2-(methylamino)imidazo[4,5-c]pyridin-6-yl]phenol |
| 797 | 4-[1-benzyl-2-(methylamino)imidazo[4,5-c]pyridin-6-yl]-2-methoxy-phenol |
| 798 | 1-benzyl-6-(3-furyl)-N-methyl-imidazo[4,5-c]pyridin-2-amine |
| 799 | N-[4-[1-benzyl-2-(methylamino)imidazo[4,5-c]pyridin-6-yl]phenyl]acetamide |
| 800 | N-methyl-6-(3-pyridyl)-1-(3-pyridylmethyl)imidazo[4,5-c]pyridin-2-amine |
| 801 | N-methyl-1-(3-pyridylmethyl)-6-(2-thienyl)imidazo[4,5-c]pyridin-2-amine |
| 802 | 6-(4-dimethylaminophenyl)-1-methyl-N-(m-tolylmethyl)imidazo[4,5-c]pyridin-2-amine |
| 803 | 6-(3,4-dimethoxyphenyl)-1-methyl-N-(m-tolylmethyl)imidazo[4,5-c]pyridin-2-amine |
| 804 | N-[3-[1-methyl-2-(m-tolylmethylamino)imidazo[4,5-c]pyridin-6-yl]phenyl]acetamide |
| 805 | 6-(4-chlorophenyl)-1-methyl-N-(m-tolylmethyl)imidazo[4,5-c]pyridin-2-amine |
| 806 | 1-methyl-6-(4-methylsulfonylphenyl)-N-(m-tolylmethyl)imidazo[4,5-c]pyridin-2-amine |
| 807 | 6-(4-methoxyphenyl)-1-methyl-N-(m-tolylmethyl)imidazo[4,5-c]pyridin-2-amine |
| 808 | 2-methoxy-4-[1-methyl-2-(m-tolylmethylamino)imidazo[4,5-c]pyridin-6-yl]phenol |

(continued)

| compound | name |
|---|---|
| 809 | 6-(3-furyl)-1-methyl-N-(m-tolylmethyl)imidazo[4,5-c]pyridin-2-amine |
| 810 | 6-(2-methoxy-3-pyridyl)-1-methyl-N-(m-tolylmethyl)imidazo[4,5-c]pyridin-2-amine |
| 811 | 6-(6-amino-3-pyridyl)-1-methyl-N-(m-tolylmethyl)imidazo[4,5-c]pyridin-2-amine |
| 812 | 1-methyl-6-phenyl-N-(3,4,5-trimethoxyphenyl)imidazo[4,5-c]pyridin-2-amine |
| 813 | 1-methyl-6-(3-pyridyl)-N-(3,4,5-trimethoxyphenyl)imidazo[4,5-c]pyridin-2-amine |
| 814 | 6-(3-fluorophenyl)-1-methyl-N-(3,4,5-trimethoxyphenyl)imidazo[4,5-c]pyridin-2-amine |
| 815 | 6-(6-amino-3-pyridyl)-1-methyl-N-[3-(trifluoromethyl)phenyl]imidazo[4,5-c]pyridin-2-amine |
| 816 | 6-(1,3-benzodioxol-5-yl)-N-[2-(2,5-dimethoxyphenyl)ethyl]-1-methyl-imidazo[4,5-c]pyridin-2-amine |
| 817 | N-[2-(2,5-dimethoxyphenyl)ethyl]-1-methyl-6-(4-pyridyl)imidazo[4,5-c]pyridin-2-amine |
| 818 | N-[2-(2,5-dimethoxyphenyl)ethyl]-1-methyl-6-(3-thienyl)imidazo[4,5-c]pyridin-2-amine |
| 819 | N-[2-(2,5-dimethoxyphenyl)ethyl]-1-methyl-6-(3,4,5-trimethoxyphenyl)imidazo[4,5-c]pyridin-2-amine |
| 820 | 3-[2-[2-(2,5-dimethoxyphenyl)ethylamino]-1-methyl-imidazo[4,5-c]pyridin-6-yl]phenol |
| 821 | 4-[2-[2-(2,5-dimethoxyphenyl)ethylamino]-1-methyl-imidazo[4,5-c]pyridin-6-yl]-2-methoxy-phenol |
| 822 | 3-[2-[2-(2,5-dimethoxyphenyl)ethylamino]-1-methyl-imidazo[4,5-c]pyridin-6-yl]benzamide |
| 823 | N-[2-(2,5-dimethoxyphenyl)ethyl]-6-(3-methoxyphenyl)-1-methyl-imidazo[4,5-c]pyridin-2-amine |
| 824 | N-[2-(2,5-dimethoxyphenyl)ethyl]-1-methyl-6-(2-thienyl)imidazo[4,5-c]pyridin-2-amine |
| 825 | N-[3-[2-[2-(2,5-dimethoxyphenyl)ethylamino]-1-methyl-imidazo[4,5-c]pyridin-6-yl]phenyl]methanesulfonamide |
| 826 | N-(cyclopropylmethyl)-1-methyl-6-phenyl-imidazo[4,5-c]pyridin-2-amine |
| 827 | N-(cyclopropylmethyl)-1-methyl-6-(3-thienyl)imidazo[4,5-c]pyridin-2-amine |
| 828 | N-(cyclopropylmethyl)-6-[3-(dimethylamino)phenyl]-1-methyl-imidazo[4,5-c]pyridin-2-amine |
| 829 | N-(cyclopropylmethyl)-1-methyl-6-(4-piperazin-1-ylphenyl)imidazo[4,5-c]pyridin-2-amine |
| 830 | [4-[1-methyl-2-(methylamino)imidazo[4,5-c]pyridin-6-yl]phenyl]methanol |
| 831 | N,1-dimethyl-6-[3-(trifluoromethyl)phenyl]imidazo[4,5-c]pyridin-2-amine |
| 832 | 2-methoxy-4-[1-methyl-2-(methylamino)imidazo[4,5-c]pyridin-6-yl]phenol |
| 833 | N-(2-methoxyphenyl)-1-methyl-6-(3-pyridyl)imidazo[4,5-c]pyridin-2-amine |
| 834 | N-benzyl-1-methyl-6-(4-morpholinophenyl)imidazo[4,5-c]pyridin-2-amine |
| 835 | N-benzyl-6-(4-dimethylaminophenyl)-1-methyl-imidazo[4,5-c]pyridin-2-amine |
| 836 | N-benzyl-1-methyl-6-phenyl-imidazo[4,5-c]pyridin-2-amine |
| 837 | N-benzyl-6-[3-(dimethylamino)phenyl]-1-methyl-imidazo[4,5-c]pyridin-2-amine |
| 838 | 3-[2-(benzylamino)-1-methyl-imidazo[4,5-c]pyridin-6-yl]benzoic acid |
| 839 | N-benzyl-6-(4-methoxyphenyl)-1-methyl-imidazo[4,5-c]pyridin-2-amine |
| 840 | N-benzyl-1-methyl-6-pyrimidin-5-yl-imidazo[4,5-c]pyridin-2-amine |
| 841 | N-benzyl-1-methyl-6-(2-thienyl)imidazo[4,5-c]pyridin-2-amine |
| 842 | N-benzyl-1-methyl-6-(1-methylpyrazol-4-yl)imidazo[4,5-c]pyridin-2-amine |
| 843 | 6-(6-amino-3-pyridyl)-N-benzyl-1-methyl-imidazo[4,5-c]pyridin-2-amine |
| 844 | 1-methyl-6-(4-morpholinophenyl)-N-phenyl-imidazo[4,5-c]pyridin-2-amine |
| 845 | 6-(4-dimethylaminophenyl)-1-methyl-N-phenyl-imidazo[4,5-c]pyridin-2-amine |

(continued)

| compound | name |
|---|---|
| 846 | 1-methyl-6-(1-naphthyl)-N-phenyl-imidazo[4,5-c]pyridin-2-amine |
| 847 | 6-(4-methoxyphenyl)-1-methyl-N-phenyl-imidazo[4,5-c]pyridin-2-amine |
| 848 | 6-(3-fluorophenyl)-1-methyl-N-(2-morpholinoethyl)imidazo[4,5-c]pyridin-2-amine |
| 849 | 1-methyl-6-(4-morpholinophenyl)-N-(tetrahydrofuran-2-ylmethyl)imidazo[4,5-c]pyridin-2-amine |
| 850 | 1-methyl-6-(2-naphthyl)-N-(tetrahydrofuran-2-ylmethyl)imidazo[4,5-c]pyridin-2-amine |
| 851 | 1-methyl-6-(3-phenoxyphenyl)-N-(tetrahydrofuran-2-ylmethyl)imidazo[4,5-c]pyridin-2-amine |
| 852 | 1-methyl-N-(tetrahydrofuran-2-ylmethyl)-6-(2-thienyl)imidazo[4,5-c]pyridin-2-amine |
| 853 | 6-(2-methoxy-3-pyridyl)-1-methyl-N-(tetrahydrofuran-2-ylmethyl)imidazo[4,5-c]pyridin-2-amine |
| 854 | 6-[3-(dimethylamino)phenyl]-N-(2-methoxyethyl)-1-methyl-imidazo[4,5-c]pyridin-2-amine |
| 855 | N-(2-methoxyethyl)-1-methyl-6-[3-(trifluoromethoxy)phenyl]imidazo[4,5-c]pyridin-2-amine |
| 856 | N-(2-methoxyethyl)-1-methyl-6-(4-methylsulfonylphenyl)imidazo[4,5-c]pyridin-2-amine |
| 857 | N-(2-methoxyethyl)-6-(6-methoxy-3-pyridyl)-1-methyl-imidazo[4,5-c]pyridin-2-amine |
| 858 | N-(2-methoxyethyl)-1-methyl-6-(o-tolyl)imidazo[4,5-c]pyridin-2-amine |
| 859 | N-(2-methoxyethyl)-6-(4-methoxy-2-methyl-phenyl)-1-methyl-imidazo[4,5-c]pyridin-2-amine |
| 860 | N-(2-methoxyethyl)-1-methyl-6-(1-methylpyrazol-4-yl)imidazo[4,5-c]pyridin-2-amine |
| 861 | 3-[2-(2-methoxyethylamino)-1-methyl-imidazo[4,5-c]pyridin-6-yl]-N-methyl-benzamide |
| 862 | 6-(3,4-dimethoxyphenyl)-1-methyl-N-(3-pyridylmethyl)imidazo[4,5-c]pyridin-2-amine |
| 863 | N-[(2-fluorophenyl)methyl]-1-methyl-6-(4-morpholinophenyl)imidazo[4,5-c]pyridin-2-amine |
| 864 | 6-(4-dimethylaminophenyl)-N-[(2-fluorophenyl)methyl]-1-methyl-imidazo[4,5-c]pyridin-2-amine |
| 865 | 6-(3,4-dimethoxyphenyl)-N-[(2-fluorophenyl)methyl]-1-methyl-imidazo[4,5-c]pyridin-2-amine |
| 866 | 6-(3-chlorophenyl)-N-[(2-fluorophenyl)methyl]-1-methyl-imidazo[4,5-c]pyridin-2-amine |
| 867 | [4-[2-[(2-fluorophenyl)methylamino]-1-methyl-imidazo[4,5-c]pyridin-6-yl]phenyl]methanol |
| 868 | [3-[2-[(2-fluorophenyl)methylamino]-1-methyl-imidazo[4,5-c]pyridin-6-yl]phenyl]methanol |
| 869 | 4-[2-[(2-fluorophenyl)methylamino]-1-methyl-imidazo[4,5-c]pyridin-6-yl]-2-methoxy-phenol |
| 870 | 6-(4-fluorophenyl)-N-[(2-fluorophenyl)methyl]-1-methyl-imidazo[4,5-c]pyridin-2-amine |
| 871 | 1-[3-[2-[(2-fluorophenyl)methylamino]-1-methyl-imidazo[4,5-c]pyridin-6-yl]phenyl]ethanone |
| 872 | N-[(2-fluorophenyl)methyl]-1-methyl-6-(2-thienyl)imidazo[4,5-c]pyridin-2-amine |
| 873 | 6-(3-aminophenyl)-1-[(3,4-dimethoxyphenyl)methyl]-N-methyl-imidazo[4,5-c]pyridin-2-amine |
| 874 | 1-[(3,4-dimethoxyphenyl)methyl]-N-methyl-6-(2-naphthyl)imidazo[4,5-c]pyridin-2-amine |
| 875 | 1-[(3,4-dimethoxyphenyl)methyl]-N-methyl-6-phenyl-imidazo[4,5-c]pyridin-2-amine |
| 876 | 1-[(3,4-dimethoxyphenyl)methyl]-N-methyl-6-(1-naphthyl)imidazo[4,5-c]pyridin-2-amine |
| 877 | 1-[(3,4-dimethoxyphenyl)methyl]-N-methyl-6-(3-thienyl)imidazo[4,5-c]pyridin-2-amine |
| 878 | 1-[(3,4-dimethoxyphenyl)methyl]-6-(4-methoxyphenyl)-N-methyl-imidazo[4,5-c]pyridin-2-amine |
| 879 | 4-[1-[(3,4-dimethoxyphenyl)methyl]-2-(methylamino)imidazo[4,5-c]pyridin-6-yl]-2-methoxy-phenol |
| 880 | 1-[(3,4-dimethoxyphenyl)methyl]-N-methyl-6-(2-thienyl)imidazo[4,5-c]pyridin-2-amine |
| 881 | 6-(5-methoxy-3-pyridyl)-N-(tetrahydrofuran-2-ylmethyl)-1H-imidazo[4,5-c]pyridin-2-amine |
| 882 | N-(2-methoxyethyl)-6-(2-naphthyl)-1H-imidazo[4,5-c]pyridin-2-amine |
| 883 | 6-(4-dimethylaminophenyl)-1-(3-fluorophenyl)-N-methyl-imidazo[4,5-c]pyridin-2-amine |

(continued)

| compound | name |
|---|---|
| 884 | 1-(3-fluorophenyl)-N-methyl-6-(1-naphthyl)imidazo[4,5-c]pyridin-2-amine |
| 885 | 6-(1,3-benzodioxol-5-yl)-1-(3-fluorophenyl)-N-methyl-imidazo[4,5-c]pyridin-2-amine |
| 886 | 6-[3-(dimethylamino)phenyl]-1-(3-fluorophenyl)-N-methyl-imidazo[4,5-c]pyridin-2-amine |
| 887 | 1-(3-fluorophenyl)-N-methyl-6-(4-methylsulfonylphenyl)imidazo[4,5-c]pyridin-2-amine |
| 888 | 4-[1-(3-fluorophenyl)-2-(methylamino)imidazo[4,5-c]pyridin-6-yl]-2-methoxy-phenol |
| 889 | 1-(3-fluorophenyl)-N-methyl-6-(2-thienyl)imidazo[4,5-c]pyridin-2-amine |

**5.** Compound according to any one of claims 1 - 4 for use as pharmaceutically active agent in medicine.

**6.** Compound according to any one of claims 1 - 4 for the inhibition of GRK5.

**7.** Compound according to any one of claims 1 - 4 for use in treatment or prophylaxis of a metabolic disease.

**8.** Compound according to claim 7 for use in treatment or prophylaxis of a disease selected from diabetes, obesity and impaired adipogenesis.

**9.** Compound according to claim 7 or 8, wherein the disease is diabetes mellitus type 2.

**10.** Compound according to any one of claims 1 - 9 for use in glucose dependent regulation of insulin production and/or release of insulin.

**11.** Compound according to any one of claims 1 - 10 for increasing glucose uptake.

**12.** Pharmaceutical composition comprising at least one compound according to any one of claims 1 - 4 together with at least one pharmaceutically acceptable carrier, solvent or excipient.

**13.** Pharmaceutical composition according to claim 12 further comprising at least one active agent selected from the group consisting of an anti-diabetic drug.

**14.** Pharmaceutical composition according to claim 12 or 14, wherein the anti-diabetic drug is selected from thiazolidinediones, metformin and sulfonylurea.

Europäisches Patentamt
European Patent Office
Office européen des brevets

# EUROPEAN SEARCH REPORT

Application Number
EP 13 17 3968

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TOMOKO MINENO ET AL: "A convergent synthesis of the imidazopyridine scaffold of fluorescent alkaloid ageladine A", TETRAHEDRON LETTERS, vol. 52, no. 24, 1 June 2011 (2011-06-01), pages 3131-3132, XP055077087, ISSN: 0040-4039, DOI: 10.1016/j.tetlet.2011.04.024 * compound 9 * ----- | 1 | INV. C07D471/04 A61K31/437 A61P3/00 A61P3/10 |
| X | WO 2010/089292 A1 (ORTHO MCNEIL JANSSEN PHARM [US]; GIJSEN HENRICUS JACOBUS MARIA [NL]; V) 12 August 2010 (2010-08-12) * intermediates 24 and 41 * ----- | 1 | |
| X | WO 2010/094647 A1 (ORTHO MCNEIL JANSSEN PHARM [US]; GIJSEN HENRICUS JACOBUS MARIA [NL]; B) 26 August 2010 (2010-08-26) * intermediates 34, 35, 73 * ----- | 1 | |
| X | NAOKI ANDO ET AL: "Synthesis and Matrix Metalloproteinase-12 Inhibitory Activity of Ageladine A Analogs", CHEMICAL & PHARMACEUTICAL BULLETIN, vol. 59, no. 5, 1 January 2011 (2011-01-01), pages 579-596, XP055077090, ISSN: 0009-2363, DOI: 10.1248/cpb.59.579 * table 3; compounds 31,33 * ----- | 1,5,12 | TECHNICAL FIELDS SEARCHED (IPC) C07D |
| X | WO 2009/103966 A1 (CANCER REC TECH LTD [GB]; COLLINS IAN [GB]; READER JOHN CHARLES [GB];) 27 August 2009 (2009-08-27) * synthese 7-1-b, p.103; synthese 9-2-A, p.106; * ----- -/-- | 1,5,12 | |

1

The present search report has been drawn up for all claims

| Place of search | Date of completion of the search | Examiner |
|---|---|---|
| Munich | 2 September 2013 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3968

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/123745 A1 (CANCER REC TECH LTD [GB]; BAVETSIAS VASSILIOS [GB]; ATRASH BUTRUS [GB]) 20 September 2012 (2012-09-20) * compounds 138-141, 144-189; compounds 66,70,94,111-114,118,119,122,126-128,130-135 * | 1,2,5,12 | |
| X | WO 2006/114180 A1 (MERCK PATENT GMBH [DE]; HEINRICH TIMO [DE]; BLAUKAT ANDREE [DE]; STAEH) 2 November 2006 (2006-11-02) * compounds 43,48 * | 1,2,5,12 | |
| X | EP 0 079 083 A1 (WELLCOME FOUND [GB]) 18 May 1983 (1983-05-18) * examples 39,46-48 * | 1,3 | |
| X | KIYAMA R ET AL: "Synthesis and Evaluation of Novel Nonpeptide Angiotensin II Receptor Antagonists: Imidazo[4,5-c]pyridine Derivatives with an Aromatic Substituent", CHEMICAL AND PHARMACEUTICAL BULLETIN, PHARMACEUTICAL SOCIETY OF JAPAN, JP, vol. 43, no. 3, 1 March 1995 (1995-03-01), pages 450-460, XP001206481, ISSN: 0009-2363 * table II; compounds 11a-11c, 11e-11k * * chart 7, compound 33; * | 1,3,5,12 | TECHNICAL FIELDS SEARCHED (IPC) |

-/--

The present search report has been drawn up for all claims

1

| Place of search | Date of completion of the search | Examiner |
|---|---|---|
| Munich | 2 September 2013 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3968

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SUDHIR R. SHENGULE ET AL: "A One-Pot Synthesis and Biological Activity of Ageladine A and Analogues", JOURNAL OF MEDICINAL CHEMISTRY, vol. 54, no. 7, 14 April 2011 (2011-04-14), pages 2492-2503, XP055077085, ISSN: 0022-2623, DOI: 10.1021/jm200039m * compounds 3-10 * ----- | 1,3,5,12 | |
| X | YOUNG R C ET AL: "PURINE DERIVATIVES AS COMPETITIVE INHIBITORS OF HUMAN ERYTHROCYTE MEMBRANE PHOSPHATIDYLINOSITOL 4-KINASE", JOURNAL OF MEDICINAL CHEMISTRY, AMERICAN CHEMICAL SOCIETY, US, vol. 33, no. 8, 1 August 1990 (1990-08-01), pages 2073-2080, XP000573808, ISSN: 0022-2623, DOI: 10.1021/JM00170A005 * compounds 15,16 * ----- | 1,3,5,12 | |
| X | GELDENHUYS W J ET AL: "Virtual Screening to Identify Novel Antagonists for the G Protein-Coupled NK3 Receptor", JOURNAL OF MEDICINAL CHEMISTRY, AMERICAN CHEMICAL SOCIETY, US, vol. 53, no. 22, 3 November 2010 (2010-11-03), pages 8080-8088, XP002634349, ISSN: 0022-2623, DOI: 10.1021/JM1010012 * compounds 50,51 * ----- | 1,3,5,12 | TECHNICAL FIELDS SEARCHED (IPC) |
| X | WO 99/01454 A1 (DU PONT PHARM CO [US]) 14 January 1999 (1999-01-14) * compounds, 250-261, 264,265, 350-361, 364, 365, 418, 426, 428; claim 6; tables 1-5; compounds 50-61,64,65,151-161,164,165,250-261,264,265,350-36 * ----- -/-- | 1,3,5,8, 12 | |

The present search report has been drawn up for all claims

1

| Place of search | Date of completion of the search | Examiner |
|---|---|---|
| Munich | 2 September 2013 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3968

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/113802 A2 (HOFFMANN LA ROCHE [CH]; LAI YINGJIE [US]; LIANG JUN [US]; MAGNUSON STE) 22 September 2011 (2011-09-22) * see further compounds in table;; page 89, line 17; examples i, 1,3,6,8,9,12,13,18,21-134 * ----- | 1,3,5, 7-9,12 | |
| X | EP 1 221 444 A1 (EISAI CO LTD [JP]) 10 July 2002 (2002-07-10) * claims 1,16; compounds 42-44 * ----- | 1,3-14 | |
| | | | TECHNICAL FIELDS SEARCHED (IPC) |

The present search report has been drawn up for all claims

1

| Place of search | Date of completion of the search | Examiner |
|---|---|---|
| Munich | 2 September 2013 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 17 3968

This annex lists the patent family members relating to the patent documents cited in the above-mentioned European search report. The members are as contained in the European Patent Office EDP file on
The European Patent Office is in no way liable for these particulars which are merely given for the purpose of information.

02-09-2013

| Patent document cited in search report | Publication date | Patent family member(s) | Publication date |
|---|---|---|---|
| WO 2010089292 A1 | 12-08-2010 | AR 075366 A1 | 30-03-2011 |
| | | AU 2010211109 A1 | 21-07-2011 |
| | | CA 2748862 A1 | 12-08-2010 |
| | | CN 102325765 A | 18-01-2012 |
| | | EA 201171008 A1 | 28-02-2012 |
| | | EP 2393804 A1 | 14-12-2011 |
| | | JP 2012516870 A | 26-07-2012 |
| | | KR 20110113197 A | 14-10-2011 |
| | | NZ 593951 A | 25-01-2013 |
| | | SG 173510 A1 | 29-09-2011 |
| | | TW 201040176 A | 16-11-2010 |
| | | US 2011281881 A1 | 17-11-2011 |
| | | WO 2010089292 A1 | 12-08-2010 |
| WO 2010094647 A1 | 26-08-2010 | AR 075520 A1 | 06-04-2011 |
| | | AU 2010215579 A1 | 04-08-2011 |
| | | CA 2749495 A1 | 26-08-2010 |
| | | CN 102341388 A | 01-02-2012 |
| | | EA 201171066 A1 | 30-04-2012 |
| | | EP 2398793 A1 | 28-12-2011 |
| | | JP 2012518024 A | 09-08-2012 |
| | | KR 20110124772 A | 17-11-2011 |
| | | SG 173668 A1 | 29-09-2011 |
| | | TW 201040177 A | 16-11-2010 |
| | | US 2012022090 A1 | 26-01-2012 |
| | | WO 2010094647 A1 | 26-08-2010 |
| WO 2009103966 A1 | 27-08-2009 | EP 2288601 A1 | 02-03-2011 |
| | | US 2010331328 A1 | 30-12-2010 |
| | | WO 2009103966 A1 | 27-08-2009 |
| WO 2012123745 A1 | 20-09-2012 | NONE | |
| WO 2006114180 A1 | 02-11-2006 | AR 056986 A1 | 07-11-2007 |
| | | AT 524467 T | 15-09-2011 |
| | | AU 2006239632 A1 | 02-11-2006 |
| | | CA 2605738 A1 | 02-11-2006 |
| | | EP 1874769 A1 | 09-01-2008 |
| | | JP 5033119 B2 | 26-09-2012 |
| | | JP 2008538773 A | 06-11-2008 |
| | | US 2008176892 A1 | 24-07-2008 |
| | | WO 2006114180 A1 | 02-11-2006 |
| EP 0079083 A1 | 18-05-1983 | AR 241247 A1 | 31-03-1992 |
| | | AU 9030682 A | 19-05-1983 |
| | | DD 205904 A5 | 11-01-1984 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 17 3968

This annex lists the patent family members relating to the patent documents cited in the above-mentioned European search report. The members are as contained in the European Patent Office EDP file on
The European Patent Office is in no way liable for these particulars which are merely given for the purpose of information.

02-09-2013

| Patent document cited in search report | Publication date | Patent family member(s) | Publication date |
|---|---|---|---|
| | | DK 499582 A | 11-05-1983 |
| | | EP 0079083 A1 | 18-05-1983 |
| | | ES 8401486 A1 | 01-03-1984 |
| | | ES 8503351 A1 | 01-06-1985 |
| | | FI 823846 A | 11-05-1983 |
| | | GB 2113675 A | 10-08-1983 |
| | | GR 76746 A1 | 30-08-1984 |
| | | IE 56478 B1 | 14-08-1991 |
| | | MC 1491 A | 12-09-1983 |
| | | NO 823732 A | 11-05-1983 |
| | | PH 22764 A | 12-12-1988 |
| | | PT 75820 A | 01-12-1982 |
| | | US 4603139 A | 29-07-1986 |
| WO 9901454 A1 | 14-01-1999 | AR 016307 A1 | 04-07-2001 |
| | | AU 746706 B2 | 02-05-2002 |
| | | AU 8181998 A | 25-01-1999 |
| | | BR 9810508 A | 05-09-2000 |
| | | CA 2294117 A1 | 14-01-1999 |
| | | CN 1268137 A | 27-09-2000 |
| | | EE 9900607 A | 15-08-2000 |
| | | EP 0994877 A1 | 26-04-2000 |
| | | HR P980375 A2 | 30-04-1999 |
| | | HU 0100179 A2 | 28-11-2001 |
| | | JP 2002507996 A | 12-03-2002 |
| | | MY 132871 A | 31-10-2007 |
| | | NO 996483 A | 02-03-2000 |
| | | NZ 502642 A | 28-06-2002 |
| | | PL 337888 A1 | 11-09-2000 |
| | | SK 179899 A3 | 03-12-2001 |
| | | TW 589309 B | 01-06-2004 |
| | | US 6143743 A | 07-11-2000 |
| | | US 6362180 B1 | 26-03-2002 |
| | | US 2003114468 A1 | 19-06-2003 |
| | | WO 9901454 A1 | 14-01-1999 |
| WO 2011113802 A2 | 22-09-2011 | CA 2793024 A1 | 22-09-2011 |
| | | CN 102869359 A | 09-01-2013 |
| | | EP 2547338 A2 | 23-01-2013 |
| | | JP 2013522267 A | 13-06-2013 |
| | | KR 20130001272 A | 03-01-2013 |
| | | US 2013096104 A1 | 18-04-2013 |
| | | WO 2011113802 A2 | 22-09-2011 |
| EP 1221444 A1 | 10-07-2002 | AT 303387 T | 15-09-2005 |
| | | AU 778450 B2 | 09-12-2004 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 17 3968

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2013

| Patent document cited in search report | Publication date | Patent family member(s) | Publication date |
|---|---|---|---|
| | | AU 5571700 A | 22-01-2001 |
| | | CA 2376835 A1 | 11-01-2001 |
| | | CN 1365361 A | 21-08-2002 |
| | | DE 60022366 D1 | 06-10-2005 |
| | | DE 60022366 T2 | 14-06-2006 |
| | | DK 1221444 T3 | 14-11-2005 |
| | | EP 1221444 A1 | 10-07-2002 |
| | | ES 2246867 T3 | 01-03-2006 |
| | | JP 4324338 B2 | 02-09-2009 |
| | | NZ 516260 A | 27-08-2004 |
| | | PT 1221444 E | 30-11-2005 |
| | | US 6841549 B1 | 11-01-2005 |
| | | WO 0102400 A1 | 11-01-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012028335 A1


### Non-patent literature cited in the description


- *Biochem. Biophys Res. Commun.,* 2012, vol. 421, 312